# EUROPÄISCHE PATENTSCHRIFT

(11) **EP 1 438 053 B1**
(45) Veröffentlichungstag und Bekanntmachung des Hinweises auf die Patenterteilung: **10.09.2008**
(21) Anmeldenummer: 02774710.4
(22) Anmeldetag: 14.10.2002
(51) Int. Cl.: A61K 31/506, C07D 239/48, C07D 403/12, C07D 471/04, C07D 401/12, C07D 413/12, A61K 31/505

(54) **PYRIMIDINDERIVATE, ARZNEIMITTEL ENTHALTEND DIESE VERBINDUNGEN, DEREN VERWENDUNG UND VERFAHREN ZU IHRER HERSTELLUNG**
PYRIMIDINE DERIVATIVES, PHARMACEUTICAL AGENT CONTAINING SAID COMPOUNDS, USE AND METHOD FOR MAKING SAME
DERIVES PYRIMIDINE, AGENT PHARMACEUTIQUE CONTENANT CES COMPOSES, UTILISATION ET PROCEDE DE FABRICATION DE CES COMPOSES

(30) Priorität: 17.10.2001 US 330145 P
(43) Veröffentlichungstag der Anmeldung: 21.07.2004
(73) Patentinhaber: Boehringer Ingelheim Pharma GmbH & Co.KG, 55216 Ingelheim am Rhein (DE); Boehringer Ingelheim Pharmaceuticals, Inc., Ridgefield Connecticut 06877 (US); Boehringer Ingelheim International GmbH, 55216 Ingelheim am Rhein (DE)
(72) Erfinder: DAHMANN, Georg, 88448 Attenweiler (DE); HIMMELSBACH, Frank, 88441 Mittelbiberach (DE); WITTNEBEN, Helmut, 69198 Schriesheim (DE); PAUTSCH, Alexander, 89075 Ulm (DE); PROKOPOWICZ, Anthony, S., Patterson, NY 12563 (US); KRIST, Bernd, A-1040 Wien (AT); SCHNAPP, Gisela, 88400 Biberach (DE); STEEGMAIER, Martin, A-1120 Wien (AT); LENTER, Martin, 89073 Ulm (DE); SCHOOP, Andreas, A-1150 Wien (AT); STEURER, Steffen, A-1190 Wien (AT); SPEVAK, Walter, A-2105 Oberrohrbach (AT)
(74) Vertreter: Hammann, Heinz
(86) Internationale Anmeldenummer: PCT/EP2002/011453
(87) Internationale Veröffentlichungsnummer: WO 2003/032997

(56) Entgegenhaltungen:
- EP-A- 0 945 443
- WO-A-00/27825
- WO-A-00/27826
- WO-A-00/39101
- WO-A-00/53595
- WO-A-97/19065
- WO-A-99/41253

## Beschreibung

Es wurde nun überraschenderweise gefunden, dass die Verbindungen der allgemeinen Formel (I) 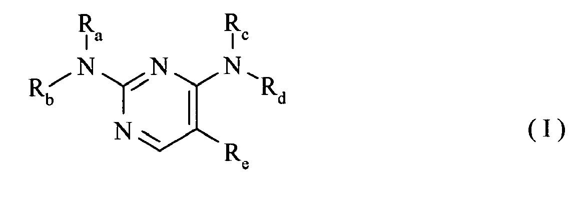 wertvolle pharmakologische Eigenschaften aufweisen, insbesondere eine inhibierende Wirkung auf Proteinkinasen wie SRC Kinasen, PLK Kinase und insbesondere Cyclin-abhängige Kinasen (CDKs, wie z.B. CDK1, CDK2, CDK3, CDK4, CDK5, CDK6, CDK7, CDK8 und CDK9 mit ihren spezifischen Cyclinen A, B1, B2, C, D1, D2, D3, E, F, G1, G2, H, I, K und virales Cyclin) sowie auf die Kinaseaktivität von Aurora B. Die Verbindungen zeigen wertvolle pharmakologische Eigenschaften, wie Neuroprotektion und inhibierende Wirkung auf die Proliferation kultivierter humaner Tumorzellen.

Auf Grund ihrer biologischen Eigenschaften eignen sich die neuen Verbindungen der allgemeinen Formel I, deren Isomere und deren physiologisch verträgliche Salze zur Behandlung von Erkrankungen, die durch exzessive oder anomale Zellteilung charakterisiert sind.

Zu diesen Erkrankungen gehören (ohne Anspruch auf Vollständigkeit): Virale Infektionen (z.B. HIV, Kaposi Sarkom, durch Adeno-, Influenza- oder Cytomegalieviren verursachte Infekte); bakterielle, Pilz- und/oder parasitäre Infektionen; Haut-Erkrankungen (z.B. Psoriasis, Ekzeme, Keratosen); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001, Science, 291, 134-137).

Zudem sind die Verbindungen nützlich zur Immunsuppression (z.B. bei Organtransplantationen) sowie zur Vorbeugung oder Behandlung von Entzündungen oder Autoimmunerkrankungen (z.B. Colitis, Arthritis, Alzheimer Erkrankung, Glomerulonephritis und Wundheilung).

Insbesondere sind diese Verbindungen als zytotoxische oder zytostatische Wirkstoffe nützlich für die Therapie oder für die Vorbeugung von Erkrankungen, die auf einer Proliferation von Tumorzellen beruhen.

Der Begriff "zytotoxische Verbindung" bezeichnet eine Chemikalie, die toxisch auf lebende Zellen wirkt, insbesondere einen Wirkstoff der Krebszellen zerstört. Der Begriff "zytostatische Verbindung" bezeichnet eine Verbindung, die Zellwachstum und -teilung unterdrückt und somit auch die Proliferation von Zellen.

Entsprechend ist ein weiterer Aspekt der Erfindung die Anwendung einer Verbindung aus der Erfindung für die Herstellung einer pharmazeutischen Zusammensetzung für die Behandlung von Krebs. Weiterhin ist ein Aspekt der Erfindung eine Methode für die Behandlung von Krebs durch Gabe einer wirksamen Menge einer pharmazeutischen Zusammensetzung aus der Erfindung am Patienten. Die Indikationen beinhalten die Behandlung von Krebs und hier insbesondere
1) die Behandlung von malignen Neoplasien und Karzinomen einschließlich Brustkrebs, Neoplasien des Verdauungstraktes (Kolorektales Karzinom, Analkarzinom, Pankreaskarzinom, Magenkarzinom, Ösophaguskarzinom, hepatozelluläres Karzinom, Gallenblasenkarzinom), Lungenkrebs, Kopf- und Halstumoren, Ovarialtumoren, Tumoren der Adnexe, Endometriumkarzinom, Prostatakarzinom, Hodentumoren, Urothelkarzinom, Nierenzellkarzinom, Hauttumoren, Schilddrüsenkarzinom und endokrin aktive Tumoren.
2) Sarkome des Knochens und Weichteilsarkome: Osteosarkom, Weichteilsarkom, Ewing-Sarkom, Chondrosarkom, Fibrosarkom, malignes fibröses Histiozytom (MFH), Leiomyosarkome und andere Weichteilsarkome;
3) Maligne Tumoren der Hämopoese: Hodgkin- und Non-Hodgkin-Lymphome; Leukämien, Multiples Myelom, myeloproliferative und myelodysplastische Syndrome;
4) Neuroektodermale Tumoren: Periphere Nervenscheidentumoren, Medulloblastome; Neuroblastome, Retinoblastome, Astrozytome und andere Gehirntumoren
5) Melanome;
6) Mesotheliome.

Die neuen Verbindungen der Formel I können zur Kurz- oder Langzeitbehandlung der vorstehend erwähnten Krankheiten auch gegebenenfalls in Kombination mit anderen "State-of-the-art" Verbindungen wie anderen Cytostatika, Antikörpern, - "targeted therapies", wie Inhibitoren des EGF, Her2 oder VEGF Signaltransduktionsweges oder Angiogenese-Inhibitoren verwendet werden. Für den CDK1 Inhibitor Olomoucine wurde in Zellkultur eine synergistische Wirkung mit zytotoxischen Substanzen gefunden (Ongkeko et al., 1995, J. Cell Sci., 108, 2897). Die genannten Verbindungen können dabei vor oder nach der Gabe von bekannten Antitumormedikamenten oder zytotoxischen Substanzen verabreicht werden. Es ist bekannt, dass die zytotoxische Aktivität des CDK Inhibitors Flavopiridol bei der Kombination mit Krebsmedikamenten durch die Reihenfolge der Verabreichung beeinflusst wird. (Cancer Research, 1997, 57, 3375).

Beispiele für Cytostatika, die geeignet sind gemeinsam mit den Verbindungen der Formel I eingesetzt zu werden, sind Anthracycline wie Doxorubicin, Analoga des Methotrexate wie Methotrexate, Pritrexime, Trimetrexate oder DDMP, Melphalan, Analoga von Cisplatin wie Cisplatin, JM216, JM335, Bis(platinum), Oxaliplatin oder Carboplatin, Analoga von Purinen und

Pyrimidinen wie Cytarabine, Gemcitabine, Azacitidine, 6-Thioguanine, Fludarabine oder 2-Deoxycoformycin und Analoga von anderen Chemotherapeutika wie 9-Aminocamptothecin, D,L-Aminoglutethimide, Trimethoprim, Pyrimethamine, Mitomycin C, Mitoxantrone, Cyclophosphamide, 5-Fluorouracil, Capecitabine, Estramustine, Podophyllotoxin, Bleomycin, Epothilone A, B, C oder D und Derivate von Epothilon wie beispielsweise in US 6,204,388 beschrieben sowie Taxane.

Gegenstand der vorliegenden Erfindung sind somit die neuen 2,4,5-trisubstituierten Pyrimidine der Formel (I) 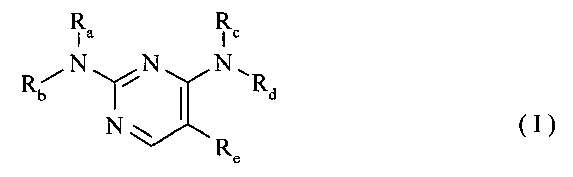 wobei
- **Rₐ**: für ein Wasserstoffatom steht,
- **R**_{b}: für eine gegebenenfalls durch die Reste **R**₁ bis **R**₃ substituierte Phenylgruppe steht, wobei
- **R₁** und **R₂**: jeweils unabhängig voneinander für
ein Fluor-, Chlor-, Brom- oder Iodatom stehen, oder
eine C₁₋₂-Alkyl- oder Hydroxygruppe,
eine C₃₋₇-Cycloalkyl- oder C₄₋₇-Cycloalkoxygruppe, die jeweils durch eine oder zwei Alkylgruppen oder durch eine Arylgruppe substituiert sein können,
eine gegebenenfalls durch eine Arylgruppe substituierte C₂₋₅-Alkenylgruppe,
eine gegebenenfalls durch eine Arylgruppe substituierte C₂₋₅-Alkinylgruppe,
eine Aryl-, Aryloxy-, Aralkyl-, Aralkoxy-, Alkylsulfenyl-, Alkylsulfinyl-, Alkylsulfonyl-, Alkylsulfonyloxy-, Trifluormethylsulfenyl-, Trifluormethylsulfinyl-, Trifluormethylsulfo-nyl-, Arylsulfenyl-, Arylsulfinyl-, Arylsulfonyl-, Aralkylsulfenyl-, Aralkylsulfinyl- oder Aralkylsulfonylgruppe,
   eine durch 1 bis 3 Fluoratome substituierte Methyl- oder Methoxygruppe,
   eine durch 1 bis 5 Fluoratome substituierte C₂₋₄-Alkyl- oder C₂₋₄-Alkoxygruppe,
   eine Nitro-, Amino-, Alkylamino-, Dialkylamino-, C₃₋₇-Cycloalkylamino-, N-Alkyl-C₃₋₇-cycloalkylamino-, Arylamino-, N-Alkyl-arylamino-, Aralkylamino- oder N-Alkyl-aralkylaminogruppe,
eine gegebenenfalls durch 1 bis 4 Alkylgruppen substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen jeweils durch eine Carbonylgruppe oder in den vorstehend erwähnten 6-bis 7-gliedrigen Alkyleniminogruppen eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein können,
eine gegebenenfalls durch 1 bis 4 Alkylgruppen substituierte (Alkylenimino)carbonyl-oder (Alkylenimino)sulfonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein kann,
eine Alkylcarbonylamino-, N-Alkyl-alkylcarbonylamino-, Alkyl-sulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Perfluoralkylsulfonylamino-, N-Alkyl-perfluoralkylsulfonylamino-, Alkylcarbonyl-, Arylcarbonyl-, Aralkylcarbonyl-, Aryl-hydroxymethyl-, Aralkyl-hydroxymethyl-, Carboxy-, Alkoxycarbonyl-, Aralkoxycarbonyl-, Aminocarbonyl-, Alkylaminocarbonyl-, Dialkylaminocarbonyl-, Arylaminocarbonyl-, N-Alkyl-arylaminocarbonyl-, Aralkylaminocarbonyl-, N-Alkyl-aralkylaminocarbonyl-, N-Hydroxy-aminocarbonyl-, N-Hydroxy-alkylaminocarbonyl-, N-Alkoxy-aminocarbonyl-, N-Alkoxy-alkylaminocarbonyl-, Cyano-, Azido-, N-Cyano-amino- oder N-Cyano-alkylaminogruppe,
eine Sulfo-, Alkoxysulfonyl-, Aminosulfonyl-, Alkylaminosulfonyl-,
   Dialkylaminosulfonyl-, Arylaminosulfonyl-, Pyridylaminosulfonyl-,
   Pyrimidinylaminosulfonyl-, N-Alkyl-arylaminosulfonyl-, Aralkylaminosulfonyl- oder N-Alkyl-aralkylaminosulfonylgruppe,
eine Phosphono-, O-Alkyl-phosphono-, O,O'-Dialkyl-phosphono-, O-Aralkyl-phosphono-oder O,O'-Diaralkyl-phosphonogruppe,
eine durch **R₄** substituierte C₁₋₂ Alkylgruppe, wobei
   **R₄** eine Hydroxy-, Alkoxy-, Aryloxy-, Aralkoxy-, Amino-, Alkylamino-, Haloalkylamino-, Dialkylamino-, Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl-, Aralkylsulfonyl-, Carboxy-, Alkoxycarbonyl-, Aminocarbonyl-, Alkylaminocarbonyl-, Dialkylaminocarbonyl- oder Cyanogruppe,
   eine gegebenenfalls durch 1 bis 4 Alkylgruppen substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen durch eine Carbonylgruppe oder in den vorstehend erwähnten 6-bis 7-gliedrigen Alkyleniminogruppen eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein können, oder
   eine gegebenenfalls durch 1 bis 4 Alkylgruppen substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen durch eine Carbonylgruppe oder in der vorstehend erwähnten 6-bis 7-gliedrigen Alkyleniminogruppen durch eine oder zwei Hydroxy-, Alkoxy-, Aminocarbonyl-, Alkylaminocabonyl-, Dialkylaminocarbonyl-, Amino-, Alkylamino- und Dialkylaminogruppe substituiert sein kann,
   eine gegebenenfalls durch 1 bis 4 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein kann, darstellt, oder
eine Gruppe der Formel 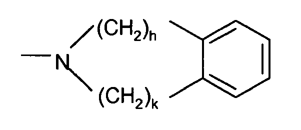 in der
**h** und **k**, die gleich oder verschieden sein können, die Zahlen 1 bis 3 oder **h** die Zahl 0 und **k** die Zahl 2, 3 oder 4 bedeuten, wobei zusätzlich der obige Benzoteil durch Fluor-, Chlor-, Brom- oder Iodatome, durch Alkyl-, Trifluormethyl-, Hydroxy-, Alkoxy-, Carboxy- oder Cyanogruppen mono- oder disubstituiert, wobei die Substituenten jeweils gleich oder verschieden sein können, und der obige gesättigte cyclische Alkyleniminoteil durch 1 oder 2 Alkylgruppen substituiert sein kann,
stehen,
- **R₃**: für ein Fluor-, Chlor- oder Bromatom, eine C₁₋₂ Alkyl-, C₁₋₂ Alkoxy- oder Trifluormethylgruppe steht, oder
für einen 5- oder 6-gliedrigen heterocyclischen, aromatischen Ring mit mindestens einem Stickstoffatom und optional einem Schwefel- oder Sauerstoffatom, der durch eine oder zwei Alkyl-, Aryl- oder Aralkylreste substituiert sein kann, steht, oder
für eine Sulfo-, Alkoxysulfonyl-, Aminosulfonyl-, Alkylaminosulfonyl-, Dialkylaminosulfonyl-, Arylaminosulfonyl-, Pyridylaminosulfonyl-, Pyrimidinylaminosulfonyl-, N-Alkyl-arylaminosulfonyl-, Aralkylaminosulfonyl- oder N-Alkyl-aralkylaminosulfonylgruppe steht,
- **R₂**: zusammen mit **R₃,** sofern diese an benachbarte Kohlenstoffatome gebunden sind, für eine gegebenenfalls durch eine oder zwei Alkylgruppen substituierte Methylendioxygruppe, oder
eine gegebenenfalls durch eine oder zwei Alkylgruppen substituierte n-C₃₋₆-Alkylengruppe, in der eine Methylengruppe durch ein Sauerstoff- oder Schwefelatom, durch eine Sulfinyl-, Sulfonyl-, N-Alkylcarbonyl-imino-, N-Alkylsulfonyl-imino-, N-Arylcarbonyl-imino- oder N-Arylsulfonyl-iminogruppe ersetzt sein kann, oder
eine gegebenenfalls durch ein oder zwei Fluor-, Chlor-, Brom- oder Iodatome, durch eine oder zwei Hydroxy-, Alkyl-, Alkoxy-, Trifluormethyl-, Carboxy-, Alkoxycarbonyl-, Aminocarbonyl-, Alkylaminocarbonyl-, Dialkylaminocarbonyl- oder Cyangruppen substituierte 1,3-Butadien-1,4-diylengruppe, wobei die Substituenten gleich oder verschieden sein können, oder
   eine Gruppe der Formel

   -(CH₂)ₘ-N(**R₅**)-(CH₂)ₙ-,

   in der
   die Methylengruppen der so gebildeten cyclischen Alkyleniminoteile zusätzlich durch 1 oder 2 Alkylgruppen substituiert sein können,
- **R**₅: ein Wasserstoffatom oder eine Alkyl-, Haloalkyl-, Aryl- oder Aralkylgruppe bedeutet, und
**m** und **n,** die gleich oder verschieden sein können, die Zahlen 1, 2 oder 3 bedeuten, wobei in den so gebildeten Alkyleniminoteilen jeweils eine oder zwei zu dem Stickstoffatom benachbarte Methylengruppen durch eine Carbonylgruppe ersetzt sein können, oder
   **m** die Zahl 0 und **n** die Zahl 2, 3 oder 4 bedeuten, wobei in den so gebildeten Alkyleniminoteilen jeweils die zu dem Stickstoffatom benachbarte Methylengruppe durch eine Carbonylgruppe ersetzt sein kann, oder
**R₂** zusammen mit **R₃** eine Gruppe, der Formel -NH-C(=O)-(CH₂)-, -NH-C(=O)-(CH₂)₂-, -NH-N=N-, -NH-N=CH-, -NH-CH=N-, -O-CH=N-, -S-CH=N- oder -NH-CH=CH- und die Tautomere der von -NH-N=N-, -NH-N=CH-, -NH-CH=N- definierten Ringsysteme, wobei jedes Wasserstoffatom durch eine Alkyl-, Aryl- oder Aralkylgruppe substituiert sein kann,
stehen,
**R**_{c}**NR**_{d} eine gegebenenfalls durch 1 bis 4 Alkylgruppen oder 1 bis 2 Arylgruppen substituierte 4-bis 8-gliedrige Alkyleniminogruppe, die zusätzlich durch den Rest **R₆** substituiert ist, wobei
**R₆** für eine Carboxy-, Alkoxycarbonyl-, Aminocarbonyl-, Alkylaminocarbonyl-, Dialkylaminocarbonyl-, Cyano-, Hydroxy-, Alkoxy-, Aryloxy-, Aralkoxy-, Alkylcarbonyloxy-, Arylcarbonyloxy-, Amino-, Alkylamino-, Hydroxy-C2-4-alkylamino-, Dialkylamino-, Cyanamino-, Formylamino-, N-(Alkyl)-N-(hydroxy-C₂₋₄-alkyl)amino-, Bis-(hydroxy-C₂₋₄-alkyl)-aminogruppe, Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl-, Aralkylsulfonyl-,
eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Alkoxycarbonylamino-, N-Alkyl-alkoxycarbonylamino-, Alkoxycarbonylalkylamino-, N-(Alkyl)-N-(alkoxycarbonylalkyl)-amino-, Aralkoxycarbonylamino- oder N-Alkyl-aralkoxycarbonylaminogruppe,
eine (N**R₈R₉**)CON**R₇**- oder (N**R₈R₉**)SO₂N**R₇**-Gruppe, in denen **R₇, R₈** und **R₉,** die gleich oder verschieden sein können, jeweils ein Wasserstoffatom oder eine Alkyl-, Aryl-oder Pyridylgruppe, oder **R₇** und **R₈** zusammen eine n-C₂₋₄-Alkylengruppe und **R₉** ein Wasserstoffatom oder eine Alkyl-, Aryl-oder Pyridylgruppe darstellen,
eine gegebenenfalls durch 1 bis 4 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann, eine gegebenenfalls durch 1 bis 4 Alkylgruppen oder eine Hydroxyalkylgruppe substituierte 4- bis 7-gliedrige Alkyleniminogruppe, wobei in den vorstehend erwähnten 5-bis 7-gliedrigen Alkyleniminogruppen jeweils eine oder zwei zu dem Stickstoffatom benachbarte Methylengruppen durch eine Carbonylgruppe ersetzt sein können,
eine gegebenenfalls durch 1 bis 4 Alkylgruppen oder eine Hydroxyalkylgruppe substituierte 6- oder 7-gliedrige Alkyleniminogruppe, wobei jeweils eine Methylengruppe in 4-Stellung des Alkyleniminoteils durch ein Sauerstoff- oder Schwefelatom, durch eine Carbonyl-, Sulfinyl-, Sulfonyl-, Imino-, N-Alkylimino-, N-Alkylcarbonyl-imino-, N-Alkylsulfonyl-imino-, N-Arylimino- oder N-Aralkyl-iminogruppe ersetzt ist und zusätzlich im Alkyleniminoteil der vorstehend erwähnten Gruppen jeweils eine oder zwei der zu den Stickstoffatomen benachbarten Methylengruppen durch eine Carbonylgruppe ersetzt sein können,
eine durch eine Hydroxy-, Alkoxy-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Carboxy-, Alkoxycarbonyl-, Aminocarbonyl-, Alkylaminocarbonyl- oder Dialkylaminocarbonylgruppe substituierte 4- bis 7-gliedrige Alkyleniminogruppe,
eine durch eine Carboxy-, Alkoxycarbonyl-, Aminocarbonyl-, Alkylaminocarbonyl- oder Dialkylaminocarbonyl-, Cyano-, Hydroxy-, Alkoxy-, Aryloxy-, Amino-, Alkylamin-, Dialkylamino-, Alkylcarbonylamino-, N-Alkyl-alkylcarbonylamino-, Alkoxycarbonylamino-, N-Alkyl-alkoxycarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aminocarbonylalkylamino-, N-(Alkyl)-N-(aminocarbonylalkyl)-amino-, Alkylaminocarbonylalkylamino-, N-(Alkyl)-N-(alkylaminocarbonylalkyl)-amino-, Dialkylaminocarbonylalkylamino-, N-(Alkyl)-N-(dialkylaminocarbonylalkyl)-amino-, Dialkylaminoalkoxy-, Alkylsulfenyl-, Alkylsulfinyl-, Alkylsulfonyl-, Arylsulfenyl-, Arylsulfinyl- oder Arylsulfonylgruppe substituierte Alkylgruppe,
eine gegebenenfalls durch 1 bis 4 Alkylgruppen substituierte (Alkylenimino)alkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino- oder N-Alkylcarbonyl-iminogruppe ersetzt sein kann,
eine gegebenenfalls durch 1 bis 4 Alkylgruppen substituierte (Alkylenimino)carbonylalkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,
eine (Carboxyalkyl)oxy-, (Alkoxycarbonylalkyl)oxy-, (Aminocarbonylalkyl)oxy-, (Alkylaminocarbonylalkyl)oxy- oder (Dialkylaminocarbonylalkyl)oxy-gruppe, eine gegebenenfalls durch 1 bis 4 Alkylgruppen substituierte [(Alkylenimino)carbonylalkyl]oxy-gruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,
eine C₅₋₇-Cycloalkylgruppe, in der eine Methylengruppe durch ein Sauerstoff- oder Schwefelatom, durch eine Sulfinyl-, Sulfonyl-, Imino- oder N-Alkyl-imino-, Alkylcarbonylimino-, Alkylsulfonyliminogruppe ersetzt ist,
eine gegebenenfalls jeweils im Aryteil durch ein oder zwei Fluor-, Chlor-, Brom- oder Iodatome oder eine oder zwei Nitro-, Alkyl-, Alkoxy- oder Cyanogruppen, wobei die Substituenten gleich oder verschieden sein können, substituierte 3,4-Dihydro-1*H-*quinazolin-2-on-3-yl- oder 1*H*-Benzimidazol-2-on-1-yl-gruppe, darstellt,
stehen, oder
**R_{c}**N**R_{d}** eine gegebenenfalls durch 1 bis 4 Alkylgruppen oder durch eine Arylgruppe substituierte 6- bis 8-gliedrige Alkyleniminogruppe, die zusätzlich durch den Rest **R₆** substituiert sein kann, wobei in den vorstehend erwähnten Alkyleniminogruppen jeweils eine Methylengruppe in 4-Stellung durch ein Sauerstoff- oder Schwefelatom, durch eine Carbonyl-, Sulfinyl-, Sulfonyl-, N-Oxido-N-alkylimino- oder **R₁₀**N-Gruppe ersetzt ist, wobei
- **R₁₀**: fürein Wasserstoffatom, eine Alkyl-, Hydroxy-C₂₋₄-alkyl-, Alkoxy-C₂₋₄-alkyl-, Amino-C₂₋₄-alkyl-, Alkylamino-C₂₋₄-alkyl-, Dialkylamino-C₂₋₄-alkyl-, (Hydroxy-C₂₋₄-alkoxy)-C₂₋₄-alkyl-, Aminocarbonylalkyl-, Alkylaminocarbonylalkyl-, Dialkylaminocarbonylalkyl-, Aryl-, Formyl-, Alkylcarbonyl-, Alkylsulfonyl-, Arylcarbonyl-, Aryl-sulfonyl-, Aralkylcarbonyl-, Aralkylsulfonyl-, Alkoxycarbonyl-, Aralkoxycarbonyl-, Cyano-, Aminocarbonyl-, Alkylaminocarbonyl- oder Dialkylaminocarbonylgruppe, eine Amino-alkylcarbonyl-, Alkylamino-alkylcarbonyl-, Dialkylamino-alkylcarbonyl-gruppe,
eine durch eine, zwei oder drei Arylgruppen substituierte Alkylgruppe,
   eine 8-Alkyl-8-aza-bicyclo[3.2.1]oct-3-ylgruppe,
   eine Aryl- oder eine 2-, 3- oder 4-Pyridylgruppe oder 2-, 4- oder 5 Pyrimidinylgruppe,
eine (Alkylenimino)carbonyl- oder (Alkylenimino)carbonylalkyl-gruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino- oder N-Aralkyl-iminogruppe ersetzt sein kann,
steht, oder
- **R_{c}**N**R_{d}**: für eine in 4-Position durch eine Carboxy-, Alkoxycarbonyl-, Aminocarbonyl-, Alkylaminocarbonyl-, Dialkylaminocarbonylgruppe substituierte 3-Thiazolidinyl-Gruppe steht, oder
- **R_{c}**N**R_{d}**: eine gegebenenfalls durch 1 bis 4 Alkylgruppen substituierte 1-Pyrrolidinylgruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 2 bis 4 Kohlenstoffatome enthält, wenn sich die zwei Wasserstoffatome an Kohlenstoffatomen befinden, die durch ein Atom getrennt sind, wobei die vorstehend erwähnten 1-Pyrrolidinylgruppen zusätzlich durch den Rest **R₆,** der wie vorstehend erwähnt definiert ist, substituiert sind, steht
eine gegebenenfalls durch 1 bis 4 Alkylgruppen substituierte 1-Piperidinyl- oder 1-Azacyclohept-1-yl-gruppe, in denen zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 1 bis 4 Kohlenstoffatome enthält, wenn sich die zwei Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind, wobei die vorstehend erwähnten 1-Piperidinyl- und 1-Azacyclohept-1-yl-gruppen zusätzlich durch den Rest **R₆,** der wie vorstehend erwähnt definiert ist, substituiert sind,
eine gegebenenfalls durch 1 bis 4 Alkylgruppen substituierte 1-Pyrrolidinylgruppe, in der zwei Wasserstoffatome in 3-Stellung durch eine -O-CH₂CH₂-O- oder -O-CH₂CH₂CH₂-O-Gruppe substituiert sind,
eine gegebenenfalls durch 1 bis 4 Alkylgruppen substituierte 1-Piperidinyl- oder 1-Azacyclohept-1-yl-gruppe, in denen in 3-Stellung oder in 4-Stellung jeweils zwei Wasserstoffatome durch eine -O-CH₂CH₂-O- oder
   -O-CH₂CH₂CH₂-O-Gruppe substituiert sind,
eine gegebenenfalls durch eine Alkylgruppe substituierte 1-Azetidinylgruppe, in der die beiden Wasserstoffatome einer Methylengruppe durch eine geradkettige C₄₋₆-Alkylenbrücke ersetzt sind, wobei jeweils eine Methylengruppe in der C₄₋₆-Alkylenbrücke durch eine **R₁₀**N-Gruppe ersetzt ist, wobei **R₁₀** wie vorstehend definiert ist, wobei der so gebildete bicyclische Ring zusätzlich durch eine Hydroxy-, Alkoxy-, Amino-, Alkylamino-, Dialkylamino-, Cyano-, Alkylcarbonylamino-, Alkylsulfonylamino-, Alkoxycarbonylamino-, Arylcarbonyl-, Carboxy-, Alkoxycarbonyl-, Aminocarbonyl-, Alkylaminocarbonyl-oder Dialkylaminocarbonylgruppe substituiert sein kann, eine gegebenenfalls durch 1 bis 2 Alkylgruppen substituierte 1-Pyrrolidinyl-, 1-Piperidinyl- oder 1-Azacyclohept-1-ylgruppe, in der die beiden Wasserstoffatome einer Methylengruppe durch eine geradkettige C₃₋₆-Alkylenbrücke ersetzt sind, wobei jeweils eine Methylengruppe in der C₃₋₆-Alkylenbrücke durch eine **R₁₀**N-Gruppe ersetzt ist, wobei **R₁₀** wie vorstehend definiert ist, wobei der so gebildete bicyclische Ring zusätzlich durch eine Hydroxy-, Alkoxy-, Amino-, Alkylamino-, Dialkylamino-, Cyano-, Alkylcarbonylamino-, Alkylsulfonylamino-, Alkoxycarbonylamino-, Arylcarbonyl-, Carboxy-, Alkoxycarbonyl-, Aminocarbonyl-, Alkylaminocarbonyl-oder Dialkylaminocarbonylgruppe substituiert sein kann,
eine gegebenenfalls in den Alkylenteilen durch 1 oder 2 Alkylgruppen substituierte Gruppe der Struktur 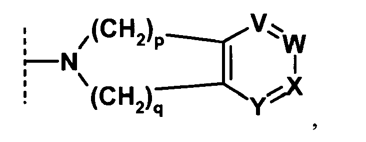
worin
**p** und **q**, die gleich oder verschieden sein können, die Zahl 1 oder 2 bedeuten, und
die Einheit-V=W-X=Y- eine der Gruppen (a), (b), (c), (d) oder (e) bedeutet:

-N=C-C=C- (a),

-C=N-C=C- (b),

-C=N-N=C- (c),

-N=C-C=N- (d),

-N=C-N=C- (e),

oder -V=W- zusammengenommen ein Sauerstoff- oder Schwefelatom und -X=Y- eine der Gruppen -N=C-, -C=N- oder -C=C- bedeuten,
   oder -V=W- zusammengenommen eine Imino-, N-Alkyl-imino, N-Aralkyl-imino oder N-Aryl-imino-Gruppe und -X=Y- eine der Gruppen -N=N-, -N=C-, -C=N- oder -C=C-bedeuten,
oder, sofern **p** und **q** nicht gleich sind,
   -X=Y- zusammengenommen ein Sauerstoff- oder Schwefelatom und -V=W- eine der Gruppen -N=C-, -C=N- oder -C=C- bedeuten,
oder -X=Y- zusammengenommen eine Imino-, N-Alkyl-imino-, N-Aralkyl-imino- oder N-Aryl-imino-Gruppe und -V=W- eine der Gruppen -N=N-, -N=C-, -C=N- oder -C=C-bedeuten,
wobei eines oder zwei der verfügbaren Kohlenstoffatome der Einheit -V=W-X=Y- jeweils durch eine Hydroxy-, Alkoxy-, Amino-, Alkylamino-, Dialkylamino-, Carboxy-, Alkoxycarbonyl, Aminocarbonyl-, Alkylaminocarbonyl-, Dialkylaminocarbonyl- oder Hydrazinocarbonylgruppe, wobei die Substituenten gleich oder verschieden sein können, substituiert sein kann und die verbleibenden verfügbaren Kohlenstoffatome der Einheit - V=W-X=Y- durch ein Wasserstoffatom, eine Alkyl-, Aralkyl- oder Arylgruppe substituiert sind,
oder
- **R_{c}**: ein Wasserstoffatom oder eine C₁₋₈-Alkylgruppe,
eine C₃₋₇-Cycloalkyl-, C₃₋₇-Cycloalkyl-alkyl- oder Aralkylgruppe, die jeweils durch eine oder zwei Alkylgruppen oder durch eine Arylgruppe substituiert sein können,
eine Alkylgruppe, die
   durch eine Hydroxy-, Alkoxy-, Aryloxy-, Aralkoxy-, Alkylsulfenyl-, Alkylsulfinyl-,
   Alkylsulfonyl-, Arylsulfenyl-, Arylsulfinyl-, Arylsulfonyl-, Amino-, Alkylamino-,
   Dialkylamino-, Alkylcarbonylamino-, N-Alkyl-alkylcarbonylamino-, Alkylsulfonylamino-,
   N-Alkyl-alkylsulfonylamino-, Trifluormethylsulfonylamino-,
   N-Alkyl-trifluormethylsulfonylamino-, Carboxy-, Alkoxycarbonyl-, Aralkoxycarbonyl-,
   Aminocarbonyl-, Alkylaminocarbonyl-, Dialkylaminocarbonyl-, Cyanogruppe,
durch eine 2-, 3- oder 4-Pyridylgruppe,
durch eine gegebenenfalls durch 1 bis 4 Alkylgruppen substituierte Alkylenimino- oder (Alkylenimino)carbonylgruppe mit jeweils 4 bis 7 Ringatomen im Alkyleniminoteil, wobei in den vorstehend erwähnten 6- bis 7-gliedrigen Alkyleniminogruppen eine Methylengruppe in 4-Stellung durch ein Sauerstoff- oder Schwefelatom, durch eine Imino-, N-Alkyl-imino-, N-Aryl-imino-, N-Aralkyl-imino-, N-Arylcarbonyl-imino- oder N-Alkylcarbonyl-iminogruppe ersetzt sein kann,
   substituiert ist,
eine gegebenenfalls durch eine Arylgruppe substituierte C₃₋₅-Alkenylgruppe, wobei der Vinylteil nicht mit dem Stickstoffatom der **R_{c}**N**R_{d}**-Gruppe verknüpft sein kann,
eine gegebenenfalls durch eine Arylgruppe substituierte C₃₋₅-Alkinylgruppe, wobei der Ethinylteil nicht mit dem Stickstoffatom der **R_{c}**N**R_{d}**-Gruppe verknüpft sein kann, und
- **R_{d}**: eine C₁₋₁₆-Alkylgruppe, die durch eine Gruppe ausgewählt aus den Gruppen (a) bis (n) substituiert ist:
(a) eine Carboxy-, Alkoxycarbonyl-, Aminocarbonyl-, Alkylaminocarbonyl-, Dialkylaminocarbonyl-, Hydroxy-C₂₋₄-alkylaminocarbonyl-, Cyano-, Hydroxy-, Alkoxy-, Aryloxy-, Aralkoxy-, C₂₋₄-Alkylendioxy-, Alkylcarbonyloxy-, Arylcarbonyloxy-, Formylamino-, Alkylcarbonylamino-, Arylcarbonylamino-, Amino-, Alkylamino-, Dialkylamino-, Naphthylamino-, Aralkylamino-, Diaralkylamino- oder N-Alkyl-aralkylaminogruppe,
(b) eine gegebenenfalls im Arylteil durch ein oder zwei Fluor-, Chlor-, Brom- oder Iodatome oder eine oder zwei Nitro-, Trifluormethyl-, Alkyl-, Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl- oder Cyanogruppen substituierte Phenylamino-, N-Alkyl-N-phenylamino-, Pyridylamino oder N-Alkyl-N-pyridylaminogruppe, wobei die Substituenten gleich oder verschieden sein können,
(c) eine durch einen, zwei oder drei Arylreste substituierte Alkoxygruppe,
(d) eine Hydroxy-C₂₋₄-alkylaminocarbonyl-, Alkoxy-C₂₋₄-alkylaminocarbonyl-, Amino-C₂₋₄-alkylaminocarbonyl-, Alkylamino-C₂₋₄-alkylaminocarbonyl-, Dialkylamino-C₂₋₄-alkylaminocarbonyl-, Carboxyalkylaminocarbonyl-, Alkoxycarbonylalkylaminocarbonyl-, Aminocarbonylalkylaminocarbonyl-, Alkylaminocarbonylalkylaminocarbonyl-, Dialkylaminocarbonylalkylaminocarbonyl-, Arylaminocarbonyl-, N-Alkyl-arylaminocarbonyl-, Aralkylaminocarbonyl-, N-Alkyl-aralkylaminocarbonyl-,
(e) eine Gruppe der Formel -C(=NH)NH₂ oder -NH-C(=NH)NH₂, die gegebenenfalls durch eine Cyano- oder Alkoxycarbonylgruppe substituiert ist,
(f) eine gegebenenfalls durch 1 bis 4 Alkylgruppen substituierte (Alkylenimino)carbonylgruppe mit jeweils 4 bis 7 Ringatomen im Alkyleniminoteil, wobei in einem 6- oder 7-gliedrigen Alkyleniminoteil jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,
(g) eine gegebenenfalls durch 1 bis 4 Alkylgruppen substituierte 4- bis 7-gliedrige Alkyleniminogruppe, wobei in den vorstehend erwähnten 6- oder 7-gliedrigen Alkyleniminogruppen jeweils eine Methylengruppe in 4-Stellung durch ein Sauerstoff- oder Schwefelatom, durch eine Sulfinyl-, Sulfonyl- oder **R₁₀**N-Gruppe ersetzt sein kann, wobei **R₁₀** wie vorstehend definiert ist, und zusätzlich in den vorstehend erwähnten 5- bis 7-gliedrigen Alkyleniminogruppen jeweils eine oder zwei zu den Stickstoffatomen benachbarte Methylengruppen durch eine Carbonylgruppe ersetzt sein können,
(h) eine gegebenenfalls durch 1 bis 2 Alkylgruppen substituierte 5- bis 7-gliedrige Alkyleniminogruppe, die durch eine Hydroxyalkyl-, Aminoalkyl-, Alkylaminoalkyl- oder Dialkylaminoalkylgruppe substituiert ist,
(i) eine Alkylsulfonylamino-, N-Alkyl-alkylsulfonylamino-, Arylcarbonylamino-, N-Alkyl-arylcarbonylamino-, Alkylcarbonylamino-, N-Alkyl-alkylcarbonylamino-, Arylsulfonylamino-, N-Alkyl-arylsulfonylamino-, Aralkylcarbonylamino-, N-Alkyl-aralkylcarbonylamino-, Alkoxyalkyl-carbonylamino-, Alkoxyalkyl-N-alkyl-carbonylamino-, Dialkylamino-alkylcarbonylamino-, Alkylamino-alkylcarbonylamino-, Amino-alkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe,
(j) eine (**R₉**N**R₈**)-CO-N**R₇**- oder (**R₉**N**R₈**)-SO₂-N**R₇**-Gruppe, wobei **R₇, R₈** und **R₉** wie vorstehend definiert sind,
(k) eine Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl- oder Aralkylsulfonylgruppe,
(l) eine durch **R**₆ und gegebenenfalls zusätzlich durch 1 bis 4 Alkylgruppen substituierte C₄₋₇-Cycloalkylgruppe, wobei **R₆** wie vorstehend definiert ist,
(m) eine gegebenenfalls durch 1 bis 4 Alkylgruppen substituierte C₅₋₇-Cycloalkylgruppe, in der eine Methylengruppe durch ein Sauerstoff- oder Schwefelatom, durch eine Sulfinyl-, Sulfonyl- oder N**R₁₀**-Gruppe ersetzt ist, wobei **R₁₀** wie vorstehend definiert ist,
(n) eine gegebenenfalls durch 1 bis 4 Alkylgruppen substituierte 4-Piperidinyl-alkylgruppe, die in 1-Stellung durch **R₁₀** und zusätzlich in der 4-Stellung durch eine Hydroxygruppe substituiert ist, wobei **R₁₀** wie vorstehend definiert ist, und bei der zusätzlich jeweils ein Wasserstoffatom in Position 2 und 6 des Piperidinyl-Gerüsts zusammen durch eine C₂₋₃-Alkylenbrücke ersetzt sind,
oder
eine durch eine 3-Hydroxy-1,3-dihydro-indol-2-on-3-yl- oder 2-Aminocarbonyl-1,3-dihydro-isoindol-5-yl-gruppe substituierte Methylgruppe,
eine im Arylteil durch eine Carboxy-, Alkoxycarbonyl-, Aminocarbonyl-, Alkylaminocarbonyl-, Dialkylaminocarbonyl-, Carboxyalkyl-, Alkoxycarbonylalkyl-, Aminocarbonylalkyl-, Alkylaminocarbonylalkyl- oder Dialkylaminocarbonylalkylgruppe und gegebenenfalls zusätzlich im Alkylenteil durch 1 oder 2 Alkylgruppen substituierte Gruppe der Struktur 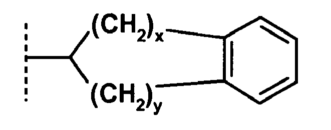 wobei x und y, die gleich oder verschieden sein können, unabhängig voneinander die Zahl 0, 1 oder 2 bedeuten, wobei jedoch x und y zusammen mindestens die Zahl 2 ergeben müssen,
eine durch eine Hydroxygruppe und zusätzlich durch eine Amino-, Alkylamino-, Dialkylamino-, Hydroxy-, Alkoxy-, 1-Pyrrolidinyl-, 1-Piperidinyl- oder Morpholinogruppe substituierte C₃₋₈-Alkylgruppe,
eine durch eine Carboxygruppe und zusätzlich durch eine Amino-, Hydroxy-, Aminocarbonyl-oder Benzyloxycarbonylaminogruppe substituierte C₂₋₈-Alkylgruppe,
eine C₂₋₄-Alkylgruppe, die durch eine C₂₋₄-Alkylsulfenyl- oder C₂₋₄-Alkoxygruppe substituiert ist, welche in ω-Position durch eine Amino-, Hydroxy- oder Alkoxygruppe substituiert ist,
eine C₂₋₄-Alkylgruppe, die durch eine C₂₋₄-Alkoxy-C₂₋₄-alkoxygruppe substituiert ist, welche in ω-Position durch eine Amino- oder Hydroxygruppe substituiert ist,
eine Cyclopropylgruppe, die durch eine Carboxy-, Alkoxycarbonyl-, Aminocarbonyl-, Alkylaminocarbonyl- oder Dialkylaminocarbonylgruppe oder durch eine gegebenenfalls durch 1 bis 4 Alkylgruppen substituierte (Alkylenimino)carbonylgruppe mit jeweils 4 bis 7 Ringatomen im Alkyleniminoteil substituiert ist, wobei in den vorstehend erwähnten 6- oder 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,
eine gegebenenfalls durch 1 bis 4 Alkylgruppen substituierte C₄₋₇-Cycloalkylgruppe, die zusätzlich durch **R₆,** der wie vorstehend definiert ist, substituiert ist,
eine gegebenenfalls durch 1 bis 2 Alkylgruppen substituierte C₅₋₇-Cycloalkylgruppe, die zusätzlich durch eine N,N-Dialkyl-N-oxido-aminogruppe substituiert ist,
eine gegebenenfalls durch 1 bis 4 Alkylgruppen substituierte C₄₋₇-Cycloalkylgruppe, die zusätzlich durch **R₆** substituiert sein kann, wobei in dem Cycloalkylteil eine Methylengruppe durch ein Sauerstoff- oder Schwefelatom, durch eine Sulfinyl-, Sulfonyl-, N-Alkyl-N-oxido-imino- oder **R₁₀**N-Gruppe ersetzt ist, wobei **R₆** und **R₁₀** wie vorstehend definiert sind,
eine gegebenenfalls durch 1 bis 4 Alkylgruppen substituierte C₅-C₇-Cycloalkyl- oder C₅-C₇-Cycloalkylalkylgruppe, in denen jeweils eine Methylengruppe im Cycloalkylteil durch eine Carbonylgruppe ersetzt ist,
eine gegebenenfalls durch 1 bis 4 Alkylgruppen substituierte Cyclopentyl- oder Cyclopentylalkylgruppe, in denen jeweils zwei Wasserstoffatome im Cyclopentylteil durch eine geradkettige Alkylenbrücke ersetzt sind, wobei diese Brücke 2 bis 6 Kohlenstoffatome enthält, wenn sich die zwei Wasserstoffatome am selben Kohlenstoffatom befinden, oder 1 bis 5 Kohlenstoffatome enthält, wenn sich die zwei Wasserstoffatome an benachbarten Kohlenstoffatomen befinden, oder 2 bis 4 Kohlenstoffatome enthält, wenn sich die zwei Wasserstoffatome an Kohlenstoffatomen befinden, die durch ein Kohlenstoffatom getrennt sind, wobei die vorstehend erwähnten Ringe zusätzlich durch den Rest **R₆,** der wie vorstehend definiert ist, substituiert sind,
eine gegebenenfalls durch 1 bis 4 Alkylgruppen substituierte Cyclohexyl-, Cyclohexylalkyl-, Cycloheptyl- oder Cycloheptylalkylgruppe, in denen jeweils zwei Wasserstoffatome im Cycloalkylteil durch eine geradkettige Alkylenbrücke ersetzt sind, wobei diese Brücke 2 bis 6 Kohlenstoffatome enthält, wenn sich die zwei Wasserstoffatome am selben Kohlenstoffatom befinden, oder 1 bis 5 Kohlenstoffatome enthält, wenn sich die zwei Wasserstoffatome an benachbarten Kohlenstoffatomen befinden, oder 1 bis 4 Kohlenstoffatome enthält, wenn sich die zwei Wasserstoffatome an Kohlenstoffatomen befinden, die durch ein Kohlenstoffatom getrennt sind, oder 1 bis 3 Kohlenstoffatome enthält, wenn sich die zwei Wasserstoffatome an Kohlenstoffatomen befinden, die durch zwei Kohlenstoffatome getrennt sind, wobei die vorstehend erwähnten Ringe zusätzlich durch den Rest **R₆,** der wie vorstehend definiert ist, substituiert sind,
eine durch eine 3-Hydroxy-1,3-dihydro-indol-2-on-3-yl- oder 2-Aminocarbonyl-1,3-dihydro-isoindol-5-yl-gruppe substituierte Alkylgruppe,
eine durch eine Arylgruppe substituierte C₁₋₁₀-Alkylgruppe, wobei der vorstehend erwähnte Arylteil durch eine Alkoxycarbonyl-, Carboxy-, Carboxyalkyl-, Aminosulfonyl-, Trifluormethoxy-, Cyano-, Aminoalkyl-, Amino-, Alkylamino-, Dialkylamino-, Nitro-, 2*H-*Pyridazin-3-on-6-yl-, Hydroxyphenyl-, Hydroxyalkyl-, Hydroxy- oder Alkoxygruppe substituiert ist,
eine Aralkylgruppe, die im Arylteil durch eine Hydroxy- oder Alkoxygruppe und zusätzlich durch eine Carboxy-, Alkoxycarbonyl-, Hydroxy- oder Alkoxygruppe substituiert ist,
eine durch eine Pyrrolyl-, Imidazolyl-, Pyrazolyl-, Pyridyl-, Pyrimidinyl-, Pyrazinyl-, Indolyl- oder Benzimidazolylgruppe substituierte C₁₋₁₀-Alkylgruppe, wobei die vorstehend erwähnten Heteroarylteile an den verfügbaren Kohlenstoffatomen zusätzlich jeweils durch eine oder zwei Gruppen ausgewählt aus Fluor-, Chlor-, Brom- oder Iodatomen, Alkyl-, Alkoxycarbonyl-, Carboxy-, Trifluormethyl-, Trifluormethoxy-, Cyano-, Amino-, Alkylamin-, Dialkylamino-, Nitro-, Hydroxy- oder Alkoxygruppen substituiert sein können, wobei die Substituenten gleich oder verschieden sein können,
eine durch eine Carboxy-, Alkoxycarbonyl-, Aminocarbonyl-, Alkylaminocarbonyl-, Dialkylaminocarbonyl-, Aralkylaminocarbonyl-, Cyano-, Hydroxy-, Alkoxy-, Aryloxy-, Amino-, Alkylamino-, Dialkylamino-, Alkylcarbonylamino- oder Alkoxycarbonylaminogruppe substituierte C₁₋₁₀-Alkylgruppe, die zusätzlich durch eine oder zwei Arylgruppen oder eine Pyrrolyl-, Imidazolyl-, Pyrazolyl-, Pyridyl-, Pyrimidinyl-, Pyrazinyl-, Indolyl- oder Benzimidazolyl-gruppe substituiert ist, wobei die vorstehend erwähnten Aryl- oder Heteroarylteile an den verfügbaren Kohlenstoffatomen zusätzlich jeweils durch eine oder zwei Gruppen ausgewählt aus Fluor-, Chlor-, Brom- oder Iodatomen, Alkyl-, Alkoxycarbonyl-, Carboxy-, Trifluormethyl-, Trifluormethoxy-, Cyano-, Amino-, Alkylamino-, Dialkylamino-, Nitro-, Hydroxy- oder Alkoxygruppen substituiert sein können, wobei die Substituenten gleich oder verschieden sein können,
eine durch eine Arylgruppe substituierte C₁₋₆-Alkylgruppe, die im Arylteil durch eine Hydroxy-oder Aminogruppe und zusätzlich durch zwei Fluor-, Chlor-, Brom- oder Iodatome, wobei die Substituenten gleich oder verschieden sein können, substituiert ist,
eine durch eine Carboxy- oder Alkoxycarbonylgruppe substituierte C₂₋₆-Alkylgruppe, die zusätzlich durch eine Amino-, Alkylamino-, Dialkylamino-, Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe substituiert ist,
eine 3-Chinuclidinyl-, 4-Chinuclidinyl-, 2-Chinuclidinyl-alkyl-, 3-Chinuclidinyl-alkyl- oder 4-Chinuclidinyl-alkylgruppe, oder
**R_{c}** ein Wasserstoffatom oder eine Alkylgruppe und **R_{d}** eine Hydroxy- oder Alkoxygruppe darstellen, und
**Rₑ** eine Nitro- oder Trifluormethylgruppe,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
wobei, sofern nichts anderes erwähnt wurde,
unter den bei der Definition der vorstehend erwähnten Reste erwähnten Arylteilen eine Phenylgruppe zu verstehen ist, in der ein oder zwei Kohlenstoff-Atome jeweils durch ein Stickstoffatom ersetzt sein können, wobei die vorstehend genannten Arylteile jeweils durch **R₁₁** monosubstituiert, durch **R₁₂** mono-, di- oder trisubstituiert oder durch **R₁₁** monosubstituiert und zusätzlich durch **R₁₂** mono- oder disubstituiert sein können, wobei die Substituenten gleich oder verschieden sein können, und
**R₁₁** eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Perfluoralkyl-, Perfluoralkoxy-, Nitro-, Amino-, Alkylcarbonylamino-, N-Alkyl-alkylcarbonylamino-, Alkylamino-, Dialkylamino-, Hydroxy-C₂₋₄-alkylamino-, N-Alkyl-(hydroxy-C₂₋₄-alkyl)amino-, Bis-(hydroxy-C₂₋₄-alkyl)amino-, Phenylalkylcarbonylamino-, Phenylcarbonylamino-, Alkylsulfonylamino-, Phenylalkylsulfonylamino-, Phenylsulfonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkyl-phenylsulfonylamino-, Aminosulfonyl-, Alkylaminosulfonyl-, Dialkylaminosulfonyl-, (**R₉NR₈**)-CO-N**R₇**- oder (**R₉**N**R₈**)-SO₂-N**R₇**-Gruppe, wobei **R₇, R₈** und **R₉** wie vorstehend definiert sind,
eine gegebenenfalls durch 1 bis 4 Alkylgruppen oder eine Hydroxyalkylgruppe substituierte 5- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoffatom oder eine **R₁₀**N-Gruppe ersetzt sein kann, wobei **R₁₀** wie vorstehend definiert ist,
eine gegebenenfalls durch 1 bis 4 Alkylgruppen oder eine Hydroxyalkylgruppe substituierte 5- bis 7-gliedrige Alkyleniminogruppe, wobei jeweils eine oder zwei zu dem Stickstoffatom benachbarte Methylengruppen jeweils durch eine Carbonylgruppe ersetzt ist, und
**R₁₂** eine Alkyl-, Hydroxy- oder Alkoxygruppe, ein Fluor-, Chlor-, Brom- oder Iodatom darstellen, wobei zwei Reste **R₁₂,** sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,
sowie, soweit nichts anderes erwähnt wurde, die vorstehend erwähnten Alkyl-, Alkylen- und Alkoxyteile jeweils 1 bis 4 Kohlenstoffatome enthalten,
wobei, sofern nichts anderes erwähnt wurde, jedes Kohlenstoffatom in den vorstehend erwähnten Alkyl-, Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

Bevorzugt sind die Verbindungen der Formel I, worin
**R_{b}** eine gegebenenfalls durch die Reste **R₁** bis **R₃** substituierte Phenylgruppe bedeutet,
wobei
- **R₁**: ein Fluor-, Chlor-, Brom- oder Iodatom,
eine C₁₋₂-Alkyl- oder Hydroxygruppe,
eine C₃₋₆-Cycloalkyl- oder C₅₋₆-Cycloalkoxygruppe,
eine C₂₋₅-Alkenylgruppe,
eine C₂₋₅-Alkinylgruppe,
eine Aryl-, Aryloxy-, Aralkyl-, Aralkoxy-, Alkylsulfenyl-, Alkylsulfinyl-, Alkylsulfonyl-,
   Alkylsulfonyloxy-, Trifluormethylsulfenyl-, Trifluormethylsulfonyl-, Arylsulfenyl-,
   Arylsulfinyl-, Arylsulfonyl-, Aralkylsulfenyl-, Aralkylsulfinyl- oder
   Aralkylsulfonylgruppe,
eine durch 1 bis 3 Fluoratome substituierte Methyl- oder Methoxygruppe,
eine durch 1 bis 5 Fluoratome substituierte C₂₋₄-Alkyl- oder C₂₋₄-Alkoxygruppe,
eine Nitro-, Amino-, Alkylamino-, Dialkylamino-, C₃₋₆-Cycloalkylamino-, N-Alkyl-C₃₋₆-cycloalkylamino-, Arylamino-, N-Alkyl-arylamino-, Aralkylamino- oder N-Alkyl-aralkylaminogruppe,
eine 5- bis 7-gliedrige Alkyleniminogruppe, wobei jeweils eine oder zwei zu dem Stickstoffatom benachbarte Methylengruppen jeweils durch eine Carbonylgruppe oder in den vorstehend erwähnten 6- bis 7-gliedrigen Alkyleniminogruppen eine Methylengruppe in 4-Stellung durch ein Sauerstoffatom, durch eine Imino-, N-Aryl-imino- oder N-Alkyl-iminogruppe ersetzt sein können und die Alkyleniminogruppen zusätzlich durch 1-2 Methylgruppen substituiert sein kann,
eine (Alkylenimino)carbonyl- oder (Alkylenimino)sulfonylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoffatom, durch eine Imino-, N-Aryl-imino- oder N-Alkyl-iminogruppe ersetzt sein kann,
eine Alkylcarbonylamino-, N-Alkyl-alkylcarbonylamino-, Alkyl-sulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Trifluormethylsulfonylamino-, N-Alkyl-trifluormethylsulfonylamino-, Alkylcarbonyl-, Arylcarbonyl-, Aralkylcarbonyl-, Carboxy-, Alkoxycarbonyl-, Aminocarbonyl-, Alkylaminocarbonyl-, Dialkylaminocarbonyl-, Arylaminocarbonyl-, N-Alkyl-arylaminocarbonyl-, Aralkylaminocarbonyl-, N-Alkyl-aralkylaminocarbonyl-, N-Hydroxy-aminocarbonyl-, N-Hydroxy-alkylaminocarbonyl-, N-Alkoxy-aminocarbonyl-, N-Alkoxy-alkylaminocarbonyl-, Cyano-, Azido-, N-Cyano-amino- oder N-Cyano-alkylaminogruppe,
eine Sulfo-, Aminosulfonyl-, Alkylaminosulfonyl-, Dialkylaminosulfonyl-, Arylaminosulfonyl-, Pyridylaminosulfonyl-, N-Alkyl-arylaminosulfonyl-, Aralkylaminosulfonyl- oder N-Alkyl-aralkylaminosulfonylgruppe, oder
eine durch **R₄** substituierte C₁₋₂Alkylgruppe
bedeutet, wobei
- **R₄**: eine Hydroxy-, Alkoxy-, Aryloxy-, Amino-, Alkylamin-, Fluoralkylamino-, Dialkylamino-, Alkylsulfenyl-, Alkylsulfinyl-, Alkylsulfonyl-, Arylsulfenyl-, Arylsulfinyl-, Arylsulfonyl-, Carboxy-, Alkoxycarbonyl-, Aminocarbonyl-, Alkylaminocarbonyl-, Dialkylaminocarbonyl- oder Cyanogruppe,
eine gegebenenfalls durch eine oder zwei Alkylgruppen substituierte 5- bis 7-gliedrige Alkyleniminogruppe, wobei in den vorstehend erwähnten 6- bis 7-gliedrigen Alkyleniminogruppen eine Methylengruppe in 4-Stellung durch ein Sauerstoff- oder Schwefelatom, durch eine Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein kann, oder durch eine Hydroxy-, Alkoxy-, Aminocarbonyl-, Alkylaminocabonyl-, Dialkylaminocarbonyl-, Amino-, Alkylamino- und Dialkylaminogruppe substituiert sein kann, oder
eine gegebenenfalls durch eine oder zwei Alkylgruppen substituierte (Alkylenimino)carbonylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Imino-, N-Alkyl-imino- oder N-Alkylcarbonyl-iminogruppe ersetzt sein kann, oder
eine Gruppe der Formel 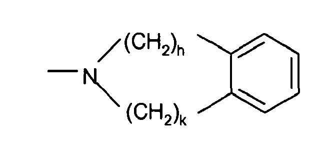 in der
   **h** und **k**, die gleich oder verschieden sein können, die Zahlen 1 bis 2 oder **h** die Zahl 0 und **k** die Zahl 2 oder 3 bedeuten, wobei zusätzlich der obige Benzoteil durch ein Fluor-, Chlor-, Brom- oder Iodatom oder durch eine Alkyl-, Trifluormethyl-, Hydroxy-, Alkoxy-, Carboxy- oder Cyanogruppe und der obige gesättigte cyclische Iminoteil durch 1 oder 2 Alkylgruppen substituiert sein kann,
- **R₂**: ein Fluor-, Chlor- oder Bromatom, eine C₁₋₂ Alkyl-, Trifluormethyl-, Hydroxy-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Trifluormethylsulfonylamino-, N-Alkyl-trifluormethylsulfonylamino- oder Cyanogruppe,
bedeutet, und
- **R₃**: ein Fluor-, Chlor- oder Bromatom, eine C₁₋₂ Alkyl-, Trifluormethyl- oder Alkoxygruppe,
eine Gruppe der Struktur 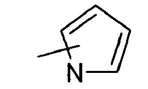 worin der Anknüpfungspunkt ein Kohlenstoff oder ein Stickstoffatom sein kann und bis zu drei Kohlenstoffatome durch ein Stickstoffatom ersetzt sein können und der Ring über jedes der Atome durch eine oder zwei Alkyl-, Aryl- oder Aralkylreste substituiert sein kann oder
eine Sulfo-, Aminosulfonyl-, Alkylaminosulfonyl-, Dialkylaminosulfonyl-, Arylaminosulfonyl-, Pyridylaminosulfonyl-, N-Alkyl-arylaminosulfonyl-, Aralkylaminosulfonyl- oder N-Alkyl-aralkylaminosulfonylgruppe bedeutet
- **R₂**: zusammen mit **R₃,** sofern diese an benachbarte Kohlenstoffatome gebunden sind, eine gegebenenfalls durch eine oder zwei Alkylgruppen substituierte Methylendioxygruppe, oder eine gegebenenfalls durch eine oder zwei Alkylgruppen substituierte n-C₃₋₅-Alkylengruppe, in der eine Methylengruppe durch ein Sauerstoffatom, durch eine Imino-, N-Alkyl-imino- oder N-Aralkyl-iminogruppe ersetzt sein kann, oder
eine gegebenenfalls durch ein Fluor-, Chlor- oder Bromatom, durch eine Hydroxy-, Alkyl-, Alkoxy-, Trifluormethyl-, Carboxy oder Cyangruppe substituierte 1,3-Butadien-1,4-diylengruppe oder
eine Gruppe der Formel -NH-C(=O)-(CH₂)- oder -NH-C(=O)-(CH₂)₂-, die im Alkylenteil zusätzlich durch 1 oder 2 Alkylgruppen substituiert sein können, oder eine Gruppe der Formel -NH-N=N-, -NH-N=CH-, -NH-CH=N-, -O-CH=N-, -S-CH=N-, -NH-CH=CH-und deren Tautomeren, wobei jedes Wasserstoffatom durch eine Alkyl-, Aryl- oder Aralkylgruppe substituiert sein kann, bedeuten, oder
eine Gruppe der Formel -(CH₂)ₘ-N**R₅**-(CH₂)ₙ-,
   worin **m** und **n** jeweils gleich oder verschieden 1 oder 2 bedeuten, und
   **R₅** für Wassestoff, C₁₋₆ Alkyl oder C₁₋₆ Fluoralkyl steht,

**R_{c}**N**R_{d}** eine gegebenenfalls durch 1 bis 2 Alkyl- oder Arylgruppen substituierte 4- bis 7-gliedrige Alkyleniminogruppe bedeuten, die zusätzlich durch den Rest **R₆** substituiert ist, wobei
- **R₆**: eine Carboxy-, Alkoxycarbonyl-, Aminoalkyl-, Aminocarbonyl-, Alkylaminocarbonyl-,
Dialkylaminocarbonyl-, Cyano-, Hydroxy-, Alkoxy-, Aryloxy-, Aralkoxy-,
Alkylcarbonyloxy-, Arylcarbonyloxy-, Amino-, Alkylamino-, Hydroxy-C₂₋₄-alkylamino-,
Dialkylamino-, Cyanamino-, Formylamino-, Alkylsulfenyl-, Alkylsulfinyl-,
Alkylsulfonyl-, Arylsulfenyl-, Arylsulfinyl-, Arylsulfonyl-, Aralkylsulfenyl-,
Aralkylsulfinyl- oder Aralkylsulfonylgruppe,
eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Alkoxycarbonylamino-, N-Alkyl-alkoxycarbonylamino-, Alkoxycarbonylalkylamino-, N-(Alkyl)-N-(alkoxycarbonylalkyl)-amino-, Aralkoxycarbonylamino- oder N-Alkyl-aralkoxycarbonylaminogruppe,
eine (N**R₈R₉**)CON**R₇**-Gruppe, in der
   **R₇** und **R₈** jeweils ein Wasserstoffatom oder eine Alkylgruppe und **R₉** ein
   Wasserstoffatom oder eine Alkyl-, Aryl-oder Pyridylgruppe, wobei die Reste **R₇,**
   **R₈** und **R₉** gleich oder verschieden sein können, oder
   **R₇** und **R₈** zusammen eine n-C₂₋₄-Alkylengruppe und **R₉** ein Wasserstoffatom oder eine Alkyl-, Aryl-oder Pyridylgruppe
   darstellen,
eine gegebenenfalls durch 1 bis 2 Alkylgruppen substituierte Alkyleniminogruppe mit 5 bis 7 Ringatomen im Alkyleniminoteil, wobei in den vorstehend erwähnten 6- bis 7-gliedrigen Alkyleniminoteilen jeweils eine Methylengruppe in 4-Stellung des Alkyleniminoteils durch ein Sauerstoff- oder Schwefelatom, durch eine Carbonyl-, Sulfinyl-, Sulfonyl-, Imino-, N-Alkylimino-, N-Alkylcarbonyl-imino-, N-Alkylsulfonyl-imino-, N-Arylimino- oder N-Aralkyl-iminogruppe ersetzt sein kann,
eine gegebenenfalls durch 1 bis 2 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,
eine durch eine Hydroxy-, Alkoxy-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Hydroxyalkyl-, Carboxy-, Alkoxycarbonyl-, Aminocarbonyl-, Alkylaminocarbonyl- oder Dialkylaminocarbonyl-gruppe substituierte 4-bis 7-gliedrige Alkyleniminogruppe,
eine durch eine Carboxy-, Alkoxycarbonyl-, Aminocarbonyl-, Alkylaminocarbonyl-, Dialkylaminocarbonyl-, Cyano-, Hydroxy-, Alkoxy-, Aryloxy-, Amino-, Alkylamino-, Dialkylamino-, Alkylcarbonylamino-, N-Alkyl-alkylcarbonylamino-, Alkoxycarbonylamino-, N-Alkyl-alkoxycarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Dialkylaminocarbonylalkylamino-, N-(Alkyl)-N-(dialkylaminocarbonylalkyl)-amino-, Dialkylaminoalkoxy-, Alkylsulfenyl-, Alkylsulfinyl-, Alkylsulfonyl-, Arylsulfenyl-, Arylsulfinyl- oder Arylsulfonylgruppe substituierte Alkylgruppe,
eine gegebenenfalls durch 1 bis 2 Alkylgruppen substituierte (Alkylenimino)alkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino- oder N-Alkylcarbonyl-iminogruppe ersetzt sein kann,
eine gegebenenfalls durch 1 bis 2 Alkylgruppen substituierte (Alkylenimino)carbonylalkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,
eine (Carboxyalkyl)oxy-, (Alkoxycarbonylalkyl)oxy-, (Aminocarbonylalkyl)oxy-, (Alkylaminocarbonylalkyl)oxy- oder (Dialkylaminocarbonylalkyl)oxy-gruppe,
eine gegebenenfalls jeweils im Aryteil durch ein oder zwei Fluor-, Chlor-, Brom- oder Iodatome oder eine oder zwei Nitro-, Alkyl-, Alkoxy- oder Cyanogruppen, wobei die Substituenten gleich oder verschieden sein können, substituierte 3,4-Dihydro-1*H-*quinazolin-2-on-3-yl- oder 1*H*-Benzimidazol-2-on-1-yl-gruppe, darstellt, bedeutet, oder

**R_{c}**N**R_{d}** eine gegebenenfalls durch 1 bis 2 Alkylgruppen oder durch eine Arylgruppe substituierte 6- bis 7-gliedrige Alkyleniminogruppe, die zusätzlich durch den Rest **R₆** substituiert sein kann, wobei in den vorstehend erwähnten Alkyleniminogruppen jeweils eine Methylengruppe in 4-Stellung durch ein Sauerstoff- oder Schwefelatom, durch eine Carbonyl-, Sulfinyl-, Sulfonyl- oder **R₁₀**N-Gruppe ersetzt ist, bedeutet, wobei
   **R₁₀** ein Wasserstoffatom, eine Alkyl-, Hydroxy-C₂₋₄-alkyl-, Amino-C₂₋₄-alkyl-, Alkylamino-C₂₋₄-alkyl-, Dialkylamino-C₂₋₄-alkyl-, (Hydroxy-C₂₋₄-alkoxy)-C₂₋₄-alkyl-, Aminocarbonylalkyl-, Alkylaminocarbonylalkyl-, Dialkylaminocarbonylalkyl-, Aryl-, Formyl-, Alkylcarbonyl-, Alkylsulfonyl-, Arylcarbonyl-, Arylsulfonyl-, Aralkylcarbonyl-, Aralkylsulfonyl-, Alkoxycarbonyl-, Aralkoxycarbonyl-, Cyano-, Aminocarbonyl-, Alkylaminocarbonyl- oder Dialkylaminocarbonylgruppe,
   eine Amino-alkylcarbonyl-, Alkylamino-alkylcarbonyl-, Dialkylamino-alkylcarbonylgruppe,
   eine durch eine oder zwei Arylgruppen substituierte Methylgruppe, wobei die Arylteile unabhängig voneinander jeweils durch ein oder zwei Fluor-, Chlor-, Brom- oder Iodatome oder eine oder zwei Nitro-, Alkyl-, Hydroxy- oder Alkoxygruppen substituiert sein können, wobei die Substituenten gleich oder verschieden sein können,
   eine 2-, 3- oder 4-Pyridylgruppe,
   eine 2-, 4- oder 5-Pyrimidinylgruppe,
   eine gegebenenfalls durch ein oder zwei Fluor-, Chlor-, Brom- oder Iodatome oder eine oder zwei Nitro-, Trifluormethyl-, Alkyl-, Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl- oder Cyanogruppen substituierte Phenylgruppe, wobei die Substituenten gleich oder verschieden sein können,
   eine 8-Methyl-8-aza-bicyclo[3.2.1]oct-3-ylgruppe,
   oder eine (Alkylenimino)carbonyl- oder (Alkylenimino)carbonylalkylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino- oder N-Aralkyl-iminogruppe ersetzt sein kann, darstellt, oder
**R_{c}**N**R_{d}** eine in 4-Position durch eine Carboxy-, Alkoxycarbonyl-, Aminocarbonyl-, Alkylaminocarbonyl- oder Dialkylaminocarbonylgruppe substituierte 3-Thiazolidinyl-Gruppe bedeutet, oder
**R_{c}**N**R_{d}** eine gegebenenfalls durch 1 bis 2 Alkylgruppen substituierte 1-Piperidinyl-gruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 1 bis 4 Kohlenstoffatome enthält, wenn sich die zwei Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind, wobei die vorstehend erwähnten 1-Piperidinylgruppen zusätzlich durch den Rest **R₆,** der wie vorstehend erwähnt definiert ist, substituiert sind,
eine gegebenenfalls durch 1 bis 2 Alkylgruppen substituierte 1-Pyrrolidinyl- oder 1-Piperidinylgruppe, in der die beiden Wasserstoffatome einer Methylengruppe durch eine geradkettige C₃₋₆-Alkylenbrücke ersetzt sind, wobei jeweils eine Methylengruppe in der C₃₋₆-Alkylenbrücke durch eine **R₁₀**N-Gruppe ersetzt ist, wobei **R₁₀** wie vorstehend definiert ist, wobei der so gebildete bicyclische Ring gegebenenfalls zusätzlich durch eine Hydroxy-, Alkoxy-, Amino-, Alkylamino-, Dialkylamino-, Cyano-, Alkylcarbonylamino-, Alkylsulfonylamino-, Alkoxycarbonylamino-, Arylcarbonyl-, Carboxy-, Alkoxycarbonyl-, Aminocarbonyl-, Alkylaminocarbonyl- oder Dialkylaminocarbonylgruppe substituiert ist,
eine gegebenenfalls in den Alkylenteilen durch 1 oder 2 Alkylgruppen substituierte Gruppe der Struktur 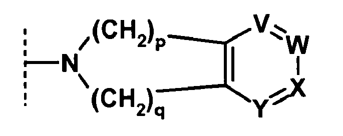 worin
   p und q, die gleich oder verschieden sein können, unabhängig voneinander die Zahl 1 oder 2 bedeuten, und
   die Einheit-V=W-X=Y- eine der Gruppen (a) oder (b) bedeutet:

      -N=C-C=C- (a),

      -C=N-C=C- (b),

      wobei eines der verfügbaren Kohlenstoffatome der Gruppen (a) oder (b) durch eine Hydroxy-, Alkoxy-, Amino-, Alkylamino- oder Dialkylaminogruppe substituiert sein kann und die verbleibenden verfügbaren Kohlenstoffatome der Gruppen (a) oder (b) durch ein Wasserstoffatom, eine Alkyl- oder Arylgruppe substituiert sind,
      oder
   -V=W- zusammengenommen ein Sauerstoff- oder Schwefelatom oder eine Imino-, N-Alkyl-imino- oder N-Aryl-imino-Gruppe und -X=Y- eine der Gruppen -N=C- oder -C=N-bedeuten, oder,
      sofern n und m nicht gleich sind,
      -X=Y- zusammengenommen ein Sauerstoff- oder Schwefelatom oder eine Imino-, N-Alkyl-imino- oder N-Aryl-imino-Gruppe und -V=W- eine der Gruppen -N=C- oder -C=N-bedeuten,
oder
- **R_{c}**: ein Wasserstoffatom, eine Aralkyl- oder eine C₁₋₆-Alkylgruppe,
- eine: Alkylgruppe, die
durch eine Hydroxy-, Alkoxy-, Aryloxy-, Aralkoxy-, Amino-, Alkylamino-,
Dialkylamino-, Alkylcarbonylamino-, N-Alkyl-alkylcarbonylamino-, Alkylsulfonylamino-,
N-Alkyl-alkylsulfonylamino-, Trifluormethylsulfonylamino-,
N-Alkyl-trifluormethylsulfonylamino-, Carboxy-, Alkoxycarbonyl-, Aminocarbonyl-,
Alkylaminocarbonyl-, Dialkylaminocarbonyl-, Cyano- oder durch eine 2-,3- oder 4-Pyridylgruppe mit der Maßgabe, dass die Heteroatome von dem Stickstoffatom der
**R_{c}**N**R_{d}**-Gruppe durch zwei oder mehr Kohlenstoffatome getrennt sind, substituiert ist,

eine C₃₋₅-Alkenylgruppe, wobei der Vinylteil nicht mit dem Stickstoffatom der **R_{c}**N**R_{d}**-Gruppe verknüpft sein kann,
eine C₃₋₅-Alkinylgruppe, wobei der Ethinylteil nicht mit dem Stickstoffatom der **R_{c}**N**R_{d}**-Gruppe verknüpft sein kann, und
**R_{d}** eine C₁₋₁₀-Alkylgruppe, die durch.eine Gruppe ausgewählt aus den Gruppen (a) bis (n) substituiert ist:
   (a) eine Carboxy-, Alkoxycarbonyl-, Aminocarbonyl-, Alkylaminocarbonyl-, Dialkylaminocarbonyl-, Cyano-, Hydroxy-, Alkoxy-, Aryloxy-, Aralkoxy-, Alkylcarbonylamino-, Amino-, Alkylamino-, Dialkylamino-, Naphthylamino-, Aralkylamino-, Diaralkylamino- oder N-Alkyl-aralkylaminogruppe,
   (b) eine gegebenenfalls im Arylteil durch ein Fluor-, Chlor-, Brom- oder Iodatom oder eine Nitro-, Trifluormethyl-, Alkyl-, Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl- oder Cyanogruppe substituierte Phenylamino- oder Pyridylaminogruppe,
   (c) eine durch einen, zwei oder drei Arylreste substituierte Methoxygruppe,
   (d) eine Carboxyalkylaminocarbonyl-, Alkoxycarbonylalkylaminocarbonyl-, Aminocarbonylalkylaminocarbonyl-, Alkylaminocarbonylalkylaminocarbonyl-, Dialkylaminocarbonylalkylaminocarbonyl-, Arylaminocarbonyl-, N-Alkyl-arylaminocarbonyl-, Aralkylaminocarbonyl-, N-Alkyl-aralkylaminocarbonyl-,
   (e) eine Gruppe der Formel -C(=NH)NH₂,
   (f) eine gegebenenfalls durch 1 bis 2 Alkylgruppen substituierte (Alkylenimino)carbonylgruppe mit jeweils 5 bis 7 Ringatomen im Alkyleniminoteil, wobei in den vorstehend erwähnten 6- bis 7-gliedrigen Alkyleniminogruppen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,
   (g) eine gegebenenfalls durch 1 bis 2 Alkylgruppen substituierte 4- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoff- oder Schwefelatom, durch eine Sulfinyl-, Sulfonyl- oder **R₁₀**N-Gruppe ersetzt sein kann, wobei **R₁₀** wie vorstehend definiert ist, und zusätzlich in den vorstehend erwähnten 5- bis 7-gliedrigen Alkyleniminogruppen jeweils eine zu den Stickstoffatomen benachbarte Methylengruppe durch eine Carbonylgruppe ersetzt sein kann,
   (h) eine gegebenenfalls durch 1 bis 2 Alkylgruppen substituierte 5- bis 7-gliedrige Alkyleniminogruppe, die durch eine Hydroxyalkyl-, Aminoalkyl-, Alkylaminoalkyl-oder Dialkylaminoalkylgruppe substituiert ist,
   (i) eine Alkylsulfonylamino-, N-Alkyl-alkylsulfonylamino-, Arylcarbonylamino-, N-Alkyl-arylcarbonylamino-, Alkylcarbonylamino-, N-Alkyl-alkylcarbonylamino-, Alkoxy-alkylcarbonylamino-, Dialkylamino-alkylcarbonylamino-, Arylsulfonylamino-, N-Alkyl-arylsulfonylamino-, Aralkylcarbonylamino-, N-Alkylaralkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe,
   (j) eine (**R₉**N**R₈**)-CO-N**R₇**-Gruppe, wobei **R₇, R₈** und **R₉** wie vorstehend definiert sind,
   (k) eine 2-Aza-bicyclo[2.2.1]hept-5-en-2-yl-gruppe,
   (l) eine Alkylsulfenyl-, Alkylsulfinyl-, Alkylsulfonyl-, Arylsulfenyl-, Arylsulfinyl- oder Arylsulfonylgruppe,
   (m) eine durch **R₆** und gegebenenfalls zusätzlich durch 1 bis 2 Alkylgruppen substituierte C₄₋₇-Cycloalkylgruppe, wobei **R₆** wie vorstehend definiert ist,
   (n) eine gegebenenfalls durch 1 bis 4 Alkylgruppen substituierte C₅₋₇-Cycloalkylgruppe, in der eine Methylengruppe durch ein Sauerstoffatom oder eine N**R₁₀**-Gruppe ersetzt ist, wobei **R₁₀** wie vorstehend definiert ist,
eine 4-Piperidinyl-methylgruppe, die in 1-Stellung durch **R₁₀** und zusätzlich in der 4-Stellung durch eine Hydroxygruppe substituiert ist, wobei **R₁₀** wie vorstehend definiert ist, und bei der zusätzlich jeweils ein Wasserstoffatom in Position 2 und 6 des Piperidinyl-Gerüsts zusammen durch eine C₂₋₃-Alkylenbrücke ersetzt sind,
eine durch eine 3-Hydroxy-1,3-dihydro-indol-2-on-3-yl- oder 2-Aminocarbonyl-1,3-dihydro-isoindol-5-yl-gruppe substituierte Methylgruppe,
eine im Arylteil durch eine Carboxy- oder Carboxyalkylgruppe und gegebenenfalls zusätzlich im Allcylenteil durch 1 oder 2 Alkylgruppen substituierte Gruppe der Struktur 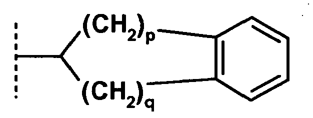 wobei **p** und **q,** die gleich oder verschieden sein können, die Zahl 0, 1 oder 2 bedeuten,
   wobei jedoch **p** und **q** zusammen mindestens die Zahl 2 ergeben müssen,
eine durch eine Hydroxygruppe und zusätzlich durch eine Amino-, Alkylamino-, Dialkylamino-, Hydroxy-, Alkoxy-, 1-Pyrrolidinyl-, 1-Piperidinyl- oder Morpholinogruppe substituierte C₃₋₆-Alkylgruppe,
eine durch eine Carboxygruppe und zusätzlich durch eine Amino-, Hydroxy-, Aminocarbonyl-oder Benzyloxycarbonylaminogruppe substituierte C₂₋₆-Alkylgruppe,
eine C₂₋₄-Alkylgruppe, die durch eine C₂₋₄-Alkylsulfenylgruppe substituiert ist, welche in ω-Position durch eine ω-Aminogruppe substituiert ist,
eine C₂₋₄-Alkylgruppe, die durch eine C₂₋₄-Alkoxygruppe substituiert ist, welche in ω-Position durch eine Amino-, Hydroxy- oder Alkoxygruppe substituiert ist,
eine C₂₋₄-Alkylgruppe, die durch eine C₂₋₄-Alkoxy-C₂₋₄-alkoxygruppe substituiert ist, welche in ω-Position durch eine Amino- oder Hydroxygruppe substituiert ist,
eine gegebenenfalls durch 1 bis 2 Alkylgruppen substituierte C₄₋₇-Cycloalkylgruppe, die zusätzlich durch **R₆,** der wie vorstehend definiert ist, substituiert ist,
eine gegebenenfalls durch 1 bis 2 Alkylgruppen substituierte C₄₋₇-Cycloalkylgruppe, die zusätzlich durch **R₆** substituiert sein kann, wobei in dem Cycloalkylteil eine Methylengruppe durch ein Sauerstoff- oder Schwefelatom, durch eine Sulfinyl-, Sulfonyl- oder **R₁₀**N-Gruppe ersetzt ist, wobei **R₆** und **R₁₀** wie vorstehend definiert sind,
eine durch eine 3-Hydroxy-1,3-dihydro-indol-2-on-3-yl- oder 2-Aminocarbonyl-1,3-dihydro-isoindol-5-yl-gruppe substituierte Methylgruppe,
eine durch eine Arylgruppe substituierte C₁₋₆-Alkylgruppe, wobei der vorstehend erwähnte Arylteil durch eine Alkoxycarbonyl-, Carboxy-, Carboxyalkyl-, Aminosulfonyl-, Trifluormethoxy-, Cyano-, Aminoalkyl-, Amino-, Alkylamino-, Dialkylamino-, Nitro-, 2*H*-Pyridazin-3-on-6-yl-, Hydroxyphenyl-, Hydroxyalkyl-, Hydroxy- oder Alkoxygruppe substituiert ist,
eine Aralkylgruppe, die im Arylteil durch eine Alkoxy- oder Hydroxygruppe und zusätzlich durch eine Alkoxycarbonyl-, Carboxy-, Alkoxy- oder Hydroxygruppe substituiert ist,
eine durch eine 2-Pyridyl-, 3-Pyridyl-, 4-Pyridyl-, 2-Pyrazinyl-,1*H*-Pyrrol-2-yl-, 1*H*-Pyrazol-4-yl-, 1*H*-Pyrazol-5-yl-, 1*H-*Imidazol-1-yl-, 1*H*-Imidazol-4-yl-, 1*H*-Indol-3-yl-, 1*H*-Benzimidazol-2-yl-gruppe substituierte C₁₋₆-Alkylgruppe, wobei die vorstehend erwähnten Heteroarylteile an den verfügbaren Kohlenstoffatomen zusätzlich jeweils durch eine oder zwei Gruppen ausgewählt aus Fluor-, Chlor-, Brom- oder Iodatomen, Alkyl-, Alkoxycarbonyl-, Carboxy-, Trifluormethyl-, Trifluormethoxy-, Cyano-, Amino-, Alkylamino-, Dialkylamino-, Nitro-, Hydroxy- oder Alkoxygruppen substituiert sein können, wobei die Substituenten gleich oder verschieden sein können,
eine durch eine Carboxy-, Alkoxycarbonyl-, Aminocarbonyl-, Alkylaminocarbonyl-, Dialkylaminocarbonyl-, Aralkylaminocarbonyl-, Cyano-, Hydroxy-, Alkoxy-, Aryloxy-, Amino-, Alkylamino-, Dialkylamino-, Alkylcarbonylamino- oder Alkoxycarbonylaminogruppe substituierte C₁₋₆-Alkylgruppe, die zusätzlich durch eine oder zwei Arylgruppen oder eine Heteroarylgruppe substituiert ist, wobei die Heteroarylgruppe eine 2-Pyridyl-, 3-Pyridyl-, 4-Pyridyl-, 2-Pyrazinyl-, 1*H*-Pyrrol-2-yl-, 1*H*-Pyrazol-4-yl-, 1*H*-Pyrazol-5-yl-, 1*H*-Imidazol-1-yl-, 1*H*-Imidazol-4-yl-, 1*H*-Indol-3-yl-, 1*H*-Benzimidazol-2-yl-gruppe darstellt, wobei die vorstehend erwähnten Aryl- oder Heteroarylteile an den verfügbaren Kohlenstoffatomen zusätzlich jeweils durch eine oder zwei Gruppen ausgewählt aus Fluor-, Chlor-, Brom- oder Iodatomen, Alkyl-, Alkoxycarbonyl-, Carboxy-, Trifluormethyl-, Trifluormethoxy-, Cyano-, Amino-, Alkylamino-, Dialkylamino-, Nitro-, Hydroxy- oder Alkoxygruppen substituiert sein können, wobei die Substituenten gleich oder verschieden sein können,
eine durch eine Arylgruppe substituierte C₁₋₆-Alkylgruppe, die im Arylteil durch eine Hydroxy-oder Aminogruppe und zusätzlich durch zwei Fluor-, Chlor-, Brom- oder Iodatome, wobei die Substituenten gleich oder verschieden sein können, substituiert ist,
eine durch eine Carboxy- oder Alkoxycarbonylgruppe substituierte C₂₋₆-Alkylgruppe, die zusätzlich durch eine Amino-, Alkylamino-, Dialkylamino-, Alkylcarbonylamino-, Arylcarbonylamino-, Arylsulfonylamino-, Alkoxycarbonylamino- oder Aralkoxycarbonylaminogruppe substituiert ist,
eine 3-Chinuclidinyl- oder 4-Chinuclidinylgruppe,
bedeutet, und
wobei, soweit nichts anderes erwähnt wurde, die vorstehend erwähnten Alkyl-, Alkylen- und Alkoxyteile jeweils 1 bis 4 Kohlenstoffatome enthalten,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
wobei, sofern nichts anderes erwähnt wurde, jedes Kohlenstoffatom in den vorstehend erwähnten Alkyl-, Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

Besonders bevorzugt sind Verbindungen der Formel I, wobei
**R_{b}** eine gegebenenfalls durch ein Fluor-, Chlor- oder Bromatom oder eine Carboxy-, C₁₋₂ Alkyl-, C₁₋₂ Alkoxy-, Cyano- oder Trifluormethylgruppe substituierte Naphthylgruppe,
eine gegebenenfalls durch eine Methylgruppe am Stickstoff substituierte 5- oder 6-Indazolylgruppe oder eine 1,3-Dihydro-2-oxo-indol-6-yl-gruppe oder
eine gegebenenfalls durch die Reste **R₁** bis **R₃** substituierte Phenylgruppe darstellt, wobei
   **R₁** ein Fluor-, Chlor-, Brom- oder Iodatom, eine C₁₋₂ Alkyl-, Trifluormethyl-, Aminocarbonyl-, Carboxy-, Alkoxycarbonyl-, Cyano-, Phenylaminocarbonyl-, Benzylaminocarbonyl-, C₁₋₃-Alkylsulfonyl-, Aminosulfonyl-, Methylaminosulfonyl-, Dimethylaminosulfonyl-, Morpholinosulfonyl-, N-Methylpiperazinosulfonyl-, Homopiperazinosulfonyl-, 2,6-Dimethylpiperazin-4-yl-, 2-Aminopyridyl-N-sulfonyl-, Morpholino-, 4-Methyl-1-piperazinyl-, (N-Methyl-N-methylsulfonyl)amino-, 2-Carboxy-1-ethyl-, Dimethylamino-1-ethyl- oder Nitrogruppe,
   eine Methylgruppe, die durch eine 1,2,4,5-Tetrahydro-benzo[*d*]azepin-3-yl-, eine Dialkylamino- oder eine Pyrrolidino-, Piperidino-, 2,6-Dimethyl-piperidino-1-yl-, 4-Methoxy-piperidino-1-yl-, Morpholino-, S-Dioxo-thiomorpholino-, Piperazino- oder 4-Methyl-1-piperazinylgruppe oder eine Fluoralkylaminogruppe der Formel

      -(CH₂)ᵣ-(CF₂)ₛ-Q,

      worin
      **r** 0 oder eine ganze Zahl von 1 bis 3,
      **s** eine ganze Zahl von 1 bis 3, und
      **Q** Wasserstoff, Fluor oder Chlor bedeuten,
substituiert ist,
- **R₂**: ein Fluor- oder Chloratom, eine Hydroxy-, Amino- oder Methylgruppe und
- **R₃**: ein Chloratom, oder
eine Tetrazolyl-, Triazolyl-, Imidazolyl- oder Pyrazolylgruppe,
worin der Anknüpfungspunkt ein Kohlenstoffatom oder ein Stickstoffatom ist und an dem Ring ein Wasserstoffatome durch einen Alkylreste ersetzt sein kann, oder
eine Aminosulfonyl-, Methylaminosulfonyl-, Dimethylaminosulfonyl-, Morpholinosulfonyl-, N-Methylpiperazinosulfonyl-, Homopiperazinosulfonyl-, oder 2-Aminopyridyl-N-sulfonylgruppe,
darstellen, oder
- **R₂ und R₃**: zusammengenommen eine Gruppe der Formel

-(CH₂)m-N**R₅**-(CH₂)ₙ-

worin n und m jeweils unabhängig voneinander 1 oder 2 bedeuten, und
- **R₅**: für eine Fluoralkylgruppe der Formel

-(CH₂)ᵣ-(CF₂)_{s'}-**Q'**

steht,
worin
**r'** 0 oder eine ganze Zahl von 1 bis 3,
**s'** eine ganze Zahl von 1 bis 3, und
**Q'** Wasserstoff, Fluor oder Chlor bedeuten,
die Gruppe **R_{c}**N**R_{d}**
eine durch den Rest **R₆** substituierte 5- bis 7-gliedrige Alkyleniminogruppe,
wobei **R₆** eine Hydroxy-, Alkoxy-, Aryloxy-, Amino-, Alkylamino-, Dialkylamino-, Alkylcarbonylamino-, N-Alkyl-alkylcarbonylamino-, Alkoxycarbonylalkylamino-, N-(Alkyl)-N-(alkoxycarbonylalkyl)-amino-, Alkylsulfenyl-, Alkylsulfinyl-, Alkylsulfonyl-, Arylsulfenyl-, Arylsulfinyl-, Arylsulfonyl-, Carboxy-, Alkoxycarbonyl-, Aminocarbonyl-, Alkylaminocarbonyl-, Dialkylaminocarbonyl- oder Cyanogruppe,
eine Alkylgruppe, die durch eine Hydroxy-, Amino-, Alkylamino-, Dialkylamino-, Alkylcarbonylamino-, N-Alkyl-alkylcarbonylamino-, Dialkylaminocarbonylalkylamino-, N-(Alkyl)-N-(dialkylaminocarbonylalkyl)-amino-, Alkoxycarbonyl-, Carboxy-, Dialkylaminoalkoxy-gruppe oder durch eine 5- bis 7-gliedrige Alkyleniminogruppe, wobei in den vorstehend erwähnten 6- bis 7-gliedrigen Alkyleniminogruppen eine Methylengruppe in 4-Stellung durch ein Sauerstoff- oder Schwefelatom oder eine Imino-, N-Alkyl-imino- oder N-Alkylcarbonyl-iminogruppe ersetzt sein kann, substituiert ist,
eine Alkyleniminogruppe mit 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine N-Alkyl-imino-, N-Alkylcarbonyl-imino- oder N-Aralkyl-iminogruppe ersetzt sein kann,
eine durch eine Hydroxy-, Amino-, Alkylamino-, Dialkylamino-, Carboxy-, Alkoxycarbonyl-, Aminocarbonyl-, Alkylaminocarbonyl- oder Dialkylaminocarbonylgruppe substituierte Alkyleniminogruppe mit 5 bis 7 Ringatomen im Alkyleniminoteil,
eine gegebenenfalls jeweils im Aryteil durch ein Fluor-, Chlor-, oder Bromatom oder eine Nitro-, Alkyl-, Alkoxy- oder Cyanogruppe substituierte 3,4-Dihydro-1*H-*quinazolin-2-on-3-yl- oder eine 1*H*-Benzimidazol-2-on-1-yl-gruppe, darstellt,
eine gegebenenfalls durch 1 oder 2 Alkylgruppen substituierte 6- bis 7-gliedrige Alkyleniminogruppe, wobei eine Methylengruppe in 4-Stellung durch ein Sauerstoff- oder Schwefelatom, durch eine Sulfinyl-, Sulfonyl- oder eine N**R₁₀**-Gruppe ersetzt ist,
wobei **R₁₀** ein Wasserstoffatom oder eine Alkyl-, Aralkyl- Amino-C₂₋₄-alkyl-, Hydroxy-C₂₋₄alkyl-, Alkylcarbonyl-, Aralkoxycarbonyl-, Alkylsulfonyl-, Arylcarbonyl-, Arylsulfonyl-,
eine (Alkylenimino)carbonylalkylgruppe mit 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine N-Alkyl-imino-, N-Alkylcarbonyl-imino- oder N-Aralkyl-iminogruppe ersetzt sein kann,
eine 2-, 3- oder 4-Pyridylgruppe,
eine 2-, 3- oder 4-Pyrimidylgruppe,
eine gegebenenfalls durch ein oder zwei Fluor-, Chlor-, Brom- oder Iodatome oder eine oder zwei Nitro-, Alkyl-, Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl- oder Cyanogruppen substituierte Phenylgruppe, wobei die Substituenten gleich oder verschieden sein können,
eine 8-Methyl-8-aza-bicyclo[3.2.1]oct-3-ylgruppe,
eine Benzhydrylgruppe, in der unabhängig voneinander jeder Phenylteil jeweils durch ein Fluor-, Chlor-, Brom- oder Iodatom oder eine Nitro-, Alkyl-, Hydroxy-, Alkoxygruppe substituiert sein kann, wobei die Substituenten gleich oder verschieden sein können,

eine durch eine Phenylgruppe substituierte 6- oder 7-gliedrige Alkyleniminogruppe, die zusätzlich durch eine Hydroxy-, Carboxy-, Alkoxycarbonyl- oder Cyanogruppe substituiert ist oder in der eine Methylengruppe in 4-Stellung durch eine Carbonylgruppe ersetzt ist,
eine in 4-Position durch eine Carboxy-, Alkoxycarbonyl-, Aminocarbonyl-, Alkylaminocarbonyl-, Dialkylaminocarbonylgruppe substituierte 3-Thiazolidinyl-Gruppe,
eine Gruppe der Struktur 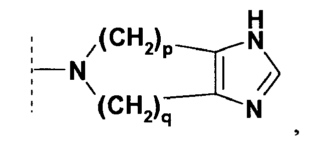 worin **p** und **q**, die gleich oder verschieden sein können, unabhängig voneinander die Zahl 1 oder 2 bedeuten, wobei der Imidazoring durch eine oder zwei Alkyl- oder Arylgruppen substituiert sein kann, wobei die Substituenten gleich oder verschieden sein können,
eine 1-Pyrrolidinyl- oder 1-Piperidinylgruppe, in der die beiden Wasserstoffatome einer Methylengruppe durch eine geradkettige C₃₋₅-Alkylenbrücke ersetzt sind, wobei jeweils eine Methylengruppe in der C₃₋₅-Alkylenbrücke durch eine Imino-, N-Alkyl-imino- oder N-(Aralkyl)imino-Gruppe ersetzt ist, wobei der so gebildete bicyclische Ring gegebenenfalls zusätzlich durch eine Hydroxygruppe substituiert ist,
eine 1-Piperidinylgruppe, die in 4-Stellung durch eine Hydroxy-, Alkoxy- oder Aralkoxygruppe substituiert ist und bei der zusätzlich jeweils eines der Wasserstoffatome in Position 2 und 6 des Piperidinyl-Gerüsts zusammen durch eine Ethylenbrücke ersetzt sind,
   oder
**R_{c}** ein Wasserstoffatom oder eine C₁₋₆-Alkylgruppe,
eine durch eine Phenyl- oder eine 2-, 3- oder 4-Pyridylgruppe substituierte Alkylgruppe,
eine durch eine Hydroxy- oder Alkoxygruppe substituierte C₂₋₄-Alkylgruppe, und
**R_{d}** eine C₁₋₆-Alkylgruppe bedeuten, die durch eine Gruppe ausgewählt aus den Gruppen (a) bis (j) substituiert ist:
   (a) eine Gruppe der Formel -C(=NH)NH₂,
   (b) eine Carboxy-, Alkoxycarbonyl-, Carboxymethylaminocarbonyl-, Aminocarbonyl-, Alkylaminocarbonyl-, Alkylcarbonylamino-, Dialkylaminocarbonyl-, Arylaminocarbonyl-, N-Alkyl-arylaminocarbonyl-, Aralkylaminocarbonyl-, N-Alkyl-aralkylaminocarbonyl- oder Cyanogruppe,
   (c) eine Hydroxy-, Amino-, Alkoxy-, Alkylamino-, Dialkylamino-, Alkylcarbonylamino-, N-Alkyl-alkylcarbonylamino-, Alkoxycarbonylamino-, Alkoxyacetylamino-, Dialkylaminoacetylamino-, N-Alkyl-alkoxycarbonylamino-, Alkylsulfonylamino-, N-Alkyl-alkylsulfonylamino-, Arylamino-, Naphthylamino-, Aralkylamino-, Diaralkylamino-, N-Alkyl-aralkylamino-, Alkylsulfenylgruppe,
   (d) eine Nitro-2-pyridyl-amino-gruppe,
   (e) eine durch einen, zwei oder drei Arylreste substituierte Methoxygruppe,
   (f) eine 4- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoff- oder Schwefelatom, durch eine Imino-, N-Alkyl-imino-, N-(Hydroxy-C₂₋₄-alkyl)-imino- oder N-(Amino-C₂₋₄-alkyl)-iminogruppe ersetzt sein kann, und zusätzlich in den vorstehend erwähnten 5- bis 7-gliedrigen Alkyleniminogruppen jeweils eine zu den Stickstoffatomen benachbarte Methylengruppe durch eine Carbonylgruppe ersetzt sein kann,
   (g) eine durch eine Dialkylaminoalkylgruppe substituierte 1-Piperidinylgruppe,
   (h) eine 2-Aza-bicyclo[2.2.1]hept-5-en-2-yl-gruppe,
   (i) eine 5- bis 7-gliedrige (Alkylenimino)carbonylgruppe, wobei in den vorstehend erwähnten 6- bis 7-gliedrigen Alkyleniminogruppen eine Methylengruppe in 4-Stellung durch ein Sauerstoff- oder Schwefelatom, durch eine Imino- oder N-Alkyl-iminogruppe ersetzt sein kann, und
   (j) eine (**R₈R₉**)CON**R₇**-Gruppe, in der
      **R₇, R₈** und **R₉,** die gleich oder verschieden sein können, jeweils ein Wasserstoffatom oder eine Methylgruppe oder
      **R₇** und **R₈** zusammen eine n-C₂₋₃-Alkylengruppe und **R₉** ein Wasserstoffatom oder eine Methyl- oder 4-Pyridylgruppe oder
      **R₇** und **R₈** ein Wasserstoffatom und **R₉** eine Aryl-C₁₋₂-alkyl- oder Phenylgruppe darstellen,
eine in 2-, 3- oder 4-Stellung durch eine Hydroxy-, Amino-, Alkylamino-, Dialkylamino-, Aminomethyl-, Hydroxymethyl-, Alkoxycarbonyl-, Aminocarbonyl-, Alkylaminocarbonyl-, Dialkylaminocarbonyl- oder Carboxygruppe substituierte Cyclohexylgruppe,
eine in 4-Stellung durch eine Carboxyalkylgruppe substituierte Cyclohexylgruppe,
eine in 2-Stellung durch eine 2-Amino-1-ethylthio-, 2-Hydroxy-1-ethoxy-, 2-(2-Amino-1-ethoxy)-1-ethoxy- oder 2-(2-Hydroxy-1-ethoxy)-1-ethoxy-gruppe substituierte Ethylgruppe,
eine in 3-Stellung durch eine 3-Amino-1-propoxy- oder 2-(3-Amino-1-propoxy)-1-ethoxy-gruppe substituierte Propylgruppe,
eine durch eine C₅₋₆-Cycloalkylgruppe substituierte C₁₋₂-Alkylgruppe, wobei der Cycloalkylteil durch eine Hydroxy-, Aminomethyl-, Dimethylaminomethyl-, 2-Carboxyethyl- oder tert.-Butyloxycarbonylaminomethylgruppe substituiert ist oder wobei im Cycloalkylteil eine Methylengruppe durch ein Sauerstoffatom, eine N-Alkyl-imino- oder N-(2-Dialkylaminoacetyl)iminogruppe ersetzt ist,
eine 4-Piperidinyl-methylgruppe, die in 1-Stellung durch eine Alkyl- oder Aralkylgruppe und zusätzlich in der 4-Stellung durch eine Hydroxygruppe substituiert ist und bei der zusätzlich jeweils ein Wasserstoffatom in Position 2 und 6 des Piperidinyl-Gerüsts zusammen durch eine Ethylenbrücke ersetzt sind,
eine 3-Pyrrolidinyl- oder eine 3- oder 4-Piperidinylgruppe, die jeweils in 1-Stellung durch eine Alkyl-, Aralkyl- oder Arylsulfonylgruppe substituiert ist,
eine 4-Piperidinylgruppe, die in 1-Stellung durch eine Alkyl-, Aralkyl- oder Arylgruppe und zusätzlich in 4-Position durch eine Carboxygruppe substituiert ist,
eine Aralkylgruppe, die im Arylteil durch eine Hydroxy-, Aminosulfonyl-, Carboxy-, Nitro-, Amino-, Aminomethyl-, 2-Amino-1-ethyl-, Alkoxycarbonyl-, 4-Hydroxyphenyl- oder 2*H-*pyridazin-3-on-6-yl-gruppe substituiert ist,
eine durch eine 3-Hydroxy-1,3-dihydro-indol-2-on-3-yl- oder 2-Aminocarbonyl-1,3-dihydro-isoindol-5-yl-gruppe substituierte Methylgruppe,
eine im Arylteil durch eine 3-Carboxy-1-propylgruppe substituierte 2-Indanylgruppe,
eine durch eine 1*H*-2-Benzimidazolyl- oder 4-Amino-3,5-dichlorphenylgruppe substituierte Alkylgruppe,
eine Aralkylgruppe, die im Alkylteil durch eine Hydroxy-, Amino-, Alkylamino-, Dialkylamino-, Aminocarbonyl-, Alkylaminocarbonyl-, Dialkylaminocarbonyl-, Aralkylaminocarbonyl-, Carboxy- oder Cyanogruppe substituiert ist und gegebenenfalls zusätzlich im Arylteil durch ein oder zwei Fluor-, Chlor- oder Bromatome oder eine oder zwei Hydroxy- oder Alkoxygruppen substituiert ist, wobei die Substituenten gleich oder verschieden sein können,
eine durch eine Carboxygruppe und zusätzlich zwei Phenylgruppen substituierte Alkylgruppe,
eine durch eine Carboxygruppe und zusätzlich durch eine Hydroxy-, Aminocarbonyl-, 1*H-*Imidazol-4-yl- oder Benzyloxycarbonylaminogruppe substituierte C₂₋₆-Alkylgruppe,
eine durch eine Alkoxycarbonylgruppe und zusätzlich durch eine Pyridylgruppe substituierte Alkylgruppe,
eine durch eine Hydroxygruppe und zusätzlich durch eine Amino-, Alkylamino-, Dialkylamino-, Hydroxy-, Alkoxy-, 1-Pyrrolidinyl-, 1-Piperidinyl- oder Morpholinogruppe substituierte C₃₋₆-Alkylgruppe,
eine Aralkylgruppe, die im Arylteil durch eine Alkoxy- und zusätzlich durch eine Carboxy- oder Hydroxygruppe substituiert ist,
eine durch eine 2-Pyridyl-, 3-Pyridyl-, 4-Pyridyl-, 2-Pyrazinyl-, 3-Chlor-5-trifluormethyl-2-pyridyl-, 1-Methyl-1*H*-pyrrol-2-yl-, 1*H*-Pyrazol-4-yl-, 4-Ethoxycarbonyl-1*H*-pyrazol-5-yl-, 1*H-*Imidazol-1-yl-, 1*H-*Imidazol-4-yl-, 1*H-*Indol-3-yl-, 6-Methoxy-1*H*-benzimidazol-2-yl-gruppe substituierte Alkylgruppe,
eine in 5-Stellung durch eine Alkoxycarbonylgruppe substituierte 1-Pentylgruppe, die zusätzlich in 5-Stellung durch eine Amino-, Alkylcarbonylamino-, Arylcarbonylamino-, Arylsulfonylamino-, Alkoxycarbonylamino- oder Aralkoxycarbonylaminogruppe substituiert ist,
wobei, soweit nichts anderes erwähnt wurde, die vorstehend erwähnten Alkyl-, Alkylen- und Alkoxyteile jeweils 1 bis 4 Kohlenstoffatome enthalten,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
wobei, sofern nichts anderes erwähnt wurde, jedes Kohlenstoffatom in den vorstehend erwähnten Alkyl-, Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

Ganz besonders bevorzugt sind Verbindungen der Formel I, wobei
**R_{b}** eine 1-Naphthylgruppe oder eine gegebenenfalls in 5 Position durch eine Carboxy-Gruppe substituierte 2-Naphthylgruppe,
eine 1,3-Dihydro-2-oxo-indol-6-ylgruppe, Benzotriazol-5-yl-, Benzimidazol-5-yl, Indazol-5-yl-, Indazol-6-yl- oder 1-Methyl-1*H*-indazol-6-ylaminogruppe,
eine gegebenenfalls in 4-Position des Phenylteils durch ein Fluor-, Chlor- oder Bromatom, eine Cyano-, Propyl-2-sulfonyl-, Aminosulfonyl-, Methylaminosulfonyl-, Dimethylaminosulfonyl-, Morpholinosulfonyl-, N-Methylpiperazinosulfonyl-, Homopiperazinosulfonyl-, 2,6-Dimethylpiperazin-4-yl-, 2-Aminopyridyl-N-sulfonyl-, Carboxy-, Piperidinomethyl-, 1,2,4,5-Tetrahydro-benzo[*d*]azepin-3-yl-methyl-, 2-Carboxy-1-ethyl-, Phenylaminocarbonyl-, Benzylaminocarbonyl-, C₁₋₃-Alkylsulfonyl-, Aminocarbonyl-, Methoxycarbonyl-, (N-Methyl-N-methylsulfonyl)amino -, Diethylaminomethyl-, 3-Diethylamino-1-propyloxy-, Morpholino-, 4-Methyl-1-piperazinyl-, 2*H*-Tetrazol-5-yl-, 1*H*-Imidazol-4-yl oder Nitrogruppe substituierte Phenylgruppe,
eine in 3-Position des Phenylteils durch ein Chlor- oder Bromatom, eine Cyano-, Aminocarbonyl-, Carboxy-, Ethoxycarbonyl- oder Nitrogruppe oder durch eine Gruppe der Formel

   -CH₂-NH-(CH₂)ᵣ-CₛF₂ₛ₊₁,

   worin **r** 1 oder 2 und **s** 1, 2 oder 3 bedeutet,
substituierte Phenylgruppe,
eine 3,4-Dichlorphenyl-, 3,5-Dichlorphenyl-, 4-Amino-3,5-dichlorphenyl-, 3-Chlor-4-fluorphenyl-, 4-Chlor-3-methylphenyl-, 4-Chlor-3-trifluormethylphenyl-, 4-Brom-3-chlorphenyl-oder 3-Hydroxy-4-methylphenylgruppe, oder
eine Gruppe der Formel 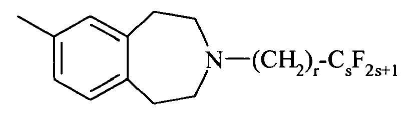 worin **r** 1 oder 2 und **s** 1, 2 oder 3 bedeutet,
die Gruppe **R_{c}**N**R_{d}**
   eine in 2-Stellung durch eine Hydroxymethyl-, 1-Pyrrolidinylmethyl- oder 2-Ethoxycarbonyl-1-ethyl-gruppe substituierte 1-Pyrrolidinylgruppe,
   eine in 3-Stellung durch eine Amino-, Acetylamino-, N-Methyl-acetylamino oder tert.-Butyloxycarbonylamino substituierte 1-Pyrrolidinylgruppe,
   eine 4-Carboxy-3-thiazolidinyl-, eine 7-Methyl-2,7-diaza-spiro[4.4]non-2-yl- oder eine 5-Hydroxy-2-methyl-2,8-diaza-spiro[5.5]undec-8-yl-Gruppe,
eine Morpholino- oder S-Oxido-thiomorpholinogruppe,
eine in 2-Stellung durch eine Ethoxycarbonyl-, Hydroxymethyl-, 3-Hydroxypropyl-, 3-Diethylamino-1-propyl- oder 2-(2-Diethylaminoethoxy)-1-ethyl-gruppe substituierte 1-Piperidinylgruppe,
eine in 3-Stellung durch eine Hydroxy-, Hydroxymethyl-, 3-Diethylamino-1-propyl-, Aminocarbonyl-, Dimethylaminocarbonyl-, Carboxy-, 1-Pyrrolidinylmethyl-, 4-(1-Pyrrolidinyl)-1-butyl-, Methoxycarbonylmethyl- oder Acetylaminomethylgruppe substituierte 1-Piperidinylgruppe,
eine in 4-Stellung durch eine Ethoxycarbonyl-, 3-Hydroxypropyl-, Hydroxy-, Aminomethyl-, 2-(2-Diethylaminoethoxy)-1-ethyl-, 2-Carboxy-1-ethyl-, N-(2-Methoxycarbonyl-1-ethyl)-N-methylamino-, 2-(N-(Dimethylaminocarbonylmethyl-)-N-methyl-amino)-1-ethyl, N-Acetyl-N-methylaminomethyl-, 8-Methoxy-3,4-dihydro-1H-quinazolin-2-on-3-yl-, 1-Piperidinyl-, 3-Hydroxy-1-piperidinyl- oder 4-Ethoxycarbonyl-1-piperidinylgruppe substituierte 1-Piperidinylgruppe,
eine 3,5-Dimethyl-1-piperazinyl-, 1,4,6,7-Tetrahydro-imidazo[4,5-c]pyridin-5-yl-, 2-Methyl-1,4,6,7-tetrahydro-imidazo[4,5-c]pyridin-5-yl-, 1,4,5,6,7,8-Hexahydro-imidazo[4,5-d]azepin-6-yl-, 2-Methyl-1,4,5,6,7,8-hexahydro-imidazo[4,5-d]azepin-6-yl-, 3-Phenyl-azepan-4-on-1-yl- oder 4-Carboxy-4-phenyl-1-piperidinylgruppe,
eine 1-Piperazinylgruppe, die in 4-Stellung gegebenenfalls durch eine Methyl-, Acetyl-, Benzyloxycarbonyl-, 2-Pyridyl-, 2-Pyrimidinyl-, 2-Nitrophenyl-, 3-Methoxyphenyl-, 4-Cyanophenyl-, 3,4-Dimethoxyphenyl-, 4-[Bis-(4-methoxy-phenyl)]-methyl-, 8-Methyl-8-aza-bicyclo[3.2.1]oct-3-yl-, Morpholinocarbonylmethyl-, 2-Amino-1-ethyl- oder 3-Hydroxy-1-propylgruppe substituiert ist,
eine 1-Homopiperazinylgruppe, die in 4-Stellung gegebenenfalls durch eine Methylgruppe substituiert ist,
eine 3-Hydroxy-8-aza-bicyclo[3.2.1]oct-8-ylgruppe,
bedeutet, oder
**R_{c}** ein Wasserstoffatom oder eine Methyl-, Ethyl-, 2-Methoxyethyl-, 2-Hydroxyethyl-, i-Propyl, n-Propyl-, n-Butyl-, Benzyl- oder 3-Pyridylmethylgruppe bedeutet, und
**R_{d}** eine durch eine Gruppe der Formel -C(=NH)NH₂ oder eine Cyano-, Carboxy-, Ethoxycarbonyl-, Aminocarbonyl-, C₁₋₂-Alkylcarbonylamino-2,2-dimethyl-ethylgruppe, C₁₋₂-Alkylcarbonylamino-1,1-dimethyl-ethylgruppe, C₁₋₂-Alkylcarbonylamino-2,2-dimethyl-propylgruppe, Carboxymethylaminocarbonyl-, 1-Hydroxy-1-cyclohexyl-, Aminomethylcyclohexyl-, 3-Hydroxy-8-methyl-8-aza-bicyclo[3.2.1]oct-3-yl-, 3-Hydroxy-1,3-dihydro-indol-2-on-3-yl-, 2-Aminocarbonyl-1,3-dihydro-isoindol-5-yl-, 2-Tetrahydrofuryl-, 1-Ethyl-2-pyrrolidinyl-, 1*H*-Imidazol-4-yl-, 1-Methyl-4-piperidinyl-, 1-(2-Dimethylaminoacetyl)-4-piperidinyl-, 2-Pyridyl-, 3-Pyridyl-, 4-Pyridyl-, 2-Pyrazinyl-, 3-Chlor-5-trifluormethyl-2-pyridyl-, 4-Ethoxycarbonyl-1*H*-pyrazol-5-yl-, 2-Carboxyphenyl-, 3-Carboxyphenyl-, 2-Hydroxyphenyl-, 4-Hydroxyphenyl-, 2-Nitrophenyl-, 3-Nitrophenyl-, 4-Nitrophenyl-, 3-Aminophenyl-, 4-Aminophenyl-, 4-(Aminosulfonyl)phenyl-, 4'-Hydroxybiphenyl-, 4-(Aminomethyl)phenyl- oder 4-Hydroxy-3-methoxyphenyl-gruppe substituierte Methylgruppe,
eine durch eine Carboxygruppe substituierte C₂₋₅-Alkylgruppe,
eine durch eine Hydroxy-, Acetylamino-, Amino- oder Dimethylaminogruppe substituierte C₂₋₅-Alkylgruppe, mit der Maßgabe, dass die Heteroatome der vorstehend genannten Substituenten von dem Stickstoffatom der **R_{c}**N**R_{d}**-Gruppe durch mindestens zwei Kohlenstoffatome getrennt sind,
eine im Methylenteil durch eine Carboxy- oder Cyanogruppe substituierte Benzylgruppe,
eine durch eine Carboxygruppe und eine 4-Hydroxyphenylgruppe substituierte Methylgruppe,
eine in 1-Stellung durch eine Methoxycarbonyl- oder eine 1*H*-Benzimidazol-2-ylgruppe substituierte Ethylgruppe,
eine in 2-Stellung durch eine Methoxy-, Diphenylmethoxy-, Methylthio-, Methylamino-, Diethylamino-, Diisopropylamino-, Acetylamino-, N-Methylacetylamino-, 2-Methoxyacetylamino-, 2-Dimethylaminoacetylamino-, Isopropylcarbonylamino-, 2-Methyl-propylcarbonylamino-, Phenyl-acetylamino-, *tert*.-Butyloxycarbonylamino-, Methylsulfonylamino-, Benzoylamino-, Phenylamino-, 1-Naphthylamino-, 4-Nitro-2-pyridylamino-, Cyano-, Ethoxycarbonyl-, Aminocarbonyl-, Methylaminocarbonyl-, Dimethylaminocarbonyl-, 2-Hydroxy-1-ethoxy-, 2-(2-Amino-1-ethoxy)-1-ethoxy-, 2-(2-Hydroxy-1-ethoxy)-1-ethoxy-, 2-Amino-1-ethylthio-, 1-Methyl-2-pyrrolidinyl-, 1-Pyrrolidinyl-, 2-Oxo-pyrrolidin-1-yl-, 1-Piperidinyl-, 2-Oxo-piperidin-1-yl-, Morpholino-, 4-(2-Hydroxyethyl)-1-piperazinyl-, 2-(2-Dimethylaminoethyl)-1-piperidinyl-, 4-Methyl-1-piperazinocarbonyl-, 3-Carboxy-2-methoxy-phenyl-, 2-Hydroxyphenyl-, 3-Hydroxyphenyl-, 4-Hydroxyphenyl-, 4-(Aminosulfonyl)phenyl-, 4-Nitrophenyl- , 3-Methoxycarbonylphenyl-, 2-(2-Amino-1-ethyl)phenyl-, 4-Pyridyl-, 1*H*-Imidazol-1-yl-, 1*H-*Imidazol-4-yl-, 1*H*-Pyrazol-4-yl-, 1-Methyl-1*H-*pyrrol-2-yl-, 1*H*-Indol-3-yl-, 6-Methoxy-1*H*-benzoimidazol-2-yl-, 4-(2*H*-pyridazin-3-on-6-yl)-phenyl- oder Imidazolidin-2-on-1-ylgruppe substituierte Ethylgruppe,
eine in 1-Stellung durch eine Carboxygruppe und in 2-Stellung zusätzlich durch eine Hydroxy-, Aminocarbonyl-, 2-Chlorphenyl-, 4-Chlorphenyl-, 1*H*-Imidazol-4-yl- oder 4-Hydroxyphenylgruppe substituierte Ethylgruppe,
eine in 1-Stellung durch eine Aminocarbonylgruppe und in 2-Stellung zusätzlich durch eine 4-Methoxyphenylgruppe substituierte Ethylgruppe,
eine in 1-Stellung durch eine 4-Phenyl-1-butylaminocarbonylgruppe und in 2-Stellung zusätzlich durch eine Phenylgruppe substituierte Ethylgruppe,
eine in 2-Stellung durch eine Hydroxygruppe und in 2-Stellung zusätzlich durch eine Phenyl-, 3-Hydroxyphenyl-, 4-Hydroxyphenyl- oder 4-Hydroxy-3-methoxyphenylgruppe substituierte Ethylgruppe,
eine in 1-Stellung durch eine Phenylgruppe und in 2-Stellung zusätzlich durch eine Hydroxy- oder Carboxygruppe substituierte Ethylgruppe,
eine in 1-Stellung durch eine 3-Pyridylgruppe und in 2-Stellung zusätzlich durch eine Ethoxycarbonylgruppe substituierte Ethylgruppe,
eine in 1-Stellung durch eine Carboxygruppe und in 2-Stellung zusätzlich durch zwei Phenylgruppen substituierte Ethylgruppe,
eine in 2-Stellung durch eine Hydroxygruppe und in 3-Stellung zusätzlich durch eine Amino-, Hydroxy- oder Morpholinogruppe substituierte n-Propylgruppe,
eine in 3-Stellung durch eine Methoxy-, Isopropylamino-, Methylamino-, Diethylamino-, Dibenzylamino-, 1-Pyrrolidinyl-, 1-Piperidinyl-, Morpholino-, 4-Methyl-1-piperazinyl, -tert.-Butyloxycarbonylamino-, 2-Oxo-1-pyrrolidinyl-, 2-Oxo-piperidin-1-yl-, Ethoxycarbonyl-, 4-Pyridyl-, 4-Amino-3,5-dichlorphenyl-, 3-Amino-1-propoxy-, 2-(3-Amino-1-propoxy)-1-ethoxy-, 1*H*-Imidazol-1-yl-, 2-Aza-bicyclo[2.2.1]hept-5-en-2-yl-, 4-(3-Amino-1-propyl)-1-piperazinyl-oder 2-Diethylaminomethyl-1-piperidinyl-gruppe substituierte n-Propylgruppe,
eine in 4-Stellung durch 4-Hydroxyphenylgruppe substituierte n-Butylgruppe,
eine in 4-Stellung durch eine Dimethylaminogruppe und in 2-Stellung zusätzlich durch eine Phenylgruppe substituierte n-Butylgruppe,
eine in 3-Position durch eine Phenylaminocarbonylamino- oder eine 1-(4-Pyridyl)-3-imidazolin-2-on-3-yl substituierte 2-Methyl-2-butylgruppe,
eine in 1-Stellung durch eine Carboxygruppe und zusätzlich in 5-Stellung durch eine Benzyloxycarbonylaminogruppe substituierte n-Pentylgruppe,
eine in 5-Stellung durch eine Methoxycarbonylgruppe und in 5-Stellung zusätzlich durch eine Acetylaminogruppe substituierte 1-Pentylgruppe,
eine in 6-Stellung durch eine Hydroxy-, Amino-, tert.-Butyloxycarbonylamino- oder N-Methyl-N-phenethylaminogruppe substituierte n-Hexylgruppe,
eine in 2-Stellung durch eine Hydroxy-, Amino-, Dimethylamino- oder Hydroxymethyl-gruppe substituierte Cyclohexylgruppe,
eine in 3-Stellung durch eine Amino- oder Carboxygruppe substituierte Cyclohexylgruppe,
eine in 4-Stellung durch eine Hydroxy-, Amino-, Carboxy-, 2-Carboxyethyl-, 3-Carboxypropyl-, Methoxycarbonyl- oder Dimethylaminogruppe substituierte Cyclohexylgruppe,
eine in 3-Stellung des Cyclohexylteils durch eine Aminomethyl- oder eine tert.-Butyloxycarbonylaminomethylgruppe substituierte Cyclohexylmethylgruppe,
eine in 4-Stellung des Cyclohexylteils durch eine Aminomethyl-, Dimethylaminomethyl- oder 2-Carboxyethylgruppe substituierte Cyclohexylmethylgruppe,
eine in 1-Stellung durch eine Methyl-, Benzyl- oder Phenylsulfonylgruppe substituierte 4-Piperidinylgruppe,
eine 1-Methyl-4-carboxy-4-piperidinylgruppe,
eine 1-Ethyl-3-piperidinyl-, 1-Benzyl-3-pyrrolidinyl- oder 5-(3-Carboxy-1-propyl)-indan-2-yl)-gruppe,
bedeutet,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Insbesonders bevorzugt sind solche Verbindungen der Formel I, worin:
**R_{b}** eine gegebenenfalls in 4-Position des Phenylteils durch ein Fluor-, Chlor- oder Bromatom, eine Cyano-, Propyl-2-sulfonyl-, Aminosulfonyl-, Dimethylaminosulfonyl-, Carboxy-, Piperidinomethyl-, 1,2,4,5-Tetrahydro-benzo[*d*]azepin-3-yl-methyl-, 2-Carboxy-1-ethyl-, Phenylaminocarbonyl-, Benzylaminocarbonyl-, C₁₋₃-Alkylsulfonyl-, Aminocarbonyl-, Methoxycarbonyl-, (N-Methyl-N-methylsulfonyl)amino -, Diethylaminomethyl-, 3-Diethylamino-1-propyloxy-, Morpholino-, 4-Methyl-1-piperazinyl-, 2*H*-Tetrazolo-5-yl-, 1*H*-Imidazol-4-yl oder Nitrogruppe oder eine Gruppe der Formel

   -CH₂-NH-CH₂-C₂ₛFₛ₊₁,

   worin s 1 oder 2 bedeutet,
substituierte Phenylgruppe, oder eine in 3-Position des Phenylteils durch ein Chlor- oder Bromatom, eine Cyano-, Aminocarbonyl-, Carboxy-, Ethoxycarbonyl- oder Nitrogruppe substituierte Phenylgruppe, oder eine 3,4-Dichlorphenyl-, 3,5-Dichlorphenyl-, 4-Amino-3,5-dichlorphenyl-, 3-Chlor-4-fluorphenyl-, 4-Chlor-3-methylphenyl-, 4-Chlor-3-trifluormethylphenyl-, 4-Brom-3-chlorphenyl-, 3-Hydroxy-4-methylphenylgruppe, Benzotriazol-5-yl-, Benzimidazol-5-yl, Indazol-5-yl- oder Indazol-6-yl- oder eine Gruppe der Formel 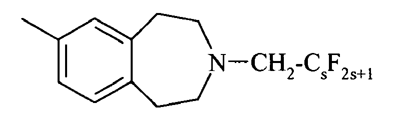 worin s 1 oder 2 bedeutet,
bedeutet.

Die besten Resultate werden mit Verbindungen der Formel I erzielt, worin
die Gruppe **R_{c}**N**R_{d}** ausgewählt ist aus den folgenden Gruppen: 2-Amino-1-ethylamino, 2-Acetylamino-ethylamino, 2-Aminocarbonyl-1-ethylamino, 2-Methoxy-1-ethylamino, 2-Morpholino-1-ethylamino, 3-Aminopropyl-amino, 1-Carboxy-2-propylamino, 4-Aminobutylamino, 5-Hydroxy-1-pentylamino, 3-(3-Aminopropoxy-1-propylamino, 2-(3-Hydroxyphenyl)-1-ethyl-amino, 2-(4-Hydroxy-3-methoxy-phenyl)-2-hydroxy-1-ethylamino, 2-(2-(2-Amino-1-ethyl)-phenyl)-1-ethyl-amino, 4-Hydroxy-cyclohexylamino, 3-Amino-cyclohexylamino, 4-Aminomethyl-cyclohexylmethylamino, 4-Dimethylamino-cyclohexylamino, 1-Methyl-piperidin-4-yl-methylamino, N-(4-Methyl-piperidin-4-yl)-N-methyl-amino, 3-(2-Oxo-pyrrolidin-1-yl)-propyl-1-amino, 1,4,6,7-Tetrahydro-imidazo[4,5-*c*]pyridin-5-yl, 2-Hydroxymethyl-pyrrolidin-1-yl, 4-Aminomethyl-piperidin-1-yl, 3-Hydroxymethyl-piperidin-1-yl, 3-Acetylaminomethyl-piperidin-1-yl, 4-(N-Acetyl-N-methyl-aminomethyl)-piperidin-1-yl, 3-(4-(Pyrrolidin-1-yl)butyl)-piperidin-1-yl, 3-(2-Aza-bicyclo[2.2.1]hept-5-en-2-yl)-propylamino, 7-Methyl-2,7-diaza-spiro[4.4]non-2-yl.

Weiterhin Gegenstand der Erfindung sind die physiologisch verträglichen Salze der Verbindungen der Formel I.

Ein weiterer Gegenstand der Erfindung sind die Verbindungen der Formel I zur Verwendung als Arzneimittel.

Ein weiterer Aspekt der Erfindung ist ein Verfahren zur Herstellung der Verbindungen der Formel I, wobei
a. eine Verbindung der allgemeine Formel 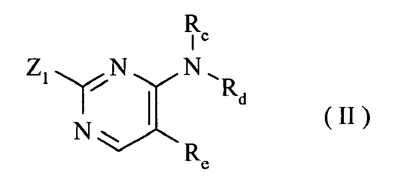 in der
   **R_{c}** bis **Rₑ** wie voranstehend erwähnt definiert sind und
   **Z₁** eine Austrittsgruppe darstellt, mit einem Amin der allgemeinen Formel

   H-(**Rₐ**N**R_{b}**) (III)

   worin
   **Rₐ** und **R_{b}** wie voranstehend erwähnt definiert sind, umgesetzt wird; oder
b. eine Verbindung der allgemeine Formel IV 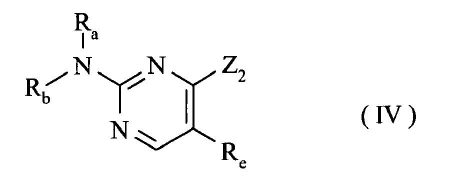 in der
   **Rₐ, R_{b}** und **Rₑ** wie voranstehend erwähnt definiert sind, und
   **Z₂** eine Austrittsgruppe darstellt, mit einem Amin der allgemeinen Formel

   H-(**R_{c}**N**R_{d}**) (V)
worin
**R_{c}** und **R_{d}** wie voranstehend erwähnt definiert sind, umgesetzt wird.

Die Umsetzung wird zweckmäßigerweise in einem Lösungsmittel wie Ethanol, Isopropanol, Butanol, Tetrahydrofuran, Dioxan, Toluol, Chlorbenzol, Dimethylformamid, Dimethylsulfoxid, Ethylenglycolmonomethylether, Ethylenglycoldiethylether oder Sulfolan gegebenenfalls in Gegenwart einer anorganischen Base, z.B. Natriumcarbonat oder Kaliumhydroxid, oder einer tertiären organischen Base, z.B. Triethylamin, N-Ethyl-düsopropylamin oder Pyridin, wobei letztere gleichzeitig auch als Lösungsmittel dienen können, und gegebenenfalls in Gegenwart eines Reaktionsbeschleunigers wie eines Kupfersalzes, eines entsprechenden Amin-hydrohalogenids oder Alkalihalogenids bei Temperaturen zwischen 0 und 250°C, vorzugsweise jedoch bei Temperaturen zwischen 20 und 200°C, durchgeführt. Die Umsetzung kann jedoch auch ohne Lösungsmittel oder in einem Überschuss der eingesetzten Verbindung der Formeln III bzw. V durchgeführt werden.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Ge-mische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und

Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine saure Gruppe wie eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der Formeln II bis V sind teilweise literaturbekannt oder man erhält diese nach an sich literaturbekannten Verfahren.

Wie bereits eingangs erwähnt, weisen die erfindungsgemäßen Verbindungen der Formel I und ihre physiologisch verträglichen Salze wertvolle pharmakologische Eigenschaften auf, insbesondere eine Hemmwirkung auf die Proliferation von Zellen, insbesondere von Endothelzellen.

Weiterhin Gegenstand der Erfindung ist die Verwendung von Verbindungen der Formel I zur Herstellung eines Medikamentes zur Therapie und zur Vorbeugung von Krankheiten, die durch exzessive oder anomale Zellproliferation charakterisiert sind, sowie pharmazeutische Zusammensetzungen, welche durch einen Gehalt einer oder mehrer Verbindungen der Formel I gekennzeichnet sind.

Im Folgenden werden exemplarische Vorgehensweisen zur Herstellung der erfindungsgemäßen Verbindungen detaillierter beschrieben. Die nachfolgenden Synthesebeispiele dienen ausschließlich einer detaillierteren Erläuterung.

### Herstellung der Ausgangsverbindungen:

Die folgenden Ausgangsverbindungen können entsprechend der jeweils angegebenen Literaturstelle hergestellt werden:
Synthese von 2-Chlor-4-thiocyanato-5-nitro-pyrimidin: Takahashi et al., Chem. Pharm. Bull. (1958) 334
Synthese von 2,4-Dichlor-5-trifluormethylsulfanyl-pyrimidin: Haas, A.; Lieb, M.; J. Heterocycl. Chem. (1986) 1079-1084; 2,4-Dichlor-5-methylsulfanyl-pyrimidin:
   a) Ishikawa, Katsutoshi et al.; Preparation and fungicidal activity of halothiocyanopyrimidines. JP 62 053973
   b) Maggiali, C. et al., Farmaco, Ed. Sci. (1988), 43(3), 277-91.
Synthese von 2,4-Dichlor-5-trifluormethyl-pyrimidin: Shen; Lewis; Ruyle; J. Org. Chem. 30 (1965) 835
Synthese von 2,4-Dichlor-5-nitro-pyrimidin: Albert et al.; J.Chem.Soc. (1951) 474
Synthese von 4,5,6,7-tetrahydro-1(3)H-imidazo[4,5-c]pyridin: Dale; Dudley; J. Pharmacol. exper. Therap.; 18; 106; Chem. Zentralbl.; GE; 93; I; 1922; 770. Lit 2: Fraenkel; Zeimer; Biochem.Z.; 110; 1920; 238.
Synthese von(N-(trans-4-hydroxy-cyclohexyl)-N-methyl-amin: US Patent US 2,152,960.

HPLC-MethodenMethode A:Die HPLC/MS-Daten wurden mit einer HP-1100-MSD-Anlage erstellt.

Als mobile Phase wurde eingesetzt:
A: Wasser mit 0,1% Ameisensäure
B: Acetonitril mit 0,1% Ameisensäure

| Zeit in min | %A | %B | Flussrate in ml/min |
|---|---|---|---|
| 0,0 | 95 | 5 | 0,4 |
| 0,5 | 95 | 5 | 0,4 |
| 5,5 | 5 | 95 | 0,4 |
| 8,5 | 5 | 95 | 0,4 |
| 9,5 | 95 | 5 | 0,4 |

Als stationäre Phase diente eine Säule Waters X-Terra^{™} MS C₁₈ 2,5µm, 2,1mm x 50mm (Säulentemperatur: konstant bei 30°C (+-0,5°C))
Die UV-Detektion erfolgte bei zwei Wellenlängen: Signal A bei 230nm (+-2nm), Signal B bei 254nm (+-2nm).
Bereich der massenspektrometrischen Detektion: m/z 100 bis m/z 1000

### Methode B:

ThermoFinnigan LCQ Deca, Surveyor-HPLC
Als mobile Phase wurde eingesetzt:
A: Wasser mit 0,1% Trifluoressigsäure
B: Acetonitril mit 0,1% Trifluoressigsäure

| Zeit in min | %A | %B | Flussrate in ml/min |
|---|---|---|---|
| 0,0 | 95 | 5 | 0,5 |
| 4,5 | 2 | 98 | 0,5 |
| 5,5 | 2 | 98 | 0,5 |
| 5,6 | 95 | 5 | 0,5 |
| 7,0 | 95 | 5 | 0,5 |

Als stationäre Phase diente eine Säule Waters X-Terra^{™} MS C₁₈ 2,5µm, 2,1mm x 50mm (Säulentemperatur: konstant bei 40°C)

Die Diodenarraydetektion erfolgte im Wellenlängenbereich 210-500 nm
Bereich der massenspektrometrischen Detektion: m/z 150 bis m/z 1500

### Methode C:

Analog Methode B mit dem Gradienten:

| Zeit in min | %A | %B | Flussrate in ml/min |
|---|---|---|---|
| 0,0 | 95 | 5 | 0,6 |
| 4,0 | 5 | 95 | 0,6 |
| 6,0 | 5 | 95 | 0,6 |
| 8,0 | 95 | 5 | 0,6 |
| 1 min post run | 95 | 5 | 0,6 |

### Methode D:

Analog Methode B mit dem Gradienten:

| Zeit in min | %A | %B | Flussrate in ml/min |
|---|---|---|---|
| 0,0 | 95 | 5 | 0,4 |
| 5,5 | 5 | 95 | 0,4 |
| 9,5 | 5 | 95 | 0,4 |
| 3min post run | 95 | 5 | 0,4 |

### Methode E:

Analog Methode B mit dem Gradienten:

| Zeit in min | %A | %B | Flussrate in ml/min |
|---|---|---|---|
| 0,0 | 90 | 10 | 0,7 |
| 4,0 | 5 | 95 | 0,7 |
| 4,5 | 5 | 95 | 0,7 |
| 6,0 | 90 | 10 | 0,7 |

### Methode F:

Als mobile Phase wurde eingesetzt:
A: Wasser mit 0.1 % Ameisensäure
B: Acetonitril mit 0.1 % Ameisensäure

| Zeit in min | %A | %B | Flussrate in ml/min |
|---|---|---|---|
| 0,5 | 95 | 5 | 1,5 |
| 5,0 | 0 | 100 | 1,5 |

Als stationäre Phase diente eine Säule Develosil RPAqueous 4.6x50mm Die UV-Detektion erfolgte bei 254 nm

### Methode G:

Analog Methode F mit dem Gradienten:

| Zeit in min | %A | %B | Flussrate in ml/min |
|---|---|---|---|
| 0,5 | 90 | 10 | 1,5 |
| 5,0 | 0 | 100 | 1,5 |

### Methode H:

Analog Methode F mit dem Gradienten:

| Zeit in min | %A | %B | Flussrate in ml/min |
|---|---|---|---|
| 0,5 | 80 | 20 | 1,5 |
| 5,0 | 0 | 100 | 1,5 |

### Methode I:

Analog Methode F mit dem Gradienten:

| Zeit in min | %A | %B | Flussrate in ml/min |
|---|---|---|---|
| 0,5 | 70 | 30 | 1,5 |
| 5,0 | 0 | 100 | 1,5 |

### Methode J:

Analog Methode F mit dem Gradienten:

| Zeit in min | %A | %B | Flussrate in ml/min |
|---|---|---|---|
| 0,5 | 95 | 5 | 1,5 |
| 4,5 | 70 | 30 | 1,5 |
| 5,0 | 0 | 100 | 1,5 |

### Methode K:

Analog Methode F mit dem Gradienten:

| Zeit in min | %A | %B | Flussrate in ml/min |
|---|---|---|---|
| 0,5 | 60 | 40 | 1,5 |
| 5,0 | 0 | 100 | 1,5 |

### Methode L:

Analog Methode F mit dem Gradienten:

| Zeit in min | %A | %B | Flussrate in ml/min |
|---|---|---|---|
| 0,5 | 40 | 60 | 1,5 |
| 5,0 | 0 | 100 | 1,5 |

### Methode M:

Analog Methode F mit dem Gradienten:

| Zeit in min | %A | %B | Flussrate in ml/min |
|---|---|---|---|
| 0,5 | 30 | 70 | 1,5 |
| 5,0 | 0 | 100 | 1,5 |

### Beispiel I

### 2-(3,4-Dichlorphenylamino)-4-thiocyanato-5-nitro-pyrimidin

Zu 3,00 g 2-Chlor-4-thiocyanato-5-nitro-pyrimidin in 40 ml Toluol werden bei Raumtemperatur 2,47 g 3,4-Dichloranilin in 12 ml Ethanol zugegeben. Es wird 16 Stunden nachgerührt, der Feststoff wird abgesaugt, zweimal mit je 30 ml Toluol und dann einmal mit 30 ml Ethanol gewaschen und getrocknet.
Ausbeute: 2.86 g (60 % der Theorie), Smp: 240-242°C

Analog Beispiel I werden folgende Verbindungen erhalten:
(1) 2-(4-Amino-3,5-dichlorphenylamino)-4-thiocyanato-5-nitro-pyrimidin
(2) 2-(4-Aminosulfonyl-phenylamino)-4-thiocyanato-5-nitro-pyrimidin
(3) 2-(4-Chlorphenylamino)-4-thiocyanato-5-nitro-pyrimidin Schmelzpunkt: 224-226°C
(4) 2-(4-Carboxyphenylamino)-4-thiocyanato-5-nitro-pyrimidin
(5) 2-(3-Chlor-4-fluor-phenylamino)-4-thiocyanato-5-nitro-pyrimidin
(6) 2-(3-Aminocarbonyl-phenylamino)-4-thiocyanato-5-nitro-pyrimidin
(7) 2-(4-Phenylaminocarbonyl-phenylamino)-4-thiocyanato-5-nitro-pyrimidinh
(8) 2-(4-Nitro-phenylamino)-4-thiocyanato-5-nitro-pyrimidin
(9) 2-(4-Cyano-phenylamino)-4-thiocyanato-5-nitro-pyrimidin
(10) 2-(3-Brom-phenylamino)-4-thiocyanato-5-nitro-pyrimidin
(11) 2-(4-Brom-phenylamino)-4-thiocyanato-5-nitro-pyrimidin
(12) 2-(3-Nitro-phenylamino)-4-thiocyanato-5-nitro-pyrimidin
(13) 2-(4-(2-Carboxy-1-ethyl)phenylamino)-4-thiocyanato-5-nitro-pyrimidin
(14) 2-(4-Aminocarbonyl-phenylamino)-4-thiocyanato-5-nitro-pyrimidin
(15) 2-(4-Chlor-3-methyl-phenylamino)-4-thiocyanato-5-nitro-pyrimidin
(16) 2-(4-Methoxycarbonyl-phenylamino)-4-thiocyanato-5-nitro-pyrimidin
(17) 2-(3-Cyano-phenylamino)-4-thiocyanato-5-nitro-pyrimidin
(18) 2-(4-Benzylaminocarbonyl-phenylamino)-4-thiocyanato-5-nitro-pyrimidin
(19) 2-(4-Fluor-phenylamino)-4-thiocyanato-5-nitro-pyrimidin
(20) 2-(Benzylamino)-4-thiocyanato-5-nitro-pyrimidin
(21) 2-(2-Chlorbenzylamino)-4-thiocyanato-5-nitro-pyrimidin
(22) 2-(3-Carboxyphenylamino)-4-thiocyanato-5-nitro-pyrimidin
(23) 2-(3-Ethoxycarbonyl-phenylamino)-4-thiocyanato-5-nitro-pyrimidin

### Beispiel II

### 2-Chlor-4-(2-acetylamino-ethylamino)-5-trifluormethyl-pyrimidin

Zu 3,91 g 2,4-Dichlor-5-trifluormethyl-pyrimidin in 20 ml Dioxan werden bei Raumtemperatur 1,84 g 2-Acetylamino-ethylamin in 10 ml Dioxan gelöst zugegeben. Dann gibt man 3,7 ml 5 M Kaliumcarbonat-Lösung hinzu und rührt drei Tage bei Raumtemperatur. Dann wird über Alox B filtriert und mit Dioxan nachgewaschen. Das Filtrat wird eingedampft und der Rückstand durch Chromatographie über eine Kieselgelsäule mit Cyclohexan : Essigsäureethylester : Methanol (5:4:1) getrennt.
Ausbeute: 2.30 g (45 % der Theorie)
Smp: 185°C

Analog Beispiel II werden folgende Verbindungen erhalten:
(1) 2-Chlor-4-(2-acetylamino-ethylamino)-5-nitro-pyrimidinHergestellt aus 2,4-Dichlor-5-nitro-pyrimidin in Gegenwart von 2N Natronlauge.
(2) 2-Chlor-4-(2-acetylamino-ethylamino)-5-methylsulfanyl-pyrimidin Hergestellt aus 2,4-Dichlor-5-methylsulfanyl-pyrimidin.
(3) 2-Chlor-4-(2-acetylamino-ethylamino)-5-trifluormethylsulfanyl-pyrimidin R_{f} = 0.15 (Kieselgel; Methylenchlorid:Methanol = 20:1)
   Hergestellt aus 2,4-Dichlor-5-trifluormethylsulfanyl-pyrimidin.
(4) 2-Chlor-4-(2-acetylamino-ethylamino)-5-brom-pyrimidin
   Hergestellt aus 2,4-Dichlor-5-brom-pyrimidin.
   R_{f} = 0.16 (Kieselgel; Essigsäureethylester:Cyclohexan = 1:1)
(5) 2-Chlor-4-(N-(trans-4-hydroxy-cyclohexyl)-N-methyl-amino)-5-nitro-pyrimidin
(6) 2-Chlor-4-(trans-4-hydroxy-cyclohexylamino)-5-nitro-pyrimidin
(7) 2-Chlor-4-(2-pyridyl-methylamino)-5-nitro-pyrimidin
(8) 2-Chlor-4-(ethoxycarbonyl-methylamino)-5-nitro-pyrimidin
(9) 2-Chlor-4-[N-(2-hydroxyethyl)-N-methyl-amino]-5-nitro-pyrimidin
(10) N-[2-(2-Chloro-5-trimethylsilanylethynyl-pyrimidin-4-ylamino)-ethyl]acetamid
   UVmax (Ethanol) = 215, 265, 314 nm
   1H-NMR(D₆-DMSO, 400MHz) δ: 0.27 (s, 9 H), 1.82 (s, 3 H), 3.26 (m, 2 H), 3.43 (m, 2 H), 7.50 (t, 1 H), 8.08 (t, 1 H), 8.14 (s, 1 H).
(11) 2-Chlor-4-[3-(2-oxo-pyrrolidin-1-yl)-propylamino]-5-brom-pyrimidin
   5.0 g 5-Brom-2,4-Dichlor-pyrimidin werden zusammen mit 3.1 g 1-(3-Aminopropyl)-pyrrolidin-2-on in 50 ml 1,4-Dioxan vorgelegt. Bei Raumtemperatur gibt man 4.39 ml 5 M Kaliumcarbonat-Lösung zu und lässt 1 Stunden rühren. Anschließend wird das Reaktionsgemisch vollständig eingeengt, in Methanol aufgenommen, über Aluminiumoxid filtriert und erneut eingeengt. Der kristalline Rückstand wird in 170 ml Essigsäureethylester aufgenommen, filtriert, eingedampft und aus Essigsäureethylester umkristallisiert. Man erhält 5.45 g (75 %) des gewünschten Produkts.
   Rf (Methylenchlorid/Methanol = 9:1; SiO2) = 0.51; (M+H)+= 333/335/337 (Cl, Br);
   1H-NMR (D6-DMSO, 400 MHz) δ: 1.72 (quint, 2 H), 1.95 (quint, 2 H), 2.22 (t, 2 H), 3.20 (t, 2 H), 3.28 - 3.40 (m, 4 H), 7.71 (t, 1 H), 8.22 (s, 1 H).
(12) 2-Chlor-4-(2-acetylamino-ethylamino)-5-methyl-pyrimidin
   1.0 g 2,4-Dichlor-5-methyl-pyrimidin wird in DMA (0.1 M) vorgelegt und bei 0°C mit einer Lösung von 0.69 g (1.2 Äq.) N-Acetylethylendiamin und 2.0 ml (2 Äq.) Ethyldiisopropylamin in DMA versetzt. Man lässt das Reaktionsgemisch 1 - 2 Stunden bei Raumtemperatur rühren und engt anschließend zur Trockne ein. Nach Zugabe von ges. Natriumhydrogencarbonat-Lösung wird mit Essigsäureethylester extrahiert und die organische Phase anschliessend über Natriumsulfat getrocknet und eingeengt. Zur weiteren Reinigung wird das Rohprodukt an Kieselgel chromatographiert. Man erhält 78% des gewünschten Produkts.
   1H-NMR (D6-DMSO, 300 MHz) δ: 1.80 (s, 3 H), 1.94 (s, 3 H), 3.23 (m, 2 H), 3.36 (m, 2 H), 7.31 (s, 1 H), 7.77 (s, 1 H), 7.97 (s, 1 H).
(13) 2-Chlor-4-(2-acetylamino-ethylamino)-5-chlor-pyrimidin2-Chlor-4-(2-acetylamino-ethylamino)-5-chlor-pyrimidinwird analog zu II (12) aus 2,4,5-Trichlor-pyrimidin in einer Ausbeute von 58 % erhalten.
   HPLC/MS (Methode F): RT = 3.40 min.; [M+H]+ = 249/251; Abs. λ max = 247,5 nm 1H-NMR (D6-DMSO, 300 MHz) δ: 8.15 (s,1H); 8.00-7.92 (m, 2H,*N*-H); 3.40 (q, *J*=5.8 Hz, 2H); 3.24 (q,*J* = 5.8Hz, 2H); 1.79 (s, 3 H).
(14) 2-Chlor-4-(2-acetylamino-ethylamino)-5-methoxy-pyrimidin1.0 g 2,4-Dichlor-5-methoxy-pyrimidin wird rasch zu einer Lösung von 0.69 g (1.2 Äq.) N-Acetylethylendiamin und 1.2 ml (1.25 Äq.) Ethyldiisopropylamin in 20 ml Ethanol gegeben. Man lässt das Reaktionsgemisch 2 - 15 Stunden bei Raumtemperatur rühren und engt anschließend zur Trockne ein. Nach Zugabe von Essigsäureethylester wird mit gesättigter Natriumhydrogencarbonat-Lösung und gesättigter Natriumchlorid-Lösung extrahiert und die organische Phase anschliessend über Natriumsulfat getrocknet. Zur weiteren Reinigung wird das Rohprodukt an Kieselgel chromatographiert. Man erhält 793 mg (58 %) des gewünschten Produkts.
   1H-NMR (D6-DMSO, 300 MHz) δ: 1.79 (s, 3 H), 3.21 (q, 2 H), 3.34 (q, 2 H), 3.83 (s, 3 H), 7.53 (t, 1 H), 7.67 (s, 1 H), 7.96 (t, 1 H).
(15) 2-Chlor-4-(1,4,6,7-tetrahydro-imidazo[4,5-c]pyridin-5-yl)-5-chloro-pyrimidin2-Chlor-4-(1,4,6,7-tetrahydro-imidazo[4,5-*c*]pyridin-5-yl)-5-chloro-pyrimidin wird analog zu II (12) aus 2,4,5-Trichlor-pyrimidin in einer Ausbeute von 20 % erhalten.
   HPLC/MS (Methode F): RT = 3.35 min.; [M+H]+ = 270/272; Abs. λ max = 211,4 nm
   1H-NMR (D6-DMSO, 300 MHz) δ: 12.0 (bs, 1 H); 8.30 (s,1 H); 7.49 (s, 1 H); 4. 59 (m, 2H); 3.94 (m, 2 H); 2.79 (m, 2 H).
(16) 2-Chlor-4-(2-acetylamino-ethylamino)-5-methylsulfonyl-pyrimidin2-Chlor-4-(2-acetylamino-ethylamino)-5-methylsulfonyl-pyrimidin wird analog zu II (14) aus 2,4-Dichlor-5-methylsulfonyl-pyrimidin in einer Ausbeute von 50 % erhalten.
   1H-NMR (D6-DMSO, 300 MHz) δ: 1.79 (s, 3 H), 3.16 - 3.29 (m, 5 H), 3.53 (q, 2 H), 7.84 (t, 1 H), 7.99 (t, 1 H), 8.41 (s, 1 H).
(17) 2-Chlor-4-(2-acetylamino-ethylamino)-5-dimethylamino-pyrimidin2-Chlor-4-(2-acetylamino-ethylamino)-5-dimethylamino-pyrimidin wird analog zu II (14) aus 2,4-Dichlor-5-dimethylamino-pyrimidin in einer Ausbeute von 49 % erhalten.
   1H-NMR (D6-DMSO, 300 MHz) δ: 1.80 (s, 3 H), 2.57 (s, 6 H), 3.24 (q, 2 H), 3.38 (q, 2 H), 7.30 (t, 1 H), 7.71 (s, 1 H), 7.97 (t, 1 H).
(18) 2-Chlor-4-(2-acetylamino-ethylamino)-5-isopropoxy-pyrimidin2-Chlor-4-(2-acetylamino-ethylamino)-5-isopropoxy-pyrimidin wird analog zu II (14) aus 2,4-Dichlor-5-isopropoxy-pyrimidin in einer Ausbeute von 66 % erhalten.
   1H-NMR (D6-DMSO, 300 MHz) δ: 1.28 (d, 6 H), 1.80 (s, 3 H), 3.22 (q, 2 H), 3.35 (q, 2 H), 4.58 (sept, 1 H), 7.35 (t, 1 H), 7.69 (s, 1 H), 7.98 (t, 1 H).
(19) 2-Chlor-4-(2-acetylamino-ethylamino)-5-isopropyl-pyrimidin2-Chlor-4-(2-acetylamino-ethylamino)-5-isopropyl-pyrimidin wird analog zu II (14) aus 2,4-Dichlor-5-isopropyl-pyrimidin in einer Ausbeute von 70 % erhalten.
   1H-NMR (D6-DMSO, 300 MHz) δ: 1.15 (d, 6 H), 1.81 (s, 3 H), 2.82 (sept, 1 H), 3.23 (q, 2 H), 3.39 (q, 3 H), 7.41 (t, 1 H), 7.84 (s, 1 H), 7.99 (t, 1 H).
(20) 2-Chlor-4-[3-(2-oxo-pyrrolidin-1-yl)-propylamino]-5-chlor-pyrimidin2-Chlor-4-[3-(2-oxo-pyrrolidin-1-yl)-propylamino]-5-chlor-pyrimidin wird analog zu II (12) aus 2,4,5-Trichlor-pyrimidin in einer Ausbeute von 68 % erhalten.
   HPLC/MS (Methode F): RT = 3.96 min.; [M+H]+ = 289/291; Abs. λ max = 249,4 nm
   1H-NMR (D6-DMSO, 300 MHz) δ: 8.13 (s, 1 H); 7.88 (bs, 1 H,N-H), 3.31 (m, 4 H); 3.16 (m, 2 H); 2.22 (t, J=7.9 Hz, 2 H); 1.93 (m, 2 H); 1.72 (m, 2 H).
(21) 2-Chlor-4-[3-(2-oxo-pyrrolidin-1-yl)-propylamino]-5-methoxy-pyrimidin1.0 g 2,4-Dichlor-5-methoxy-pyrimidin wird rasch zu einer Lösung von 0.95 g (1.2 Äq.) 1-(3-Aminopropyl)-pyrolidin-2-on und 1.2 ml (1.25 Äq.) Ethyldiisopropylamin in 20 ml Ethanol gegeben. Man lässt das Reaktionsgemisch 15 Stunden bei Raumtemperatur rühren und engt anschließend zur Trockne ein. Nach Zugabe von Essigsäureethylester wird mit gesättigter Natriumhydrogencarbonat-Lösung und gesättigter Natriumchlorid-Lösung extrahiert und die organische Phase anschliessend über Natriumsulfat getrocknet. Zur weiteren Reinigung wird das Rohprodukt an Kieselgel chromatographiert. Man erhält 1.15 g (72 %) des gewünschten Produkts.
   1H-NMR (D6-DMSO, 300 MHz) δ: 1.70 (m, 2 H), 1,93 (m, 2 H), 2.22 (t, 2 H), 3.38-3.17 (m, 6 H), 3.84 (s, 3 H), 7.50 (m, 1 H), 7.65 (s, 1 H).
(22) 2-Chlor-4-[3-(2-oxo-pyrrolidin-1-yl)-propylamino]-5-methyl-pyrimidin 2-Chlor-4-[3-(2-oxo-pyrrolidin-1-yl)-propylamino]-5-methyl-pyrimidin wird analog zu II (12) aus 2,4-Dichlor-5-methyl-pyrimidin in einer Ausbeute von 46 % erhalten.
   HPLC/MS (Methode F): RT = 3.15 min.; [M+H]+ = 269/271
   1H-NMR (D6-DMSO, 300 MHz) δ: 1.72 (m, 2 H), 1,93 (m, 2 H), 1.96 (s, 3 H), 2.22 (t, 2 H), 3.22 (t, 2 H), 3.26 - 3.38 (m, 4 H), 7.23 (s, 1 H), 7.77 (s, 1 H).
(23) 2-Chlor-4-[3-(2-oxo-pyrrolidin-1-yl)-propylamino]-5-methylsulfonyl-pyrimidin2-Chlor-4-[3-(2-oxo-pyrrolidin-1-yl)-propylamino]-5-methylsulfonyl-pyrimidin wird analog zu II (21) aus 2,4-Dichlor-5-methylsulfonyl-pyrimidin in einer Ausbeute von 34 % erhalten.
   1H-NMR (D6-DMSO, 300 MHz) δ: 1.76 (quint, 2 H), 1,94 (quint, 2 H), 2.32 (t, 2 H), 3.23 (t, 2 H), 3.26 - 3.49 (m, 7 H), 7.84 (t, 1 H), 8.39 (s, 1 H).
(24) 2-Chlor-4-[3-(2-oxo-pyrrolidin-1-yl)-propylamino]-5-dimethylamino-pyrimidin2-Chlor-4-[3-(2-oxo-pyrrolidin-1-yl)-propylamino]-5-dimethylamino-pyrimidin wird analog zu II (21) aus 2,4-Dichlor-5-dimethylamino-pyrimidin in einer Ausbeute von 59 % erhalten.
   1H-NMR (D6-DMSO, 300 MHz) δ: 1.71 (quint, 2 H), 1.93 (quint, 2 H), 2.22 (t, 2 H), 2.58 (s, 6 H), 3.19 - 3.38 (m, 6 H), 7.30 (t, 1 H), 7.70 (s, 1 H).
(25) 2-Chlor-4-[3-(2-oxo-pyrrolidin-1-yl)-propylamino]-5-isopropoxy-pyrimidin2-Chlor-4-[3-(2-oxo-pyrrolidin-1-yl)-propylamino]-5-isopropoxy-pyrimidin wird analog zu II (21) aus 2,4-Dichlor-5-isopropoxy-pyrimidin in einer Ausbeute von 83 % erhalten.
   1H-NMR (D6-DMSO, 300 MHz) δ: 1.28 (d, 6 H), 1.69 (quint, 2 H), 1.93 (quint, 2 H), 2.23 (t, 2 H), 3.18 - 3.38 (m, 6 H), 4.86 (sept, 1 H), 7.29 (t, 1 H), 7.68 (s, 1 H).
(26) 2-Chlor-4-[3-(2-oxo-pyrrolidin-1-yl)-propylamino]-5-isopropyl-pyrimidin2-Chlor-4-[3-(2-oxo-pyrrolidin-1-yl)-propylamino]-5-isopropyl-pyrimidin wird analog zu II (21) aus 2,4-Dichlor-5-isopropyl-pyrimidin in einer Ausbeute von 54 % erhalten.
   1H-NMR (D6-DMSO, 300 MHz) δ:1.16 (d, 6 H), 1.71 (quint, 2 H), 1.94 (quint, 2 H), 2.24 (t, 2 H), 2.84 (sept, 1 H), 3.22 (t, 2 H), 3.26 - 3.34 (m, 4 H), 7.34 (t, 1 H), 7.83 (s, 1 H).
(27) 2-Chlor-4-(4,5,7,8-tetrahydro-1H-imidazo[4,5-d]azepin-6-yl)-5-chloro-pyrimidin 1.0 g 2,4,5-Trichlor-pyrimidin in Isopropanol (0.1 M) wird bei 0°C mit einer Lösung von 0.69 g (1.2 Äq.) 4,5,7,8-Tetrahydro-1H-imidazo[4,5-d]azepin und 2 ml (2 Äq.) Ethyldiisopropylamin in Isopropanol gegeben. Man lässt das Reaktionsgemisch 1-2 Stunden bei Raumtemperatur rühren und engt anschließend zur Trockne ein. Nach Zugabe von ges. Natriumhydrogencarbonat-Lösung wird mit Dichlormethan und Essigsäureethylester extrahiert und die organische Phase anschliessend über Natriumsulfat getrocknet und eingeengt. Zur weiteren Reinigung wird das Rohprodukt an Kieselgel chromatographiert. Man erhält 95% des gewünschten Produkts.
   HPLC/MS (Methode F): RT = 3.32 min.; [M+H]+ = 284/286; Abs. λ max = 260,8 nm
   1H-NMR (D6-DMSO, 300 MHz) δ: 8.18 (s, 1 H); 7.37 (s, 1 H); 4.11-4.08 (m, 4 H); 2.89-2.86 (m, 4 H).
(28) 2-Chlor-4-(4,5,7,8-tetrahydro-1H-imidazo[4,5-d]azepin-6-yl)-5-methyl-pyrimidin 2-Chlor-4-(4,5,7,8-tetrahydro-1H-imidazo[4,5-d]azepin-6-yl)-5-methyl-pyrimidin wird analog zu II (27) aus 2,4-Dichlor-5-methyl-pyrimidin in einer Ausbeute von 36 % erhalten.
   HPLC/MS (Methode F): RT = 3.22 min.; [M+H]+ = 264/266
   1H-NMR (D6-DMSO, 300 MHz) δ: 2.30 (s, 3 H), 2.83 (m, 4 H), 3.93 (m, 4 H), 7.39 (s, 1 H), 7.87 (s, 1 H).
(29) 2-Chlor-4-(1,4,6,7-tetrahydro-imidazo[4,5-*c*]pyridin-5-yl)-5-methyl-pyrimidin 2-Chlor-4-(1,4,6,7-tetrahydro-imidazo[4,5-*c*]pyridin-5-yl)-5-methyl-pyrimidin wird analog zu II (12) aus 2,4-Dichlor-5-methyl-pyrimidin in einer Ausbeute von 26 % erhalten.
   HPLC/MS (Methode F): RT = 3.19 min.; [M+H]+ = 250/252
   1H-NMR (D6-DMSO, 300 MHz) δ: 2.24 (s, 3 H), 2.74 (m, 2 H), 3.74 (m, 2 H), 4.46 (m, 2 H), 7.48 (s, 1 H), 8.01 (s, 1 H), 11.82 (s, 1 H).
(30) 2-Chlor-4-(1,4,6,7-tetrahydro-imidazo[4,5-*c*]pyridin-5-yl)-5-methoxy-pyrimidin 2-Chlor-4-(1,4,6,7-tetrahydro-imidazo[4,5-*c*]pyridin-5-yl)-5-methoxy-pyrimidin wird analog zu II (21) aus 2,4-Dichlor-5-methoxy-pyrimidin in einer Ausbeute von 88 % erhalten.
   1H-NMR (D6-DMSO, 300 MHz) δ: 2.71 (m, 2 H), 3.87 (s, 3 H), 3.97 (m, 2 H), 4.67 (m, 2 H), 7.49 (s, 1 H), 7.91 (s, 1 H), 11.86 (s, 1 H).
(31) 2-Chlor-4-(4,5,7,8-tetrahydro-1H-imidazo[4,5-d]azepin-6-yl)-5-bromo-pyrimidin 2-Chlor-4-(4,5,7,8-tetrahydro-1H-imidazo[4,5-d]azepin-6-yl)-5-bromo-pyrimidin wird analog zu II (27) aus 2,4-Dichlor-5-brom-pyrimidin in einer Ausbeute von 98 % erhalten.
   HPLC/MS (Methode F): RT = 3.66 min.; [M+H]+ = 328/330/332
   1H-NMR (D6-DMSO, 300 MHz) δ: 2.89 (m, 4 H), 4.12 (m, 4 H), 7.36 (d, 1 H), 8.30 (s, 1 H), 11.61 (s, 1 H).
(32) 2-Chlor-4-(1,4,6,7-tetrahydro-imidazo[4,5-*c*]pyridin-5-yl)-5-bromo-pyrimidin 2-Chlor-4-(1,4,6,7-tetrahydro-imidazo[4,5-*c*]pyridin-5-yl)-5-bromo-pyrimidin wird analog zu II (12) aus 2,4-Dichlor-5-brom-pyrimidin in einer Ausbeute von 32 % erhalten.
   HPLC/MS (Methode F): RT = 3.47 min
   1H-NMR (D6-DMSO, 300 MHz) δ: 2.80 (m, 2 H), 3.94 (m, 2 H), 4.58 (m, 2 H), 7.51 (s, 1 H), 8.42 (s, 1 H), 11.89 (s, 1 H).
(33) 2-Chlor-4-(1,4,6,7-tetrahydro-imidazo[4,5-*c*]pyridin-5-yl)-5-dimethylamino-pyrimidin2-Chlor-4-(1,4,6,7-tetrahydro-imidazo[4,5-*c*]pyridin-5-yl)-5-dimethylamino-pyrimidin wird analog zu II (21) aus 2,4-Dichlor-5-dimethylamino-pyrimidin in einer Ausbeute von 43 % erhalten.
   1H-NMR (D6-DMSO, 300 MHz) δ: 2.64 (s, 6 H), 2.76 (m, 2 H), 3,97 (t, 2 H), 4.69 (s, 2 H), 7.48 (s, 1 H), 7.86 (s, 1 H), 11.85 (s, 1 H).
(34) 2-Chlor-4-(1,4,6,7-tetrahydro-imidazo[4,5-*c*]pyridin-5-yl)-5-isopropyl-pyrimidin 2-Chlor-4-(1,4,6,7-tetrahydro-imidazo[4,5-*c*]pyridin-5-yl)-5-isopropyl-pyrimidin wird analog zu II (21) aus 2,4-Dichlor-5-isopropyl-pyrimidin in einer Ausbeute von 50 % erhalten.
   1H-NMR (D6-DMSO, 300 MHz) δ: 1.26 (d, 6 H), 2.79 (m, 2 H), 3.00 (sept., 1 H), 3.66 (m, 2 H), 4.35 (m, 2 H), 7.50 (s, 1 H), 8.30 (s, 1 H), 11.88 (s, 1 H).

### Beispiel III

### 2-(3,4-Dichlorphenylamino)-4-chlor-5-trifluormethyl-pyrimidin

Zu 6,51 g 2,4-Dichlor-5-trifluormethyl-pyrimidin in 40 ml Dioxan werden bei Raumtemperatur 4,86 g 3,4-Dichloranilin in 10 ml Dioxan gelöst zugegeben. Dann gibt man 6 ml 5 M Kaliumcarbonat-Lösung hinzu und rührt drei Tage bei Raumtemperatur. Dann wird über Alox B (20 ml) filtriert und mit Dioxan nachgewaschen. Das Filtrat wird eingedampft, der Rückstand in 50 ml Methylenchlorid gelöst und diese Lösung in einem Trockeneis-Aceton-Bad gekühlt. Der Niederschlag wird abgesaugt und das Filtrat erneut gekühlt. Nach erneutem Absaugen werden die Niederschläge vereinigt und das Filtrat eingedampft. Der Rückstand wird durch Chromatographie über eine RP18-Säule (Gradient: Acetonitril: H2O = 20:80 bis 80:20) getrennt.
Die Niederschläge und das durch Chromatographie des Filtrats erhaltene Produkt werden vereinigt. Ausbeute: 3,90 g.
1H-NMR (D6-DMSO, 300 MHz) δ: 7.57 (d, 1 H), 7.64 (dd, 1 H), 8.06 (d, 1 H), 8.86 (s, 1 H), 10.90 (s, 1 H).

Analog Beispiel III werden folgende Verbindungen erhalten:
(1) 2-(3-Chlorphenylamino)-4-chlor-5-trifluormethyl-pyrimidin
(2) 2-(Phenylamino)-4-chlor-5-trifluormethyl-pyrimidin
(3) 2-(4-Chlorphenylamino)-4-chlor-5-trifluormethyl-pyrimidin R_{f} = 0.88 (Kieselgel; Methylenchlorid)
(4) 2-(4-Bromphenylamino)-4-chlor-5-trifluormethyl-pyrimidin
(5) 2-(3-Bromphenylamino)-4-chlor-5-trifluormethyl-pyrimidin
(6) 2-(4-Carboxyphenylamino)-4-chlor-5-trifluormethyl-pyrimidin
(7) 2-(4-(2-Carboxy-1-ethyl-)phenylamino)-4-chlor-5-trifluormethyl-pyrimidin
(8) 2-(2-Naphthylamino)-4-chlor-5-trifluormethyl-pyrimidin
(9) 2-(3,5-Dichlorphenylamino)-4-chlor-5-trifluormethyl-pyrimidin
(10) 2-(4-(1-Piperidinyl-methyl-)phenylamino)-4-chlor-5-trifluormethyl-pyrimidinHergestellt unter Verwendung von 4-(Piperidin-1-yl-)-methyl-anilin. Die Chromatographie erfolgte über Kieselgel.
(11) 2-(3,4-Dichlorphenylamino)-4-chlor-5-cyano-pyrimidin Hergestellt unter Verwendung von 2,4-Dichlor-5-cyano-pyrimidin.
(12) 2-[4-(1,2,4,5-tetrahydro-benzo[*d*]azepin-3-yl-methyl)-phenylamino]-4-chlor-5-trifluormethyl-pyrimidin
(13) (6) 2-(4-Aminocarbonylphenylamino)-4-chlor-5-trifluormethyl-pyrimidin

### Beispiel IV

### 4-(3,5-Dimethyl-piperazin-1-sulfonyl)-phenylamin

3 g 4-Nitrobenzolsulfonylchlorid werden in 100ml Dioxan gelöst und hierzu 1,56 g 2,6-Dimethylpiperazin und 2,8 ml einer 5 M wässrigen Kaliumcarbonat Lösung zupipettiert. Der Ansatz wird bei Raumtemperatur über 3 Stunden gerührt. Der entstehende Niederschlag wird abfiltriert, mit frischem Dioxan nachgespült und die vereinte organische Lösung eingeengt.
Ausbeute 3,3 g
Rf Wert 0,26 (Kieselgel, Dichlormethan: Methanol= 95:5)
Das Zwischenprodukt 4-(3,5-Dimethyl-piperazin-1-sulfonyl)-nitrobenzol wird in 25 ml Ethanol aufgenommen und mit 0,3 g Palladium auf Kohle (10%) versetzt. Bei Raumtemperatur und 5 bar Wasserstoff wird der Ansatz bis zum vollständigen Umsatz hydriert. Nach dem Abfiltrieren des Katalysators und dem Abziehen des Lösungsmittels erhält man das Produkt als gelbliche Feststoff. Ausbeute: 3,0 g
Rf Wert 0,19 (Kieselgel, Dichlormethan: Methanol= 95:5)

### Beispiel V

### 4-(4-tert-Butyl-oxycarbonyl-homo-piperazin-1-sulfonyl)-phenylamin

2,8 g 4-Nitrobenzolsulfonylchlorid werden in 90ml Dichlormethan vorgelegt und hierzu 5 g N-(tert.-Butyl-oxycarbonyl)-homopiperazin und 5,1 ml Triethylamin zugetropft. Der Ansatz wird bei Raumtemperatur über 1,5 Stunden gerührt. Die organische Lösung wird mit 1 M wässrigen Natriumacetat und Wasser gewaschen und anschließend eingeengt. Ausbeute 4,8 g
Rf Wert 0,61 (Kieselgel, Dichlormethan: Methanol= 95:5)
Das Zwischenprodukt wird in 25 ml Methanol und 10 ml Ethanol aufgenommen und mit 0,5 g Palladium auf Kohle (10%) versetzt. Bei Raumtemperatur und 5 bar Wasserstoff wird der Ansatz bis zum vollständigen Umsatz hydriert. Nach dem Abfiltrieren des Katalysators und dem Abziehen des Lösungsmittels erhält man das Produkt als gelbliche Feststoff.
Ausbeute: 3,9 g
Rf Wert 0,41 (Kieselgel, Dichlormethan: Methanol= 95:5)

### Beispiel VI

### 1-[2-(Methylamino)-ethyl]-pyrrolidin-2-on

48 ml N-Methyl-ethylendiamin und 42 ml Butyrolacton werden zusammen in einem Autoklaven 7 Stunden auf 250 °C erhitzt. Aus dem erhaltenen braunen Öl destilliert bei 150-155 °C und 0,01 Torr das Produkt. Ausbeute 8,8 g
Rf Wert 0,29 (Kieselgel, Essigsäureethylester: Methanol= 1:1)

### Beispiel VII

### 4-(N-Methyl-N-methylsulfonyl-amino)-phenylamin

4,3 g von 4-(N-Methylsulfonylamino)-nitrobenzol werden in 40 ml DMSO gelöst und mit 2,5 g Kalium-tert-butylat für 1 Stunden bei RT gerührt. Zu der Lösung tropft man 4,2 g Methyliodid in 10 ml DMSO zu und rührt über Nacht bei Raumtemperatur. Der Ansatz wird dann auf ca. 120 ml Eiswasser gegossen und mit Essigsäureethylester extrahiert. Die organische Phase wird 3 mal mit Wasser gewaschen, dann über Natruimsulfat getrocknet und eingedampft. Der Rückstand wird mit Diethylether verrieben, abgesaugt und getrocknet. Ausbeute: 4,6 g
Schmelzpunkt 105-106°C.
Rf Wert 0,52 (Kieselgel, Toluol: Essigsäureethylester 7:3)

4,1 g 4-(N-Methyl-N-methylsulfonyl-amino)-nitrobenzol werden in 80 ml Methanol aufgenommen, mit 1 g Palladiumkohle versetzt und bei Raumtemperatur unter 5 bar hydriert. Nach 30 Minuten filtriert man den Katalysator ab, engt den Ansatz ein und verreibt das zurückbleibende Produkt mit Diethylether. Ausbeute: 3,6 g
Schmelzpunkt 116°C.
Rf Wert 0,14 (Kieselgel, Toluol: Essigsäureethylester= 7:3)

### Beispiel VIII

### 2-Methyl-4,5,7,8-tetrahydro-1H-imidazo[4,5-d]azepin

3 Äquivalente (4.463 g) Natriummethanolat werden bei Raumtemperatur in MeOH vorgelegt, hierzu 7,8 g Acetamidin-hydrochlorid zugeben und für 30 min gerührt. Anschließend werden 10 g N-Benzyl-5-bromohexahydro-4-azepinon zugeben. Nach weiteren 30 Minuten werden zu dem Ansatz zusätzlich 2 Äq. (2.976 g) Natriummethanolat gegeben und für 11 Stunden unter Rückfluss erhitzt. Nach dem Abkühlen auf Raumtemperatur wird im Vakuum vollständig eingeengt, das zurückbleibende Material mit ca. 140 ml Isopropanol verrieben, abfiltrieren und das Filtrat wiederum eingeengt. Das Rohprodukt nimmt man in 50 ml 1N K₂CO₃-Lösung auf und extrahiert 3x mit je 40 ml CH₂Cl₂. Die organischen Phasen trocknet man mit MgSO₄, filtriert ab und engt zur Trockne ein. Der Rückstand wird über Kieselgel (CH₂Cl₂ / MeOH = 87:13 bis 70:30) chromatographiert. Ausbeute: 1,7 g
MS (M+H)+ = 242
1H-NMR (D6-DMSO; 400 MHz) δ: 2.13 (s, 3 H), 2.58 (t, 4 H), 2.79 (t, 4 H), 3.72 (s, 2 H), 7.20 - 7.40 (m, 5 H), 11.11 (br s, 1 H).

Das N-benzylierte 2-Methyl-4,5,7,8-tetrahydro-1H-imidazo[4,5-d]azepin wird in 55 ml Ethanol gelöst und mit 500 mg Palladium auf Kohle (5%) versetzt. In einem Schüttelautoklaven wird bei Raumtemperatur und 50 psi Druck über 48 Stunden hydriert. Der Katalysator wird anschließend abfiltriert und der Ansatz im Vakuum eingeengt. Ausbeute: 900mg
MS (M+H)+ = 152

### Beispiel IX

### 3-Pyrrolidin-1-ylmethyl-piperidin

Das Edukt 3-(1-Pyrrolidinylmethyl)-pyridine (24.6 g) wird in 250 ml Eisessig aufgenommen und an 2 g PtO₂ unter 3 bar H₂ bei Raumtemperatur hydriert. Die abfiltrierte Lösung wird eingeengt, mit Eis versetzt und unter Kühlung mit festem KOH alkalisch eingestellt. Nach dreifacher Extraktion mit 250 ml Diethylether wird das Rohprodukt mit MgSO₄ getrocknet. Nach dem Abfiltirieren des MgSO₄ wird das Lösungsmittel abgezogen und das Amin im Wasserstrahlvakuum bei einem Siedepunkt von 123°C destilliert.

| | |
|---|---|
| Ausbeute: 17.9 g | |
| Schmelzpunkt: 47 °C | |
| CHN-Analyse berechnet: 71.37%/11.98%/16.65% | |
| gefunden: 71.00%/12.04%/16.01% | |

### Beispiel X

### N,N-Dimethyl-2-[methyl-(2-piperidin-4-yl-ethyl)-amino]-acetamid

4-[2-(Methylamino)ethyl]pyridin (34.4 g) und 29.18 g Chloressigsäure-dimethylamid werden in 200 ml Methanol gelöst. Nach Zugabe von 20 g Natriumbicarbonat wird der Ansatz unter Rühren für 5 Stunden unter Rückfluss gekocht. Zur Aufarbeitung wird mit 1000 ml Tetrahydrofuran versetzt, von den ausgefallenen Salzen dekantiert und die Lösung über Aktivkohle filtriert. Nach dem Abziehen des Lösungsmittels erhält man *N,N*-Dimethyl-2-[methyl-(2-pyridin-4-yl-ethyl)-amino]-acetamid als Rohprodukt.
Von dem rohen *N,N*-Dimethyl-2-[methyl-(2-pyridin-4-yl-ethyl)-amino]-acetamid werden 44g in 500 ml Eisessig gelöst und mit 4 g PtO2 bei Raumtemperatur unter 3 bar H2 hydriert. Nach dem Absaugen des Katalysators engt man das Filtrat ein. Der Rückstand wird unter Eiskühlung mit 50%iger Kalilauge alkalisch gestellt und das anfallende Produkt in Ether aufgenommen. Die Etherlösung wird über Natriumsulfat getrocknet, abfiltriert und die Lösung eingeengt. Das Produkt bleibt als gelbes Öl zurück. Ausbeute: 42g

### Beispiel XI

### 2-Methyl-2,8-diaza-spiro[5.5]undecan-5-ol

Zu 75 ml Triton B und 277 g 1-Methyl-4-oxo-piperidin-3-carbonsäure-ethylester in 1500 ml Dioxan werden 87 g Acrylnitril unter Rühren bei Raumtemperatur zugetropft, wobei die Temperatur des Ansatzes bis auf 48°C ansteigt. Die Lösung wird für weitere 4 Stunden bei Raumtemperatur weitergerührt bevor man das Lösungsmittel im Vakuum abdestilliert. Der Rückstand wird mit Diethylether versetzt und zweimal mit gesättigter Kochsalz-Lösung ausgeschüttelt. Nach dem Trocknen der organischen Phase wird das Lösungsmittel abdestilliert. Das Produkt wird im Hochvakuum destilliert. Ausbeute: 220.5 g.

Im Autoklaven werden 90 g 3-(2-Cyano-ethyl)-1-methyl-4-oxo-piperidin-3-carbonsäure-ethylester in 1800 ml methanolischem Ammoniak und 15 g Raney Nickel hydriert (3 bar). Nachdem der Ansatz keinen Wasserstoff mehr aufnimmt saugt man den Katalysator ab und engt das Filtrat im Vakuum ein. Das Produkt erhält man nach Chomatographie über Kieselgel.
Ausbeute: 42.6 g

Von dem erhaltenen 11-Hydroxy-8-methyl-2,8-diaza-spiro[5.5]undecan-1-on werden 2 g in 20 ml absolutem Tetrahydrofuran aufgenommen; diese Lösung tropft man unter Rührung zu 1 g LiAlH4 in 50 ml absolutem Tetrahydrofuran. Unter starker Wasserstoffentwicklung steigt die Temperatur auf 36 °C. Nach weiteren 3 Stunden bei Raumtemperatur wird 10 Stunden unter Rückfluss erhitzt. Die Reaktion bricht man unter Eiskühlung durch Zugabe von 2 ml Wasser und 2 ml 1N wässriger Natronlauge ab. Das Produkt wird mit Essigsäureethylester extrahiert und das Lösungsmittel im Vakuum abgezogen. Anschließend wird das Amin aus absolutem Ethanol mit HCl Gas als Hydrochlorid gefällt. Ausbeute 1.44 g.
Analytik: Schmelzpunkt 293-295 °C Zersetzung

### Beispiel XII

### 2,4-Dichloro-5-trimethylsilanylethynyl-pyrimidin

66,55 g 2,4-Dichlor-5-iod-pyrimidin wird in 1,2 1 absolutem THF mit 70 ml Triethylamin, 4,9 g Palladiumchlorid, 13,3 g Triphenylphosphin, 4g Kupferiodid und 39,3 ml Trimethylsilylacetylen bei 40 °C über drei Stunden gerührt. Nach Abschluß der Reaktion wird das Lösungsmittel im Vakuum abgezogen und das zurückbleibende dunkelrote Öl einer fraktionierten Destillation unterworfen. Bei 0,01 Torr und 100°C destilliert das Produkt über.
1H-NMR (D6-DMSO, 400 MHz) δ: 0.20 (s, 9 H), 8,90 (s, 1 H)

### Beispiel XIII

### 2,4-Dichlor-5- methylsulfonyl -pyrimidin

Die Verbindung wird in einer 3-stufigen Synthese ausgehend von 5-Bromuracil hergestellt.

### 5-Thiomethyl-uracil

126 g 5-Bromuracil werden in 1.0 1 einer 21%igen wässrigen Natriumthiomethanolat-Lösung 5 Stunden unter Rückfluss erhitzt. Nach beendeter Reaktion wird auf 10 °C abgekühlt und mit 330 ml konz. Salzsäure langsam auf pH 7 eingestellt (Temperatur sollte 30 °C nicht übersteigen). Man lässt das Reaktionsgemisch über Nacht bei Raumtemperatur stehen, filtriert den Niederschlag ab, wäscht mit 300 ml kaltem Wasser nach und trocknet den Feststoff (100 g; 96 %) im Trockenschrank bei 80 °C.
Rf= 0.29 (Kieselgel; n-Butanol)

### 2,4-Dichlor-5-thiomethyl-pyrimidin

100 g 5-Thiomethyluracil werden in 580 ml Phosphoroxychlorid vorgelegt. Bei Raumtemperatur gibt man 80 ml Dimethylanilin zu und erhitzt das Reaktionsgemisch 3 Stunden unter Rückfluss. Anschließend destilliert man das überschüssige Phosphoroxychlorid ab, gießt den Rückstand auf 500 ml Eiswasser und extrahiert die wässrige Phase dreimal mit je 400 ml Diethylether. Die EtherExtrakte werden viermal mit je 75 ml Wasser gewaschen, über Natriumsulfat getrocknet und eingeengt. Zurück bleibt ein Feststoff, der zweimal aus Cyclohexan umkristallisiert wird (20.0 g; 16 %).

### 2,4-Dichlor-5-methylsulfonyl-pyrimidin

Zu 20.0 g 2,4-Dichlor-5-thiomethyl-pyrimidin in 250 ml Methylenchlorid wird bei -5 °C innerhalb 1 Stunden eine Lösung aus 51,2 g 3-Chlorperbenzoesäure (98 %) in 450 ml Methylenchlorid zugetropft. Man lässt das Reaktionsgemisch auf Raumtemperatur auftauen und 24 Stunden rühren. Anschließend filtriert man den Niederschlag ab, wäscht das Filtrat nacheinander mit 50 ml gesättigter Natriumsulfit-Lösung, 50 ml gesättigter Natriumhydrogencarbonat-Lösung und 50 ml Wasser. Die organische Phase wird über Natriumsulfat getrocknet und eingeengt, wobei das Produkt als kristalliner Niederschlag anfällt (18.,5 g; 80 %). Weitere Reinigungsschritte sind nicht nötig.
Rf = 0.49 (Kieselgen; Cyclohexan/Essigsäureethylester = 1:1)
GC/MS: (M+H)⁺ = 226/228 (2Cl);
1H-NMR (D6-DMSO, 400 MHz) δ: 3.42 (s, 3 H), 9.18 (s, 1 H).

### Beispiel XIV

### 2,4-Dichlor-5- isopropoxy -pyrimidin.

### Die Verbindung wird ausgehend von Chloressigsäure und Thioharnstoff hergestellt. Isopropoxyessigsäuremethylester

116.5 g des Natriumsalzes der Chloressigsäure werden bei 80 °C zu einer Natriumisopropanolat-Lösung (aus 23 g Natrium und 250 ml Isopropylalkohol frisch hergestellt) langsam zugegeben. Das Reaktionsgemisch wird 2 Stunden unter Rückfluss erhitzt und dann mit 500 ml Wasser versetzt. Das Gemisch wird auf 200 ml Gesamtvolumen eingeengt und mit konz. Schwefelsäure auf pH 1 eingestellt. Der ausgefallene Niederschlag wird abfiltriert, die beiden Phasen des Filtrates getrennt und die organische Phase im Vakuum fraktioniert destilliert (101 - 103 °C/10 Torr; 83 g; 70 %). Die so erhaltene 2-Isopropoxy-essigsäure wird zusammen mit 0.2 ml konz. Schwefelsäure in 57 ml Methanol und 200 ml Benzol 7 Stunden am Wasserabscheider erhitzt. Nach Abdestillieren des überschüssigen Methanols und Benzols wird der Rückstand im Vakuum fraktioniert destilliert (50 - 55 °C/10 Torr; 81.6 g; 88 %).

### 4-Hydroxy-5-isopropoxy-2-mercapto-pyrimidin

81.6 g Isopropoxyessigsäuremethylester werden zusammen mit 50.3 ml Ethylformiat zu einer zuvor hergestellten Suspension aus 14.2 g Natrium in 200 ml Toluol gegeben. Man lässt das Reaktionsgemisch über Nacht stehen, dekantiert das Toluol ab und verwendet den ungereinigten Rückstand des 2-Isopropoxy-2-methoxycarbonyl-natriumethenolats ohne weitere Aufreinigung für den nächsten Schritt.
Der komplette Rückstand wird in 150 ml Ethanol unter Erwärmen gelöst. Anschließend gibt man 47.0 g Thioharnstaoff hinzu und erhitzt das Reaktionsgemisch 5 Stunden unter Rückfluss. Nach Entfernen des Lösungsmittels am Rotationsverdampfer nimmt man den Rückstand in 300 ml Wasser auf und stellt auf pH 2 ein, wobei das gewünschte Produkt als Niederschlag ausfällt, abfiltriert und über Nacht getrocknet wird (60.8 g; 53 %).

### 5-Isopropoxyuracil

60.8 g 4-Hydroxy-5-isopropoxy-2-mercapto-pyrimidin werden zusammen mit 60 g Chloressigsäure in 1.21 Wasser 2.5 Stunden unter Rückfluss erhitzt, wobei sich der Niederschlag vollständig auflöst. Man gibt 200 ml konz. Salzsäure zu und erhitzt weitere 7 Stunden unter Rückfluss. Anschließend engt man das Reaktionsgemisch auf 500 ml ein, wobei das gewünschte Produkt als Niederschlag ausfällt, abfiltriert und über Nacht bei 70 °C getrocknet wird (28.6 g; 52 %).

### 2,4-Dichlor-5-isopropoxy-pyrimidin

28.6 g 5-Isopropoxyuracil werden zusammen mit 140 ml Phosphoroxychlorid und 44 ml Dimethylanilin 2 Stunden unter Rückfluss erhitzt. Anschließen wird das überschüssige Phosphoroxychlorid im Vakuum abdestilliert. Man gießt den Rückstand auf 300 ml Eiswasser, extrahiert zweimal mit je 250 ml Diethylether, wäscht viermal mit je 50 ml Wasser und trocknet die Etherextrakte über Natriumsulfat. Das Lösungsmittel wird am Rotationsverdampfer entfernt und der Rückstand im Hochvakuum fraktioniert destilliert (82 - 85 °C/10⁻² Torr; 19.0 g; 55 %).
Rf = 0.62 (Cyclohexan/Essigsäureethylester = 1:1);
GC-MS (M+H)⁺ = 206/208 (2Cl);
1H-NMR (D6-DMSO; 400 MHz) δ: 1.38 (s, 6 H), 4.89 (m, 1 H), 8.65 (s, 1 H).

### Beispiel XV

### 2,4-Dichlor-5- isopropyl -pyrimidin.

23.0 g 5-Isopropyluracil werden zusammen mit 139 ml Phosphoroxychlorid und 38.8 ml Dimethylanilin 4 Stunden unter Rückfluss erhitzt. Anschließend wird das überschüssige Phosphoroxychlorid im Vakuum abdestilliert. Man gießt den Rückstand auf 400 ml Eiswasser und extrahiert zweimal mit je 250 ml Diethylether, wäscht dreimal mit je 50 ml Wasser und trocknet die Etherextrakte über Natriumsulfat. Das Lösungsmittel wird am Rotationsverdampfer entfernt und der Rückstand im Hochvakuum fraktioniert destilliert (70 - 78 °C/10⁻² Torr; 25.6 g; 90 %).
Rf = 0.69 (Essigsäureethylester);
GC-MS (M+H)⁺ = 190/192 (2Cl);
1H-NMR (D6-DMSO; 400 MHz) δ: 1.33 (s, 6 H), 3.22 (m, 1 H), 8.80 (s, 1 H).

### Beispiel XVI

### 3-Phenyl-nerhydro-azepin-4-on

Zu 313 g 3-Benzylamino-2-phenyl-propionsäure-ethylester in 800 ml Methylethylketon werden 281 g 4-Brombuttersäure-ethylester, 305 g Kaliumkarbonat und 5.5 g Kaliumiodid gegeben und für 24 Stunden unter Rückfluss gekocht. Zur Aufarbeitung wird vom Niederschlag abfiltriert, die Niederschlag mit Aceton nachgewaschen und die vereinten organischen Lösungen eingeengt. Der Rückstand wird in 1000 ml Diethylether aufgenommen, mit 500 ml 3N Salzsäure versetzt und die wässrige Phase sowie das ausgeölte Hydrochlorid isoliert. Die ätherische Phase wird zweimal mit 3 N Salzsäure nachextrahiert, die vereinten wässrigen Phasen mit konzentriertem wässrigen Ammoniak wieder alkalisch gestellt und zweimal mit Ether extrahiert. Nach dem Trocknen der organischen Phase über Magnesiumsulfat und dem Einengen im Vakuum erhält man das Produkt als klares Öl.
Ausbeute: 314 g

87.3 g NaH wird in 1300 ml Toluol suspendiert, hierzu 7.7 ml Ethanol rasch zugetropft. Innerhalb von 10 Minuten wird hierzu 199 g 4-[Benzyl-(2-ethoxycarbonyl-2-phenyl-ethyl)-amino]-buttersäure-ethylester in 230 ml Toluol zugetropft und drei Stunden unter Rühren gekocht. Nach dem Abkühlen auf 40 °C werden 160 ml Ethanol zugetropft und dann der Ansatz auf 700 ml eisgekühlte 6N HCl gegossen. Die wässrige Phase, sowie das ölige Hydrochlorid werden abgetrennt und die Toluolphase dreimal mit 300 ml Wasser nachextrahiert. Die vereinigten sauren Phasen, sowie das isolierte Öl werden 90 Minuten auf 140 °C erhitzt und anschließend über Nacht bei Raumtemperatur gerührt. Unter Eiskühlung wird dann mit konz. wässrigem Ammoniak alkalisch gestellt, zweimal mit Diethylether extrahiert, die organische Phase über Magnesiumsulfat getrocknet und eingeengt. Das Produkt kann aus Petrolether umkristallisiert werden. Ausbeute: 85.8 g.

22.1 g 1-Benzyl-3-phenyl-perhydro-azepin-4-one Hydrochlorid werden in 250 ml Methanol und 10 ml Wasser gelöst und mit 2.5 g Pd/C (10%) 3 Stunden unter 5 bar H₂ hydriert. Anschließend gibt man nochmals 2.5 g Katalysator zu und wiederholt den Hydrierungsschritt. Der Katalysator wird abfiltriert und der Ansatz eingeengt. Der Rückstand wird aus Ethanol umkristallisiert.

| | | |
|---|---|---|
| Ausbeute: 8.2 g | | |
| MS: [M]+ = 189 | | |
| CHNCI-Analyse | berechnet | 63.85% / 7.14% / 6.21% / 15.71% |
| | gefunden | 61.90% / 7.13% / 5.94% / 16.79% |
| Schmelzpunkt: 187 °C | Zersetzung | |

### Beispiel XVII

### 3-(4-Aminomethyl-cyclohexyl)-propionsäure

In 130 ml Methanol werden 11.2 g 3-[4-(Acetylamino-methyl)-phenyl]-propionsäure-methylester gelöst und unter Rühren 24 ml 8 N wässrige Natronlauge zugegeben. Nach 2 Stunden bei Raumtemperatur wird mit Eisessig neutralisiert und eingedampft. Den Rückstand nimmt man in Wasser auf und stellt mit 2 N wässriger Salzsäure auf einen pH Wert von 1-2 ein. Der entstehende weiße Niederschlag wird abgesaugt und mit Wasser gewaschen. Ausbeute 12,6 g
Schmelzpunkt 144-147 °C

Das Zwischenprodukt 3-[4-(Acetylamino-methyl)-phenyl]-propionsäure (12.6 g) wird in 200 ml Methanol gelöst und mit 1.2 g Rh/Pt Katalysator unter 3 bar H₂ Atmosphäre hydriert. Nach 90 Minuten bei Raumtemperatur wird vom Katalysator abfiltriert und die Lösung zur Trockne eingeengt. Ausbeute 13 g
Schmelzpunkt 91-94 °C

Zu 200 ml halbkonzentrierter wässriger Salzsäure werden 13 g 3-[4-(Acetylamino-methyl)-cyclohexyl]-propionsäure gegeben und die erhaltene Lösung über Nacht unter Rückfluss gekocht. Der Ansatz wird mit Aceton versetzt, umkristallisiert und der Niederschlag abgetrennt. Beim schrittweisen Einengen der Mutterlauge werden weitere Fraktionen des kristallinen Produkts isoliert. Die Fraktionen werden vereinigt, mit Isopropanol umkristallisiert und abschließend getrocknet. Ausbeute: 10.9 g.

### Beispiel XVIII

### 4-[(Pyridin-3-ylmethyl)-amino]-cyclohexan-carboxylsäure-methylester

In 250 ml Methanol werden 6.13 g Pyridin-3-aldehyd, 5.77 g Triethylamin und 9 g 4-Aminocyclohexancarbonsäure-methylester gemischt. Hierzu gibt man 3 g Raney Nickel zu, um unter Rührung ca. 6.5 Stunden bei 50 °C und 3 bar zu hydrieren. Nach Absaugen des Katalysators wird eingeenbt. Das Rohprodukt wird über Kieselgel mit Methylenchlorid/ Methanol gereinigt. Ausbeute 4.1 g.
Schmelzpunkt 47 °C

### Beispiel 1

### 2-(3,4-Dichlorphenylamino)-4-(2-acetylamino-ethylamino)-5-nitro-pyrimidin

Zu 800 mg 2-(3,4-Dichlorphenylamino)-4-thiocyanato-5-nitro-pyrimidin (Verbindung des Beispiels I) in 5 ml Dimethylformamid (DMF) werden bei Raumtemperatur 716 mg 2-Acetylamino-ethylamin in 8 ml DMF gegeben. Die Mischung geht unter leicht exothermer Reaktion in Lösung, nach 1,5 Stunden fällt ein gelblicher Niederschlag aus. Nach 3,5 Stunden werden 30 ml Wasser zugegeben. Der Niederschlag wird abgesaugt und getrocknet. Der Rückstand wird mit 30 ml Methylenchlorid gerührt, abgesaugt und getrocknet.
Ausbeute: 795 mg (88 % der Theorie)
Smp: 232°C
R_{f} = 0.6 (Kieselgel; Methylenchlorid:Methanol = 9:1)

Analog Beispiel 1 werden folgende Verbindungen erhalten:
(1) 2-(3,4-Dichlorphenylamino)-4-(4-aminomethyl-cyclohexylmethylamino)-5-nitro-pyrimidin
   Smp: 210°C Zersetzung
   R_{f} = 0.3 (Kieselgel; Methylenchlorid:Methanol:konz.Ammoniak = 9:1:0.1)
   Hergestellt aus der Verbindung des Beispiels I.
(2) 2-(3,4-Dichlorphenylamino)-4-(2-acetylamino-ethylamino)-5-cyano-pyrimidin
   Smp: 281°C
   R_{f} = 0.6 (Kieselgel; Cyclohexan:Essigsäureethylester:Methanol = 5:4:2)
   Hergestellt aus der Verbindung (11) des Beispiels III, durchgeführt in DMSO unter Mikrowellenbestrahlung (900 Watt).
(3) 2-(2-Naphthylamino)-4-(3-(2-oxo-pyrrolidin-1-yl)-propyl-1-amino)-5-trifluormethyl-pyrimidin Smp: 142°C
   HPLC/MS (Methode A): RT = 6.13 min.; [M+H]+ = 430.2
   R_{f} = 0.5 (Kieselgel; Cyclohexan:Essigsäureethylester:Methanol = 5:4:1)
   Hergestellt aus der Verbindung (8) des Beispiels III, durchgeführt in DMSO.
(4) 2-(4-Chlorphenylamino)-4-(1,4,6,7-tetrahydro-imidazo[4,5-*c*]pyridin-5-yl)-5-trifluormethyl-pyrimidin
   Smp: 230°C
   HPLC/MS (Methode A): RT = 5.54 min.; [M+H]+ = 395.1
   Hergestellt aus der Verbindung (3) des Beispiels III unter Verwendung von DMF, Dioxan und Hünigbase.
(5) 2-(3-Chlorphenylamino)-4-(1,4,6,7-tetrahydro-imidazo[4,5-*c*]pyridin-5-yl)-5-trifluormethyl-pyrimidin
   Smp: 210°C
   HPLC/MS (Methode A): RT = 5.61 min.; [M+H]+ = 395.1
   R_{f} = 0.26 (Kieselgel; Cyclohexan:Essigsäureethylester:Methanol = 5:4:1)
   Hergestellt aus der Verbindung (1) des Beispiels m unter Verwendung von DMSO und 2N NaOH.
(6) 2-(4-(1-Piperidinyl-methyl-)phenylamino)-4-(1,4,6,7-tetrahydro-imidazo[4,5-*c*]pyridin-5-yl)-5-trifluormethyl-pyrimidin
   Smp: 105°C
   HPLC/MS (Methode A): RT = 4.75 min.; [M+H]+ = 458.3
   R_{f} = 0.21 (Kieselgel; Methylenchlorid:Methanol:konz.Ammoniak = 9:1:0.1)
   Hergestellt aus der Verbindung (10) des Beispiels III unter Verwendung von DMSO und 2N NaOH.
(7) 2-(4-Amino-3,5-dichlorphenylamino)-4-(bis-(2-hydroxy-ethyl)-amino)-5-nitro-pyrimidin HPLC/MS (Methode A): RT = 5.67 min.; [M+H]+ = 403.1
(8) 2-(4-Aminosulfonyl-phenylamino)-4-(3-(2-oxo-pyrrolidin-1-yl)-propyl-1-amino)-5-nitro-pyrimidin
   HPLC/MS (Methode A): RT = 5.4 min.; [M+H]+ = 436.1
(9) 2-(3,4-Dichlorphenylamino)-4-(3-(2-aza-bicyclo[2.2.1]hept-5-en-2-yl)-propylamino)-5-trifluormethyl pyrimidin
(10) 2-(4-Chlorphenylamino)-4-(1,4,6,7-tetrahydro-imidazo[4,5-c]pyridin-5-yl)-5-nitro-pyrimidin HPLC/MS (Methode A): RT = 5.38 min.; [M+H]+ = 372.1
(11) 2-(4-Chlorphenylamino)-4-(3-(2-oxo-pyrrolidin-1-yl)-propyl-1-amino)-5-trifluormethyl-pyrimidin
   HPLC/MS (Methode A): RT = 6.12min.; [M+H]+ = 414.1
   Smp: 260-262 °C
   Rf-Wert: 0,82 (Kieselgel; Cyclohexan / EE / MeOH = 5:4:1)
   HPLC/MS (Methode D): RT = 5.837 min.; [M+H]+ = 414; Abs./max 250 nm
(12) 2-(3-Chlorphenylamino)-4-(3-aminopropyl-amino)-5-trifluormethyl-pyrimidin
   HPLC/MS (Methode A): RT = 5.13min.; [M+H]+ = 346.1
(13) 2-(4-Carboxyphenylamino)-4-[N-(3-dimethylamino-propyl-)N-methyl]-amino-5-nitro-pyrimidin
   HPLC/MS (Methode A): RT = 4.83min.; [M+H]+ = 375.2
(14) 2-(3,5-Dichlorphenylamino)-4-(1,4,6,7-tetrahydro-imidazo[4,5-c]pyridin-5-yl)-5-trifluormethyl-pyrimidin
   HPLC/MS (Methode A): RT = 5.85min.; [M+H]+ = 429.1
(15) 2-(3-Chlor-4-fluor-phenylamino)-4-(1,4,6,7-tetrahydro-imidazo[4,5-c]pyridin-5-yl)-5-nitro-pyrimidin
   HPLC/MS (Methode A): RT = 5.39min.; [M+H]+ = 390.1
(16) 2-(3,4-Dichlor-phenylamino)-4-(2-nitrobenzylamino)-5-trifluormethyl-pyrimidin
   HPLC/MS (Methode A): RT = 7.77min.; [M+H]+= 458.1
(17) 2-(4-Carboxy-phenylamino)-4-(1,4,6,7-tetrahydro-imidazo[4,5-c]pyridin-5-yl)-5-trifluormethyl-pyrimidin
   HPLC/MS (Methode A): RT = 5.12min.; [M+H]+ = 405.1
(18) 2-(4-Carboxy-phenylamino)-4-(trans-4-dimethylamino-cyclohexylamino)-5-nitro-pyrimidin HPLC/MS (Methode A): RT = 4.97min.; [M+H]+ = 401.2
(19) 2-(4-Brom-phenylamino)-4-(1,4,6,7-tetrahydro-imidazo[4,5-c]pyridin-5-yl)-5-trifluormethyl-pyrimidin
   HPLC/MS (Methode A): RT = 5.61 min.; [M+H]+ = 439.1
(20) 2-(3,5-Dichlor-phenylamino)-4-(3-amino-cyclohexylamino)-5-trifluormethyl-HPLC/MS (Methode A): RT = 5.71 min.; [M+H]+ = 420.1
(21) 2-(4-Carboxy-phenylamino)-4-(3-(2-oxo-pyrrolidin-1-yl)-propyl-1-amino)-5-trifluormethyl-pyrimidin
   HPLC/MS (Methode A): RT = 5.44min.; [M+H]+ = 424.2
(22) 2-(3-Aminocarbonyl-phenylamino)-4-(1,4,6,7-tetrahydro-imidazo[4,5-c]pyridin-5-yl)-5-nitro-pyrimidin
   HPLC/MS (Methode A): RT = 4.71min.; [M+H]+ = 381.2
(23) 2-(4-(2-Carboxy-1-ethyl-)phenylamino)-4-(1,4,6,7-tetrahydro-imidazo[4,5-c]pyridin-5-yl)-5-trifluormethyl-pyrimidin
   HPLC/MS (Methode A): RT = 5.13min.; [M+H]+ = 433.2
(24) 2-(3-Brom-phenylamino)-4-(3-amino-propylamino)-5-trifluormethyl-pyrimidin
   HPLC/MS (Methode A): RT = 5.16min.; [M+H]+ = 390.1
(25) 2-(3,4-Dichlor-phenylamino)-4-(2-aminocarbonyl-1-ethylamino)-5-trifluormethyl-pyrimidin
   HPLC/MS (Methode A): RT = 6.36min.; [M+H]+ = 394.1
(26) 2-(4-Phenylaminocarbonyl-phenylamino)-4-(1,4,6,7-tetrahydro-imidazo[4,5-c]pyridin-5-yl)-5-nitro-pyrimidin
   HPLC/MS (Methode A): RT = 5.3min.; [M+H]+ = 457.2
(27) 2-(4-Nitro-phenylamino)-4-(1,4,6,7-tetrahydro-imidazo[4,5-c]pyridin-5-yl)-5-nitro-pyrimidin
   HPLC/MS (Methode A): RT = 5.21min.; [M+H]+ = 383.2
(28) 2-(4-Chlor-phenylamino)-4-(3-(4-(pyrrolidin-1-yl)butyl)-piperidin-1-yl)-5-nitro-pyrimidin HPLC/MS (Methode A): RT = 5.89min.; [M+H]+ = 459.2
(29) 2-(4-Carboxy-phenylamino)-4-(1,4,6,7-tetrahydro-imidazo[4,5-c]pyridin-5-yl)-5-nitro-pyrimidin
   HPLC/MS (Methode A): RT = 4.85min.; [M+H]+ = 382.1
(30) 2-(3,4-Dichlor-phenylamino)-4-(1-methyl-piperidin-4-yl-methylamino)-5-trifluormethyl-pyrimidin
   HPLC/MS (Methode A): RT = 5.58min.; [M+H]+ = 434.1
(31) 2-Phenylamino-4-(3-(2-oxo-pyrrolidin-1-yl)-propyl-1-amino)-5-trifluormethyl-pyrimidin
   HPLC/MS (Methode A): RT = 5.36min.; [M+H]+ = 380.2
(32) 2-(4-Cyano-phenylamino)-4-(1,4,6,7-tetrahydro-imidazo[4,5-c]pyridin-5-yl)-5-nitro-pyrimidin
   HPLC/MS (Methode A): RT = 5.11min.; [M+H]+ = 363.1
(33) 2-Phenylamino-4-(4-aminomethyl-cyclohexylmethylamino)-5-trifluormethyl-pyrimidin
   HPLC/MS (Methode A): RT = 4.93min.; [M+H]+ = 380.2
(34) 2-(4-Chlor-phenylamino)-4-(3-amino-cyclohexylamino)-5-nitro-pyrimidin
   HPLC/MS (Methode A): RT = 5.41min.; [M+H]+ = 363.1
(35) 2-(3,4-Dichlor-phenylamino)-4-(3-amino-propylamino)-5-trifluormethyl-pyrimidin
   HPLC/MS (Methode A): RT = 5.45min.; [M+H]+ = 380.1
(36) 2-(4-Chlor-phenylamino)-4-(3-amino-propylamino)-5-trifluormethyl-pyrimidin
   HPLC/MS (Methode A): RT = 5.02min.; [M+H]+ = 346.1
(37) 2-(3,4-Dichlor-phenylamino)-4-(cis-4-hydroxy-cyclohexylamino)-5-trifluormethyl-pyrimidin
   HPLC/MS (Methode A): RT = 6.84min.; [M+H]+ = 421.1
(38) 2-(3-Brom-phenylamino)-4-(1,4,6,7-tetrahydro-imidazo[4,5-c]pyridin-5-yl)-5-trifluormethyl-pyrimidin
   HPLC/MS (Methode A): RT = 5.6min.; [M+H]+ = 439.1
(39) 2-(2-Naphthylamino)-4-(3-amino-cyclohexylamino)-5-trifluormethyl-pyrimidin
   HPLC/MS (Methode A): RT = 5.34min.; [M+H]+ = 402.2
(40) 2-(3,4-Dichlor-phenylamino)-4-(3-acetylaminomethyl-piperidin-1-yl)-5-trifluormethyl-pyrimidin
   HPLC/MS (Methode A): RT = 7.12min.; [M+H]+ = 462.1
(41) 2-(3,4-Dichlor-phenylamino)-4-(3-(2-oxo-pyrrolidin-1-yl)-propyl-1-amino)-5-trifluormethyl-pyrimidin
   HPLC/MS (Methode A): RT = 6.85min.; [M+H]+ = 448.1D
(42) 2-(3-Brom-phenylamino)-4-(4-aminomethyl-piperidin-1-yl)-5-nitro-pyrimidin
   HPLC/MS (Methode A): RT = 5.36min.; [M+H]+ = 407.1
(43) 2-(4-Brom-phenylamino)-4-(4-aminomethyl-piperidin-1-yl)-5-nitro-pyrimidin
   HPLC/MS (Methode A): RT = 5.53min.; [M+H]+ = 430.1
(44) 2-(4-Chlor-phenylamino)-4-(N-(1-methyl-piperidin-4-yl)-N-methyl-amino)-5-nitro-pyrimidin
   HPLC/MS (Methode A): RT = 5.32min.; [M+H]+ = 377.2
(45) 2-(3,4-Dichlor-phenylamino)-4-(5-hydroxy-1-pentylamino)-5-trifluormethyl-pyrimidin
   HPLC/MS (Methode A): RT = 6.79min.; [M+H]+ = 409.1
(46) 2-(4-(2-Carboxy-1-ethyl)-phenylamino)-4-(2-(3-hydroxyphenyl)-1-ethyl-amino)-5-trifluormethyl-pyrimidin
   HPLC/MS (Methode A): RT = 5.64min.; [M+H]+ = 447.1
(47) 2-(3-Brom-phenylamino)-4-(3-(2-oxo-pyrrolidin-1-yl)-propyl-1-amino)-5-trifluormethyl-pyrimidin
   HPLC/MS (Methode A): RT = 6.37min.; [M+H]+ = 458.1D
(48) 2-(4-Chlor-phenylamino)-4-(*(1S)*-1-carboxy-1-ethylamino)-5-nitro-pyrimidin
   HPLC/MS (Methode B): RT = 2,5 min.; [M+H]+ = 338
(49) 2-(3,5-Dichlor-phenylamino)-4-(3-(2-oxo-pyrrolidin-1-yl)-propyl-1-amino)-5-trifluormethyl-pyrimidin
   HPLC/MS (Methode A): RT = 7.15min.; [M+H]+ = 448.1

(50) 2-(3-Nitro-phenylamino)-4-(3-hydroxymethyl-piperidin-1-yl)-5-nitro-pyrimidin
   HPLC/MS (Methode A): RT = 6.39min.; [M+H]+ = 375.2
(51) 2-(4-Brom-phenylamino)-4-(3-amino-propylamino)-5-trifluormethyl-pyrimidin
   HPLC/MS (Methode A): RT = 5.12min.; [M+H]+ = 390.1
(52) 2-(4-Chlor-phenylamino)-4-(4-(N-acetyl-N-methyl-aminomethyl)-piperidin-1-yl)-5-nitro-pyrimidin
   HPLC/MS (Methode A): RT = 6.61min.; [M+H]+ = 419.2
(53) 2-(3,4-Dichlor-phenylamino)-4-(2-(4-hydroxy-3-methoxy-phenyl)-2-hydroxy-1-ethylamino)-5-trifluormethyl-pyrimidin
   HPLC/MS (Methode A): RT = 6.79min.; [M+H]+ = 489.1
(54) 2-(4-Carboxy-phenylamino)-4-(trans-4-dimethylamino-cyclohexylamino)-5-trifluormethyl-pyrimidin
   HPLC/MS (Methode A): RT = 4.93min.; [M+H]+ = 424.2
(55) 2-(4-Chlor-phenylamino)-4-(*(2S)*-2-hydroxymethyl-pyrrolidin-1-yl)-5-nitro-pyrimidin HPLC/MS (Methode A): RT = 6.59min.; [M+H]+ = 350.1
   Hergestellt unter Verwendung von L-Prolinol.
(56) 2-(4-Brom-phenylamino)-4-(3-(2-oxo-pyrrolidin-1-yl)-propyl-1-amino)-5-trifluormethyl-pyrimidin
   HPLC/MS (Methode A): RT = 6.19min.; [M+H]+ = 458.1
(57) 2-(3,4-Dichlor-phenylamino)-4-(3-amino-cyclohexylamino)-5-trifluormethyl-pyrimidin
   HPLC/MS (Methode A): RT = 5.66min.; [M+H]+ = 420.1
(58) 2-(3,4-Dichlor-phenylamino)-4-(3-(isopropylamino)-1-propylamino)-5-trifluormethyl-pyrimidin
   HPLC/MS (Methode A): RT = 5.66min.; [M+H]+ = 422.1
(59) 2-(4-Chlor-phenylamino)-4-(2-(3-hydroxy-phenyl)-2-hydroxy-1-ethylamino)-5-nitro-pyrimidin
   HPLC/MS (Methode A): RT = 6.49min.; [M+H]+ = 402.1
(60) 2-(3,4-Dichlor-phenylamino)-4-(1,4,6,7-tetrahydro-imidazo[4,5-c]pyridin-5-yl)-5-trifluormethyl-pyrimidin
   HPLC/MS (Methode A): RT = 5.78min.; [M+H]+ = 429.1
(61) 2-(3,4-Dichlor-phenylamino)-4-(trans-4-carboxy-cyclohexylamino)-5-trifluormethyl-pyrimidin
   HPLC/MS (Methode A): RT = 6.99min.; [M+H]+ = 449.1
(62) 2-(3,4-Dichlor-phenylamino)-4-(1,1-dimethyl-2-hydroxy-1-ethylamino)-5-trifluormethyl-pyrimidin
   HPLC/MS (Methode A): RT = 7.34min.; [M+H]+ = 395.1
(63) 2-(3,4-Dichlor-phenylamino)-4-(5-amino-pentylamino)-5-trifluormethyl-pyrimidin
   HPLC/MS (Methode A): RT = 5.57min.; [M+H]+ = 408.1
(64) 2-(4-Amino-3,5-dichlorphenylamino)-4-(3-hydroxymethyl-piperidin-1-yl)-5-nitro-pyrimidin
   HPLC/MS (Methode A): RT = 6.43min.; [M+H]+ = 413.1
(65) 2-(3,4-Dichlor-phenylamino)-4-(6-hydroxy-1-hexylamino)-5-trifluormethyl-pyrimidin
   HPLC/MS (Methode A): RT = 7.06min.; [M+H]+ = 423.1
(66) 2-(3,4-Dichlor-phenylamino)-4-(*(1S)*-1-carboxy-2-(1*H*-imidazol-4-yl)-ethylamino)-5-trifluormethyl-pyrimidin
   HPLC/MS (Methode A): RT = 5.51min.; [M+H]+ = 461.1
   Hergestellt unter Verwendung von L-Histidin.
(67) 2-(3-Chlor-phenylamino)-4-(3-(2-oxo-pyrrolidin-1-yl)-propyl-1-amino)-5-trifluormethyl-pyrimidin
   HPLC/MS (Methode A): RT = 6.24min.; [M+H]+ = 414.2
   Rf-Wert: 0,63 (Kieselgel; Methylenchlorid / MeOH = 95:5)
   Schmelzpunkt: 155-157 °C
   HPLC/MS (Methode D): RT = 5,964 min.; [M+H]+ = 415; Abs./max 246 nm
(68) 2-(3,4-Dichlorphenylamino)-4-(2-(1H-imidazol-4-yl)-ethylamino)-5-trifluormethyl-pyrimidin
   HPLC/MS (Methode A): RT = 5.55min.; [M+H]+ = 417.1
(69) 2-(3,4-Dichlor-phenylamino)-4-(4-hydroxy-but-1-ylamino)-5-trifluormethyl-pyrimidin
   HPLC/MS (Methode A): RT = 6.66min.; [M+H]+ = 395.1
(70) 2-(4-Aminosulfonyl-phenylamino)-4-(4-aminomethyl-piperidin-1-yl)-5-nitro-pyrimidin
   HPLC/MS (Methode A): RT = 4.66min.; [M+H]+ = 408.2
(71) 2-(3,4-Dichlor-phenylamino)-4-(4-carboxy-1-butylamino)-5-trifluormethyl-pyrimidin
   HPLC/MS (Methode A): RT = 6.78min.; [M+H]+ = 423.1
(72) 2-(3,4-Dichlor-phenylamino)-4-(1-methyl-4-piperidinyl-amino)-5-trifluormethyl-pyrimidin
   HPLC/MS (Methode A): RT = 5.58min.; [M+H]+ = 420.1
(73) 2-(3,4-Dichlor-phenylamino)-4-(3-(3-aminopropoxy-1-propylamino)-5-trifluormethyl-pyrimidin
   HPLC/MS (Methode A): RT = 5.65min.; [M+H]+ = 438.1
(74) 2-(4-Carboxy-phenylamino)-4-(1-hydroxy-2-propylamino)-5-nitro-pyrimidin
   HPLC/MS (Methode A): RT = 5.55min.; [M+H]+ = 334.1
(75) 2-(4-Aminosulfonyl-phenylamino)-4-(4-aminomethyl-cyclohexylmethylamino)-5-nitro-pyrimidin
   HPLC/MS (Methode A): RT = 4.93min.; [M+H]+ = 436.2
(76) 2-(3,4-Dichlor-phenylamino)-4-(2-(3-hydroxy-phenyl)-2-hydroxy-1-ethylamino)-5-trifluormethyl-pyrimidin
   HPLC/MS (Methode A): RT = 6.85min.; [M+H]+ = 459.1
(77) 2-(3-Chlor-phenylamino)-4-(5-amino-1-pentylamino)-5-trifluormethyl-pyrimidin
   HPLC/MS (Methode A): RT = 5.19min.; [M+H]+= 374.1
(78) 2-(3,4-Dichlorphenylamino)-4-(4-nitrobenaylamino)-5-trifluormethyl-HPLC/MS (Methode A): RT = 7.72min.; [M+H]+= 458.1
(79) 2-(4-Chlor-phenylamino)-4-(5-amino-pentyl-1-amino)-5-trifluormethyl-pyrimidin
   HPLC/MS (Methode A): RT = 5.12min.; [M+H]+= 374.1
(80) 2-(3,4-Dichlor-phenylamino)-4-(2-amino-1-ethylamino)-5-trifluormethyl-pyrimidin
   100 mg (4-Chlor-5-trifluormethyl-pyrimidine-2-yl)-(3,4-dichloro-phenyl)-amin, (2-Amino-ethyl)-carbaminsäure tert-butylester (1 eq.) und Diisopropylethylamin (2 eq.) wurden in 2 ml Isopropanol für mehrere Stunden bei Raumtemperatur gerührt. Das Reaktionsgemisch wurde mit 2 ml ges. NaHCO3-Lösung versetzt und mit Essigsäureethylester extrahiert. Die org. Phase wurde über MgSO4 getrocknet und eingeengt. Das Rohprodukt wurde mit Diethylether und Dichlormethan gewaschen und durch Chromatographie (CH2Cl2/MeOH-Gradient, Kieselgel) gereinigt. Anschließende wurde das Produkt mit TFA/CH2Cl2 (1:1) behandelt, mit NaHCO3 versetzt, mit Essigsäureethylester extrahiert, über MgSO4 getrocknet und eingeengt.HPLC/MS (Methode A): RT = 5.54min.; [M+H]+ = 366.1
   Schmelzpunkt: 115-117°C
   R_{f} = 0,13 (Kieselgel; Methylenchlorid: Methanol = 4:1)
   HPLC/MS (Methode F): RT = 3,84 min.; [M+H]+ = 367; Abs. λ max = 258,9 nm
(81) 2-(3,4-Dichlor-phenylamino)-4-(4-dimethylaminomethyl-cyclohexylmethylamino)-5-trifluormethyl-pyrimidin
   HPLC/MS (Methode A): RT = 5.78min.; [M+H]+ = 476.2
(82) 2-(4-Chlor-phenylamino)-4-(N-methyl-N-(2-cyano-1-ethyl)-amino)-5-nitro-pyrimidin
   HPLC/MS (Methode A): RT = 6.74min.; [M+H]+ = 333.1
(83) 2-(4-Chlor-phenylamino)-4-(3-acetylaminomethyl-piperidin-1-yl)-5-nitro-pyrimidin
   HPLC/MS (Methode A): RT = 6.46min.; [M+H]+ = 405.2
(84) 2-(4-Carboxy-phenylamino)-4-(4-(2-pyridyl)-piperazin-1-yl)-5-nitro-pyrimidin
   HPLC/MS (Methode A): RT = 5.04min.; [M+H]+ = 422.2
(85) 2-(3,4-Dichlor-phenylamino)-4-(2-(1-methyl-2-pyrrolidinyl)-1-ethylamino)-5-trifluormethyl-pyrimidin
   R_{f}= 0,16 (Kieselgel; Methylenchlorid: Methanol = 9:1)
   HPLC/MS (Methode G): RT = 3,66min.; [M+H]+ = 435 ; Abs. λ max = 266,5 nm
(86) 2-(4-Chlor-phenylamino)-4-(4-carboxy-1-butylamino)-5-nitro-pyrimidin
   HPLC/MS (Methode A): RT = 6.55min.; [M+H]+ = 366.1
(87) 2-(3,4-Dichlor-phenylamino)-4-(bis-(2-hydroxy-ethyl)-amino)-5-trifluormethyl-pyrimidin
   HPLC/MS (Methode A): RT = 6.4min.; [M+H]+ = 411.1
(88) 2-(3-Brom-phenylamino)-4-(4-aminomethyl-cyclohexylmethylamino)-5-trifluormethyl-pyrimidin
   HPLC/MS (Methode A): RT = 5.46min.; [M+H]+ = 458.1
(89) 2-(3,4-Dichlor-phenylamino)-4-(2-(4-hydroxy-phenyl)-2-hydroxy-1-ethylamino)-5-trifluormethyl-pyrimidin
   HPLC/MS (Methode A): RT = 6.73min.; [M+H]+ = 459.1
(90) 2-(3,4-Dichlor-phenylamino)-4-(4-aminomethyl-piperidin-1-yl)-5-trifluormethyl-pyrimidin
   HPLC/MS (Methode A): RT = 5.78min.; [M+H]+ = 420.1
(91) 2-(4-Aminocarbonyl-phenylamino)-4-(2-(3-hydroxypropyl)-piperidin-1-yl)-5-nitro-pyrimidin
   HPLC/MS (Methode A): RT = 5.65min.; [M+H]+ = 401.2
(92) 2-(3-Nitro-phenylamino)-4-(4-aminomethyl-cyclohexylmethylamino)-5-nitro-pyrimidin
   HPLC/MS (Methode A): RT = 5.41min.; [M+H]+ = 402.2
(93) 2-(3,4-Dichlor-phenylamino)-4-(2-methoxy-1-ethylamino)-5-trifluormethyl-pyrimidin
   HPLC/MS (Methode A): RT = 7.37min.; [M+H]+ = 381.1
(94) 2-(4-Methoxycarbonyl-phenylamino)-4-(4-aminomethyl-cyclohexylmethylamino)-5-nitro-pyrimidin
   HPLC/MS (Methode A): RT = 5.37min.; [M+H]+ = 415.2
(95) 2-(4-Chlor-phenylamino)-4-(3-(2-oxo-pyrrolidin-1-yl)-propyl-1-amino)-5-nitro-pyrimidin
   HPLC/MS (Methode A): RT = 6.43min.; [M+H]+ = 391.2
(96) 2-(3,4-Dichlor-phenylamino)-4-(3-hydroxymethyl-piperidin-1-yl)-5-trifluormethyl-pyrimidin
   HPLC/MS (Methode A): RT = 7.42min.; [M+H]+ = 421.1
(97) 2-(3-Cyano-phenylamino)-4-(1,4,6,7-tetrahydro-imidazo[4,5-c]pyridin-5-yl)-5-nitro-pyrimidin
   HPLC/MS (Methode A): RT = 5.12min.; [M+H]+ = 363.2
(98) 2-(4-Benzylaminocarbonyl-phenylamino)-4-(4-aminomethyl-cyclohexylmethylamino)-5-nitro-pyrimidin
   HPLC/MS (Methode A): RT = 5.45min.; [M+H]+ = 490.3
(99) 2-(3,4-Dichlor-phenylamino)-4-(2-(4-(2-hydroxyethyl)-piperazin-1-yl)-1-ethylamino))-5-trifluormethyl-pyrimidin
   HPLC/MS (Methode A): RT = 5.42min.; [M+H]+ = 479.2

(100) 2-(4-Amino-3,5-dichlorphenylamino)-4-(4-aminomethyl-cyclohexylmethylamino)-5-nitro-pyrimidin
   HPLC/MS (Methode A): RT = 5.44min.; [M+H]+ = 440.2
(101) 2-(4-Aminocarbonyl-phenylamino)-4-(3-hydroxymethyl-piperidin-1-yl)-5-nitro-pyrimidin
   HPLC/MS (Methode A): RT = 5.44min.; [M+H]+ = 373.2
(102) 2-(3,4-Dichlor-phenylamino)-4-(2-(3-hydroxyphenyl)-1-ethylamino)-5-trifluormethyl-pyrimidin
   HPLC/MS (Methode A): RT = 7.32min.; [M+H]+ = 443.1
(103) 2-(3,4-Dichlor-phenylamino)-4-(3-hydroxy-piperidin-1-yl)-5-trifluormethyl-pyrimidin
   HPLC/MS (Methode A): RT = 7.31min.; [M+H]+ = 407.1
(104) 2-(3,4-Dichlor-phenylamino)-4-(2-morpholino-1-ethylamino)-5-trifluormethyl-pyrimidin
(105) 2-(4-Brom-phenylamino)-4-(4-aminomethyl-cyclohexylmethylamino)-5-trifluormethyl-pyrimidin
   HPLC/MS (Methode A): RT = 5.42min.; [M+H]+ = 458.1
(106) 2-(4-Chlor-phenylamino)-4-(5-aminopentylamino)-5-nitro-pyrimidin
   HPLC/MS (Methode A): RT = 5.41min.; [M+H]+ = 351.1
(107) 2-(4-Chlor-phenylamino)-4-(4-aminomethyl-cyclohexylmethylamino)-5-trifluormethyl-pyrimidin
   HPLC/MS (Methode A): RT = 5.33min.; [M+H]+ = 414.2
(108) 2-(3-Nitro-phenylamino)-4-(2-(3-hydroxypropyl)-piperidin-1-yl)-5-nitro-pyrimidin
   HPLC/MS (Methode A): RT = 6.68min.; [M+H]+ = 403.2
(109) 2-(3,4-Dichlorphenylamino)-4-(4-amino-cyclohexylamino)-5-trifluormethyl-pyrimidin
   HPLC/MS (Methode A): RT = 5.55min.; [M+H]+ = 420.1
(110) 2-(4-Chlor-phenylamino)-4-(2-methoxy-1-ethylamino)-5-nitro-pyrimidin
   Schmelzpunkt: 153-155°C
(111) 2-(4-Carboxyphenylamino)-4-(4-amino-cyclohexylamino)-5-trifluormethyl-pyrimidin
   HPLC/MS (Methode A): RT = 4.93min.; [M+H]+ = 396.2
(112) 2-(4-(2-Carboxy-1-ethyl)-phenylamino)-4-(1,4,6,7-tetrahydro-imidazo[4,5-c]pyridin-5-yl)-5-nitro-pyrimidin
   HPLC/MS (Methode A): RT = 4.98min.; [M+H]+ = 410.2
(113) 2-(4-Amino-3,5-dichlorphenylamino)-4-(1,4,6,7-tetrahydro-imidazo[4,5-c]pyridin-5-yl)-5-nitro-pyrimidin
   HPLC/MS (Methode A): RT = 5.24min.; [M+H]+ = 421.1
(114) 2-(4-Aminocarbonyl-phenylamino)-4-(1,4,6,7-tetrahydro-imidazo[4,5-c]pyridin-5-yl)-5-nitro-pyrimidin
   HPLC/MS (Methode A): RT = 4.67min.; [M+H]+ = 381.2
(115) 2-(4-Chlor-3-methyl-phenylamino)-4-(4-aminomethyl-cyclohexylmethylamino)-5-nitro-pyrimidin
   HPLC/MS (Methode A): RT = 5.66min.; [M+H]+ = 405.2
(116) 2-(4-Bromphenylamino)-4-(1,4,6,7-tetrahydro-imidazo[4,5-c]pyridin-5-yl)-5-nitro-pyrimidin
   HPLC/MS (Methode A): RT = 5.41min.; [M+H]+ = 416.1
(117) 2-(3,4-Dichlorphenylamino)-4-(7-methyl-2,7-diaza-spiro[4.4]non-2-yl)-5-trifluormethyl-pyrimidin
(118) 2-Phenylamino-4-(1,4,6,7-tetrahydro-imidazo[4,5-c]pyridin-5-yl)-5-trifluormethyl-pyrimidin
   HPLC/MS (Methode A): RT = 5.23min.; [M+H]+ = 361.2
(119) 2-(3-Bromphenylamino)-4-(1,4,6,7-tetrahydro-imidazo[4,5-c]pyridin-5-yl)-5-nitro-pyrimidin
   HPLC/MS (Methode A): RT = 5.4min.; [M+H]+ = 416.1
(120) 2-(3,4-Dichlorphenylamino)-4-(4-dimethylamino-cyclohexylamino)-5-trifluormethyl-pyrimidin
   HPLC/MS (Methode A): RT = 5.62min.; [M+H]+ = 448.1
(121) 2-(3,4-Dichlorphenylamino)-4-(2-(imidazol-1-yl)-1-ethylamino)-5-trifluormethyl-pyrimidin
   HPLC/MS (Methode A): RT = 5.63min.; [M+H]+ = 417.1
(122) 2-(3-Nitro-phenylamino)-4-(1,4,6,7-tetrahydro-imidazo[4,5-c]pyridin-5-yl)-5-nitro-pyrimidin
   HPLC/MS (Methode A): RT = 5.2min.; [M+H]+ = 383.1
(123) 2-(4-Chlor-phenylamino)-4-(N-(2-hydroxybenzyl)-N-methyl-amino)-5-nitro-pyrimidin
   HPLC/MS (Methode A): RT = 7.07min.; [M+H]+ = 386.1
(124) 2-(4-Phenylaminocarbonyl-phenylamino)-4-(4-aminomethyl-cyclohexylmethylamino)-5-nitro-pyrimidin
   HPLC/MS (Methode A): RT = 5.5min.; [M+H]+ = 476.3
(125) 2-(4-Fluor-phenylamino)-4-(1,4,6,7-tetrahydro-imidazo[4,5-c]pyridin-5-yl)-5-nitro-pyrimidin
   HPLC/MS (Methode A): RT = 5.13min.; [M+H]+ = 356.1
(126) 2-(3,4-Dichlorphenylamino)-4-(3-(3-hydroxy-8-methyl-8-aza-bicyclo[3.2.1]octyl)-methylamino)-5-trifluormethyl-pyrimidin
   HPLC/MS (Methode A): RT = 5.61min.; [M+H]+ = 476.1
(127) 2-(4-Carboxy-phenylamino)-4-(4-ethoxycarbonyl-piperidin-1-yl)-5-nitro-pyrimidin
   HPLC/MS (Methode A): RT = 6.37min.; [M+H]+ = 416.2
(128) 2-(3,4-Dichlorphenylamino)-4-(trans-4-hydroxy-cyclohexylamino)-5-trifluormethyl-pyrimidin
   HPLC/MS (Methode A): RT = 6.87min.; [M+H]+ = 421.1
(129) 2-(3,4-Dichlorphenylamino)-4-(2-(1H-pyrazol-4-yl)-1-ethylamino)-5-trifluormethyl-pyrimidin
(130) 2-(Phenylamino)-4-(1-methyl-pipendin-4-yl-methylamino)-5-trifluormethyl-pyrimidin
   HPLC/MS (Methode A): RT = 4.68min.; [M+H]+ = 352.2
(131) 2-(4-Chlor-phenylamino)-4-(1-methyl-piperidin-4-yl-methylamino)-5-trifluormethyl-pyrimidin
   HPLC/MS (Methode A): RT = 5.23min.; [M+H]+ = 386.2
(132 2-(4-Brom-phenylamino)-4-(1-methyl-piperidin-4-yl-methylamino)-5-trifluormethyl-pyrimidin
   HPLC/MS (Methode A): RT = 5.32min.; [M+H]+ = 430.1D
(133) 2-(3-Brom-phenylamino)-4-(1-methyl-piperidin-4-yl-methylamino)-5-trifluormethyl-pyrimidin
   HPLC/MS (Methode A): RT = 5.39min.; [M+H]+ = 430.1
(134) 2-(3-Chlor-phenylamino)-4-(4-aminomethyl-piperidin-1-yl)-5-trifluormethyl-pyrimidin
   HPLC/MS (Methode A): RT = 5.50min.; [M+H]+ = 386.2
(135) 2-(Phenylamino)-4-(4-aminomethyl-piperidin-1-yl)-5-trifluormethyl-pyrimidin
   HPLC/MS (Methode A): RT = 5.05min.; [M+H]+ = 352.2
(136) 2-(4-Chlor-phenylamino)-4-(4-aminomethyl-piperidin-1-yl)-5-trifluormethyl-pyrimidin
   HPLC/MS (Methode A): RT = 5.45min.; [M+H]+ = 386.1
(137) 2-(3-Brom-phenylamino)-4-(4-aminomethyl-piperidin-1-yl)-5-trifluormethyl-pyrimidin
   HPLC/MS (Methode A): RT = 5.54min.; [M+H]+ = 430.1
(138) 2-(3-Chlor-phenylamino)-4-(4-aminomethyl-cyclohexylmethylamino)-5-trifluormethyl-pyrimidin
   HPLC/MS (Methode A): RT = 5.40min.; [M+H]+ = 414.2
(139) 2-(3-Chlor-phenylamino)-4-(2-amino-cyclohexylamino)-5-trifluormethyl-pyrimidin
   HPLC/MS (Methode A): RT = 5.63min.; [M+H]+ = 386.2
(140) 2-(4-Chlorphenylamino)-4-(2-amino-1-ethylamino)-5-nitro-pyrimidin
   Rf-Wert: 0,30 (Kieselgel; Methylenchlorid/Methanol/konz wäßriges Ammoniak = 9:1:0,1)
(141) 2-(4-Chlorphenylamino)-4-[2-(acetylamino)-1-ethylamino]-5-nitro-pyrimidin
   Hergestellt aus der Verbindung 140 des Beispiels 1 durch nachträgliche Umsetzung mit Acetanhydrid/Triethylamin.
   Schmelzpunkt: 224-226°C
(142) 2-(4-Chlorphenylamino)-4-[4-(dimethylamino)butylamino]-5-nitro-pyrimidin
   Schmelzpunkt: 131-132°C
(143) 2-(3,4-Dichlorphenylamino)-4-(1-carboxy-1-ethylamino)-5-trifluormethyl-HPLC/MS (Methode A): RT = 7.04min.; [M+H]+ = 395.0
(144) 2-(4-Carboxy-phenylamino)-4-[N-(2-hydroxyethyl)-N-benzylamino]-5-nitro-pyrimidin
   HPLC/MS (Methode A): RT = 6.05min.; [M+H]+ =410.2
(145) 2-(3,4-Dichlorphenylamino)-4-(*(1R)*-1-carboxy-2-(1H-imidazol-4-yl)-1-ethylamino)-5-trifluormethyl-pyrimidin
   HPLC/MS (Methode A): RT = 5.51min.; [M+H]+ = 461.0
   Hergestellt unter Verwendung von D-Histidin.
(146) 2-(3,4-Dichlorphenylamino)-4-(3-hydroxy-1,3-dihydro-2-oxo-indol-3-yl-methylamino)-5-trifluormethyl-pyrimidin
   HPLC/MS (Methode A): RT = 6.83min.; [M+H]+ = 484.1
(147) 2-(3,4-Dichlorphenylamino)-4-(4-(2-carboxy-1-ethyl)-cyclohexylamino)-5-trifluormethyl-pyrimidin
   HPLC/MS (Methode A): RT = 7.42min.; [M+H]+ = 477.1
   (148) 2-(4-Chlorphenylamino)-4-(trans-4-carboxy-cyclohexylamino)-5-nitro-HPLC/MS (Methode A): RT = 6.86min.; [M+H]+ = 392.1
(149) 2-(4-Chlor-phenylamino)-4-(*(2R)*-2-hydroxymethyl-pyrrolidin-1-yl)-5-nitro-pyrimidin
   HPLC/MS (Methode A): RT = 6.59min.; [M+H]+ = 350.1
   Hergestellt unter Verwendung von D-Prolinol.
(150) 2-(4-Carboxy-phenylamino)-4-(3-(2-oxo-pyrrolidin-1-yl)-propyl-1-amino)-5-nitro-pyrimidin
   HPLC/MS (Methode A): RT = 5.57min.; [M+H]+ = 401.1

(151) 2-(4-Chlor-phenylamino)-4-(2-morpholino-1-ethylamino)-5-nitro-pyrimidinHPLC/MS
   (Methode B): RT = 2,16 min.; [M+H]+ = 379,1
(152) 2-(2-Naphthylamino)-4-(1,4,6,7-tetrahydro-imidazo[4,5-c]pyridin-5-yl)-5-trifluormethyl-pyrimidin
   HPLC/MS (Methode A): RT = 5.66min.; [M+H]+ = 411.2
(153) 2-(4-Chlor-phenylamino)-4-(2-(1H-imidazol-4-yl)-ethylamino)-5-nitro-pyrimidinHPLC/MS
   (Methode B): RT = 2,21 min.; [M+H]+ = 360,1
(154) 2-(4-Chlor-phenylamino)-4-(2-(4-hydroxy-phenyl)-2-hydroxy-1-ethylamino)-5-nitro-pyrimidin
   HPLC/MS (Methode B): RT = 2,81 min.; [M+H]+ = 402,1
(155) 2-(4-Chlor-phenylamino)-4-(2-(4-hydroxy-3-methoxy-phenyl)-2-hydroxy-1-ethylamino)-5-nitro-pyrimidin
   HPLC/MS (Methode B): RT = 2,68 min.; [M+H]+ = 432,2
(156) 2-(4-Chlor-phenylamino)-4-(2-(1-methyl-2-pyrrolidinyl)-1-ethylamino)-5-nitro-pyrimidin
   HPLC/MS (Methode B): RT = 2,29 min.; [M+H]+ = 377,2
(157) 2-(4-Chlor-phenylamino)-4-(4-hydroxy-butylamino)-5-nitro-pyrimidin
   Schmelzpunkt: 178-182°C
(158) 2-(4-Chlor-phenylamino)-4-(6-hydroxy-1-hexylamino)-5-nitro-pyrimidin
   HPLC/MS (Methode B): RT = 2,96 min.; [M+H]+ = 366,2
(159) 2-(4-Chlor-phenylamino)-4-(5-hydroxy-1-pentylamino)-5-nitro-pyrimidin
   HPLC/MS (Methode B): RT = 2,97 min.; [M+H]+ = 352,2
(160) 2-(4-Chlor-phenylamino)-4-(1,1-dimethyl-2-hydroxy-1-ethylamino)-5-nitro-pyrimidin
   HPLC/MS (Methode B): RT = 2,66 min.; [M+H]+ = 338,17
(161) 2-(4-Carboxyphenylamino)-4-(2-(3-hydroxy-phenyl)-1-ethylamino)-5-nitro-pyrimidin
   HPLC/MS (Methode B): RT = 2,4 min.; [M+H]+ = 396,3
(162) 2-(Benzylamino)-4-(4-aminomethyl-cyclohexylmethylamino)-5-nitro-pyrimidin
   Schmelzpunkt: 169°C
(163) 2-(Benzylamino)-4-(4-aminomethyl-cyclohexylmethylamino)-5-trifluormethyl-pyrimidin
   Schmelzpunkt: 119°C
(164) 2-(4-Carboxyphenylamino)-4-(2-(4-hydroxy-phenyl)-1-ethylamino)-5-nitro-pyrimidin
   HPLC/MS (Methode B): RT = 2,36 min.; [M+H]+ = 396,2
(165) 2-(2-Chlorbenzylamino)-4-(4-aminomethyl-cyclohexylmethylamino)-5-nitro-pyrimidin
   Schmelzpunkt: 189°C
(166) 2-(3-Carboxyphenylamino)-4-(2-(4-hydroxy-phenyl)-1-ethylamino)-5-nitro-pyrimidin
   HPLC/MS (Methode B): RT = 2,34 min.; [M+H]+ = 396,2
(167) 2-(4-Chlor-phenylamino)-4-(4-aminomethyl-cyclohexylmethylamino)-5-nitro-pyrimidin
(168) 2-(3-Carboxyphenylamino)-4-(2-(3-hydroxy-phenyl)-1-ethylamino)-5-nitro-pyrimidin
   HPLC/MS (Methode B): RT = 2,41 min.; [M+H]+ = 396,3
(169) 2-(4-Chlor-phenylamino)-4-(2-(imidazolidin-2-on-1-yl)-1-ethylamino)-5-nitro-pyrimidin
   HPLC/MS (Methode B): RT = 2,5 min.; [M+H]+ = 378,2
(170) 2-(4-Brom-phenylamino)-4-(2-(1*H*-imidazol-4-yl)-1-ethylamino)-5-trifluormethyl-pyrimidin
   HPLC/MS (Methode B): RT = 2 min.; [M+H]+ = 427,1
(171) 2-(3-Brom-phenylamino)-4-(4-aminomethyl-cyclohexylmethylamino)-5-nitro-pyrimidin
   HPLC/MS (Methode B): RT = 2,56 min.; [M+H]+ = 435,2
(172) 2-(4-Brom-phenylamino)-4-(4-aminomethyl-cyclohexylmethylamino)-5-nitro-pyrimidin
   HPLC/MS (Methode B): RT = 2,66 min.; [M+H]+ = 435,2
(173) 2-(4-Chlor-phenylamino)-4-(1-methyl-4-piperidinyl-amino)-5-nitro-pyrimidin
   HPLC/MS (Methode B): RT = 2,24 min.; [M+H]+ = 363,2
(174) 2-(4-Chlor-phenylamino)-4-(6-amino-1-hexylamino)-5-nitro-pyrimidin
   HPLC/MS (Methode B): RT = 2,46 min.; [M+H]+ = 365,2
(175) 2-(4-Chlor-phenylamino)-4-(3-aminomethyl-cyclohexylmethylamino)-5-nitro-pyrimidin
   HPLC/MS (Methode B): RT = 2,56 min.; [M+H]+ = 391,2
(176) 2-(4-Benzylaminocarbonyl-phenylamino)-4-(4-acetyl-1-piperazinyl)-5-nitro-pyrimidin
   HPLC/MS (Methode B): RT = 2,53 min.; [M+H]+ = 476,2
(177) 2-(3-Carboxy-phenylamino)-4-(4-aminosulfonyl-benzylamino)-5-nitro-pyrimidin
   HPLC/MS (Methode B): RT = 2,31 min.; [M+H]+ = 445,1
(178) 2-(4-Chlor-phenylamino)-4-[N-(1-methyl-4-piperidinyl-methyl)-N-methyl-amino]-5-nitro-pyrimidin
   HPLC/MS (Methode B): RT = 2,31 min.; [M+H]+ = 391,2
(179) 2-(3-Carboxy-phenylamino)-4-(4-aminomethyl-cyclohexylmethylamino)-5-nitro-pyrimidin (Gemisch der Isomeren)
   HPLC/MS (Methode B): RT = 1,97 min.; [M+H]+ = 401,2
(180) 2-(3-Ethoxycarbonyl-phenylamino)-4-(4-aminomethyl-cyclohexylmethylamino)-5-nitro-pyrimidin (Gemisch der Isomeren)
   HPLC/MS (Methode B): RT = 2,42 min.; [M+H]+ = 429,3
(181) 2-(4-Benzylaminocarbonyl-phenylamino)-4-(4-carboxy-4-phenyl-1-piperidinyl)-5-nitro-pyrimidin
   HPLC/MS (Methode B): RT = 3,11 min.; [M+H]+ = 553,3
(182) 2-(3,4-Dichlorphenylamino)-4-(2-(3-carboxy-2-methoxy-phenyl)-1-ethylamino)-5-trifluormethyl-pyrimidin
   HPLC/MS (Methode B): RT = 2,96 min.; [M+H]+ = 501,2
(183) 2-(4-Chlorphenylamino)-4-(7-methyl-2,7-diaza-spiro[4.4]non-2-yl)-5-nitro-pyrimidin
   HPLC/MS (Methode B): RT = 2,34 min.; [M+H]+ = 389,2
(184) 2-(4-Carboxy-phenylamino)-4-(4-hydroxy-benzylamino)-5-nitro-pyrimidin
   HPLC/MS (Methode B): RT = 2,2 min.; [M+H]+ = 382,1
(185) 2-(3,4-Dichlorphenylamino)-4-(3-carboxy-cyclohexylamino)-5-trifluormethyl-pyrimidin
   HPLC/MS (Methode B): RT = 2,9 min.; [M+H]+ = 449,2
(186) 2-(3,4-Dichlorphenylamino)-4-(4-aminomethyl-cyclohexylmethylamino)-5-trifluormethyl-pyrimidin
   HPLC/MS (Methode B): RT = 2,35 min.; [M+H]+ = 448,1
(187) 2-(3,4-Dichlorphenylamino)-4-(1-carboxy-2,2-diphenyl-1-ethylamino)-5-trifluormethyl-pyrimidin
   HPLC/MS (Methode B): RT = 3,17 min.; [M+H]+ = 547,28
(188) 2-(3,4-Dichlorphenylamino)-4-(3-aminomethyl-cyclohexylmethylamino)-5-trifluormethyl-pyrimidin
   HPLC/MS (Methode B): RT = 2,15 min.; [M+H]+ = 448,0
   Schmelzpunkt: 140-142 °C
   R_{f}= 0,08 (Kieselgel; Methylenchlorid: Methanol = 4:1)
   HPLC/MS (Methode G): RT = 3,78 min.; [M+H]+ = 449; Abs. λ max = 260,8 nm
(189) 2-(Benzylamino)-4-(2-(3-hydroxyphenyl)-1-ethylamino)-5-trifluormethyl-pyrimidin
(190) 2-(4-Chlor-phenylamino)-4-(3-hydroxy-piperidin-1-yl)-5-nitro-pyrimidin
   HPLC/MS (Methode B): RT = 2,9 min.; [M+H]+ = 350,2
(191) 2-(4-Chlor-phenylamino)-4-(trans-4-hydroxy-cyclohexylamino)-5-nitro-pyrimidin
   HPLC/MS (Methode B): RT = 2,92 min.; [M+H]+ = 364,2
(192) 2-(4-Chlor-phenylamino)-4-(4-amino-cyclohexylamino)-5-nitro-pyrimidin
   HPLC/MS (Methode B): RT = 2,4 min.; [M+H]+ = 393,2
(193) 2-(4-Chlor-phenylamino)-4-(4-dimethylamino-cyclohexylamino)-5-nitro-pyrimidin
   HPLC/MS (Methode B): RT = 2,36 min.; [M+H]+ = 391,2
(194) 2-(4-Chlor-phenylamino)-4-((*1R*)-1-carboxy-1-ethylamino)-5-nitro-pyrimidin
   HPLC/MS (Methode B): RT = 2,51 min.; [M+H]+= 338,14
(195) 2-(4-Chlor-phenylamino)-4-(3-amino-propylamino)-5-nitro-pyrimidin
   HPLC/MS (Methode B): RT = 2,15 min.; [M+H]+ = 323,1
(196) 2-(4-Chlor-phenylamino)-4-(3-(3-aminopropoxy-1-propylamino)-5-nitro-pyrimidin
(197) 2-(4-Chlor -phenylamino)-4-(4-aminomethyl-piperidin-1-yl)-5-nitro-pyrimidin
(198) 2-(4-Chlor-phenylamino)-4-(3-(isopropylamino)-1-propylamino)-5-nitro-pyrimidin
   HPLC/MS (Methode B): RT = 2,32 min.; [M+H]+ = 365,1
(199) 2-(4-Chlor-phenylamino)-4-(2-(4-(2-hydroxyethyl)-piperazin-1-yl)-1-ethylamino))-5-nitro-pyrimidin

(200) 2-(4-Chlor-phenylamino)-4-(3-(3-hydroxy-8-methyl-8-aza-blcyclo[3.2.1]octyl)-methylamino)-5-nitro-pyrimidin
   HPLC/MS (Methode B): RT = 2,17 min.; [M+H]+ = 419,2
(201) 2-(4-Chlor-phenylamino)-4-(3-hydroxy-1,3-dihydro-2-oxo-indol-3-yl-methylamino)-5-nitro-pyrimidin
(202) 2-(4-Chlor-phenylamino)-4-(2-(3-carboxy-2-methoxy-phenyl)-1-ethylamino)-5-nitro-pyrimidin
   HPLC/MS (Methode B): RT = 3,2 min.; [M+H]+ = 444,1
(203) 2-(4-Chlor-phenylamino)-4-(3-hydroxymethyl-piperidin-1-yl)-5-nitro-pyrimidin
   HPLC/MS (Methode B): RT = 2,97 min.; [M+H]+ = 364,2
(204) 2-(4-Chlor-phenylamino)-4-(bis-(2-hydroxy-ethyl)-amino)-5-nitro-pyrimidin
   HPLC/MS (Methode B): RT = 2,41 min.; [M+H]+ = 354,1
(205) 2-(4-Chlor-phenylamino)-4-(4-nitrobenzylamino)-5-nitro-pyrimidin
   HPLC/MS (Methode B): RT = 3,49 min.; [M+H]+ = 401,2
(206) 2-(4-Chlor-phenylamino)-4-(2-aminocarbonyl-1-ethylamino)-5-nitro-pyrimidin
(207) 2-(4-Chlor-phenylamino)-4-(1-carboxy-2-(1H-imidazol-4-yl)-1-ethylamino)-5-nitro-pyrimidin
   HPLC/MS (Methode B): RT = 1,67 min.; [M+H]+ = 404,15
(208) 2-(4-Chlor-phenylamino)-4-(1-carboxy-2,2-diphenyl-1-ethylamino)-5-nitro-pyrimidin
   HPLC/MS (Methode B): RT = 3,25 min.; [M+H]+ = 490,2
(209) 2-(4-Chlor-phenylamino)-4-(3-carboxy-cyclohexylamino)-5-nitro-pyrimidin
   HPLC/MS (Methode B): RT = 3,15 min.; [M+H]+ = 392,2
(210) 2-(4-Chlor-phenylamino)-4-(4-(2-carboxy-1-ethyl)-cyclohexylamino)-5-nitro-pyrimidin
   HPLC/MS (Methode B): RT = 3,41 min.; [M+H]+ = 420,3
(211) 2-(4-Chlor-phenylamino)-4-(2-(3-hydroxyphenyl)-1-ethylamino)-5-nitro-pyrimidin
   HPLC/MS (Methode B): RT = 3,29 min.; [M+H]+ = 386,2
(212) 2-(4-Chlor-phenylamino)-4-(2-(1H-pyrazol-4-yl)-1-ethylamino)-5-nitro-pyrimidin
   HPLC/MS (Methode B): RT = 2,38 min.; [M+H]+ = 360,1
(213) 2-(4-Chlor-phenylamino)-4-(3-(2-aza-bicyclo[2.2.1]hept-5-en-2-yl)-propylamino)-5-nitro-pyrimidin
(214) 2-(4-Chlor-phenylamino)-4-(1-methyl-piperidin-4-yl-methylamino)-5-nitro-pyrimidin
   HPLC/MS (Methode B): RT = 2,28 min.; [M+H]+ = 377,2
(215) 2-(4-Chlor-phenylamino)-4-(cis-4-hydroxy-cyclohexylamino)-5-nitro-pyrimidin
   HPLC/MS (Methode B): RT = 2,92 min.; [M+H]+ = 364,2
(216) 2-(4-Chlor-phenylamino)-4-(4-dimethylaminomethyl-cyclohexylmethylamino)-5-nitro-pyrimidin
   HPLC/MS (Methode B): RT = 2,15 min.; [M+H]+ = 357,1
(217) 2-(4-Chlor-phenylamino)-4-(2-(imidazol-1-yl)-1-ethylamino)-5-nitro-pyrimidin
   HPLC/MS (Methode B): RT = 2,26 min.; [M+H]+ = 371,1
(218) 2-(3,4-Dichlor-phenylamino)-4-(6-amino-1-hexylamino)-5-trifluormethyl-pyrimidin
   HPLC/MS (Methode B): RT = 2,31 min.; [M+H]+ = 422,1
(219) 2-(3,4-Dichlor-phenylamino)-4-(N-(1-methyl-piperidin-4-yl)-N-methyl-amino)-5-trifluormethyl-pyrimidin
   HPLC/MS (Methode B): RT = 2,46 min.; [M+H]+ = 434,1
(220) 2-(3,4-Dichlor-phenylamino)-4-(N-methyl-N-(2-hydroxybenzyl)-amino)-5-trifluormethyl-pyrimidin
   HPLC/MS (Methode B): RT = 3,3 min.; [M+H]+ = 443,1
(221) 2-(3,4-Dichlorphenylamino)-4-(N-methyl-N-(2-cyano-1-ethyl)-amino)-5-trifluormethyl-pyrimidin
   HPLC/MS (Methode B): RT = 3,27 min.; [M+H]+ = 390,1
(222) 2-(3,4-Dichlorphenylamino)-4-(3-(4-(1-pyrrolidinyl)-butyl)-piperidin-1-yl)-5-trifluormethyl-pyrimidin
   HPLC/MS (Methode B): RT = 2,87 min.; [M+H]+ = 516,1
(223) 2-(3,4-Dichlorphenylamino)-4-(*(2S)*-2-hydrooymethyl-pyrrolidin-1-yl)-5-trifluormethyl-pyrimidin
   HPLC/MS (Methode B): RT = 2,85 min.; [M+H]+ = 407,2
(224) 2-(3,4-Dichlorphenylamino)-4-(*(2R)*-2-hydroxymethyl-pyrrolidin-1-yl)-5-trifluormethyl - pyrimidin
   HPLC/MS (Methode B): RT = 2,62 min.; [M+H]+ = 407,2
(225) 2-(3,4-Dichlorphenylamino)-4-(2-(imidazolidin-2-on-1-yl)-1-ethylamino)-5-trifluormethyl - pyrimi din
   HPLC/MS (Methode B): RT = 2,49 min.; [M+H]+ = 435,1
(226) 2-(3,4-Dichlorphenylamino)-4-(4-(N-acetyl-N-methyl-aminomethyl)-piperidin-1-yl)-5-trifluormethyl-pyrimidin
   HPLC/MS (Methode B): RT = 2,59 min.; [M+H]+ = 476,3
(227) 2-(3,4-Dichlorphenylamino)-4-[N-(1-methyl-4-piperidinyl-methyl)-N-methyl-amino]-5-trifluormethyl-pyrimidin
   HPLC/MS (Methode B): RT = 2,4 min.; [M+H]+ = 434,2
(228) 2-(3,4-Dichlorphenylamino)-4-(4-methylpiperazino)-5-trifluormethyl-pyrimidin
   HPLC/MS (Methode B): RT = 2,41 min.; [M+H]+ = 406,2
(229) 2-(3,4-Dichlorphenylamino)-4-(4-hydroxy-1-piperidinyl)-5-trifluormethyl-pyrimidin
   HPLC/MS (Methode B): RT = 2,63 min.; [M+H]+ = 407,2
(230) 2-(3,4-Dichlorphenylamino)-4-(2-dimethylamino-1-ethylamino)-5-trifluormethyl-pyrimidin
   HPLC/MS (Methode B): RT = 2,24 min.; [M+H]+ = 394,1
(231) 2-(3,4-Dichlorphenylamino)-4-(3-(4-morpholinyl)-1-propylamino)-5-trifluormethyl-pyrimidin
   HPLC/MS (Methode B): RT = 2,15 min.; [M+H]+ = 450,1
(232) 2-(3,4-Dichlorphenylamino)-4-(2-carboxy-1-ethylamino)-5-trifluormethyl-pyrimidin
   HPLC/MS (Methode B): RT = 2,34 min.; [M+H]+ = 395,2
(233) 2-(3,4-Dichlorphenylamino)-4-(3-(1H-1-imidazolyl)-1-propylamino)-5-trifluormethyl-pyrimidin
   HPLC/MS (Methode B): RT = 2,01 min.; [M+H]+ = 431,1
(234) 2-(3,4-Dichlorphenylamino)-4-(3-dimethylamino-1-propylamino)-5-trifluormethyl-pyrimidin
   HPLC/MS (Methode B): RT = 1,87 min.; [M+H]+ = 408,1
(235) 2-(3,4-Dichlorphenylamino)-4-(2-diisopropylamino-1-ethylamino)-5-trifluormethyl-pyrimidin
   HPLC/MS (Methode B): RT = 2,5 min.; [M+H]+ = 450,3
(236) 2-(3,4-Dichlorphenylamino)-4-(bis-(2-methoxyethyl)amino)-5-trifluormethyl-pyrimidin
   HPLC/MS (Methode B): RT = 3,44 min.; [M+H]+ = 439,2
(237) 2-(3,4-Dichlorphenylamino)-4-(N-methyl-N-(2-methylamino-1-ethyl)-amino)-5-trifluormethyl-pyrimidin
   HPLC/MS (Methode B): RT = 2,56 min.; [M+H]+ = 394,0
(238) 2-(3,4-Dichlorphenylamino)-4-(2-(4-pyridyl)-1-ethylamino)-5-trifluormethyl-pyrimidin
   HPLC/MS (Methode B): RT = 2,17 min.; [M+H]+ = 428,1
(239) 2-(3,4-Dichlorphenylamino)-4-(4-aminosulfonyl-benzylamino)-5-trifluormethyl-pyrimidin
   HPLC/MS (Methode B): RT = 2,72 min.; [M+H]+ = 492,2
(240) N2-(3,4-dichloro-phenyl)-N4-(2-methylamino-ethyl)-5-trifluormethyl-pyrimidine-2,4-diamin
   Die Herstellung erfolgt analog Beispiel 1(80).Schmelzpunkt: 147 °C
   R_{f}= 0,12 (Kieselgel; Methylenchlorid: Methanol = 4:1)
   HPLC/MS (Methode G): RT = 3,76 min.; [M+H]+ = 381; Abs. λ max = 270,3 nm
(241) 2-(3,4-Dichlorphenylamino)-4-(4-pyridyl-methylamino)-5-trifluormethyl-pyrimidin
   HPLC/MS (Methode B): RT = 2,31 min.; [M+H]+ = 414,2
(242) 2-(3,4-Dichlorphenylamino)-4-((3-chlor-5-trifluormethyl-2-pyridyl)-methylamino)-5-trifluormethyl-pyrimidin
(243) 2-(3,4-Dichlorphenylamino)-4-((4-ethoxycarbonyl-1*H*-pyrazol-5-yl)-methylamino)-5-trifluormethyl-pyrimidin
(244) 2-(3,4-Dichlorphenylamino)-4-(3-nitrobenrylamino)-5-trifluormethyl-pyrimidin
   HPLC/MS (Methode B): RT = 3,3 min.; [M+H]+ = 458,2
(245) 2-(3,4-Dichlorphenylamino)-4-(4-(2-carboxy-1-ethyl)-1-piperidinyl)-5-trifluormethyl-pyrimidin
   HPLC/MS (Methode B): RT = 3,11 min.; [M+H]+ = 463,2
(246) 2-(3,4-Dichlorphenylamino)-4-(3-(1-pyrrolidinyl)-1-propylamino)-5-trifluormethyl-pyrimidin
   HPLC/MS (Methode B): RT = 2,26 min.; [M+H]+ = 434,1
(247) 2-(3,4-Dichlorphenylamino)-4-(5-acetylamino-5-methoxycarbonyl-1-pentylamino)-5-trifluormethyl-pyrimidin
   HPLC/MS (Methode B): RT = 2,4 min.; [M+H]+ = 508,2
(248) 2-(3,4-Dichlorphenylamino)-4-((1-hydroxy-1-cyclohexyl)-methylamino)-5-trifluormethyl-pyrimidin
   HPLC/MS (Methode B): RT = 3,16 min.; [M+H]+ = 435,1
(249) 2-(3,4-Dichlorphenylamino)-4-(2-(1*H*-indol-3-yl)-1-ethylamino)-5-trifluormethyl-pyrimidin
   HPLC/MS (Methode B): RT = 3,26 min.; [M+H]+ = 466,2

(250) 2-(3,4-Dichlorphenylamino)-4-(2-(4-nitro-2-pyridyl-amino)-1-ethylamino)-5-trifluormethyl-pyrimidin
   HPLC/MS (Methode B): RT = 2,99 min.; [M+H]+ = 488,1
(251) 2-(3,4-Dichlorphenylamino)-4-(2-hydroxy-2-phenyl-1-ethylamino)-5-trifluormethyl-pyrimidin
   HPLC/MS (Methode B): RT = 3,02 min.; [M+H]+ = 443,1
(252) 2-(3,4-Dichlorphenylamino)-4-(2-phenylamino-1-ethylamino)-5-trifluormethyl-pyrimidin
   HPLC/MS (Methode B): RT = 3,16 min.; [M+H]+ = 442,2
(253) 2-(3,4-Dichlorphenylamino)-4-(2-(4-hydroxyphenyl)-1-ethylamino)-5-trifluormethyl-pyrimidin
   HPLC/MS (Methode B): RT = 2,91 min.; [M+H]+ = 443,2
(254) 2-(3,4-Dichlorphenylamino)-4-(2-(4-aminosulfonylphenyl)-1-ethylamino)-5-trifluormethyl-pyrimidin
   HPLC/MS (Methode B): RT = 2,74 min.; [M+H]+ = 506,1
(255) 2-(3,4-Dichlorphenylamino)-4-(2-(1-naphthylamino)-1-ethylamino)-5-trifluormethyl-pyrimidin
   HPLC/MS (Methode B): RT = 3,6 min.; [M+H]+ = 492,2
(256) 2-(3,4-Dichlorphenylamino)-4-(2-(4-nitrophenyl)-1-ethylamino)-5-trifluormethyl-pyrimidin
   HPLC/MS (Methode B): RT = 3,33 min.; [M+H]+ = 472,2
(257) 2-(3,4-Dichlorphenylamino)-4-(3-ethoxycarbonyl-1-propylamino)-5-trifluormethyl-pyrimidin
   HPLC/MS (Methode B): RT = 3,14 min.; [M+H]+ = 437,2
(258) 2-(3,4-Dichlorphenylamino)-4-(aminocarbonylmethylamino)-5-trifluormethyl-pyrimidin
   HPLC/MS (Methode B): RT = 2,33 min.; [M+H]+ = 380,1
(259) 4-[4-(2-Amino-ethylamino)-5-trifluormethyl-pyrimidin-2-ylamino]-benzamid
   Schmelzpunkt: 211-213 °C
   R_{f}= 0,04 (Kieselgel; Methylenchlorid: Methanol = 4:1)
   HPLC/MS (Methode F): RT = 2,79 min.; [M+H]+ = 341; Abs. λ max = 277,9 nm
(260) 2-(3,4-Dichlorphenylamino)-4-(2-tert.-butyloxycarbonylamino-1-ethylamino)-5-trifluormethyl-pyrimidin
   HPLC/MS (Methode B): RT = 2,96 min.; [M+H]+ = 466,1
(261) 2-(3,4-Dichlorphenylamino)-4-(1-ethyl-2-pyrrolidinyl-methylamino)-5-trifluormethyl-pyrimidin
   HPLC/MS (Methode B): RT = 2,52 min.; [M+H]+ = 434,2
(262) 2-(3,4-Dichlorphenylamino)-4-(2-(1-pyrrolidinyl)-1-ethylamino)-5-trifluormethyl-pyrimidin
   HPLC/MS (Methode B): RT = 2,33 min.; [M+H]+ = 420,2
(263) 2-(3,4-Dichlorphenylamino)-4-(2-tetrahydrofuryl-methylamino)-5-trifluormethyl-pyrimidin
(264) 2-(3,4-Dichlorphenylamino)-4-(2-(1-piperidinyl)-1-ethylamino)-5-trifluormethyl-pyrimidin
   HPLC/MS (Methode B): RT = 2,4 min.; [M+H]+ = 434,2
(265) 2-(3,4-Dichlorphenylamino)-4-(2-hydroxy-1-propylamino)-5-trifluormethyl-pyrimidin
   HPLC/MS (Methode B): RT = 2,63 min.; [M+H]+ = 381,1
(266) 2-(3,4-Dichlorphenylamino)-4-(2,3-dihydroxy-1-propylamino)-5-trifluormethyl-pyrimidin
   HPLC/MS (Methode B): RT = 2,31 min.; [M+H]+ = 397,2
(267) 2-(3,4-Dichlorphenylamino)-4-(2-diethylamino-1-ethylamino)-5-trifluormethyl-pyrimidin
   HPLC/MS (Methode B): RT = 2,39 min.; [M+H]+ = 422,1
(268) 2-(3,4-Dichlorphenylamino)-4-(2-(2-hydroxyethoxy)-1-ethylamino)-5-trifluormethyl-pyrimidin
   HPLC/MS (Methode B): RT = 2,5 min.; [M+H]+ = 411,2
(269) 2-(3,4-Dichlorphenylamino)-4-(2-hydroxy-1-ethylamino)-5-trifluormethyl-pyrimidin
   HPLC/MS (Methode B): RT = 2,46 min.; [M+H]+ = 3 67,2
(270) 2-(3,4-Dichlorphenylamino)-4-(3-diethylamino-1-propylamino)-5-trifluormethyl-pyrimidin
   HPLC/MS (Methode B): RT = 2,26 min.; [M+H]+ = 436,2
(271) 2-(3,4-Dichlorphenylamino)-4-(3-hydroxy-1-propylamino)-5-trifluormethyl-pyrimidin
   HPLC/MS (Methode B): RT = 2,49 min.; [M+H]+ = 3 81,2
(272) 2-(3,4-Dichlorphenylamino)-4-(2-(1-methyl-1H-pyrrol-2-yl)-1-ethylamino)-5-trifluormethyl-pyrimidin
   HPLC/MS (Methode B): RT = 3,23 min.; [M+H]+ = 430,1
(273) 2-(3,4-Dichlorphenylamino)-4-(4-hydroxy-3-methoxy-benzylamino)-5-trifluormethyl-pyrimidin
   HPLC/MS (Methode B): RT = 2,89 min.; [M+H]+ = 459,1
(274) 2-(3,4-Dichlorphenylamino)-4-(2-methylsulfanyl-1-ethylamino)-5-trifluormethyl-pyrimidin
   HPLC/MS (Methode B): RT = 3,17 min.; [M+H]+ = 397,1
(275) 2-(3,4-Dichlorphenylamino)-4-(3-methoxy-1-propylamino)-5-trifluormethyl-pyrimidin
   HPLC/MS (Methode B): RT = 3,06 min.; [M+H]+ = 395,2
(276) 2-(3,4-Dichlorphenylamino)-4-(2,2-dimethyl-3-dimethylamino-1-propylamino)-5-trifluormethyl-pyrimidin
   HPLC/MS (Methode B): RT = 2,41 min.; [M+H]+ = 436,1
(277) 2-(3,4-Dichlorphenylamino)-4-(2,2-dimethyl-3-hydroxy-1-propylamino)-5-trifluormethyl-pyrimi din
   HPLC/MS (Methode B): RT = 3,03 min.; [M+H]+ = 409,2
(278) 2-(3,4-Dichlorphenylamino)-4-cyanomethylamino-5-trifluormethyl-pyrimidin
   HPLC/MS (Methode B): RT = 2,66 min.; [M+H]+ = 362,2
(279) 2-(3,4-Dichlorphenylamino)-4-(3-aminocarbonyl-1-piperidinyl)-5-trifluormethyl-pyrimidin
   HPLC/MS (Methode B): RT = 2,6 min.; [M+H]+ = 434,2
(280) 2-(3,4-Dichlorphenylamino)-4-(4-acetyl-1-piperazinyl)-5-trifluormethyl-pyrimidin
   HPLC/MS (Methode B): RT = 2,81 min.; [M+H]+ = 434,2
(281) 2-(3,4-Dichlorphenylamino)-4-(4-(1-piperidinyl)-1-piperidinyl)-5-trifluormethyl-pyrimidin
   HPLC/MS (Methode B): RT = 2,45 min.; [M+H]+ = 474,2
(282) 2-(3,4-Dichlorphenylamino)-4-(4-(morpholinocarbonylmethyl)-1-piperazinyl)-5-trifluormethyl-pyrimidin
   HPLC/MS (Methode B): RT = 2,41 min.; [M+H]+ = 519,2
(283) 2-(3,4-Dichlorphenylamino)-4-piperazino-5-trifluormethyl-pyrimidin
   HPLC/MS (Methode B): RT = 2,31 min.; [M+H]+ = 392,2
(284) 2-(3,4-Dichlorphenylamino)-4-(3-(4-(3-amino-1-propyl)-1-piperazinyl)-1-propylamino)-5-trifluormethyl-pyrimidin
   HPLC/MS (Methode B): RT = 1,7 min.; [M+H]+ = 506,2
(285) 2-(3,4-Dichlor-phenylamino)-4-(cis-4-carboxy-cyclohexylamino)-5-trifluormethyl-pyrimidin
(286) 2-(3,4-Dichlorphenylamino)-4-(3-dibenzylamino-1-propylamino)-5-trifluormethyl-pyrimidin
   HPLC/MS (Methode B): RT = 2,65 min.; [M+H]+ = 560,1
(287) 2-(3,4-Dichlor-phenylamino)-4-(N-[4-methoxycarbonyl-cyclohexyl]-N-[3-pyridylmethyl]amino)-5-trifluormethyl-pyrimidin
   HPLC/MS (Methode B): RT = 2,5 min.; [M+H]+ = 554,1
(288) 2-(3,4-Dichlorphenylamino)-4-(2-acetylamino-ethylamino)-5-methoxymethyl-pyrimidin
(289) 2-(3,4-Dichlorphenylamino)-4-(2-phenyl-1-(4-phenyl-1-butyl-aminocarbonyl)-1-ethylamino)-5-trifluormethyl-pyrimidin
   HPLC/MS (Methode B): RT = 3,73 min.; [M+H]+ = 602,28
(290) 2-(3,4-Dichlorphenylamino)-4-(1-aminocarbonyl-2-(4-methoxyphenyl)-1-ethylamino)-5-trifluormethyl-pyrimidin
   HPLC/MS (Methode B): RT = 2,61 min.; [M+H]+ = 500,24
(291) 2-(3,4-Dichlorphenylamino)-4-(1-dimethylaminomethylcarbonyl-4-piperidinyl-methylamino)-5-trifluormethyl-pyrimidin
(292) 2-(3,4-Dichlor-phenylamino)-4-(N-ethyl-N-(4-pyridylmethyl)-amino)-5-trifluormethyl-pyrimidin
   HPLC/MS (Methode B): RT = 2,66 min.; [M+H]+ = 435,2
(293) 2-(3,4-Dichlor-phenylamino)-4-(3-phenyl-azepan-4-on-1-yl)-5-trifluormethyl-pyrimidin
   HPLC/MS (Methode B): RT = 2,49 min.; [M+H]+ = 381,2
(294) 2-(3,4-Dichlorphenylamino)-4-(2-(3-hydroxy-1-propyl)-1-piperidinyl)-5-trifluormethyl-pyrimidin
   HPLC/MS (Methode B): RT = 3,2 min.; [M+H]+ = 449,0
(295) 2-(3,4-Dichlorphenylamino)-4-(4-(8-methoxy-3,4-dihydro-*1H*-quinazlin-2-on-3-yl)-1-piperidinyl)-5-trifluormethyl-pyrimidin
   HPLC/MS (Methode B): RT = 3,1 min.; [M+H]+ = 567,08
(296) 4-{4-(2-(1-Methyl-pyrrolidin-2-yl)-ethylamino]-5-trifluormethyl-pyrimidin-2-ylamino}-benzamid
   Schmelzpunkt: 228-229°C
   R_{f}= 0,07 (Kieselgel; Methylenchlorid: Methanol = 4:1)
   HPLC/MS (Methode G): RT = 2,61 min.; [M+H]+ = 409; Abs. λ max = 276 nm
(297) 2-(3,4-Dichlorphenylamino)-4-(4-(3,4-dimethoxyphenyl)-1-piperazinyl)-5-trifluormethyl-pyrimidin
   HPLC/MS (Methode B): RT = 3,02 min.; [M+H]+ = 443,1
(298) 2-(3,4-Dichlorphenylamino)-4-(4-(4-cyanophenyl)-1-piperazinyl)-5-trifluormethyl-pyrimidin
   HPLC/MS (Methode B): RT = 2,41 min.; [M+H]+ = 396,3
(299) 2-(3,4-Dichlorphenylamino)-4-(1-benzyl-3-pyrrolidinyl-amino)-5-trifluormethyl-pyrimidin
   HPLC/MS (Methode B): RT = 2,54 min.; [M+H]+ = 482,2

(300) 2-(3,4-Dichlorphenylamino)-4-(1-hydroxy-2-propylamino)-5-trifluormethyl-pyrimidin
   HPLC/MS (Methode B): RT = 2,48 min.; [M+H]+ = 381,2
(301) 2-(3,4-Dichlorphenylamino)-4-(3-(1-piperidinyl)-1-propylamino)-5-trifluormethyl-pyrimidin
   HPLC/MS (Methode B): RT = 2,08 min.; [M+H]+ = 448,1
(302) 2-(3,4-Dichlorphenylamino)-4-(1-benzyl-4-piperidinyl-amino)-5-trifluormethyl-pyrimidin
   HPLC/MS (Methode B): RT = 2,41 min.; [M+H]+ = 496,2
(303) 2-(3,4-Dichlorphenylamino)-4-(4-aminomethyl-benzylamino)-5-trifluormethyl-pyrimidin
   HPLC/MS (Methode B): RT = 2,14 min.; [M+H]+ = 442,1
(304) 2-(3,4-Dichlorphenylamino)-4-(4-aminobutylamino)-5-trifluormethyl-pyrimidin
   HPLC/MS (Methode B): RT = 2,11 min.; [M+H]+ = 394,1
(305) 2-(3,4-Dichlorphenylamino)-4-(3-amino-2,2-dimethyl-1-propylamino)-5-trifluormethyl-pyrimidin
   HPLC/MS (Methode B): RT = 2,4 min.; [M+H]+ = 408,1
(306) 2-(3,4-Dichlorphenylamino)-4-(trans-2-amino-cyclohexylamino)-5-trifluormethyl-pyrimidin
   HPLC/MS (Methode B): RT = 2,61 min.; [M+H]+ = 420,1
(307) 2-(3,4-Dichlorphenylamino)-4-(2-(2-(2-amino-1-ethoxy)-1-ethoxy)-1-ethylamino)-5-trifluormethyl-pyrimidin
   HPLC/MS (Methode B): RT = 2,25 min.; [M+H]+ = 454,0
(308) 2-(3,4-Dichlorphenylamino)-4-(3-amino-benzylamino)-5-trifluormethyl-pyrimidin
   HPLC/MS (Methode B): RT = 2,16 min.; [M+H]+= 428,2
(309) 2-(3,4-Dichlorphenylamino)-4-(3-amino-2-hydroxy-1-propylamino)-5-trifluormethyl-pyrimidin
   HPLC/MS (Methode B): RT = 2,11 min.; [M+H]+ = 396,1
(310) 2-(3,4-Dichlorphenylamino)-4-(2-(2-amino-1-ethylsulfanyl)-1-ethylamino)-5-trifluormethyl-pyrimidin
   HPLC/MS (Methode B): RT = 2,28 min.; [M+H]+ = 426,0
(311) 2-(3,4-Dichlorphenylamino)-4-(N-[2-dimethylamino-1-ethyl]-N-ethyl-amino)-5-trifluormethyl-pyrimidin
   HPLC/MS (Methode B): RT = 2,76 min.; [M+H]+ = 422,1
(312) 2-(3,4-Dichlorphenylamino)-4-(N-[3-dimethylamino-1-propyl]-N-methyl-amino)-5-trifluormethyl-pyrimidin
   HPLC/MS (Methode B): RT = 2,39 min.; [M+H]+ = 422,0
(313) 2-(3,4-Dichlorphenylamino)-4-(3-(4-methyl-1-piperazinyl)-1-propylamino)-5-trifluormethyl-pyrimidin
   HPLC/MS (Methode B): RT = 2,09 min.; [M+H]+ = 463,1
(314) 2-(3,4-Dichlor-phenylamino)-4-(N-[2-cyano-1-ethyl]-N-[3-pyridylmethyl]-amino)-5-trifluormethyl-pyrimidin
   HPLC/MS (Methode B): RT = 2,4 min.; [M+H]+ = 467,0
(315) 2-(3,4-Dichlor-phenylamino)-4-(4-(2-pyridyl)-1-piperazinyl)-5-trifluormethyl-pyrimidin
   HPLC/MS (Methode B): RT = 2,48 min.; [M+H]+ = 469,1
(316) 2-(3,4-Dichlor-phenylamino)-4-(4-[bis-(4-methoxy-phenyl)]-methyl-1-piperazinyl)-5-trifluormethyl-pyrimidin
(317) 2-(3,4-Dichlor-phenylamino)-4-(4-(3-methoxy-phenyl)-1-piperazinyl)-5-trifluormethyl-pyrimidin
   HPLC/MS (Methode B): RT = 2,4 min.; [M+H]+ = 396,3
(318) 2-(3,4-Dichlor-phenylamino)-4-(N-benzyl-N-[2-cyano-1-ethyl]-amino)-5-trifluormethyl-pyrimidin
   HPLC/MS (Methode B): RT = 3,92 min.; [M+H]+ = 466,2
(319) 2-(3,4-Dichlor-phenylamino)-4-(N-benzyl-N-[2-hydroxy-1-ethyl]-amino)-5-trifluormethyl-pyrimidin
   HPLC/MS (Methode B): RT = 3,4 min.; [M+H]+= 457,1
(320) 2-(3,4-Dichlorphenylamino)-4-(3-carboxy-1-propyl-amino)-5-trifluormethyl-pyrimidin
(321) 2-(3,4-Dichlor-phenylamino)-4-(N-benzyl-N-[ethoxycarbonylmethyl]-amino)-5-trifluormethyl-pyrimidin
   HPLC/MS (Methode B): RT = 4,11 min.; [M+H]+ = 499,2
(322) 2-(3,4-Dichlor-phenylamino)-4-(N-[4-nitrobenzyl]-N-propyl-amino)-5-trifluormethyl-pyrimidin
   HPLC/MS (Methode B): RT = 4,03 min.; [M+H]+ = 500,2
(323) 2-(3,4-Dichlor-phenylamino)-4-(cyano-phenyl-methylamino)-5-trifluormethyl-pyrimidin
(324) 2-(3,4-Dichlor-phenylamino)-4-(N-benzyl-N-[4-hydroxy-1-butyl]-amino)-5-trifluormethyl-pyrimidin
   HPLC/MS (Methode B): RT = 3,43 min.; [M+H]+ = 485,2
(325) 2-(3,4-Dichlor-phenylamino)-4-(N-benzyl-N-[2-hydroxymethyl-1-cyclohexyl]-amino)-5-trifluormethyl-pyrimidin
   HPLC/MS (Methode B): RT = 2,8 min.; [M+H]+ = 525,26
(326) N-1-[2-(3,4-Dichloro-phenylamino)-5-trifluormethyl-pyrimidin-4-yl]-pyrrolidin-3-yl)-carbaminsäure-tert-butylester
   Die Herstellung erfolgt analog Beispiel 1(80)
   Schmelzpunkt: 198-200°C
   R_{f}= 0,44 (Kieselgel; Methylenchlorid: Methanol = 99:1)
   HPLC/MS (Methode K): RT = 4,37 min.; [M+H]+ = 493; Abs. λ max = 270,3 nm
(327) 2-(3,4-Dichlor-phenylamino)-4-(*(1S)*-1-carboxy-2-hydroxy-1-ethyl-amino)-5-trifluormethyl-pyrimidin
   HPLC/MS (Methode B): RT = 2 min.; [M+H]+ = 411
(328) 2-(3,4-Dichlor-phenylamino)-4-(5-carboxy-1-pentylamino)-5-trifluormethyl-pyrimidin
   HPLC/MS (Methode B): RT = 2,7 min.; [M+H]+ = 437,0
(329) 2-(3,4-Dichlor-phenylamino)-4-(2-aminocarbonyl-1-carboxy-1-ethylamino)-5-trifluormethyl-pyrimidin
(330) 2-(3,4-Dichlor-phenylamino)-4-(2-carboxy-2-propylamino)-5-trifluormethyl-pyrimidin
   HPLC/MS (Methode B): RT = 2,5 min.; [M+H]+ = 409
(331) 2-(3,4-Dichlor-phenylamino)-4-(1-carboxy-3-methyl-1-propylamino)-5-trifluormethyl-pyrimidin
(332) 2-(3,4-Dichlor-phenylamino)-4-(*(1R)*-1-carboxy-2-hydroxy-1-ethylamino)-5-trifluormethyl-pyrimidin
(333) 2-(3,4-Dichlorphenylamino)-4-(4-(2-amino-1-ethyl)-1-piperazinyl)-5-trifluormethyl-pyrimidin
   HPLC/MS (Methode B): RT = 2,36 min.; [M+H]+ = 435,2
(334) 2-(3,4-Dichlorphenylamino)-4-(3,5-dimethyl-1-piperazinyl)-5-trifluormethyl-pyrimidin
   HPLC/MS (Methode B): RT = 2,7 min.; [M+H]+ = 420,1
(335) 2-(3,4-Dichlorphenylamino)-4-(cis-2-amino-cyclohexylamino)-5-trifluormethyl-pyrimidin
   HPLC/MS (Methode B): RT = 2,68 min.; [M+H]+ = 420,2
(336) 2-(3,4-Dichlor-phenylamino)-4-(N-methyl-N-[3-methylamino-1-propyl]-amino)-5-trifluormethyl-pyrimidin
(337) 2-(3,4-Dichlor-phenylamino)-4-(3-hydroxy-8-aza-bicyclo[3.2.1]oct-8-yl)-5-trifluormethyl-pyrimidin
   HPLC/MS (Methode B): RT = 2,75 min.; [M+H]+ = 433,2
(338) 2-(3,4-Dichlor-phenylamino)-4-(3-amino-1-pyrrolidinyl)-5-trifluormethyl-pyrimidin
   HPLC/MS (Methode B): RT = 2,24 min.; [M+H]+ = 392,2
   Schmelzpunkt: 157-158 °C
   R_{f}= 0, 03 (Kieselgel; Methylenchlorid: Methanol = 9:1)
   HPLC/MS (Methode G): RT = 3,63 min.; [M+H]+ = 393; Abs. λ max = 272,2 nm
(339) 2-(3,4-Dichlorphenylamino)-4-(4-benzyloxycarbonyl-1-piperazinyl)-5-trifluormethyl-pyrimidin
   HPLC/MS (Methode B): RT = 3,99 min.; [M+H]+ = 526,2
(340) 2-(3,4-Dichlorphenylamino)-4-(3-(2-(3-amino-1-propoxy)-1-ethoxy)-1-propylamino)-5-trifluormethyl-pyrimidin
   HPLC/MS (Methode B): RT = 2,35 min.; [M+H]+ = 482,1
(341) 2-(3,4-Dichlorphenylamino)-4-(N-benzyl-N-[2-hydroxy-1-phenyl-1-ethyl]-amino)-5-trifluormethyl-pyrimidin
   HPLC/MS (Methode B): RT = 3,27 min.; [M+H]+ = 533,0
(342) 2-(3,4-Dichlorphenylamino)-4-(1-homopiperazinyl)-5-trifluormethyl-pyrimidin
   HPLC/MS (Methode B): RT = 2,48 min.; [M+H]+ = 406,1
(343) 2-(3,4-Dichlorphenylamino)-4-(2-(2-(2-hydroxy-1-ethoxy)-1-ethoxy)-1-ethylamino)-5-trifluormethyl-pyrimidin
   HPLC/MS (Methode B): RT = 2,56 min.; [M+H]+ = 455,2
(344) 2-(3,4-Dichlorphenylamino)-4-(2-(3-methoxycarbonylphenyl)-1-ethyl-amino)-5-trifluormethyl-pyrimidin
   HPLC/MS (Methode B): RT = 3,37 min.; [M+H]+ = 485,2
(345) 2-(3,4-Dichlor-phenylamino)-4-(2-hydroxy-3-(4-morpholinyl)-1-propyl-amino)-5-trifluormethyl-pyrimidin
   HPLC/MS (Methode B): RT = 2,19 min.; [M+H]+ = 466,1
(346) 2-(3,4-Dichlorphenylamino)-4-(N-methyl-N-[2-nitrobenzyl]-amino)-5-trifluormethyl-pyrimidin
   HPLC/MS (Methode B): RT = 3,7 min.; [M+H]+ = 472,1
(347) 2-(3,4-Dichlorphenylamino)-4-(2-carboxy-1-phenyl-1-ethyl-amino)-5-trifluormethyl-pyrimi din
   HPLC/MS (Methode B): RT = 3,15 min.; [M+H]+ = 471,1
(348) 2-(3,4-Dichlorphenylamino)-4-(N-methyl-N-[6-[N-methyl-N-(2-phenyl-1-ethyl)-amino]-1-hexyl]-amino)-5-trifluormethyl-pyrimidin
   HPLC/MS (Methode B): RT = 2,92 min.; [M+H]+ = 554,1
(349) 2-(3,4-Dichlorphenylamino)-4-(2-(2-(2-amino-1-ethyl)-phenyl)-1-ethyl-amino)-5-trifluormethyl-pyrimidin
   HPLC/MS (Methode B): RT = 2,27 min.; [M+H]+ = 470,0

(350) 2-(3,4-Dichlorphenylamino)-4-(N-[2-diethylamino-1-ethyl]-N-ethyl-amino)-5-trifluormethyl-pyrimidin
   HPLC/MS (Methode B): RT = 2,86 min.; [M+H]+ = 450,1
(351) 2-(3,4-Dichlorphenylamino)-4-(2-ethoxycarbonyl-1-piperidinyl)-5-trifluormethyl-pyrimidin
   HPLC/MS (Methode B): RT = 4,01 min.; [M+H]+ = 463,2
(352) 2-(3,4-Dichlorphenylamino)-4-(4-methyl-1-homopiperazinyl)-5-trifluormethyl-pyrimidin
   HPLC/MS (Methode B): RT = 2,52 min.; [M+H]+ = 420,1
(353) 2-(3,4-Dichlorphenylamino)-4-(N-cyanomethyl-N-butyl-amino)-5-trifluormethyl-pyrimidin
(354) 2-(3,4-Dichlorphenylamino)-4-(N-[2-dimethylamino-1-ethyl]-N-methyl-amino)-5-trifluormethyl-pyrimidin
   HPLC/MS (Methode B): RT = 2,58 min.; [M+H]+ = 408,1
(355) 2-(3,4-Dichlorphenylamino)-4-(2-(1-pyrrolidinyl-methyl)-1-pyrrolidinyl)-5-trifluormethyl-pyrimidinHPLC/MS (Methode B): RT = 2,67 min.; [M+H]+ = 460,1
(356) 2-(3,4-Dichlorphenylamino)-4-(3-methoxycarbonylmethyl-1-piperidinyl)-5-trifluormethyl-pyrimidin
   HPLC/MS (Methode B): RT = 3,59 min.; [M+H]+ = 463,3
(357) 2-(3,4-Dichlorphenylamino)-4-(3-(3-diethylamino-1-propyl)-1-piperidinyl)-5-trifluormethyl-pyrimidin
   HPLC/MS (Methode B): RT = 2,94 min.; [M+H]+ = 504,1
(358) 2-(3,4-Dichlorphenylamino)-4-(5-hydroxy-2-methyl-2,8-diaza-spiro[5.5]undec-8-yl)-5-trifluormethyl-pyrimidin
   HPLC/MS (Methode B): RT = 2,6 min.; [M+H]+ = 490,2
(359) 2-(3,4-Dichlorphenylamino)-4-(3-(1-pyrrolidinyl-methyl)-1-piperidinyl)-5-trifluormethyl-pyrimidin
   HPLC/MS (Methode B): RT = 2,74 min.; [M+H]+ = 474,1
(360) 2-(3,4-Dichlorphenylamino)-4-(3-carboxy-1-piperidinyl)-5-trifluormethyl-pyrimidin
   HPLC/MS (Methode B): RT = 3,12 min.; [M+H]+ = 435,2
(361) 2-(3,4-Dichlorphenylamino)-4-(2-(2-(2-dimethylamino-1-ethyl)-1-piperidinyl)-1-ethylamino)-5-trifluormethyl-pyrimidin
   HPLC/MS (Methode B): RT = 2,2 min.; [M+H]+ = 505,2
(362) 2-(3,4-Dichlorphenylamino)-4-(3-(2-diethylaminomethyl-1-piperidinyl)-1-propylamino)-5-trifluormethyl-pyrimidin
   HPLC/MS (Methode B): RT = 2,21 min.; [M+H]+ = 533,2
(363) 2-(3,4-Dichlorphenylamino)-4-(4-(8-methyl-8-aza-bicyclo[3.2.1]oct-3-yl)-1-piperazinyl)-5-trifluormethyl-pyrimidin
   HPLC/MS (Methode B): RT = 2,16 min.; [M+H]+ = 515,2
(364) 2-(3,4-Dichlorphenylamino)-4-(1-carboxy-2-(4-chlorphenyl)-1-ethyl-amino)-5-trifluormethyl-pyrimidin
   HPLC/MS (Methode B): RT = 3,14 min.; [M+H]+ = 505,04
(365) 2-(3,4-Dichlorphenylamino)-4-(carboxymethylaminocarbonylmethylamino)-5-trifluormethyl-pyrimidin
   HPLC/MS (Methode B): RT = 1,8 min.; [M+H]+= 438
(366) 2-(3,4-Dichlorphenylamino)-4-(carboxy-phenyl-methylamino)-5-trifluormethyl-pyrimidin
   HPLC/MS (Methode B): RT = 2,85 min.; [M+H]+ = 457,22
(367) 2-(3,4-Dichlorphenylamino)-4-(4'-hydroxy-biphenyl-4-ylmethylamino)-5-trifluormethyl-pyrimidin
   HPLC/MS (Methode B): RT = 3,28 min.; [M+H]+ = 505,1
(368) 2-(3,4-Dichlorphenylamino)-4-(N-[4-amino-benzyl]-N-[2-methoxy-1-ethyl]-amino)-5-trifluormethyl-pyrimidin
   HPLC/MS (Methode B): RT = 2,64 min.; [M+H]+ = 486,0
(369) 2-(3,4-Dichlorphenylamino)-4-(4-hydroxy-benzylamino)-5-trifluormethyl-pyrimidin
   HPLC/MS (Methode B): RT = 2,9 min.; [M+H]+ = 429,1
(370) 2-(3,4-Dichlorphenylamino)-4-(2-diphenylmethoxy-1-ethylamino)-5-trifluormethyl-pyrimidin
(371) 2-(3,4-Dichlorphenylamino)-4-(N-aminocarbonylmethyl-N-methyl-amino)-5-trifluormethyl-pyrimidin
   HPLC/MS (Methode B): RT = 2,55 min.; [M+H]+ = 394,1
(372) 2-(3,4-Dichlorphenylamino)-4-(2-methylaminocarbonyl-1-ethylamino)-5-trifluormethyl-pyrimidin
   HPLC/MS (Methode B): RT = 2,5 min.; [M+H]+ = 408,1
(373) 2-(3,4-Dichlorphenylamino)-4-(2-dimethylaminocarbonyl-1-ethylamino)-5-trifluormethyl-pyrimidin
   HPLC/MS (Methode B): RT = 2,69 min.; [M+H]+ = 422,1
(374) 2-(3,4-Dichlorphenylamino)-4-(2-(4-methyl-1-piperazinyl)-carbonyl-1-ethylamino)-5-trifluormethyl-pyrimidin
   HPLC/MS (Methode B): RT = 2,2 min.; [M+H]+ = 477,1
(375) 2-(3,4-Dichlorphenylamino)-4-(4-carboxy-3-thiazolidinyl)-5-trifluormethyl-pyrimidin
   HPLC/MS (Methode B): RT = 2,84 min.; [M+H]+ = 439,11
(376) 2-(3,4-Dichlorphenylamino)-4-(*(R)*carboxy-(4-hydroxyphenyl)-methylamino)-5-trifluormethyl-pyrimidin
   HPLC/MS (Methode B): RT = 2,4 min.; [M+H]+ = 473,13
(377) 2-(3,4-Dichlorphenylamino)-4-(1-carboxy-5-benzyloxycarbonylamino-1-pentylamino)-5-trifluormethyl-pyrimidin
   HPLC/MS (Methode B): RT = 2,84 min.; [M+H]+ = 586,12
(378) 2-(3,4-Dichlorphenylamino)-4-(1-(*1H*-benzimidazol-2-yl)-1-ethylamino)-5-trifluormethyl-pyrimidin
   HPLC/MS (Methode B): RT = 2,65 min.; [M+H]+ = 467,1
(379) 2-(3,4-Dichlorphenylamino)-4-(4-(4-ethoxycarbonyl-1-piperidinyl)-1-piperidinyl)-5-trifluormethyl-pyrimidin
   HPLC/MS (Methode B): RT = 2,84 min.; [M+H]+ = 546,2
(380) 2-(3,4-Dichlorphenylamino)-4-(4-(3-hydroxy-1-piperidinyl)-1-piperidinyl)-5-trifluormethyl-pyrimidin
   HPLC/MS (Methode B): RT = 2,56 min.; [M+H]+ = 490,2
(381) 2-(3,4-Dichlorphenylamino)-4-(N-methyl-N-[2-pyrazinyl-methyl]-amino)-5-trifluormethyl-pyrimidin
   HPLC/MS (Methode B): RT = 3,06 min.; [M+H]+ = 429,2
(382) 2-(3,4-Dichlorphenylamino)-4-(*(S)*carboxy-(4-hydroxyphenyl)-methylamino)-5-trifluormethyl-pyrimidin
   HPLC/MS (Methode B): RT = 2,39 min.; [M+H]+ = 473,33
(383) 2-(3,4-Dichlorphenylamino)-4-(1-phenylsulfonyl-4-piperidinylamino)-5-trifluormethyl-pyrimidin
(384) 2-(3,4-Dichlorphenylamino)-4-(4-(4-hydroxyphenyl)-1-butylamino)-5-trifluormethyl-pyrimidin
   HPLC/MS (Methode B): RT = 3,14 min.; [M+H]+ = 471,3
(385) (2-[2-(3,4-dichloro-phenylamino)-5-trifluormethyl-pyrimidine-4-ylamino]-ethyl)--methyl-carbaminsäure-tert-butylester
   Schmelzpunkt: 140-141 °C
   R_{f} = 0,43 (Kieselgel; Methylenchlorid: Methanol = 9:1)
   HPLC/MS (Methode K): RT = 3,58 min.; [M+H]+ = 481; Abs. λ max = 266,5 nm
(386) 2-(3,4-Dichlorphenylamino)-4-(4-(3-carbooy-1-propyl)-cyclohexylamino)-5-trifluormethyl-pyrimidin
   HPLC/MS (Methode B): RT = 2,36 min.; [M+H]+ = 396,2
(387) 2-(3,4-Dichlorphenylamino)-4-(4-(2-carboxy-1-ethyl)-cyclohexylmethylamino)-5-trifluormethyl-pyrimidin
   HPLC/MS (Methode B): RT = 3,05 min.; [M+H]+ = 491,3
(388) 2-(3,4-Dichlorphenylamino)-4-(1-carboxy-2-propylamino)-5-trifluormethyl-pyrimidin
   HPLC/MS (Methode B): RT = 2,76 min.; [M+H]+ = 409,2
(389) 2-(3,4-Dichlorphenylamino)-4-(2-(2-hydroxyphenyl)-1-ethylamino)-5-trifluormethyl-pyrimidin
   HPLC/MS (Methode B): RT = 3,16 min.; [M+H]+ = 443,2
(390) (1-[2-(4-Carbamoyl-phenylamino)-5-trifluormethyl-pyrimidin-4-yl]-pyrrolidin-3-yl)-carbaminsäure-tert-butylester
   Schmelzpunkt: 225-228 °C
   R_{f} = 0,20 (Kieselgel; Methylenchlorid: Methanol = 9:1)
   HPLC/MS (Methode G): RT = 3,70 min.; [M+H]+ = 467; Abs. λ max = 283,6 nm
(391) 2-(3,4-Dichlorphenylamino)-4-(3-carboxy-benzylamino)-5-trifluormethyl-pyrimidin
   HPLC/MS (Methode B): RT = 2,91 min.; [M+H]+ = 457,2
(392) 2-(3,4-Dichlorphenylamino)-4-(6-tert.-butyloxycarbonylamino-1-hexylamino)-5-trifluormethyl-pyrimidin
   HPLC/MS (Methode B): RT = 3,47 min.; [M+H]+ = 522,0
(393) 2-(3,4-Dichlorphenylamino)-4-(3-tert.-butyloxycarbonylamino-1-propylamino)-5-trifluormethyl-pyrimidin
   HPLC/MS (Methode B): RT = 3,28 min.; [M+H]+ = 480,0
(394) 2-(3,4-Dichlorphenylamino)-4-(4-carboxy-1-methyl-4-piperidinylamino)-5-trifluormethyl-pyrimidin
   HPLC/MS (Methode B): RT = 2,09 min.; [M+H]+ = 464,1
(395) 2-(3,4-Dichlorphenylamino)-4-(1-carboxy-2-(2-chlorphenyl)-1-ethylamino)-5-trifluormethyl-pyrimidin
   HPLC/MS (Methode B): RT = 2,96 min.; [M+H]+ = 504,77
(396) 2-(3,4-Dichlorphenylamino)-4-(N-benzyl-N-[1-methoxycarbonyl-1-ethyl]-amino)-5-trifluormethyl-pyrimidin
(397) 2-(3,4-Dichlorphenylamino)-4-(N-[ethoxycarbonylmethyl]-N-isopropyl-amino)-5-trifluormethyl-pyrimidin
   HPLC/MS (Methode B): RT = 3,7 min.; [M+H]+ = 451
(398) 2-(3,4-Dichlorphenylamino)-4-(2-(2-ethoxycarbonyl-1-ethyl)-1-pyrrolidinyl)-5-trifluormethyl-pyrimidin
   HPLC/MS (Methode B): RT = 3,42 min.; [M+H]+ = 477,2
(399) 2-(3,4-Dichlorphenylamino)-4-(carbamimidoyl-methylamino)-5-trifluormethyl-pyrimidin
   HPLC/MS (Methode B): RT = 1,74 min.; [M+H]+ = 379,13

(400) 2-(3,4-Dichlorphenylamino)-4-(N-[4-hydroxycyclohexyl]-N-methylamino)-5-trifluormethyl-pyrimidin
   HPLC/MS (Methode B): RT = 2,86 min.; [M+H]+ = 435,2
(401) 2-(3,4-Dichlorphenylamino)-4-(2-(6-methoxy-1*H*-benzimidazol-2-yl)-1-ethyl)-5-trifluormethyl-pyrimidin
   HPLC/MS (Methode B): RT = 2,31 min.; [M+H]+ = 497,0
(402)2-(3,4-Dichlorphenylamino)-4-(2-carboxy-benzylamino)-5-trifluormethyl-pyrimidin
   HPLC/MS (Methode B): RT = 2,59 min.; [M+H]+ = 457,26
(403) 2-(3,4-Dichlorphenylamino)-4-(2-aminocarbonyl-1,3-dihydro-isoindol-5-yl-methylamino)-5-trifluormethyl-pyrimidin
   HPLC/MS (Methode B): RT = 2,68 min.; [M+H]+ = 497,2
(404) 2-(3,4-Dichlorphenylamino)-4-(3-(tert.-butyloxycarbonylaminomethyl)-cyclohexylmethylamino)-5-trifluonmethyl-pyrimidin
   HPLC/MS (Methode B): RT = 3,61 min.; [M+H]+ = 548,0
(405) 2-(3,4-Dichlorphenylamino)-4-(2-methyl-4-phenylaminocarbonylamino-2-butylamino)-5-trifluormethyl-pyrimidin
(406) 2-(3,4-Dichlorphenylamino)-4-(3-dimethylaminocarbonyl-1-piperidinyl)-5-trifluormethyl-pyrimidin
   HPLC/MS (Methode B): RT = 3,1 min.; [M+H]+ = 462,2
(407) 2-(3,4-Dichlorphenylamino)-4-(2-hydroxy-cyclohexylamino)-5-trifluormethyl-pyrimidin
   HPLC/MS (Methode B): RT = 2,96 min.; [M+H]+ = 421,2
(408) 2-(3,4-Dichlorphenylamino)-4-(2-hydroxymethyl-1-piperidinyl)-5-trifluormethyl-pyrimidin
   HPLC/MS (Methode B): RT = 2,62 min.; [M+H]+ = 421,1
(409) 2-(3,4-Dichlorphenylamino)-4-(N-methyl-N-[2-pyridyl-methyl]-amino)-5-trifluormethyl-pyrimidin
   HPLC/MS (Methode B): RT = 2,48 min.; [M+H]+ = 428,1
(410) 2-(3,4-Dichlorphenylamino)-4-(N-methyl-N-[3-pyridyl-methyl]-amino)-5-trifluormethyl-pyrimidin
   HPLC/MS (Methode B): RT = 2,91 min.; [M+H]+ = 443,2
(411) 2-(3,4-Dichlorphenylamino)-4-(1-ethyl-3-piperidinylamino)-5-trifluormethyl-pyrimidin
   HPLC/MS (Methode B): RT = 3,6 min.; [M+H]+ = 492,2
(412) 2-(3,4-Dichlorphenylamino)-4-(2-dimethylamino-cyclohexylamino)-5-trifluormethyl-pyrimidin
   HPLC/MS (Methode B): RT = 2,74 min.; [M+H]+ = 506,1
(413) 2-(3,4-Dichlorphenylamino)-4-(4-(3-hydroxy-1-propyl)-1-piperidinyl)-5-trifluormethyl-pyrimidin
   HPLC/MS (Methode B): RT = 2,46 min.; [M+H]+ = 367,2
(414) 2-(3,4-Dichlorphenylamino)-4-(4-(3-hydroxy-1-propyl)-1-piperazinyl)-5-trifluormethyl-pyrimidin
   HPLC/MS (Methode B): RT = 2,5 min.; [M+H]+ = 411,2
(415) 2-(3,4-Dichlorphenylamino)-4-(N-methyl-N-[3-(4-pyridyl)-1-propyl]-amino)-5-trifluormethyl-pyrimidin
   HPLC/MS (Methode B): RT = 2,5 min.; [M+H]+ = 456,1
(416) 2-(3,4-Dichlorphenylamino)-4-(4-dimethylamino-2-phenyl-1-butylamino)-5-trifluormethyl-pyrimidin
   HPLC/MS (Methode B): RT = 2,37 min.; [M+H]+ = 498,1
(417) 2-(3,4-Dichlorphenylamino)-4-(2-(3-diethylamino-1-propyl)-1-piperidinyl)-5-trifluormethyl-pyrimidin
   HPLC/MS (Methode B): RT = 2,92 min.; [M+H]+ = 504,1
(418) 2-(3,4-Dichlorphenylamino)-4-(bis-[3-pyridylmethyl]-amino)-5-trifluormethyl-pyrimidin
   HPLC/MS (Methode B): RT = 2,01 min.; [M+H]+ = 505,2
(419) 2-(3,4-Dichlorphenylamino)-4-(4-(N-methyl-N-[2-methoxycarbonyl-1-ethyl]-amino)-1-piperidinyl)-5-trifluormethyl-pyrimidin
   HPLC/MS (Methode B): RT = 2,23 min.; [M+H]+ = 506,12
(420) 2-(3,4-Dichlorphenylamino)-4-(2-(4-(2*H*-pyridazin-3-on-6-yl)-phenyl)-1-ethylamino)-1-piperidinyl)-5-trifluormethyl-pyrimidin
(421) 2-(3,4-Dichlorphenylamino)-4-(3-(4-amino-3,5-dichlorphenyl)-1-propylamino)-5-trifluormethyl-pyrimidin
   HPLC/MS (Methode B): RT = 3,6 min.; [M+H]+ = 524,0
(422) 2-(3,4-Dichlorphenylamino)-4-(4-(2-(N-[dimethylaminocarbonylmethyl]-N-methyl-amino)-1-ethyl-amino)-1-piperidinyl)-5-trifluormethyl-pyrimidin
   HPLC/MS (Methode B): RT = 2,13 *min.; [M+H]+ = 533,03
(423) 2-(3,4-Dichlorphenylamino)-4-(2-(2-(2-diethylamino-1-ethoxy)-1-ethyl)-1-piperidinyl)-5-trifluormethyl-pyrimidin
   HPLC/MS (Methode B): RT = 3,02 min.; [M+H]+ = 534,2
(424) 2-(3,4-Dichlorphenylamino)-4-(4-(2-(2-diethylamino-1-ethoxy)-1-ethyl)-1-piperidinyl)-5-trifluormethyl-pyrimidin
   HPLC/MS (Methode B): RT = 2,67 min.; [M+H]+ = 534,2
(425) 2-(3,4-Dichlorphenylamino)-4-(5-(3-carboxy-1-propyl)-indan-2-ylamino)-5-trifluormethyl-pyrimidin
   HPLC/MS (Methode B): RT = 3,16 min.; [M+H]+ = 525,2
(426) 2-(3,4-Dichlorphenylamino)-4-(2-ethoxycarbonyl-1-(3-pyridyl)-1-ethylamino)-5-trifluormethyl-pyrimidin
   HPLC/MS (Methode B): RT = 2,78 min.; [M+H]+ = 500,2
(427) 2-(3,4-Dichlorphenylamino)-4-(1,1-dimethyl-3-(2-oxo-3-pyridin-4-yl-imidazolidin-1-yl)-propylamino)-5-trifluormethyl-pyrimidin
   HPLC/MS (Methode B): RT = 2,05 min.; [M+H]+ = 554,3
(428) 2-(4-Chlorphenylamino)-4-(4-methylpiperazino)-5-nitro-pyrimidin
   Schmelzpunkt: 178-180°C
(429) 2-(4-Chlorphenylamino)-4-(4-hydroxy-1-piperidinyl)-5-nitro-pyrimidin
   HPLC/MS (Methode B): RT = 2,76 min.; [M+H]+ = 350,2
(430) 2-(4-Chlorphenylamino)-4-[2-(dimethylamino)-1-ethylamino]-5-nitro-pyrimidin
   Schmelzpunkt: 179-181 °C
(431) 2-(4-Chlorphenylamino)-4-(3-(4-morpholinyl)-1-propylamino)-5-nitro-pyrimidin
   HPLC/MS (Methode B): RT = 2,23 min.; [M+H]+ = 393,2
(432) 2-(4-Chlorphenylamino)-4-(2-carboxy-1-ethylamino)-5-nitro-pyrimidin
   Hergestellt aus der Verbindung 632 des Beispiels 1 durch nachträgliche Umsetzung mit 1N Natronlauge in Tetrahydrofuran.
   Schmelzpunkt: >300°C
   Rf-Wert: 0,40 (Kieselgel; Methylenchlorid/Methanol = 9:1)
(433) 2-(4-Chlorphenylamino)-4-(3-(*1H*-1-imidazolyl)-1-propylamino)-5-nitro-pyrimidin
   HPLC/MS (Methode B): RT = 2,28 min.; [M+H]+ = 374,2
(434) 2-(4-Chlorphenylamino)-4-(3-dimethylamino-1-propylamino)-5-nitro-pyrimidin
   Schmelzpunkt: 148-150°C
(435) 2-(4-Chlorphenylamino)-4-(2-diisopropylamino-1-ethylamino)-5-nitro-pyrimidin
   HPLC/MS (Methode B): RT = 2,4 min.; [M+H]+ = 393,2
(436) 2-(4-Chlorphenylamino)-4-(bis-(2-methoxyethyl)amino)-5-nitro-pyrimidin
   HPLC/MS (Methode B): RT = 3,31 min.; [M+H]+ = 382,1
(437) 2-(4-Chlorphenylamino)-4-(N-methyl-N-(2-methylamino-1-ethyl)-amino)-5-nitro-pyrimidin
   HPLC/MS (Methode B): RT = 2,4 min.; [M+H]+ = 337,0
(438) 2-(4-Chlorphenylamino)-4-(2-(4-pyridyl)-1-ethylamino)-5-nitro-pyrimidin
   HPLC/MS (Methode B): RT = 2,26 min.; [M+H]+ = 371,1
(439) 2-(4-Chlorphenylamino)-4-(4-aminosulfonyl-benzylamino)-5-nitro-pyrimidin
   HPLC/MS (Methode B): RT = 2,89 min.; [M+H]+ = 435,2
(440) 2-(3,4-Dichlorphenylamino)-4-(2-acetylamino-ethylamino)-5-fluor-pyrimidin
(441) 2-(4-Chlorphenylamino)-4-(4-pyridyl-methylamino)-5-nitro-pyrimidin
   HPLC/MS (Methode B): RT = 2,15 min.; [M+H]+= 357,1
(442) 2-(4-Chlorphenylamino)-4-((3-chlor-5-trifluormethyl-2-pyridyl)-methylamino)-5-nitro-pyrimidin
   HPLC/MS (Methode B): RT = 3,91 min.; [M+H]+ = 459,1
(443) 2-(4-Chlorphenylamino)-4-((4-ethoxycarbonyl-1*H*-pyrazol-5-yl)-methylamino)-5-nitro-pyrimidin
(444) 2-(4-Chlorphenylamino)-4-(3-nitrobenzylamino)-5-nitro-pyrimidin
   HPLC/MS (Methode B): RT = 3,31 min.; [M+H]+ = 401,2
(445) 2-(4-Chlorphenylamino)-4-(4-(2-carboxy-1-ethyl)-1-piperidinyl)-5-nitro-pyrimidin
   HPLC/MS (Methode B): RT = 3,08 min.; [M+H]+ = 406,2
(446) 2-(4-Chlorphenylamino)-4-(3-(1-pyrrolidinyl)-1-propylamino)-5-nitro-pyrimidin
   HPLC/MS (Methode B): RT = 2,31 min.; [M+H]+ = 377,2
(447) 2-(4-Chlorphenylamino)-4-(5-acetylamino-5-methoxycarbonyl-1-pentylamino)-5-nitro-pyrimidin
   HPLC/MS (Methode B): RT = 2,66 min.; [M+H]+ = 451,2
(448) 2-(4-Chlorphenylamino)-4-((1-hydroxy-1-cyclohexyl)-methylamino)-5-nitro-pyrimidin
   HPLC/MS (Methode B): RT = 3,45 min.; [M+H]+ = 378,2
(449) 2-(4-Chlorphenylamino)-4-(2-(1*H*-indol-3-yl)-1-ethylamino)-5-nitro-pyrimidin
   HPLC/MS (Methode B): RT = 3,64 min.; [M+H]+ = 409,2
(450) 2-(4-Chlorphenylamino)-4-(2-(4-nitro-2-pyridyl-amino)-1-ethylamino)-5-nitro-pyrimidin
   HPLC/MS (Methode B): RT = 3,05 min.; [M+H]+ = 431,1

(451) 2-(4-Chlorphenylamino)-4-(2-hydroxy-2-phenyl-1-ethylamino)-5-nitro-pyrimidin
   HPLC/MS (Methode B): RT = 3,3 min.; [M+H]+ = 386,3
(452) 2-(4-Chlorphenylamino)-4-(2-phenylamino-1-ethylamino)-5-nitro-pyrimidin
   HPLC/MS (Methode B): RT = 3,08 min.; [M+H]+ = 385,1
(453) 2-(4-Chlorphenylamino)-4-(2-(4-hydroxyphenyl)-1-ethylamino)-5-nitro-pyrimidin
   HPLC/MS (Methode B): RT = 3,24 min.; [M+H]+ = 386,2
(454) 2-(4-Chlorphenylamino)-4-(2-(4-aminosulfonylphenyl)-1-ethylamino)-5-nitro-pyrimidin
   HPLC/MS (Methode B): RT = 2,85 min.; [M+H]+ = 449,1
(455) 2-(4-Chlorphenylamino)-4-(2-(1-naphthylamino)-1-ethylamino)-5-nitro-pyrimidin
   HPLC/MS (Methode B): RT = 3,8 min.; [M+H]+ = 435,2
(456) 2-(4-Chlorphenylamino)-4-(2-(4-nitrophenyl)-1-ethylamino)-5-nitro-pyrimidin
   HPLC/MS (Methode B): RT = 3,5 min.; [M+H]+ = 415,3
(457) 2-(4-Chlorphenylamino)-4-(3-ethoxycarbonyl-1-propylamino)-5-nitro-pyrimidin
   Schmelzpunkt: 133-135°C
(458) 2-(4-Chlorphenylamino)-4-(aminocarbonylmethylamino)-5-nitro-pyrimidin
   HPLC/MS (Methode B): RT = 2,36 min.; [M+H]+ = 323,1
(459) 4-[4-{3-Amino-pyrrolidin-1-yl)-5-trifluormethyl-pyrimidin-2-ylamino)-benzamid
   Herstellung analog 1(80).
   R_{f} = 0,13 (Kieselgel; Methylenchlorid: Methanol = 4:1)
   HPLC/MS (Methode G): RT = 2,59 min.; [M+H]+ = 367; Abs. λ max = 281,7 nm
(460) 2-(4-Chlorphenylamino)-4-(2-tert.-butyloxycarbonylamino-1-ethylamino)-5-nitro-pyrimidin
   HPLC/MS (Methode B): RT = 3,24 min.; [M+H]+ = 409,1
(461) 2-(4-Chlorphenylamino)-4-(1-ethyl-2-pyrrolidinyl-methylamino)-5-nitro-pyrimidin
   HPLC/MS (Methode B): RT = 2,28 min.; [M+H]+ = 377,2
(462) 2-(4-Chlorphenylamino)-4-(2-(1-pyrrolidinyl)-1-ethylamino)-5-nitro-pyrimidin
   HPLC/MS (Methode B): RT = 2,25 min.; [M+H]+ = 363,2
(463) 2-(4-Chlorphenylamino)-4-(2-tetrahydrofuryl-methylamino)-5-nitro-pyrimidin
(464) 2-(4-Chlorphenylamino)-4-(2-(1-piperidinyl)-1-ethylamino)-5-nitro-pyrimidin
   HPLC/MS (Methode B): RT = 2,3 min.; [M+H]+ = 377,1
(465) 2-(4-Chlorphenylamino)-4-(2-hydroxy-1-propylamino)-5-nitro-pyrimidin
   HPLC/MS (Methode B): RT = 2,85 min.; [M+H]+ = 324,2
(466) 2-(4-Chlorphenylamino)-4-(2,3-dihydroxy-1-propylamino)-5-nitro-pyrimidin
   HPLC/MS (Methode B): RT = 2,41 min.; [M+H]+ = 340,2
(467) 2-(4-Chlorphenylamino)-4-(2-diethylamino-1-ethylamino)-5-nitro-pyrimidin
   HPLC/MS (Methode B): RT = 2,27 min.; [M+H]+ = 365,2
(468) 2-(4-Chlorphenylamino)-4-(2-(2-hydroxyethoxy)-1-ethylamino)-5-nitro-pyrimidin
   HPLC/MS (Methode B): RT = 2,71 min.; [M+H]+ = 354,2
(469) 2-(4-Chlorphenylamino)-4-(2-hydroxy-1-ethylamino)-5-nitro-pyrimidin
   Schmelzpunkt: 226-228°C
(470) 2-(4-Chlorphenylamino)-4-(3-diethylamino-1-propylamino)-5-nitro-pyrimidin
   HPLC/MS (Methode B): RT = 2,35 min.; [M+H]+ = 379,2
(471) 2-(4-Chlorphenylamino)-4-(3-hydroxy-1-propylamino)-5-nitro-pyrimidin
   Schmelzpunkt: 190-194°C
(472) 2-(4-Chlorphenylamino)-4-(2-(1-methyl-1*H*-pyrrol-2-yl)-1-ethylamino)-5-nitro-pyrimidin
   HPLC/MS (Methode B): RT = 3,41 min.; [M+H]+ = 373,2
(473) 2-(4-Chlorphenylamino)-4-(4-hydroxy-3-methoxy-benzylamino)-5-nitro-pyrimidin
   HPLC/MS (Methode B): RT = 3,15 min.; [M+H]+ = 402,1
(474) 2-(4-Chlorphenylamino)-4-(2-methylsulfanyl-1-ethylamino)-5-nitro-pyrimidin
   HPLC/MS (Methode B): RT = 3,46 min.; [M+H]+ = 340,1
(475) 2-(4-Chlorphenylamino)-4-(3-methooy-1-propylamino)-5-nitro-pyrimidin
   Schmelzpunkt: 148-150°C
(476) 2-(4-Chlorphenylamino)-4-(2,2-dimethyl-3-dimethylamino-1-propylamino)-5-nitro-pyrimidin
   HPLC/MS (Methode B): RT = 2,4 min.; [M+H]+ = 379,2
(477) 2-(4-Chlorphenylamino)-4-(2,2-dimethyl-3-hydroxy-1-propylamino)-5-nitro-pyrimidin
   HPLC/MS (Methode B): RT = 3,17 min.; [M+H]+ = 352,2
(478) 2-(4-Chlorphenylamino)-4-cyanomethylamino-5-nitro-pyrimidin
   HPLC/MS (Methode B): RT = 2,49 min.; [M+H]+ = 305,14
(479) 2-(4-Chlorphenylamino)-4-(3-aminocarbonyl-1-piperidinyl)-5-nitro-pyrimidin
   HPLC/MS (Methode B): RT = 2,69 min.; [M+H]+ = 377,2
(480) 2-(4-Chlorphenylamino)-4-(4-acetyl-1-piperazinyl)-5-nitro-pyrimidin
   HPLC/MS (Methode B): RT = 3,64 min.; [M+H]+ = 409,2
(481) 2-(4-Chlorphenylamino)-4-(4-(1-piperidinyl)-1-piperidinyl)-5-nitro-pyrimidin
   HPLC/MS (Methode B): RT = 2,31 min.; [M+H]+ = 377,2
(482) 2-(4-Chlorphenylamino)-4-(4-(morpholinocarbonylmethyl)-1-piperazinyl)-5-nitro-pyrimidin
   HPLC/MS (Methode B): RT = 2,28 min.; [M+H]+ = 462,2
(483) 2-(4-Chlorphenylamino)-4-piperazino-5-nitro-pyrimidin
   Rf-Wert: 0,20 (Kieselgel; Methylenchlorid/Methanol/konz. wäßriges Ammoniak = 9:1:0,1)
(484) 2-(4-Chlorphenylamino)-4-(3-[4-(3-amino-1-propyl)-1-piperazinyl]-1-propylamino)-5-nitro-pyrimidin
   HPLC/MS (Methode B): RT = 2,41 min.; [M+H]+ = 519,2
(485) 2-(4-Chlorphenylamino)-4-(cis-4-carboxy-cyclohexylamino)-5-nitro-pyrimidin
   HPLC/MS (Methode B): RT = 2,38 min.; [M+H]+ = 392,0
(486) 2-(4-Chlorphenylamino)-4-(3-dibenzylamino-1-propylamino)-5-nitro-pyrimidin
   HPLC/MS (Methode B): RT = 2,65 min.; [M+H]+ = 560,1
(487) 2-(4-Chlorphenylamino)-4-(N-[4-methoxycarbonyl-cyclohexyl]-N-[3-pyridylmethyl]amino)-5-nitro-pyrimidin
(488) 2-(3,4-Dichlorphenylamino)-4-(2-acetylamino-ethylamino)-5-dimethylaminomethyl-pyrimidin
(489) 2-(4-Chlorphenylamino)-4-(2-phenyl-1-(4-phenyl-1-butyl-aminocarbonyl)-1-ethylamino)-5-nitro-pyrimidin
   HPLC/MS (Methode B): RT = 3,6 min.; [M+H]+ = 545
(490) 2-(4-Chlorphenylamino)-4-(1-aminocarbonyl-2-(4-methoxyphenyl)-1-ethylamino)-5-nitro-pyrimidin
   HPLC/MS (Methode B): RT = 2,66 min.; [M+H]+ = 443,2
(491) 2-(4-Chlorphenylamino)-4-(1-dimethylaminomethylcarbonyl-4-piperidinyl-methylamino)-5-nitro-pyrimidin
(492) 2-(4-Chlorphenylamino)-4-(N-ethyl-N-[4-pyridylmethyl]amino)-5-nitro-pyrimidin
   HPLC/MS (Methode B): RT = 2,38 min.; [M+H]+ = 385,1
(493) 2-(4-Chlorphenylamino)-4-(3-phenyl-azepan-4-on-1-yl)-5-nitro-pyrimidin
(494) 2-(4-Chlorphenylamino)-4-(2-(3-hydroxy-1-propyl)-1-piperidinyl)-5-nitro-pyrimidin
   HPLC/MS (Methode B): RT = 3,17 min.; [M+H]+ = 392,3
(495) 2-(4-Chlorphenylamino)-4-(4-(8-methoxy-3,4-dihydro-1H-quinazolin-2-on-3-yl)-1-piperidinyl)-5-nitro-pyrimidin
   HPLC/MS (Methode B): RT = 3,03 min.; [M+H]+ = 510,1
(496) 2-(4-Chlorphenylamino)-4-(4-(2-nitrophenyl)-1-piperazinyl)-5-nitro-pyrimidin
   HPLC/MS (Methode B): RT = 3,6 min.; [M+H]+ = 456,0
(497) 2-(4-Chlorphenylamino)-4-(4-(3,4-dimethoxyphenyl)-1-piperazinyl)-5-nitro-pyrimidin
   HPLC/MS (Methode B): RT = 2,34 min.; [M+H]+ = 396,2
(498) 2-(4-Chlorphenylamino)-4-(4-(4-cyanophenyl)-1-piperazinyl)-5-nitro-pyrimidin
   HPLC/MS (Methode B): RT = 3,62 min.; [M+H]+ = 436,2
(499) 2-(4-Chlorphenylamino)-4-(1-benzyl-3-pyrrolidinyl-amino)-5-nitro-pyrimidin
   HPLC/MS (Methode B): RT = 2,58 min.; [M+H]+ = 425,3

(500) 2-(4-Chlorphenylamino)-4-(1-hydroxy-2-propylamino)-5-nitro-pyrimidin
   HPLC/MS (Methode B): RT = 2,74 min.; [M+H]+ = 324,2
(501) 2-(4-Chlorphenylamino)-4-(3-(1-piperidinyl)-1-propylamino)-5-nitro-pyrimidin
   HPLC/MS (Methode B): RT = 2,36 min.; [M+H]+ = 391,2
(502) 2-(4-Chlorphenylamino)-4-(1-benzyl-4-piperidinyl-amino)-5-nitro-pyrimidin
   HPLC/MS (Methode B): RT = 2,47 min.; [M+H]+ = 439,2
(503) 2-(4-Chlorphenylamino)-4-(4-aminomethyl-benzylamino)-5-nitro-pyrimidin
   HPLC/MS (Methode B): RT = 2,39 min.; [M+H]+ = 385,1
(504) 2-(4-Chlorphenylamino)-4-(4-aminobutylamino)-5-nitro-pyrimidin
   HPLC/MS (Methode B): RT = 2,24 min.; [M+H]+ = 337,1
(505) 2-(4-Chlorphenylamino)-4-(3-amino-2,2-dimethyl-1-propylamino)-5-nitro-pyrimidin
   HPLC/MS (Methode B): RT = 2,35 min.; [M+H]+ = 351,1
(506) 2-(4-Chlorphenylamino)-4-(trans-2-amino-cyclohexylamino)-5-nitro-pyrimidin HPLC/MS (Methode B): RT = 2,5 min.; [M+H]+ = 363,2
(507) 2-(4-Chlorphenylamino)-4-(2-(2-(2-amino-1-ethoxy)-1-ethoxy)-1-ethylamino)-5-nitro-pyrimidin
   HPLC/MS (Methode B): RT = 2,13 min.; [M+H]+ = 397,2
(508) 2-(4-Chlorphenylamino)-4-(3-amino-benzylamino)-5-nitro-pyrimidin
   HPLC/MS (Methode B): RT = 2,26 min.; [M+H]+ = 371,1
(509) 2-(4-Chlorphenylamino)-4-(3-amino-2-hydroxy-1-propylamino)-5-nitro-pyrimidin
(510) 2-(4-Chlorphenylamino)-4-(2-(2-amino-1-ethylsulfanyl)-1-ethylamino)-5-nitro-pyrimidin
   HPLC/MS (Methode B): RT = 2,34 min.; [M+H]+ = 369,0
(511) 2-(4-Chlorphenylamino)-4-(N-[2-dimethylamino-1-ethyl]-N-ethyl-amino)-5-nitro-pyrimidin
   HPLC/MS (Methode B): RT = 2,4 min.; [M+H]+ = 365,0
(512) 2-(4-Chlorphenylamino)-4-(N-[3-dimethylamino-1-propyl]-N-methyl-amino)-5-nitro-pyrimidin
   HPLC/MS (Methode B): RT = 2,25 min.; [M+H]+ = 365,1
(513) 2-(4-Chlorphenylamino)-4-(3-(4-methyl-1-piperazinyl)-1-propylamino)-5-nitro-pyrimidin
   HPLC/MS (Methode B): RT = 1,84 min.; [M+H]+ = 406,2
(514) 2-(4-Chlorphenylamino)-4-(N-[2-cyano-1-ethyl]-N-[3-pyridylmethyl]-amino)-5-nitro-pyrimidin
   HPLC/MS (Methode B): RT = 2,38 min.; [M+H]+ = 410,2
(515) 2-(4-Chlorphenylamino)-4-(4-(2-pyridyl)-1-piperazinyl)-5-nitro-pyrimidin
   HPLC/MS (Methode B): RT = 2,28 min.; [M+H]+ = 412,1
(516) 2-(4-Chlorphenylamino)-4-(4-[bis-(4-methoxy-phenyl)]-methyl-1-piperazinyl)-5-nitro-pyrimidin
(517) 2-(4-Chlorphenylamino)-4-(4-(3-methoxy-phenyl)-1-piperazinyl)-5-nitro-pyrimidin HPLC/MS (Methode B): RT = 3,51 min.; [M+H]+ = 441,2
(518) 2-(4-Chlorphenylamino)-4-(N-benzyl-N-[2-cyano-1-ethyl]-amino)-5-nitro-pyrimidin
   HPLC/MS (Methode B): RT = 3,53 min.; [M+H]+ = 409,2
(519) 2-(4-Chlorphenylamino)-4-(N-benzyl-N-[2-hydroxy-1-ethyl]-amino)-5-nitro-pyrimidin
   HPLC/MS (Methode B): RT = 3,27 min.; [M+H]+ = 400,2
(520) 2-(4-Chlorphenylamino)-4-(3-carboxy-1-propyl-amino)-5-nitro-pyrimidin
   Hergestellt aus der Verbindung 457 des Beispiels 1 durch nachträgliche Umsetzung mit 1N Natronlauge in Tetrahydrofuran.
   Schmelzpunkt: 258-260°C
(521) 2-(4-Chlorphenylamino)-4-(N-benzyl-N-[ethoxycarbonylmethyl]-amino)-5-nitro-pyrimidin
   HPLC/MS (Methode B): RT = 3,76 min.; [M+H]+ = 442,2
(522) 2-(4-Chlorphenylamino)-4-(N-[4-nitrobenzyl]-N-propyl-amino)-5-nitro-pyrimidin
   HPLC/MS (Methode B): RT = 3,87 min.; [M+H]+ = 443,2
(523) 2-(4-Chlorphenylamino)-4-(cyano-phenyl-methylamino)-5-nitro-pyrimidin
   HPLC/MS (Methode B): RT = 3,91 min.; [M+H]+ = 459,1
(524) 2-(4-Chlorphenylamino)-4-(N-benzyl-N-[4-hydroxy-1-butyl]-amino)-5-nitro-pyrimidin
   HPLC/MS (Methode B): RT = 3,45 min.; [M+H]+ = 378,2
(525) 2-(4-Chlorphenylamino)-4-(N-benzyl-N-[2-hydroxymethyl-1-cyclohexyl]-amino)-5-nitro-pyrimidin
   HPLC/MS (Methode B): RT = 2,45 min.; [M+H]+ = 468,33
(526) 2-(4-Chlorphenylamino)-4-(1-carboxy-2-(4-hydroxyphenyl)-1-ethyl-amino)-5-nitro-pyrimidin
   HPLC/MS (Methode B): RT = 2,43 min.; [M+H]+ = 430,24
(527) 2-(4-Chlorphenylamino)-4-(*(1S)*-1-carboxy-2-hydroxy-1-ethyl-amino)-5-nitro-pyrimidin
(528) 2-(4-Chlorphenylamino)-4-(5-carboxy-1-pentyl-amino)-5-nitro-pyrimidin
   HPLC/MS (Methode B): RT = 3 min.; [M+H]+ = 380,0
(529) 2-(4-Chlorphenylamino)-4-(1-carboxy-2-aminocarbonyl-1-ethyl-amino)-5-nitro-pyrimidin
   HPLC/MS (Methode B): RT = 1,88 min.; [M+H]+ = 381,15
(530) 2-(4-Chlorphenylamino)-4-(2-carboxy-2-propyl-amino)-5-nitro-pyrimidin
   HPLC/MS (Methode B): RT = 2,59 min.; [M+H]+ = 352,2
(531) 2-(4-Chlorphenylamino)-4-(1-carboxy-3-methyl-1-propylamino)-5-nitro-pyrimidin
   HPLC/MS (Methode B): RT = 2,8 min.; [M+H]+ = 366,19
(532) 2-(4-Chlorphenylamino)-4-(*(1R)*-1-carboxy-2-hydroxy-1-ethyl-amino)-5-nitro-pyrimidin
   HPLC/MS (Methode B): RT = 2,03 min.; [M+H]+ = 354,19
(533) 2-(4-Chlorphenylamino)-4-(4-(2-amino-1-ethyl)-1-piperazinyl)-5-nitro-pyrimidin
(534) 2-(4-Chlorphenylamino)-4-(3,5-dimethyl-1-piperazinyl)-5-nitro-pyrimidin
   HPLC/MS (Methode B): RT = 2,28 min.; [M+H]+ = 363,2
(535) 2-(4-Chlorphenylamino)-4-(cis-2-amino-cyclohexylamino)-5-nitro-pyrimidin
   HPLC/MS (Methode B): RT = 2,51 min.; [M+H]+ = 363,2
(536) 2-(4-Chlorphenylamino)-4-(N-methyl-N-[3-methylamino-1-propyl]-amino)-5-nitro-pyrimidin
(537) 2-(4-Chlorphenylamino)-4-(3-hydroxy-8-aza-bicyclo[3.2.1]oct-8-yl)-5-nitro-pyrimidin
   HPLC/MS (Methode B): RT = 3,04 min.; [M+H]+ = 376,2
(538) 2-(4-Chlorphenylamino)-4-(3-amino-1-pyrrolidinyl)-5-nitro-pyrimidin
   HPLC/MS (Methode B): RT = 2,16 min.; [M+H]+ = 335,1
(539) 2-(4-Chlorphenylamino)-4-(4-benzyloxycarbonyl-1-piperazinyl)-5-nitro-pyrimidin
   HPLC/MS (Methode B): RT = 3,57 min.; [M+H]+ = 469,2
(540) 2-(4-Chlorphenylamino)-4-(3-(2-(3-amino-1-propoxy)-1-ethoxy)-1-propylamino)-5-nitro-pyrimidin
   HPLC/MS (Methode B): RT = 2,46 min.; [M+H]+ = 425,2
(541) 2-(4-Chlorphenylamino)-4-(N-benzyl-N-[2-hydroxy-1-phenyl-1-ethyl]-amino)-5-nitro-pyrimidin
   HPLC/MS (Methode B): RT = 3,66 min.; [M+H]+ = 476,1
(542) 2-(4-Chlorphenylamino)-4-(1-homopiperazinyl)-5-nitro-pyrimidin
   HPLC/MS (Methode B): RT = 2,22 min.; [M+H]+ = 349,1
(543) 2-(4-Chlorphenylamino)-4-(2-(2-(2-hydroxy-1-ethoxy)-1-ethoxy)-1-ethylamino)-5-nitro-pyrimidin
   HPLC/MS (Methode B): RT = 2,73 min.; [M+H]+ = 398,2
(544) 2-(4-Chlorphenylamino)-4-(2-(3-methoxycarbonylphenyl)-1-ethyl-amino)-5-nitro-pyrimidin
   HPLC/MS (Methode B): RT = 3,58 min.; [M+H]+ = 428,2
(545) 2-(4-Chlorphenylamino)-4-(2-hydroxy-3-(4-morpholinyl)-1-propyl-amino)-5-nitro-pyrimidin
   HPLC/MS (Methode B): RT = 2,14 min.; [M+H]+ = 409,2
(546) 2-(4-Chlorphenylamino)-4-(N-methyl-N-[2-nitrobenzyl]-amino)-5-nitro-pyrimidin
(547) 2-(4-Chlorphenylamino)-4-(2-carboxy-1-phenyl-1-ethyl-amino)-5-nitro-pyrimidin
(548) 2-(4-Chlorphenylamino)-4-(N-methyl-N-[6-[N-methyl-N-(2-phenyl-1-ethyl)-amino]-1-hexyl]-amino)-5-nitro-pyrimidin
   HPLC/MS (Methode B): RT = 2,95 min.; [M+H]+ = 497,4
(549) 2-(4-Chlorphenylamino)-4-(2-(2-(2-amino-1-ethyl)-phenyl)-1-ethyl-amino)-5-nitro-pyrimidin
   HPLC/MS (Methode B): RT = 2,65 min.; [M+H]+ = 413,2

(550) 2-(4-Chlorphenylamino)-4-(N-[2-diethylamino-1-ethyl]-N-ethyl-amino)-5-nitro-pyrimidin HPLC/MS (Methode B): RT = 2,51 min.; [M+H]+ = 393,1
(551) 2-(4-Chlorphenylamino)-4-(2-ethoxycarbonyl-1-piperidinyl)-5-nitro-pyrimidin
   HPLC/MS (Methode B): RT = 3,81 min.; [M+H]+ = 406,2
(552) 2-(4-Chlorphenylamino)-4-(4-methyl-1-homopiperazinyl)-5-nitro-pyrimidin
   HPLC/MS (Methode B): RT = 2,26 min.; [M+H]+ = 363,2
(553) 2-(4-Chlorphenylamino)-4-(N-cyanomethyl-N-butyl-amino)-5-nitro-pyrimidin
   HPLC/MS (Methode B): RT = 3,52 min.; [M+H]+ = 361,1
(554) 2-(4-Chlorphenylamino)-4-(N-[2-dimethylamino-1-ethyl]-N-methyl-amino)-5-nitro-pyrimidin
   HPLC/MS (Methode B): RT = 2,21 min.; [M+H]+ = 351,1
(555) 2-(4-Chlorphenylamino)-4-(2-(1-pyrrolidinyl-methyl)-1-pyrrolidinyl)-5-nitro-pyrimidinHPLC/MS (Methode B): RT = 2,41 min.; [M+H]+ = 403,2
(556) 2-(4-Chlorphenylamino)-4-(3-methoxycarbonylmethyl-1-piperidinyl)-5-nitro-pyrimidin
   HPLC/MS (Methode B): RT = 3,47 min.; [M+H]+ = 406,2
(557) 2-(4-Chlorphenylamino)-4-(3-(3-diethylamino-1-propyl)-1-piperidinyl)-5-nitro-pyrimidin
   HPLC/MS (Methode B): RT = 2,75 min.; [M+H]+= 447,4
(558) 2-(4-Chlorphenylamino)-4-(5-hydroxy-2-methyl-2,8-diaza-spiro[5.5]undec-8-yl)-5-nitro-pyrimidin
   HPLC/MS (Methode B): RT = 2,26 min.; [M+H]+ = 433,2
(559) 2-(4-Chlorphenylamino)-4-(3-(1-pyrrolidinyl-methyl)-1-piperidinyl)-5-nitro-pyrimidin
   HPLC/MS (Methode B): RT = 2,5 min.; [M+H]+ = 417,2
(560) 2-(4-Chlorphenylamino)-4-(3-carboxy-1-piperidinyl)-5-nitro-pyrimidin
   HPLC/MS (Methode B): RT = 2,83 min.; [M+H]+ = 378,2
(561) 2-(4-Chlorphenylamino)-4-(2-(2-(2-dimethylamino-1-ethyl)-1-piperidinyl)-1-ethylamino)-5-nitro-pyrimidin
   HPLC/MS (Methode B): RT = 1,82 min.; [M+H]+ = 448,2
(562) 2-(4-Chlorphenylamino)-4-(3-(2-diethylaminomethyl-1-piperidinyl)-1-propylamino)-5-nitro-pyrimidin
   HPLC/MS (Methode B): RT = 1,97 min.; [M+H]+ = 476,2
(563) 2-(4-Chlorphenylamino)-4-(4-(8-methyl-8-aza-bicyclo[3.2.1]oct-3-yl)-1-piperazinyl)-5-nitro-pyrimidin
   HPLC/MS (Methode B): RT = 2,04 min.; [M+H]+ = 458,3
(564) 2-(4-Chlorphenylamino)-4-(1-carboxy-2-(4-chlorphenyl)-1-ethyl-amino)-5-nitro-pyrimidin
   HPLC/MS (Methode B): RT = 3,13 min.; [M+H]+ = 448,25
(565) 2-(4-Chlorphenylamino)-4-(carboxymethylaminocarbonylmethylamino)-5-nitro-pyrimidin
   HPLC/MS (Methode B): RT = 1,93 min.; [M+H]+ = 381,29
(566) 2-(4-Chlorphenylamino)-4-(carboxy-phenyl-methylamino)-5-nitro-pyrimidin
   HPLC/MS (Methode B): RT = 2,87 min.; [M+H]+ = 400,2
(567) 2-(4-Chlorphenylamino)-4-(4'-hydroxy-biphenyl-4-ylmethylamino)-5-nitro-pyrimidin
   HPLC/MS (Methode B): RT = 3,4 min.; [M+H]+ = 448,1
(568) 2-(4-Chlorphenylamino)-4-(N-[4-amino-benzyl]-N-[2-methoxy-1-ethyl]-amino)-5-nitro-pyrimidin
(569) 2-(4-Chlorphenylamino)-4-(4-hydroxy-benzylamino)-5-nitro-pyrimidin
   HPLC/MS (Methode B): RT = 3,01 min.; [M+H]+ = 372,1
(570) 2-(4-Chlorphenylamino)-4-(2-diphenylmethoxy-1-ethylamino)-5-nitro-pyrimidin
(571) 2-(4-Chlorphenylamino)-4-(N-aminocarbonylmethyl-N-methyl-amino)-5-nitro-pyrimidin
(572) 2-(4-Chlorphenylamino)-4-(2-methylaminocarbonyl-1-ethylamino)-5-nitro-pyrimidin
(573) 2-(4-Chlorphenylamino)-4-(2-dimethylaminocarbonyl-1-ethylamino)-5-nitro-pyrimidin
(574) 2-(4-Chlorphenylamino)-4-(2-(4-methyl-1-piperazinyl)-carbonyl-1-ethylamino)-5-nitro-pyrimidin
(575) 2-(4-Chlorphenylamino)-4-(4-carboxy-3-thiazolidinyl)-5-nitro-pyrimidin
   HPLC/MS (Methode B): RT = 2,6 min.; [M+H]+ = 382
(576) 2-(4-Chlorphenylamino)-4-(*(R)*carboxy-(4-hydroxyphenyl)-methylamino)-5-nitro-pyrimidin
   HPLC/MS (Methode B): RT = 2,44 min.; [M+H]+ = 416,2
(577) 2-(4-Chlorphenylamino)-4-(1-carboxy-5-benryloxycarbonylamino-1-pentylamino)-5-nitro-pyrimidin
   HPLC/MS (Methode B): RT = 3,01 min.; [M+H]+ = 529,14
(578) 2-(4-Chlorphenylamino)-4-(1-(*1H*-benzimidazol-2-yl)-1-ethylamino)-5-nitro-pyrimidin
   HPLC/MS (Methode B): RT = 2,21 min.; [M+H]+ = 410,1
(579) 2-(4-Chlorphenylamino)-4-(4-(4-etlxoxycarbonyl-1-piperidinyl)-1-piperidinyl)-5-nitro-pyrimidin
   HPLC/MS (Methode B): RT = 2,6 min.; [M+H]+ = 489,3
(580) 2-(4-Chlorphenylamino)-4-(4-(3-hydroxy-1-piperidinyl)-1-piperidinyl)-5-nitro-pyrimidin
   HPLC/MS (Methode B): RT = 2,31 min.; [M+H]+ = 433,3
(581) 2-(4-Chlorphenylamino)-4-(N-methyl-N-[2-pyrazinyl-methyl]-amino)-5-nitro-pyrimidin
   HPLC/MS (Methode B): RT = 2,91 min.; [M+H]+ = 372,1
(582) 2-(4-Chlorphenylamino)-4-(*(S)*carboxy-(4-hydroxyphenyl)-methylamino)-5-nitro-pyrimidin
   HPLC/MS (Methode B): RT = 2,47 min.; [M+H]+ = 416,09
(583) 2-(4-Chlorphenylamino)-4-(1-phenylsulfonyl-4-piperidinylamino)-5-nitro-pyrimidin
(584) 2-(4-Chlorphenylamino)-4-(4-(4-hydroxyphenyl)-1-butylamino)-5-nitro-pyrimidin HPLC/MS (Methode B): RT = 3,38 min.; [M+H]+ = 414,3
(585) N-(2-Methyl-2-{2-[4-(morpholin-4-sulfonyl)-phenylamino]-5-trifluormethyl-pyrimidin-4-ylamino}-propyl)-acetamid
   Schmelzpunkt: 69-70 °C
   Rf-Wert: 0,39 (Kieselgel; Ethylacetat)
   HPLC/MS (Methode D): RT = 5,86 min.; [M+H]+ = 517; Abs./max 302 nm
(586) 2-(4-Chlorphenylamino)-4-(4-(3-carboxy-1-propyl)-cyclohexylamino)-5-nitro-pyrimidin
(587) 2-(4-Chlorphenylamino)-4-(4-(2-carboxy-1-ethyl)-cyclohexylmethylamino)-5-nitro-pyrimidin
   HPLC/MS (Methode B): RT = 3,41 min.; [M+H]+ = 434,3
(588) 2-(4-Chlorphenylamino)-4-(1-carboxy-2-propylamino)-5-nitro-pyrimidin
   HPLC/MS (Methode B): RT = 2,95 min.; [M+H]+ = 352,2
(589) 2-(4-Chlorphenylamino)-4-(2-(2-hydroxyphenyl)-1-ethylamino)-5-nitro-pyrimidin
(590) (2-[2-(4-Carbamoyl-phenylamino)-5-trifluormethyl-pyrimidin-4-ylamino]-ethyl)-3-yl)-methyl-carbaminsäure-tert-butylester
   Schmelzpunkt: 186-187°C
   R_{f} = 0,24 (Kieselgel; Methylenchlorid: Methanol = 9:1)
   HPLC/MS (Methode G): RT = 3,58 min.; [M+H]+ = 455; Abs. λ max = 279,8 nm
(591) 2-(4-Chlorphenylamino)-4-(3-carboxy-benzylamino)-5-nitro-pyrimidin
   HPLC/MS (Methode B): RT = 3,06 min.; [M+H]+ = 400,2
(592) 2-(4-Chlorphenylamino)-4-(6-tert.-butyloxycarbonylamino-1-hexylamino)-5-nitro-pyrimidin
   HPLC/MS (Methode B): RT = 3,74 min.; [M+H]+ = 465,0
(593) 2-(4-Chlorphenylamino)-4-(3-tert.-butyloxycarbonylamino-1-propylamino)-5-nitro-pyrimidin
   HPLC/MS (Methode B): RT = 3,41 min.; [M+H]+ = 423,0
(594) 2-(4-Chlorphenylamino)-4-(4-carboxy-1-methyl-4-piperidinylamino)-5-nitro-pyrimidin
   HPLC/MS (Methode B): RT = 1,67 min.; [M+H]+ = 407,3
(595) 2-(4-Chlorphenylamino)-4-(1-carboxy-2-(2-chlorphenyl)-1-ethylamino)-5-nitro-pyrimidin
   HPLC/MS (Methode B): RT = 3,1 min.; [M+H]+ = 448,18
(596) 2-(4-Chlorphenylamino)-4-(N-benzyl-N-[1-methoxycarbonyl-1-ethyl]-amino)-5-nitro-pyrimidin
   HPLC/MS (Methode B): RT = 3,72 min.; [M+H]+ = 442,2
(597) 2-(4-Chlorphenylamino)-4-(N-[ethoxycarbonylmethyl]-N-isopropyl-amino)-5-nitro-pyrimidin
   HPLC/MS (Methode B): RT = 3,53 min.; [M+H]+ = 394,1
(598) 2-(4-Chlorphenylamino)-4-(2-(2-ethoxycarbonyl-1-ethyl)-1-pyrrolidinyl)-5-nitro-pyrimidin
(599) 2-(4-Chlorphenylamino)-4-(carbamimidoyl-methylamino)-5-nitro-pyrimidin
   HPLC/MS (Methode B): RT = 1,51 min.; [M+H]+ = 322,22

(600) 2-(4-Chlorphenylamino)-4-(N-[4-hydroxycyclohexyl]-N-methylamino)-5-nitro-pyrimidin
(601) 2-(4-Chlorphenylamino)-4-(2-(6-methoxy-1*H*-benzimidazol-2-yl)-1-ethyl)-5-nitro-pyrimidin
   HPLC/MS (Methode B): RT = 2,31 min.; [M+H]+ = 440,1
(602) 2-(4-Chlorphenylamino)-4-(2-carboxy-benzylamino)-5-nitro-pyrimidin
   HPLC/MS (Methode B): RT = 2,88 min.; [M+H]+ = 400,25
(603) 2-(4-Chlorphenylamino)-4-(2-aminocarbonyl-1,3-dihydro-isoindol-5-yl-methylamino)-5-nitro-pyrimidin
   HPLC/MS (Methode B): RT = 2,84 min.; [M+H]+ = 440,2
(604) 2-(4-Chlorphenylamino)-4-(3-(tert.-butyloxycarbonylaminomethyl)-cyclohexylmethylamino)-5-nitro-pyrimidin
   HPLC/MS (Methode B): RT = 3,97 min.; [M+H]+ = 491,0
(605) 2-(4-Chlorphenylamino)-4-(2-methyl-4-phenylaminocarbonylamino-2-butylamino)-5-nitro-pyrimidin
   HPLC/MS (Methode B): RT = 3,18 min.; [M+H]+ = 470,1
(606) 2-(4-Chlorphenylamino)-4-(3-dimethylaminocarbonyl-1-piperidinyl)-5-nitro-pyrimidin
   HPLC/MS (Methode B): RT = 2,99 min.; [M+H]+ = 405,2
(607) 2-(4-Chlorphenylamino)-4-(2-hydroxy-cyclohexylamino)-5-nitro-pyrimidin
   HPLC/MS (Methode B): RT = 2,2 min.; [M+H]+ = 382,1
(608) 2-(4-Chlorphenylamino)-4-(2-hydroxymethyl-1-piperidinyl)-5-nitro-pyrimidin
   HPLC/MS (Methode B): RT = 3,08 min.; [M+H]+ = 364,2
(609) 2-(4-Chlorphenylamino)-4-(N-methyl-N-[2-pyridyl-methyl]-amino)-5-nitro-pyrimidin
   HPLC/MS (Methode B): RT = 2,34 min.; [M+H]+ = 371,1
(610) 2-(4-Chlorphenylamino)-4-(N-methyl-N-[3-pyridyl-methyl]-amino)-5-nitro-pyrimidin
   HPLC/MS (Methode B): RT = 2,26 min.; [M+H]+ = 371,1
(611) 2-(4-Chlorphenylamino)-4-(1-ethyl-3-piperidinylamino)-5-nitro-pyrimidin
   HPLC/MS (Methode B): RT = 2,34 min.; [M+H]+ = 377,2
(612) 2-(4-Chlorphenylamino)-4-(2-dimethylamino-cyclohexylamino)-5-nitro-pyrimidin
   HPLC/MS (Methode B): RT = 2,53 min.; [M+H]+ = 391,2
(613) 2-(4-Chlorphenylamino)-4-(4-(3-hydroxy-1-propyl)-1-piperidinyl)-5-nitro-pyrimidin
   HPLC/MS (Methode B): RT = 3,16 min.; [M+H]+ = 392,2
(614) 2-(4-Chlorphenylamino)-4-(4-(3-hydroxy-1-propyl)-1-piperazinyl)-5-nitro-pyrimidin
   HPLC/MS (Methode B): RT = 2,19 min.; [M+H]+ = 393,2
(615) 2-(4-Chlorphenylamino)-4-(N-methyl-N-[3-(4-pyridyl)-1-propyl]-amino)-5-nitro-pyrimidin
   HPLC/MS (Methode B): RT = 2,39 min.; [M+H]+ = 399,2
(616) 2-(4-Chlorphenylamino)-4-(4-dimethylamino-2-phenyl-1-butylamino)-5-nitro-pyrimidin HPLC/MS (Methode B): RT = 2,6 min.; [M+H]+ = 441,2
(617) 2-(4-Chlorphenylamino)-4-(2-(3-diethylamino-1-propyl)-1-piperidinyl)-5-nitro-pyrimidin
   HPLC/MS (Methode B): RT = 2,77 min.; [M+H]+ = 447,3
(618) 2-(4-Chlorphenylamino)-4-(bis-[3-pyridylmethyl]-amino)-5-nitro-pyrimidin
   HPLC/MS (Methode B): RT = 1,85 min.; [M+H]+ = 448,2
(619) 2-(4-Chlorphenylamino)-4-(4-(N-methyl-N-[2-methoxycarbonyl-1-ethyl]-amino)-1-piperidinyl)-5-nitro-pyrimidin
   HPLC/MS (Methode B): RT = 2,11 min.; [M+H]+ = 449,3
(620) 2-(4-Chlorphenylamino)-4-(2-(4-(2*H*-pyridazin-3-on-6-yl)-phenyl)-1-ethylamino)-1-piperidinyl)-5-nitro-pyrimidin
(621) 2-(4-Chlorphenylamino)-4-(3-(4-amino-3,5-dichlorphenyl)-1-propylamino)-5-nitro-pyrimidin
(622) 2-(4-Chlorphenylamino)-4-(4-(2-(N-[dimethylaminocarbonylmethyl]-N-methyl-amino)-1-ethyl-amino)-1-piperidinyl)-5-nitro-pyrimidin
   HPLC/MS (Methode B): RT = 2,21 min.; [M+H]+ = 476,34
(623) 2-(4-Chlorphenylamino)-4-(2-(2-(2-diethylamino-1-ethoxy)-1-ethyl)-1-piperidinyl)-5-nitro-pyrimidin
(624) 2-(4-Chlorphenylamino)-4-(4-(2-(2-diethylamino-1-ethoxy)-1-ethyl)-1-piperidinyl)-5-nitro-pyrimidin
(625) 2-(4-Chlorphenylamino)-4-(5-(3-carboxy-1-propyl)-indan-2-ylamino)-5-nitro-pyrimidin
(626) 2-(4-Chlorphenylamino)-4-(2-ethoxycarbonyl-1-(3-pyridyl)-1-ethylamino)-5-nitro-pyrimidin
   HPLC/MS (Methode B): RT = 2,43 min.; [M+H]+ = 443,2
(627) 2-(4-Chlorphenylamino)-4-(1,1-dimethyl-3-(2-oxo-3-pyridin-4-yl-imidazolidin-1-yl)-propylamino)-5-nitro-pyrimidin
   HPLC/MS (Methode B): RT = 2,4 min.; [M+H]+ = 497,0
(628) 2-(2-Brom-benzylamino)-4-(S-oxido-thiomorpholino)-5-nitro-pyrimidin
   Hergestellt aus 2-Chlor-4-thiocyanato-5-nitro-pyrimidin, 2-Brombenzylamin und Hünigbase in Dioxan, Eindampfen des Reaktionsgemisches und weitere Umsetzung mit Thiomorpholin-S-oxid und Hünigbase in DMF. (Das Zwischenprodukt wurde nicht isoliert.)
   Smp.: 246-250°C
   Rf = 0.41 (Kieselgel; Cyclohexan:Essigsäureethylester:Methanol = 10 : 8 : 2)
(629) 2-(4-Chlorphenylamino)-4-morpholino-5-nitro-pyrimidin
   Schmelzpunkt: 218-220°C
(630) 2-(4-Chlorphenylamino)-4-(2-cyanethylamino)-5-nitro-pyrimidin
   Schmelzpunkt: 203°C
(631) 2-(4-Chlorphenylamino)-4-(ethoxycarbonylmethylamino)-5-nitro-pyrimidin
   Schmelzpunkt: 202-204°C
(632) 2-(4-Chlorphenylamino)-4-[2-(ethoxycarbonyl)ethylamino]-5-nitro-pyrimidin
   Schmelzpunkt: 163-165°C
(633) 2-(3,4-Dichlorphenylamino)-4-[3-(dimethylamino)propylamino]-5-nitro-pyrimidin
   Schmelzpunkt: 168-170°C
(634) 2-(3,4-Dichlorphenylamino)-4-(2-hydroxyethylamino)-5-nitro-pyrimidin
   Schmelzpunkt: 196°C
(635) 2-(3,4-Dichlorphenylamino)-4-(2-methoxyethylamino)-5-nitro-pyrimidin
   Schmelzpunkt: 165°C
(636) 2-(3,4-Dichlorphenylamino)-4-[2-(dimethylamino)ethylamino]-5-nitro-pyrimidin
   Schmelzpunkt: 175-176°C
(637) 2-(3,4-Dichlorphenylamino)-4-(2-morpholinoethylamino)-5-nitro-pyrimidin
   Schmelzpunkt: 190°C
(638) 2-(3,4-Dichlorphenylamino)-4-[4-(dimethylamino)butylamino]-5-nitro-pyrimidin
   Schmelzpunkt: 110°C
(639) 2-(3,4-Dichlorphenylamino)-4-[(2-ethoxycarbonyl-ethyl)amino]-5-nitro-pyrimidin
   Schmelzpunkt: 137°C
(640) 2-(4-Chlorphenylamino)-4-[2-(methansulfonylamino)ethylamino]-5-nitro-pyrimidin
   Hergestellt aus der Verbindung 140 des Beispiels 1 durch nachträgliche Umsetzung mit
   Methansulfonylchlorid/Triethylamin.
   Schmelzpunkt: 231-235°C
(641) 2-(4-Chlorphenylamino)-4-(carboxymethylamino)-5-nitro-pyrimidin
   Hergestellt aus der Verbindung 631 des Beispiels 1 durch nachträgliche Umsetzung mit 1N Natronlauge in Tetrahydrofuran.
   Schmelzpunkt: >300°C
   Rf-Wert: 0,36 (Kieselgel; Methylenchlorid/Methanol = 9:1)
(642) 2-(3,4-Dichlorphenylamino)-4-[(2-carboxyethyl)amino]-5-nitro-pyrimidin
   Hergestellt aus der Verbindung 639 des Beispiels 1 durch nachträgliche Umsetzung mit 1N Natronlauge in Tetrahydrofuran.
   Rf-Wert: 0,16 (Kieselgel; Cyclohexan/Essigsäureethylester/Methanol = 7:2:1)
(643) 2-(3-Bromphenylamino)-4-[1-hydroxy-3-methyl-2-butylamino]-5-trifluormethyl-pyrimidin
   Hergestellt aus der Verbindung 5 des Beispiels 3.
(644) 2-[4-(1,2,4,5-Tetrahydro-benzo[*d*]azepin-3-yl-methyl)-phenylamino]-4-morpholino-5-trifluormethyl-pyrimidin
   Schmelzpunkt: 172°C
(645) 2-[4-(1,2,4,5-Tetrahydro-benzo[*d*]azepin-3-yl-methyl)-phenylamino]-4-(4-methyl-1-piperazinyl)-5-trifluormethyl-pyrimidin
   Schmelzpunkt: 217°C (Zersetzung)
(646) 2-[4-(1,2,4,5-Tetrahydro-benzo[*d*]azepin-3-yl-methyl)-phenylamino]-4-(1,4,6,7-tetrahydro-imidazo[4,5-c]pyridin-5-yl)-5-trifluormethyl-pyrimidin
   Schmelzpunkt: 350°C (Zersetzung)
(647) 2-[4-(1,2,4,5-Tetrahydro-benzo[*d*]azepin-3-yl-methyl)-phenylamino]-4-[(2-carboxyethyl)amino]-5-trifluormethyl-pyrimidin
   Schmelzpunkt: 120°C (Zersetzung)
   Hergestellt unter Verwendung von beta-Alanin in Natronlauge.
(648) 2-[4-(1,2,4,5-Tetrahydro-benzo[*d*]azepin-3-yl-methyl)-phenylamino]-4-(trans-4-dimethylamino-cyclohexylamino)-5-trifluormethyl-pyrimidin-dihydrochlorid
   Schmelzpunkt: 293°C (Zersetzung)
(649) 2-[4-(1,2,4,5-Tetrahydro-benzo[*d*]azepin-3-yl-methyl)-phenylamino]-4-(2-acetylamino-ethylamino)-5-trifluormethyl-pyrimidin-dihydrochlorid
   Schmelzpunkt: 205°C (Zersetzung)

(650) 4-[4-(2-Methylamino-ethylamino)-5-trifluormethyl-pyrimidin-2-ylamino]-benzamid
   Herstellung analog 1(80).
   Schmelzpunkt: 187-190 °C
   R_{f} = 0,08 (Kieselgel; Methylenchlorid: Methanol = 4:1)
   HPLC/MS (Methode G): RT = 2,45 min.; [M+H]+= 355; Abs. λ max = 277,9 nm
(651) N-{2-[2-(3-Dimethylsulfamoyl-phenylamino)-5-trifluormethyl-pyrimidin-4-ylamino]-ethyl}-acetamid
   Schmelzpunkt: 202-203 °C
   Rf-Wert: 0,57 (Kieselgel; Methylenchlorid/Methanol = 9:1)
   HPLC/MS (Methode D): RT = 5,33 min.; [M+H]+ = 447; Abs./max 235 nm
(652) N-{2-[5-Bromo-2-(4-dimethylsulfamoyl-phenylamino)-pyrimidin-4-ylamino]-ethyl}-acetamid
   Schmelzpunkt: 226
   Rf-Wert: 0,42 (Kieselgel; Methylenchlorid/Methanol = 9:1)
   HPLC/MS (Methode D): RT = 4,84 min.; [M+H]+ = 459; Abs./max 273 nm
(653) N-(2-{2-[3-(Morpholin-4-sulfonyl)-phenylamino]-5-trifluormethyl-pyrimidin-4-ylamino}-ethyl)-acetamid
   Schmelzpunkt: 214°C
   Rf-Wert: 0,51 (Kieselgel; Methylenchlorid/Methanol = 9:1)
   HPLC/MS (Methode D): RT = 5,31 min.; [M+H]+ = 489; Abs./max 235 nm
(654) N-{2-[2-(1-Methyl-1H-indazol-6-ylamino)-5-trifluormethyl-pyrimidin-4-ylamino]-ethyl}-acetamid
   Schmelzpunkt: 260-261 °C
   Rf-Wert: 0,39 (Kieselgel; Methylenchlorid/Methanol = 9:1)
   HPLC/MS (Methode D): RT = 4,78 min.; [M+H]+ = 394; Abs./max 250 nm
(655) N-{2-[2-(1-Methyl-1H-indazol-5-ylamino)-5-trifluormethyl-pyrimidin-4-ylamino]-ethyl}-acetamid
   Schmelzpunkt: 265-266 °C
   Rf-Wert: 0,25 (Kieselgel; Methylenchlorid/Methanol = 9:1)
   HPLC/MS (Methode D): RT = 4,47 min.; [M+H]+ = 394; Abs./max 254 nm
(656) N-{2-[2-(2-Methyl-2H-indazol-6-ylamino)-5-trifluormethyl-pyrimidin-4-ylamino]-ethyl}-acetamid
   Schmelzpunkt: 254-255 °C
   Rf-Wert: 0,45 (Kieselgel; Methylenchlorid/Methanol = 9:1)
   HPLC/MS (Methode D): RT = 4,45 min.; [M-H]- = 394; Abs./max 250 nm
(657) {2-[5-Bromo-2-(3-dimethylsulfamoyl-phenylamino)-pyrimidin-4-ylamino]-ethyl}-acetamid
   Schmelzpunkt: 192 °C
   Rf-Wert: 0,43 (Kieselgel; Methylenchlorid/Methanol = 9:1)
   HPLC/MS (Methode D): RT = 4,75 min.; [M+H]+ = 459; Abs. λ max = 268 nm
(658) N-(2-{5-Bromo-2-[3-(morpholin-4-sulfonyl)-phenylamino]-pyrimidin-4-ylamino}-ethyl)-acetamid
   Schmelzpunkt: 212 °C
   Rf-Wert: 0,47 (Kieselgel; Methylenchlorid/Methanol = 9:1)
   HPLC/MS (Methode D): RT = 4,76 min.; [M+H]+ = 501; Abs. λ max = 268 nm
(659) N-(2-{2-[4-(2-Dimethylamino-ethyl)-phenylamino]-5-trifluormethyl-pyrimidin-4-ylamino}-ethyl)-acetamid Schmelzpunkt: 110 °C
   R_{f} = 0,13 (Kieselgel; Methylenchlorid: Methanol = 1:2)
   HPLC/MS (Methode D): RT = 1,96 min.; [M+H]+ = 411; Abs. λ max = 256
(660) N-{2-[2-(4-Piperidin-1-ylmethyl-phenylamino)-5-trifluormethyl-pyrimidin-4-ylamino]-ethyl}-acetamid
   Schmelzpunkt: 223-226 °C
   R_{f} = 0.23 (Kieselgel; Methylenchlorid: Methanol = 1:1)
   HPLC/MS (Methode D): RT = 3,94 min.; [M+H]+ = 437; Abs. λ max = 265 nm
(661) 1-{3-[5-Bromo-2-(3,4-dichloro-phenylamino)-pyrimidin-4-ylamino]-propyl}-pyrrolidin-2-on
   Schmelzpunkt: 234 °C
   R_{f} = 0.56 (Kieselgel; Methylenchlorid: Methanol = 9:1)
   HPLC/MS (Methode C): RT = 4,30 min.; [M+H]+ = 460; Abs. λ max = 274 nm
(662) 1-{3-[5-Bromo-2-(3,4-dichloro-phenylamino)-pyrimidin-4-ylamino]-propyl}-pyrrolidin-2-on
   Schmelzpunkt: 216-218 °C
   R_{f} = 0.55 (Methylenchlorid: Methanol = 5:1)
   HPLC/MS (Methode C): RT = 3,11 min.; [M+H]+ = 435; Abd. λ max = 278
(663) 4-[4-(2-Acetylamino-ethylamino)-5-bromo-pyrimidin-2-ylamino]-benzamid
   Schmelzpunkt: 265-266 °C
   R_{f} = 0.22 (Kieselgel; Methylenchlorid: Methanol = 9:1)
   HPLC/MS (Methode D): RT = 3,93 min.; [M+H]+ = 395; Abs. λ max = 278
(664) N-(2-{2-[3-(Benzyl-methyl-sulfamoyl)-phenylamino]-5-trifluormethyl-pyrimidin-4-ylamino}-ethyl)-acetamid
   Schmelzpunkt: 126 °C
   R_{f} = 0.61 (Kieselgel; Methylenchlorid: Methanol = 9: 1)
   HPLC/MS (Methode D): RT = 6,33 min.; [M+H]+ = 523; Abs. λ max = 238 nm
(665) N-(2-{2-[3-(4-Methyl-piperazin-1-sulfonyl)-phenylamino]-5-trifluormethyl-pyrimidin-4-ylamino}-ethyl)-acetamid
   Schmelzpunkt: 247 °C
   R_{f} = 0.44 (Kieselgel; Methylenchlorid: Methanol = 9:1)
   HPLC/MS (Methode D): RT = 4,36 min.; [M+H]+ = 502; Abs. λ max = 234 nm
(666) 4-{4-[(3-Aminomethyl-cyclohexylmethyl)-amino]-5-trifluormethyl-pyrimidin-2-ylamino}-benzamid
   Schmelzpunkt: 209-212°C
   R_{f} = 0,03 (Kieselgel; Methylenchlorid: Methanol = 4:1)
   HPLC/MS (Methode G): RT = 2,87 min.; [M+H]+ = 423; Abs. λ max = 279,8nm
(667) N-{2-[2-(4-Morpholin-4-ylmethyl-phenylamino)-5-trifluormethyl-pyrimidin-4-ylamino]-ethyl}-acetamid
   Schmelzpunkt: 70 °C
   R_{f} = 0.69 (Kieselgel; Methylenchlorid: Methanol = 9:1)
   HPLC/MS (Methode D): RT = 3,98 min.; [M+H]+ = 439; Abs. λ max = 266 nm
(668) N-{2-[2-(4-Cyano-3-trifluormethyl-phenylamino)-5-trifluormethyl-pyrimidin-4-ylamino]-ethyl}-acetamid
   Schmelzpunkt: 237 °C
   R_{f} = 0.50 (Kieselgel; Methylenchlorid: Methanol = 9:1)
   HPLC/MS (Methode D): RT = 6,35 min.; [M+H]+ = 433; Abs. λ max = 318 nm
(669) N-{2-[2-(3-Chloro-4-cyano-phenylamino)-5-trifluormethyl-pyrimidin-4-ylamino]-ethyl}-acetamid
   Schmelzpunkt: 250 °C
   R_{f} = 0.45 (Kieselgel; Methylenchlorid: Methanol = 9:1)
   HPLC/MS (Methode D): RT = 6,20 min.; [M+H]+ = 399; Abs. λ max = 309 nm
(670) 1-(2-{[2-(3,4-Dichloro-phenylamino)-5-trifluormethyl-pyrimidin-4-yl]-methyl-amino}-ethyl)-pyrrolidin-2-on
   Schmelzpunkt: 164 °C
   R_{f} = 0.11 (Kieselgel; Hexan: Essigsäureethylester =1:1)
   HPLC/MS (Methode D): RT = 6,862 min.; [M+H]+ = 450; Abs. λ max = 270 nm
(671) 1-{2-[2-(3,4-Dichloro-phenylamino)-5-trifluormethyl-pyrimidin-4-ylamino]-ethyl}-pyrrolidin-2-on
   Schmelzpunkt: 218 °C
   R_{f} = 0.23 (Kieselgel; Hexan: Essigsäureethylester = 1:1)
   HPLC/MS (Methode D): RT = 6,496 min.; [M+H]+ = 436; Abs. λ max = 274 nm
(672) 4-[4-(4-Methansulfonylamino-piperidin-1-yl)-5-trifluormethyl-pyrimidin-2-ylamino]-N,N-dimethyl-phenylsulfonamid
   Schmelzpunkt: 218 °C
   R_{f} = 0.46 (Kieselgel; Hexan: Essigsäureethylester = 33:67)
   HPLC/MS (Methode D): RT = 6,353 min.; [M+H]+ = 523; Abs. λ max = 294 nm
(673) 4-{4-[4-(Methanesulfonyl-methyl-amino)-piperidin-1-yl]-5-trifluormethyl-pyrimidin-2-ylamino}-N,N-dimethyl-Phenylsulfonamid
   Schmelzpunkt: 226 °C
   R_{f} = 0.27 (Kieselgel; Hexan: Essigsäureethylester = 1:1)
   HPLC/MS (Methode D): RT = 6,652 min.; [M+H]+ = 537; Abs. λ max = 294 nm
(674) 3-[2-(1-Methyl-1H-indazol-6-ylamino)-5-trifluormethyl-pyrimidin-4-ylamino]-propylamin
   4,7 g N-Z-1,3-Diaminopropan Hydrochlorid, 10 ml Hünig Base und 5,2 g 2-(1-Methyl-1H-indazol-6-ylamino)-4-chloro-5-trifluormethyl-pyrimidin werden in 160ml Dioxan suspendiert und mit DMF versetzt bis alle Komponenten gelöst sind. Nach 17 Stunden bei 80 °C wird der Ansatz in Ethylacetat aufgenommen, mit H₂O extrahiert, die organische Phase anschließend getrocknet und einrotiert. Das Produkt wird mit Toluol: Ethylacetat (1:1) über Kieselgel chromatographiert. Das Zwischenprodukt wird in Ethanol, Methanol und Toluol (4:1:3, 800ml) gelöst, 1 g Pd(OH)₂ zugegeben und bei 50 psi und 40 °C über 28 Stunden hydriert. Nach dem Abfiltrieren des Katalysators und dem Einengen der Lösung bleibt das Produkt zurück.
   Schmelzpunkt: 201 °C, Subl.
   R_{f} = 0,15 (Kieselgel; Methylenchlorid: Methanol = 95:5)
   HPLC/MS (Methode B): RT = 4,35 min.; [M+H]+ = 366; Abs. λ max = 226 nm
(675) N-{1-[2-(3,4-Dichloro-phenylamino)-5-trifluormethyl-pyrimidin-4-yl]-piperidin-4-yl}-methansulfonamid
   Schmelzpunkt: 200 °C
   R_{f} = 0.42 (Kieselgel; Hexan: Essigsäureethylester = 1:1)
   HPLC/MS (Methode D): RT = 7,105 min.; [M+H]+ = 486; Abs. λ max = 274 nm
(676) (4-Chloro-phenyl)-[4-(1-oxo-1,4-thiomorpholin-4-yl)-5-trifluormethyl-pyrimidin-2-yl]-amin
   Schmelzpunkt: 213-216 °C
   R_{f} = 0.42 (Kieselgel; Hexan: Essigsäureethylester = 2:1)
   HPLC/MS (Methode D): RT = 6,015 min.; [M+H]+ = 391; Abs. λ max = 259 nm
(677) (3-Chloro-phenyl)-[4-(4-pyridin-2-yl-piperazin-1-yl)-5-trifluormethyl-pyrimidin-2-yl]-amin
   Schmelzpunkt: 146-147 °C
   R_{f} = 0.66 (Kieselgel; Methylenchlorid: Methanol = 95:5)
   HPLC/MS (Methode D): RT = 5,203 min.; [M+H]+ = 435; Abs. λ max = 271 nm
(678) 1-{3-[2-(3,5-Dichloro-phenylamino)-5-trifluormethyl-pyrimidin-4-ylamino]-propyl}-pyrrolidin-2-on
   Schmelzpunkt: 174-175 °C
   R_{f} = 0.10 (Kieselgel; Hexan: Essigsäureethylester = 1:1)
   HPLC/MS (Methode D): RT = 6,309 min.; [M+H]+ = 450; Abs. λ max = 230 nm
(679) (3-Chloro-phenyl)-[4-(1-oxo-1-thiomorpholin-4-yl)-5-trifluormethyl-pyrimidin-2-yl]-amin
   Schmelzpunkt: 214-217 °C
   R_{f} = 0.51 (Kieselgel; Methylenchlorid: Methanol = 95:5)
   HPLC/MS (Methode D): RT = 5,571 min.; [M+H]+ = 391; Abs. λ max = 270 nm
(680) (4-Chloro-phenyl)-[4-(4-pyridin-2-yl-piperazin-1-yl)-5-trifluormethyl-pyrimidin-2-yl]-amin
   Schmelzpunkt: 164-166 °C
   R_{f} = 0.43 (Kieselgel; Hexan: Essigsäureethylester : Methanol = 5:4:1)
(681) {1-[2-(4-Chloro-phenylamino)-5-trifluormethyl-pyrimidin-4-yl]-piperidin-4-yl}-essigsäuremethylester
   R_{f} = 0.86 (Kieselgel; Hexan: Essigsäureethylester = 1: 1)
   HPLC/MS (Methode D): RT = 7,521 min.; [M+H]+ = 429; Abs. λ max = 266 nm
(682) {1-[2-(4-Chloro-phenylamino)-5-trifluormethyl-pyrimidin-4-yl]-piperidin-4-yl}-essigsäure
   Schmelzpunkt: 200-201 °C
   R_{f} = 0.44 (Kieselgel; Hexan: Essigsäureethylester = 1:1)
   HPLC/MS (Methode D): RT = 6,623 min.; [M+H]+ = 415; Abs. λ max = 270 nm
(683) N,N-Dimethyl-4-{4-[2-(2-oxo-pyrrolidin-1-yl)-ethylamino]-5-trifluormethyl-pyrimidin-2-ylamino} -phenylsulfonamid
   Schmelzpunkt: 258-261 °C
   R_{f} = 0.14 (Kieselgel; Hexan: Essigsäureethylester = 1:2)
   HPLC/MS (Methode D): RT = 5,828 min.; [M+H]+ = 473; Abs. λ max = 302 nm
(684) N,N-Dimethyl-4-(4-{methyl-[2-(2-oxo-pyrrolidin-1-yl)-ethyl]-amino}-5-trifluormethyl-pyrimidin-2-ylamino)-benzolsulfonamid
   Schmelzpunkt: 147-150 °C
   R_{f} = 0.13 (Kieselgel; Hexan: Essigsäureethylester = 2:1)
   HPLC/MS (Methode D): RT = 6,15 min.; [M+H]+ = 487; Abs. λ max = 290 nm
(685) N-(1,1-Dimethyl-2-{2-[4-(morpholin-4-sulfonyl)-phenylamino]-5-trifluormethyl-pyrimidin-4-ylamino}-ethyl)-acetamid
   Schmelzpunkt: 105-107 °C
   R_{f} = 0.39 (Kieselgel; Essigsäureethylester)
   HPLC/MS (Methode D): RT = 5,89 min.; [M+M]+ = 517; Abs. λ max = 302 nm
(686) N4-Methyl-N4-(2-methylamino-ethyl)-N2-[4-(morpholin-4-sulfonyl)-phenyl]-5-trifluormethyl-pyrimidin-2,4-diamin formiat
   Schmelzpunkt: 184-186 °C
   R_{f} = 0.09 (Kieselgel; Methylenchlorid: Methanol = 9:1)
   HPLC/MS (Methode D): RT = 4,83 min.; [M+H]+ = 475; Abs. λ max = 302 nm
(687) N,N-Dimethyl-4-{4-[methyl-(2-methylamino-ethyl)-amino]-5-trifluormethyl-pyrimidin-2-ylamino}-benzolsulfonamid hydrochlorid
   Schmelzpunkt: 235-238 °C
   R_{f} = 0.10 (Kieselgel; Methylenchlorid: Methanol = 9:1)
   HPLC/MS (Methode D): RT = 4,86 min.; [M+H]+ = 433; Abs. λ max = 298 nm
(688) (2-{2-[4-(Morpholin-4-sulfonyl)-phenylamino]-5-trifluormethyl-pyrimidin-4-ylamino}-ethyl)-kohlensäure benzyl ester
   Schmelzpunkt: 166-169 °C
   R_{f} = 0.61 (Kieselgel; Essigsäureethylester)
   HPLC/MS (Methode D): RT = 6,64 min.; [M+H]+ = 581; Abs. λ max = 306 nm
(689) N4-(2-Amino-ethyl)-N2-[4-(morpholin-4-sulfonyl)-phenyl]-5-trifluormethyl-pyrimidin-2,4-diamin
   Schmelzpunkt: 169-170 °C
   R_{f} = 0.20 (Kieselgel; Methylenchlorid: Methanol =1:1)
   HPLC/MS (Methode D): RT = 4,88 min.; [M+H]+ = 447; Abs. λ max = 302 nm
(690) N-(2-{[2-(4-Dimethylsulfamoyl-phenylamino)-5-trifluormethyl-pyrimidin-4-yl]-methylamino}-ethyl)-N-methyl-acetamid
   Schmelzpunkt: 156-158 °C
   R_{f} = 0.17 (Kieselgel; Essigsäureethylester)
   HPLC/MS (Methode D): RT = 6,12 min.; [M+H]+ = 475; Abs. λ max = 282 nm
(691) N-Methyl-N-[2-(methyl-{2-[4-(morpholin-4-sulfonyl)-phenylamino]-5-trifluormethyl-pyrimidin-4-yl}-amino)-ethyl]-acetamid
   Schmelzpunkt: 158-161 °C
   R_{f} = 0.15 (Kieselgel; Essigsäureethylester)
   HPLC/MS (Methode D): RT = 6,04 min.; [M+H]+ = 517; Abs. λ max = 294 nm
(692) N-(2-{2-[4-(Propane-2-sulfonyl)-phenylamino]-5-trifluormethyl-pyrimidin-4-ylamino}-ethyl)-acetamid
   Schmelzpunkt: 198-200 °C
   R_{f} = 0.60 (Kieselgel; Methylenchlorid: Methanol = 9:1)
   HPLC/MS (Methode C): RT = 3,97 min.; [M+H]+ = 446; Abs. λ max = 302 nm
(693) 4-[4-(3-Amino-propylamino)-5-trifluormethyl-pyrimidin-2-ylamino]-benzamid
   Die Verbindung wurde analog Beispiel 1(674) erhalten.
(694) N,N-Dimethyl-4-[4-(3,4,6,7-tetrahydro-imidazo[4,5-c]pyridin-5-yl)-5-trifluormethyl-pyrimidin-2-ylamino]-phenylsulfonamid
   Schmelzpunkt: 230 °C Zersetzung
   R_{f} = 0.28 (Kieselgel; Methylenchlorid: Methanol = 95:5)
   HPLC/MS (Methode D): RT = 5,20 min.; [M+H]+ = 468; Abs. λ max = 286, 302 nm
(695) [4-(Morpholin-4-sulfonyl)-phenyl]-[4-(3,4,6,7-tetrahydro-imidazo[4,5-5]pyridin-5-yl)-5-trifluormethyl-pyrimidin-2-yl]-amin
   Schmelzpunkt: >270 °C Zersetzung
   R_{f} = 0.33 (Kieselgel; Methylenchlorid: Methanol = 95:5)
   HPLC/MS (Methode D): RT = 5,30 min.; [M+H]+ = 510; Abs. λ max = 302 nm
(696) {3-[2-(1-Methyl-1H-indazol-6-ylamino)-5-trifluormethyl-pyrimidin-4-ylamino]-propyl}-carbaminsäure-benzylester
   Schmelzpunkt: 139 °C
   R_{f} = 0,30 (Kieselgel; Toluol: Essigsäureethylester = 1:1)
   HPLC/MS (Methode D): RT = 6,37 min.; [M+H]+ = 500; Abs. λ max = 250 nm

### Beispiel 2

### 2-(4-Carboxyphenylamino)-4-(2-acetylamino-ethylamino)-5-nitro-pyrimidin-hydrochlorid

Zu 173 mg 2-Chlor-4-(2-acetylamino-ethylamino)-5-nitro-pyrimidin (Verbindung (1) des Beispiels II)in 5 ml Ethanol werden bei Raumtemperatur 100 mg 4-Aminobenzoesäure in 5 ml Ethanol gegeben. Man gibt zwei Tropfen konzentrierter Salzsäure hinzu und rührt 12 h. Dann werden 50 ml Wasser zugegeben. Der Niederschlag wird abgesaugt und an der Luft getrocknet. Der Rückstand wird mit 30 ml Methylenchlorid gerührt, abgesaugt und getrocknet.
Ausbeute: 135 mg (51 % der Theorie), Smp: 290°C (Zersetzung)
R_{f} = 0.2 (Kieselgel; Methylenchlorid:Methanol = 9:1)

Analog Beispiel 2 werden folgende Verbindungen erhalten:
(1) 2-(3,4-Dichlorphenylamino)-4-(2-acetylamino-ethylamino)-5-trifluormethylsulfanyl-pyrimidin-hydrochlorid
   Smp: 196°C
   R_{f} = 0.5 (Kieselgel; Methylenchlorid:Methanol:konz.Ammoniak = 16 : 3 : 1)
   Hergestellt aus der Verbindung (3) des Beispiels II.
(2) 2-(3,4-Dichlorphenylamino)-4-(2-acetylamino-ethylamino)-5-brom-pyrimidin-hydrochlorid
   Smp: 260°C (Zersetzung)
   Hergestellt aus der Verbindung (4) des Beispiels II.
(3) 2-(3,4-Dichlorphenylamino)-4-(2-acetylamino-ethylamino)-5-trifluormethyl-pyrimidin-hydrochlorid
   Smp: 227°C
   Hergestellt aus der Verbindung des Beispiels II.
(4) N-{2-[2-(4-Amino-phenylamino)-5-trifluormethyl-pyrimidin-4-ylamino]-ethyl}-acetamid
   Zu 3 g N-[2-(2-Chloro-5-trifluormethyl-pyrimidin-4-ylamino)-ethyl]-acetamid in 25 ml Eisessig werden 6,9 g p-Phenylendiamin gegeben und der Ansatz über 2 Stunden bei Raumtemperatur gerührt. Nach dem Abziehen der Essigsäure unter reduziertem Druck wird das Rohprodukt in Methylenchlorid aufgenommen und mit 2 M Natriumcarbonatlösung extrahiert. Die wässrige Phase wäscht man mit Methylenchlorid, vereint die organischen Phasen und trocknet über Natriumsulfat. Nach dem Einengen wird das Material über Kieselgel mit Methylenchlorid/Isopropanol (20:1) chromatographiert. Ausbeute: 3g
   Schmelzpunkt: 175 °C Zersetzung
   R_{f} = 0.35 (Kieselgel; Methylenchlorid: Isopropanol = 8:2)
   APCI-MS [M+H]+ = 355
   1H-NMR(D₆-DMSO, 300MHz) δ: 1.80 (s, 3 H), 3.25 (m, 2 H), 3.47 (m, 2 H), 4.78 (bs, 2 H), 6.50 (d, 2 H), 6.93 (bs, 2 H), 7.29 (d, 2 H), 7.90 (t, 1 H), 8.08 (s, 1 H), 9.13 (s, 1 H).
(5) 2-(3,4-Dichlorphenylamino)-4-(2-acetylamino-ethylamino)-5-methylsulfanyl-pyrimidin-hydrochlorid
   Hergestellt aus der Verbindung (2) des Beispiels II.
   Smp: 220°C
(6) 2-(1-Naphthylamino)-4-[N-(trans-4-hydroxy-cyclohexyl)-N-methyl-amino]-5-nitro-pyrimidin
   HPLC/MS (Methode B): RT = 2,55 min.; [M+H]+ = 394,2
(7) 2-(4-Bromphenylamino)-4-(2-acetylamino-ethylamino)-5-trifluormethyl-pyrimidin
   HPLC/MS (Methode A): RT = 5.83min.; [M+H]+ = 420.1
(8) 2-(4-Aminosulfonyl-phenylamino)-4-(2-acetylamino-ethylamino)-5-trifluormethyl-pyrimidin
   HPLC/MS (Methode A): RT = 4.95min.; [M+H]+ = 419.1
(9) 2-(3-Chlor-phenylamino)-4-(2-acetylamino-ethylamino)-5-trifluormethyl-pyrimidin
   HPLC/MS (Methode A): RT = 5.84min.; [M+H]+ = 374.1
(10) 2-(3-Nitro-phenylamino)-4-(2-acetylamino-ethylamino)-5-trifluormethyl-pyrimidin
   Schmelzpunkt: 201-204 °C
   R_{f} = 0.60 (Kieselgel; Methylenchlorid: Methanol = 95:5)
   HPLC/MS (Methode D): RT = 5,398 min.; [M+H]+ = 385; Abs. λ max = 266 nm
(11) 2-(4-(N-Methyl-N-methylsulfonyl)amino-phenylamino)-4-(2-acetylamino-ethylamino)-5-trifluormethyl-pyrimidin
   HPLC/MS (Methode A): RT = 5.15min.; [M+H]+ = 447.2
(12) 2-(3-Brom-phenylamino)-4-(2-acetylamino-ethylamino)-5-trifluormethyl-pyrimidin
   R_{f} = 0.60 (Kieselgel; Cyclohexan: Essigsäureethylester = 1:2)
   HPLC/MS (Methode D): RT = 5,68 min.; [M+H]+ = 419; Abs. λ max = 254 nm
(13) 2-(4-Chlor-3-trifluormethyl-phenylamino)-4-(2-acetylamino-ethylamino)-5-trifluormethyl-pyrimidin
   HPLC/MS (Methode A): RT = 6.71min.; [M+H]+ = 442.1
(14) 2-(4-Brom-3-chlor-phenylamino)-4-(2-acetylamino-ethylamino)-5-trifluormethyl-pyrimidin
   HPLC/MS (Methode A): RT = 6.57min.; [M+H]+ = 454.0
(15) 2-(3,5-Dichlor-phenylamino)-4-(2-acetylamino-ethylamino)-5-trifluormethyl-pyrimidin
   HPLC/MS (Methode A): RT = 6.73min.; [M+H]+ = 408.1
(16) 2-(4-Chlor-phenylamino)-4-(2-acetylamino-ethylamino)-5-trifluormethyl-pyrimidin
   HPLC/MS (Methode A): RT = 5.71 min.; [M+H]+ = 374.1
(17) 2-(4-Morpholino-phenylamino)-4-(2-acetylamino-ethylamino)-5-nitro-pyrimidin
   HPLC/MS (Methode A): RT = 5.22min.; [M+H]+ = 402.2
(18) 2-(4-(N-Methyl-N-methylsulfonyl)amino-phenylamino)-4-(N-(trans-4-hydroxy-cyclohexyl)-N-methyl-amino)-5-nitro-pyrimidin
   HPLC/MS (Methode A): RT = 5.89min.; [M+H]+ = 451.2
(19) 2-(4-Diethylaminomethyl-phenylamino)-4-(2-acetylamino-ethylamino)-5-nitro-pyrimidin
   HPLC/MS (Methode A): RT = 4.7min.; [M+H]+ = 402.2
(20) 2-(1,3-Dihydro-2-oxo-indol-6-ylamino)-4-[N-(trans-4-hydroxy-cyclohexyl)-N-methylamino]-5-nitro-pyrimidin
   HPLC/MS (Methode A): RT = 5.51min.; [M+H]+ = 399.2
(21) 2-(4-(N-Methyl-N-methylsulfonyl)amino-phenylamino)-4-(2-acetylamino-ethylamino)-5-nitro-pyrimidin
   HPLC/MS (Methode A): RT = 5.46min.; [M+H]+ = 424.2
(22) 2-(3-Hydroxy-4-methyl-phenylamino)-4-(trans-4-hydroxy-cyclohexylamino)-5-trifluormethyl-pyrimidin
   HPLC/MS (Methode B): RT = 1,61 min.; [M+H]+ = 383,3
(23) 2-(3-Hydroxy-4-methyl-phenylamino)-4-[N-(trans-4-hydroxy-cyclohexyl)-N-methyl-amino]-5-nitro-pyrimidin
   HPLC/MS (Methode B): RT = 2,2 min.; [M+H]+ = 374,2
(24) 2-(6-Indazolylamino)-4-[N-(trans-4-hydroxy-cyclohexyl)-N-methyl-amino]-5-nitro-pyrimidin
   HPLC/MS (Methode B): RT = 2,26 min.; [M+H]+ = 384,2
(25) 2-(1-Naphthylamino)-4-(trans-4-hydroxy-cyclohexylamino)-5-trifluormethyl-pyrimidin
(26) 2-(4-(3-Diethylamino-1-propyloxy)-phenylamino)-4-(2-pyridyl-methylamino)-5-nitro-pyrimidin
   HPLC/MS (Methode B): RT = 1,26 min.; [M+H]+ = 452,3
(27) 2-(3-Hydroxy-4-methyl-phenylamino)-4-(2-acetylamino-1-ethylamino)-5-trifluormethyl-pyrimidin
   HPLC/MS (Methode B): RT = 1,49 min.; [M+H]+ = 370,2
(28) 2-(4-Benzylaminocarbonyl-phenylamino)-4-[N-(2-hydroxyethyl)-N-methyl-amino]-5-nitro-pyrimidin
   HPLC/MS (Methode B): RT = 2,47 min.; [M+H]+ = 423,2
(29) 2-(3-Carboxy-phenylamino)-4-[N-(trans-4-hydroxy-cyclohexyl)-N-methyl-amino]-5-nitro-pyrimidin
   HPLC/MS (Methode B): RT = 2,21 min.; [M+H]+ = 388,2
(30) 2-(5-Carboxy-2-naphthylamino)-4-(2-acetylamino-1-ethylamino)-5-nitro-pyrimidin
   HPLC/MS (Methode B): RT = 2,22 min.; [M+H]+ = 411,2
(31) 2-(5-Carboxy-2-naphthylamino)-4-(2-pyridyl-methylamino)-5-nitro-pyrimidin
(32) 2-(5-Carboxy-2-naphthylamino)-4-(ethoxycarbonyl-methylamino)-5-nitro-pyrimidin
   HPLC/MS (Methode B): RT = 2,71 min.; [M+H]+ = 412,2
(33) 2-(4-Phenylaminocarbonyl-phenylamino)-4-[N-(2-hydroxyethyl)-N-methyl-amino]-5-nitro-pyrimidin
   HPLC/MS (Methode B): RT = 2,56 min.; [M+H]+ = 409,2
(34) 2-(4-(4-Methyl-1-piperazinyl)-phenylamino)-4-[N-(trans-4-hydroxy-cyclohexyl)-N-methylamino]-5-nitro-pyrimidin
   HPLC/MS (Methode B): RT = 1,77 min.; [M+H]+ = 442,3
(35) 2-(3,4-Dichlorphenylamino)-4-(2-acetylamino-ethylamino)-5-methyl-pyrimidin-hydrochlorid
   Schmelzpunkt: 254°C
(36) 1-{3-[2-(1H-Benzotriazol-5-ylamino)-5-trifluormethyl-pyrimidin-4-ylamino]-propyl}-pyrrolidin-2-one
   R_{f} = 0.16 (Kieselgel; Methylenchlorid: Methanol = 95:5)
   Schmelzpunkt: 118 °C
(37) 1-{3-[2-(1H-Benzimidazol-5-ylamino)-5-trifluormethyl-pyrimidin-4-ylamino]-propyl}-pyrrolidin-2-one
   Schmelzpunkt: 110-113 °C
   R_{f} = 0.43 (Kieselgel; Methylenchlorid: Methanol = 95:5)
   HPLC/MS (Methode D): RT = 5,00 min.; [M+H]+ = 420; Abs. λ max = 246 nm
(38) 1-{3-[2-(1H-Indazol-6-ylamino)-5-trifluormethyl-pyrimidin-4-ylamino]-propyl}-pyrrolidin-2-one
   R_{f} = 0.18 (Kieselgel; Methylenchlorid: Methanol = 95:5)
   Schmelzpunkt: 210 °C
   HPLC/MS (Methode D): RT = 5,20 min.; [M+H]+ = 420; Abs. λ max = 246 nm
(39) 1-{3-[2-(1H-Indazol-5-ylamino)-5-trifluormethyl-2,3-dihydro-pyrimidin-4-ylamino]-propyl}-pyrrolidin-2-on
   R_{f} = 0.27 (Kieselgel; Methylenchlorid: Methanol = 95:5)
   Schmelzpunkt: 202 °C
   HPLC/MS (Methode D): RT = 4,50 min.; [M+H]+ = 420; Abs. λ max = 237 nm
(40) N-{2-[5-Chloro-2-(1H-indazol-6-ylamino)-pyrimidin-4-ylamino]-ethyl}-acetamid
   Schmelzpunkt: >260 °C Zersetzung
   R_{f} = 0,29 (Kieselgel; Methylenchlorid: Methanol = 9:1)
   HPLC/MS (Methode F): RT = 3,04 min.; [M+H]+ = 346; Abs. λ max = 284 nm
(41) N,N-Dimethyl-4-{4-[3-(2-oxo-pyrrolidin-1-yl)-propylamino]-5-trifluormethyl-pyrimidin-2-ylamino}-phenylsulfonamid
   R_{f} = 0.43 (Kieselgel; Methylenchlorid: Methanol = 95:5)
   Schmelzpunkt: 190 - 193 °C
   HPLC/MS [M-H]- = 485
(42) N-{2-[2-(1H-Benzotriazol-5-ylamino)-5-trifluormethyl-pyrimidin-4-ylamino]-ethyl}-acetamid
   R_{f} = 0.17 (Kieselgel; Methylenchlorid: Methanol = 95:5)
   Schmelzpunkt: >300 °C, Zersetzung
(43) N-{2-[2-(1H-Benzimidazol-5-ylamino)-5-trifluormethyl-pyrimidin-4-ylamino]-ethyl}-acetamid
   R_{f} = 0.33 (Kieselgel; Methylenchlorid: Methanol = 95:5)
   Schmelzpunkt: 208-210 °C
   HPLC/MS (Methode D): RT = 3,90 min.; [M+H]+ = 380; Abs. λ max = 240 nm
(44) N-{2-[2-(1H-Indazol-6-ylamino)-5-trifluormethyl-pyrimidin-4-ylamino]-ethyl}-acetamid
   R_{f} = 0.21 (Kieselgel; Methylenchlorid: Methanol = 95:5)
   Schmelzpunkt: >300 °C
   HPLC/MS (Methode D): RT = 4,80 min.; [M+H]+ = 380; Abs. λ max = 244 nm
(45) N-{2-[2-(1H-Indazol-5-ylamino)-5-trifluormethyl-pyrimidin-4-ylamino]-ethyl}-acetamid
   R_{f} = 0.17 (Kieselgel; Methylenchlorid: Methanol = 95:5)
   Schmelzpunkt: >300 °C Zersetzung
   HPLC/MS (Methode D): RT = 4,20 min.; [M+H]+ = 380; Abs. λ max = 246 nm
(46) N-(2-{2-[4-(2H-Tetrazol-5-yl)-phenylamino]-5-trifluormethyl-pyrimidin-4-ylamino}-ethyl)-acetamid
   Schmelzpunkt: >300 °C Zersetzung
   R_{f} = 0.86 (Kieselgel; Methylenchlorid: Methanol = 1:1)
   HPLC/MS (Methode D): RT = 5,00 min.; [M+H]+ = 408; Abs. λ max = 290 nm
(47) N-{2-[2-(4-Dimethylsulfamoyl-phenylamino)-5-trifluormethyl-pyrimidin-4-ylamino]-ethyl}-acetamid
   Schmelzpunkt: 204 °C
   R_{f} = 0,79 (Kieselgel; Methylenchlorid: Methanol = 95:5)
   HPLC/MS (Methode D): RT = 5,60 min.; [M+H]+ = 447; Abs. λ max = 300 nm
(48) 1-{3-[5-Chloro-2-(1H-indazol-6-ylamino)-pyrimidin-4-ylamino]-propyl}-pyrrolidin-2-on
   Schmelzpunkt: 188-191°C
   R_{f} = 0,41 (Kieselgel; Methylenchlorid: Methanol = 9:1)
   HPLC/MS (Methode F): RT = 3,23 min.; [M+H]+ = 386; Abs. λ max = 249 nm
(49) N,N-Dimethyl-4-{4-[2-(2-oxo-imidazolidin-1-yl)-ethylamino]-5-trifluormethyl-pyrimidin-2-ylamino}-phenylsulfonamid
   Schmelzpunkt: 163-164 °C
   R_{f} = 0,13 (Kieselgel; Methylenchlorid: Methanol = 98:2)
   HPLC/MS (Methode D): RT = 6,82 min.; [M+H]+ = 474; Abs. λ max = 306 nm
(50) 2-Chloro-5-{4-[3-(2-oxo-pyrrolidin-1-yl)-propylamino]-5-trifluormethyl-pyrimidin-2-ylamino}-benzoesäure
   Schmelzpunkt: 236-239 °C
   R_{f} = 0,1 (Kieselgel; Methylenchlorid: Methanol = 9:1)
   HPLC/MS (Methode G): RT = 3,35 min.; [M+H]+ = 458; Abs. λ max = 267 nm
(51) 1-(2-{2-[4-(Morpholin-4-sulfonyl)-phenylamino]-5-trifluormethyl-pyrimidin-4-ylamino}-ethyl)-imidazolidin-2-on
   Schmelzpunkt: 164°C
   R_{f} = 0.10 (Kieselgel; Methylenchlorid: Methanol = 98:2)
   HPLC/MS (Methode D): RT = 5,63 min.; [M+H]+ = 516; Abs. λ max = 302 nm
(52) 1-{3-[2-(4-Hydroxymethyl-phenylamino)-5-trifluormethyl-pyrimidin-4-ylamino]-propyl}-pyrrolidin-2-on
   Schmelzpunkt: 153 °C
   R_{f} = 0.18 (Kieselgel; Methylenchlorid: Methanol = 9:1)
   HPLC/MS (Methode D): RT = 4,75 min.; [M+H]+ = 410; Abs. λ max = 256 nm
(53) 2-{4-[4-(2-Acetylamino-ethylamino)-5-trifluormethyl-pyrimidin-2-ylamino]-phenyl}-N,N-dimethyl-acetamid
   Schmelzpunkt: 182-184 °C
   R_{f} = 0,09 (Kieselgel; Hexan: Essigsäureethylester: Methanol = 5:4:1)
   HPLC/MS (Methode D): RT = 4,357 min.; [M+H]+ = 425; Abs. λ max = 246 nm
(54) 1-[2-(3,4-Dichloro-phenylamino)-5-trifluormethyl-pyrimidin-4-yl]-azepan-4-on
   Schmelzpunkt: 147-149 °C
   R_{f} = 0.82 (Kieselgel; Methylenchlorid: Methanol = 95:5)
   HPLC/MS (Methode D): RT = 7,27 min.; [M+H]+ = 421; Abs. λ max = 274 nm
(55) (3,4-Dichloro-phenyl)-[4-(4,5,7,8-tetrahydro-1H-imidazo[4,5-d]azepin-6-yl)-5-trifluormethyl-pyrimidin-2-yl]-amin
   Schmelzpunkt: 247 °C Zersetzung
   R_{f} = 0.19 (Kieselgel; Methylenchlorid: Methanol = 95:5)
   HPLC/MS (Methode D): RT = 5,51 min.; [M+H]+ = 443; Abs. λ max = 274 nm
(56) (3,4-Dichloro-phenyl)-[4-(2-methyl-4,5,7,8-tetrahydro-1H-imidazo[4,5-d]azepin-6-yl)-5-trifluormethyl-pyrimidin-2-yl]-amin
   Schmelzpunkt: 245 °C Zersetzung
   R_{f} = 0.14 (Kieselgel; Methylenchlorid: Methanol = 95:5)
   HPLC/MS (Methode D): RT = 5,59 min.; [M+H]+ = 459; Abs. λ max = 274 nm
(57) N-(2-{2-[4-(Morpholin-4-sulfonyl)-phenylamino]-5-trifluormethyl-pyrimidin-4-ylamino}-ethyl)-acetamid
   Schmelzpunkt: 73-75 °C
   R_{f} = 0.19 (Kieselgel; Methylenchlorid: Methanol = 95:5)
   HPLC/MS (Methode D): RT = 5,40 min.; [M-H]- = 487; Abs. λ max = 277 nm
(58) N-(2-{2-[4-(4-Methyl-piperazin-1-sulfonyl)-phenylamino]-5-trifluormethyl-pyrimidin-4-ylamino}-ethyl)-acetamid
   Schmelzpunkt: 125-127 °C
   R_{f} = 0.10 (Kieselgel; Methylenchlorid: Methanol = 95:5)
   HPLC/MS (Methode D): RT = 4,40 min.; [M+H]+ = 502; Abs. λ max = 270 nm
(59) N-(2-{2-[4-(Pyridin-2-ylsulfamoyl)-phenylamino]-5-trifluormethyl-pyrimidin-4-ylamino}-ethyl)-acetamid
   Schmelzpunkt: 228°C
   R_{f} = 0.54 (Kieselgel; Methylenchlorid: Methanol = 95:5)
   HPLC/MS (Methode D): RT = 4,80 min.; [M+H]+ = 496; Abs. λ max = 246 nm
(60) N-(2-{2-[4-(Perhydro-1,4-diazepin-1-sulfonyl)-phenylamino]-5-trifluormethyl-pyrimidin-4-ylamino}-ethyl)-acetamid
   Schmelzpunkt: 167-169 °C
   R_{f} = 0.36 (Kieselgel; Methylenchlorid: Methanol = 95:5)
   HPLC/MS (Methode D): RT = 4,40 min.; [M+H]+ = 502 ; Abs. λ max = 270 nm
(61) N-(2-{2-[4-(3,5-Dimethyl-piperazin-1-sulfonyl)-phenylamino]-5-trifluormethyl-pyrimidin-4-ylamino}-ethyl)-acetamid
   Schmelzpunkt: 117 °C
   R_{f} = 0.16 (Kieselgel; Methylenchlorid: Methanol = 95:5)
   HPLC/MS (Methode D): RT = 4,50 min.; [M+H]+ = 516; Abs. λ max = 270 nm
(62) N-[2-(2-Phenylamino-5-trifluormethylpyrimidin-4-ylamino)-ethyl]-acetamid
   350 mg 2-Chlor-4-(2-acetylamino-ethylamino)-5-trifluormethyl-pyrimidin und 630 mg 4-(4-[tert-Butyl-oxycarbonyl]-homopiperazin-1-sulfonyl)-phenylamin werden mit 3ml Dioxan vorgelegt und mit N,N-Dimethylformamid versetzt bis alle Komponenten gelöst sind. Danach werden 0,25 ml 4.0M Salzsäure in 1,4-Dioxane zugetropft und 2 Stunden unter Rühren auf 80°C erwärmt. Danach wird zusätzliche 4.0M Salzsäure in 1,4-Dioxane (1 ml) zugesetzt und für 15 Minuten auf 85°C erwärmt. Den entstehenden Niederschlag filtriert man ab und wäscht mit Dioxan nach. Nach dem Lösen in Wasser wird über eine RP C-18 Säule mit der mobilen Phase H₂O/Acetonitril gereinigt. Nach dem Einengen bleibt das Produkt als Feststoff zurück.
   Schmelzpunkt: 175 °C Zersetzung
   R_{f} = 0.44 (Kieselgel; Methylenchlorid: Methanol = 9:1)
   HPLC/MS (Methode D): RT = 4,605 min.; [M+H]+ = 340
(63) 4-{5-Isopropyl-4-[3-(2-oxo-pyrrolidin-1-yl)-propylamino]-pyrimidin-2-ylamino}-benzamid
   Schmelzpunkt: 204-205 °C
   R_{f} = 0,13 (Kieselgel; Methylenchlorid: Methanol = 9:1)
   HPLC/MS (Methode G): RT = 2,99 min.; [M+H]+ =397; Abs. λ max = 279,8 nm
(64) 1-{3-[2-(3-Chloro-4-morpholin-4-yl-phenylamino)-5-trifluormethyl-pyrimidin-4-ylamino]-propyl}-pyrrolidin-2-on
   Schmelzpunkt: 131-134 °C
   R_{f} = 0,56 (Kieselgel; Methylenchlorid: Methanol = 9:1)
   HPLC/MS (Methode G): RT = 3,61 min.; [M+H]+ = 499; Abs. λ max = 230 nm
(65) N-[2-(2-Benzylamino-5-trifluormethyl-pyrimidin-4-ylamino)-ethyl]-acetamid
   Schmelzpunkt: 190-191 °C
   R_{f} = 0,57 (Kieselgel; Methylenchlorid: Methanol = 9:1)
   HPLC/MS (Methode G): RT = 3,37 min.; [M+H]+ = 354; Abs. λ max = 230 nm
(66) N-(2-{2-[4-(4-Methoxy-piperidin-1-ylmethyl)-phenylamino]-5-trifluormethyl-pyrimidin-4-ylamino}-ethyl)-acetamid
(67) N-{2-[2-(3-Amino-phenylamino)-5-trifluormethyl-pyrimidin-4-ylamino]-ethyl}-acetamid
   Schmelzpunkt: 198-200 °C
   R_{f} = 0,48 (Kieselgel; Methylenchlorid: Methanol = 9:1)
   HPLC/MS (Methode F): RT = 2,97 min.; [M+H]+ = 355; Abs. λ max = 253 nm
   1H-NMR(D₆-DMSO, 300MHz) δ: 1.80 (s, 3 H), 3.30 (m, 2 H), 3.52 (m, 2 H), 4.99 (m, 2 H), 6.20 (d, 1 H), 6.92-6.79 (m, 2 H), 7.05 (m, 2 H), 7.99 (t, 1 H), 8.14 (s, 1 H), 9.30 (s, 1 H).
(68) 4-[4-(4-Pyrimidin-2-yl-piperazin-1-yl)-5-trifluormethyl-pyrimidin-2-ylamino]-benzamid
   Schmelzpunkt: 260-262°C
   R_{f} = 0,23 (Kieselgel; Methylenchlorid: Methanol = 9:1)
   HPLC/MS (Methode G): RT = 3,60 min.; [M+H]+ =445; Abs. λ max = 287,4 nm
(69) *N*-{2-[5-Isopropyl-2-(4-piperidin-1-ylmethyl-phenylamino)-pyrimidin-4-ylamino]-ethyl}-acetamid
   Schmelzpunkt: 165-168°C
   R_{f} = 0,07 (Kieselgel; Methylenchlorid: Methanol = 4:1)
   HPLC/MS (Methode F): RT = 3,01 min.; [M+H]+ =411; Abs. λ max = 260,8 nm
(70) 1-{3-[5-Methoxy-2-(4-piperidin-1-ylmethyl-phenylamino)-pyrimidin-4-ylamino]-propyl}-pyrrolidin-2-on
   R_{f} = 0,07 (Kieselgel; Methylenchlorid: Methanol = 4:1)
   HPLC/MS (Methode F): RT = 3,00 min.; [M+H]+ =439; Abs. λ max = 260,8 nm
(71) 4-{5-Dimethylamino-4-[3-(2-oxo-pyrrolidin-1-yl)-propylamino]-pyrimidin-2-ylamino}-benzamid
   Schmelzpunkt: 202-203 °C
   R_{f} = 0,14 (Kieselgel; Methylenchlorid: Methanol = 9:1)
   HPLC/MS (Methode G): RT = 2,88 min.; [M+H]+ =398; Abs. λ max =295 nm
(72) 4-{4-[4-(2-Nitro-phenyl)-piperazin-1-yl]-5-trifluormethyl-pyrimidin-2-ylamino}-benzamid
   Schmelzpunkt: 223-225 °C
   R_{f} = 0,06 (Kieselgel; Methylenchlorid: Methanol = 99:1)
   HPLC/MS (Methode G): RT = 4,45 min.; [M+H]+ =488; Abs. λ max = 258,9 nm
(73) N,N-Dimethyl-4-[4-(4,5,7,8-tetrahydro-1H-imidazo[4,5-d]azepin-6-yl)-5-trifluormethyl-pyrimidin-2-ylamino]-phenylsulfonamid
   Schmelzpunkt: 152-155 °C
   R_{f} = 0,26 (Kieselgel; Methylenchlorid: Methanol = 95:5)
   HPLC/MS (Methode D): RT = 5,35 min.; [M+H]+ = 482; Abs. λ max = 298 nm
(74) N-{2-[2-(1H-Indazol-6-ylamino)-5-methyl-pyrimidin-4-ylamino]-ethyl}-acetamid
   Schmelzpunkt: 240-243°C
   R_{f} = 0,22 (Kieselgel; Methylenchlorid: Methanol = 4:1)
   HPLC/MS (Methode F): RT = 3,05 min.; [M+H]+ = 326; Abs. λ max = 248 nm
(75) 1-{3-[2-(1H-Indazol-6-ylamino)-5-methyl-pyrimidin-4-ylamino]-propyl}-pyrrolidin-2-on
   Schmelzpunkt: 227-230 °C
   R_{f} = 0,17 (Kieselgel; Methylenchlorid: Methanol = 9:1)
   HPLC/MS (Methode F): RT = 3,24 min.; [M+H]+ = 366; Abs. λ max = 248 nm
(76) 1-(3-{2-[4-(2H-Tetrazol-5-yl)-phenylamino]-5-trifluormethyl-pyrimidin-4-ylamino}-propyl)-pyrrolidin-2-on
   Schmelzpunkt: >300 °C Zersetzung
   R_{f} = 0,06 (Kieselgel; Methylenchlorid: Methanol = 95:5)
   HPLC/MS (Methode D): RT = 5,10 min.; [M+H]+ = 448; Abs. λ max = 236 nm
(77) N-{2-[2-(4-Sulfamoyl-benzylamino)-5-trifluormethyl-pyrimidin-4-ylamino]-ethyl}-acetamid
   Schmelzpunkt: 205-207 °C
   R_{f} = 0,39 (Kieselgel; Methylenchlorid: Methanol = 85:15)
   HPLC/MS (Methode G): RT = 2,31 min.; [M+H]+ = 433; Abs. λ max = 232 nm
(78) N-{2-[5-Bromo-2-(1H-indazol-6-ylamino)-pyrimidin-4-ylamino]-ethyl}-acetamid
   Schmelzpunkt: 270-272°C
   R_{f} = 0,32 (Kieselgel; Methylenchlorid: Methanol = 9:1)
   HPLC/MS (Methode F): RT = 3,09 min.; [M+H]+ = 391; Abs. λ max = 251 nm
(79) 1-{3-[2-(3-Dimethylamino-phenylamino)-5-trifluormethyl-pyrimidin-4-ylamino]-propyl}-pyrrolidin-2-on
   Schmelzpunkt: 152-155°C
   R_{f} = 0,6 (Kieselgel; Methylenchlorid: Methanol = 9:1)
   HPLC/MS (Methode G): RT = 2,49 min.; [M+H]+ = 423; Abs. λ max = 253 nm
(80) 1-{3-[2-(2,4-Dichloro-phenylamino)-5-trifluormethyl-pyrimidin-4-ylamino]-propyl}-pyrrolidin-2-on
   Schmelzpunkt: 148-150°C
   R_{f} = 0,67 (Kieselgel; Methylenchlorid: Methanol = 9:1)
   HPLC/MS (Methode G): RT = 4,3 min.; [M+H]+ = 449; Abs. λ max = 234 nm
(81) 1-{3-[2-(4-Methoxy-2-methyl-phenylamino)-5-trifluormethyl-pyrimidin-4-ylamino]-propyl}-pyrrolidin-2-on
   Schmelzpunkt: 127-130 °C
   R_{f} = 0,58 (Kieselgel; Methylenchlorid: Methanol = 9:1)
   HPLC/MS (Methode G): RT = 3,68 min.; [M+H]+ = 424; Abs. λ max = 236 nm
(82) 1-{3-[2-(2,5-Dichloro-phenylamino)-5-trifluormethyl-pyrimidin-4-ylamino]-propyl}-pyrrolidin-2-on
   R_{f} = 0,65 (Kieselgel; Methylenchlorid: Methanol = 9:1)
   HPLC/MS (Methode I): RT = 3,66 min.; [M+H]+ = 449; Abs. λ max = 244 nm
(83) 1-{3-[2-(3-Fluor-5-trifluormethyl-phenylamino)-5-trifluormethyl-pyrimidin-4-ylamino]-propyl}-pyrrolidin-2-on
   Schmelzpunkt: 183-184°C
   R_{f} = 0,6 (Kieselgel; Methylenchlorid: Methanol = 9:1)
   HPLC/MS (Methode I): RT = 4,07 min.; [M+H]+ = 466; Abs. λ max = 257 nm
(84) 1-{3-[2-(2-Fluor-5-trifluormethyl-phenylamino)-5-trifluormethyl-pyrimidin-4-ylamino]-propyl}-pyrrolidin-2-on
   Schmelzpunkt: 148-149 °C
   R_{f} = 0,65 (Kieselgel; Methylenchlorid: Methanol = 91:1)
   HPLC/MS (Methode I): RT = 3,51 min.; [M+H]+ = 466; Abs. λ max = 244 nm
(85) N-{2-[2-(3-Bromo-benzylamino)-5-trifluormethyl-pyrimidin-4-ylamino]-ethyl}-acetamid
   Schmelzpunkt: 162-165°C
   R_{f} = 0,44 (Kieselgel; Methylenchlorid: Methanol = 9:1)
   HPLC/MS (Methode G): RT = 3,65 min.; [M+H]+ = 433; Abs. λ max = 236 nm
(86) N-{2-[2-(1-Phenyl-ethylamino)-5-trifluormethyl-pyrimidin-4-ylamino]-ethyl}-acetamid
   Schmelzpunkt: 125-140 °C
   R_{f} = 0,46 (Kieselgel; Methylenchlorid: Methanol = 9:1)
   HPLC/MS (Methode F): RT = 3,53 min.; [M+H]+ = 368; Abs. λ max = 230 nm
(87) 1-{3-[2-(2-Isopropyl-phenylamino)-5-trifluormethyl-pyrimidin-4-ylamino]-propyl}-pyrrolidin-2-on
   Schmelzpunkt: 115-118 °C
   R_{f} = 0,26 (Kieselgel; Methylenchlorid: Methanol = 9:1)
   HPLC/MS (Methode G): RT = 3,94 min.; [M+H]+ = 422; Abs. λ max = 232 nm
(88) 1-{3-[2-(Biphenyl-4-ylamino)-5-trifluormethyl-pyrimidin-4-ylamino]-propyl}-pyrrolidin-2-on
   Schmelzpunkt: 155-156 °C
   R_{f} = 0,74 (Kieselgel; Methylenchlorid: Methanol = 9:1)
   HPLC/MS (Methode H): RT = 4,07 min.; [M+H]+ = 456; Abs. λ max = 242 nm
(89) 1-{3-[2-(2,4-Difluor-phenylamino)-5-trifluormethyl-pyrimidin-4-ylamino]-propyl}-pyrrolidin-2-on
   Schmelzpunkt: 117-120 °C
   R_{f} = 0,76 (Kieselgel; Methylenchlorid: Methanol = 8:2)
   HPLC/MS (Methode G): RT = 3,76 min.; [M+H]+ = 416; Abs. λ max = 238 nm
(90) 1-{3-[2-(2-Chloro-4-methyl-phenylamino)-5-trifluormethyl-pyrimidin-4-ylamino]-propyl}-pyrrolidin-2-on
   Schmelzpunkt: 110-113 °C
   R_{f} = 0,71 (Kieselgel; Methylenchlorid: Methanol = 9:1)
   HPLC/MS (Methode G): RT = 4,02 min.; [M+H]+ = 428; Abs. λ max = 242 nm
(91) 1-{3-[2-(2-Chloro-5-trifluormethyl-phenylamino)-5-trifluormethyl-pyrimidin-4-ylamino]-propyl}-pyrrolidin-2-on
   Schmelzpunkt: 139-140°C
   R_{f} = 0,77 (Kieselgel; Methylenchlorid: Methanol = 9:1)
   HPLC/MS (Methode H): RT = 4,51 min.; [M+H]+ = 482; Abs. λ max = 248 nm
(92) 1-{3-[2-(3,5-Difluor-phenylamino)-5-trifluormethyl-pyrimidin-4-ylamino]-propyl}-pyrrolidin-2-on
   Schmelzpunkt: 210-212°C
   R_{f} = 0,71 (Kieselgel; Methylenchlorid: Methanol = 9:1)
   HPLC/MS (Methode G): RT = 4,32 min.; [M+H]+ = 416; Abs. λ max = 253 nm
(93) N-{2-[2-(3,4-Dichloro-benzylamino)-5-trifluormethyl-pyrimidin-4-ylamino]-ethyl}-acetamid
   Schmelzpunkt: 168°C
   R_{f} = 0,57 (Kieselgel; Methylenchlorid: Methanol = 9:1)
   HPLC/MS (Methode G): RT = 3,79 min.; [M+H]+ = 422; Abs. λ max = 230 nm
(94) 1-{3-[5-Trifluormethyl-2-(2-trifluormethyl-phenylamino)-pyrimidin-4-ylamino]-propyl}-pyrrolidin-2-on
   R_{f} = 0,65 (Kieselgel; Methylenchlorid: Methanol = 9:1)
   HPLC/MS (Methode H): RT = 3,29 min.; [M+H]+ = 448; Abs. λ max = 234 nm
(95) 1-{3-[2-(4-Isopropyl-phenylamino)-5-trifluormethyl-pyrimidin-4-ylamino]-propyl}-pyrrolidin-2-on
   Schmelzpunkt: 124-126 °C
   R_{f} = 0,62 (Kieselgel; Methylenchlorid: Methanol = 9:1)
   HPLC/MS (Methode H): RT = 3,75 min.; [M+H]+ = 422; Abs. λ max = 259 nm
(96) 1-{3-[2-(4-Dimethylamino-phenylamino)-5-trifluormethyl-pyrimidin-4-ylamino]-propyl}-pyrrolidin-2-on
   Schmelzpunkt: 156-158 °C
   R_{f} = 0,54 (Kieselgel; Methylenchlorid: Methanol = 9:1)
   HPLC/MS (Methode F): RT = 3,25 min.; [M+H]+ = 423; Abs. λ max = 257 nm
(97) 1-{3-[2-(4-Morpholin-4-yl-phenylamino)-5-trifluormethyl-pyrimidin-4-ylamino]-propyl}-pyrrolidin-2-on
   Schmelzpunkt: 115-118 °C
   R_{f} = 0,52 (Kieselgel; Methylenchlorid: Methanol = 9:1)
   HPLC/MS (Methode H): RT = 3,25 min.; [M+H]+ = 465; Abs. λ max = 257 nm
(98) N-{2-[2-(4-Dimethylsulfamoyl-phenylamino)-5-methyl-pyrimidin-4-ylamino]-ethyl}-acetamid
   Schmelzpunkt: 204-206 °C
   R_{f} = 0,3 (Kieselgel; Methylenchlorid: Methanol = 9:1)
   HPLC/MS (Methode F): RT = 3,15 min.; [M+H]+ = 393; Abs. λ max = 282 nm
(99) 4-[5-Chloro-4-(4,5,7,8-tetrahydro-1H-imidazo[4,5-*d*]azepin-6-yl)-pyrimidin-2-ylamino]-N,N-dimethyl-phenylsulfonamid
   Schmelzpunkt: 160-162°C
   R_{f} = 0,22 (Kieselgel; Methylenchlorid: Methanol = 9:1)
   HPLC/MS (Methode F): RT = 3,79 min.; [M+H]+ = 448; Abs. λ max = 286 nm
(100) 4-{5-Bromo-4-[3-(2-oxo-pyrrolidin-1-yl)-propylamino]-pyrimidin-2-ylamino}-N,N-dimethyl-phenylsulfonamid
   Schmelzpunkt: 165,1-167,7 °C
   R_{f} = 0,51 (Kieselgel; Methylenchlorid: Methanol = 9:1)
   HPLC/MS (Methode F): RT = 3,85 min.; [M+H]+ = 498; Abs. λ max = 284 nm
(101) N-{2-[5-Chloro-2-(4-dimethylsulfamoyl-phenylamino)-pyrimidin-4-ylamino]-ethyl}-acetamid
   Schmelzpunkt: >300 °C Zersetzung
   R_{f} = 0,42 (Kieselgel; Methylenchlorid: Methanol = 9:1)
   HPLC/MS (Methode F): RT = 3,58 min.; [M+H]+ = 413; Abs. λ max = 284 nm
(102) N-{2-[5-Bromo-2-(4-piperidin-1-ylmethyl-phenylamino)-pyrimidin-4-ylamino]-ethyl}-acetamid
   Schmelzpunkt: 154-157 °C
   R_{f} = 0,1 (Kieselgel; Methylenchlorid: Methanol = 9:1)
   HPLC/MS (Methode F): RT = 2,7 min.; [M+H]+ =448 ; Abs. λ max = 253 nm
(103) 1-{3-[5-Bromo-2-(4-piperidin-1-ylmethyl-phenylamino)-pyrimidin-4-ylamino]-propyl}-pyrrolidin-2-on
   Schmelzpunkt: 137-138 °C
   R_{f} = 0,25 (Kieselgel; Methylenchlorid: Methanol = 9:1)
   HPLC/MS (Methode F): RT = 3,16 min.; [M+H]+ = 488; Abs. λ max = 268 nm
(104) N-{2-[5-Methyl-2-(4-piperidin-1-ylmethyl-phenylamino)-pyrimidin-4-ylamino]-ethyl}-acetamid
   Schmelzpunkt: 150-152 °C
   R_{f} = 0,06 (Kieselgel; Methylenchlorid: Methanol = 4:1)
   HPLC/MS (Methode F): RT = 2,52 min.; [M+H]+ = 383; Abs. λ max = 263 nm
(105) N,N-Dimethyl-4-{5-methyl-4-[3-(2-oxo-pyrrolidin-1-yl)-propylamino]-pyrimidin-2-ylamino}-phenylsulfonamid
   Schmelzpunkt: 186-189°C
   R_{f} = 0,41 (Kieselgel; Methylenchlorid: Methanol = 9:1)
   HPLC/MS (Methode F): RT = 3,6 min.; [M+H]+ = 433; Abs. λ max = 282 nm
(106) 1-{3-[5-Bromo-2-(1H-indazol-6-ylamino)-pyrimidin-4-ylamino]-propyl}-pyrrolidin-2-on
   Schmelzpunkt: 211-213 °C
   R_{f} = 0,44 (Kieselgel; Methylenchlorid: Methanol = 9:1)
   HPLC/MS (Methode F): RT = 3,62 min.; [M+H]+ = 431; Abs. λ max = 251 nm
(107) N-{2-[2-(2-Fluor-benzylamino)-5-trifluormethyl-pyrimidin-4-ylamino]-ethyl}-acetamid
   Schmelzpunkt: 188-189 °C
   R_{f} = 0,44 (Kieselgel; Methylenchlorid: Methanol = 9:1)
   HPLC/MS (Methode G): RT = 3,34 min.; [M+H]+ = 372; Abs. λ max = 229 nm
(108) N-(2-{2-[1-(4-Bromo-phenyl)-ethylamino]-5-trifluormethyl-pyrimidin-4-ylamino}-ethyl)-acetamid
   Schmelzpunkt: 143-145 °C
   R_{f} = 0,46 (Kieselgel; Methylenchlorid: Methanol = 9:1)
   HPLC/MS (Methode H): RT = 3,29 min.; [M+H]+ = 447; Abs. λ max = 230 nm
(109) 4-{5-Chloro-4-[3-(2-oxo-pyrrolidin-1-yl)-propylamino]-pyrimidin-2-ylamino}-N,N-dimethyl-phenylsulfonamid
   Schmelzpunkt: 159-161 °C
   R_{f} = 0,53 (Kieselgel; Methylenchlorid: Methanol = 9:1)
   HPLC/MS (Methode F): RT = 3,55 min.; [M+H]+ = 453; Abs. λ max = 284 nm
(110) 1-{3-[5-Chloro-2-(4-piperidin-1-ylmethyl-phenylamino)-pyrimidin-4-ylamino]-propyl}-pyrrolidin-2-on
   Schmelzpunkt: 137-138 °C
   R_{f} = 0,20 (Kieselgel; Methylenchlorid: Methanol = 9:1)
   HPLC/MS (Methode F): RT = 3,21 min.; [M+H]+ = 443; Abs. λ max = 267 nm
(111) 4-[4-(2-Acetylamino-ethylamino)-5-dimethylamino-pyrimidin-2-ylamino]-benzamid
   Schmelzpunkt: 218-220 °C
   R_{f} = 0,46 (Kieselgel; Methylenchlorid: Methanol = 4:1)
   HPLC/MS (Methode G): RT = 2,63 min.; [M+H]+ =358; Abs. λ max = 293,1 nm
(112) 4-[4-(2-Acetylamino-ethylamino)-5-isopropyl-pyrimidin-2-ylamino]-benzamid
   Schmelzpunkt: 229 °C
   R_{f} = 0,42 (Kieselgel; Methylenchlorid: Methanol = 4:1)
   HPLC/MS (Methode G): RT = 2,77 min.; [M+H]+ =357; Abs. λ max = 285,5 nm
(113) 4-{5-Methanesulfonyl-4-[3-(2-oxo-pyrrolidin-1-yl)-propylamino]-pyrimidin-2-ylamino}-benzamid
   Schmelzpunkt: 244-246°C
   R_{f} = 0,07 (Kieselgel; Methylenchlorid: Methanol = 99:1)
   HPLC/MS (Methode F): RT = 3,15 min.; [M+H]+ =433; Abs. λ max = 287,4 nm
(114) 1-{3-[2-(3,4-Dichloro-phenylamino)-5-methylsulfonyl-pyrimidin-4-ylamino]-propyl}-pyrrolidin-2-on
   Schmelzpunkt: 200-203 °C
   R_{f} = 0,48 (Kieselgel; Methylenchlorid: Methanol = 99:1)
   HPLC/MS (Methode G): RT = 4,34 min.; [M+H]+ =459; Abs. λ max = 262,7 nm
(115) 4-[5-Dimethylamino-4-(4-pyridin-2-yl-piperazin-1-yl)-pyrimidin-2-ylamino]-benzamid
   Schmelzpunkt: 237-238°C
   R_{f} = 0,20 (Kieselgel; Methylenchlorid: Methanol = 9:1)
   HPLC/MS (Methode G): RT = 2,69 min.; [M+H]+ =419; Abs. λ max = 298,8 nm
(116) 1-{3-[2-(3,4-Dichloro-phenylamino)-5-isopropyl-pyrimidin-4-ylamino]-propyl}-pyrrolidin-2-on
   Schmelzpunkt: 147-150 °C
   R_{f} = 0,08 (Kieselgel; Methylenchlorid: Methanol = 99:1)
   HPLC/MS (Methode G): RT = 4,00 min.; [M+H]+ =423; Abs. λ max = 264,6 nm
(117) 4-{5-Methoxy-4-[3-(2-oxo-pyrrolidin-1-yl)-propylamino]-pyrimidin-2-ylamino}-benzamid
   Schmelzpunkt: 212-213 °C
   R_{f} = 0,12 (Kieselgel; Methylenchlorid: Methanol = 9:1)
   HPLC/MS (Methode G): RT = 2,74min.; [M+H]+ =385; Abs. λ max = 291,2 nm
(118) 1-{3-[2-(3,4-Dichloro-phenylamino)-5-methoxy-pyrimidin-4-ylamino]-propyl}-pyrrolidin-2-on
   Schmelzpunkt: 148-150 °C
   R_{f} = 0,05 (Kieselgel; Methylenchlorid: Methanol = 99:1)
   HPLC/MS (Methode H): RT = 3,39 min.; [M+H]+ =411; Abs. λ max = 264,6 nm
(119) *N*-{2-[5-Methoxy-2-(4-piperidin-1-ylmethyl-phenylamino)-pyrimidin-4-ylamino]-ethyl}-acetamid
   Schmelzpunkt: 109-111 °C
   R_{f} = 0,09 (Kieselgel; Methylenchlorid: Methanol = 4:1)
   HPLC/MS (Methode F): RT = 2,89 min.; [M+H]+ =399; Abs. λ max = 257 nm
(120) [5-Methoxy-4-(4-pyridin-2-yl-piperazin-1-yl)-pyrimidin-2-yl]-(4-piperidin-1-ylmethyl-phenyl)-amin
   Schmelzpunkt: 158-159 °C
   R_{f} = 0,12 (Kieselgel; Methylenchlorid: Methanol = 9:1)
   HPLC/MS (Methode F): RT = 2,92 min.; [M+H]+ =460; Abs. λ max = 266,5 nm
(121) *N*-{2-[5-Dimethylamino-2-(4-piperidin-1-ylmethyl-phenylamino)-pyrimidin-4-ylamino]-ethyl}-acetamid
   R_{f} = 0,08 (Kieselgel; Methylenchlorid: Methanol = 4:1)
   HPLC/MS (Methode F): RT = 2,94 min.; [M+H]+ =412; Abs. λ max = 257 nm
(122) 1-{3-[2-(3,4-Dichloro-phenylamino)-5-dimethylamino-pyrimidin-4-ylamino]-propyl}-pyrrolidin-2-on
   Schmelzpunkt: 103-106°C
   R_{f}=0,22 (Kieselgel; Methylenchlorid: Methanol = 99:1)
   HPLC/MS (Methode G): RT = 3,87 min.; [M+H]+ =424; Abs. λ max = 268,4 nm
(123) [5-Isopropoxy-4-(4-pyridin-2-yl-piperazin-1-yl)-pyrimidin-2-yl]-(4-piperidin-1-ylmethyl-phenyl)-amin
   Schmelzpunkt: 143-145°C
   R_{f} = 0,13 (Kieselgel; Methylenchlorid: Methanol = 9:1)
   HPLC/MS (Methode F): RT = 3,06 min.; [M+H]+ =488; Abs. λ max = 272,2 nm
(124) 1-{3-[5-Isopropoxy-2-(4-piperidin-1-ylmethyl-phenylamino)-pyrimidin-4-ylamino]-propyl}-pyrrolidin-2-on
   R_{f} = 0,13 (Kieselgel; Methylenchlorid: Methanol = 9:1)
   HPLC/MS (Methode F): RT = 3,21 min.; [M+H]+ =467; Abs. λ max = 253,2 nm
(125) *N*-{2-[5-Isopropoxy-2-(4-piperidin-1-ylmethyl-phenylamino)-pyrimidin-4-ylamino]-ethyl}-acetamid
   Schmelzpunkt: 105-107 °C
   R_{f} = 0,06 (Kieselgel; Methylenchlorid: Methanol = 9:1)
   HPLC/MS (Methode F): RT = 3,06 min.; [M+H]+ =427; Abs. λ max = 255,1 nm
(126) *N*-{2-[2-(3,4-Dichloro-phenylamino)-5-isopropyl-pyrimidin-4-ylamino]-ethyl}-acetamid
   Schmelzpunkt: 206-207 °C
   R_{f} = 0,18 (Kieselgel; Methylenchlorid: Methanol = 99:1)
   HPLC/MS (Methode G): RT = 3,70 min.; [M+H]+ = 383; Abs. λ max = 264,6 nm
(127) *N*-{2-[2-(3,4-Dichloro-phenylamino)-5-dimethylamino-pyrimidin-4-ylamino]-ethyl}-acetamid
   Schmelzpunkt: 165-168 °C
   R_{f} = 0,05 (Kieselgel; Methylenchlorid: Methanol = 99:1)
   HPLC/MS (Methode G): RT = 3,57 min.; [M+H]+ = 384; Abs. λ max = 264,6 nm
(128) N-(2-[2-(4-Amino-phenylamino)-5-trifluormethyl-pyrimidin-4-ylamino]-ethyl)-acetamid
   3 g N-[2-(2-Chloro-5-trifluormethyl-pyrimidin-4-ylamino)-ethyl]-acetamid werden in 25 ml Eisessig mit 6.9 g p-Phenylendiamin versetzt und für 4 Stunden bei Raumtemperatur gerührt. Nach dem Entfernen der Essigsäure im Vakuum wird die Reaktionsmischung in Dichlormethan aufgenommen und mit ges. Natriumcarbonatlösung gewaschen. Die wässrige Phase wird mit Dichlormethan extrahiert. Die vereinten org. Phasen werden über Natriumsulfat getrocknet und eingeengt. Das Rohprodukt wird durch Chromatographie (Kieselgel, CH2Cl2/iPrOH = 20/1) gereinigt. Man erhält 3.0 g eines grauen Feststoffs.
   Rf (CH2Cl2/iPrOH = 8/2 + 1% NH3; SiO2) = 0.35
   1H-NMR(D₆-DMSO, 300MHz) δ: 1.80 (s, 3 H), 3.25 (m, 2 H), 3.47 (m, 2 H), 4.78 (m, 2 H), 6.50 (d, 1 H), 6.93 (m, 1 H), 7.29 (d, 2 H), 7.90 (t, 1 H), 8.08 (s, 1 H), 9.13 (s, 1 H).
(129) 1-{3-[5-Isopropyl-2-(4-piperidin-1-ylmethyl-phenylamino)-pyrimidin-4-ylamino]-propyl}-pyrrolidin-2-on
   Schmelzpunkt: 142-144°C
   R_{f} = 0,05 (Kieselgel; Methylenchlorid: Methanol = 9:1)
   HPLC/MS (Methode F): RT = 3,2 min.; [M+H]+ =451; Abs. λ max = 253,2 nm
(130) N-{2-[2-(4-Methylamino-phenylamino)-5-trifluormethyl-pyrimidin-4-ylamino]-ethyl}-acetamid
   Die Verbindung wird ausgehend von N-tert-Butyl-oxycarbonyl-N-methyl-amino-4-aminobenzol und Beispiel II dargestellt. Das Zwischenprodukt wird mit 5 Äq. 4.0M Salzsäure in 1,4-Dioxane versetzt und 0,5 Stunden unter Rühren auf 85 °C erwärmt. Nach dem Entfernen des Lösungsmittels im Vakuum wird das Rohprodukt in Dichlormethan aufgenommen und durch Chromatographie (Kieselgel, CH2Cl2/Methanol) gereinigt.
   Schmelzpunkt: 169-171°C
   R_{f} = 0,37 (Kieselgel; Methylenchlorid: Methanol = 9:1)
   HPLC/MS (Methode F): RT = 2,96 min.; [M+H]+ = 369; Abs. λ max = 257 nm
(131) N-{2-[2-(3-Methylamino-phenylamino)-5-trifluormethyl-pyrimidin-4-ylamino]-ethyl}-acetamid
   Die Herstellung erfolgt analog 2(130).
   Schmelzpunkt: 191-193°C
   R_{f} = 0.49 (Kieselgel; Methylenchlorid: Methanol = 9:1)
   HPLC/MS (Methode G): RT = 2,68 min.; [M+H]+ = 369; Abs. λ max = 253 nm
(132) N-{2-[2-(3-Formyl-phenylamino)-5-trifluormethyl-pyrimidin-4-ylamino]-ethyl}-acetamid
   12 g Mangandioxyd werden in 200 ml Dichlormethan suspendiert, auf 0°C gekühlt und eine Lösung von N-{2-[2-(3-Hydroxymethyl-phenylamino)-5-trifluormethyl-pyrimidin-4-ylamino]-ethyl}-acetamid (Herstellung analog zu 2(52)) in 300 ml THF wird langsam bei 0°C hinzugegeben. Die Reaktionsmischung wird auf Raumtemperatur erwärmt und für 6 h gerührt. Nach Filtration der Reaktionsmischung wird der Filterkuchen gut mit THF gewaschen und die Lösung eingeengt. Der Rückstand wird in Essigester aufgenommen und nach Behandlung im Ultraschallbad erneut filtriert. Die Prozedure wird mit Ethylether erneut wiederholt und man erhält schliesslich einen weissen Feststoff in 70% Ausbeute.
   R_{f} = 0.40 (Kieselgel; Methylenchlorid: Methanol = 20:1)
   1H-NMR (D6-DMSO, 300 MHz) δ: 1.81 (s, 3 H), 3.30 (m, 2 H), 3.55 (m, 2 H), 7.30 (m, 1 H), 7.82 (m, 2 H), 7.96 (m, 4 H), 8.25 (s, 1 H), 9.82 (s, 1 H), 10.11 (s, 1 H).
(133) N-{2-[2-(4-Formyl-phenylamino)-5-trifluormethyl-pyrimidin-4-ylamino]-ethyl}-acetamid
   N-{2-[2-(4-Formyl-phenylamino)-5-trifluormethyl-pyrimidin-4-ylamino]-ethyl}-acetamid wird analog 2(132) aus N-{2-[2-(4-Hydroxymethyl-phenylamino)-5-trifluormethyl-pyrimidin-4-ylamino]-ethyl}-acetamid (Herstellung analog zu 2(52)) in 76% Ausbeute erhalten.
   R_{f} = 0.35 (Kieselgel; Methylenchlorid: Methanol = 20:1)
   HPLC/MS (Methode F): RT = 3.48 min.; [M+H]+ = 368
   1H-NMR (D6-DMSO, 300 MHz) δ: 1.80 (s, 3 H), 3.31 (m, 2 H), 3.57 (m, 2 H), 7.22 (m, 1 H), 7.51 (m, 2 H), 7.91 (m, 3 H), 8.21 (s, 1 H), 8.42 (s, 1 H), 9.90 (s, 1 H), 9.93 (s, 1 H).
(134) [5-Chloro-4-(1,4,6,7-tetrahydro-imidazo[4,5-c]pyridin-5-yl)-pyrimidin-2-yl]-(1H-indazol-6-yl)-amin
   Schmelzpunkt: >300 °C Zersetzung
   R_{f} = 0,42 (Kieselgel; Methylenchlorid: Methanol = 4:1)
   HPLC/MS (Methode F): RT = 3,09 min.; [M+H]+ = 367; Abs. λ max = 282 nm
(135) (1H-Indazol-6-yl)-[5-methyl-4-(4,5,7,8-tetrahydro-1H-imidazo[4,5-*d*]azepin-6-yl)-pyrimidin-2-yl]-amin
   Schmelzpunkt: > 300°C Zersetzung
   R_{f} = 0,08 (Kieselgel; Methylenchlorid: Methanol = 4:1)
   HPLC/MS (Methode F): RT = 2,92 min.; [M+H]+ = 361; Abs. λ max = 246 nm
(136) 4-[5-Chloro-4-(1,4,6,7-tetrahydro-imidazo[4,5-*c*]pyridin-5-yl)-pyrimidin-2-ylamino]-N,N-dimethyl-phenylsulfonamid
   Schmelzpunkt: 171-173 °C
   R_{f} = 0,29 (Kieselgel; Methylenchlorid: Methanol = 9:1)
   HPLC/MS (Methode F): RT = 3,71 min.; [M+H]+ = 434; Abs. λ max = 291 nm
(137) [5-Chloro-4-(4,5,7,8-tetrahydro-1H-imidazo[4,5-*d*]azepin-6-yl)-pyrimidin-2-yl]-(1H-indazol-6-yl)-amin
   Schmelzpunkt: >290 °C Zersetzung
   R_{f} = 0,19 (Kieselgel; Methylenchlorid: Methanol = 4:1)
   HPLC/MS (Methode F): RT = 3,03 min.; [M+H]+= 381; Abs. λ max = 282 nm
(138) (1H-Indazol-6-yl)-[5-methyl-4-(1,4,6,7-tetrahydro-imidazo[4,5-*c*]pyridin-5-yl)-pyrimidin-2-yl]-amin
   Schmelzpunkt: > 320°C Zersetzung
   R_{f} = 0,3 (Kieselgel; Methylenchlorid: Methanol = 8:2)
   HPLC/MS (Methode F): RT = 2,87 min.; [M+H]+ = ; 347 Abs. λ max = 251 nm
(139) [5-Methoxy-4-(1,4,6,7-tetrahydro-imidazo[4,5-*c*]pyridin-5-yl)-pyrimidin-2-yl]-(4-piperidin-1-ylmethyl-phenyl)-amin
   Schmelzpunkt: 105-108°C
   R_{f} = 0,06 (Kieselgel; Methylenchlorid: Methanol = 4:1)
   HPLC/MS (Methode F): RT = 2,83 min.; [M+H]+ = 420; Abs. λ max = 268,4 nm
(140) (3,4-Dichloro-phenyl)-[5-methoxy-4-(1,4,6,7-tetrahydro-imidazo[4,5-c]pyridin-5-yl)-pyrimidin-2-yl]-amin
   R_{f} = 0,13 (Kieselgel; Methylenchlorid: Methanol = 9:1)
   HPLC/MS (Methode G): RT = 3,22 min.; [M+H]+ = 392; Abs. λ max = 272,2 nm
(141) [5-Bromo-4-(4,5,7,8-tetrahydro-1H-imidazo[4,5-*d*]azepin-6-yl)-pyrimidin-2-yl]-(1H-indazol-6-yl)-amin
   Schmelzpunkt: 258-260 °C
   R_{f} = 0,22 (Kieselgel; Methylenchlorid: Methanol = 9:1)
   HPLC/MS (Methode F): RT = 3,05 min.; [M+H]+ = 426; Abs. λ max = 283 nm
(142) [5-Bromo-4-(1,4,6,7-tetrahydro-imidazo[4,5-*c*]pyridin-5-yl)-pyrimidin-2-yl]-(1H-indazol-6-yl)-amin
   Schmelzpunkt: >300 °C Zersetzung
   R_{f} = 0,2 (Kieselgel; Methylenchlorid: Methanol = 9:1)
   HPLC/MS (Methode F): RT = 3,12 min.; [M+H]+ =412; Abs. λ max = 255 nm
(143) N,N-Dimethyl-4-[5-methyl-4-(1,4,6,7-tetrahydro-imidazo[4,5-*c*]pyridin-5-yl)-pyrimidin-2-ylamino]-phenylsulfonamid
   Schmelzpunkt: 228-230 °C
   R_{f} = 0,27 (Kieselgel; Methylenchlorid: Methanol = 9:1)
   HPLC/MS (Methode F): RT = 3,14min.; [M+H]+ = 414; Abs. λ max = 282 nm
(144) N,N-Dimethyl-4-[5-methyl-4-(4,5,7,8-tetrahydro-1H-imidazo[4,5-*d*]azepin-6-yl)-pyrimidin-2-ylamino]-phenylsulfonamid
   Schmelzpunkt: 173-176 °C
   R_{f} = 0,2 (Kieselgel; Methylenchlorid: Methanol = 9:1)
   HPLC/MS (Methode F): RT = 3,04min.; [M+H]+ = 428; Abs. λ max = 256 nm
(145) *N*⁵,*N*⁵-Dimethyl-*N*⁵-(4-piperidin-1-ylmethyl-phenyl)-4-(1,4,6,7-tetrahydro-imidazo[4,5-*c*]pyridin-5-yl)-pyrimidin-2,5-diamin
   Schmelzpunkt: 126-129 °C
   R_{f} = 0,08 (Kieselgel; Methylenchlorid: Methanol = 4:1)
   HPLC/MS (Methode F): RT = 2,89 min.; [M+H]+ = 433; Abs. λ max = 257 nm
(146) [5-Isopropyl-4-(1,4,6,7-tetrahydro-imidazo[4,5-*c*]pyridin-5-yl)-pyrimidin-2-yl]-(4-piperidin-1-ylmethyl-phenyl)-amin
   Schmelzpunkt: 234-237 °C
   R_{f} = 0,13 (Kieselgel; Methylenchlorid: Methanol = 9:1)
   HPLC/MS (Methode F): RT = 2,95 min.; [M+H]+ = 432; Abs. λ max = 266,5 nm
(147) N-{2-[2-(3,4-Dichloro-phenylamino)-5-methanesulfonyl-pyrimidin-4-ylamino]-ethyl}-acetamid
   Schmelzpunkt: 245-248 °C
   R_{f} = 0,36 (Kieselgel; Methylenchlorid: Methanol = 9:1)
   HPLC/MS (Methode F): RT = 4,15 min.; [M+H]+ = 419; Abs. λ max = 276 nm
(148) N-(2-{5-Methyl-2-[3-(2,2,2-trifluor-ethyl)-2,3,4,5-tetrahydro-1H-benzo[*d*]azepin-7-ylamino]-pyrimidin-4-ylamino}-ethyl)-acetamid
   Schmelzpunkt: 154-156 °C
   R_{f} = 0,27 (Kieselgel; Methylenchlorid: Methanol = 9:1)
   HPLC/MS (Methode D): RT = 2,12 min.; [M+H]+ = 437; Abs. / max = 253 nm
(149) N-(2-{5-Chlor-2-[3-(2,2,2-trifluor-ethyl)-2,3,4,5-tetrahydro-1H-benzo[*d*]azepin-7-ylamino]-pyrimidin-4-ylamino}-ethyl)-acetamid
   Schmelzpunkt: 205-208 °C
   R_{f} = 0,44 (Kieselgel; Methylenchlorid: Methanol = 9:1)
   HPLC/MS (Methode D): RT = 3,07 min.; [M+H]+ = 457; Abs. / max = 263 nm
(150) 1-(3-{2-[3-(2,2,2-Trifluor-ethyl)-2,3,4,5-tetrahydro-1H-benzo[*d*]azepin-7-ylamino]-5-trifluormethyl-pyrimidin-4-ylamino}-propyl)-pyrrolidin-2-on
   Schmelzpunkt: 132-133 °C
   R_{f} = 0,62 (Kieselgel; Methylenchlorid: Methanol = 9:1)
   HPLC/MS (Methode F): RT = 2,95 min.; [M+H]+ = 531; Abs. / max = 259 nm
(151) N-(2-{5-Brom-2-[3-(2,2,2-trifluor-ethyl)-2,3,4,5-tetrahydro-1H-benzo[*d*]azepin-7-ylamino]-pyrimidin-4-ylamino}-ethyl)-acetamid
   Schmelzpunkt: 186-191 °C
   R_{f} = 0,44 (Kieselgel; Methylenchlorid: Methanol = 9:1)
   HPLC/MS (Methode D): RT = 3,12 min.; [M+H]+ = 502; Abs. / max = 267 nm
(152) 1-(3-{5-Methyl-2-[3-(2,2,2-trifluor-ethyl)-2,3,4,5-tetrahydro-1H-benzo[*d*]azepin-7-ylamino]-pyrimidin-4-ylamino}-propyl)-pyrrolidin-2-on
   Schmelzpunkt: Öl
   R_{f} = 0,38 (Kieselgel; Methylenchlorid: Methanol = 9:1)
   HPLC/MS (Methode D): RT = 3,67 min.; [M+H]+ = 477; Abs. / max = 259 nm
(153) 1-(3-{5-Chlor-2-[3-(2,2,2-trifluor-ethyl)-2,3,4,5-tetrahydro-1H-benzo[*d*]azepin-7-ylamino]-pyrimidin-4-ylamino}-propyl)-pyrrolidin-2-on
   Schmelzpunkt: 57-75 °C
   R_{f} = 0,79 (Kieselgel; Methylenchlorid: Methanol = 8:2)
   HPLC/MS (Methode D): RT = 3,70 min.; [M+H]+ = 497; Abs. / max = 265 nm
(154) 1-(3-{5-Brom-2-[3-(2,2,2-trifluor-ethyl)-2,3,4,5-tetrahydro-1H-benzo[*d*]azepin-7-ylamino]-pyrimidin-4-ylamino}-propyl)-pyrrolidin-2-on
   Schmelzpunkt: 138-144 °C
   R_{f} = 0,55 (Kieselgel; Methylenchlorid: Methanol = 9:1)
   HPLC/MS (Methode D): RT = 3,71 min.; [M+H]+ = 542; Abs. / max = 263 nm

### Beispiel 3

### N-{2-[2-(3,4-Dichloro-phenylamino)-5-trimethylsilanylethynyl-pyrimidin-4-ylamino]-ethyl}-acetamid hydrochlorid

Analog Beispiel 2 wurden 229 mg N-{2-[2-(3,4-Dichloro-phenylamino)-5-trimethylsilanylethynyl-pyrimidin-4-ylamino]-ethyl}-acetamid hydrochlorid aus 104 mg 3,4-Dichloranilin und 200 mg N-[2-(2-Chloro-5-trimethylsilanylethynyl-pyrimidin-4-ylamino)-ethyl]acetamid Beispiel II(10) erhalten.
Smp.: 206-208°C
Rf (Ethylacetat; SiO2) = 0.51
RT (HPLC, Methode D) = 7.29 min., UVmax = 286 nm

Analog Beispiel 3 wurde folgende Verbindung erhalten:
N-{2-[2-(4-Dimethylsulfamoyl-phenylamino)-5-trimethylsilanylethynyl-pyrimidin-4-ylamino]-ethyl}-acetamid hydrochlorid

### Beispiel 4

### N-{2-[2-(3,4-Dichloro-phenylamino)-5-ethynyl-pyrimidin-4-ylamino]-ethyl}-acetamid

145 mg Tetrabutylammoniumfluorid x 3H₂O wird in 10 ml Methanol gelöst und mit 100 mg N-{2-[2-(3,4-Dichloro-phenylamino)-5-trimethylsilanylethynyl-pyrimidin-4-ylamino]-ethyl}-acetamid versetzt, im Ultraschallbad homogenisiert und über Nacht bei RT gerührt.
Der Ansatz wird anschließend im Vakuum eingeengt und über Kieselgel mit Methanol/Dichlormethan (1/10) über Kieselgel filtriert. 54 mg N-{2-[2-(3,4-Dichloro-phenylamino)-5-ethynyl-pyrimidin-4-ylamino]-ethyl}-acetamid wird in 64%iger Ausbeue isoliert.
Smp.: 221-224°C
Rf (Ethylacetat/SiO2) = 0.32
RT (HPLC, Methode D) = 5.30 min., UVmax = 282 nm

Analog Beispiel 4 wurde folgende Verbindung erhalten:
4(1) N-{2-[2-(4-Dimethylsulfamoyl-phenylamino)-5-ethynyl-pyrimidin-4-ylamino]-ethyl}-acetamid
Smp.: 179-185°C
Rf (Ethylacetat/SiO2) = 0.14
RT (HPLC, Methode D) = 4.71 min., UVmax = 294 nm

### Beispiel 5

### N-{2-[2-(4-Dimethylsulfamoyl-phenylamino)-5-ethyl-pyrimidin-4-ylamino]-ethyl}-acetamid Hydrochlorid

50 mg N-{2-[2-(3,4-Dichloro-phenylamino)-5-ethynyl-pyrimidin-4-ylamino]-ethyl}-acetamid werden in 15 ml Ethanol und 15 ml Essigsäureethylester gelöst und mit 25 mg Pd/C (5%) versetzt. Im Schüttelautoklaven hydriert man bei Raumtemperatur und 3,5 bar (50 psi) über 3,5 Stunden.
Anschliessend wird der Katalysator abfiltriert, die Lösung mit HCl in Dioxan versetzt und eingeengt. Das Produkt fällt in 50 mg Ausbeute an.
Smp.: >219 °C Zersetzung
Rf (Ethylacetat/SiO2) = 0.39
RT (HPLC, Methode D) = 4.37 min., UVmax = 282 nm

### Beispiel 6

4-[4-(2-Acetylamino-ethylamino)-5-isopropyl-pyrimidin-2-ylamino]-benzoesäure 150 mg N-[2-(2-Chloro-5-isopropyl-pyrimidin-4-ylamino)-ethyl]-acetamid, Methyl-4-aminobenzoat (5eq.) und 10 mg 4-Dimethylaminopyridin wird werden in 2 ml Isopropanol in einem verschlossenen Reaktionsglas auf 150°C für 48 Stunden erhitzt. Nach Extraktion mit Essigsäureethylester aus ges. Bicarbonatlösung wird über Natriumsulfat getrocknet und eingeengt. Chromatographie (CH2CL2/MeOH-Gradient, Kieselgel) liefert den Methylester. Dieser wird in 3 ml Methanol aufgenommen, mit einer 1 M LiOH-Lösung (10 eq.) versetzt und bei 50°C bis zu 24 Stunden behandelt. Durch Zugabe einer 10%igen NaH2PO4-Lösung wird der pH auf 5 eingestellt (alternativ mit einer 1 M HCl-Lösung auf pH = 4) und das Produkt ausgefällt. Nach Filtration wird das Produkt mit Wasser, Diethylether und Essigsäureethylester gewaschen und im Vakuum getrocknet. Man erhält 95 mg Produkt.
Schmelzpunkt: 275-278°C
R_{f}= 0,04 (Kieselgel; Methylenchlorid: Methanol = 4:1)
HPLC/MS (Methode G): RT =3,01 min.; [M+H]+ = 358; Abs. λ max = 291,2 nm

Analog Beispiel 6 werden folgende Verbindungen erhalten:
(1) 4-(5-Methanesulfonyl-4-[3-(2-oxo-pyrrolidin-1-yl)-propylamino]-pyrimidin-2-ylamino]-benzoesäure
   Schmelzpunkt: >270 °C Zersetzung
   R_{f}= 0,17 (Kieselgel; Methylenchlorid: Methanol = 9:1)
   HPLC/MS (Methode F): RT = 3,43 min.; [M+H]+ = 434; Abs. λ max = 293 nm
(2) 4-(5-Methoxy-4-[3-(2-oxo-pyrrolidin-1-yl)-propylamino]-pyrimidin-2-ylamino]- benzoesäure
   Schmelzpunkt: >218 °C Zersetzung
   R_{f}= 0,12 (Kieselgel; Methylenchlorid: Methanol = 9:1)
   HPLC/MS (Methode J): RT = 4,88 min.; [M+H]+ = 386; Abs. λ max = 298,8 nm
(3) 4-(5-Dimethylamino-4-[3-(2-oxo-pyrrolidin-1-yl)-propylamino]-pyrimidin-2-ylamino]-benzoesäure
   Schmelzpunkt: > 235 °C Zersetzung
   R_{f}= 0,1 (Kieselgel; Methylenchlorid: Methanol = 9:1)
   HPLC/MS (Methode G): RT = 3,08 min.; [M+H]+ = 399; Abs. λ max = 298,8 nm
(4) 4-[4-(2-Acetylamino-ethylamino)-5-trifluormethyl-pyrimidin-2-ylamino]-2-chloro-benzoesäure
   Schmelzpunkt: 261 °C
   R_{f}= 0,15 (Kieselgel; Methylenchlorid: Methanol = 4: 1)
   HPLC/MS (Methode G): RT = 3,28 min.; [M+H]+ = 418 ; Abs. λ max = 251,3 nm
   1H-NMR(D₆-DMSO, 300MHz) δ: 1.80 (s, 3 H), 3.34 (m, 2 H), 3.54 (m, 2 H), 7.54 (m, 1 H), 7.78 (m, 1 H), 7.84 (m, 1 H), 8.03 (m, 2 H), 8.30 (s, 1 H), 10.23 (s, 1 H).
(5) 4-[4-(2-Acetylamino-ethylamino)-5-trifluormethyl-pyrimidin-2-ylamino]-3-chloro-benzoesäure
   Schmelzpunkt: 245 °C
   R_{f}= 0,33 (Kieselgel; Methylenchlorid: Methanol = 4:1)
   HPLC/MS (Methode G): RT = 3,35 min.; [M+H]+ = 418; Abs. λ max = 253,2 nm
   1H-NMR(D₆-DMSO, 300MHz) □: 1.78 (s, 3 H), 3.24 (m, 2 H), 3.43 (m, 2 H), 7.31 (t, 1 H), 7.90 (m, 3 H), 8.20 (m, 2 H), 8.76 (s, 1 H), 13.04 (s, 1 H).
(6) 4-[5-Methoxy-4-(4-pyridin-2-yl-piperazin-1-yl)-pyrimidin-2-ylamino]-benzoesäure
   Schmelzpunkt: > 260 °C Zersetzung
   R_{f}= 0,23 (Kieselgel; Methylenchlorid: Methanol = 9:1)
   HPLC/MS (Methode G): RT = 2,84 min.; [M+H]+ = 407; Abs. λ max = 298,8 nm
(7) 4-(5-Isopropyl-4-[3-(2-oxo-pyrrolidin-1-yl)-propylamino]-pyrimidin-2-ylamino]- benzoesäure
   Schmelzpunkt: > 315°C
   R_{f}= 0,08 (Kieselgel; Methylenchlorid: Methanol = 9:1)
   HPLC/MS (Methode G): RT = 3,21 min.; [M+H]+ = 398 ; Abs. λ max = 257 nm

### Beispiel 7

### N-(2-[2-(3,4-Dichloro-phenylamino)-5-trifluormethyl-pyrimidin-4-ylamino]-ethyl)-benzamid

100 mg N4-(2-Amino-ethyl)-N2-(3,4-dichloro-phenyl)-5-trifluormethyl-pyrimidine-2,4-diamin wurden in Pyridin/CH2Cl2 (1ml/1ml) gelöst, auf 0°C gekühlt und 1.1 eq Benzoesäurechlorid wurden in 1 ml CH2Cl2 langsam hinzugegeben. Man ließ die Reaktionsmischung auf Raumtemperatur erwärmen und über Nacht rühren. Nach Zugabe von 2 ml ges. NaHCO3-Lösung wurde mit Essigsäureethylester extrahiert, über Na2SO4 getrocknet und eingeengt. Anschließend wurde der Rückstand mit Essigsäureethylester, Diethylether und Dichlormethan gewaschen und über Kieselgel (CH₂Cl₂, MeOH) chromatographiert. Man erhält 83 mg Ausbeute.
Schmelzpunkt: 226-228°C
R_{f}= 0,57 (Kieselgel; Methylenchlorid: Methanol = 99:1)
HPLC/MS (Methode I): RT = 4,04 min.; [M+H]+ = 471; Abs. λ max = 266,5 nm

Analog Beispiel 7 werden folgende Verbindungen erhalten:
(1) N-(2-[2-(3,4-Dichloro-phenylamino)-5-trifluormethyl-pyrimidin-4-ylamino]-ethyl)-methansulfonamid
   100 mg N4-(2-Amino-ethyl)-N2-(3,4-dichloro-phenyl)-5-trifluormethyl-pyrimidin-2,4-diamin wurden mit Diisopropylethylamin (4 eq.) in THF (2ml) gelöst und 1.1 eq Methansulfonsäurechlorid wurden langsam hinzugegeben. Man ließ die Reaktionsmischung auf für 4 Stunden rühren. Nach Zugabe von 2 ml ges. NaHCO3-Lösung wurde mit Essigsäureethylester extrahiert, über Na2SO4 getrocknet und eingeengt. Anschließend wurde der Rückstand mit Essigsäureethylester, Diethylether und Dichlormethan gewaschen und über Kieselgel (CH₂Cl₂, MeOH) chromatographiert. Man erhält 91 mg Ausbeute.
   Schmelzpunkt: 195-196 °C
   R_{f}= 0,50 (Kieselgel; Methylenchlorid: Methanol = 99:1)
   HPLC/MS (Methode K): RT = 2,44 min.; [M+H]+ = 445; Abs. λ max = 266,5 nm
(2) *N*-{3-[2-(1-Methyl-1*H*-indazol-6-ylamino)-5-trifluormethyl-pyrimidin-4-ylamino]-propyl}-isobutyramid
   Herstellung ausgehend von Verbindung 1 (674).
   Schmelzpunkt: 268 °C
   R_{f}= 0,38 (Kieselgel; Methylenchlorid: Methanol = 95:5)
   HPLC/MS (Methode D): RT = 5,50 min.; [M+H]+ = 436; Abs. λ max = 250 nm
(3) N-(2-[2-(3,4-Dichloro-phenylamino)-5-trifluormethyl-pyrimidin-4-ylamino]-ethyl)-dimethylamino-acetamid
   100 mg N4-(2-Amino-ethyl)-N2-(3,4-dichloro-phenyl)-5-trifluormethyl-pyrimidin-2,4-diamin [ 1(80) ] wurden mit Dimethylaminoessigsäure (1 eq.), Diisopropylethylamin (2 eq.), HOBT (1.3 eq.) und HBTU (1.3 eq.) in DMF (2ml) gelöst und über Nacht bei Raumtemperatur gerührt. Nach Zugabe von 2 ml 2 M NaHCO3-Lösung wurde mit Essigsäureethylester extrahiert, über Na2SO4 getrocknet und eingeengt. Anschließend wurde der über Kieselgel (CH₂Cl₂, MeOH) chromatographiert. Man erhält 62 mg Ausbeute.
   Schmelzpunkt: 165-167°C
   R_{f}= 0,17 (Kieselgel; Methylenchlorid: Methanol = 99:1)
   HPLC/MS (Methode H): RT = 3,07min.; [M+H]+ = 452; Abs. λ max = 264,6 nm
(4) N-(2-[2-(3,4-Dichloro-phenylamino)-5-trifluormethyl-pyrimidin-4-ylamino]-ethyl)-isobutyramid
   Herstellung ausgehend von Verbindung 1 (80).
   Schmelzpunkt: 232-235°C
   R_{f}= 0,55 (Kieselgel; Methylenchlorid: Methanol = 99:1)
   HPLC/MS (Methode I): RT = 2,74 min.; [M+H]+ = 437; Abs. λ max = 266,5 nm
(5) N-(2-[2-(3,4-Dichloro-phenylamino)-5-trifluormethyl-pyrimidin-4-ylamino]-ethyl)-methoxy-acetamid
   Herstellung ausgehend von Verbindung 1 (80).
   Schmelzpunkt: 197-201 °C
   R_{f}= 0,45 (Kieselgel; Methylenchlorid: Methanol = 99:1)
   HPLC/MS (Methode G): RT = 3,27 min.; [M+H]+= 439; Abs. λ max = 266,5 nm
(6) *N*-{3-[2-(1-Methyl-1*H*-indazol-6-ylamino)-5-trifluormethyl-pyrimidin-4-ylamino]-propyl}-propionamid
   Herstellung ausgehend von Verbindung 1 (674).
   Schmelzpunkt: 261 °C
   R_{f}= 0,37 (Kieselgel; Methylenchlorid: Methanol = 95:5)
   HPLC/MS (Methode D): RT = 5,30 min.
(7) *N*-{3-[2-(1-Methyl-1*H*-indazol-6-ylamino)-5-trifluoromethyl-pyrimidin-4-ylamino]-propyl}-methansulfonamid
   Herstellung ausgehend von Verbindung 1 (674).
   Schmelzpunkt: 230 °C
   R_{f}= 0,20 (Kieselgel; Methylenchlorid: Methanol = 95:5)
   HPLC/MS (Methode D): RT = 5,20 min.; [M+H]+ = 444; Abs. λ max = 250 nm
(8) N-(2-[2-(3,4-Dichloro-phenylamino)-5-trifluormethyl-pyrimidin-4-ylamino]-ethyl)-N-methyl-isobutyramid
   Herstellung ausgehend von Verbindung 1 (240).
   Schmelzpunkt: 138-140 °C
   R_{f}= 0,33 (Kieselgel; Methylenchlorid: Methanol = 99:1)
   HPLC/MS (Methode G): RT = 4,80 min.; [M+H]+ = 451; Abs. λ max = 274,1 nm
(9) N-(2-[2-(3,4-Dichloro-phenylamino)-5-trifluormethyl-pyrimidin-4-ylamino]-ethyl)-N-methyl-acetamid
   Herstellung ausgehend von Verbindung 1 (240).
   Schmelzpunkt: 174-175 °C
   R_{f}= 0,14 (Kieselgel; Methylenchlorid: Methanol =9 9:1)
   HPLC/MS (Methode G): RT = 4,19 min.; [M+H]+ = 423; Abs. λ max = 266,5 nm
(10) N-(3-[2-(3,4-Dichloro-phenylamino)-5-trifluormethyl-pyrimidin-4-ylamino]-propyl)-acetamid
   Herstellung ausgehend von Verbindung 1 (35).
   Schmelzpunkt: 200-203 °C
   R_{f}= 0,32 (Kieselgel; Methylenchlorid: Methanol = 99:1)
   HPLC/MS (Methode F): RT = 4,19 min.; [M+H]+ = 423; Abs. λ max = 266,5 nm
(11) N-(3-[2-(3,4-Dichloro-phenylamino)-5-trifluormethyl-pyrimidin-4-ylamino]-propyl)-2-methoxy-acetamid
   Herstellung ausgehend von Verbindung 1 (35).
   Schmelzpunkt: 160-162 °C
   R_{f}= 0,61 (Kieselgel; Methylenchlorid: Methanol = 99:1)
   HPLC/MS (Methode G): RT = 4,27 min.; [M+H]+= 453; Abs. λ max = 266,5 nm
(12) 4-Fluor-*N*-{3-[2-(1-methyl-1*H*-indazol-6-ylamino)-5-trifluormethyl-pyrimidin-4-ylamino]-propyl}-benzensulfonamid
   Herstellung ausgehend von Verbindung 1 (674).
   Schmelzpunkt: 256 °C
   R_{f}= 0,30 (Kieselgel; Methylenchlorid: Methanol = 95:5)
   HPLC/MS (Methode D): RT = 6,20 min.; [M+H]+ = 524; Abs. λ max = 242 nm
(13) 2-Methoxy-*N*-{3-[2-(1-methyl-1*H*-indazol-6-ylamino)-5-trifluormethyl-pyrimidin-4-ylamino]-propyl}-acetamid
   Herstellung ausgehend von Verbindung 1 (674).
   Schmelzpunkt: 241 °C
   R_{f}= 0,38 (Kieselgel; Methylenchlorid: Methanol = 95:5)
   HPLC/MS (Methode D): RT = 5,20 min.; [M+H]+ = 438; Abs. λ max = 250 nm
(14) N-1-[2-(3,4-Dichloro-phenylamino)-5-trifluormethyl-pyrimidin-4-yl]-pyrrolidin-3-yl)-acetamid
   Herstellung ausgehend von Verbindung 1 (338).
   Schmelzpunkt: 244-245°C
   R_{f}= 0,11 (Kieselgel; Methylenchlorid: Methanol = 99:1)
   HPLC/MS (Methode F): RT = 4,37 min.; [M+H]+ = 435; Abs. λ max = 268,4 nm
(15) 3-Methyl-*N*-{3-[2-(1-methyl-1*H*-indazol-6-ylamino)-5-trifluormethyl-pyrimidin-4-ylamino]-propyl}-butyramid
   Herstellung ausgehend von Verbindung 1 (674).
   Schmelzpunkt: 254 °C
   R_{f}= 0,40 (Kieselgel; Methylenchlorid: Methanol = 95:5)
   HPLC/MS (Methode D): RT = 5,70 min.; [M+H]+ = 450; Abs. λ max = 250 nm
(16) 2-Fluor-*N*-{3-[2-(1-methyl-1*H*-indazol-6-ylamino)-5-trifluormethyl-pyrimidin-4-ylamino]-propyl}-benzensulfonamid
   Herstellung ausgehend von Verbindung 1 (674).
   Schmelzpunkt: 253 °C
   R_{f}= 0,32 (Kieselgel; Methylenchlorid: Methanol = 95:5)
   HPLC/MS (Methode D): RT = 6,10 min.; [M+H]+ = 524; Abs. λ max = 250 nm
(17) 4-[4-(2-Acetyl-methyl-amino)-ethylamino)-5-trifluormethyl-pyrimidin-2-ylamino]-benzamid
   Herstellung ausgehend von Verbindung 1 (650).
   Schmelzpunkt: 250-252 °C
   R_{f}= 0,14 (Kieselgel; Methylenchlorid: Methanol = 9:1)
   HPLC/MS (Methode G): RT = 2,76 min.; [M+H]+ = 397; Abs. λ max = 277,9 nm
(18) 4-{4-[2-(2-Dimethylamino-acetylamino)-ethylamino]-5-trifluormethyl-pyrimidin-2-ylamino}-benzamid
   Herstellung ausgehend von Verbindung 1 (259).
   Schmelzpunkt: 215-218°C
   R_{f}= 0,24 (Kieselgel; Methylenchlorid: Methanol = 4:1)
   HPLC/MS (Methode F): RT = 2,86 min.; [M+H]+ = 426; Abs. λ max = 279,8 nm
(19) 4-[4-(2-Isobutyrylamino-ethylamino)-5-trifluormethyl-pyrimidin-2-ylamino]-benzamid
   Herstellung ausgehend von Verbindung 1 (259).
   Schmelzpunkt: 247-250 °C
   R_{f}= 0,16 (Kieselgel; Methylenchlorid: Methanol = 9:1)
   HPLC/MS (Methode F): RT = 3,18 min.; [M+H]+ = 411; Abs. λ max = 279,8 nm
(20) 4-[4-(2-Methansulfonylamino-ethylamino)-5-trifluormethyl-pyrimidin-2-ylamino]-benzamid
   Herstellung ausgehend von Verbindung 1 (259).
   Schmelzpunkt: 255-256 °C
   R_{f}= 0,13 (Kieselgel; Methylenchlorid: Methanol = 9:1)
   HPLC/MS (Methode G): RT = 2,83 min.; [M+H]+ = 419; Abs. λ max = 279,8 nm
(21) *N*-{3-[2-(1-Methyl-1*H*-indazol-6-ylamino)-5-trifluormethyl-pyrimidin-4-ylamino]-propyl}-2-phenyl-acetamid
   Herstellung ausgehend von Verbindung 1 (674).
   Schmelzpunkt: 230 °C
   R_{f}= 0,46 (Kieselgel; Methylenchlorid: Methanol = 95:5)
   HPLC/MS (Methode D): RT = 5,90 min.; [M+H]+ = 484; Abs. λ max = 250 nm
(22) {3-[2-(1-Methyl-1*H*-indazol-6-ylamino)-5-trifluormethyl-pyrimidin-4-ylamino]-propyl}-methylcarbamat
   Herstellung ausgehend von Verbindung 1 (674).
   Schmelzpunkt: 136°C
   R_{f}= 0,46 (Kieselgel; Methylenchlorid: Methanol = 95:5)
   HPLC/MS (Methode D): RT = 5,50 min.; [M+H]+ = 424; Abs. λ max = 250 nm
(23) 4-(4-[2-(Isobutyryl-methyl-amino)-ethylamino)-5-trifluormethyl-pyrimidin-2-ylamino)-benzamid
   Herstellung ausgehend von Verbindung 1 (650).
   Schmelzpunkt: 250-253 °C
   R_{f}= 0,19 (Kieselgel; Methylenchlorid: Methanol = 9:1)
   HPLC/MS (Methode G): RT = 3,12 min.; [M+H]+ = 424 ; Abs. λ max = 279,8 nm
(24) {3-[2-(1-Methyl-1*H*-indazol-6-ylamino)-5-trifluormethyl-pyrimidin-4-ylamino]-propyl}-isobutylcarbamat
   Herstellung ausgehend von Verbindung 1 (674).
   Schmelzpunkt: 226 °C
   R_{f}= 0,50 (Kieselgel; Methylenchlorid: Methanol = 95:5)
   HPLC/MS (Methode C): RT = 4,40 min.; [M+H]+ = 466; Abs. λ max = 250 nm
(25) {3-[2-(1-Methyl-1*H*-indazol-6-ylamino)-5-trifluormethyl-pyrimidin-4-ylamino]-propyl}-2-chlorbenzylcarbamat
   Herstellung ausgehend von Verbindung 1 (674).
   Schmelzpunkt: 194 °C
   R_{f} = 0,60 (Kieselgel; Methylenchlorid: Methanol = 95:5)
   HPLC/MS (Methode D): RT = 6,60 min.; [M+H]+ = 536; Abs. λ max = 254 nm
(26) N-(3-[2-(3,4-Dichloro-phenylamino)-5-trifluormethyl-pyrimidin-4-ylamino]-propyl)-2-dimethylamino-acetamid
   Herstellung ausgehend von Verbindung 1 (35).
   Schmelzpunkt: 187-189°C
   R_{f}= 0,09 (Kieselgel; Methylenchlorid: Methanol = 99:1)
   HPLC/MS (Methode G): RT = 3,85 min.; [M+H]+ = 466; Abs. λ max = 268,4 nm
(27) 4-[4-(3-Acetylamino-propylamino)-5-trifluormethyl-pyrimidin-2-ylamino]-benzamid
   Herstellung ausgehend von Verbindung 1 (697).
   Schmelzpunkt: 212-213 °C
   R_{f}= 0,13 (Kieselgel; Methylenchlorid: Methanol = 9:1)
   HPLC/MS (Methode F): RT = 3,01 min.; [M+H]+= 397; Abs. λ max = 279,8 nm
(28) 4-[4-(3-(2-Methoxy-acetamino)-propylamino)-5-trifluormethyl-pyrimidin-2-ylamino]-benzamid
   Herstellung ausgehend von Verbindung 1 (697).
   Schmelzpunkt: 212-213 °C
   R_{f}= 0,09 (Kieselgel; Methylenchlorid: Methanol = 99:1)
   HPLC/MS (Methode F): RT = 3,12 min.; [M+H]+= 467; Abs. λ max = 279,8 nm
(29) 4-(4-[3-(2-Dimethylamino-acetylamino)-propylamino]-5-trifluormethyl-pyrimidin-2-ylamino)-benzamid
   Herstellung ausgehend von Verbindung 1 (697).
   Schmelzpunkt: 184-187 °C
   R_{f}= 0,10 (Kieselgel; Methylenchlorid: Methanol = 9: 1)
   HPLC/MS (Methode F): RT = 2,90 min.; [M+H]+= 440; Abs. λ max = 279,8 nm
(30) *N*-{3-[2-(1-Methyl-1*H*-indazol-6-ylamino)-5-trifluormethyl-pyrimidin-4-ylamino]-propyl}-benzensulfonamid
   Herstellung ausgehend von Verbindung 1 (674).
   Schmelzpunkt: 264 °C
   R_{f}= 0,28 (Kieselgel; Methylenchlorid: Methanol = 95:5)
   HPLC/MS (Methode D): RT = 6,10 min.; [M+H]+ = 506; Abs. λ max = 238 nm
(31) 4-[4-{3-Acetylamino-pyrrolidin-1-yl)-5-trifluormethyl-pyrimidin-2-ylamino)-benzamid
   Herstellung ausgehend von Verbindung 1 (459).
   Schmelzpunkt: 244-246 °C
   R_{f}= 0,1 (Kieselgel; Methylenchlorid: Methanol = 9:1)
   HPLC/MS (Methode G): RT = 2,90 min.; [M+H]+ = 409; Abs. λ max = 283,6 nm
(32) 1,1-Diethyl-3-{3-[2-(1-methyl-1*H*-indazol-6-ylamino)-5-trifluonnethyl-pyrimidin-4-ylamino]-propyl}-harnstoff
   Herstellung ausgehend von Verbindung 1 (674).
   Schmelzpunkt: 228 °C
   R_{f}= 0,36 (Kieselgel; Methylenchlorid: Methanol = 95:5)
   HPLC/MS (Methode D): RT = 5,70 min.; [M+H]+= 465; Abs. λ max = 250 nm
(33) 4-[4-(2-Benzoylamino-ethylamino)-5-trifluormethyl-pyrimidin-2-ylamino]-benzamid
   Herstellung ausgehend von Verbindung 1 (259).
   Schmelzpunkt: 238-240 °C
   R_{f}= 0,26 (Kieselgel; Methylenchlorid: Methanol = 9:1)
   HPLC/MS (Methode G): RT = 3,22 min.; [M+H]+ = 445; Abs. λ max = 279,8 nm
(34) 4-(4-[2-(2-Methoxy-acetylamino)-ethylamino]-5-trifluormethyl-pyrimidin-2-ylamino]-benzamid
   Herstellung ausgehend von Verbindung 1 (259).
   Schmelzpunkt: 232-234°C
   R_{f}= 0,29 (Kieselgel; Methylenchlorid: Methanol =9:1)
   HPLC/MS (Methode G): RT = 2,77 min.; [M+H]+= 413; Abs. λ max = 279,8 nm
(35) 1,1-Dimethyl-3-{3-[2-(1-methyl-1*H*-indazol-6-ylamino)-5-triuormethyl-pyrimidin-4-ylamino]-propyl}-harnstoff
   Herstellung ausgehend von Verbindung 1 (674).
   Schmelzpunkt: 259 °C
   R_{f}= 0,59 (Kieselgel; Methylenchlorid: Methanol = 95:5)
   HPLC/MS (Methode D): RT = 5,20 min.; [M+H]+= 437; Abs. λ max = 250 nm
(36) 1-Isopropyl-3-{3-[2-(1-methyl-1H-indazol-6-ylamino)-5-trifluormethyl-pyrimidin-4-ylamino]-propyl}-harnstoff
   Herstellung ausgehend von Verbindung 1 (674).
   Schmelzpunkt: 190 °C
   R_{f}= 0,08 (Kieselgel; Methylenchlorid: Methanol = 98:2)

### Beispiel 8

### 4-[4-(2-Acetylamino-ethylamino)-5-trifluormethyl-pyrimidin-2-ylamino]-N-methyl-benzamid

100 mg 4-[4-(2-Acetylamino-ethylamino)-5-trifluormethyl-pyrimidin-2-ylamino]-benzoe-säure 9(1), Methylamin (1.3 eq.), HOBT (1.3 eq.), HBTU (1.3 eq.) und Diisopropylethylamin (3 eq.) werden in 2 ml DMF gegeben und über Nacht gerührt. Die Reaktionsmischung wird mit ges. Bicarbonatlösung versetzt und mit Essigsäureethylester extrahiert. Anschließend wird die org. Phase mit Wasser gewaschen, über Natriumsulfat getrocknet und eingeengt. Das Rohprodukt wird mit Essigsäureethylester, Diethylether und Dichlormethan gewaschen und gegebenenfalls mittels Chromatographie (Kieselgel, CH2Cl2/MeOH-Gradient) gereinigt. Man erhält 39 mg an Ausbeute.
Smp.: 196 °C
R_{f}= 0,11 (Kieselgel; Methylenchlorid: Methanol = 95:5)
Rt (HPLC, Methode F) = 3,06 min; [M+H]+ = 397; Abs. λ max = 279,8 nm

Analog Beispiel 8 werden folgende Verbindungen erhalten:
(1) 3-[4-(2-Acetylamino-ethylamino)-5-trifluormethyl-pyrimidin-2-ylamino]-N-methyl-benzamid
   Herstellung ausgehend von Verbindung 9 (0).
   Schmelzpunkt: 291 °C
   R_{f}= 0,12 (Kieselgel; Methylenchlorid: Methanol =9:1)
   HPLC/MS (Methode G): RT =2,88 min.; [M+H]+= 397; Abs. λ max = 239,9 nm
(2) 4-[4-(2-Acetylamino-ethylamino)-5-trifluormethyl-pyrimidin-2-ylamino]-3-chloro-N-methyl-benzamid
   Herstellung ausgehend von Verbindung 6 (5).
   Schmelzpunkt: 252 °C
   R_{f}= 0,09 (Kieselgel; Methylenchlorid: Methanol = 9:1)
   HPLC/MS (Methode G): RT = 3,04 min.; [M+H]+ = 431; Abs. λ max = 251,3 nm
(3) 4-[4-(2-Acetylamino-ethylamino)-5-trifluormethyl-pyrimidin-2-ylamino]-2-chloro-N-methyl-benzamid
   Herstellung ausgehend von Verbindung 6 (4).
   Schmelzpunkt: 183 °C
   R_{f}= 0,10 (Kieselgel; Methylenchlorid: Methanol = 9:1)
   HPLC/MS (Methode G): RT = 2,89 min.; [M+H]+ = 431; Abs. λ max = 270,3 nm
(4) 4-[4-(2-Acetylamino-ethylamino)-5-trifluormethyl-pyrimidin-2-ylamino]-N,N-dimethyl-benzamid
   Herstellung ausgehend von Verbindung 9 (1).
   Schmelzpunkt: 185 °C
   R_{f}= 0,14 (Kieselgel; Methylenchlorid: Methanol = 9:1)
   HPLC/MS (Methode G): RT = 2,84 min.; [M+H]+ = 411; Abs. λ max = 262,7 nm
(5) 3-[4-(2-Acetylamino-ethylamino)-5-trifluormethyl-pyrimidin-2-ylamino]-N,N-dimethyl-benzamid
   Herstellung ausgehend von Verbindung 9 (0).
   Schmelzpunkt: 205 °C
   R_{f} = 0,19 (Kieselgel; Methylenchlorid: Methanol = 9:1)
   HPLC/MS (Methode G): RT = 2,88 min.; [M+H]+ = 411; Abs. λ max = 245,6 nm
(6) 4-[4-(2-Acetylamino-ethylamino)-5-trifluormethyl-pyrimidin-2-ylamino]-3-chloro-N,N-dimethyl-benzamid
   Herstellung ausgehend von Verbindung 6 (5).
   Schmelzpunkt: 135 °C
   R_{f} = 0,54 (Kieselgel; Methylenchlorid: Methanol = 9:1)
   HPLC/MS (Methode G): RT = 3,01 min.; [M+H]+ = 445; Abs. λ max = 219 nm
(7) 4-[4-(2-Acetylamino-ethylamino)-5-trifluormethyl-pyrimidin-2-ylamino]-2-chloro-N,N-dimethyl-benzamid
   Herstellung ausgehend von Verbindung 6 (4).
   Schmelzpunkt: 198 °C
   R_{f}= 0,14 (Kieselgel; Methylenchlorid: Methanol = 9:1)
   HPLC/MS (Methode G): RT = 3,09 min.; [M+H]+ = 445; Abs. λ max = 253 nm
(8) 4-[4-(2-Acetylamino-ethylamino)-5-trifluormethyl-pyrimidin-2-ylamino]-N-benzyl-N-methyl-benzamid
   Herstellung ausgehend von Verbindung 9 (1).
   Schmelzpunkt: 214 °C
   R_{f}= 0,18 (Kieselgel; Methylenchlorid: Methanol = 9:1)
   HPLC/MS (Methode G): RT = 3,52 min.; [M+H]+ = 487; Abs. λ max = 253,2 nm
(9) 3-[4-(2-Acetylamino-ethylamino)-5-trifluormethyl-pyrimidin-2-ylamino]-N-benzyl-N-methyl-benzamid
   Herstellung ausgehend von Verbindung 9 (0).
   Schmelzpunkt: 157 °C
   R_{f}= 0,24 (Kieselgel; Methylenchlorid: Methanol = 9:1)
   HPLC/MS (Methode G): RT = 3,59 min.; [M+H]+ = 487; Abs. λ max = 203,8 nm
(10) 4-[4-(2-Acetylamino-ethylamino)-5-trifluormethyl-pyrimidin-2-ylamino]-3-chloro-N-benzyl-N-methyl-benzamid
   Herstellung ausgehend von Verbindung 6 (5).
   Schmelzpunkt: 152 °C
   R_{f}= 0,22 (Kieselgel; Methylenchlorid: Methanol = 9:1)
   HPLC/MS (Methode G): RT = 3,78 min.; [M+H]+ = 521; Abs. λ max = 209,5nm
(11) 4-[4-(2-Acetylamino-ethylamino)-5-trifluormethyl-pyrimidin-2-ylamino]-2-chloro-N-benzyl-N-methyl-benzamid
   Herstellung ausgehend von Verbindung 6 (4).
   Schmelzpunkt: 180 °C
   R_{f}= 0,20 (Kieselgel; Methylenchlorid: Methanol = 9:1)
   HPLC/MS (Methode G): RT = 3,85 min.; [M+H]+ = 522; Abs. λ max = 268,4 nm
(12) N-(2-(2-[4-(piperidin-1-carbonyl)-phenylamino]-5-trifluormethyl-pyrimidin-4-ylamino)-ethyl)-acetamid
   Herstellung ausgehend von Verbindung 9 (1).
   Schmelzpunkt: 199 °C
   R_{f}= 0,67 (Kieselgel; Methylenchlorid: Methanol = 85:15)
   HPLC/MS (Methode G): RT = 3,25 min.; [M+H]+ = 451; Abs. λ max = 266,5 nm
(13) N-(2-(2-[3-(Piperidin-1-carbonyl)-phenylamino]-5-trifluormethyl-pyrimidin-4-ylamino)-ethyl)-acetamid
   Herstellung ausgehend von Verbindung 9 (0).
   Schmelzpunkt: 179 °C
   R_{f}= 0,21 (Kieselgel; Methylenchlorid: Methanol = 9:1)
   HPLC/MS (Methode G): RT = 3,30 min.; [M+H]+ = 451; Abs. λ max = 245,6 nm
(14) N-(2-(2-[2-Chloro-4-(piperidin-1-carbonyl)-phenylamino]-5-trifluormethyl-pyrimidin-4-ylamino)-ethyl)-acetamid
   Herstellung ausgehend von Verbindung 6 (5).
   Schmelzpunkt: 108 °C
   R_{f}= 0,22 (Kieselgel; Methylenchlorid: Methanol = 9:1)
   HPLC/MS (Methode G): RT = 3,44 min.; [M+H]+ = 485; Abs. λ max = 243,7 nm
(15) N-(2-(2-[3-Chloro-4-(piperidin-1-carbonyl)-phenylamino]-5-trifluormethyl-pyrimidin-4-ylamino)-ethyl)-acetamid
   Herstellung ausgehend von Verbindung 6 (4).
   Schmelzpunkt: 193 °C
   R_{f}= 0,22 (Kieselgel; Methylenchlorid: Methanol = 9:1)
   HPLC/MS (Methode G): RT = 3,52 min.; [M+H]+ = 485 ; Abs. λ max = 266,5 nm
(16) N-(2-(2-[4-(Morpholin-4-carbonyl)-phenylamino]-5-trifluormethyl-pyrimidin-4-ylamino)-ethyl)-acetamid
   Herstellung ausgehend von Verbindung 9 (1).
   Schmelzpunkt: 221 °C
   R_{f}= 0,65 (Kieselgel; Methylenchlorid: Methanol = 85:15)
   HPLC/MS (Methode G): RT = 2,85 min.; [M+H]+ = 453; Abs. λ max = 262,7 nm
(17) N-(2-(2-[3-(Morpholin-4-carbonyl)-phenylamino]-5-trifluormethyl-pyrimidin-4-ylamino)-ethyl)-acetamid
   Herstellung ausgehend von Verbindung 9 (0).
   Schmelzpunkt: 196 °C
   R_{f} = 0,18 (Kieselgel; Methylenchlorid: Methanol = 9:1)
   HPLC/MS (Methode G): RT = 2,89 min.; [M+H]+ = 453; Abs. λ max = 245,6 nm
(18) N-(2-(2-[2-Chloro-4-(morpholin-4-carbonyl)-phenylamino]-5-trifluormethyl-pyrimidin-4-ylamino)-ethyl)-acetamid
   Herstellung ausgehend von Verbindung 6 (5).
   Schmelzpunkt: 40 °C
   R_{f}= 0,20 (Kieselgel; Methylenchlorid: Methanol = 9:1)
   HPLC/MS (Methode G): RT = 3,31 min.; [M+H]+ = 487; Abs. λ max = 220,9 nm
(19) N-(2-(2-[3-Chloro-4-(morpholin-4-carbonyl)-phenylamino]-5-trifluormethyl-pyrimidin-4-ylamino)-ethyl)-acetamid
   Herstellung ausgehend von Verbindung 6 (4).
   Schmelzpunkt: 197 °C
   R_{f}= 0,20 (Kieselgel; Methylenchlorid: Methanol = 9:1)
   HPLC/MS (Methode G): RT = 3,09 min.; [M+H]+ = 487; Abs. λ max = 266,5 nm
(20) *N*-(2-{2-[4-(4-Methyl-piperazin-1-carbonyl)-phenylamino]-5-trifluormethyl-pyrimidin-4-ylamino}-ethyl)-acetamid
   Herstellung ausgehend von Verbindung 9 (1).
   Schmelzpunkt: 228 °C
   R_{f}= 0,37 (Kieselgel; Methylenchlorid: Methanol = 85:15)
   HPLC/MS (Methode G): RT = 2,39 min.; [M+H]+ = 466; Abs. λ max = 272,2 nm
(21) N-(2-(2-[3-(4-Methyl-piperazin-1-carbonyl)-phenylamino]-5-trifluormethyl-pyrimidin-4-ylamino)-ethyl)-acetamid
   Herstellung ausgehend von Verbindung 9 (0).
   Schmelzpunkt: 186°C
   R_{f}= 0,54 (Kieselgel; Methylenchlorid: Methanol = 4:1)
   HPLC/MS (Methode G): RT = 2,50 min.; [M+H]+ = 466; Abs. λ max = 245,6 nm
(22) 4-[4-(2-Acetylamino-ethylamino)-5-trifluormethyl-pyrimidin-2-ylamino]-N-pyridin-2-ylmethyl-benzamid
   Herstellung ausgehend von Verbindung 9 (1).
   Schmelzpunkt: 229°C
   R_{f} = 0,61 (Kieselgel; Methylenchlorid: Methanol = 85:15)
   HPLC/MS (Methode G): RT = 2,68 min.; [M+H]+= 474; Abs. λ max = 281,7 nm
(23) 3-[4-(2-Acetylamino-ethylamino)-5-trifluormethyl-pyrimidin-2-ylamino]-N-pyridin-2-ylmethyl-benzamid
   Herstellung ausgehend von Verbindung 9 (0).
   Schmelzpunkt: 216 °C
   R_{f} = 0,69 (Kieselgel; Methylenchlorid: Methanol = 85:15)
   HPLC/MS (Methode G): RT = 2,70 min.; [M+H]+= 474; Abs. λ max = 260,8 nm
(24) 4-[4-(2-Acetylamino-ethylamino)-5-trifluormethyl-pyrimidin-2-ylamino]-N-pyridin-2-yl-benzamid
   Herstellung ausgehend von Verbindung 9 (1).
   Schmelzpunkt: 225 °C
   R_{f}= 0,09 (Kieselgel; Methylenchlorid: Methanol = 85:15)
   HPLC/MS (Methode G): RT = 4,24 min.; [M+H]+ = 460; Abs. λ max = 205,7 nm
(25) 3-[4-(2-Acetylamino-ethylamino)-5-trifluormethyl-pyrimidin-2-ylamino]-N-pyridin-2-yl-benzamid
   Herstellung ausgehend von Verbindung 9 (0).
   Schmelzpunkt: 251 °C
   R_{f}= 0,20 (Kieselgel; Methylenchlorid: Methanol = 9:1)
   HPLC/MS (Methode G): RT = 2,99 min.; [M+H]+ = 460 ; Abs. λ max = 247,5 nm
(26) 4-[4-(2-Acetylamino-ethylamino)-5-trifluormethyl-pyrimidin-2-ylamino]-N-(3,5-difluorbenzyl)-benzamid
   Herstellung ausgehend von Verbindung 9 (1).
   Schmelzpunkt: 231 °C
   R_{f}= 0,11 (Kieselgel; Methylenchlorid: Methanol = 9:1)
   HPLC/MS (Methode G): RT = 3,67 min.; [M+H]+= 509; Abs. λ max = 281,7 nm
(27) 3-[4-(2-Acetylamino-ethylamino)-5-trifluormethyl-pyrimidin-2-ylamino]-N-(3,5-difluorbenzyl)-benzamid
   Herstellung ausgehend von Verbindung 9 (0).
   Schmelzpunkt: 226 °C
   R_{f}= 0,72 (Kieselgel; Methylenchlorid: Methanol = 85:15)
   HPLC/MS (Methode G): RT = 3,71 min.; [M+H]+ = 509; Abs. λ max = 239,9 nm
(28) 4-[4-(2-Acetylamino-ethylamino)-5-trifluormethyl-pyrimidin-2-ylamino]-N-(1-phenylethyl)-benzamid
   Herstellung ausgehend von Verbindung 9 (1).
   Schmelzpunkt: 237°C
   R_{f}= 0,10 (Kieselgel; Methylenchlorid: Methanol = 9:1)
   HPLC/MS (Methode G): RT = 3,60 min.; [M+H]+= 487; Abs. λ max = 279,8 nm
(29) 3-[4-(2-Acetylamino-ethylamino)-5-trifluormethyl-pyrimidin-2-ylamino]-N-(1-phenylethyl)-benzamid
   Herstellung ausgehend von Verbindung 9 (0).
   Schmelzpunkt: 234°C
   R_{f} = 0,23 (Kieselgel; Methylenchlorid: Methanol = 9: 1)
   HPLC/MS (Methode G): RT = 3,66 min.; [M+H]+ = 487; Abs. λ max = 241,8 nm

### Beispiel 9

3-[4-(2-Acetylamino-ethylamino)-5-trifluormethyl-pyrimidin-2-ylamino]-benzoesäure N-[2-(2-Chloro-5-trifluormethyl-pyrimidin-4-ylamino)-ethyl]-acetamid (1 eq.) 3-Aminobenzoesäure (4 eq.) und Isopropanol werden über Nacht bei 60 °C erhitzt. Die Reaktionsmischung wird mit Essigsäureethylester verdünnt und mit 0.01 N HCL-Lösung gewaschen. Dar gebildete Feststoff wird abfiltriert und mit Essigsäureethylester durch Zentrifugation gewaschen und soweit erforderlich durch Chromatographie (Kieselgel CH2Cl2/MeOH/AcOH =10:1:0.1) gereinigt. Man erhält einen weißen Feststoff in 89% Ausbeute.
Schmelzpunkt: 282 °C
R_{f}= 0,33 (Kieselgel; Methylenchlorid: Methanol = 4:1)
HPLC/MS (Methode G): RT = 3,02 min.; [M+H]+ = 384; Abs. λ max = 238 nm
1H-NMR(D₆-DMSO, 300MHz) δ: 1.81 (s, 3 H), 3.32 (m, 2 H), 3.55 (m, 2 H), 7.22 (m, 1 H), 7.42 (m, 1 H), 7.56 (m, 1 H), 7.90 (m, 2 H), 8.22 (s, 1 H), 8.52 (s, 1 H), 8.85 (s, 1 H).

Analog Beispiel 9 wurde folgende Verbindung erhalten:
(1) 4-[4-(2-Acetylamino-ethylamino)-5-trifluormethyl-pyrimidin-2-ylamino]-benzoesäure
   Smp: 260°C
   R_{f}= 0.33 (Kieselgel; Methylenchlorid:Methanol:konz.Ammoniak = 4 : 1 : 0.25)
   Rt (HPLC, Methode E) = 3.21 min.
   1H-NMR(D₆-DMSO, 300MHz) δ: 1.82 (s, 3 H), 3.33 (m, 2 H), 3.54 (m, 2 H), 7.29 (m, 1 H), 7.88 (m, 5 H), 8.00 (m, 1 H), 8.25 (s, 1 H), 10.00 (s, 1 H).

### Beispiel 10

### N-(2-[2-(3-Acetylamino-phenylamino)-5-trifluormethyl-pyrimidin-4-ylamino]-ethyl)-acetamid

100 mg N-(2-[2-(3-Amino-phenylamino)-5-trifluormethyl-pyrimidin-4-ylamino]-ethyl)-acetamid [2(67)] werden bei 0°C in 1 ml Pyridin und 1 ml Dichlormethan mit Acetylchlorid (1.1 eq.) in 0.5 ml Dichlormethan versetzt, auf Raumtemperatur erwärmt und über Nacht gerührt. Die Reaktionsmischung wird mit 2 ml ges. Bicarbonatlösung versetzt und mit Essigsäureethylester extrahiert. Die org. Phasen werden über Natriumsulfat getrocknet und eingeengt. Das Rohprodukt wird mit Essigsäureethylester, Diethylether und Dichlormethan gewaschen und gegebenenfalls mittels Chromatographie (Kieselgel, CH2Cl2/MeOH-Gradient) gereinigt. Man erhält 102 mg eines Feststoffs.
Schmelzpunkt: 220 °C
R_{f}= 0,11 (Kieselgel; Methylenchlorid: Methanol = 9:1)
HPLC/MS (Methode F): RT = 3,12 min.; [M+H]+= 397; Abs. λ max = 245,6 nm
(1) Pyridin-2-carbonsäure (3-[4-(2-acetylamino-ethylamino)-5-trifluormethyl-pyrimidin-2-ylamino]-phenyl)-amid
   100 mg N-(2-[2-(3-Amino-phenylamino)-5-trifluormethyl-pyrimidin-4-ylamino]-ethyl)-acetamid [2(67)]werden mit 2-Pyridincarbonsäure (1.2 eq.), HOBT (1.2 eq.), HBTU (1.2 eq.) und Diisopropylethylamin (2 eq.) in 2 ml DMFüber Nacht bei Raumtemperatur gerührt. Die Reaktionsmischung wird mit 2 ml ges. Bicarbonatlösung versetzt und mit Essigsäureethylester extrahiert. Die org. Phasen werden über Natriumsulfat getrocknet und eingeengt. Das Rohprodukt wird mit Essigsäureethylester, Diethylether und Dichlormethan gewaschen und gegebenenfalls mittels Chromatographie (Kieselgel, CH₂Cl₂/MeOH-Gradient) gereinigt. Man erhält 109 mg eines Feststoffs.
   Schmelzpunkt: 184 °C
   R_{f}= 0,24 (Kieselgel; Methylenchlorid: Methanol = 9:1)
   HPLC/MS (Methode F): RT = 3,69 min.; [M+H]+ = 460; Abs. λ max = 262,7 nm
   Analog Beispiel 10 oder 10 (1) werden folgende Verbindungen erhalten:
(2) N-{4-[4-(2-Acetylamino-ethylamino)-5-trifluormethyl-pyrimidin-2-ylamino]-phenyl}-3,5-difluor-N-methyl-benzamid
   Herstellung ausgehend von Verbindung 2 (130).
   Schmelzpunkt: 103-106°C
   R_{f}= 0,45 (Kieselgel; Methylenchlorid: Methanol = 9:1)
   HPLC/MS (Methode F): RT = 3,83 min.; [M+H]+ = 509; Abs. λ max = 261 nm
(3) N-(2-[2-(4-Acetylamino-phenylamino)-5-trifluormethyl-pyrimidin-4-ylamino]-ethyl)-acetamid
   Herstellung ausgehend von Verbindung 2 (4).
   Schmelzpunkt: 222°C
   R_{f}= 0,65 (Kieselgel; Methylenchlorid: Methanol = 4:1)
   HPLC/MS (Methode F): RT = 3,05 min.; [M+H]+ = 397 ; Abs. λ max = 266,5 nm
(4) N-(3-[4-(2-Acetylamino-ethylamino)-5-trifluormethyl-pyrimidin-2-ylamino]-phenyl)-benzamid
   Herstellung ausgehend von Verbindung 2 (67).
   Schmelzpunkt: 215 °C
   R_{f}= 0,29 (Kieselgel; Methylenchlorid: Methanol = 9:1)
   HPLC/MS (Methode F): RT = 3,67 min.; [M+H]+= 459; Abs. λ max = 257 nm
(5) N-(4-[4-(2-Acetylamino-ethylamino)-5-trifluormethyl-pyrimidin-2-ylamino]-phenyl)-benzamid
   Herstellung ausgehend von Verbindung 2 (4).
   Schmelzpunkt: 252 °C
   R_{f}= 0,17 (Kieselgel; Methylenchlorid: Methanol = 9:1)
   HPLC/MS (Methode F): RT = 3,59 min.; [M+H]+= 459 ; Abs. λ max = 219 nm
(6) Pyridin-2-carbonsäure (4-[4-(2-acetylamino-ethylamino)-5-trifluormethyl-pyrimidin-2-ylamino]-phenyl)-amid
   Herstellung ausgehend von Verbindung 2 (4).
   Schmelzpunkt: 215°C
   R_{f}= 0,22 (Kieselgel; Methylenchlorid: Methanol = 9:1)
   HPLC/MS (Methode F): RT = 3,61min.; [M+H]+ = 460; Abs. λ max = 220,9 nm
(7) Pyridin-2-carbonsäure {4-[4-(2-acetylamino-ethylamino)-5-trifluormethyl-pyrimidin-2-ylamino]-phenyl}-methyl-amid
   Herstellung ausgehend von Verbindung 2 (130).
   Schmelzpunkt: 144-145 °C
   R_{f}= 0,43 (Kieselgel; Methylenchlorid: Methanol = 9:1)
   HPLC/MS (Methode F): RT = 3,32 min.; [M+H]+ = 474; Abs. λ max = 263 nm
(8) N-(2-{2-[4-(Phenylsulfonyl-methyl-amino)-phenylamino]-5-trifluormethyl-pyrimidin-4-ylamino}-ethyl)-acetamid
   Herstellung ausgehend von Verbindung 2 (130).
   Schmelzpunkt: 162-164°C
   R_{f}= 0,56 (Kieselgel; Methylenchlorid: Methanol = 9:1)
   HPLC/MS (Methode F): RT = 3,99 min.; [M+H]+ = 509; Abs. λ max = 267 nm
(9) N-{3-[4-(2-Acetylamino-ethylamino)-5-trifluormethyl-pyrimidin-2-ylamino]-phenyl}-3,5-difluor-N-methyl-benzamid
   Herstellung ausgehend von Verbindung 2 (131).
   Schmelzpunkt: 193-195 °C
   R_{f}= 0,43 (Kieselgel; Methylenchlorid: Methanol = 9:1)
   HPLC/MS (Methode H): RT = 3,31 min.; [M+H]+ = 509; Abs. λ max = 253 nm
(10) Pyridin-2-carbonsäure {3-[4-(2-acetylamino-ethylamino)-5-trifluormethyl-pyrimidin-2-ylamino]-phenyl}-methyl-amid
   Herstellung ausgehend von Verbindung 2 (131).
   Schmelzpunkt: 188-190 °C
   R_{f}= 0,49 (Kieselgel; Methylenchlorid: Methanol = 9:1)
   HPLC/MS (Methode G): RT = 3,16 min.; [M+H]+ = 474; Abs. λ max = 253 nm
(11) N-(2-{2-[3-(Phenylsulfonyl-methyl-amino)-phenylamino]-5-trifluormethyl-pyrimidin-4-ylamino}-ethyl)-acetamid
   Herstellung ausgehend von Verbindung 2 (131).
   Schmelzpunkt: 117-120 °C
   R_{f}= 0,62 (Kieselgel; Methylenchlorid: Methanol = 9:1)
   HPLC/MS (Methode F): RT = 3,97 min.; [M+H]+ = ; 509 Abs. λ max = 234 nm
(12) N-(4-[4-(2-Acetylamino-ethylamino)-5-trifluormethyl-pyrimidin-2-ylamino]-phenyl)-3,5-difluor-benzamid
   Herstellung ausgehend von Verbindung 2 (4).
   Schmelzpunkt: 277°C
   R_{f}= 0,18 (Kieselgel; Methylenchlorid: Methanol = 9:1)
   HPLC/MS (Methode G): RT = 3,74 min.; [M+H]+ = 495 ; Abs. λ max = 219 nm
(13) N-(2-[2-(3-Methansulfonylamido-phenylamino)-5-trifluormethyl-pyrimidin-4-ylamino]-ethyl)-acetamid
   Herstellung ausgehend von Verbindung 2 (67).
   Schmelzpunkt: 188 °C
   R_{f} = 0,15 (Kieselgel; Methylenchlorid: Methanol = 9:1)
   HPLC/MS (Methode F): RT = 3,22 min.; [M+H]+ = 433; Abs. λ max = 243,7 nm
(14) N-(2-[2-(4-Methansulfonylamido-phenylamino)-5-trifluormethyl-pyrimidin-4-ylamino]-ethyl)-acetamid
   Herstellung ausgehend von Verbindung 2 (4).
   Schmelzpunkt: 237 °C
   R_{f} = 0,17 (Kieselgel; Methylenchlorid: Methanol = 9:1)
   HPLC/MS (Methode F): RT = 3,14 min.; [M+H]+ = 433; Abs. λ max = 263 nm
(15) N-(2-[2-(3-Phenylfonylamido-phenylamino)-5-trifluormethyl-pyrimidin-4-ylamino]-ethyl)-acetamid
   Herstellung ausgehend von Verbindung 2 (67).
   Schmelzpunkt: 208°C
   R_{f}= 0,20 (Kieselgel; Methylenchlorid: Methanol = 9:1)
   HPLC/MS (Methode F): RT = 3,75 min.; [M+H]+= 495; Abs. λ max = 236,1 nm
(16) N-(2-[2-(4-Phenylsulfonylamido-phenylamino)-5-trifluormethyl-pyrimidin-4-ylamino]-ethyl)-acetamid
   Herstellung ausgehend von Verbindung 2 (4).
   Schmelzpunkt: 242 °C
   R_{f}= 0,22 (Kieselgel; Methylenchlorid: Methanol = 9: 1)
   HPLC/MS (Methode F): RT = 3.58 min.; [M+H]+ = 495; Abs. λ max = 264,6 nm
(17) N-(3-[4-(2-Acetylamino-ethylamino)-5-trifluormethyl-pyrimidin-2-ylamino]-phenyl)-3,5-difluor-benzamid
   Herstellung ausgehend von Verbindung 2 (67).
   Schmelzpunkt: 231 °C
   R_{f}= 0,24 (Kieselgel; Methylenchlorid: Methanol = 9: 1)
   HPLC/MS (Methode F): RT = 4,00 min.; [M+H]+ = 495; Abs. λ max = 262,7 nm

### Beispiel 11

### N-{2-[2-(3-Piperidin-1-ylmethyl-phenylamino)-5-trifluormethyl-pyrimidin-4-ylamino]-ethyl}-acetamid

80 mg N-{2-[2-(3-Formyl-phenylamino)-5-trifluormethyl-pyrimidin-4-ylamino]-ethyl}-acetamid, 1.1 Äq. Piperidin and 2 Äq. NaBH(OAc)3 werden in 2 ml THF gelöst und der Ansatz über Nacht bei Raumtemperatur gerührt. Die Reaktion bricht man durch Zugabe von 2 ml gesättigter wässriger Natriumcarbonatlösung ab und extrahiert zweimal mit je 10 ml Methylenchlorid. Die organische Phase wird mit Wasser gewaschen, über Natriumsulfat getrocknet und zur Trockne eingeengt. Das Rohprodukt reinigt man über Kieselgel mit Methylenchlorid/Methanol. Schmelzpunkt: 154-155°C
R_{f}= 0,11 (Kieselgel; Methylenchlorid: Methanol = 9:1)
HPLC/MS (Methode F): RT = 3,35min.; [M+H]+ = 437; Abs. λ max = 253 nm

Analog zu Beispiel 11 werden folgende Verbindungen erhalten:
(1) N-(2-{2-[3-(3-Oxo-piperazin-1-ylmethyl)-phenylamino]-5-trifluormethyl-pyrimidin-4-ylamino}-ethyl)-acetamid
   Schmelzpunkt: 196-198 °C
   R_{f}= 0,23 (Kieselgel; Methylenchlorid: Methanol = 9:1)
   HPLC/MS (Methode F): RT = 3,14min.; [M+H]+ = 452; Abs. λ max = 255 nm
(2) N-{2-[2-(3-Pyrrolidin-1-ylmethyl-phenylamino)-5-trifluormethyl-pyrimidin-4-ylamino]-ethyl}-acetamid
   Schmelzpunkt: 172-173°C
   R_{f}= 0,16 (Kieselgel; Methylenchlorid: Methanol = 85:15)
   HPLC/MS (Methode F): RT = 3,25min.; [M+H]+ = 423; Abs. λ max = 255 nm
(3) N-{2-[2-(3-Dimethylaminomethyl-phenylamino)-5-trifluormethyl-pyrimidin-4-ylamino]-ethyl}-acetamid
   Schmelzpunkt: 140-141 °C
   R_{f} = 0,21 (Kieselgel; Methylenchlorid: Methanol = 85:15)
   HPLC/MS (Methode F): RT = 3,22 min.; [M+H]+ = 397; Abs. λ max = 253 nm
(4) 1-{3-[2-(3-Dimethylaminomethyl-phenylamino)-5-trifluormethyl-pyrimidin-4-ylamino]-propyl}-pyrrolidin-2-on
   Schmelzpunkt: 126-128 °C
   R_{f}= 0,19 (Kieselgel; Methylenchlorid: Methanol = 9:1)
   HPLC/MS (Methode F): RT = 3,43 min.; [M+H]+= 437; Abs. λ max = 253 nm
(5) 1-{3-[2-(3-Pyrrolidin-1-ylmethyl-phenylamino)-5-trifluormethyl-pyrimidin-4-ylamino]-propyl}-pyrrolidin-2-on
   Schmelzpunkt: 122-124°C
   R_{f}= 0,14 (Kieselgel; Methylenchlorid: Methanol = 9:1)
   HPLC/MS (Methode F): RT = 3,5 min.; [M+H]+= 463; Abs. λ max = 253 nm
(6) 1-{3-[2-(3-Piperidin-1-ylmethyl-phenylamino)-5-trifluormethyl-pyrimidin-4-ylamino]-propyl}-pyrrolidin-2-on
   Schmelzpunkt: 130-132°C
   R_{f} = 0,23 (Kieselgel; Methylenchlorid: Methanol = 9:1)
   HPLC/MS (Methode F): RT = 3,52 min.; [M+H]+ = 477; Abs. λ max = 253 nm
(7) 1-{3-[2-(4-Dimethylaminomethyl-phenylamino)-5-trifluormethyl-pyrimidin-4-ylamino]-propyl}-pyrrolidin-2-on
   Schmelzpunkt: 93-95 °C
   R_{f}= 0,09 (Kieselgel; Methylenchlorid: Methanol = 9:1)
   HPLC/MS (Methode G): RT = 3,03 min.; [M+H]+ = 437; Abs. λ max = 259 nm
(8) N-[2-(2-{4-[(Isobutyl-methyl-amino)-methyl]-phenylaminol-5-trifluormethyl-pyrimidin-4-ylamino)-ethyl]-acetamid
   Schmelzpunkt: 162-163 °C
   R_{f}= 0,38 (Kieselgel; Methylenchlorid: Methanol = 85:15)
   HPLC/MS (Methode G): RT = 3,11 min.; [M+H]+ = 439; Abs. λ max = 255 nm
(9) N-{2-[2-(4-Pyrrolidin-1-ylmethyl-phenylamino)-5-trifluormethyl-pyrimidin-4-ylamino]-ethyl}-acetamid
   Schmelzpunkt: 170-173 °C
   R_{f}= 0,18 (Kieselgel; Methylenchlorid: Methanol = 85:15)
   HPLC/MS (Methode G): RT = 2,83 min.; [M+H]+ = 423; Abs. λ max = 261 nm
(10) 1-{3-[2-(4-Pyrrolidin-1-ylmethyl-phenylamino)-5-trifluormethyl-pyrimidin-4-ylamino]-propyl}-pyrrolidin-2-on
   Schmelzpunkt: 122-125 °C
   R_{f}= 0,23 (Kieselgel; Methylenchlorid: Methanol = 85:15)
   HPLC/MS (Methode G): RT = 3,11 min.; [M+H]+ = 463; Abs. λ max = 263 nm
(11) 1-{3-[2-(4-Morpholin-4-ylmethyl-phenylamino)-5-trifluormethyl-pyrimidin-4-ylamino]-propyl}-pyrrolidin-2-on
   Schmelzpunkt: 128-130 °C
   R_{f} = 0,51 (Kieselgel; Methylenchlorid: Methanol = 9: 1)
   HPLC/MS (Methode G): RT = 3,02 min.; [M+H]+ = 479; Abs. λ max = 259 nm
(12) 1-(3-{2-[4-(3,5-Dimethyl-piperazin-1-ylmethyl)-phenylamino]-5-trifluormethyl-pyrimidin-4-ylamino}-propyl)-pyrrolidin-2-on
   Schmelzpunkt: 83-85°C
   R_{f}= 0,11 (Kieselgel; Methylenchlorid: Methanol = 9:1)
   HPLC/MS (Methode G): RT = 2,95 min.; [M+H]+ = 506; Abs. λ max = 265 nm
(13) N-[2-(2-{3-[(Diisopropylamino)-methyl]-phenylamino}-5-trifluormethyl-pyrimidin-4-ylamino)-ethyl]-acetamid
   Schmelzpunkt: 60-63°C
   R_{f}= 0,21 (Kieselgel; Methylenchlorid: Methanol = 9:1)
   HPLC/MS (Methode F): RT = 3,03 min.; [M+H]+ = 453; Abs. λ max = 253 nm
(14) 1-(3-{2-[4-(4-Acetyl-piperazin-1-ylmethyl)-phenylamino]-5-trifluormethyl-pyrimidin-4-ylamino}-propyl)-pyrrolidin-2-on
   Schmelzpunkt: 163 -163°C
   R_{f}= 0,46 (Kieselgel; Methylenchlorid: Methanol = 9:1)
   HPLC/MS (Methode F): RT = 3,00 min.; [M+H]+ = 520; Abs. λ max = 263 nm
(15) N-{2-[2-(4-Methylaminomethyl-phenylamino)-5-trifluormethyl-pyrimidin-4-ylamino]-ethyl}-acetamid
   Schmelzpunkt: 130-132°C
   R_{f}= 0,05 (Kieselgel; Methylenchlorid: Methanol = 8:2)
   HPLC/MS (Methode F): RT = 2,8 min.; [M+H]+ = 383; Abs. λ max = 261 nm
(16) 1-[3-(2-{3-[(Diisopropylamino)-methyl]-phenylamino}-5-trifluormethyl-pyrimidin-4-ylamino)-propyl]-pyrrolidin-2-on
   Schmelzpunkt: 141-143 °C
   R_{f}= 0,37 (Kieselgel; Methylenchlorid: Methanol = 9:1)
   HPLC/MS (Methode F): RT = 3,24 min.; [M+H]+ = 493; Abs. λ max = 249 nm
(17) 1-{3-[2-(3-Methylaminomethyl-phenylamino)-5-trifluormethyl-pyrimidin-4-ylamino]-propyl}-pyrrolidin-2-on
   Schmelzpunkt: 65-68 °C
   HPLC/MS (Methode F): RT = 2,98 min.; [M+H]+= 423; Abs. λ max = 251 nm
(18) N-[2-(2-{4-[(Diisopropylamino)-methyl]-phenylamino}-5-trifluormethyl-pyrimidin-4-ylamino)-ethyl]-acetamid
   Schmelzpunkt: 140-143°C
   R_{f}= 0,26 (Kieselgel; Methylenchlorid: Methanol = 85:15)
   HPLC/MS (Methode F): RT = 3,04 min.; [M+H]+ = 453; Abs. λ max = 251 nm
(19) N-{2-[2-(3-Methylaminomethyl-phenylamino)-5-trifluormethyl-pyrimidin-4-ylamino]-ethyl}-acetamid
   Schmelzpunkt: 137-140°C
   R_{f}= 0,09 (Kieselgel; Methylenchlorid: Methanol = 9:1)
   HPLC/MS (Methode F): RT = 2,77 min.; [M+H]+ =383; Abs. λ max = 253 nm
(20) 1-[3-(2-{4-[(Isobutyl-methyl-amino)-methyl]-phenylamino}-5-trifluormethyl-pyrimidin-4-ylamino)-propyl]-pyrrolidin-2-on
   Schmelzpunkt: 110-113 °C
   R_{f}= 0,36 (Kieselgel; Methylenchlorid: Methanol = 9:1)
   HPLC/MS (Methode G): RT = 3,31 min.; [M+H]+ = 479; Abs. λ max = 265 nm
(21) N-{2-[2-(4-Azepan-1-ylmethyl-phenylamino)-5-trifluormethyl-pyrimidin-4-ylamino]-ethyl}-acetamid
   Schmelzpunkt: 170-172°C
   R_{f}= 0,11 (Kieselgel; Methylenchlorid: Methanol = 85:15)
   HPLC/MS (Methode G): RT = 3,07 min.; [M+H]+ = 451; Abs. λ max = 263 nm
(22) 1-{3-[2-(4-Azepan-1-ylmethyl-phenylamino)-5-trifluormethyl-pyrimidin-4-ylamino]-propyl}-pyrrolidin-2-on
   Schmelzpunkt: 142-145°C
   R_{f}= 0,19 (Kieselgel; Methylenchlorid: Methanol = 85:15)
   HPLC/MS (Methode G): RT = 3,31 min.; [M+H]+ = 491; Abs. λ max = 265 nm
(23) N-(2-{2-[4-(Isobutylamino-methyl)-phenylamino]-5-trifluormethyl-pyrimidin-4-ylamino}-ethyl)-acetamid
   Schmelzpunkt: 163-165°C
   R_{f}= 0,13 (Kieselgel; Methylenchlorid: Methanol = 9: 1)
   HPLC/MS (Methode G): RT = 3,06min.; [M+H]+ = ; 425 Abs. λ max = 261nm
(24) 1-{3-[5-Methyl-2-(4-piperidin-1-ylmethyl-phenylamino)-pyrimidin-4-ylamino]-propyl}-pyrrolidin-2-on
   Schmelzpunkt: 144-145 °C
   R_{f}= 0,10 (Kieselgel; Methylenchlorid: Methanol = 8:2)
   HPLC/MS (Methode F): RT = 2,12 min.; [M+H]+ = 423; Abs. λ max = 263 nm
(25) 1-(3-{2-[4-(Isobutylamino-methyl)-phenylamino]-5-trifluormethyl-pyrimidin-4-ylamino}-propyl)-pyrrolidin-2-on
   Schmelzpunkt: 112-114°C
   R_{f}= 0,21 (Kieselgel; Methylenchlorid: Methanol = 9: 1)
   HPLC/MS (Methode G): RT = 3,28 min.; [M+H]+ = 465; Abs. λ max = 261 nm
(26) N-(2-{2-[4-(3,5-Dimethyl-piperazin-1-ylmethyl)-phenylamino]-5-trifluormethyl-pyrimidin-4-ylamino}-ethyl)-acetamid
   Schmelzpunkt: 186-189°C
   R_{f}= 0,10 (Kieselgel; Methylenchlorid: Methanol = 85:15)
   HPLC/MS (Methode F): RT = 3 min.; [M+H]+ = 466; Abs. λ max = 261 nm
(27) N-{2-[5-Chloro-2-(4-piperidin-1-ylmethyl-phenylamino)-pyrimidin-4-ylamino]-ethyl}-acetamid
   Schmelzpunkt: 162-164°C
   R_{f}= 0,25 (Kieselgel; Methylenchlorid: Methanol = 4:1)
   HPLC/MS (Methode F): RT = 2,88 min.; [M+H]+ =; 403 Abs. λ max = 267 nm
(28) N-(2-{2-[4-(4-Acetyl-piperazin-1-ylmethyl)-phenylamino]-5-trifluormethyl-pyrimidin-4-ylamino}-ethyl)-acetamid
   Schmelzpunkt: 80-83 °C
   R_{f} = 0,31 (Kieselgel; Methylenchlorid: Methanol = 9: 1)
   HPLC/MS (Methode F): RT = 3,01 min.; [M+H]+ = 480; Abs. λ max = 257 nm
(29) 1-{3-[2-(4-Methylaminomethyl-phenylamino)-5-trifluormethyl-pyrimidin-4-ylamino]-propyl}-pyrrolidin-2-on
   HPLC/MS (Methode F): RT = 2,28 min.; [M+H]+ = 423; Abs. λ max = 261 nm
(30) 1-(3-{2-[4-(2,5-Dimethyl-pyrrolidin-1-ylmethyl)-phenylamino]-5-trifluormethyl-pyrimidin-4-ylamino}-propyl)-pyrrolidin-2-on
   Schmelzpunkt: 131-134 °C
   R_{f}= 0,18 (Kieselgel; Methylenchlorid: Methanol = 9:1)
   HPLC/MS (Methode G): RT = 3,09 min.; [M+H]+ = 491; Abs. λ max = 263 nm
(31) 1-{3-[2-(3-Morpholin-4-ylmethyl-phenylamino)-5-trifluormethyl-pyrimidin-4-ylamino]-propyl}-pyrrolidin-2-on
   Schmelzpunkt: 132-135 °C
   R_{f}= 0,47 (Kieselgel; Methylenchlorid: Methanol = 9:1)
   HPLC/MS (Methode G): RT = 2,88 min.; [M+H]+ = 479; Abs. λ max = 253 nm
(32) 1-[3-(2-{4-[(Diisopropylamino)-methyl]-phenylamino}-5-trifluormethyl-pyrimidin-4-ylamino)-propyl]-pyrrolidin-2-on
   Schmelzpunkt: 121 °C
   R_{f}= 0,31 (Kieselgel; Methylenchlorid: Methanol = 9:1)
   HPLC/MS (Methode F): RT = 3,42 min.; [M+H]+ = 493; Abs. λ max = 263 nm
(33) N-{2-[2-(3-Morpholin-4-ylmethyl-phenylamino)-5-trifluormethyl-pyrimidin-4-ylamino]-ethyl}-acetamid
   Schmelzpunkt: 169-170°C
   R_{f}= 0,443 (Kieselgel; Methylenchlorid: Methanol = 9:1)
   HPLC/MS (Methode F): RT = 2,53 min.; [M+H]+ = 439; Abs. λ max = 255 nm
(34) 1-[3-(2-{3-[(2,2,2-Trifluor-ethylamino)-methyl]-phenylamino}-5-trifluoromethyl-pyrimidin-4-ylamino)-propyl]-pyrrolidin-2-on
   Schmelzpunkt: 93-94°C
   R_{f} = 0,61 (Kieselgel; Methylenchlorid: Methanol = 9:1)
   HPLC/MS (Methode E): RT = 2,92 min.; [M+H]+= 491; Abs. λ max = 253 nm
(35) N-[2-(2-{3-[(2,2,2-Trifluor-ethylamino)-methyl]-phenylamino}-5-trifluoromethyl-pyrimidin-4-ylamino)-ethyl]-acetamid
   Schmelzpunkt: 139-144°C
   Rf = 0,44 (Kieselgel; Methylenchlorid: Methanol = 9:1)
   HPLC/MS (Methode E): RT = 3,11 min.; [M+H]+= 451; Abs. λ max = 255 nm
(36) 1-[3-(2-{4-[(2,2,2-Trifluor-ethylamino)-methyl]-phenylamino}-5-trifluoromethyl-pyrimidin-4-ylamino)-propyl]-pyrrolidin-2-on
   Schmelzpunkt: 92-98°C
   Rf = 0,61 (Kieselgel; Methylenchlorid: Methanol = 9:1)
   HPLC/MS (Methode E): RT = 3,23 min.; [M+H]+ = 491; Abs. λ max = 251 nm
(37) N-[2-(2-{4-[(2,2,2-Trifluor-ethylamino)-methyl]-phenylamino}-5-trifluoromethyl-pyrimidin-4-ylamino)-ethyl]-acetamid
   Schmelzpunkt: 156-165°C
   Rf = 0,45 (Kieselgel; Methylenchlorid: Methanol = 9:1)
   HPLC/MS (Methode E): RT = 2,92 min.; [M+H]+ = 451; Abs. λ max = 257 nm

### Beispiel 12

### Herstellung von rekombinanten Cyclin-CDK Enzymen

Die entsprechenden cDNAs für humanes Cyclin B1 (Cyclin E, bzw. Cyclin D1) und humanes CDK1 (CDK2, bzw. CDK4) wurden mittels RT-PCR nach Standardmethoden kloniert, und in einen Transfervektor (Cycline in pAcG2T von Pharmingen, CDKs in p2Bac von Invitrogen) für das Baculovirussystem kloniert. Rekombinantes Cyclin B1-CDK1 (bzw. Cyclin E-CDK2, Cyclin D1-CDK4) wurde in High Five Insektenzellen (Trichoplusia ni) durch Coinfektion mit beiden rekombinanten Baculoviren (nach 4. Amplifikationsrunde, >1 x 10⁸ Viren/ml) exprimiert. 72 Stunden nach der Infektion wurden die High Five Zellen geerntet, und in flüssigem Stickstoff tiefgefroren. Nach dem Auftauen wurden die Zellen in Lysepuffer (50 mM HEPES pH 7.5, 10 mM MgCl₂, 1 mM DTT, 5 µg/ml Leupeptin, 5 µg/ml Aprotinin, 100 µM NaF, 100 µM PMSF, 10 mM β-Glycerolphosphat, 100 µM Na₃VO₄, 30 mM Nitrophenylphosphat, 17.5 ml Lysepuffer pro 10⁸ Zellen) resuspendiert und 30 min auf Eis inkubiert. Das Zell-Lysat wurde durch Zentrifugation von der Zelldebris befreit und die Menge an rekombinantem Cyclin B1-CDK1 Enzym (bzw. Cyclin E-CDK2, Cyclin D1-CDK4) im Gesamtlysat (ca. 1-5 mg/ml) wurde durch SDS-Polyacrylamid Gelelektrophorese bestimmt. Cyclin D1-CDK4 wurde über einen GST-tag am Cyclin D1 anschließend über Glutathion Beads gereinigt (Gesamtprotein ca. 0.2 mg/ml).

### Beispiel 13

### Cyclin B1-CDK1 Kinase Inhibitionstest

Alle Kinasetests wurden in 96-well Mikrotiterplatten (Greiner PS ) in einem Endvolumen von 60 µl durchgeführt. Der Kinasetest enthielt 1 % DMSO (v/v), 5 µg Histon H1 (Kalbsthymus, Roche Molecular Biochemicals), 1 bis 5 µg eines Zell-Lysates mit rekombinantem Cyclin B1/CDK1, die Testsubstanz (von 1 nM bis 10 µM Endkonzentration) und Kinasepuffer (15 mM MgCl₂, 25 mM MOPS, pH 7.0, 0.1 mM DTT). Als negativ Kontrolle wurde die Kinasereaktion in Abwesenheit des Substrates Histon H1 durchgeführt. Als Positivkontrolle wurde die Kinasereaktion in Abwesenheit einer Testsubstanz durchgeführt. Als interne Kontrolle wurden 30 µM und 300 µM (Endkonzentration) des Kinaseinhibitors Olomoucin (Alexis) eingesetzt.

Die PS Mikrotiterplatten wurden auf Eis gestellt, und nacheinander wurden 10 µl der Testsubstanz in verschiedenen Konzentrationen (jeweils in 6% DMSO), 20 µl des Histon H1 (250 µg/ml in Kinasepuffer ), 20 µl Cyclin B1/CDK1 (1 bis 5 µg des rekombinanten Zell-Lysates in 20 µl Kinasepuffer) zupipettiert und gemischt. Die Kinasereaktion wird gestartet durch die Zugabe von 10 µl ATP-Mix (0.045 mM ATP, 0.5 µCi ³³P-γATP in Kinase Puffer) und für 30 min bei 30 °C und 600 rpm in einem Schüttelinkubator inkubiert. Nach der Inkubation wurden die Platten auf Eis gestellt und die Proteine durch Zugabe von 125 µl eiskalter 5% Tricloressigsäwe präzipitiert. Nach 15 min auf Eis wurden die Präzipitate auf Packard Unifilter 96 GF/B Platten mit dem Packard Harvester System transferiert, und durch Vakuumfiltration gesammelt. Die Präzipitate wurden 4 mal mit dest. H₂O bei Raumtemperatur gewaschen. Die Filterplatten wurden anschließend bei 60°C getrocknet und mit 50 µl Szintillationsflüssigkeit pro well versehen (Ultima Gold, Packard). Die Platte wurde mit Sealing Tape verschlossen und nach 1 Stunden in einem Szintillationsmeßgerät (Micro Beta von Wallac) gemessen.

Die Inhibition der Substanzen wurde in Prozent der Kontrolle (Cyclin B 1-CDK1 ohne Inhibitor) berechnet und die Wirkstoffkonzentration, die die Enzymaktivität zu 50% hemmt (IC50) abgeleitet.

### Beispiel 14

### Cyclin E-CDK2 Kinase Inhibitionstest

Der Inhibitionstest mit Cyclin E-CDK2 wurde nach dem gleichen Protokoll wie für Cyclin B 1-CDK1 durchgeführt, nur das als Enzym rekombinantes Cyclin E-CDK2 verwendet wurde.

### Beispiel 15

### Cyclin D1-CDK4 Kinase Inhibitionstest

Für den Inhibitionstest mit Cyclin D1-CDK4 wurde rekombinantes Retinoblastoma Protein (pRB) von aa379-928, das am N-terminus einen GST-tag enthält, als Substrat verwendet. GST-pRB wurde in Bakterien exprimiert und anschließend über Glutathion Beads gereinigt ( ca. 0.2 mg/ml). Der Kinasetest enthielt 1% DMSO (v/v), 10 µg pRB, 0.4 µg eines Zell-Lysates mit rekombinantem Cyclin D1-CDK4, die Testsubstanz (von 1 nM bis 10 µM Endkonzentration) und Kinasepuffer (15 mM MgCl₂, 25 mM MOPS, pH 7.0, 0.1 mM DTT). Als negativ Kontrolle wurde die Kinasereaktion in Abwesenheit des Substrates pRB durchgerührt. Als Positivkontrolle wurde die Kinasereaktion in Abwesenheit einer Testsubstanz durchgeführt. Als interne Kontrolle wurden 30 µM und 300 µM (Endkonzentration) des Kinaseinhibitors Olomoucin (Alexis) eingesetzt. Die PS Mikrotiterplatten wurden auf Eis gestellt, und nacheinander wurden 10 µl der Testsubstanz in verschiedenen Konzentrationen (jeweils in 6% DMSO), 20 µl pRB (10 µg in Kinasepuffer ), 20 µl Cyclin D1-CDK4 (0.4 µg des rekombinanten Zell-Lysates in 20 µl Kinasepuffer) zupipettiert und gemischt. Die Kinasereaktion wurde durch die Zugabe von 10 µl ATP-Mix (0.045 mM ATP, 1 µCi 33P-□ATP in Kinase Puffer) gestartet und für 45 min bei 32 °C und 600 rpm in einem Schüttelinkubator inkubiert. Nach der Inkubation wurden 50 µl des Reaktionsansatzes auf P81 Filter (Whatmann) pipettiert. Nach 20 sec Einwirkzeit wurden die Filter 4 mal mit 1.5% Phosphorsäure gewaschen (ca. 5min pro Waschschritt) und dabei leicht geschüttelt. Nach dem Waschen wurden die Filter bei 85°C getrocknet, mit Szintillationsflüssigkeit versehen und in einem Szintillationszähler (Micro Beta von Wallac) gemessen.

### Beispiel 16

### Messung der Cytotoxizität an kultivierten humanen Tumorzellen

Zellen der der nicht-kleinzelligen Lungen Tumorzell-Linie NCI H-460 (erhalten von American Type Culture Collection (ATCC)) wurden in RPMI1640 Medium (Gibco) und 10% fötalem Rinderserum (Gibco) kultiviert und in der log-Wachstumsphase geerntet. Anschließend wurden die NCI H-460 Zellen in 96-well Platten (Costar) mit einer Dichte von 1000 Zellen pro well eingebracht und über Nacht in einem Inkubator (bei 37°C und 5 % CO₂) inkubiert, wobei auf jeder Platte 6 wells nur mit Medium gefüllt wurden (3 wells zur Mediumkontrolle, 3 wells zur Inkubation mit reduzierten AlamarBlue). Die Wirksubstanzen wurden in verschiedenen Konzentrationen (gelöst in DMSO; Endkonzentration: 1%) zu den Zellen zugegeben (jeweils als Dreifachbestimmung) Nach 72 Stunden Inkubation wurden zu jeden well 20 µl AlamarBlue (AccuMed International) zugesetzt, und die Zellen für weitere 5 Stunden inkubiert. Zur Kontrolle wurde zu 3 wells je 20 µl reduziertes Alamar Blue gegeben (AlamarBlue Reagenz, das für 30 min autoklaviert wurde). Nach 5 Stunden Inkubation wurde der Farbumsatz des AlamarBlue Reagenz in den einzelnen wells in einem Perkin Elmer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wurde in Prozent der Kontrolle (NCI H-460 Zellen ohne Inhibitor) berechnet und die Wirkstoffkonzentration, die die Zellaktivität zu 50% hemmt (IC50) abgeleitet. Die Werte wurden hierbei aus dem Mittelwert von drei Einzelbestimmungen - unter Korrektur des Leerwertes (Mediumkontrolle)- berechnet.

### verwendete Abkürzungen:

| | |
|---|---|
| ATP | Adenosintriphosphat |
| Ci | Curie |
| DTT | 1,4-Dithiothreit |
| DMSO | Dimethylsulfoxid |
| GST | Glutathion-S-Transferase |
| HEPES | N-2-Hydroxyethylpiperazin-N'-2'-Ethansulfonsäure |
| MOPS | 3-(N-Morpholino)-propansulfonsäure |
| NaF | Natriumfluorid |
| PMSF | Phenylmethylsulfonylfluorid |

Folgende erfindungsgemäßen Verbindungen weisen im CDK1-Tests (Beispiel 13) einen CDK1/CyclinB1 ICso Wert von weniger als 100 nM auf:

### Beispiel 1:

Laufende Nummern: 003, 004, 005, 008, 009, 010, 011,012, 014, 015, 016, 017, 018, 019, 020, 021, 022, 023, 024, 025, 026, 027, 028, 029, 030, 031, 032, 033, 034, 035, 037, 038, 039, 040, 041, 042, 043, 044, 045, 046, 047, 049, 050, 052, 053, 054, 055, 067, 073, 077, 079, 080, 088, 093, 104, 107, 109, 111, 112, 113, 115, 116, 117, 118, 119, 120, 152, 304, 349, 388, 585, 628, 651, 652, 654, 655, 656, 661, 662, 663, 683, 685, 689, 690, 692, 693, 694, 695

### Beispiel 2:

Laufende Nummern: 002, 003, 004, 007, 008, 009, 010, 011, 012, 013, 014, 015, 016, 036, 037, 038, 041, 042, 043, 044, 045, 046, 047, 048, 051, 052, 053, 057, 058, 059, 060, 061, 062, 075, 076, 106, 109, 134, 136, 141, 142

### Beispiel 4:

Laufende Nummer: 001

### Beispiel 5:

Laufende Nummer: 000

### Beispiel 7:

Laufende Nummern: 001, 002, 006, 010, 012, 013, 015, 021, 022, 024, 027, 028, 029, 032, 035, 036

### Beispiel 8:

Laufende Nummern: 003, 007, 024, 026, 028

### Beispiel 9:

Laufende Nummer: 001

### Beispiel 10:

Laufende Nummern: 000, 014, 015, 016

### Beispiel 11:

Laufende Nummer: 034

### Darreichungsformen

Die erfindungsgemäßen Verbindungen können oral, transdermal, inhalativ oder parenteral verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragées, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 1 und 100, vorzugsweise zwischen 1 und 50, besonders bevorzugt zwischen 5-30 mg/Dosis, bei intravenöser oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbessemdes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 800 mg, bevorzugt 10 - 300 mg pro Erwachsener.

Die nachfolgenden Beispiele illustrieren die vorliegende Erfindung ohne sie jedoch in ihrem Umfang zu beschränken:

### Pharmazeutische Formulierungsbeispiele

| A) Tabletten | pro Tablette |
|---|---|
| Wirkstoff | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) Tabletten | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) Dragées | pro Dragée |
|---|---|
| Wirkstoff | 5 mg |
| Maisstärke | 41,5 mg |
| Milchzucker | 30 mg |
| Polyvinylpyrrolidon | 3 mg |
| Magnesiumstearat | 0.5 mg |
| | 80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragées werden mit Wachs poliert.

| D) Kapseln | pro Kapsel |
|---|---|
| Wirkstoff | 50 mg |
| Maisstärke | 268,5 mg |
| Magnesiumstearat | 1.5 mg |
| | 320 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

| E) Ampullenlösung | |
|---|---|
| Wirkstoff | 50 mg |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff

| F) Suppositorien | |
|---|---|
| Wirkstoff | 50 mg |
| Adeps solidus | 1650 mg |
| | 1700 mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

## Patentansprüche

1. Trisubstituierte Pyrimidine der Formel I, 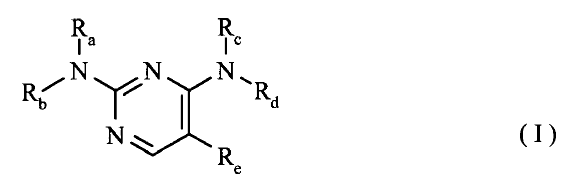 wobei
Rₐ für ein Wasserstoffatom steht,
R_{b} für eine gegebenenfalls durch die Reste R₁ bis R₃ substituierte Phenylgruppe steht, wobei
R₁ und R₂ jeweils unabhängig voneinander für
ein Fluor-, Chlor-, Brom- oder Iodatom stehen, oder
eine C₁₋₂-Alkyl- oder Hydroxygruppe,
eine C₃₋₇-Cycloalkyl- oder C₄₋₇-Cycloalkoxygruppe, die jeweils durch eine oder zwei
Alkylgruppen oder durch eine Arylgruppe substituiert sein können,
eine gegebenenfalls durch eine Arylgruppe substituierte C₂₋₅-Alkenylgruppe,
eine gegebenenfalls durch eine Arylgruppe substituierte C₂₋₅-Alkinylgruppe
eine Aryl-, Aryloxy-, Aralkyl-, Aralkoxy-, Alkylsulfenyl-, Alkylsulfinyl-, Alkylsulfonyl-, Alkylsulfonyloxy-, Trifluormethylsulfenyl-, Trifluormethylsulfinyl-, Trifluormethylsulfo-nyl-, Arylsulfenyl-, Arylsulfinyl-, Arylsulfonyl-, Aralkylsulfenyl-, Aralkylsulfinyl- oder Aralkylsulfonylgruppe,
eine durch 1 bis 3 Fluoratome substituierte Methyl- oder Methoxygruppe,
eine durch 1 bis 5 Fluoratome substituierte C₂₋₄-Alkyl- oder C₂₋₄-Alkoxygruppe,
eine Nitro-, Amino-, Alkylamino-, Dialkylamino-, C₃₋₇-Cycloalkylamino-,
N-Alkyl-C₃₋₇-cycloalkylamino-, Arylamino-, N-Alkyl-arylamino-, Aralkylamino- oder N-Alkyl-aralkylaminogruppe,
eine gegebenenfalls durch 1 bis 4 Alkylgruppen substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen jeweils durch eine Carbonylgruppe oder in den vorstehend erwähnten 6-bis 7-gliedrigen Alkyleniminogruppen eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein können,
eine gegebenenfalls durch 1 bis 4 Alkylgruppen substituierte (Alkylenimino)carbonyl-oder (Alkylenimino)sulfonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein kann,
eine Alkylcarbonylamino-, N-Alkyl-alkylcarbonylamino-, Alkyl-sulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Perfluoralkylsulfonylamino-, N-Alkyl-perfluoralkylsulfonylamino-, Alkylcarbonyl-, Arylcarbonyl-, Aralkylcarbonyl-, Aryl-hydroxymethyl-, Aralkyl-hydroxymethyl-, Carboxy-, Alkoxycarbonyl-, Aralkoxycarbonyl-, Aminocarbonyl-, Alkylaminocarbonyl-, Dialkylaminocarbonyl-, Arylaminocarbonyl-, N-Alkyl-arylaminocarbonyl-, Aralkylaminocarbonyl-, N-Alkyl-aralkylaminocarbonyl-, N-Hydroxy-aminocarbonyl-, N-Hydroxy-alkylaminocarbonyl-, N-Alkoxy-aminocarbonyl-, N-Alkoxy-alkylaminocarbonyl-, Cyano-, Azido-, N-Cyano-amino- oder N-Cyano-alkylaminogruppe,
eine Sulfo-, Alkoxysulfonyl-, Aminosulfonyl-, Alkylaminosulfonyl-, Dialkylaminosulfonyl-, Arylaminosulfonyl-, Pyridylaminosulfonyl-, Pyrimidinylaminosulfonyl-, N-Alkyl-arylaminosulfonyl-, Aralkylaminosulfonyl- oder N-Alkyl-aralkylaminosulfonylgruppe,
eine Phosphono-, O-Alkyl-phosphono-, O,O'-Dialkyl-phosphono-, O-Aralkyl-phosphono-oder O,O'-Diaralkyl-phosphonogruppe,
eine durch R₄ substituierte C₁₋₂ Alkylgruppe, wobei
R₄ eine Hydroxy-, Alkoxy-, Aryloxy-, Aralkoxy-, Amino-, Alkylamino-, Haloalkylamino-, Dialkylamino-, Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl-, Aralkylsulfonyl-, Carboxy-, Alkoxycarbonyl-, Aminocarbonyl-, Alkylaminocarbonyl-, Dialkylaminocarbonyl- oder Cyanogruppe, eine gegebenenfalls durch 1 bis 4 Alkylgruppen substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen durch eine Carbonylgruppe oder in den vorstehend erwähnten 6-bis 7-gliedrigen Alkyleniminogruppen eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein können, oder
eine gegebenenfalls durch 1 bis 4 Alkylgruppen substituierte 4- bis 7-gliedrige Alkyleniminogruppe, wobei in den vorstehend erwähnten 5- bis 7-gliedrigen Alkyleniminogruppen jeweils eine oder zwei zu dem Stickstoffatom benachbarte Methylengruppen durch eine Carbonylgruppe oder in der vorstehend erwähnten 6-bis 7-gliedrigen Alkyleniminogruppen durch eine oder zwei Hydroxy-, Alkoxy-, Aminocarbonyl-, Alkylaminocabonyl-, Dialkylaminocarbonyl-, Amino-, Alkylamino- und Dialkylaminogruppe substituiert sein kann eine gegebenenfalls durch 1 bis 4 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein kann, darstellt, oder
eine Gruppe der Formel 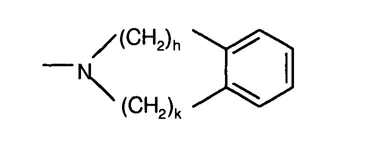 in der
h und k, die gleich oder verschieden sein können, die Zahlen 1 bis 3 oder h die Zahl 0 und k die Zahl 2, 3 oder 4 bedeuten, wobei zusätzlich der obige Benzoteil durch Fluor-, Chlor-, Brom- oder Iodatome, durch Alkyl-, Trifluormethyl-, Hydroxy-, Alkoxy-, Carboxy- oder Cyanogruppen mono- oder disubstituiert, wobei die Substituenten jeweils gleich oder verschieden sein können, und der obige gesättigte cyclische Alkyleniminoteil durch 1 oder 2 Alkylgruppen substituiert sein kann,
stehen,
R₃ für ein Fluor-, Chlor- oder Bromatom, eine C₁₋₂ Alkyl-, C₁₋₂ Alkoxy- oder Trifluormethylgruppe steht, oder
für einen 5- oder 6-gliedrigen heterocyclischen, aromatischen Ring mit mindestens einem Stickstoffatom und optional einem Schwefel- oder Sauerstoffatom, der durch eine oder zwei Alkyl-, Aryl- oder Aralkylreste substituiert sein kann, steht, oder
für eine Sulfo-, Alkoxysulfonyl-, Aminosulfonyl-, Alkylaminosulfonyl-, Dialkylaminosulfonyl-, Arylaminosulfonyl-, Pyridylaminosulfonyl-, Pyrimidinylaminosulfonyl-, N-Alkyl-arylaminosulfonyl-, Aralkylaminosulfonyl- oder N-Alkyl-aralkylaminosulfonylgruppe steht,
R₂ zusammen mit R₃, sofern diese an benachbarte Kohlenstoffatome gebunden sind, für eine gegebenenfalls durch eine oder zwei Alkylgruppen substituierte Methylendioxygruppe, oder
eine gegebenenfalls durch eine oder zwei Alkylgruppen substituierte n-C₃₋₆-Alkylengruppe, in der eine Methylengruppe durch ein Sauerstoff- oder Schwefelatom, durch eine Sulfinyl-, Sulfonyl-, N-Alkylcarbonyl-imino-, N-Alkylsulfonyl-imino-, N-Arylcarbonyl-imino- oder N-Arylsulfonyl-iminogruppe ersetzt sein kann, oder
eine gegebenenfalls durch ein oder zwei Fluor-, Chlor-, Brom- oder Iodatome, durch eine oder zwei Hydroxy-, Alkyl-, Alkoxy-, Trifluormethyl-, Carboxy-, Alkoxycarbonyl-, Aminocarbonyl-, Alkylaminocarbonyl-, Dialkylaminocarbonyl- oder Cyangruppen substituierte 1,3-Butadien-1,4-diylengruppe, wobei die Substituenten gleich oder verschieden sein können, oder
eine Gruppe der Formel
-(CH₂)ₘ-N(R₅)-(CH₂)ₙ-,
in der
die Methylengruppen der so gebildeten cyclischen Alkyleniminoteile zusätzlich durch 1 oder 2 Alkylgruppen substituiert sein können,
R₅ ein Wasserstoffatom oder eine Alkyl-, Haloalkyl-, Aryl- oder Aralkylgruppe bedeutet, und
m und n, die gleich oder verschieden sein können, die Zahlen 1, 2 oder 3 bedeuten, wobei in den so gebildeten Alkyleniminoteilen jeweils eine oder zwei zu dem Stickstoffatom benachbarte Methylengruppen durch eine Carbonylgruppe ersetzt sein können, oder
m die Zahl 0 und n die Zahl 2, 3 oder 4 bedeuten, wobei in den so gebildeten Alkyleniminoteilen jeweils die zu dem Stickstoffatom benachbarte Methylengruppe durch eine Carbonylgruppe ersetzt sein kann, oder
R₂ zusammen mit R₃ eine Gruppe, der Formel -NH-C(=O)-(CH₂)-, -NH-C(=O)-(CH₂)₂-, -NH-N=N-, -NH-N=CH-, -NH-CH=N-, -O-CH=N-, -S-CH=N- oder -NH-CH=CH- und die Tautomere der von -NH-N=N-, -NH-N=CH-, -NH-CH=N- definierten Ringsysteme, wobei jedes Wasserstoffatom durch eine Alkyl-, Aryl- oder Aralkylgruppe substituiert sein kann,
stehen,
R_{c}NR_{d} eine gegebenenfalls durch 1 bis 4 Alkylgruppen oder 1 bis 2 Arylgruppen substituierte 4-bis 8-gliedrige Alkyleniminogruppe, die zusätzlich durch den Rest R₆ substituiert ist, wobei
R₆ für eine Carboxy-, Alkoxycarbonyl-, Aminocarbonyl-, Alkylaminocarbonyl-, Dialkylaminocarbonyl-, Cyano-, Hydroxy-, Alkoxy-, Aryloxy-, Aralkoxy-, Alkylcarbonyloxy-, Arylcarbonyloxy-, Amino-, Alkylamino-, Hydroxy-C₂₋₄-alkylamino-, Dialkylamino-, Cyanamino-, Formylamino-, N-(Alkyl)-N-(hydroxy-C₂₋₄-alkyl)amino-, Bis-(hydroxy-C₂₋₄-alkyl)-aminogruppe, Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl-, Aralkylsulfonyl-, eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Alkoxycarbonylamino-, N-Alkyl-alkoxycarbonylamino-, Alkoxycarbonylalkylamino-, N-(Alkyl)-N-(alkoxycarbonylalkyl)-amino-, Aralkoxycarbonylamino- oder N-Alkyl-aralkoxycarbonylaminogruppe,
eine (NR₈R₉)CONR₇- oder (NR₈R₉)SO₂NR₇-Gruppe, in denen R₇, R₈ und R₉, die gleich oder verschieden sein können, jeweils ein Wasserstoffatom oder eine Alkyl-, Aryl-oder Pyridylgruppe, oder R₇ und R₈ zusammen eine n-C₂₋₄-Alkylengruppe und R₉ ein Wasserstoffatom oder eine Alkyl-, Aryl-oder Pyridylgruppe darstellen,
eine gegebenenfalls durch 1 bis 4 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann, eine gegebenenfalls durch 1 bis 4 Alkylgruppen oder eine Hydroxyalkylgruppe substituierte 4- bis 7-gliedrige Alkyleniminogruppe, wobei in den vorstehend erwähnten 5-bis 7-gliedrigen Alkyleniminogruppen jeweils eine oder zwei zu dem Stickstoffatom benachbarte Methylengruppen durch eine Carbonylgruppe ersetzt sein können,
eine gegebenenfalls durch 1 bis 4 Alkylgruppen oder eine Hydroxyalkylgruppe substituierte 6- oder 7-gliedrige Alkyleniminogruppe, wobei jeweils eine Methylengruppe in 4-Stellung des Alkyleniminoteils durch ein Sauerstoff- oder Schwefelatom, durch eine Carbonyl-, Sulfinyl-, Sulfonyl-, Imino-, N-Alkylimino-, N-Alkylcarbonyl-imino-, N-Alkylsulfonyl-imino-, N-Arylimino- oder N-Aralkyl-iminogruppe ersetzt ist und zusätzlich im Alkyleniminoteil der vorstehend erwähnten Gruppen jeweils eine oder zwei der zu den Stickstoffatomen benachbarten Methylengruppen durch eine Carbonylgruppe ersetzt sein können,
eine durch eine Hydroxy-, Alkoxy-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Carboxy-, Alkoxycarbonyl-, Aminocarbonyl-, Alkylaminocarbonyl- oder Dialkylaminocarbonylgruppe substituierte 4- bis 7-gliedrige Alkyleniminogruppe,
eine durch eine Carboxy-, Alkoxycarbonyl-, Aminocarbonyl-, Alkylaminocarbonyl- oder Dialkylaminocarbonyl-, Cyano-, Hydroxy-, Alkoxy-, Aryloxy-, Amino-, Alkylamino-, Dialkylamino-, Alkylcarbonylamino-, N-Alkyl-alkylcarbonylamino-, Alkoxycarbonylamino-, N-Alkyl-alkoxycarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aminocarbonylalkylamino-, N-(Alkyl)-N-(aminocarbonylalkyl)-amino-, Alkylaminocarbonylalkylamino-, N-(Alkyl)-N-(alkylaminocarbonylalkyl)-amino-, Dialkylaminocarbonylalkylamino-, N-(Alkyl)-N-(dialkylaminocarbonylalkyl)-amino-, Dialkylaminoalkoxy-, Alkylsulfenyl-, Alkylsulfinyl-, Alkylsulfonyl-, Arylsulfenyl-, Arylsulfinyl- oder Arylsulfonylgruppe substituierte Alkylgruppe,
eine gegebenenfalls durch 1 bis 4 Alkylgruppen substituierte (Alkylenimino)alkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino- oder N-Alkylcarbonyl-iminogruppe ersetzt sein kann,
eine gegebenenfalls durch 1 bis 4 Alkylgruppen substituierte (Alkylenimino)carbonylalkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,
eine (Carboxyalkyl)oxy-, (Alkoxycarbonylalkyl)oxy-, (Aminocarbonylalkyl)oxy-, (Alkylaminocarbonylalkyl)oxy- oder (Dialkylaminocarbonylalkyl)oxy-gruppe, eine gegebenenfalls durch 1 bis 4 Alkylgruppen substituierte [(Alkylenimino)carbonylalkyl]oxy-gruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,
eine C₅₋₇-Cycloalkylgruppe, in der eine Methylengruppe durch ein Sauerstoff- oder Schwefelatom, durch eine Sulfinyl-, Sulfonyl-, Imino- oder N-Alkyl-imino-, Alkylcarbonylimino-, Alkylsulfonyliminogruppe ersetzt ist,
eine gegebenenfalls jeweils im Aryteil durch ein oder zwei Fluor-, Chlor-, Brom- oder Iodatome oder eine oder zwei Nitro-, Alkyl-, Alkoxy- oder Cyanogruppen, wobei die Substituenten gleich oder verschieden sein können, substituierte 3,4-Dihydro-1*H-*quinazolin-2-on-3-yl- oder 1*H*-Benzimidazol-2-on-1-yl-gruppe, darstellt,
stehen, oder
R_{c}NR_{d} eine gegebenenfalls durch 1 bis 4 Alkylgruppen oder durch eine Arylgruppe substituierte 6- bis 8-gliedrige Alkyleniminogruppe, die zusätzlich durch den Rest R₆ substituiert sein kann, wobei in den vorstehend erwähnten Alkyleniminogruppen jeweils eine Methylengruppe in 4-Stellung durch ein Sauerstoff- oder Schwefelatom, durch eine Carbonyl-, Sulfinyl-, Sulfonyl-, N-Oxido-N-alkylimino- oder R₁₀N-Gruppe ersetzt ist, wobei
R₁₀ für ein Wasserstoffatom, eine Alkyl-, Hydroxy-C₂₋₄-alkyl-, Alkoxy-C₂₋₄-alkyl-, Amino-C₂₋₄-alkyl-, Alkylamino-C₂₋₄-alkyl-, Dialkylamino-C₂₋₄-alkyl-, (Hydroxy-C₂₋₄-alkoxy)-C₂₋₄-alkyl-, Aminocarbonylalkyl-, Alkylaminocarbonylalkyl-, Dialkylaminocarbonylalkyl-, Aryl-, Formyl-, Alkylcarbonyl-, Alkylsulfonyl-, Arylcarbonyl-, Aryl-sulfonyl-, Aralkylcarbonyl-, Aralkylsulfonyl-, Alkoxycarbonyl-, Aralkoxycarbonyl-, Cyano-, Aminocarbonyl-, Alkylaminocarbonyl- oder Dialkylaminocarbonylgruppe, eine Amino-alkylcarbonyl-, Alkylamino-alkylcarbonyl-, Dialkylamino-alkylcarbonyl-gruppe,
eine durch eine, zwei oder drei Arylgruppen substituierte Alkylgruppe,
eine 8-Alkyl-8-aza-bicyclo[3.2.1]oct-3-ylgruppe,
eine Aryl- oder eine 2-, 3- oder 4-Pyridylgruppe oder 2-, 4- oder 5 Pyrimidinylgruppe
eine (Alkylenimino)carbonyl- oder (Alkylenimino)carbonylalkyl-gruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino- oder N-Aralkyl-iminogruppe ersetzt sein kann,
steht, oder
R_{c}NR_{d} für eine in 4-Position durch eine Carboxy-, Alkoxycarbonyl-, Aminocarbonyl-, Alkylaminocarbonyl-, Dialkylaminocarbonylgruppe substituierte 3-Thiazolidinyl-Gruppe steht, oder
R_{c}NR_{d} eine gegebenenfalls durch 1 bis 4 Alkylgruppen substituierte 1-Pyrrolidinylgruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 2 bis 4 Kohlenstoffatome enthält, wenn sich die zwei Wasserstoffatome an Kohlenstoffatomen befinden, die durch ein Atom getrennt sind, wobei die vorstehend erwähnten 1-Pyrrolidinylgruppen zusätzlich durch den Rest R₆, der wie vorstehend erwähnt definiert ist, substituiert sind, steht
eine gegebenenfalls durch 1 bis 4 Alkylgruppen substituierte 1-Piperidinyl- oder 1-Azacyclohept-1-yl-gruppe, in denen zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 1 bis 4 Kohlenstoffatome enthält, wenn sich die zwei Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind, wobei die vorstehend erwähnten 1-Piperidinyl- und 1-Azacyclohept-1-yl-gruppen zusätzlich durch den Rest R₆, der wie vorstehend erwähnt definiert ist, substituiert sind,
eine gegebenenfalls durch 1 bis 4 Alkylgruppen substituierte 1-Pyrrolidinylgruppe, in der zwei Wasserstoffatome in 3-Stellung durch eine -O-CH₂CH₂-O- oder -O-CH₂CH₂CH₂-O-Gruppe substituiert sind,
eine gegebenenfalls durch 1 bis 4 Alkylgruppen substituierte 1-Piperidinyl- oder 1-Azacyclohept-1-yl-gruppe, in denen in 3-Stellung oder in 4-Stellung jeweils zwei Wasserstoffatome durch eine -O-CH₂CH₂-O- oder -O-CH₂CH₂CH₂-O-Gruppe substituiert sind,
eine gegebenenfalls durch eine Alkylgruppe substituierte 1-Azetidinylgruppe, in der die beiden Wasserstoffatome einer Methylengruppe durch eine geradkettige C₄₋₆-Alkylenbrücke ersetzt sind, wobei jeweils eine Methylengruppe in der C₄₋₆-Alkylenbrücke durch eine R₁₀N-Gruppe ersetzt ist, wobei R₁₀ wie vorstehend definiert ist, wobei der so gebildete bicyclische Ring zusätzlich durch eine Hydroxy-, Alkoxy-, Amino-, Alkylamino-, Dialkylamino-, Cyano-, Alkylcarbonylamino-, Alkylsulfonylamino-, Alkoxycarbonylamino-, Arylcarbonyl-, Carboxy-, Alkoxycarbonyl-, Aminocarbonyl-, Alkylaminocarbonyl-oder Dialkylaminocarbonylgruppe substituiert sein kann,
eine gegebenenfalls durch 1 bis 2 Alkylgruppen substituierte 1-Pyrrolidinyl-, 1-Piperidinyl- oder 1-Azacyclohept-1-ylgruppe, in der die beiden Wasserstoffatome einer Methylengruppe durch eine geradkettige C₃₋₆-Alkylenbrücke ersetzt sind, wobei jeweils eine Methylengruppe in der C₃₋₆-Alkylenbrücke durch eine R₁₀N-Gruppe ersetzt ist, wobei R₁₀ wie vorstehend definiert ist, wobei der so gebildete bicyclische Ring zusätzlich durch eine Hydroxy-, Alkoxy-, Amino-, Alkylamino-, Dialkylamino-, Cyano-, Alkylcarbonylamino-, Alkylsulfonylamino-, Alkoxycarbonylamino-, Arylcarbonyl-, Carboxy-, Alkoxycarbonyl-, Aminocarbonyl-, Alkylaminocarbonyl-oder Dialkylaminocarbonylgruppe substituiert sein kann,
eine gegebenenfalls in den Alkylenteilen durch 1 oder 2 Alkylgruppen substituierte Gruppe der Struktur 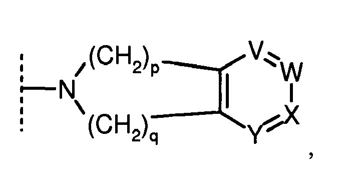
worin
p und q, die gleich oder verschieden sein können, die Zahl 1 oder 2 bedeuten, und
die Einheit-V=W-X=Y- eine der Gruppen (a), (b), (c), (d) oder (e) bedeutet:
-N=C-C=C- (a),
-C=N-C=C- (b),
-C=N-N=C- (c),
-N=C-C=N- (d),
-N=C-N=C- (e),
oder -V=W- zusammengenommen ein Sauerstoff- oder Schwefelatom und -X=Y- eine der Gruppen -N=C-, -C=N- oder -C=C- bedeuten,
oder -V=W- zusammengenommen eine Imino-, N-Alkyl-imino, N-Aralkyl-imino oder N-Aryl-imino-Gruppe und -X=Y- eine der Gruppen -N=N-, -N=C-, -C=N- oder -C=C-bedeuten,
oder, sofern p und q nicht gleich sind,
-X=Y- zusammengenommen ein Sauerstoff- oder Schwefelatom und -V=W- eine der Gruppen -N=C-, -C=N- oder -C=C- bedeuten,
oder -X=Y- zusammengenommen eine Imino-, N-Alkyl-imino-, N-Aralkyl-imino- oder N-Aryl-imino-Gruppe und -V=W- eine der Gruppen -N=N-, -N=C-, -C=N- oder -C=C-bedeuten,
wobei eines oder zwei der verfügbaren Kohlenstoffatome der Einheit -V=W-X=Y- jeweils durch eine Hydroxy-, Alkoxy-, Amino-, Alkylamino-, Dialkylamino-, Carboxy-, Alkoxycarbonyl, Aminocarbonyl-, Alkylaminocarbonyl-, Dialkylaminocarbonyl- oder Hydrazinocarbonylgruppe, wobei die Substituenten gleich oder verschieden sein können, substituiert sein kann und die verbleibenden verfügbaren Kohlenstoffatome der Einheit - V=W-X=Y- durch ein Wasserstoffatom, eine Alkyl-, Aralkyl- oder Arylgruppe substituiert sind,
oder
R_{c} ein Wasserstoffatom oder eine C₁₋₈-Alkylgruppe,
eine C₃₋₇-Cycloalkyl-, C₃₋₇-Cycloalkyl-alkyl- oder Aralkylgruppe, die jeweils durch eine oder zwei Alkylgruppen oder durch eine Arylgruppe substituiert sein können,
eine Alkylgruppe, die
durch eine Hydroxy-, Alkoxy-, Aryloxy-, Aralkoxy-, Alkylsulfenyl-, Alkylsulfinyl-, Alkylsulfonyl-, Arylsulfenyl-, Arylsulfinyl-, Arylsulfonyl-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Trifluormethylsulfonylamino-,
N-Alkyl-trifluormethylsulfonylamino-, Carboxy-, Alkoxycarbonyl-, Aralkoxycarbonyl-, Aminocarbonyl-, Alkylaminocarbonyl-, Dialkylaminocarbonyl-, Cyanogruppe,
durch eine 2-, 3- oder 4- Pyridylgruppe,
durch eine gegebenenfalls durch 1 bis 4 Alkylgruppen substituierte Alkylenimino- oder (Alkylenimino)carbonylgruppe mit jeweils 4 bis 7 Ringatomen im Alkyleniminoteil, wobei in den vorstehend erwähnten 6- bis 7-gliedrigen Alkyleniminogruppen eine Methylengruppe in 4-Stellung durch ein Sauerstoff- oder Schwefelatom, durch eine Imino-, N-Alkyl-imino-, N-Aryl-imino-, N-Aralkyl-imino-, N-Arylcarbonyl-imino- oder N-Alkylcarbonyl-iminogruppe ersetzt sein kann,
substituiert ist,
eine gegebenenfalls durch eine Arylgruppe substituierte C₃₋₅-Alkenylgruppe, wobei der Vinylteil nicht mit dem Stickstoffatom der R_{c}NR_{d}-Gruppe verknüpft sein kann,
eine gegebenenfalls durch eine Arylgruppe substituierte C₃₋₅-Alkinylgruppe, wobei der Ethinylteil nicht mit dem Stickstoffatom der R_{c}NR_{d}-Gruppe verknüpft sein kann, und
R_{d} eine C₁₋₁₆-Alkylgruppe, die durch eine Gruppe ausgewählt aus den Gruppen (a) bis (n) substituiert ist:
(a) eine Carboxy-, Alkoxycarbonyl-, Aminocarbonyl-, Alkylaminocarbonyl-, Dialkylaminocarbonyl-, Hydroxy-C₂₋₄-alkylaminocarbonyl-, Cyano-, Hydroxy-, Alkoxy-, Aryloxy-, Aralkoxy-, C₂₋₄-Alkylendioxy-, Alkylcarbonyloxy-, Arylcarbonyloxy-, Formylamino-, Alkylcarbonylamino-, Arylcarbonylamino-, Amino-, Alkylamino-, Dialkylamino-, Naphthylamino-, Aralkylamino-, Diaralkylamino- oder N-Alkyl-aralkylaminogruppe,
(b) eine gegebenenfalls im Arylteil durch ein oder zwei Fluor-, Chlor-, Brom- oder Iodatome oder eine oder zwei Nitro-, Trifluormethyl-, Alkyl-, Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl- oder Cyanogruppen substituierte Phenylamino-, N-Alkyl-N-phenylamino-, Pyridylamino oder N-Alkyl-N-pyridylaminogruppe, wobei die Substituenten gleich oder verschieden sein können,
(c) eine durch einen, zwei oder drei Arylreste substituierte Alkoxygruppe,
(d) eine Hydroxy-C₂₋₄-alkylaminocarbonyl-, Alkoxy-C₂₋₄-alkylaminocarbonyl-, Amino-C₂₋₄-alkylaminocarbonyl-, Alkylamino-C₂₋₄-alkylaminocarbonyl-, Dialkylamino-C₂₋₄-alkylaminocarbonyl-, Carboxyalkylaminocarbonyl-, Alkoxycarbonylalkylaminocarbonyl-, Aminocarbonylalkylaminocarbonyl-, Alkylaminocarbonylalkylaminocarbonyl-, Dialkylaminocarbonylalkylaminocarbonyl-, Arylaminocarbonyl-, N-Alkyl-arylaminocarbonyl-, Aralkylaminocarbonyl-, N-Alkyl-aralkylaminocarbonyl-,
(e) eine Gruppe der Formel -C(=NH)NH₂ oder -NH-C(=NH)NH₂, die gegebenenfalls durch eine Cyano- oder Alkoxycarbonylgruppe substituiert ist,
(f) eine gegebenenfalls durch 1 bis 4 Alkylgruppen substituierte (Alkylenimino)carbonylgruppe mit jeweils 4 bis 7 Ringatomen im Alkyleniminoteil, wobei in einem 6- oder 7-gliedrigen Alkyleniminoteil jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,
(g) eine gegebenenfalls durch 1 bis 4 Alkylgruppen substituierte 4- bis 7-gliedrige Alkyleniminogruppe, wobei in den vorstehend erwähnten 6- oder 7-gliedrigen Alkyleniminogruppen jeweils eine Methylengruppe in 4-Stellung durch ein Sauerstoff- oder Schwefelatom, durch eine Sulfinyl-, Sulfonyl- oder R₁₀N-Gruppe ersetzt sein kann, wobei R₁₀ wie vorstehend definiert ist, und zusätzlich in den vorstehend erwähnten 5- bis 7-gliedrigen Alkyleniminogruppen jeweils eine oder zwei zu den Stickstoffatomen benachbarte Methylengruppen durch eine Carbonylgruppe ersetzt sein können,
(h) eine gegebenenfalls durch 1 bis 2 Alkylgruppen substituierte 5- bis 7-gliedrige Alkyleniminogruppe, die durch eine Hydroxyalkyl-, Aminoalkyl-, Alkylaminoalkyl- oder Dialkylaminoalkylgruppe substituiert ist,
(i) eine Alkylsulfonylamino-, N-Alkyl-alkylsulfonylamino-, Arylcarbonylamino-, N-Alkyl-arylcarbonylamino-, Alkylcarbonylamino-, N-Alkyl-alkylcarbonylamino-, Arylsulfonylamino-, N-Alkyl-arylsulfonylamino-, Aralkylcarbonylamino-, N-Alkyl-aralkylcarbonylamino-, Alkoxyalkyl-carbonylamino-, Alkoxyalkyl-N-alkyl-carbonylamino-, Dialkylamino-alkylcarbonylamino-, Alkylamino-alkylcarbonylamino-, Amino-alkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe,
(j) eine (R₉NR₈)-CO-NR₇- oder (R₉NR₈)-SO₂-NR₇-Gruppe, wobei R₇, R₈ und R₉ wie vorstehend definiert sind,
(k) eine Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl- oder Aralkylsulfonylgruppe,
(l) eine durch R₆ und gegebenenfalls zusätzlich durch 1 bis 4 Alkylgruppen substituierte C₄₋₇Cycloalkylgruppe, wobei R₆ wie vorstehend definiert ist,
(m) eine gegebenenfalls durch 1 bis 4 Alkylgruppen substituierte C₅₋₇-Cycloalkylgruppe, in der eine Methylengruppe durch ein Sauerstoff- oder Schwefelatom, durch eine Sulfinyl-, Sulfonyl- oder NR₁₀-Gruppe ersetzt ist, wobei R₁₀ wie vorstehend definiert ist,
(n) eine gegebenenfalls durch 1 bis 4 Alkylgruppen substituierte 4-Piperidinyl-alkylgruppe, die in 1-Stellung durch R₁₀ und zusätzlich in der 4-Stellung durch eine Hydroxygruppe substituiert ist, wobei R₁₀ wie vorstehend definiert ist, und bei der zusätzlich jeweils ein Wasserstoffatom in Position 2 und 6 des Piperidinyl-Gerüsts zusammen durch eine C₂₋₃-Alkylenbrücke ersetzt sind,
oder
eine durch eine 3-Hydroxy-1,3-dihydro-indol-2-on-3-yl- oder 2-Aminocarbonyl-1,3-dihydro-isoindol-5-yl-gruppe substituierte Methylgruppe,
eine im Arylteil durch eine Carboxy-, Alkoxycarbonyl-, Aminocarbonyl-, Alkylaminocarbonyl-, Dialkylaminocarbonyl-, Carboxyalkyl-, Alkoxycarbonylalkyl-, Aminocarbonylalkyl-, Alkylaminocarbonylalkyl- oder Dialkylaminocarbonylalkylgruppe und gegebenenfalls zusätzlich im Alkylenteil durch 1 oder 2 Alkylgruppen substituierte Gruppe der Struktur 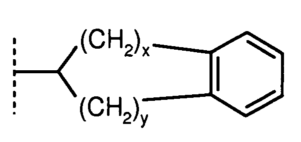 wobei x und y, die gleich oder verschieden sein können, unabhängig voneinander die Zahl 0, 1 oder 2 bedeuten, wobei jedoch x und y zusammen mindestens die Zahl 2 ergeben müssen,
eine durch eine Hydroxygruppe und zusätzlich durch eine Amino-, Alkylamino-, Dialkylamino-, Hydroxy-, Alkoxy-, 1-Pyrrolidinyl-, 1-Piperidinyl- oder Morpholinogruppe substituierte C₃₋₈-Alkylgruppe,
eine durch eine Carboxygruppe und zusätzlich durch eine Amino-, Hydroxy-, Aminocarbonyl-oder Benzyloxycarbonylaminogruppe substituierte C₂₋₈-Alkylgruppe,
eine C₂₋₄-Alkylgruppe, die durch eine C₂₋₄-Alkylsulfenyl- oder C₂₋₄-Alkoxygruppe substituiert ist, welche in ω-Position durch eine Amino-, Hydroxy- oder Alkoxygruppe substituiert ist,
eine C₂₋₄-Alkylgruppe, die durch eine C₂₋₄-Alkoxy-C₂₋₄-alkoxygruppe substituiert ist, welche in ω-Position durch eine Amino- oder Hydroxygruppe substituiert ist,
eine Cyclopropylgruppe, die durch eine Carboxy-, Alkoxycarbonyl-, Aminocarbonyl-, Alkylaminocarbonyl- oder Dialkylaminocarbonylgruppe oder durch eine gegebenenfalls durch 1 bis 4 Alkylgruppen substituierte (Alkylenimino)carbonylgruppe mit jeweils 4 bis 7 Ringatomen im Alkyleniminoteil substituiert ist, wobei in den vorstehend erwähnten 6- oder 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,
eine gegebenenfalls durch 1 bis 4 Alkylgruppen substituierte C₄₋₇-Cycloalkylgruppe, die zusätzlich durch R₆, der wie vorstehend definiert ist, substituiert ist,
eine gegebenenfalls durch 1 bis 2 Alkylgruppen substituierte C₅₋₇-Cycloalkylgruppe, die zusätzlich durch eine N,N-Dialkyl-N-oxido-aminogruppe substituiert ist,
eine gegebenenfalls durch 1 bis 4 Alkylgruppen substituierte C₄₋₇-Cycloalkylgruppe, die zusätzlich durch R₆ substituiert sein kann, wobei in dem Cycloalkylteil eine Methylengruppe durch ein Sauerstoff- oder Schwefelatom, durch eine Sulfinyl-, Sulfonyl-, N-Alkyl-N-oxido-imino- oder R₁₀N-Gruppe ersetzt ist, wobei R₆ und R₁₀ wie vorstehend definiert sind,
eine gegebenenfalls durch 1 bis 4 Alkylgruppen substituierte C₅-C₇-Cycloalkyl- oder C₅-C₇-Cycloalkylalkylgruppe, in denen jeweils eine Methylengruppe im Cycloalkylteil durch eine Carbonylgruppe ersetzt ist,
eine gegebenenfalls durch 1 bis 4 Alkylgruppen substituierte Cyclopentyl- oder Cyclopentylalkylgruppe, in denen jeweils zwei Wasserstoffatome im Cyclopentylteil durch eine geradkettige Alkylenbrücke ersetzt sind, wobei diese Brücke 2 bis 6 Kohlenstoffatome enthält, wenn sich die zwei Wasserstoffatome am selben Kohlenstoffatom befinden, oder 1 bis 5 Kohlenstoffatome enthält, wenn sich die zwei Wasserstoffatome an benachbarten Kohlenstoffatomen befinden, oder 2 bis 4 Kohlenstoffatome enthält, wenn sich die zwei Wasserstoffatome an Kohlenstoffatomen befinden, die durch ein Kohlenstoffatom getrennt sind, wobei die vorstehend erwähnten Ringe zusätzlich durch den Rest R₆, der wie vorstehend definiert ist, substituiert sind,
eine gegebenenfalls durch 1 bis 4 Alkylgruppen substituierte Cyclohexyl-, Cyclohexylalkyl-, Cycloheptyl- oder Cycloheptylalkylgruppe, in denen jeweils zwei Wasserstoffatome im Cycloalkylteil durch eine geradkettige Alkylenbrücke ersetzt sind, wobei diese Brücke 2 bis 6 Kohlenstoffatome enthält, wenn sich die zwei Wasserstoffatome am selben Kohlenstoffatom befinden, oder 1 bis 5 Kohlenstoffatome enthält, wenn sich die zwei Wasserstoffatome an benachbarten Kohlenstoffatomen befinden, oder 1 bis 4 Kohlenstoffatome enthält, wenn sich die zwei Wasserstoffatome an Kohlenstoffatomen befinden, die durch ein Kohlenstoffatom getrennt sind, oder 1 bis 3 Kohlenstoffatome enthält, wenn sich die zwei Wasserstoffatome an Kohlenstoffatomen befinden, die durch zwei Kohlenstoffatome getrennt sind, wobei die vorstehend erwähnten Ringe zusätzlich durch den Rest R₆, der wie vorstehend definiert ist, substituiert sind,
eine durch eine 3-Hydroxy-1,3-dihydro-indol-2-on-3-yl- oder 2-Aminocarbonyl-1,3-dihydro-isoindol-5-yl-gruppe substituierte Alkylgruppe,
eine durch eine Arylgruppe substituierte C₁₋₁₀-Alkylgruppe, wobei der vorstehend erwähnte Arylteil durch eine Alkoxycarbonyl-, Carboxy-, Carboxyalkyl-, Aminosulfonyl-, Trifluormethoxy-, Cyano-, Aminoalkyl-, Amino-, Alkylamino-, Dialkylamino-, Nitro-, 2H-Pyridazin-3-on-6-yl-, Hydroxyphenyl-, Hydroxyalkyl-, Hydroxy- oder Alkoxygruppe substituiert ist,
eine Aralkylgruppe, die im Arylteil durch eine Hydroxy- oder Alkoxygruppe und zusätzlich durch eine Carboxy-, Alkoxycarbonyl-, Hydroxy- oder Alkoxygruppe substituiert ist,
eine durch eine Pyrrolyl-, Imidazolyl-, Pyrazolyl-, Pyridyl-, Pyrimidinyl-, Pyrazinyl-, Indolyl- oder Benzimidazolylgruppe substituierte C₁₋₁₀-Alkylgruppe, wobei die vorstehend erwähnten Heteroarylteile an den verfügbaren Kohlenstoffatomen zusätzlich jeweils durch eine oder zwei Gruppen ausgewählt aus Fluor-, Chlor-, Brom- oder Iodatomen, Alkyl-, Alkoxycarbonyl-, Carboxy-, Trifluormethyl-, Trifluormethoxy-, Cyano-, Amino-, Alkylamino-, Dialkylamino-, Nitro-, Hydroxy- oder Alkoxygruppen substituiert sein können, wobei die Substituenten gleich oder verschieden sein können,
eine durch eine Carboxy-, Alkoxycarbonyl-, Aminocarbonyl-, Alkylaminocarbonyl-, Dialkylaminocarbonyl-, Aralkylaminocarbonyl-, Cyano-, Hydroxy-, Alkoxy-, Aryloxy-, Amino-, Alkylamino-, Dialkylamino-, Alkylcarbonylamino- oder Alkoxycarbonylaminogruppe substituierte C₁₋₁₀-Alkylgruppe, die zusätzlich durch eine oder zwei Arylgruppen oder eine Pyrrolyl-, Imidazolyl-, Pyrazolyl-, Pyridyl-, Pyrimidinyl-, Pyrazinyl-, Indolyl- oder Benzimidazolyl-gruppe substituiert ist, wobei die vorstehend erwähnten Aryl- oder Heteroarylteile an den verfügbaren Kohlenstoffatomen zusätzlich jeweils durch eine oder zwei Gruppen ausgewählt aus Fluor-, Chlor-, Brom- oder Iodatomen, Alkyl-, Alkoxycarbonyl-, Carboxy-, Trifluormethyl-, Trifluormethoxy-, Cyano-, Amino-, Alkylamino-, Dialkylamino-, Nitro-, Hydroxy- oder Alkoxygruppen substituiert sein können, wobei die Substituenten gleich oder verschieden sein können,
eine durch eine Arylgruppe substituierte C₁₋₆-Alkylgruppe, die im Arylteil durch eine Hydroxy-oder Aminogruppe und zusätzlich durch zwei Fluor-, Chlor-, Brom- oder Iodatome, wobei die Substituenten gleich oder verschieden sein können, substituiert ist,
eine durch eine Carboxy- oder Alkoxycarbonylgruppe substituierte C₂₋₆-Alkylgruppe, die zusätzlich durch eine Amino-, Alkylamino-, Dialkylamino-, Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe substituiert ist,
eine 3-Chinuclidinyl-, 4-Chinuclidinyl-, 2-Chinuclidinyl-alkyl-, 3-Chinuclidinyl-alkyl- oder 4-Chinuclidinyl-alkylgruppe, oder
R_{c} ein Wasserstoffatom oder eine Alkylgruppe und R_{d} eine Hydroxy- oder Alkoxygruppe darstellen, und
Rₑ eine Nitro- oder Trifluormethylgruppe,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
wobei, sofern nichts anderes erwähnt wurde,
unter den bei der Definition der vorstehend erwähnten Reste erwähnten Arylteilen eine Phenylgruppe zu verstehen ist, in der ein oder zwei Kohlenstoff-Atome jeweils durch ein Stickstoffatom ersetzt sein können, wobei die vorstehend genannten Arylteile jeweils durch R₁₁ monosubstituiert, durch R₁₂ mono-, di- oder trisubstituiert oder durch R₁₁ monosubstituiert und zusätzlich durch R₁₂ mono- oder disubstituiert sein können, wobei die Substituenten gleich oder verschieden sein können, und
R₁₁ eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Perfluoralkyl-, Perfluoralkoxy-, Nitro-, Amino-, Alkylcarbonylamino-, N-Alkyl-alkylcarbonylamino-, Alkylamino-, Dialkylamino-, Hydroxy-C2-4-alkylamino-, N-Alkyl-(hydroxy-C₂₋₄-alkyl)amino-, Bis-(hydroxy-C₂₋₄-alkyl)amino-, Phenylalkylcarbonylamino-, Phenylcarbonylamino-, Alkylsulfonylamino-, Phenylalkylsulfonylamino-, Phenylsulfonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkyl-phenylsulfonylamino-, Aminosulfonyl-, Alkylaminosulfonyl-, Dialkylaminosulfonyl-, (R₉NR₈)-CO-NR₇- oder (R₉NR₈)-SO₂-NR₇-Gruppe, wobei R₇, R₈ und R₉ wie vorstehend definiert sind,
eine gegebenenfalls durch 1 bis 4 Alkylgruppen oder eine Hydroxyalkylgruppe substituierte 5- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoffatom oder eine R₁₀N-Gruppe ersetzt sein kann, wobei R₁₀ wie vorstehend definiert ist,
eine gegebenenfalls durch 1 bis 4 Alkylgruppen oder eine Hydroxyalkylgruppe substituierte 5- bis 7-gliedrige Alkyleniminogruppe, wobei jeweils eine oder zwei zu dem Stickstoffatom benachbarte Methylengruppen jeweils durch eine Carbonylgruppe ersetzt ist, und
R₁₂ eine Alkyl-, Hydroxy- oder Alkoxygruppe, ein Fluor-, Chlor-, Brom- oder Iodatom darstellen, wobei zwei Reste R₁₂, sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,
sowie, soweit nichts anderes erwähnt wurde, die vorstehend erwähnten Alkyl-, Alkylen- und Alkoxyteile jeweils 1 bis 4 Kohlenstoffatome enthalten,
wobei, sofern nichts anderes erwähnt wurde, jedes Kohlenstoffatom in den vorstehend erwähnten Alkyl-, Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

2. Verbindungen der Formel I nach Anspruch 1, worin
R_{b} eine gegebenenfalls durch die Reste R₁ bis R₃ substituierte Phenylgruppe bedeutet,
wobei
R₁ ein Fluor-, Chlor-, Brom- oder Iodatom,
eine C₁₋₂-Alkyl- oder Hydroxygruppe,
eine C₃₋₆-Cycloalkyl- oder C₅₋₆-Cycloalkoxygruppe,
eine C₂₋₅-Alkenylgruppe,
eine C₂₋₅-Alkinylgruppe,
eine Aryl-, Aryloxy-, Aralkyl-, Aralkoxy-, Alkylsulfenyl-, Alkylsulfinyl-, Alkylsulfonyl-, Alkylsulfonyloxy-, Trifluormethylsulfenyl-, Trifluormethylsulfonyl-, Arylsulfenyl-, Arylsulfinyl-, Arylsulfonyl-, Aralkylsulfenyl-, Aralkylsulfinyl- oder Aralkylsulfonylgruppe,
eine durch 1 bis 3 Fluoratome substituierte Methyl- oder Methoxygruppe,
eine durch 1 bis 5 Fluoratome substituierte C₂₋₄-Alkyl- oder C₂₋₄-Alkoxygruppe,
eine Nitro-, Amino-, Alkylamino-, Dialkylamino-, C₃₋₆-Cycloalkylamino-, N-Alkyl-C₃₋₆-cycloalkylamino-, Arylamino-, N-Alkyl-arylamino-, Aralkylamino- oder N-Alkyl-aralkylaminogruppe,
eine 5- bis 7-gliedrige Alkyleniminogruppe, wobei jeweils eine oder zwei zu dem Stickstoffatom benachbarte Methylengruppen jeweils durch eine Carbonylgruppe oder in den vorstehend erwähnten 6- bis 7-gliedrigen Alkyleniminogruppen eine Methylengruppe in 4-Stellung durch ein Sauerstoffatom, durch eine Imino-, N-Aryl-imino- oder N-Alkyl-iminogruppe ersetzt sein können und die Alkyleniminogruppen zusätzlich durch 1-2 Methylgruppen substituiert sein kann,
eine (Alkylenimino)carbonyl- oder (Alkylenimino)sulfonylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoffatom, durch eine Imino-, N-Aryl-imino- oder N-Alkyl-iminogruppe ersetzt sein kann,
eine Alkylcarbonylamino-, N-Alkyl-alkylcarbonylamino-, Alkyl-sulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Trifluormethylsulfonylamino-, N-Alkyl-trifluormethylsulfonylamino-, Alkylcarbonyl-, Arylcarbonyl-, Aralkylcarbonyl-, Carboxy-, Alkoxycarbonyl-, Aminocarbonyl-, Alkylaminocarbonyl-, Dialkylaminocarbonyl-, Arylaminocarbonyl-, N-Alkyl-arylaminocarbonyl-, Aralkylaminocarbonyl-, N-Alkyl-aralkylaminocarbonyl-, N-Hydroxy-aminocarbonyl-, N-Hydroxy-alkylaminocarbonyl-, N-Alkoxy-aminocarbonyl-, N-Alkoxy-alkylaminocarbonyl-, Cyano-, Azido-, N-Cyano-amino- oder N-Cyano-alkylaminogruppe,
eine Sulfo-, Aminosulfonyl-, Alkylaminosulfonyl-, Dialkylaminosulfonyl-, Arylaminosulfonyl-, Pyridylaminosulfonyl-, N-Alkyl-arylaminosulfonyl-, Aralkylaminosulfonyl- oder N-Alkyl-aralkylaminosulfonylgruppe, oder
eine durch R₄ substituierte C₁₋₂ Alkylgruppe
bedeutet, wobei
R₄ eine Hydroxy-, Alkoxy-, Aryloxy-, Amino-, Alkylamino-, Fluoralkylamino-, Dialkylamino-, Alkylsulfenyl-, Alkylsulfinyl-, Alkylsulfonyl-, Arylsulfenyl-, Arylsulfinyl-, Arylsulfonyl-, Carboxy-, Alkoxycarbonyl-, Aminocarbonyl-, Alkylaminocarbonyl-, Dialkylaminocarbonyl- oder Cyanogruppe,
eine gegebenenfalls durch eine oder zwei Alkylgruppen substituierte 5- bis 7-gliedrige Alkyleniminogruppe, wobei in den vorstehend erwähnten 6- bis 7-gliedrigen Alkyleniminogruppen eine Methylengruppe in 4-Stellung durch ein Sauerstoff- oder Schwefelatom, durch eine Imino-, N-Alkyl-imino-, N-Alkylcarbonyl-imino-, N-Alkylsulfonyl-imino-, N-Arylcarbonyl-imino-, N-Arylsulfonyl-imino-, N-Aryl-imino- oder N-Aralkyl-iminogruppe ersetzt sein kann, oder durch eine Hydroxy-, Alkoxy-, Aminocarbonyl-, Alkylaminocabonyl-, Dialkylaminocarbonyl-, Amino-, Alkylamino- und Dialkylaminogruppe substituiert sein kann, oder
eine gegebenenfalls durch eine oder zwei Alkylgruppen substituierte (Alkylenimino)carbonylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Imino-, N-Alkyl-imino- oder N-Alkylcarbonyl-iminogruppe ersetzt sein kann, oder
eine Gruppe der Formel 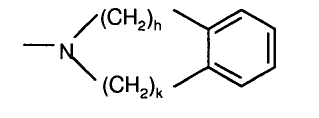 in der
h und k, die gleich oder verschieden sein können, die Zahlen 1 bis 2 oder h die Zahl 0 und k die Zahl 2 oder 3 bedeuten, wobei zusätzlich der obige Benzoteil durch ein Fluor-, Chlor-, Brom- oder Iodatom oder durch eine Alkyl-, Trifluormethyl-, Hydroxy-, Alkoxy-, Carboxy- oder Cyanogruppe und der obige gesättigte cyclische Iminoteil durch 1 oder 2 Alkylgruppen substituiert sein kann,
R₂ ein Fluor-, Chlor- oder Bromatom, eine C₁₋₂ Alkyl-, Trifluormethyl-, Hydroxy-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Trifluormethylsulfonylamino-, N-Alkyl-trifluormethylsulfonylamino- oder Cyanogruppe,
bedeutet, und
R₃ ein Fluor-, Chlor- oder Bromatom, eine C₁₋₂ Alkyl-, Trifluormethyl- oder Alkoxygruppe,
eine Gruppe der Struktur 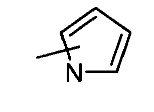 worin der Anknüpfungspunkt ein Kohlenstoff oder ein Stickstoffatom sein kann und bis zu drei Kohlenstoffatome durch ein Stickstoffatom ersetzt sein können und der Ring über jedes der Atome durch eine oder zwei Alkyl-, Aryl- oder Aralkylreste substituiert sein kann,
eine Sulfo-, Aminosulfonyl-, Alkylaminosulfonyl-, Dialkylaminosulfonyl-, Arylaminosulfonyl-, Pyridylaminosulfonyl-, N-Alkyl-arylaminosulfonyl-, Aralkylaminosulfonyl- oder N-Alkyl-aralkylaminosulfonylgruppe bedeutet,
R₂ zusammen mit R₃, sofern diese an benachbarte Kohlenstoffatome gebunden sind, eine gegebenenfalls durch eine oder zwei Alkylgruppen substituierte Methylendioxygruppe, oder eine gegebenenfalls durch eine oder zwei Alkylgruppen substituierte n-C₃₋₅-Alkylengruppe, in der eine Methylengruppe durch ein Sauerstoffatom, durch eine Imino-, N-Alkyl-imino- oder N-Aralkyl-iminogruppe ersetzt sein kann, oder
eine gegebenenfalls durch ein Fluor-, Chlor- oder Bromatom, durch eine Hydroxy-, Alkyl-, Alkoxy-, Trifluormethyl-, Carboxy oder Cyangruppe substituierte 1,3-Butadien-1,4-diylengruppe oder
eine Gruppe der Formel -NH-C(=O)-(CH₂)- oder -NH-C(=O)-(CH₂)₂-, die im Alkylenteil zusätzlich durch 1 oder 2 Alkylgruppen substituiert sein können, oder eine Gruppe der Formel -NH-N=N-, -NH-N=CH-, -NH-CH=N-, -O-CH=N-, -S-CH=N-,-NH-CH=CH- und deren Tautomeren, wobei jedes Wasserstoffatom durch eine Alkyl-, Aryl- oder Aralkylgruppe substituiert sein kann,
bedeuten, oder
eine Gruppe der Formel -(CH₂)ₘ-NR₅-(CH₂)ₙ-,
worin m und n jeweils gleich oder verschieden 1 oder 2 bedeuten, und
R₅ für Wassestoff, C₁₋₆Alkyl oder C₁₋₆ Fluoralkyl steht, und
R_{c}NR_{d} eine gegebenenfalls durch 1 bis 2 Alkyl- oder Arylgruppen substituierte 4- bis 7-gliedrige Alkyleniminogruppe bedeuten, die zusätzlich durch den Rest R₆ substituiert ist, wobei
R₆ eine Carboxy-, Alkoxycarbonyl-, Aminoalkyl-, Aminocarbonyl-, Alkylaminocarbonyl-, Dialkylaminocarbonyl-, Cyano-, Hydroxy-, Alkoxy-, Aryloxy-, Aralkoxy-, Alkylcarbonyloxy-, Arylcarbonyloxy-, Amino-, Alkylamino-, Hydroxy-C₂₋₄-alkylamino-, Dialkylamino-, Cyanamino-, Formylamino-, Alkylsulfenyl-, Alkylsulfinyl-, Alkylsulfonyl-, Arylsulfenyl-, Arylsulfinyl-, Arylsulfonyl-, Aralkylsulfenyl-, Aralkylsulfinyl- oder Aralkylsulfonylgruppe,
eine Alkylcarbonylamino-, N-Alkyl-alkylcarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Aralkylcarbonylamino-, N-Alkyl-aralkylcarbonylamino-, Aralkylsulfonylamino-, N-Alkyl-aralkylsulfonylamino-, Alkoxycarbonylamino-, N-Alkyl-alkoxycarbonylamino-, Alkoxycarbonylalkylamino-, N-(Alkyl)-N-(alkoxycarbonylalkyl)-amino-, Aralkoxycarbonylamino- oder N-Alkyl-aralkoxycarbonylaminogruppe,
eine (NR₈R₉)CONR₇-Gruppe, in der
R₇ und R₈ jeweils ein Wasserstoffatom oder eine Alkylgruppe und R₉ ein Wasserstoffatom oder eine Alkyl-, Aryl-oder Pyridylgruppe, wobei die Reste R₇, R₈ und R₉ gleich oder verschieden sein können, oder
R₇ und R₈ zusammen eine n-C₂₋₄-Alkylengruppe und R₉ ein Wasserstoffatom oder eine Alkyl-, Aryl-oder Pyridylgruppe
darstellen,
eine gegebenenfalls durch 1 bis 2 Alkylgruppen substituierte Alkyleniminogruppe mit 5 bis 7 Ringatomen im Alkyleniminoteil, wobei in den vorstehend erwähnten 6- bis 7-gliedrigen Alkyleniminoteilen jeweils eine Methylengruppe in 4-Stellung des Alkyleniminoteils durch ein Sauerstoff- oder Schwefelatom, durch eine Carbonyl-, Sulfinyl-, Sulfonyl-, Imino-, N-Alkylimino-, N-Alkylcarbonyl-imino-, N-Alkylsulfonyl-imino-, N-Arylimino- oder N-Aralkyl-iminogruppe ersetzt sein kann,
eine gegebenenfalls durch 1 bis 2 Alkylgruppen substituierte (Alkylenimino)carbonylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,
eine durch eine Hydroxy-, Alkoxy-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Hydroxyalkyl-, Carboxy-, Alkoxycarbonyl-, Aminocarbonyl-, Alkylaminocarbonyl- oder Dialkylaminocarbonyl-gruppe substituierte 4-bis 7-gliedrige Alkyleniminogruppe,
eine durch eine Carboxy-, Alkoxycarbonyl-, Aminocarbonyl-, Alkylaminocarbonyl-, Dialkylaminocarbonyl-, Cyano-, Hydroxy-, Alkoxy-, Aryloxy-, Amino-, Alkylamino-, Dialkylamino-, Alkylcarbonylamino-, N-Alkyl-alkylcarbonylamino-, Alkoxycarbonylamino-, N-Alkyl-alkoxycarbonylamino-, Alkylsulfonylamino-, N-Alkyl-alkylsulfonylamino-, Arylcarbonylamino-, N-Alkyl-arylcarbonylamino-, Arylsulfonylamino-, N-Alkyl-arylsulfonylamino-, Dialkylaminocarbonylalkylamino-, N-(Alkyl)-N-(dialkylaminocarbonylalkyl)-amino-, Dialkylaminoalkoxy-, Alkylsulfenyl-, Alkylsulfinyl-, Alkylsulfonyl-, Arylsulfenyl-, Arylsulfinyl- oder Arylsulfonylgruppe substituierte Alkylgruppe,
eine gegebenenfalls durch 1 bis 2 Alkylgruppen substituierte (Alkylenimino)alkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino- oder N-Alkylcarbonyl-iminogruppe ersetzt sein kann,
eine gegebenenfalls durch 1 bis 2 Alkylgruppen substituierte (Alkylenimino)carbonylalkylgruppe mit jeweils 4 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino- oder N-Alkyl-iminogruppe ersetzt sein kann,
eine (Carboxyalkyl)oxy-, (Alkoxycarbonylalkyl)oxy-, (Aminocarbonylalkyl)oxy-, (Alkylaminocarbonylalkyl)oxy- oder (Dialkylaminocarbonylalkyl)oxy-gruppe,
eine gegebenenfalls jeweils im Aryteil durch ein oder zwei Fluor-, Chlor-, Brom- oder Iodatome oder eine oder zwei Nitro-, Alkyl-, Alkoxy- oder Cyanogruppen, wobei die Substituenten gleich oder verschieden sein können, substituierte 3,4-Dihydro-1*H-*quinazolin-2-on-3-yl- oder 1*H*-Benzimidazol-2-on-1-yl-gruppe, darstellt,
bedeutet, oder
R_{c}NR_{d} eine gegebenenfalls durch 1 bis 2 Alkylgruppen oder durch eine Arylgruppe substituierte 6- bis 7-gliedrige Alkyleniminogruppe, die zusätzlich durch den Rest R₆ substituiert sein kann, wobei in den vorstehend erwähnten Alkyleniminogruppen jeweils eine Methylengruppe in 4-Stellung durch ein Sauerstoff- oder Schwefelatom, durch eine Carbonyl-, Sulfinyl-, Sulfonyl- oder R₁₀N-Gruppe ersetzt ist, bedeutet, wobei
R₁₀ ein Wasserstoffatom, eine Alkyl-, Hydroxy-C₂₋₄-alkyl-, Amino-C₂₋₄-alkyl-, Alkylamino-C₂₋₄-alkyl-, Dialkylamino-C₂₋₄-alkyl-, (Hydroxy-C₂₋₄-alkoxy)-C₂₋₄-alkyl-, Aminocarbonylalkyl-, Alkylaminocarbonylalkyl-, Dialkylaminocarbonylalkyl-, Aryl-, Formyl-, Alkylcarbonyl-, Alkylsulfonyl-, Arylcarbonyl-, Arylsulfonyl-, Aralkylcarbonyl-, Aralkylsulfonyl-, Alkoxycarbonyl-, Aralkoxycarbonyl-, Cyano-, Aminocarbonyl-, Alkylaminocarbonyl- oder Dialkylaminocarbonylgruppe,
eine Amino-alkylcarbonyl-, Alkylamino-alkylcarbonyl-, Dialkylamino-alkylcarbonylgruppe,
eine durch eine oder zwei Arylgruppen substituierte Methylgruppe, wobei die Arylteile unabhängig voneinander jeweils durch ein oder zwei Fluor-, Chlor-, Brom- oder Iodatome oder eine oder zwei Nitro-, Alkyl-, Hydroxy- oder Alkoxygruppen substituiert sein können, wobei die Substituenten gleich oder verschieden sein können,
eine 2-, 3- oder 4-Pyridylgruppe,
eine 2-, 4- oder 5-Pyrimidylgruppe,
eine gegebenenfalls durch ein oder zwei Fluor-, Chlor-, Brom- oder Iodatome oder eine oder zwei Nitro-, Trifluormethyl-, Alkyl-, Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl- oder Cyanogruppen substituierte Phenylgruppe, wobei die Substituenten gleich oder verschieden sein können,
eine 8-Methyl-8-aza-bicyclo[3.2.1]oct-3-ylgruppe,
oder eine (Alkylenimino)carbonyl- oder (Alkylenimino)carbonylalkylgruppe mit jeweils 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino- oder N-Aralkyl-iminogruppe ersetzt sein kann, darstellt, oder
R_{c}NR_{d} eine in 4-Position durch eine Carboxy-, Alkoxycarbonyl-, Aminocarbonyl-, Alkylaminocarbonyl- oder Dialkylaminocarbonylgruppe substituierte 3-Thiazolidinyl-Gruppe bedeutet, oder
R_{c}NR_{d} eine gegebenenfalls durch 1 bis 2 Alkylgruppen substituierte 1-Piperidinyl-gruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn die zwei Wasserstoffatome sich an benachbarten Kohlenstoffatomen befinden, oder 1 bis 4 Kohlenstoffatome enthält, wenn sich die zwei Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind, wobei die vorstehend erwähnten 1-Piperidinylgruppen zusätzlich durch den Rest R₆, der wie vorstehend erwähnt definiert ist, substituiert sind,
eine gegebenenfalls durch 1 bis 2 Alkylgruppen substituierte 1-Pyrrolidinyl- oder 1-Piperidinylgruppe, in der die beiden Wasserstoffatome einer Methylengruppe durch eine geradkettige C₃₋₆-Alkylenbrücke ersetzt sind, wobei jeweils eine Methylengruppe in der C₃₋₆-Alkylenbrücke durch eine R₁₀N-Gruppe ersetzt ist, wobei R₁₀ wie vorstehend definiert ist, wobei der so gebildete bicyclische Ring gegebenenfalls zusätzlich durch eine Hydroxy-, Alkoxy-, Amino-, Alkylamino-, Dialkylamino-, Cyano-, Alkylcarbonylamino-, Alkylsulfonylamino-, Alkoxycarbonylamino-, Arylcarbonyl-, Carboxy-, Alkoxycarbonyl-, Aminocarbonyl-, Alkylaminocarbonyl- oder Dialkylaminocarbonylgruppe substituiert ist,
eine gegebenenfalls in den Alkylenteilen durch 1 oder 2 Alkylgruppen substituierte Gruppe der Struktur 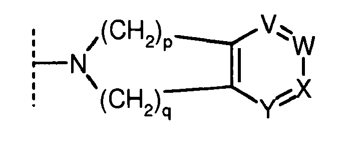 worin
p und q, die gleich oder verschieden sein können, unabhängig voneinander die Zahl 1 oder 2 bedeuten, und
die Einheit -V=W-X=Y- eine der Gruppen (a) oder (b) bedeutet:
-N=C-C=C- (a),
-C=N-C=C- (b),
wobei eines der verfügbaren Kohlenstoffatom der Gruppen (a) oder (b) durch eine Hydroxy-, Alkoxy-, Amino-, Alkylamino- oder Dialkylaminogruppe substituiert sein kann und die verbleibenden verfügbaren Kohlenstoffatome der Gruppen (a) oder (b) durch ein Wasserstoffatom, eine Alkyl- oder Arylgruppe substituiert sind,
oder
-V=W- zusammengenommen ein Sauerstoff- oder Schwefelatom oder eine Imino-, N-Alkyl-imino- oder N-Aryl-imino-Gruppe und -X=Y- eine der Gruppen -N=C- oder -C=N-bedeuten, oder,
sofern n und m nicht gleich sind,
-X=Y- zusammengenommen ein Sauerstoff- oder Schwefelatom oder eine Imino-, N-Alkyl-imino- oder N-Aryl-imino-Gruppe und -V=W- eine der Gruppen -N=C- oder -C=N-bedeuten,
oder
R_{c} ein Wasserstoffatom, eine Aralkyl- oder eine C₁₋₆-Alkylgruppe,
eine Alkylgruppe, die
durch eine Hydroxy-, Alkoxy-, Aryloxy-, Aralkoxy-, Amino-, Alkylamino-, Dialkylamino-, Alkylcarbonylamino-, N-Alkyl-alkylcarbonylamino-, Alkylsulfonylamino-, N-Alkyl-alkylsulfonylamino-, Trifluormethylsulfonylamino-,
N-Alkyl-trifluormethylsulfonylamino-, Carboxy-, Alkoxycarbonyl-, Aminocarbonyl-, Alkylaminocarbonyl-, Dialkylaminocarbonyl-, Cyano- oder durch eine 2-, 3- oder 4-Pyridylgruppe mit der Maßgabe, dass die Heteroatome von dem Stickstoffatom der R_{c}NR_{d}-Gruppe durch zwei oder mehr Kohlenstoffatome getrennt sind, substituiert ist,
eine C₃₋₅-Alkenylgruppe, wobei der Vinylteil nicht mit dem Stickstoffatom der R_{c}NR_{d}-Gruppe verknüpft sein kann,
eine C₃₋₅-Alkinylgruppe, wobei der Ethinylteil nicht mit dem Stickstoffatom der R_{c}NR_{d}-Gruppe verknüpft sein kann, und
R_{d} eine C₁₋₁₀-Alkylgruppe, die durch.eine Gruppe ausgewählt aus den Gruppen (a) bis (n) substituiert ist: .
(a) eine Carboxy-, Alkoxycarbonyl-, Aminocarbonyl-, Alkylaminocarbonyl-, Dialkylaminocarbonyl-, Cyano-, Hydroxy-, Alkoxy-, Aryloxy-, Aralkoxy-, Alkylcarbonylamino-, Amino-, Alkylamino-, Dialkylamino-, Naphthylamino-, Aralkylamino-, Diaralkylamino- oder N-Alkyl-aralkylaminogruppe,
(b) eine gegebenenfalls im Arylteil durch ein Fluor-, Chlor-, Brom- oder Iodatom oder eine Nitro-, Trifluormethyl-, Alkyl-, Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl- oder Cyanogruppe substituierte Phenylamino- oder Pyridylaminogruppe,
(c) eine durch einen, zwei oder drei Arylreste substituierte Methoxygruppe,
(d) eine Carboxyalkylaminocarbonyl-, Alkoxycarbonylalkylaminocarbonyl-, Aminocarbonylalkylaminocarbonyl-, Alkylaminocarbonylalkylaminocarbonyl-, Dialkylaminocarbonylalkylaminocarbonyl-, Arylaminocarbonyl-, N-Alkyl-arylaminocarbonyl-, Aralkylaminocarbonyl-, N-Alkyl-aralkylaminocarbonyl-,
(e) eine Gruppe der Formel -C(=NH)NH₂,
(f) eine gegebenenfalls durch 1 bis 2 Alkylgruppen substituierte (Alkylenimino)carbonylgruppe mit jeweils 5 bis 7 Ringatomen im Alkyleniminoteil, wobei in den vorstehend erwähnten 6- bis 7-gliedrigen Alkyleniminogruppen jeweils eine Methylengruppe in 4-Stellung durch ein Sauerstoff- oder Schwefelatom, durch eine Sulfinyl-, Sulfonyl-, Imino-, N-Alkyl-imino-, N-Alkylcarbonyl-imino-, N-Alkylsulfonyl-imino-, N-Aryl-imino- oder N-Aralkyl-iminogruppe ersetzt sein kann,
(g) eine gegebenenfalls durch 1 bis 2 Alkylgruppen substituierte 4- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoff- oder Schwefelatom, durch eine Sulfinyl-, Sulfonyl- oder R₁₀N-Gruppe ersetzt sein kann, wobei R₁₀ wie vorstehend definiert ist, und zusätzlich in den vorstehend erwähnten 5- bis 7-gliedrigen Alkyleniminogruppen jeweils eine zu den Stickstoffatomen benachbarte Methylengruppe durch eine Carbonylgruppe ersetzt sein kann,
(h) eine gegebenenfalls durch 1 bis 2 Alkylgruppen substituierte 5- bis 7-gliedrige Alkyleniminogruppe, die durch eine Hydroxyalkyl-, Aminoalkyl-, Alkylaminoalkyl-oder Dialkylaminoalkylgruppe substituiert ist,
(i) eine Alkylsulfonylamino-, N-Alkyl-alkylsulfonylamino-, Arylcarbonylamino-, N-Alkyl-arylcarbonylamino-, Alkylcarbonylamino-, N-Alkyl-alkylcarbonylamino-, Alkoxy-alkylcarbonylamino-, Dialkylamino-alkylcarbonylamino-, Arylsulfonylamino-, N-Alkyl-arylsulfonylamino-, Aralkylcarbonylamino-, N-Alkylaralkylcarbonylamino-, Aralkylsulfonylamino-, N-Alkyl-aralkylsulfonylamino-, Alkoxycarbonylamino-, N-Alkyl-alkoxycarbonylamino-, Aralkoxycarbonylamino- oder N-Alkyl-aralkoxycarbonylaminogruppe,
(j) eine (R₉NR₈)-CO-NR₇-Gruppe, wobei R₇, R₈ und R₉ wie vorstehend definiert sind,
(k) eine 2-Aza-bicyclo[2.2.1]hept-5-en-2-yl-gruppe,
(l) eine Alkylsulfenyl-, Alkylsulfinyl-, Alkylsulfonyl-, Arylsulfenyl-, Arylsulfinyl- oder Arylsulfonylgruppe,
(m) eine durch R₆ und gegebenenfalls zusätzlich durch 1 bis 2 Alkylgruppen substituierte C₄₋₇-Cycloalkylgruppe, wobei R₆ wie vorstehend definiert ist,
(n) eine gegebenenfalls durch 1 bis 4 Alkylgruppen substituierte C₅₋₇-Cycloalkylgruppe, in der eine Methylengruppe durch ein Sauerstoffatom oder eine NR₁₀-Gruppe ersetzt ist, wobei R₁₀ wie vorstehend definiert ist,
eine 4-Piperidinyl-methylgruppe, die in 1-Stellung durch R₁₀ und zusätzlich in der 4-Stellung durch eine Hydroxygruppe substituiert ist, wobei R₁₀ wie vorstehend definiert ist, und bei der zusätzlich jeweils ein Wasserstoffatom in Position 2 und 6 des Piperidinyl-Gerüsts zusammen durch eine C₂₋₃-Alkylenbrücke ersetzt sind,
eine durch eine 3-Hydroxy-1,3-dihydro-indol-2-on-3-yl- oder 2-Aminocarbonyl-1,3-dihydro-isoindol-5-yl-gruppe substituierte Methylgruppe,
eine im Arylteil durch eine Carboxy- oder Carboxyalkylgruppe und gegebenenfalls zusätzlich im Alkylenteil durch 1 oder 2 Alkylgruppen substituierte Gruppe der Struktur 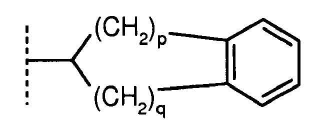 wobei p und q, die gleich oder verschieden sein können, die Zahl 0, 1 oder 2 bedeuten,
wobei jedoch p und q zusammen mindestens die Zahl 2 ergeben müssen,
eine durch eine Hydroxygruppe und zusätzlich durch eine Amino-, Alkylamino-, Dialkylamino-, Hydroxy-, Alkoxy-, 1-Pyrrolidinyl-, 1-Piperidinyl- oder Morpholinogruppe substituierte C₃₋₆-Alkylgruppe,
eine durch eine Carboxygruppe und zusätzlich durch eine Amino-, Hydroxy-, Aminocarbonyl-oder Benzyloxycarbonylaminogruppe substituierte C₂₋₆-Alkylgruppe,
eine C₂₋₄-Alkylgruppe, die durch eine C₂₋₄-Alkylsulfenylgruppe substituiert ist, welche in ω-Position durch eine ω-Aminogruppe substituiert ist,
eine C₂₋₄-Alkylgruppe, die durch eine C₂₋₄-Alkoxygruppe substituiert ist, welche in ω-Position durch eine Amino-, Hydroxy- oder Alkoxygruppe substituiert ist,
eine C₂₋₄-Alkylgruppe, die durch eine C₂₋₄-Alkoxy-C₂₋₄-alkoxygruppe substituiert ist, welche in ω-Position durch eine Amino- oder Hydroxygruppe substituiert ist,
eine gegebenenfalls durch 1 bis 2 Alkylgruppen substituierte C₄₋₇-Cycloalkylgruppe, die zusätzlich durch R₆, der wie vorstehend definiert ist, substituiert ist,
eine gegebenenfalls durch 1 bis 2 Alkylgruppen substituierte C₄₋₇-Cycloalkylgruppe, die zusätzlich durch R₆ substituiert sein kann, wobei in dem Cycloalkylteil eine Methylengruppe durch ein Sauerstoff- oder Schwefelatom, durch eine Sulfinyl-, Sulfonyl- oder R₁₀N-Gruppe ersetzt ist, wobei R₆ und R₁₀ wie vorstehend definiert sind,
eine durch eine 3-Hydroxy-1,3-dihydro-indol-2-on-3-yl- oder 2-Aminocarbonyl-1,3-dihydro-isoindol-5-yl-gruppe substituierte Methylgruppe,
eine durch eine Arylgruppe substituierte C₁₋₆-Alkylgruppe, wobei der vorstehend erwähnte Arylteil durch eine Alkoxycarbonyl-, Carboxy-, Carboxyalkyl-, Aminosulfonyl-, Trifluormethoxy-, Cyano-, Aminoalkyl-, Amino-, Alkylamino-, Dialkylamino-, Nitro-, 2H-Pyridazin-3-on-6-yl-, Hydroxyphenyl-, Hydroxyalkyl-, Hydroxy- oder Alkoxygruppe substituiert ist,
eine Aralkylgruppe, die im Arylteil durch eine Alkoxy- oder Hydroxygruppe und zusätzlich durch eine Alkoxycarbonyl-, Carboxy-, Alkoxy- oder Hydroxygruppe substituiert ist,
eine durch eine 2-Pyridyl-, 3-Pyridyl-, 4-Pyridyl-, 2-Pyrazinyl-,1*H*-Pyrrol-2-yl-, 1*H*-Pyrazol-4-yl-, 1*H*-Pyrazol-5-yl-, 1*H*-Imidazol-1-yl-, 1*H*-Imidazol-4-yl-, 1*H*-Indol-3-yl-, 1*H*-Benzimidazol-2-yl-gruppe substituierte C₁₋₆-Alkylgruppe, wobei die vorstehend erwähnten Heteroarylteile an den verfügbaren Kohlenstoffatomen zusätzlich jeweils durch eine oder zwei Gruppen ausgewählt aus Fluor-, Chlor-, Brom- oder Iodatomen, Alkyl-, Alkoxycarbonyl-, Carboxy-, Trifluormethyl-, Trifluormethoxy-, Cyano-, Amino-, Alkylamino-, Dialkylamino-, Nitro-, Hydroxy- oder Alkoxygruppen substituiert sein können, wobei die Substituenten gleich oder verschieden sein können,
eine durch eine Carboxy-, Alkoxycarbonyl-, Aminocarbonyl-, Alkylaminocarbonyl-, Dialkylaminocarbonyl-, Aralkylaminocarbonyl-, Cyano-, Hydroxy-, Alkoxy-, Aryloxy-, Amino-, Alkylamino-, Dialkylamino-, Alkylcarbonylamino- oder Alkoxycarbonylaminogruppe substituierte C₁₋₆-Alkylgruppe, die zusätzlich durch eine oder zwei Arylgruppen oder eine Heteroarylgruppe substituiert ist, wobei die Heteroarylgruppe eine 2-Pyridyl-, 3-Pyridyl-, 4-Pyridyl-, 2-Pyrazinyl-,1*H*-Pyrrol-2-yl-, 1*H*-Pyrazol-4-yl-,1*H*-Pyrazol-5-yl-, 1*H*-Imidazol-1-yl-, 1*H*-Imidazol-4-yl-, 1*H*-Indol-3-yl-, 1*H-*Benzimidazol-2-yl-gruppe darstellt, wobei die vorstehend erwähnten Aryl- oder Heteroarylteile an den verfügbaren Kohlenstoffatomen zusätzlich jeweils durch eine oder zwei Gruppen ausgewählt aus Fluor-, Chlor-, Brom- oder Iodatomen, Alkyl-, Alkoxycarbonyl-, Carboxy-, Trifluormethyl-, Trifluormethoxy-, Cyano-, Amino-, Alkylamino-, Dialkylamino-, Nitro-, Hydroxy- oder Alkoxygruppen substituiert sein können, wobei die Substituenten gleich oder verschieden sein können,
eine durch eine Arylgruppe substituierte C₁₋₆-Alkylgruppe, die im Arylteil durch eine Hydroxy-oder Aminogruppe und zusätzlich durch zwei Fluor-, Chlor-, Brom- oder Iodatome, wobei die Substituenten gleich oder verschieden sein können, substituiert ist,
eine durch eine Carboxy- oder Alkoxycarbonylgruppe substituierte C₂₋₆-Alkylgruppe, die zusätzlich durch eine Amino-, Alkylamino-, Dialkylamino-,
Alkylcarbonylamino-, Arylcarbonylamino-, Arylsulfonylamino-, Alkoxycarbonylamino- oder Aralkoxycarbonylaminogruppe substituiert ist,
eine 3-Chinuclidinyl- oder 4-Chinuclidinylgruppe,
bedeutet, und
wobei, soweit nichts anderes erwähnt wurde, die vorstehend erwähnten Alkyl-, Alkylen- und Alkoxyteile jeweils 1 bis 4 Kohlenstoffatome enthalten,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
wobei, sofern nichts anderes erwähnt wurde, jedes Kohlenstoffatom in den vorstehend erwähnten Alkyl-, Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

3. Verbindungen der Formel I nach Anspruch 1 oder 2, wobei
R_{b} eine gegebenenfalls durch ein Fluor-, Chlor- oder Bromatom oder eine Carboxy-, C₁₋₂ Alkyl-, C₁₋₂-Alkoxy-, Cyano- oder Trifluormethylgruppe substituierte Naphthylgruppe,
eine gegebenenfalls durch eine Methylgruppe am Stickstoff substituierte 5- oder 6-Indazolylgruppe oder eine 1,3-Dihydro-2-oxo-indol-6-ylgruppe oder
eine gegebenenfalls durch die Reste R₁ bis R₃ substituierte Phenylgruppe darstellt, wobei
R₁ ein Fluor-, Chlor-, Brom- oder Iodatom, eine C₁₋₂ Alkyl-, Trifluormethyl-, Aminocarbonyl-, Carboxy-, Alkoxycarbonyl-, Cyano-, Phenylaminocarbonyl-, Benzylaminocarbonyl-, Aminosulfonyl-, Methylaminosulfonyl-, Dimethylaminosulfonyl-, Morpholinosulfonyl-, N-Methylpiperazinosulfonyl-, Homopiperazinosulfonyl-, 2,6-Dimethylpiperazin-4-yl-, 2-Aminopyridyl-N-sulfonyl-, Morpholino-, 4-Methyl-1-piperazinyl-, (N-Methyl-N-methylsulfonyl)amino-, 2-Carboxy-1-ethyl-, Dimethylamino-1-ethyl- oder Nitrogruppe,
eine Methylgruppe, die durch eine 1,2,4,5-Tetrahydro-benzo[*d*]azepin-3-yl-, eine Dialkylamino- oder eine Pyrrolidino-, Piperidino-, 2,6-Dimethyl-piperidino-1-yl-, 4-Methoxy-piperidino-1-yl-, Morpholino-, S-Dioxo-thiomorpholino-, Piperazino- oder 4-Methyl-1-piperazinylgruppe oder eine Fluoralkylaminogruppe der Formel
-(CH₂)ᵣ-(CF₂)ₛ-Q,
worin
r 0 oder eine ganze Zahl von 1 bis 3,
s eine ganze Zahl von 1 bis 3, und
Q Wasserstoff, Fluor oder Chlor bedeuten,
substituiert ist,
R₂ ein Fluor- oder Chloratom, eine Hydroxy-, Amino- oder Methylgruppe und
R₃ ein Chloratom, oder
eine Tetrazolyl-, Triazolyl-, Imidazolyl- oder Pyrazolylgruppe,
worin der Anknüpfungspunkt ein Kohlenstoffatom oder ein Stickstoffatom ist und an dem Ring ein Wasserstoffatome durch einen Alkylreste ersetzt sein kann, Aminosulfonyl-, Methylaminosulfonyl-, Dimethylaminosulfonyl-, Morpholinosulfonyl-, N-Methylpiperazinosulfonyl-, Homopiperazinosulfonyl-, 2-Aminopyridyl-N-sulfonyl-,
darstellen, oder
R₂ und R₃ zusammengenommen eine Gruppe der Formel
-(CH₂)ₘ-NR₅-(CH₂)ₙ-
worin n und m jeweils unabhängig voneinander 1 oder 2 bedeuten, und
R₅ für eine Fluoralkylgruppe der Formel
-(CH₂)_{r'}-(CF₂)ₛ'-Q'
steht,
worin
r' 0 oder eine ganze Zahl von 1 bis 3,
s' eine ganze Zahl von 1 bis 3, und
Q' Wasserstoff, Fluor oder Chlor bedeuten,
die Gruppe R_{c}NR_{d}
eine durch den Rest R₆ substituierte 5- bis 7-gliedrige Alkyleniminogruppe,
wobei R₆ eine Hydroxy-, Alkoxy-, Aryloxy-, Amino-, Alkylamino-, Dialkylamino-, Alkylcarbonylamino-, N-Alkyl-alkylcarbonylamino-, Alkoxycarbonylalkylamino-, N-(Alkyl)-N-(alkoxycarbonylalkyl)-amino-, Alkylsulfenyl-, Alkylsulfinyl-, Alkylsulfonyl-, Arylsulfenyl-, Arylsulfinyl-, Arylsulfonyl-, Carboxy-, Alkoxycarbonyl-, Aminocarbonyl-, Alkylaminocarbonyl-, Dialkylaminocarbonyl- oder Cyanogruppe,
eine Alkylgruppe, die durch eine Hydroxy-, Amino-, Alkylamino-, Dialkylamino-, Alkylcarbonylamino-, N-Alkyl-alkylcarbonylamino-, Dialkylaminocarbonylalkylamino-, N-(Alkyl)-N-(dialkylaminocarbonylalkyl)-amino-, Alkoxycarbonyl-, Carboxy-, Dialkylaminoalkoxy-, gruppe oder durch eine 5- bis 7-gliedrige Alkyleniminogruppe, wobei in den vorstehend erwähnten 6- bis 7-gliedrigen Alkyleniminogruppen eine Methylengruppe in 4-Stellung durch ein Sauerstoff- oder Schwefelatom oder eine Imino-, N-Alkyl-imino- oder N-Alkylcarbonyl-iminogruppe ersetzt sein kann, substituiert ist,
eine Alkyleniminogruppe mit 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine N-Alkyl-imino-, N-Alkylcarbonyl-imino- oder N-Aralkyl-iminogruppe ersetzt sein kann,
eine durch eine Hydroxy-, Amino-, Alkylamino-, Dialkylamino-, Carboxy-, Alkoxycarbonyl-, Aminocarbonyl-, Alkylaminocarbonyl- oder Dialkylaminocarbonylgruppe substituierte Alkyleniminogruppe mit 5 bis 7 Ringatomen im Alkyleniminoteil,
eine gegebenenfalls jeweils im Aryteil durch ein Fluor-, Chlor-, oder Bromatom oder eine Nitro-, Alkyl-, Alkoxy- oder Cyanogruppe substituierte 3,4-Dihydro-1*H*-quinazolin-2-on-3-yl- oder eine 1*H*-Benzimidazol-2-on-1-yl-gruppe, darstellt,
eine gegebenenfalls durch 1 oder 2 Alkylgruppen substituierte 6- bis 7-gliedrige Alkyleniminogruppe, wobei eine Methylengruppe in 4-Stellung durch ein Sauerstoff- oder Schwefelatom, durch eine Sulfinyl-, Sulfonyl- oder eine NR₁₀-gruppe ersetzt ist,
wobei R₁₀ ein Wasserstoffatom oder eine Alkyl-, Aralkyl- Amino-C₂₋₄-alkyl-, Hydroxy-C₂₋₄-alkyl-, Alkylcarbonyl-, Aralkoxycarbonyl-, Alkylsulfonyl-, Arylcarbonyl-, Arylsulfonyl-,
eine (Alkylenimino)carbonylalkylgruppe mit 5 bis 7 Ringatomen im Alkyleniminoteil, wobei in den vorstehend erwähnten 6- bis 7-gliedrigen Alkyleniminoteilen jeweils eine Methylengruppe in 4-Stellung durch ein Sauerstoff- oder Schwefelatom, durch eine N-Alkyl-imino-, N-Alkylcarbonyl-imino- oder N-Aralkyl-iminogruppe ersetzt sein kann,
eine 2-, 3- oder 4-Pyridylgruppe,
eine 2-, 3- oder 4-Pyrimidylgruppe,
eine gegebenenfalls durch ein oder zwei Fluor-, Chlor-, Brom- oder Iodatome oder eine oder zwei Nitro-, Alkyl-, Hydroxy-, Alkoxy-, Amino-, Alkylamino-, Dialkylamino-, Carboxy-, Alkoxycarbonyl-, Aminocarbonyl-, Alkylaminocarbonyl-, Dialkylaminocarbonyl- oder Cyanogruppen substituierte Phenylgruppe, wobei die Substituenten gleich oder verschieden sein können,
eine 8-Methyl-8-aza-bicyclo[3.2.1]oct-3-ylgruppe,
eine Benzhydrylgruppe, in der unabhängig voneinander jeder Phenylteil jeweils durch ein Fluor-, Chlor-, Brom- oder Iodatom oder eine Nitro-, Alkyl-, Hydroxy-, Alkoxygruppe substituiert sein kann,wobei die Substituenten gleich oder verschieden sein können,
eine durch eine Phenylgruppe substituierte 6- oder 7-gliedrige Alkyleniminogruppe, die zusätzlich durch eine Hydroxy-, Carboxy-, Alkoxycarbonyl- oder Cyanogruppe substituiert ist oder in der eine Methylengruppe in 4-Stellung durch eine Carbonylgruppe ersetzt ist,
eine in 4-Position durch eine Carboxy-, Alkoxycarbonyl-, Aminocarbonyl-, Alkylaminocarbonyl-, Dialkylaminocarbonylgruppe substituierte 3-Thiazolidinyl-Gruppe,
eine Gruppe der Struktur 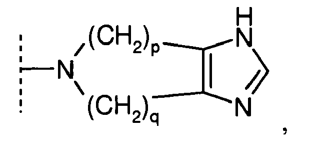 worin p und q, die gleich oder verschieden sein können, unabhängig voneinander die Zahl 1 oder 2 bedeuten, wobei der Imidazoring durch eine oder zwei Alkyl- oder Arylgruppen substituiert sein kann, wobei die Substituenten gleich oder verschieden sein können,
eine 1-Pyrrolidinyl- oder 1-Piperidinylgruppe, in der die beiden Wasserstoffatome einer Methylengruppe durch eine geradkettige C₃₋₅-Alkylenbrücke ersetzt sind, wobei jeweils eine Methylengruppe in der C₃₋₅-Alkylenbrücke durch eine Imino-, N-Alkyl-imino- oder N-(Aralkyl)imino-Gruppe ersetzt ist, wobei der so gebildete bicyclische Ring gegebenenfalls zusätzlich durch eine Hydroxygruppe substituiert ist,
eine 1-Piperidinylgruppe, die in 4-Stellung durch eine Hydroxy-, Alkoxy- oder Aralkoxygruppe substituiert ist und bei der zusätzlich jeweils eines der Wasserstoffatome in Position 2 und 6 des Piperidinyl-Gerüsts zusammen durch eine Ethylenbrücke ersetzt sind,
oder
R_{c} ein Wasserstoffatom oder eine C₁₋₆-Alkylgruppe,
eine durch eine Phenyl- oder eine 2-,3- oder 4-Pyridylgruppe substituierte Alkylgruppe,
eine durch eine Hydroxy- oder Alkoxygruppe substituierte C₂₋₄-Alkylgruppe, und
R_{d} eine C₁₋₆-Alkylgruppe bedeuten, die durch eine Gruppe ausgewählt aus den Gruppen (a) bis (j) substituiert ist:
(a) eine Gruppe der Formel -C(=NH)NH₂,
(b) eine Carboxy-, Alkoxycarbonyl-, Carboxymethylaminocarbonyl-, Aminocarbonyl-, Alkylaminocarbonyl-, Alkylcarbonylamino-, Dialkylaminocarbonyl-, Arylaminocarbonyl-, N-Alkyl-arylaminocarbonyl-, Aralkylaminocarbonyl-, N-Alkyl-aralkylaminocarbonyl- oder Cyanogruppe,
(c) eine Hydroxy-, Amino-, Alkoxy-, Alkylamino-, Dialkylamino-, Alkylcarbonylamino-, N-Alkyl-alkylcarbonylamino-, Alkoxycarbonylamino-, Alkoxyacetylamino-, Dialkylaminoacetylamino-, N-Alkyl-alkoxycarbonylamino-, Alkylsulfonylamino-, N-Alkyl-alkylsulfonylamino-, Arylamino-, Naphthylamino-, Aralkylamino-, Diaralkylamino-, N-Alkyl-aralkylamino-, Alkylsulfenylgruppe,
(d) eine Nitro-2-pyridyl-amino-gruppe,
(e) eine durch einen, zwei oder drei Arylreste substituierte Methoxygruppe,
(f) eine 4- bis 7-gliedrige Alkyleniminogruppe, wobei in den vorstehend erwähnten 6- bis 7-gliedrigen Alkyleniminogruppen jeweils eine Methylengruppe in 4-Stellung durch ein Sauerstoff- oder Schwefelatom, durch eine Imino-, N-Alkyl-imino-, N-(Hydroxy-C₂₋₄-alkyl)-imino- oder N-(Amino-C₂₋₄-alkyl)-iminogruppe ersetzt sein kann, und zusätzlich in den vorstehend erwähnten 5- bis 7-gliedrigen Alkyleniminogruppen jeweils eine zu den Stickstoffatomen benachbarte Methylengruppe durch eine Carbonylgruppe ersetzt sein kann,
(g) eine durch eine Dialkylaminoalkylgruppe substituierte 1-Piperidinylgruppe,
(h) eine 2-Aza-bicyclo[2.2.1]hept-5-en-2-yl-gruppe,
(i) eine 5- bis 7-gliedrige (Alkylenimino)carbonylgruppe, wobei in den vorstehend erwähnten 6- bis 7-gliedrigen Alkyleniminogruppen eine Methylengruppe in 4-Stellung durch ein Sauerstoff- oder Schwefelatom, durch eine Imino- oder N-Alkyl-iminogruppe ersetzt sein kann, und
(j) eine (R₈R₉)CONR₇-Gruppe, in der
R₇, R₈ und R₉, die gleich oder verschieden sein können, jeweils ein Wasserstoffatom oder eine Methylgruppe oder
R₇ und R₈ zusammen eine n-C₂₋₃-Alkylengruppe und R₉ ein Wasserstoffatom oder eine Methyl- oder 4-Pyridylgruppe oder
R₇ und R₈ ein Wasserstoffatom und R₉ eine Aryl-C₁₋₂-alkyl- oder Phenylgruppe darstellen,
eine in 2-, 3-, oder 4-Stellung durch eine Hydroxy-, Amino-, Alkylamino-, Dialkylamino-, Aminomethyl-, Hydroxymethyl-, Alkoxycarbonyl-, Aminocarbonyl-, Alkylaminocarbonyl-, Dialkylaminocarbonyl- oder Carboxygruppe substituierte Cyclohexylgruppe,
eine in 4-Stellung durch eine Carboxyalkylgruppe substituierte Cyclohexylgruppe,
eine in 2-Stellung durch eine 2-Amino-1-ethylthio-, 2-Hydroxy-1-ethoxy-, 2-(2-Amino-1-ethoxy)-1-ethoxy- oder 2-(2-Hydroxy-1-ethoxy)-1-ethoxy-gruppe substituierte Ethylgruppe,
eine in 3-Stellung durch eine 3-Amino-1-propoxy- oder 2-(3-Amino-1-propoxy)-1-ethoxy-gruppe substituierte Propylgruppe,
eine durch eine C₅₋₆-Cycloalkylgruppe substituierte C₁₋₂-Alkylgruppe, wobei der Cycloalkylteil durch eine Hydroxy-, Aminomethyl-, Dimethylaminomethyl-, 2-Carboxyethyl- oder tert.-Butyloxycarbonylaminomethylgruppe substituiert ist oder wobei im Cycloalkylteil eine Methylengruppe durch ein Sauerstoffatom, eine N-Alkyl-imino- oder N-(2-Dialkylaminoacetyl)iminogruppe ersetzt ist,
eine 4-Piperidinyl-methylgruppe, die in 1-Stellung durch eine Alkyl- oder Aralkylgruppe und zusätzlich in der 4-Stellung durch eine Hydroxygruppe substituiert ist und bei der zusätzlich jeweils ein Wasserstoffatom in Position 2 und 6 des Piperidinyl-Gerüsts zusammen durch eine Ethylenbrücke ersetzt sind,
eine 3-Pyrrolidinyl- oder eine 3- oder 4-Piperidinylgruppe, die jeweils in 1-Stellung durch eine Alkyl-, Aralkyl- oder Arylsulfonylgruppe substituiert ist,
eine 4-Piperidinylgruppe, die in 1-Stellung durch eine Alkyl-, Aralkyl- oder Arylgruppe und zusätzlich in 4-Position durch eine Carboxygruppe substituiert ist,
eine Aralkylgruppe, die im Arylteil durch eine Hydroxy-, Aminosulfonyl-, Carboxy-, Nitro-, Amino-, Aminomethyl-, 2-Amino-1-ethyl-, Alkoxycarbonyl-, 4-Hydroxyphenyl- oder 2H-pyridazin-3-on-6-yl-gruppe substituiert ist,
eine durch eine 3-Hydroxy-1,3-dihydro-indol-2-on-3-yl- oder 2-Aminocarbonyl-1,3-dihydro-isoindol-5-yl-gruppe substituierte Methylgruppe,
eine im Arylteil durch eine 3-Carboxy-1-propylgruppe substituierte 2-Indanylgruppe,
eine durch eine 1*H*-2-Benzimidazolyl- oder 4-Amino-3,5-dichlorphenylgruppe substituierte Alkylgruppe,
eine Aralkylgruppe, die im Alkylteil durch eine Hydroxy-, Amino-, Alkylamino-, Dialkylamino-, Aminocarbonyl-, Alkylaminocarbonyl-, Dialkylaminocarbonyl-, Aralkylaminocarbonyl-, Carboxy- oder Cyanogruppe substituiert ist und gegebenenfalls zusätzlich im Arylteil durch ein oder zwei Fluor-, Chlor- oder Bromatome oder eine oder zwei Hydroxy- oder Alkoxygruppen substituiert ist, wobei die Substituenten gleich oder verschieden sein können,
eine durch eine Carboxygruppe und zusätzlich zwei Phenylgruppen substituierte Alkylgruppe,
eine durch eine Carboxygruppe und zusätzlich durch eine Hydroxy-, Aminocarbonyl-, 1*H-*Imidazol-4-yl- oder Benzyloxycarbonylaminogruppe substituierte C₂₋₆-Alkylgruppe,
eine durch eine Alkoxycarbonylgruppe und zusätzlich durch eine Pyridylgruppe substituierte Alkylgruppe,
eine durch eine Hydroxygruppe und zusätzlich durch eine Amino-, Alkylamino-, Dialkylamino-, Hydroxy-, Alkoxy-, 1-Pyrrolidinyl-, 1-Piperidinyl- oder Morpholinogruppe substituierte C₃₋₆-Alkylgruppe,
eine Aralkylgruppe, die im Arylteil durch eine Alkoxy- und zusätzlich durch eine Carboxy- oder Hydroxygruppe substituiert ist,
eine durch eine 2-Pyridyl-, 3-Pyridyl-, 4-Pyridyl-, 2-Pyrazinyl-, 3-Chlor-5-trifluormethyl-2-pyridyl-, 1-Methyl-1*H*-pyrrol-2-yl-, 1*H*-Pyrazol-4-yl-, 4-Ethoxycarbonyl-1*H*-pyrazol-5-yl-, 1*H-*Imidazol-1-yl-, 1*H*-Imidazol-4-yl-, 1*H*-Indol-3-yl-, 6-Methoxy-1*H*-benzimidazol-2-yl-gruppe substituierte Alkylgruppe,
eine in 5-Stellung durch eine Alkoxycarbonylgruppe substituierte 1-Pentylgruppe, die zusätzlich in 5-Stellung durch eine Amino-, Alkylcarbonylamino-, Arylcarbonylamino-, Arylsulfonylamino-, Alkoxycarbonylamino- oder Aralkoxycarbonylaminogruppe substituiert ist,
wobei, soweit nichts anderes erwähnt wurde, die vorstehend erwähnten Alkyl-, Alkylen- und Alkoxyteile jeweils 1 bis 4 Kohlenstoffatome enthalten,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
wobei, sofern nichts anderes erwähnt wurde, jedes Kohlenstoffatom in den vorstehend erwähnten Alkyl-, Alkylen- oder Cycloalkylenteilen, das an ein Stickstoff-, Sauerstoff- oder Schwefelatom gebunden ist, an kein weiteres Halogen-, Stickstoff-, Sauerstoff- oder Schwefelatom gebunden sein kann.

4. Verbindungen der Formel I nach einem der Ansprüche 1 bis 3, wobei
R_{b} eine 1-Naphthylgruppe oder eine gegebenenfalls in 5-Position durch eine Carboxy-Gruppe substituierte 2-Naphthylgruppe,
eine 1,3-Dihydro-2-oxo-indol-6-ylgruppe, Benzotriazol-5-yl-, Benzimidazol-5-yl, Indazol-5-yl-, Indazol-6-yl- oder 1-Methyl-1*H*-indazol-6-ylaminogruppe,
eine gegebenenfalls in 4-Position des Phenylteils durch ein Fluor-, Chlor- oder Bromatom, eine Cyano-, Propyl-2-sulfonyl-, Aminosulfonyl-, Methylaminosulfonyl-, Dimethylaminosulfonyl-, Morpholinosulfonyl-, N-Methylpiperazinosulfonyl-, Homopiperazinosulfonyl-, 2,6-Dimethylpiperazin-4-yl-, 2-Aminopyridyl-N-sulfonyl-, Carboxy-, Piperidinomethyl-, 1,2,4,5-Tetrahydro-benzo[*d*]azepin-3-yl-methyl-, 2-Carboxy-1-ethyl-, Phenylaminocarbonyl-, Benzylaminocarbonyl-, C₁₋₃-Alkylsulfonyl-, Aminocarbonyl-, Methoxycarbonyl-, (N-Methyl-N-methylsulfonyl)amino -, Diethylaminomethyl-, 3-Diethylamino-1-propyloxy-, Morpholino-, 4-Methyl-1-piperazinyl-, 2*H*-Tetrazol-5-yl-, 1*H*-Imidazol-4-yl, oder Nitrogruppe substituierte Phenylgruppe,
eine in 3-Position des Phenylteils durch ein Chlor- oder Bromatom, eine Cyano-, Aminocarbonyl-, Carboxy-, Ethoxycarbonyl-, oder Nitrogruppe oder durch eine Gruppe der Formel
-CH₂-NH-(CH₂)ᵣ-CₛFₛ₊₁,
worin r 1 oder 2 und s 1, 2 oder 3 bedeutet,
substituierte Phenylgruppe,
eine 3,4-Dichlorphenyl-, 3,5-Dichlorphenyl-, 4-Amino-3,5-dichlorphenyl-, 3-Chlor-4-fluorphenyl-, 4-Chlor-3-methylphenyl-, 4-Chlor-3-trifluormethylphenyl-, 4-Brom-3-chlorphenyl-oder 3-Hydroxy-4-methylphenylgruppe, oder
eine Gruppe der Formel 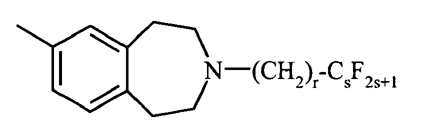 worin r 1 oder 2 und s 1, 2 oder 3 bedeutet,
die Gruppe R_{c}NR_{d}
eine in 2-Stellung durch eine Hydroxymethyl-, 1-Pyrrolidinylmethyl- oder 2-Ethoxycarbonyl-1-ethyl-gruppe substituierte 1-Pyrrolidinylgruppe,
eine in 3-Stellung durch eine Amino-, Acetylamino-, N-Methyl-acetylamino oder Tertiärbutyloxycarbonylamino substituierte 1-Pyrrolidinylgruppe,
eine 4-Carboxy-3-thiazolidinyl-, eine 7-Methyl-2,7-diaza-spiro[4.4]non-2-yl- oder eine 5-Hydroxy-2-methyl-2,8-diaza-spiro[5.5]undec-8-yl-Gruppe,
eine Morpholino- oder S-Oxido-thiomorpholinogruppe
eine in 2-Stellung durch eine Ethoxycarbonyl-, Hydroxymethyl-, 3-Hydroxypropyl-, 3-Diethylamino-1-propyl- oder 2-(2-Diethylaminoethoxy)-1-ethyl-gruppe substituierte 1-Piperidinylgruppe,
eine in 3-Stellung durch eine Hydroxy-, Hydroxymethyl-, 3-Diethylamino-1-propyl-, Aminocarbonyl-, Dimethylaminocarbonyl-, Carboxy-, 1-Pyrrolidinylmethyl-, 4-(1-Pyrrolidinyl)-1-butyl-, Methoxycarbonylmethyl- oder Acetylaminomethylgruppe substituierte 1-Piperidinylgruppe,
eine in 4-Stellung durch eine Ethoxycarbonyl-, 3-Hydroxypropyl-, Hydroxy-, Aminomethyl-, 2-(2-Diethylaminoethoxy)-1-ethyl-, 2-Carboxy-1-ethyl-, N-(2-Methoxycarbonyl-1-ethyl)-N-methylamino-, 2-(N-(Dimethylaminocarbonylmethyl-)-N-methyl-amino)-1-ethyl, N-Acetyl-N-methylaminomethyl-, 8-Methoxy-3,4-dihydro-1*H*-quinazolin-2-on-3-yl-, 1-Piperidinyl-, 3-Hydroxy-1-piperidinyl- oder 4-Ethoxycarbonyl-1-piperidinylgruppe substituierte 1-Piperidinylgruppe,
eine 3,5-Dimethyl-1-piperazinyl-, 1,4,6,7-Tetrahydro-imidazo[4,5-c]pyridin-5-yl-, 2-Methyl-1,4,6,7-tetrahydro-imidazo[4,5-c]pyridin-5-yl-, 1,4,5,6,7,8-Hexahydro-imidazo[4,5-d]azepin-6-yl-, 2-Methyl-1,4,5,6,7,8-hexahydro-imidazo[4,5-d]azepin-6-yl-, 3-Phenyl-azepan-4-on-1-yl- oder 4-Carboxy-4-phenyl-1-piperidinylgruppe,
eine 1-Piperazinylgruppe, die in 4-Stellung gegebenenfalls durch eine Methyl-, Acetyl-, Benzyloxycarbonyl-, 2-Pyridyl-, 2-Pyrimidinyl-, 2-Nitrophenyl-, 3-Methoxyphenyl-, 4-Cyanophenyl-, 3,4-Dimethoxyphenyl-, 4-[Bis-(4-methoxy-phenyl)]-methyl-, 8-Methyl-8-aza-bicyclo[3.2.1]oct-3-yl-, Morpholinocarbonylmethyl-, 2-Amino-1-ethyl- oder 3-Hydroxy-1-propylgruppe substituiert ist,
eine 1-Homopiperazinylgruppe, die in 4-Stellung gegebenenfalls durch eine Methylgruppe substituiert ist,
eine 3-Hydroxy-8-aza-bicyclo[3.2.1]oct-8-ylgruppe,
bedeutet, oder
R_{c} ein Wasserstoffatom oder eine Methyl-, Ethyl-, 2-Methoxyethyl-, 2-Hydroxyethyl-, i-Propyl, n-Propyl-, n-Butyl-, Benzyl- oder 3-Pyridylmethylgruppe bedeutet, und
R_{d} eine durch eine Gruppe der Formel -C(=NH)NH₂ oder eine Cyano-, Carboxyl-, Ethoxycarbonyl-, Aminocarbonyl-, C₁₋₂-Alkylcarbonylamino-2,2-dimethyl-ethylgruppe, C₁₋₂-Alkylcarbonylamino-1,1-dimethyl-ethylgruppe, C₁₋₂-Alkylcarbonylamino-2,2-dimethyl-propylgruppe, Carboxymethylaminocarbonyl-, 1-Hydroxy-1-cyclohexyl-, Aminomethylcyclohexyl-, 3-Hydroxy-8-methyl-8-aza-bicyclo[3.2.1]oct-3-yl-, 3-Hydroxy-1,3-dihydro-indol-2-on-3-yl-, 2-Aminocarbonyl-1,3-dihydro-isoindol-5-yl-, 2-Tetrahydrofuryl-, 1-Ethyl-2-pyrrolidinyl-, 1*H*-Imidazol-4-yl-, 1-Methyl-4-piperidinyl-, 1-(2-Dimethylaminoacetyl)-4-piperidinyl-, 2-Pyridyl-, 3-Pyridyl-, 4-Pyridyl-, 2-Pyrazinyl-, 3-Chlor-5-trifluormethyl-2-pyridyl-, 4-Ethoxycarbonyl-1*H*-pyrazol-5-yl-, 2-Carboxyphenyl-, 3-Carboxyphenyl-, 2-Hydroxyphenyl-, 4-Hydroxyphenyl-, 2-Nitrophenyl-, 3-Nitrophenyl-, 4-Nitrophenyl-, 3-Aminophenyl-, 4-Aminophenyl-, 4-(Aminosulfonyl)phenyl-, 4'-Hydroxybiphenyl-, 4-(Aminomethyl)phenyl- oder 4-Hydroxy-3-methoxyphenyl-gruppe substituierte Methylgruppe,
eine durch eine Carboxygruppe substituierte C₂₋₅-Alkylgruppe,
eine durch eine Hydroxy-, Amino- oder Dimethylaminogruppe substituierte C₂₋₅-Alkylgruppe, mit der Maßgabe, dass die Heteroatome der vorstehend genannten Substituenten von dem Stickstoffatom der R_{c}NR_{d}-Gruppe durch mindestens zwei Kohlenstoffatome getrennt sind,
eine im Methylenteil durch eine Carboxy- oder Cyanogruppe substituierte Benzylgruppe,
eine durch eine Carboxygruppe und eine 4-Hydroxyphenylgruppe substituierte Methylgruppe,
eine in 1-Stellung durch eine Methoxycarbonyl- oder eine 1*H*-Benzimidazol-2-ylgruppe substituierte Ethylgruppe,
eine in 2-Stellung durch eine Methoxy-, Diphenylmethoxy-, Methylthio-, Methylamino-, Diethylamino-, Diisopropylamino-, Acetylamino-, N-Methylacetylamino-, 2-Methoxyacetylamino-, 2-Dimethylaminoacetylamino-, Isopropylcarbonylamino-, 2-Methyl-propylcarbonylamino-, Phenyl-acetylamino-, *tert*.-Butyloxycarbonylamino-, Methylsulfonylamino-, Benzoylamino-, Phenylamino-, 1-Naphthylamino-, 4-Nitro-2-pyridylamino-, Cyano-, Ethoxycarbonyl-, Aminocarbonyl-, Methylaminocarbonyl-, Dimethylaminocarbonyl-, 2-Hydroxy-1-ethoxy-, 2-(2-Amino-1-ethoxy)-1-ethoxy-, 2-(2-Hydroxy-1-ethoxy)-1-ethoxy-, 2-Amino-1-ethylthio-, 1-Methyl-2-pyrrolidinyl-, 1-Pyrrolidinyl-, 2-Oxo-pyrrolidin-1-yl-, 1-Piperidinyl-, 2-Oxo-piperidin-1-yl-, Morpholino-, 4-(2-Hydroxyethyl)-1-piperazinyl-, 2-(2-Dimethylaminoethyl)-1-piperidinyl-, 4-Methyl-1-piperazinocarbonyl-, 3-Carboxy-2-methoxy-phenyl-, 2-Hydroxyphenyl-, 3-Hydroxyphenyl-, 4-Hydroxyphenyl-, 4-(Aminosulfonyl)phenyl-, 4-Nitrophenyl- , 3-Methoxycarbonylphenyl-, 2-(2-Amino-1-ethyl)phenyl-, 4-Pyridyl-, 1*H*-Imidazol-1-yl-,1*H*-Imidazol-4-yl-, 1*H*-Pyrazol-4-yl-, 1-Methyl-1*H-*pyrrol-2-yl-, 1*H*-Indol-3-yl-, 6-Methoxy-1*H*-benzoimidazol-2-yl-, 4-(2*H*-pyridazin-3-on-6-yl)-phenyl- oder Imidazolidin-2-on-1-ylgruppe substituierte Ethylgruppe,
eine in 1-Stellung durch eine Carboxygruppe und in 2-Stellung zusätzlich durch eine Hydroxy-, Aminocarbonyl-, 2-Chlorphenyl-, 4-Chlorphenyl-, 1*H*-Imidazol-4-yl- oder 4-Hydroxyphenylgruppe substituierte Ethylgruppe,
eine in 1-Stellung durch eine Aminocarbonylgruppe und in 2-Stellung zusätzlich durch eine 4-Methoxyphenylgruppe substituierte Ethylgruppe,
eine in 1-Stellung durch eine 4-Phenyl-1-butylaminocarbonylgruppe und in 2-Stellung zusätzlich durch eine Phenylgruppe substituierte Ethylgruppe,
eine in 2-Stellung durch eine Hydroxygruppe und in 2-Stellung zusätzlich durch eine Phenyl-, 3-Hydroxyphenyl-, 4-Hydroxyphenyl- oder 4-Hydroxy-3-methoxyphenylgruppe substituierte Ethylgruppe,
eine in 1-Stellung durch eine Phenylgruppe und in 2-Stellung zusätzlich durch eine Hydroxy- oder Carboxygruppe substituierte Ethylgruppe,
eine in 1-Stellung durch eine 3-Pyridylgruppe und in 2-Stellung zusätzlich durch eine Ethoxycarbonylgruppe substituierte Ethylgruppe,
eine in 1-Stellung durch eine Carboxygruppe und in 2-Stellung zusätzlich durch zwei Phenylgruppen substituierte Ethylgruppe,
eine in 2-Stellung durch eine Hydroxygruppe und in 3-Stellung zusätzlich durch eine Amino-, Hydroxy- oder Morpholinogruppe substituierte n-Propylgruppe,
eine in 3-Stellung durch eine Methoxy-, Isopropylamino-, Methylamino-, Diethylamino-, Dibenzylamino-, 1-Pyrrolidinyl-, 1-Piperidinyl-, Morpholino-, 4-Methyl-1-piperazinyl-, tert.-Butyloxycarbonylamino-, 2-Oxo-1-pyrrolidinyl-, 2-Oxo-piperidin-1-yl-, Ethoxycarbonyl-, 4-Pyridyl-, 4-Amino-3,5-dichlorphenyl-, 3-Amino-1-propoxy-, 2-(3-Amino-1-propoxy)-1-ethoxy-, 1*H*-Imidazol-1-yl-, 2-Aza-bicyclo[2.2.1]hept-5-en-2-yl-, 4-(3-Amino-1-propyl)-1-piperazinyl-oder 2-Diethylaminomethyl-1-piperidinyl-gruppe substituierte n-Propylgruppe,
eine in 4-Stellung durch 4-Hydroxyphenylgruppe substituierte n-Butylgruppe,
eine in 4-Stellung durch eine Dimethylaminogruppe und in 2-Stellung zusätzlich durch eine Phenylgruppe substituierte n-Butylgruppe,
eine in 3-Position durch eine Phenylaminocarbonylamino- oder eine 1-(4-Pyridyl)-3-imidazolin-2-on-3-yl substituierte 2-Methyl-2-butylgruppe,
eine in 1-Stellung durch eine Carboxygruppe und zusätzlich in 5-Stellung durch eine Benzyloxycarbonylaminogruppe substituierte n-Pentylgruppe,
eine in 5-Stellung durch eine Methoxycarbonylgruppe und in 5-Stellung zusätzlich durch eine Acetylaminogruppe substituierte 1-Pentylgruppe,
eine in 6-Stellung durch eine Hydroxy-, Amino-, tert.-Butyloxycarbonylamino- oder N-Methyl-N-phenethylaminogruppe substituierte n-Hexylgruppe,
eine in 2-Stellung durch eine Hydroxy-, Amino-, Dimethylamino- oder Hydroxymethylgruppe substituierte Cyclohexylgruppe
eine in 3-Stellung durch eine Amino- oder Carboxygruppe substituierte Cyclohexylgruppe
eine in 4-Stellung durch eine Hydroxy-, Amino-, Carboxy-, 2-Carboxyethyl-, 3-Carboxypropyl-, Methoxycarbonyl- oder Dimethylaminogruppe substituierte Cyclohexylgruppe,
eine in 3-Stellung des Cyclohexylteils durch eine Aminomethyl- oder eine tert.-Butyloxycarbonylaminomethylgruppe substituierte Cyclohexylmethylgruppe,
eine in 4-Stellung des Cyclohexylteils durch eine Aminomethyl-, Dimethylaminomethyl- oder 2-Carboxyethylgruppe substituierte Cyclohexylmethylgruppe,
eine in 1-Stellung durch eine Methyl-, Benzyl- oder Phenylsulfonylgruppe substituierte 4-Piperidinylgruppe,
eine 1-Methyl-4-carboxy-4-piperidinylgruppe,
eine 1-Ethyl-3-piperidinyl-, 1-Benzyl-3-pyrrolidinyl- oder 5-(3-Carboxy-1-propyl)-indan-2-yl)-gruppe,
bedeutet,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

5. Verbindungen der Formel I nach einem der Ansprüche 1 bis 4, worin:
R_{b} eine gegebenenfalls in 4-Position des Phenylteils durch ein Fluor-, Chlor- oder Bromatom, eine Cyano-, Propyl-2-sulfonyl-, Aminosulfonyl-, Dimethylaminosulfonyl-, Carboxy-, Piperidinomethyl-, 1,2,4,5-Tetrahydro-benzo[*d*]azepin-3-yl-methyl-, 2-Carboxy-1-ethyl-, Phenylaminocarbonyl-, Benzylaminocarbonyl-, C₁₋₃-Alkylsulfonyl-, Aminocarbonyl-, Methoxycarbonyl-, (N-Methyl-N-methylsulfonyl)amino -, Diethylaminomethyl-, 3-Diethylamino-1-propyloxy-, Morpholino-, 4-Methyl-1-piperazinyl-, 2-H-Tetrazolo-5-yl-, 1-H-Imidazol-4-yl oder Nitrogruppe oder eine Gruppe der Formel
-CH₂-NH-CH₂-CₛFₛ₊₁,
worin s 1 oder 2 bedeutet,
substituierte Phenylgruppe, oder eine in 3-Position des Phenylteils durch ein Chlor- oder Bromatom, eine Cyano-, Aminocarbonyl-, Carboxy-, Ethoxycarbonyl-, oder Nitrogruppe substituierte Phenylgruppe, oder eine 3,4-Dichlorphenyl-, 3,5-Dichlorphenyl-, 4-Amino-3,5-dichlorphenyl-, 3-Chlor-4-fluorphenyl-, 4-Chlor-3-methylphenyl-, 4-Chlor-3-trifluormethylphenyl-, 4-Brom-3-chlorphenyl-, 3-Hydroxy-4-methylphenylgruppe, Benzotriazol-5-yl-, Benzimidazol-5-yl, Indazol-5-yl- oder Indazol-6-yl- oder eine Gruppe der Formel 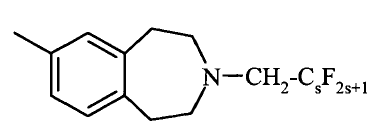 worin s 1 oder 2 bedeutet,
bedeutet.

6. Verbindungen der Formel I nach einem der Ansprüche 1 bis 5, worin:
die Gruppe R_{c}NR_{d}
ausgewählt ist aus den folgenden Gruppen:
2-Amino-1-ethylamino, 2-Acetylamino-ethylamino, 2-Aminocarbonyl-1-ethylamino, 2-Methoxy-1-ethylamino, 2-Morpholino-1-ethylamino, 3-Aminopropyl-amino, 1-Carboxy-2-propylamino, 4-Aminobutylamino, 5-Hydroxy-1-pentylamino, 3-(3-Aminopropoxy-1-propylamino, 2-(3-Hydroxyphenyl)-1-ethyl-amino, 2-(4-Hydroxy-3-methoxy-phenyl)-2-hydroxy-1-ethylamino, 2-(2-(2-Amino-1-ethyl)-phenyl)-1-ethyl-amino, 4-Hydroxy-cyclohexylamino, 3-Amino-cyclohexylamino, 4-Aminomethyl-cyclohexylmethylamino, 4-Dimethylamino-cyclohexylamino, 1-Methyl-piperidin-4-yl-methylamino, N-(4-Methyl-piperidin-4-yl)-N-methyl-amino, 3-(2-Oxo-pyrrolidin-1-yl)-propyl-1-amino, 1,4,6,7-Tetrahydro-imidazo[4,5-c]pyridin-5-yl, 2-Hydroxymethyl-pyrrolidin-1-yl, 4-Aminomethyl-piperidin-1-yl, 3-Hydroxymethyl-piperidin-1-yl, 3-Acetylaminomethyl-piperidin-1-yl, 4-(N-Acetyl-N-methyl-aminomethyl)-piperidin-1-yl, 3-(4-(Pyrrolidin-1-yl)butyl)-piperidin-1-yl, 3-(2-Aza-bicyclo[2.2.1]hept-5-en-2-yl)-propylamino, 7-Methyl-2,7-diaza-spiro[4.4]non-2-yl,

7. Physiologisch verträgliche Salze der Verbindungen der Formel I nach einem der Ansprüchen 1 bis 6.

8. Verbindungen der Formel I nach einem der Ansprüche 1 bis 6 zur Verwendung als Arzneimittel.

9. Verfahren zur Herstellung der Verbindungen der Formel I gemäß einem der Ansprüche 1 bis 6, **dadurch gekennzeichnet, dass**
a. eine Verbindung der allgemeine Formel 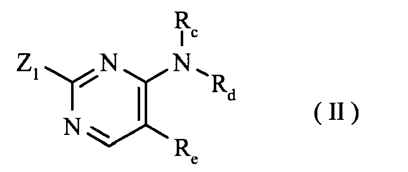 in der
R_{c} bis Rₑ wie in den Ansprüchen 1 bis 6 erwähnt definiert sind und
Z₁ eine Austrittsgruppe darstellt, mit einem Amin der allgemeinen Formel
H-(RₐNR_{b}) (III)
worin
Rₐ und R_{b} wie in den Ansprüchen 1 bis 6 erwähnt definiert sind, umgesetzt wird; oder
b. eine Verbindung der allgemeine Formel IV 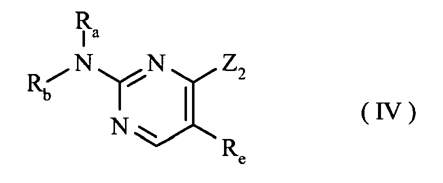 in der
Rₐ, R_{b} und Rₑ, wie in den Ansprüchen 1 bis 6 erwähnt definiert sind, und
Z₂ eine Austrittsgruppe darstellt, mit einem Amin der allgemeinen Formel
H-(R_{c}NR_{d}) (V)
worin
R_{c} und R_{d} wie in den Ansprüchen 1 bis 6 erwähnt definiert sind, umgesetzt wird.

10. Verwendung von Verbindungen der Formel I gemäß einem der Ansprüche 1 bis 6 zur Herstellung eines Medikamentes zur Therapie und zur Vorbeugung von Krankheiten, die durch exzessive oder anomale Zellproliferation charakterisiert sind.

11. Pharmazeutische Zusammensetzung **gekennzeichnet durch** einen Gehalt einer oder mehrer Verbindungen gemäß einem der Ansprüche 1 bis 6.

## Claims

1. Trisubstituted pyrimidines of formula I, 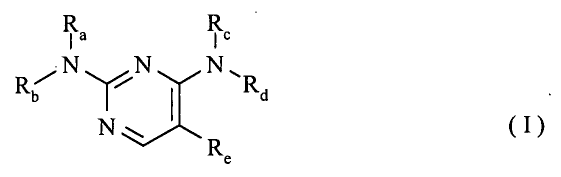 wherein
**R**ₐ denotes a hydrogen atom or an alkyl group,
**R**_{b} denotes a phenyl group optionally substituted by the groups R₁ to R₃, while
**R₁** and **R₂** in each case independently of one another denote
a fluorine, chlorine, bromine or iodine atom, or
a C₁₋₂-alkyl or hydroxy group,
a C₃₋₇-cycloalkyl or C₄₋₇-cycloalkoxy group which may be substituted in each case by one or two alkyl groups or by an aryl group,
a C₂₋₅-alkenyl group optionally substituted by an aryl group,
a C₂₋₅-alkynyl group optionally substituted by an aryl group
an aryl, aryloxy, aralkyl, aralkoxy, alkylsulphenyl, alkylsulphinyl, alkylsulphonyl, alkylsulphonyloxy, trifluoromethylsulphenyl, trifluoromethylsulphinyl, trifluoromethylsulphonyl, arylsulphenyl, arylsulphinyl, arylsulphonyl, aralkylsulphenyl, aralkylsulphinyl or aralkylsulphonyl group,
a methyl or methoxy group substituted by 1 to 3 fluorine atoms,
a C₂₋₄-alkyl or C₂₋₄-alkoxy group substituted by 1 to 5 fluorine atoms,
a nitro, amino, alkylamino, dialkylamino, C₃₋₇-cycloalkylamino,
N-alkyl-C₃₋₇-cycloalkylamino, arylamino, N-alkyl-arylamino, aralkylamino or
N-alkyl-aralkylamino group,
a 4- to 7-membered alkyleneimino group optionally substituted by 1 to 4 alkyl groups, while in the abovementioned 5- to 7-membered alkyleneimino groups in each case one or two methylene groups adjacent to the nitrogen atom may each be replaced by a carbonyl group or in the abovementioned 6- to 7-membered alkyleneimino groups a methylene group in the 4-position may be replaced by an oxygen or sulphur atom, by a sulphinyl, sulphonyl, imino, N-alkyl-imino, N-alkylcarbonyl-imino, N-alkylsulphonyl-imino, N-arylcarbonyl-imino, N-arylsulphonyl-imino, N-aryl-imino or N-aralkyl-imino group,
an (alkyleneimino)carbonyl or (alkyleneimino)sulphonyl group with in each case 4 to 7 cyclic atoms in the alkyleneimino moiety, optionally substituted by 1 to 4 alkyl groups, while in the abovementioned 6- to 7-membered alkyleneimino moieties in each case a methylene group in the 4-position may be replaced by an oxygen or sulphur atom, by a sulphinyl, sulphonyl, imino, N-alkyl-imino, N-alkylcarbonyl-imino, N-alkylsulphonyl-imino, N-arylcarbonyl-imino, N-arylsulphonyl-imino, N-aryl-imino or N-aralkyl-imino group,
an alkylcarbonylamino, N-alkyl-alkylcarbonylamino, alkyl-sulphonylamino, N-alkyl-alkylsulphonylamino, arylcarbonylamino, N-alkyl-arylcarbonylamino, arylsulphonylamino, N-alkyl-arylsulphonylamino, aralkylcarbonylamino, N-alkyl-aralkylcarbonylamino, aralkylsulphonylamino, N-alkyl-aralkylsulphonylamino, perfluoroalkylsulphonylamino, N-alkyl-perfluoralkylsulphonylamino, alkylcarbonyl, arylcarbonyl, aralkylcarbonyl, aryl-hydroxymethyl, aralkyl-hydroxymethyl, carboxy, alkoxycarbonyl, aralkoxycarbonyl, aminocarbonyl, alkylaminocarbonyl, dialkylaminocarbonyl, arylaminocarbonyl, N-alkyl-arylaminocarbonyl, aralkylaminocarbonyl, N-alkyl-aralkylaminocarbonyl, N-hydroxy-aminocarbonyl, N-hydroxy-alkylaminocarbonyl, N-alkoxy-aminocarbonyl, N-alkoxy-alkylaminocarbonyl, cyano, azido, N-cyano-amino or N-cyano-alkylamino group,
a sulpho, alkoxysulphonyl, aminosulphonyl, alkylaminosulphonyl, dialkylaminosulphonyl, arylaminosulphonyl, pyridylaminosulphonyl, pyrimidinylaminosulphonyl, N-alkyl-arylaminosulphonyl, aralkylaminosulphonyl or N-alkyl-aralkylaminosulphonyl group, a phosphono, O-alkyl-phosphono, O,O'-denotes ialkyl-phosphono, O-aralkyl-phosphono or O,O'-diaralkyl-phosphono group,
a C₁₋₂ alkyl group substituted by **R₄,** wherein
**R₄** denotes a hydroxy, alkoxy, aryloxy, aralkoxy, amino, alkylamino, haloalkylamino, dialkylamino, alkylsulphenyl, alkylsulphinyl, alkylsulphonyl, arylsulphenyl, arylsulphinyl, arylsulphonyl, aralkylsulphenyl, aralkylsulphinyl, aralkylsulphonyl, carboxy, alkoxycarbonyl, aminocarbonyl, alkylaminocarbonyl, dialkylaminocarbonyl or cyano group, a 4- to 7-membered alkyleneimino group optionally substituted by 1 to 4 alkyl groups, while in the abovementioned 5- to 7-membered alkyleneimino groups one or two methylene groups adjacent to the nitrogen atom may be replaced in each case by a carbonyl group or in the abovementioned 6- to 7-membered alkyleneimino groups a methylene group in the 4-position may be replaced by an oxygen or sulphur atom, by a sulphinyl, sulphonyl, imino, N-alkyl-imino, N-alkylcarbonyl-imino, N-alkylsulphonyl-imino, N-arylcarbonyl-imino, N-arylsulphonyl-imino, N-aryl-imino or N-aralkyl-imino group, or
a 4- to 7-membered alkyleneimino group optionally substituted by 1 to 4 alkyl groups, while in the abovementioned 5- to 7-membered alkyleneimino groups in each case one or two methylene groups adjacent to the nitrogen atom may be substituted by a carbonyl group or in the abovementioned 6- to 7-membered alkyleneimino groups may be substituted by one or two hydroxy, alkoxy, aminocarbonyl, alkylaminocarbonyl, dialkylaminocarbonyl, amino, alkylamino and dialkylamino group,
an (alkyleneimino)carbonyl group optionally substituted by 1 to 4 alkyl groups with 4 to 7 cyclic atoms in the alkyleneimino moiety in each case, while in the abovementioned 6- to 7-membered alkyleneimino moieties in each case a methylene group may be replaced in the 4-position by an oxygen or sulphur atom, by a sulphinyl, sulphonyl, imino, N-alkyl-imino, N-alkylcarbonyl-imino, N-alkylsulphonyl-imino, N-arylcarbonyl-imino, N-arylsulphonyl-imino, N-aryl-imino or N-aralkyl-imino group, or
a group of formula 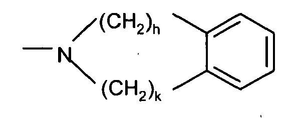 wherein
h and k, which may be identical or different, represent the numbers 1 to 3 or h denotes the number 0 and k denotes the number 2, 3 or 4, while additionally the above benzo moiety may be mono- or disubstituted by fluorine, chlorine, bromine or iodine atoms, by alkyl, trifluoromethyl, hydroxy, alkoxy, carboxy or cyano groups, while the substituents in each case may be identical or different, and the above saturated cyclic alkyleneimino moiety may be substituted by 1 or 2 alkyl groups,
**R₃** denotes a fluorine, chlorine or bromine atom, a C₁₋₂ alkyl, C₁₋₂ alkoxy or trifluoromethyl group, or
a 5 or 6-membered heterocyclic, aromatic ring with at least one nitrogen atom and optionally a sulphur or oxygen atom which may be substituted by one or two alkyl, aryl or aralkyl groups,
or
a sulpho, alkoxysulphonyl, aminosulphonyl, alkylaminosulphonyl, dialkylaminosulphonyl, arylaminosulphonyl, pyridylaminosulphonyl, pyrimidinylaminosulphonyl, N-alkyl-arylaminosulphonyl, aralkylaminosulphonyl or N-alkyl-aralkylaminosulphonyl group,
**R₂** together with **R₃,** if they are bound to adjacent carbon atoms, denote a methylenedioxy group optionally substituted by one or two alkyl groups, or
an n-C₃₋₆-alkylene group optionally substituted by one or two alkyl groups, wherein a methylene group may be replaced by an oxygen or sulphur atom, by a sulphinyl, sulphonyl, N-alkylcarbonyl-imino, N-alkylsulphonyl-imino, N-arylcarbonyl-imino or N-arylsulphonyl-imino group, or
a 1,3-butadiene-1,4-diylene group optionally substituted by one or two fluorine, chlorine, bromine or iodine atoms, by one or two hydroxy, alkyl, alkoxy, trifluoromethyl, carboxy, alkoxycarbonyl, aminocarbonyl, alkylaminocarbonyl, dialkylaminocarbonyl or cyano groups, while the substituents may be identical or different, or
a group of formula
-(CH₂)ₘ-N(**R₅**)-(CH₂)ₙ-,
wherein
the methylene groups of the cyclic alkyleneimino moieties thus formed may additionally be substituted by 1 or 2 alkyl groups,
**R₅** denotes a hydrogen atom or an alkyl, haloalkyl, aryl or aralkyl group, and
m and n, which may be identical or different, represent the numbers 1, 2 or 3, while in the alkyleneimino moieties thus formed one or two methylene groups adjacent to the nitrogen atom may be replaced in each case by a carbonyl group, or
m denotes the number 0 and n denotes the number 2, 3 or 4, while in the alkyleneimino moieties thus formed in each case the methylene group adjacent to the nitrogen atom may be replaced by a carbonyl group, or
R₂ together with R₃ denotes a group of formula-NH-C(=O)-(CH₂)-, -NH-C(=O)-(CH₂)₂, -NH-N=N, -NH-N=CH, -NH-CH=N-, -O-CH=N, -S-CH=N or -NH-CH=CH- and the tautomers of the ring systems defined by -NH-N=N, -NH-N=CH, -NH-CH=N-, while each hydrogen atom may be substituted by an alkyl, aryl or aralkyl group,
**R_{c}NR_{d}** denotes a 4- to 8-membered alkyleneimino group optionally substituted by 1 to 4 alkyl groups or 1 to 2 aryl groups, which is additionally substituted by the group **R₆,** while
**R₆** denotes a carboxy, alkoxycarbonyl, aminocarbonyl, alkylaminocarbonyl, dialkylaminocarbonyl, cyano, hydroxy, alkoxy, aryloxy, aralkoxy, alkylcarbonyloxy, arylcarbonyloxy, amino, alkylamino, hydroxy-C₂₋₄-alkylamino, dialkylamino, cyanamino, formylamino, N-(alkyl)-N-(hydroxy-C₂₋₄-alkyl)amino, bis(hydroxy-C₂₋₄-alkyl)-amino group, alkylsulphenyl, alkylsulphinyl, alkylsulphonyl, arylsulphenyl, arylsulphinyl, arylsulphonyl, aralkylsulphenyl, aralkylsulphinyl, aralkylsulphonyl, an alkylcarbonylamino, N-alkyl-alkylcarbonylamino, alkylsulphonylamino, N-alkyl-alkylsulphonylamino, arylcarbonylamino, N-alkyl-arylcarbonylamino, arylsulphonylamino, N-alkyl-arylsulphonylamino, aralkylcarbonylamino, N-alkyl-aralkylcarbonylamino, aralkylsulphonylamino, N-alkyl-aralkylsulphonylamino, alkoxycarbonylamino, N-alkyl-alkoxycarbonylamino, alkoxycarbonylalkylamino, N-(alkyl)-N-(alkoxycarbonylalkyl)-amino, aralkoxycarbonylamino or N-alkyl-aralkoxycarbonylamino group,
an (N**R₈R₉**)CON**R₇** or (N**R₈R₉**)SO2N**R₇**-group, wherein
**R₇, R₈** and **R₉,** which may be identical or different, in each case denote a hydrogen atom or an alkyl, aryl or pyridyl group, or **R₇** and **R₈** together denote an n-C₂₋₄-alkylene group and **R₉** denotes a hydrogen atom or an alkyl, aryl or pyridyl group,
an (alkyleneimino)carbonyl group optionally substituted by 1 to 4 alkyl groups with in each case 4 to 7 cyclic atoms in the alkyleneimino moiety, while in the abovementioned 6- to 7-membered alkyleneimino moieties in each case a methylene group in the 4-position may be replaced by an oxygen or sulphur atom, by a sulphinyl, sulphonyl, imino, N-alkyl-imino, N-alkylcarbonyl-imino, N-alkylsulphonyl-imino, N-aryl-imino or N-aralkyl-imino group, a 4- to 7-membered alkyleneimino group optionally substituted by 1 to 4 alkyl groups or a hydroxyalkyl group, while in the abovementioned 5- to 7-membered alkyleneimino groups in each case one or two methylene groups adjacent to the nitrogen atom may be replaced by a carbonyl group,
a 6 or 7-membered alkyleneimino group optionally substituted by 1 to 4 alkyl groups or a hydroxyalkyl group, while in each case a methylene group in the 4-position of the alkyleneimino moiety is replaced by an oxygen or sulphur atom, by a carbonyl, sulphinyl, sulphonyl, imino, N-alkylimino, N-alkylcarbonyl-imino, N-alkylsulphonyl-imino, N-arylimino or N-aralkyl-imino group and additionally in the alkyleneimino moiety of the abovementioned groups in each case one or two of the methylene groups adjacent to the nitrogen atoms-may be replaced by a carbonyl group,
a 4- to 7-membered alkyleneimino group substituted by a hydroxy, alkoxy, amino, alkylamino, dialkylamino, alkylcarbonylamino, N-alkyl-alkylcarbonylamino, alkylsulphonylamino, N-alkyl-alkylsulphonylamino, carboxy, alkoxycarbonyl, aminocarbonyl, alkylaminocarbonyl or dialkylaminocarbonyl group,
an alkyl group substituted by a carboxy, alkoxycarbonyl, aminocarbonyl, alkylaminocarbonyl or dialkylaminocarbonyl, cyano, hydroxy, alkoxy, aryloxy, amino, alkylamino, dialkylamino, alkylcarbonylamino, N-alkyl-alkylcarbonylamino, alkoxycarbonylamino, N-alkyl-alkoxycarbonylamino, alkylsulphonylamino, N-alkyl-alkylsulphonylamino, arylcarbonylamino, N-alkyl-arylcarbonylamino, arylsulphonylamino, N-alkyl-arylsulphonylamino, aminocarbonylalkylamino, N-(alkyl)-N-(aminocarbonylalkyl)-amino, alkylaminocarbonylalkylamino, N-(alkyl)-N-(alkylaminocarbonylalkyl)-amino, dialkylaminocarbonylalkylamino, N-(alkyl)-N-(dialkylaminocarbonylalkyl)-amino, dialkylaminoalkoxy, alkylsulphenyl, alkylsulphinyl, alkylsulphonyl, arylsulphenyl, arylsulphinyl or arylsulphonyl group,
an (alkyleneimino)alkyl group optionally substituted by 1 to 4 alkyl groups with in each case 4 to 7 cyclic atoms in the alkyleneimino moiety, while in the abovementioned 6- to 7-membered alkyleneimino moieties a methylene group in the 4-position may be replaced in each case by an oxygen or sulphur atom, by a sulphinyl, sulphonyl, imino, N-alkyl-imino or N-alkylcarbonyl-imino group,
an (alkyleneimino)carbonylalkyl group optionally substituted by 1 to 4 alkyl groups with in each case 4 to 7 cyclic atoms in the alkyleneimino moiety, while in the abovementioned 6- to 7-membered alkyleneimino moieties a methylene group in the 4-position may be replaced in each case by an oxygen or sulphur atom, by a sulphinyl, sulphonyl, imino or N-alkyl-imino group,
a (carboxyalkyl)oxy, (alkoxycarbonylalkyl)oxy, (aminocarbonylalkyl)oxy, (alkylaminocarbonylalkyl)oxy or (dialkylaminocarbonylalkyl)oxy group,
an [(alkyleneimino)carbonylalkyl]oxy group optionally substituted by 1 to 4 alkyl groups with in each case 4 to 7 cyclic atoms in the alkyleneimino moiety; while in the abovementioned 6- to 7-membered alkyleneimino moieties a methylene group in the 4-position may be replaced in each case by an oxygen or sulphur atom, by a sulphinyl, sulphonyl, imino or N-alkyl-imino group,
a C₅₋₇-cycloalkyl group wherein a methylene group is replaced by an oxygen or sulphur atom, by a sulphinyl, sulphonyl, imino or N-alkyl-imino, alkylcarbonylimino or alkylsulphonylimino group,
a 3,4-dihydro-1H-quinazolin-2-on-3-yl or 1H-benzimidazol-2-on-1-yl- group optionally substituted in the aryl moiety by one or two fluorine, chlorine, bromine or iodine atoms or one or two nitro, alkyl, alkoxy or cyano groups in each case, while the substituents may be identical or different,
or .
**R_{c}**N**R_{d}** denotes a 6- to 8-membered alkyleneimino group optionally substituted by 1 to 4 alkyl groups or by an aryl group, which may additionally be substituted by the group **R₆,** while in the abovementioned alkyleneimino groups a methylene group in the 4-position is replaced by an oxygen or sulphur atom, by a carbonyl, sulphinyl, sulphonyl, N-oxido-N-alkylimino or **R₁₀**N-group in each case, while
**R₁₀** denotes a hydrogen atom, an alkyl, hydroxy-C₂₋₄-alkyl, alkoxy-C₂₋₄-alkyl, amino-C₂₋₄-alkyl, alkylamino-C₂₋₄-alkyl, dialkylamino-C₂₋₄-alkyl, (hydroxy-C₂₋₄-alkoxy)-C₂₋₄-alkyl, aminocarbonylalkyl, alkylaminocarbonylalkyl, dialkylaminocarbonylalkyl, aryl, formyl, alkylcarbonyl, alkylsulphonyl, arylcarbonyl, arylsulphonyl, aralkylcarbonyl, aralkylsulphonyl, alkoxycarbonyl, aralkoxycarbonyl, cyano, aminocarbonyl, alkylaminocarbonyl or dialkylaminocarbonyl group, an amino-alkylcarbonyl, alkylamino-alkylcarbonyl, dialkylamino-alkylcarbonyl group,
an alkyl group substituted by one, two or three aryl groups, an 8-alkyl-8-aza-bicyclo[3.2.1]oct-3-yl group, an aryl or a 2-, 3- or 4-pyridyl group or 2-, 4- or 5-pyrimidinyl group
an (alkyleneimino)carbonyl or (alkyleneimino)carbonylalkyl group with 4 to 7 cyclic atoms in the alkyleneimino moiety in each case, while in the abovementioned 6- to 7-membered alkyleneimino moieties a methylene group in the 4-position may be replaced in each case by an oxygen or sulphur atom, by a sulphinyl, sulphonyl, imino, N-alkyl-imino, N-alkylcarbonyl-imino or N-aralkyl-imino group,
or
**R_{c}**N**R_{d}** denotes a 3-thiazolidinyl-group substituted in the 4-position by a carboxy, alkoxycarbonyl, aminocarbonyl, alkylaminocarbonyl, dialkylaminocarbonyl group, or
**R_{c}**N**R_{d}** denotes a 1-pyrrolidinyl group optionally substituted by 1 to 4 alkyl groups, wherein two hydrogen atoms on the carbon skeleton are replaced by a straight-chain alkylene bridge, this bridge containing 2 to 6 carbon atoms if the two hydrogen atoms are located on the same carbon atom, or 1 to 5 carbon atoms if the two hydrogen atoms are located on adjacent carbon atoms, or 2 to 4 carbon atoms if the two hydrogen atoms are located on carbon atoms separated by one atom, while the abovementioned 1-pyrrolidinyl groups are additionally substituted by the group **R₆,** which is as hereinbefore defined,
a 1-piperidinyl or 1-azacyclohept-1-yl group optionally substituted by 1 to 4 alkyl groups, wherein two hydrogen atoms on the carbon skeleton are replaced by a straight-chain alkylene bridge, this bridge containing 2 to 6 carbon atoms if the two hydrogen atoms are located on the same carbon atom, or 1 to 5 carbon atoms if the two hydrogen atoms are located on adjacent carbon atoms, or 1 to 4 carbon atoms if the two hydrogen atoms are located on carbon atoms separated by an atom, or 1 to 3 carbon atoms if the two hydrogen atoms are located on carbon atoms separated by two atoms, while the abovementioned 1-piperidinyl- and 1-azacyclohept-1-yl groups are additionally substituted by the group **R₆,** which is as hereinbefore defined ,
a 1-pyrrolidinyl group optionally substituted by 1 to 4 alkyl groups, wherein two hydrogen atoms in the 3 position are substituted by a -O-CH₂CH₂-O or -O-CH₂CH₂CH₂-O-group,
a 1-piperidinyl or 1-azacyclohept-1-yl group optionally substituted by 1 to 4 alkyl groups, wherein in the 3 position or in the 4 position two hydrogen atoms are substituted by a -O-CH₂CH₂-O or -O-CH₂CH₂CH₂-O-group in each case,
a 1-azetidinyl group optionally substituted by an alkyl group, wherein the two hydrogen atoms of a methylene group are replaced by a straight-chain C₄₋₆-alkylene bridge, while in each case a methylene group in the C₄₋₆-alkylene bridge is replaced by a **R₁₀**N- group, where **R₁₀** is as hereinbefore defined, while the bicyclic ring thus formed may additionally be substituted by a hydroxy, alkoxy, amino, alkylamino, dialkylamino, cyano, alkylcarbonylamino, alkylsulphonylamino, alkoxycarbonylamino, arylcarbonyl, carboxy, alkoxycarbonyl, aminocarbonyl, alkylaminocarbonyl or dialkylaminocarbonyl group,
a 1-pyrrolidinyl, 1-piperidinyl or 1-azacyclohept-1-yl group optionally substituted by 1 to 2 alkyl groups, wherein the two hydrogen atoms of a methylene group are replaced by a straight-chain C₃₋₆-alkylene bridge, while in each case a methylene group in the C₃₋₆-alkylene bridge is replaced by a **R₁₀**N-group, while **R₁₀** is as hereinbefore defined, while the bicyclic ring thus formed may additionally be substituted by a hydroxy, alkoxy, amino, alkylamino, dialkylamino, cyano, alkylcarbonylamino, alkylsulphonylamino, alkoxycarbonylamino, arylcarbonyl, carboxy, alkoxycarbonyl, aminocarbonyl, alkylaminocarbonyl or dialkylaminocarbonyl group,
a group of the structure 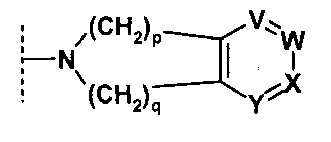
optionally substituted in the alkylene moieties by 1 or 2 alkyl groups,
wherein
p and q, which may be identical or different, represent the number 1 or 2, and
the unit -V=W-X=Y- denotes one of the groups (a), (b), (c), (d) or (e):
-N=C-C=C- (a),
-C=N-C=C- (b),
-C=N-N=C- (c),
-N=C-C=N- (d),
-N=C-N=C- (e),
or -V=W- taken together represent an oxygen or sulphur atom and -X=Y-represents one of the groups -N=C, -C=N or -C=C-,
or -V=W- taken together represent an imino, N-alkyl-imino, N-aralkyl-imino or N-aryl-imino group and -X=Y- represents one of the groups -N=N, -N=C, -C=N or -C=C-,
or, if p and q are not the same,
-X=Y- taken together represent an oxygen or sulphur atom and -V=W-represents one of the groups -N=C, -C=N or -C=C-,
or -X=Y- taken together represent an imino, N-alkyl-imino, N-aralkyl-imino or N-aryl-imino-group and -V=W- represents one of the groups -N=N, -N=C, -C=N or -C=C-,
while one or two of the available carbon atoms of the unit -V=W-X=Y- may be substituted in each case by a hydroxy, alkoxy, amino, alkylamino, dialkylamino, carboxy, alkoxycarbonyl, aminocarbonyl, alkylaminocarbonyl, dialkylaminocarbonyl or hydrazinocarbonyl group, while the substituents may be identical or different, and the remaining available carbon atoms of the unit -V=W-X=Y- are substituted by a hydrogen atom, an alkyl, aralkyl or aryl group,
or
**R_{c}** denotes a hydrogen atom or a C₁₋₈-alkyl group,
a C₃₋₇-cycloalkyl, C₃₋₇-cycloalkyl-alkyl or aralkyl group which may be substituted in each case by one or two alkyl groups or by an aryl group,
an alkyl group which is substituted
by a hydroxy, alkoxy, aryloxy, aralkoxy, alkylsulphenyl, alkylsulphinyl, alkylsulphonyl, arylsulphenyl, arylsulphinyl, arylsulphonyl, amino, alkylamino, dialkylamino, alkylcarbonylamino, N-alkyl-alkylcarbonylamino, alkylsulphonylamino, N-alkyl-alkylsulphonylamino, trifluoromethylsulphonylamino, N-alkyl-trifluoromethylsulphonylamino, carboxy, alkoxycarbonyl, aralkoxycarbonyl, aminocarbonyl, alkylaminocarbonyl, dialkylaminocarbonyl, cyano group,
by a 2-, 3- or 4-pyridyl group,
by an alkyleneimino or (alkyleneimino)carbonyl group with in each case 4 to 7 cyclic atoms in the alkyleneimino moiety, optionally substituted by 1 to 4 alkyl groups, while in the abovementioned 6- to 7-membered alkyleneimino groups a methylene group may be replaced in the 4-position by an oxygen or sulphur atom, by an imino, N-alkyl-imino, N-aryl-imino, N-aralkyl-imino, N-arylcarbonyl-imino or N-alkylcarbonyl-imino group,
a C₃₋₅-alkenyl group optionally substituted by an aryl group, while the vinyl moiety may not be attached to the nitrogen atom of the R_{c}NR_{d} group,
a C₃₋₅-alkynyl group optionally substituted by an aryl group, while the ethynyl moiety may not be attached to the nitrogen atom of the **R_{c}**N**R_{d}** group, and
**R_{d}** denotes a C₁₋₁₆-alkyl group which is substituted by a group selected from the groups (a) to (n):
(a) a carboxy, alkoxycarbonyl, aminocarbonyl, alkylaminocarbonyl, dialkylaminocarbonyl, hydroxy-C₂₋₄-alkylaminocarbonyl, cyano, hydroxy, alkoxy, aryloxy, aralkoxy, C₂₋₄-alkylenedioxy, alkylcarbonyloxy, arylcarbonyloxy, formylamino, alkylcarbonylamino, arylcarbonylamino, amino, alkylamino, dialkylamino, naphthylamino, aralkylamino, diaralkylamino or N-alkyl-aralkylamino group,
(b) a phenylamino, N-alkyl-N-phenylamino, pyridylamino or N-alkyl-N-pyridylamino group optionally substituted in the aryl moiety by one or two fluorine, chlorine, bromine or iodine atoms or one or two nitro, trifluoromethyl, alkyl, hydroxy, alkoxy, amino, alkylamino, dialkylamino, carboxy, alkoxycarbonyl, aminocarbonyl, alkylaminocarbonyl, dialkylaminocarbonyl or cyano groups, while the substituents may be identical or different,
(c) an alkoxy group substituted by one, two or three aryl groups,
(d) a hydroxy-C₂₋₄-alkylaminocarbonyl, alkoxy- C₂₋₄-alkylaminocarbonyl, amino-C₂₋₄-alkylaminocarbonyl, alkylamino- C₂₋₄-alkylaminocarbonyl, dialkylamino-C₂₋₄-alkylaminocarbonyl, carboxyalkylaminocarbonyl, alkoxycarbonylalkylaminocarbonyl, aminocarbonylalkylaminocarbonyl, alkylaminocarbonylalkylaminocarbonyl, dialkylaminocarbonylalkylaminocarbonyl, arylaminocarbonyl, N-alkyl-arylaminocarbonyl, aralkylaminocarbonyl, N-alkyl-aralkylaminocarbonyl,
(e) a group of formula -C(=NH)NH₂ or -NH-C(=NH)NH₂, which is optionally substituted by a cyano or alkoxycarbonyl group,
(f) an (alkyleneimino)carbonyl group optionally substituted by 1 to 4 alkyl groups with in each case 4 to 7 cyclic atoms in the alkyleneimino moiety, while in a 6 or 7-membered alkyleneimino moiety a methylene group in the 4-position may be replaced in each case by an oxygen or sulphur atom, by a sulphinyl, sulphonyl, imino, N-alkyl-imino, N-alkylcarbonyl-imino, N-alkylsulphonyl-imino, N-aryl-imino or N-aralkyl-imino group,
(g) a 4- to 7-membered alkyleneimino group optionally substituted by 1 to 4 alkyl groups, while in the abovementioned 6 or 7-membered alkyleneimino groups a methylene group in the 4-position may be replaced in each case by an oxygen or sulphur atom, by a sulphinyl, sulphonyl or **R₁₀**N group, where **R₁₀** is as hereinbefore defined, and additionally in the abovementioned 5- to 7-membered alkyleneimino groups in each case one or two methylene groups adjacent to the nitrogen atoms may be replaced by a carbonyl group,
(h) a 5- to 7-membered alkyleneimino group optionally substituted by 1 to 2 alkyl groups which is substituted by a hydroxyalkyl, aminoalkyl, alkylaminoalkyl or dialkylaminoalkyl group,
(i) an alkylsulphonylamino, N-alkyl-alkylsulphonylamino, arylcarbonylamino, N-alkyl-arylcarbonylamino, alkylcarbonylamino, N-alkyl-alkylcarbonylamino, arylsulphonylamino, N-alkyl-arylsulphonylamino, aralkylcarbonylamino, N-alkyl-aralkylcarbonylamino, alkoxyalkyl-carbonylamino, alkoxyalkyl-N-alkyl-carbonylamino, dialkylamino-alkylcarbonylamino, alkylamino-alkylcarbonylamino, amino-alkylcarbonylamino, aralkylsulphonylamino, N-alkyl-aralkylsulphonylamino, alkoxycarbonylamino, N-alkyl-alkoxycarbonylamino, aralkoxycarbonylamino or N-alkyl-aralkoxycarbonylamino group,
(j) a (**R₉**N**R₈**)-CO-N**R₇** or (**R₉**N**R₈**)-SO2-N**R₇** group, where **R₇, R₈** and **R₉** are as hereinbefore defined,
(k) an alkylsulphenyl, alkylsulphinyl, alkylsulphonyl, arylsulphenyl, arylsulphinyl, arylsulphonyl, aralkylsulphenyl, aralkylsulphinyl or aralkylsulphonyl group,
(1) a C₄₋₇-cycloalkyl group substituted by **R₆** and optionally additionally by 1 to 4 alkyl groups, where **R₆** is as hereinbefore defined,
(m) a C₅₋₇-cycloalkyl group optionally substituted by 1 to 4 alkyl groups wherein a methylene group is replaced by an oxygen or sulphur atom, by a sulphinyl, sulphonyl or N**R₁₀** group, where **R₁₀** is as hereinbefore defined,
(n) a 4-piperidinyl-alkyl group optionally substituted by 1 to 4 alkyl groups, which is substituted in the 1 position by **R₁₀** and additionally in the 4-position by a hydroxy group, where **R₁₀** is as hereinbefore defined, and wherein additionally hydrogen atoms in positions 2 and 6 of the piperidinyl structure are together replaced by a C₂₋₃-alkylene bridge,
a methyl group substituted by a 3-hydroxy-1,3-dihydro-indol-2-on-3-yl or 2-aminocarbonyl-1,3-dihydro-isoindol-5-yl group,
a group of the structure 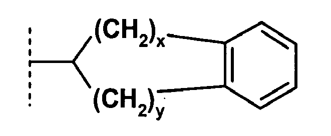 substituted in the aryl moiety by a carboxy, alkoxycarbonyl, aminocarbonyl, alkylaminocarbonyl, dialkylaminocarbonyl, carboxyalkyl, alkoxycarbonylalkyl, aminocarbonylalkyl, alkylaminocarbonylalkyl or dialkylaminocarbonylalkyl group and optionally additionally substituted in the alkylene moiety by 1 or 2 alkyl groups
wherein x and y, which may be identical or different, independently of one another represent the number 0, 1 or 2, but x and y together must yield at least the number 2,
a C₃₋₈-alkyl group substituted by a hydroxy group and additionally by an amino, alkylamino, dialkylamino, hydroxy, alkoxy, 1-pyrrolidinyl, 1-piperidinyl or morpholino group,
a C₂₋₈-alkyl group substituted by a carboxy group and additionally by an amino, hydroxy, aminocarbonyl or benzyloxycarbonylamino group,
a C₂₋₄-alkyl group which is substituted by a C₂₋₄-alkylsulphenyl or C₂₋₄-alkoxy group, which is substituted in the ω-position by an amino, hydroxy or alkoxy group,
a C₂₋₄-alkyl group which is substituted by a C₂₋₄-alkoxy-C₂₋₄-alkoxy group, which is substituted in the ω-position by an amino or hydroxy group,
a cyclopropyl group which is substituted by a carboxy, alkoxycarbonyl, aminocarbonyl, alkylaminocarbonyl or dialkylaminocarbonyl group or by an (alkyleneimino)carbonyl group optionally substituted by 1 to 4 alkyl groups with 4 to 7 cyclic atoms in the alkyleneimino moiety in each case, while in the abovementioned 6 or 7-membered alkyleneimino moieties a methylene group in the 4-position may be replaced in each case by an oxygen or sulphur atom, by a sulphinyl, sulphonyl, imino, N-alkyl-imino, N-alkylcarbonyl-imino, N-alkylsulphonyl-imino, N-aryl-imino or N-aralkyl-imino group,
a C₄₋₇-cycloalkyl group optionally substituted by 1 to 4 alkyl groups, which is additionally substituted by **R₆,** which is as hereinbefore defined,
a C₅₋₇-cycloalkyl group optionally substituted by 1 to 2 alkyl groups which is additionally substituted by an N,N-dialkyl-N-oxido-amino group,
a C₄₋₇-cycloalkyl group optionally substituted by 1 to 4 alkyl groups which may additionally be substituted by **R₆,** while in the cycloalkyl moiety a methylene group is replaced by an oxygen or sulphur atom, by a sulphinyl, sulphonyl, N-alkyl-N-oxido-imino or **R₁₀**N group, where **R₆** and **R₁₀** are as hereinbefore defined,
a C₅-C₇-cycloalkyl or C₅-C₇-cycloalkylalkyl group optionally substituted by 1 to 4 alkyl groups, wherein in each case a methylene group in the cycloalkyl moiety is replaced by a carbonyl group,
a cyclopentyl or cyclopentylalkyl group optionally substituted by 1 to 4 alkyl groups, wherein in each case two hydrogen atoms in the cyclopentyl moiety are replaced by a straight-chain alkylene bridge, this bridge containing 2 to 6 carbon atoms, if the two hydrogen atoms are located on the same carbon atom, or 1 to 5 carbon atoms, if the two hydrogen atoms are located on adjacent carbon atoms, or 2 to 4 carbon atoms if the two hydrogen atoms are located on carbon atoms separated by a carbon atom, while the abovementioned rings are additionally substituted by the group **R₆,** which is as hereinbefore defined,
a cyclohexyl, cyclohexylalkyl, cycloheptyl or cycloheptylalkyl group optionally substituted by 1 to 4 alkyl groups, wherein two hydrogen atoms in the cycloalkyl moiety are replaced by a straight-chain alkylene bridge in each case, this bridge containing 2 to 6 carbon atoms if the two hydrogen atoms are located on the same carbon atom, or 1 to 5 carbon atoms if the two hydrogen atoms are located on adjacent carbon atoms, or 1 to 4 carbon atoms if the two hydrogen atoms are located on carbon atoms separated by a carbon atom, or 1 to 3 carbon atoms if the two hydrogen atoms are located on carbon atoms separated by two carbon atoms, while the abovementioned rings are additionally substituted by the group **R₆,** which is as hereinbefore defined,
an alkyl group substituted by a 3-hydroxy-1,3-dihydro-indol-2-on-3-yl or 2-aminocarbonyl-1,3-dihydro-isoindol-5-yl group,
a C₁₋₁₀-alkyl group substituted by an aryl group, while the abovementioned aryl moiety is substituted by an alkoxycarbonyl, carboxy, carboxyalkyl, aminosulphonyl, trifluoromethoxy, cyano, aminoalkyl, amino, alkylamino, dialkylamino, nitro, 2H-pyridazin-3-on-6-yl, hydroxyphenyl, hydroxyalkyl, hydroxy or alkoxy group,
an aralkyl group which is substituted in the aryl moiety by a hydroxy or alkoxy group and additionally by a carboxy, alkoxycarbonyl, hydroxy or alkoxy group,
a C₁₋₁₀-alkyl group substituted by a pyrrolyl, imidazolyl, pyrazolyl, pyridyl, pyrimidinyl, pyrazinyl, indolyl or benzimidazolyl group, while the abovementioned heteroaryl moieties on the available carbon atoms may additionally be substituted in each case by one or two groups selected from fluorine, chlorine, bromine or iodine atoms, alkyl, alkoxycarbonyl, carboxy, trifluoromethyl, trifluoromethoxy, cyano, amino, alkylamino, dialkylamino, nitro, hydroxy or alkoxy groups, while the substituents may be identical or different,
a C₁₋₁₀-alkyl group substituted by a carboxy, alkoxycarbonyl, aminocarbonyl, alkylaminocarbonyl, dialkylaminocarbonyl, aralkylaminocarbonyl, cyano, hydroxy, alkoxy, aryloxy, amino, alkylamino, dialkylamino, alkylcarbonylamino or alkoxycarbonylamino group, which is additionally substituted by one or two aryl groups or a pyrrolyl, imidazolyl, pyrazolyl, pyridyl, pyrimidinyl, pyrazinyl, indolyl or benzimidazolyl group, while the abovementioned aryl or heteroaryl moieties at the available carbon atoms may additionally be substituted in each case by one or two groups selected from fluorine, chlorine, bromine or iodine atoms, alkyl, alkoxycarbonyl, carboxy, trifluoromethyl, trifluoromethoxy, cyano, amino, alkylamino, dialkylamino, nitro, hydroxy or alkoxy groups, while the substituents may be identical or different,
a C₁₋₆-alkyl group substituted by an aryl group which is substituted in the aryl moiety by a hydroxy or amino group and additionally by two fluorine, chlorine, bromine or iodine atoms, while the substituents may be identical or different,
a C₂₋₆-alkyl group substituted by a carboxy or alkoxycarbonyl group, which is additionally substituted by an amino, alkylamino, dialkylamino, alkylcarbonylamino, N-alkyl-alkylcarbonylamino, alkylsulphonylamino, N-alkyl-alkylsulphonylamino, arylcarbonylamino, N-alkyl-arylcarbonylamino, arylsulphonylamino, N-alkyl-arylsulphonylamino, aralkylcarbonylamino, N-alkyl-aralkylcarbonylamino, aralkylsulphonylamino, N-alkyl-aralkylsulphonylamino, alkoxycarbonylamino, N-alkyl-alkoxycarbonylamino, aralkoxycarbonylamino or N-alkyl-aralkoxycarbonylamino group,
a 3-quinuclidinyl, 4-quinuclidinyl, 2-quinuclidinyl-alkyl, 3-quinuclidinyl-alkyl or 4-quinuclidinyl-alkyl group, or
**R_{c}** denotes a hydrogen atom or an alkyl group and **R_{d}** denotes a hydroxy or alkoxy group, and
**Rₑ** denotes a nitro- or trifluoromethyl group,
optionally in the form of the tautomers, racemates, enantiomers, diastereomers and mixtures thereof, and optionally also the pharmacologically acceptable acid addition salts thereof,
while, unless otherwise stated,
by the aryl moieties mentioned in the definition of the abovementioned groups is meant a phenyl group, wherein one or two carbon atoms may be replaced by a nitrogen atom in each case, while the abovementioned aryl moieties in each case may be monosubstituted by **R₁₁,** mono-, di- or trisubstituted by **R₁₂** or monosubstituted by **R₁₁** and additionally mono- or disubstituted by **R₁₂,** while the substituents may be identical or different, and
**R₁₁** denotes a cyano, carboxy, aminocarbonyl, alkylaminocarbonyl, dialkylaminocarbonyl, alkoxycarbonyl, alkylcarbonyl, alkylsulphenyl, alkylsulphinyl, alkylsulphonyl, alkylsulphonyloxy, perfluoroalkyl, perfluoroalkoxy, nitro, amino, alkylcarbonylamino, N-alkyl-alkylcarbonylamino, alkylamino, dialkylamino, hydroxy-C2-4-alkylamino, N-alkyl-(hydroxy-C2-4-alkyl)amino, bis-(hydroxy-C2-4-alkyl)amino, phenylalkylcarbonylamino, phenylcarbonylamino, alkylsulphonylamino, phenylalkylsulphonylamino, phenylsulphonylamino, N-alkyl-phenylalkylcarbonylamino, N-alkyl-phenylcarbonylamino, N-alkyl-alkylsulphonylamino, N-alkyl-phenylalkylsulphonylamino, N-alkyl-phenylsulphonylamino, aminosulphonyl, alkylaminosulphonyl, dialkylaminosulphonyl, (**R₉**N**R₈**)-CO-N**R₇** or (**R₉**N**R₈**)-SO2-N**R₇** group, where **R₇, R₈** and **R₉** are as hereinbefore defined,
a 5- to 7-membered alkyleneimino group optionally substituted by 1 to 4 alkyl groups or a hydroxyalkyl group, while in the abovementioned 6- to 7-membered alkyleneimino groups a methylene group in the 4-position may be replaced in each case by an oxygen atom or an **R₁₀**N group, where **R₁₀** is as hereinbefore defined,
a 5- to 7-membered alkyleneimino group optionally substituted by 1 to 4 alkyl groups or a hydroxyalkyl group, while in each case one or two methylene groups adjacent to the nitrogen atom is replaced by a carbonyl group in each case, and
**R₁₂** denotes an alkyl, hydroxy or alkoxy group, a fluorine, chlorine, bromine or iodine atom, while two groups **R₁₂,** if they are bound to adjacent carbon atoms, may also denote an alkylene group with 3 to 6 carbon atoms, a 1,3-butadiene-1,4-diylene group or a methylenedioxy group,
and, unless stated to the contrary, the abovementioned alkyl, alkylene and alkoxy moieties each contain 1 to 4 carbon atoms,
while, unless otherwise stated, each carbon atom in the abovementioned alkyl, alkylene or cycloalkylene moieties, which is bound to a nitrogen, oxygen or sulphur atom, cannot be bound to any other halogen, nitrogen, oxygen or sulphur atom.

2. Compounds of formula I according to claim 1, wherein
R_{b} denotes a phenyl group optionally substituted by the groups R₁ to R₃,
while
R₁ denotes a fluorine, chlorine, bromine or iodine atom,
a C₁₋₂-alkyl or hydroxy group,
a C₃₋₆-cycloalkyl or C₅₋₆-cycloalkoxy group,
a C₂₋₅-alkenyl group,
a C₂₋₅-alkynyl group,
an aryl, aryloxy, aralkyl, aralkoxy, alkylsulphenyl, alkylsulphinyl, alkylsulphonyl, alkylsulphonyloxy, trifluoromethylsulphenyl, trifluoromethylsulphonyl, arylsulphenyl, arylsulphinyl, arylsulphonyl, aralkylsulphenyl, aralkylsulphinyl or aralkylsulphonyl group,
a methyl or methoxy group substituted by 1 to 3 fluorine atoms,
a C₂₋₄-alkyl or C₂₋₄-alkoxy group substituted by 1 to 5 fluorine atoms,
a nitro, amino, alkylamino, dialkylamino, C₃₋₆-cycloalkylamino, N-alkyl-C₃₋₆-cycloalkylamino, arylamino, N-alkyl-arylamino, aralkylamino or N-alkyl-aralkylamino group,
a 5- to 7-membered alkyleneimino group, while in each case one or two methylene groups adjacent to the nitrogen atom may each be replaced by a
carbonyl group or in the abovementioned 6- to 7-membered alkyleneimino groups a methylene group in the 4-position may be replaced by an oxygen atom, by an imino, N-aryl-imino or N-alkyl-imino group and the alkyleneimino groups may additionally be substituted by 1-2 methyl groups,
an (alkyleneimino)carbonyl or (alkyleneimino)sulphonyl group with in each case 5 to 7 cyclic atoms in the alkyleneimino moiety, while in the abovementioned 6- to 7-membered alkyleneimino moieties a methylene group in the 4-position may be replaced in each case by an oxygen atom, by an imino, N-aryl-imino or N-alkyl-imino group,
an alkylcarbonylamino, N-alkyl-alkylcarbonylamino, alkyl-sulphonylamino, N-alkyl-alkylsulphonylamino, arylcarbonylamino, N-alkyl-arylcarbonylamino, arylsulphonylamino, N-alkyl-arylsulphonylamino, aralkylcarbonylamino, N-alkyl-aralkylcarbonylamino, aralkylsulphonylamino, N-alkyl-aralkylsulphonylamino, trifluoromethylsulphonylamino, N-alkyl-trifluoromethylsulphonylamino, alkylcarbonyl, arylcarbonyl, aralkylcarbonyl, carboxy, alkoxycarbonyl, aminocarbonyl, alkylaminocarbonyl, dialkylaminocarbonyl, arylaminocarbonyl, N-alkyl-arylaminocarbonyl, aralkylaminocarbonyl, N-alkyl-aralkylaminocarbonyl, N-hydroxy-aminocarbonyl, N-hydroxy-alkylaminocarbonyl, N-alkoxy-aminocarbonyl, N-alkoxy-alkylaminocarbonyl, cyano, azido, N-cyano-amino or N-cyano-alkylamino group,
a sulpho, aminosulphonyl, alkylaminosulphonyl, dialkylaminosulphonyl, arylaminosulphonyl, pyridylaminosulphonyl, N-alkyl-arylaminosulphonyl, aralkylaminosulphonyl or N-alkyl-aralkylaminosulphonyl group, or
a C₁₋₂ alkyl group substituted by R₄,
wherein
R₄ denotes a hydroxy, alkoxy, aryloxy, amino, alkylamino, fluoroalkylamino, dialkylamino, alkylsulphenyl, alkylsulphinyl, alkylsulphonyl, arylsulphenyl, arylsulphinyl, arylsulphonyl, carboxy, alkoxycarbonyl, aminocarbonyl, alkylaminocarbonyl, dialkylaminocarbonyl or cyano group,
a 5- to 7-membered alkyleneimino group optionally substituted by one or two alkyl groups, while in the abovementioned 6- to 7-membered alkyleneimino groups a methylene group in the 4-position may be replaced by an oxygen or sulphur atom, by an imino, N-alkyl-imino, N-alkylcarbonyl-imino, N-alkylsulphonyl-imino, N-arylcarbonyl-imino, N-arylsulphonyl-imino, N-aryl-imino or N-aralkyl-imino group, or may be substituted by a hydroxy, alkoxy, aminocarbonyl, alkylaminocarbonyl, dialkylaminocarbonyl, amino, alkylamino and dialkylamino group, or
an (alkyleneimino)carbonyl group with in each case 5 to 7 cyclic atoms in the alkyleneimino moiety optionally substituted by one or two alkyl groups, while in the abovementioned 6- to 7-membered alkyleneimino moieties a methylene group in the 4-position may be replaced in each case by an oxygen or sulphur atom, by an imino, N-alkyl-imino or N-alkylcarbonyl-imino group, or
a group of formula 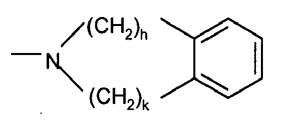 wherein
h and k, which may be identical or different, represent the numbers 1 to 2 or
h denotes the number 0 and k denotes the number 2 or 3, while additionally the above benzo moiety may be substituted by a fluorine, chlorine, bromine or iodine atom or by an alkyl, trifluoromethyl, hydroxy, alkoxy, carboxy or cyano group and the above saturated cyclic imino moiety may be substituted by 1 or 2 alkyl groups,
R₂ denotes a fluorine, chlorine or bromine atom, a C₁₋₂ alkyl, trifluoromethyl, hydroxy, amino, alkylamino, dialkylamino, alkylcarbonylamino, N-alkyl-alkylcarbonylamino, alkylsulphonylamino, N-alkyl-alkylsulphonylamino, trifluoromethylsulphonylamino, N-alkyl-trifluoromethylsulphonylamino or cyano group,
and
R₃ denotes a fluorine, chlorine or bromine atom, a C₁₋₂ alkyl, trifluoromethyl or alkoxy group,
a group of the structure 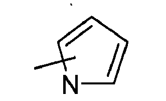 wherein the point of attachment may be a carbon or a nitrogen atom and up to three carbon atoms may be replaced by a nitrogen atom and the ring may be substituted, via each of the atoms, by one or two alkyl, aryl or aralkyl groups, or
a sulpho, aminosulphonyl, alkylaminosulphonyl, dialkylaminosulphonyl, arylaminosulphonyl, pyridylaminosulphonyl, N-alkyl-arylaminosulphonyl, aralkylaminosulphonyl or N-alkyl-aralkylaminosulphonyl group,
R₂ together with R₃, if they are bound to adjacent carbon atoms, denote a methylenedioxy group optionally substituted by one or two alkyl groups, or an n-C₃₋₅-alkylene group optionally substituted by one or two alkyl groups wherein a methylene group may be replaced by an oxygen atom, by an imino, N-alkyl-imino or N-aralkyl-imino group, or
a 1,3-butadiene-1,4-diylene group optionally substituted by a fluorine, chlorine or bromine atom, by a hydroxy, alkyl, alkoxy, trifluoromethyl, carboxy or cyano group or
a group of formula -NH-C(=O)-(CH₂) or -NH-C(=O)-(CH₂)₂, which may additionally be substituted in the alkylene moiety by 1 or 2 alkyl groups, or a group of formula -NH-N=N, -NH-N=CH, -NH-CH=N-, -O-CH=N,-S-CH=N, -NH-CH=CH- and the tautomers thereof, while each hydrogen atom may be substituted by an alkyl, aryl or aralkyl group,
or
a group of formula -(CH₂)ₘ-NR₅-(CH₂)ₙ-,
wherein m and n which may be identical or different in each case represent 1 or 2, and
R₅ denotes hydrogen, C₁₋₆ alkyl or C₁₋₆ fluoroalkyl, or
R_{c}NR_{d} represents a 4- to 7-membered alkyleneimino group optionally substituted by 1 to 2 alkyl or aryl groups which is additionally substituted by the group R₆, where
R₆ denotes a carboxy, alkoxycarbonyl, aminoalkyl, aminocarbonyl, alkylaminocarbonyl, dialkylaminocarbonyl, cyano, hydroxy, alkoxy, aryloxy, aralkoxy, alkylcarbonyloxy, arylcarbonyloxy, amino, alkylamino, hydroxy-C₂₋₄-alkylamino, dialkylamino, cyanamino, formylamino, alkylsulphenyl, alkylsulphinyl, alkylsulphonyl, arylsulphenyl, arylsulphinyl, arylsulphonyl, aralkylsulphenyl, aralkylsulphinyl or aralkylsulphonyl group,
an alkylcarbonylamino, N-alkyl-alkylcarbonylamino, alkylsulphonylamino, N-alkyl-alkylsulphonylamino, arylcarbonylamino, N-alkyl-arylcarbonylamino, arylsulphonylamino, N-alkyl-arylsulphonylamino, aralkylcarbonylamino, N-alkyl-aralkylcarbonylamino, aralkylsulphonylamino, N-alkyl-aralkylsulphonylamino, alkoxycarbonylamino, N-alkyl-alkoxycarbonylamino, alkoxycarbonylalkylamino, N-(alkyl)-N-(alkoxycarbonylalkyl)-amino, aralkoxycarbonylamino or N-alkyl-aralkoxycarbonylamino group,
a (NR₈R₉)CONR₇ group wherein
R₇ and R₈ in each case denote a hydrogen atom or an alkyl group and R₉ denotes a hydrogen atom or an alkyl, aryl or pyridyl group, while the groups R₇, R₈ and R₉ may be identical or different, or R₇ and R₈ together denote a n-C₂₋₄-alkylene group and R₉ is a hydrogen atom or an alkyl, aryl or pyridyl group,
an alkyleneimino group with 5 to 7 cyclic atoms in the alkyleneimino moiety optionally substituted by 1 to 2 alkyl groups, while in the abovementioned 6-to 7-membered alkyleneimino moieties a methylene group in the 4-position of the alkyleneimino moiety may be replaced in each case by an oxygen or sulphur atom, by a carbonyl, sulphinyl, sulphonyl, imino, N-alkylimino, N-alkylcarbonyl-imino, N-alkylsulphonyl-imino, N-arylimino or N-aralkyl-imino group,
an (alkyleneimino)carbonyl group with in each case 4 to 7 cyclic atoms in the alkyleneimino moiety optionally substituted by 1 to 2 alkyl groups, while in the abovementioned 6- to 7-membered alkyleneimino moieties a methylene group in the 4-position may be replaced in each case by an oxygen or sulphur atom, by a sulphinyl, sulphonyl, imino, N-alkyl-imino, N-alkylcarbonyl-imino, N-alkylsulphonyl-imino, N-aryl-imino or N-aralkyl-imino group,
a 4- to 7-membered alkyleneimino group substituted by a hydroxy, alkoxy, amino, alkylamino, dialkylamino, alkylcarbonylamino, N-alkyl-alkylcarbonylamino, alkylsulphonylamino, N-alkyl-alkylsulphonylamino, hydroxyalkyl, carboxy, alkoxycarbonyl, aminocarbonyl, alkylaminocarbonyl or dialkylaminocarbonyl group,
an alkyl group substituted by a carboxy, alkoxycarbonyl, aminocarbonyl, alkylaminocarbonyl, dialkylaminocarbonyl, cyano, hydroxy, alkoxy, aryloxy, amino, alkylamino, dialkylamino, alkylcarbonylamino, N-alkyl-alkylcarbonylamino, alkoxycarbonylamino, N-alkyl-alkoxycarbonylamino, alkylsulphonylamino, N-alkyl-alkylsulphonylamino, arylcarbonylamino, N-alkyl-arylcarbonylamino, arylsulphonylamino, N-alkyl-arylsulphonylamino, dialkylaminocarbonylalkylamino, N-(alkyl)-N-(dialkylaminocarbonylalkyl)-amino, dialkylaminoalkoxy, alkylsulphenyl, alkylsulphinyl, alkylsulphonyl, arylsulphenyl, arylsulphinyl or arylsulphonyl group,
an (alkyleneimino)alkyl group with in each case 4 to 7 cyclic atoms in the alkyleneimino moiety, optionally substituted by 1 to 2 alkyl groups, while in the abovementioned 6- to 7-membered alkyleneimino moieties a methylene group in the 4-position may be replaced in each case by an oxygen or sulphur atom, by a sulphinyl, sulphonyl, imino, N-alkyl-imino or N-alkylcarbonyl-imino group,
an (alkyleneimino)carbonylalkyl group with in each case 4 to 7 cyclic atoms in the alkyleneimino moiety optionally substituted by 1 to 2 alkyl groups, while in the abovementioned 6- to 7-membered alkyleneimino moieties a methylene group in the 4-position may be replaced in each case by an oxygen or sulphur atom, by a sulphinyl, sulphonyl, imino or N-alkyl-imino group,
a (carboxyalkyl)oxy, (alkoxycarbonylalkyl)oxy, (aminocarbonylalkyl)oxy, (alkylaminocarbonylalkyl)oxy or (dialkylaminocarbonylalkyl)oxy group,
a 3,4-dihydro-1H-quinazolin-2-on-3-yl or 1H-benzimidazol-2-on-1-yl group optionally substituted in the aryl moiety by one or two fluorine, chlorine, bromine or iodine atoms or one or two nitro, alkyl, alkoxy or cyano groups in each case, while the substituents may be identical or different, or
R_{c}NR_{d} denotes a 6- to 7-membered alkyleneimino group optionally substituted by 1 to 2 alkyl groups or by an aryl group, which may additionally be substituted by the group R₆, while in the abovementioned alkyleneimino groups a methylene group in the 4-position is replaced in each case by an oxygen or sulphur atom, by a carbonyl, sulphinyl, sulphonyl or R₁₀N group, where
R₁₀ denotes a hydrogen atom, an alkyl, hydroxy-C₂₋₄-alkyl, amino-C₂₋₄-alkyl, alkylamino-C₂₋₄-alkyl, dialkylamino-C₂₋₄-alkyl, (hydroxy-C₂₋₄-alkoxy)-C₂₋₄-alkyl, aminocarbonylalkyl, alkylaminocarbonylalkyl, dialkylaminocarbonylalkyl, aryl, formyl, alkylcarbonyl, alkylsulphonyl, arylcarbonyl, arylsulphonyl, aralkylcarbonyl, aralkylsulphonyl, alkoxycarbonyl, aralkoxycarbonyl, cyano, aminocarbonyl, alkylaminocarbonyl or dialkylaminocarbonyl group,
an amino-alkylcarbonyl, alkylamino-alkylcarbonyl, dialkylamino-alkylcarbonyl group,
a methyl group substituted by one or two aryl groups, while the aryl moieties may be substituted independently of one another by one or two fluorine, chlorine, bromine or iodine atoms or one or two nitro, alkyl, hydroxy or alkoxy groups in each case, while the substituents may be identical or different,
a 2-, 3- or 4-pyridyl group,
a 2-, 4- or 5-pyrimidyl group,
a phenyl group optionally substituted by one or two fluorine, chlorine, bromine or iodine atoms or one or two nitro, trifluoromethyl, alkyl, hydroxy, alkoxy, amino, alkylamino, dialkylamino, carboxy, alkoxycarbonyl, aminocarbonyl, alkylaminocarbonyl, dialkylaminocarbonyl or cyano groups, while the substituents may be identical or different,
an 8-methyl-8-aza-bicyclo[3.2.1]oct-3-yl group,
or an (alkyleneimino)carbonyl or (alkyleneimino)carbonylalkyl group with in each case 5 to 7 cyclic atoms in the alkyleneimino moiety, while in the abovementioned 6- to 7-membered alkyleneimino moieties a methylene group in the 4-position may be replaced in each case by an oxygen or sulphur atom, by a sulphinyl, sulphonyl, imino, N-alkyl-imino, N-alkylcarbonyl-imino or N-aralkyl-imino group, or
R_{c}NR_{d} denotes a 3-thiazolidinyl group substituted in the 4-position by a carboxy, alkoxycarbonyl, aminocarbonyl, alkylaminocarbonyl or dialkylaminocarbonyl group, or
R_{c}NR_{d} denotes a 1-piperidinyl group optionally substituted by 1 to 2 alkyl groups, wherein two hydrogen atoms on the carbon skeleton are replaced by a straight-chain alkylene bridge, this bridge containing 2 to 6 carbon atoms, if the two hydrogen atoms are located on the same carbon atom, or 1 to 5 carbon atoms if the two hydrogen atoms are located on adjacent carbon atoms, or 1 to 4 carbon atoms if the two hydrogen atoms are located on carbon atoms which are separated by one atom, or 1 to 3 carbon atoms if the two hydrogen atoms are located on carbon atoms which are separated by two atoms, while the abovementioned 1-piperidinyl groups are additionally substituted by the group R₆, which is as hereinbefore defined,
a 1-pyrrolidinyl or 1-piperidinyl group optionally substituted by 1 to 2 alkyl groups, wherein the two hydrogen atoms of a methylene group are replaced by a straight-chain C₃₋₆-alkylene bridge, while in each case a methylene group in the C₃₋₆-alkylene bridge is replaced by a R₁₀N group, where R₁₀ is as hereinbefore defined, while the bicyclic ring thus formed is optionally additionally substituted by a hydroxy, alkoxy, amino, alkylamino, dialkylamino, cyano, alkylcarbonylamino, alkylsulphonylamino, alkoxycarbonylamino, arylcarbonyl, carboxy, alkoxycarbonyl, aminocarbonyl, alkylaminocarbonyl or dialkylaminocarbonyl group,
a group of the structure 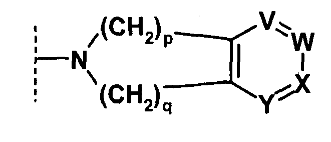 optionally substituted by 1 or 2 alkyl groups in the alkylene moieties
wherein
p and q, which may be identical or different, independently of one another denote the number 1 or 2, and
the unit -V=W-X=Y- denotes one of the groups (a) or (b):
-N=C-C=C- (a),
-C=N-C=C- (b),
while one of the available carbon atoms of the groups (a) or (b) may be substituted by a hydroxy, alkoxy, amino, alkylamino or dialkylamino group and the remaining available carbon atoms of the groups (a) or (b) are substituted by a hydrogen atom, an alkyl or aryl group,
or
-V=W- taken together represent an oxygen or sulphur atom or an imino, N-alkyl-imino or N-aryl-imino group and -X=Y- represents one of the groups
-N=C or -C=N-, or,
if n and m are not the same,
-X=Y- taken together represent an oxygen or sulphur atom or an imino,
N-alkyl-imino or N-aryl-imino group and -V=W- represents one of the groups -N=C or -C=N-,
or
R_{c} represents a hydrogen atom, an aralkyl or a C₁₋₆-alkyl group,
an alkyl group which is substituted
by a hydroxy, alkoxy, aryloxy, aralkoxy, amino, alkylamino, dialkylamino, alkylcarbonylamino, N-alkyl-alkylcarbonylamino, alkylsulphonylamino, N-alkyl-alkylsulphonylamino, trifluoromethylsulphonylamino, N-alkyl-trifluoromethylsulphonylamino, carboxy, alkoxycarbonyl, aminocarbonyl, alkylaminocarbonyl, dialkylaminocarbonyl, cyano or by a 2-, 3- or 4-pyridyl group, with the proviso that the hetero atoms are separated from the nitrogen atom of the R_{c}NR_{d} group by two or more carbon atoms,
a C₃₋₅-alkenyl group, while the vinyl moiety may not be attached to the nitrogen atom of the R_{c}NR_{d} group,
a C₃₋₅-alkynyl group, while the ethynyl moiety may not be attached to the nitrogen atom of the R_{c}NR_{d} group, and
R_{d} denotes a C₁₋₁₀-alkyl group which is substituted by a group selected from the groups (a) to (n):
(a) a carboxy, alkoxycarbonyl, aminocarbonyl, alkylaminocarbonyl, dialkylaminocarbonyl, cyano, hydroxy, alkoxy, aryloxy, aralkoxy, alkylcarbonylamino, amino, alkylamino, dialkylamino, naphthylamino, aralkylamino, diaralkylamino or N-alkyl-aralkylamino group,
(b) a phenylamino or pyridylamino group optionally substituted in the aryl moiety by a fluorine, chlorine, bromine or iodine atom or a nitro, trifluoromethyl, alkyl, hydroxy, alkoxy, amino, alkylamino, dialkylamino, carboxy, alkoxycarbonyl, aminocarbonyl, alkylaminocarbonyl, dialkylaminocarbonyl or cyano group,
(c) a methoxy group substituted by one, two or three aryl groups,
(d) a carboxyalkylaminocarbonyl, alkoxycarbonylalkylaminocarbonyl, aminocarbonylalkylaminocarbonyl, alkylaminocarbonylalkylaminocarbonyl, dialkylaminocarbonylalkylaminocarbonyl, arylaminocarbonyl, N-alkyl-arylaminocarbonyl, aralkylaminocarbonyl, N-alkyl-aralkylaminocarbonyl,
(e) a group of formula-C(=NH)NH₂,
(f) an (alkyleneimino)carbonyl group with in each case 5 to 7 cyclic atoms in the alkyleneimino moiety optionally substituted by 1 to 2 alkyl groups, while in the abovementioned 6- to 7-membered alkyleneimino groups a methylene group in the 4-position may be replaced in each case by an oxygen or sulphur atom, by a sulphinyl, sulphonyl, imino, N-alkyl-imino, N-alkylcarbonyl-imino, N-alkylsulphonyl-imino, N-aryl-imino or N-aralkyl-imino group,
(g) a 4- to 7-membered alkyleneimino group optionally substituted by 1 to 2 alkyl groups, while in the abovementioned 6- to 7-membered alkyleneimino groups a methylene group in the 4-position may be replaced in each case by an oxygen or sulphur atom, by a sulphinyl, sulphonyl or R₁₀N group, where R₁₀ is as hereinbefore defined, and additionally in the abovementioned 5- to 7-membered alkyleneimino groups a methylene group adjacent to the nitrogen atoms may be replaced by a carbonyl group in each case,
(h) a 5- to 7-membered alkyleneimino group optionally substituted by 1 to 2 alkyl groups which is substituted by a hydroxyalkyl, aminoalkyl, alkylaminoalkyl or dialkylaminoalkyl group,
(i) an alkylsulphonylamino, N-alkyl-alkylsulphonylamino, arylcarbonylamino, N-alkyl-arylcarbonylamino, alkylcarbonylamino, N-alkyl-alkylcarbonylamino, alkoxy-alkylcarbonylamino, dialkylamino-alkylcarbonylamino, arylsulphonylamino, N-alkyl-arylsulphonylamino, aralkylcarbonylamino, N-alkylaralkylcarbonylamino, aralkylsulphonylamino, N-alkyl-aralkylsulphonylamino, alkoxycarbonylamino, N-alkyl-alkoxycarbonylamino, aralkoxycarbonylamino or N-alkyl-aralkoxycarbonylamino group,
(j) a (R₉NR₈)-CO-NR₇ group, where R₇, R₈ and R₉ are as hereinbefore defined,
(k) a 2-aza-bicyclo[2.2.1]hept-5-en-2-yl group,
(l) an alkylsulphenyl, alkylsulphinyl, alkylsulphonyl, arylsulphenyl, arylsulphinyl or arylsulphonyl group,
(m) a C₄₋₇-cycloalkyl group substituted by R₆ and optionally additionally substituted by 1 to 2 alkyl groups, while R₆ is as hereinbefore defined,
(n) a C₅₋₇-cycloalkyl group optionally substituted by 1 to 4 alkyl groups wherein a methylene group is replaced by an oxygen atom or a NR₁₀ group, while R₁₀ is as hereinbefore defined,
a 4-piperidinyl-methyl group which is substituted in the 1-position by R₁₀ and additionally in the 4-position by a hydroxy group, where R₁₀ is as hereinbefore defined, and wherein additionally a hydrogen atom in each of positions 2 and 6 of the piperidinyl structure are together replaced by a C₂₋₃-alkylene bridge,
a methyl group substituted by a 3-hydroxy-1,3-dihydro-indol-2-on-3-yl or 2-aminocarbonyl-1,3-dihydro-isoindol-5-yl group,
a group of the structure 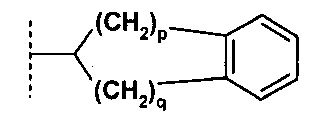 substituted in the aryl moiety by a carboxy or carboxyalkyl group and optionally additionally substituted in the alkylene moiety by 1 or 2 alkyl groups
while p and q, which may be identical or different, denote the number 0, 1 or 2, but p and q together must at least yield the number 2,
a C₃₋₆-alkyl group substituted by a hydroxy group and additionally substituted by an amino, alkylamino, dialkylamino, hydroxy, alkoxy, 1-pyrrolidinyl, 1-piperidinyl or morpholino group,
a C₂₋₆-alkyl group substituted by a carboxy group and additionally substituted by an amino, hydroxy, aminocarbonyl or benzyloxycarbonylamino group,
a C₂₋₄-alkyl group which is substituted by a C₂₋₄-alkylsulphenyl group, which is substituted in the ω-position by a ω-amino group,
a C₂₋₄-alkyl group which is substituted by a C₂₋₄-alkoxy group, which is substituted in the ω-position by an amino, hydroxy or alkoxy group,
a C₂₋₄-alkyl group which is substituted by a C₂₋₄-alkoxy-C₂₋₄-alkoxy group, which is substituted in the ω-position by an amino or hydroxy group,
a C₄₋₇-cycloalkyl group optionally substituted by 1 to 2 alkyl groups, which is additionally substituted by R₆, which is as hereinbefore defined,
a C₄₋₇-cycloalkyl group optionally substituted by 1 to 2 alkyl groups, which may additionally be substituted by R₆, while in the cycloalkyl moiety a methylene group is replaced by an oxygen or sulphur atom, by a sulphinyl, sulphonyl or R₁₀N group, while R₆ and R₁₀ are as hereinbefore defined,
a methyl group substituted by a 3-hydroxy-1,3-dihydro-indol-2-on-3-yl or 2-aminocarbonyl-1,3-dihydro-isoindol-5-yl group,
a C₁₋₆-alkyl group substituted by an aryl group, while the abovementioned aryl moiety is substituted by an alkoxycarbonyl, carboxy, carboxyalkyl, aminosulphonyl, trifluoromethoxy, cyano, aminoalkyl, amino, alkylamino, dialkylamino, nitro, 2H-pyridazin-3-on-6-yl, hydroxyphenyl, hydroxyalkyl, hydroxy or alkoxy group,
an aralkyl group which is substituted in the aryl moiety by an alkoxy or hydroxy group and additionally by an alkoxycarbonyl, carboxy, alkoxy or hydroxy group,
a C₁₋₆-alkyl group substituted by a 2-pyridyl, 3-pyridyl, 4-pyridyl, 2-pyrazinyl-,1H-pyrrol-2-yl, 1H-pyrazol-4-yl-,1H-pyrazol-5-yl, 1H-imidazol-1-yl, 1H-imidazol-4-yl, 1H-indol-3-yl or 1H-benzimidazol-2-yl group, while the abovementioned heteroaryl moieties at the available carbon atoms may additionally be substituted in each case by one or two groups selected from fluorine, chlorine, bromine or iodine atoms, alkyl, alkoxycarbonyl, carboxy, trifluoromethyl, trifluoromethoxy, cyano, amino, alkylamino, dialkylamino, nitro, hydroxy or alkoxy groups, while the substituents may be identical or different,
a C₁₋₆-alkyl group substituted by a carboxy, alkoxycarbonyl, aminocarbonyl, alkylaminocarbonyl, dialkylaminocarbonyl, aralkylaminocarbonyl, cyano, hydroxy, alkoxy, aryloxy, amino, alkylamino, dialkylamino, alkylcarbonylamino or alkoxycarbonylamino group, which is additionally substituted by one or two aryl groups or a heteroaryl group, while the heteroaryl group denotes a 2-pyridyl, 3-pyridyl, 4-pyridyl, 2-pyrazinyl-,1H-pyrrol-2-yl, 1H-pyrazol-4-yl-,1H-pyrazol-5-yl, 1H-imidazol-1-yl, 1H-imidazol-4-yl, 1H-indol-3-yl or 1H-benzimidazol-2-yl group, while the abovementioned aryl or heteroaryl moieties at the available carbon atoms may additionally be substituted in each case by one or two groups selected from fluorine, chlorine, bromine or iodine atoms, alkyl, alkoxycarbonyl, carboxy, trifluoromethyl, trifluoromethoxy, cyano, amino, alkylamino, dialkylamino, nitro, hydroxy or alkoxy groups, while the substituents may be identical or different,
a C₁₋₆-alkyl group substituted by an aryl group which is substituted in the aryl moiety by a hydroxy or amino group and is additionally substituted by two fluorine, chlorine, bromine or iodine atoms, while the substituents may be identical or different,
a C₂₋₆-alkyl group substituted by a carboxy or alkoxycarbonyl group which is additionally substituted by an amino, alkylamino, dialkylamino, alkylcarbonylamino, arylcarbonylamino, arylsulphonylamino, alkoxycarbonylamino or aralkoxycarbonylamino group,
a 3-quinuclidinyl or 4-quinuclidinyl group, and
while, unless otherwise specified, the abovementioned alkyl, alkylene and alkoxy moieties in each case contain 1 to 4 carbon atoms,
optionally in the form of the tautomers, racemates, enantiomers, diastereomers and mixtures thereof, and optionally also the pharmacologically acceptable acid addition salts thereof,
while, unless otherwise stated, each carbon atom in the abovementioned alkyl, alkylene or cycloalkylene moieties which is bound to a nitrogen, oxygen or sulphur atom, cannot be bound to any other halogen, nitrogen, oxygen or sulphur atom.

3. Compounds of formula I according to claim 1 or 2, wherein
R_{b} denotes a naphthyl group optionally substituted by a fluorine, chlorine or bromine atom or by a carboxy, C₁₋₂ alkyl, C₁₋₂ alkoxy, cyano or trifluoromethyl group,
a 5 or 6-indazolyl group optionally substituted at the nitrogen by a methyl group, or a 1,3-dihydro-2-oxo-indol-6-yl group or
a phenyl group optionally substituted by the groups R₁ to R₃, where
R₁ denotes a fluorine, chlorine, bromine or iodine atom, a C₁₋₂ alkyl, trifluoromethyl, aminocarbonyl, carboxy, alkoxycarbonyl, cyano, phenylaminocarbonyl, benzylaminocarbonyl, C₁₋₃-alkylsulphonyl, aminosulphonyl, methylaminosulphonyl, dimethylaminosulphonyl, morpholinosulphonyl, N-methylpiperazinosulphonyl, homopiperazinosulphonyl, 2,6-dimethylpiperazin-4-yl, 2-aminopyridyl-N-sulphonyl, morpholino, 4-methyl-1-piperazinyl, (N-methyl-N-methylsulphonyl)amino, 2-carboxy-1-ethyl, dimethylamino-1-ethyl or nitro group,
a methyl group which is substituted by a 1,2,4,5-tetrahydro-benzo[*d*]azepin-3-yl, a dialkylamino or a pyrrolidino, piperidino, 2,6-dimethyl-piperidino-1-yl, 4-methoxy-piperidino-1-yl, morpholino, S-dioxo-thiomorpholino, piperazino or 4-methyl-1-piperazinyl group, a fluoroalkylamino group of formula
-(CH₂)ᵣ-(CF₂)ₛ-Q,
wherein
r denotes 0 or an integer from 1 to 3,
s denotes an integer from 1 to 3, and
Q denotes hydrogen, fluorine or chlorine,
R₂ denotes a fluorine or chlorine atom, a hydroxy, amino or methyl group and
R₃ denotes a chlorine atom, or
a tetrazolyl, triazolyl, imidazolyl or pyrazolyl group, wherein the point of attachment is a carbon atom or a nitrogen atom and on the ring a hydrogen atom may be replaced by an alkyl group, or an aminosulphonyl, methylaminosulphonyl, dimethylaminosulphonyl, morpholinosulphonyl, N-methylpiperazinosulphonyl, homopiperazinosulphonyl or 2-aminopyridyl-N-sulphonyl group,
R₂ and R₃ taken together represent a group of the formula
-(CH₂)ₘ-NR₅-(CH₂)ₙ
wherein n and m independently of each other denote 1 or 2, and
R₅ denotes a fluoroalkyl group of formula
-(CH₂)ᵣ'-(CF₂)ₛ'-Q',
wherein
r' denotes 0 or an integer from 1 to 3,
s'denotes an integer from 1 to 3, and
Q' denotes hydrogen, fluorne or chlorine,
the group R_{c}NR_{d}
denotes a 5- to 7-membered alkyleneimino group substituted by the group R₆,
while R₆ denotes a hydroxy, alkoxy, aryloxy, amino, alkylamino, dialkylamino, alkylcarbonylamino, N-alkyl-alkylcarbonylamino, alkoxycarbonylalkylamino, N-(alkyl)-N-(alkoxycarbonylalkyl)-amino, alkylsulphenyl, alkylsulphinyl, alkylsulphonyl, arylsulphenyl, arylsulphinyl, arylsulphonyl, carboxy, alkoxycarbonyl, aminocarbonyl, alkylaminocarbonyl, dialkylaminocarbonyl or cyano group,
an alkyl group which is substituted by a hydroxy, amino, alkylamino, dialkylamino, alkylcarbonylamino, N-alkyl-alkylcarbonylamino, dialkylaminocarbonylalkylamino, N-(alkyl)-N-(dialkylaminocarbonylalkyl)-amino, alkoxycarbonyl, carboxy or dialkylaminoalkoxy group or by a 5- to 7-membered alkyleneimino group, while in the abovementioned 6- to 7-membered alkyleneimino groups a methylene group in the 4-position may be replaced by an oxygen or sulphur atom or by an imino, N-alkyl-imino or N-alkylcarbonyl-imino group,
an alkyleneimino group with 5 to 7 cyclic atoms in the alkyleneimino moiety, while in the abovementioned 6- to 7-membered alkyleneimino moieties a methylene group in the 4-position may be replaced in each case by an oxygen or sulphur atom, by an N-alkyl-imino, N-alkylcarbonyl-imino or N-aralkyl-imino group,
an alkyleneimino group with 5 to 7 cyclic atoms in the alkyleneimino moiety substituted by a hydroxy, amino, alkylamino, dialkylamino, carboxy, alkoxycarbonyl, aminocarbonyl, alkylaminocarbonyl or dialkylaminocarbonyl group,
a 3,4-dihydro-1H-quinazolin-2-on-3-yl or a 1H-benzimidazol-2-on-1-yl group optionally substituted in the aryl moiety by a fluorine, chlorine or bromine atom or a nitro, alkyl, alkoxy or cyano group in each case,
a 6- to 7-membered alkyleneimino group optionally substituted by 1 or 2 alkyl groups, while a methylene group in the 4-position is replaced by an oxygen or sulphur atom, by a sulphinyl, sulphonyl or an NR₁₀ group,
where R₁₀ denotes a hydrogen atom or an alkyl, aralkyl, amino-C₂₋₄-alkyl, hydroxy-C2-4-alkyl, alkylcarbonyl, aralkoxycarbonyl, alkylsulphonyl, arylcarbonyl, arylsulphonyl,
an (alkyleneimino)carbonylalkyl group with 5 to 7 cyclic atoms in the alkyleneimino moiety, while in the abovementioned 6- to 7-membered alkyleneimino moieties a methylene group in the 4-position may be replaced in each case by an oxygen or sulphur atom, by an N-alkyl-imino, N-alkylcarbonyl-imino or N-aralkyl-imino group,
a 2-, 3- or 4-pyridyl group,
a 2-, 3- or 4-pyrimidyl group,
a phenyl group optionally substituted by one or two fluorine, chlorine, bromine or iodine atoms or one or two nitro, alkyl, hydroxy, alkoxy, amino, alkylamino, dialkylamino, carboxy, alkoxycarbonyl, aminocarbonyl, alkylaminocarbonyl, dialkylaminocarbonyl or cyano groups, while the substituents may be identical or different,
an 8-methyl-8-aza-bicyclo[3.2.1]oct-3-yl group,
a benzhydryl group, wherein independently of one another each phenyl moiety may be substituted by a fluorine, chlorine, bromine or iodine atom or a nitro, alkyl, hydroxy, alkoxy group, while the substituents may be identical or different,
a 6 or 7-membered alkyleneimino group substituted by a phenyl group, which is additionally substituted by a hydroxy, carboxy, alkoxycarbonyl or cyano group or wherein a methylene group in the 4-position is replaced by a carbonyl group,
a 3-thiazolidinyl group substituted in the 4-position by a carboxy, alkoxycarbonyl, aminocarbonyl, alkylaminocarbonyl or dialkylaminocarbonyl group,
a group of the structure 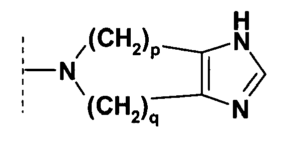 wherein p and q, which may be identical or different, independently of one another represent the number 1 or 2, while the imidazo ring may be substituted by one or two alkyl or aryl groups, while the substituents may be identical or different,
a 1-pyrrolidinyl or 1-piperidinyl group, wherein the two hydrogen atoms of a methylene group are replaced by a straight-chain C₃₋₅-alkylene bridge, while in each case a methylene group in the C₃₋₅-alkylene bridge is replaced by an imino, N-alkyl-imino or N-(aralkyl)imino group, while the bicyclic ring thus formed is optionally additionally substituted by a hydroxy group,
a 1-piperidinyl group which is substituted in the 4-position by a hydroxy, alkoxy or aralkoxy group and wherein additionally one of the hydrogen atoms in each of positions 2 and 6 of the piperidinyl structure are together replaced by an ethylene bridge,
or
R_{c} denotes a hydrogen atom or a C₁₋₆-alkyl group,
an alkyl group substituted by a phenyl or a 2-, 3- or 4-pyridyl group,
a C₂₋₄-alkyl group substituted by a hydroxy or alkoxy group, and
R_{d} represents a C₁₋₆-alkyl group which is substituted by a group selected from the groups (a) to (j):
(a) a group of formula-C(=NH)NH₂,
(b) a carboxy, alkoxycarbonyl, carboxymethylaminocarbonyl, aminocarbonyl, alkylaminocarbonyl, alkylcarbonylamino, dialkylaminocarbonyl, arylaminocarbonyl, N-alkyl-arylaminocarbonyl, aralkylaminocarbonyl, N-alkyl-aralkylaminocarbonyl or cyano group,
(c) a hydroxy, amino, alkoxy, alkylamino, dialkylamino, alkylcarbonylamino, N-alkyl-alkylcarbonylamino, alkoxycarbonylamino, alkoxyacetylamino, dialkylaminoacetylamino, N-alkyl-alkoxycarbonylamino, alkylsulphonylamino, N-alkyl-alkylsulphonylamino, arylamino, naphthylamino, aralkylamino, diaralkylamino, N-alkyl-aralkylamino or alkylsulphenyl group,
(d) a nitro-2-pyridyl-amino group,
(e) a methoxy group substituted by one, two or three aryl groups,
(f) a 4- to 7-membered alkyleneimino group, while in the abovementioned 6-to 7-membered alkyleneimino groups a methylene group in the 4-position may be replaced in each case by an oxygen or sulphur atom, by an imino, N-alkyl-imino, N-(hydroxy-C₂₋₄-alkyl)-imino or N-(amino-C₂₋₄-alkyl)-imino group, and additionally in the abovementioned 5- to 7-membered alkyleneimino groups a methylene group adjacent to the nitrogen atoms may be replaced in each case by a carbonyl group,
(g) a 1-piperidinyl group substituted by a dialkylaminoalkyl group,
(h) a 2-aza-bicyclo[2.2.1]hept-5-en-2-yl group,
(i) a 5- to 7-membered (alkyleneimino)carbonyl group, while in the abovementioned 6- to 7-membered alkyleneimino groups a methylene group in the 4-position may be replaced by an oxygen or sulphur atom or by an imino or N-alkyl-imino group, and
(j) a (R₈R₉)CONR₇ group wherein
R₇, R₈ and R₉, which may be identical or different, in each case denote a hydrogen atom or a methyl group or
R₇ and R₈ together denote an n-C₂₋₃-alkylene group and R₉ denotes a hydrogen atom or a methyl or 4-pyridyl group or
R₇ and R₈ denote a hydrogen atom and R₉ denotes an aryl-C₁₋₂-alkyl or phenyl group,
a cyclohexyl group substituted in the 2-, 3- or 4-position by a hydroxy, amino, alkylamino, dialkylamino, aminomethyl, hydroxymethyl, alkoxycarbonyl, aminocarbonyl, alkylaminocarbonyl, dialkylaminocarbonyl or carboxy group,
a cyclohexyl group substituted in the 4-position by a carboxyalkyl group,
an ethyl group substituted in the 2-position by a 2-amino-1-ethylthio, 2-hydroxy-1-ethoxy, 2-(2-amino-1-ethoxy)-1-ethoxy or 2-(2-hydroxy-1-ethoxy)-1-ethoxy group,
a propyl group substituted in the 3-position by a 3-amino-1-propoxy or 2-(3-amino-1-propoxy)-1-ethoxy group,
a C₁₋₂-alkyl group substituted by a C₅₋₆-cycloalkyl group, while the cycloalkyl moiety is substituted by a hydroxy, aminomethyl, dimethylaminomethyl, 2-carboxyethyl or tert.-butyloxycarbonylaminomethyl group or wherein in the cycloalkyl moiety a methylene group is replaced by an oxygen atom, an N-alkyl-imino or N-(2-dialkylaminoacetyl)imino group,
a 4-piperidinyl-methyl group which is substituted in the 1-position by an alkyl or aralkyl group and additionally in the 4-position by a hydroxy group and wherein additionally in each case a hydrogen atom in each of positions 2 and 6 of the piperidinyl structure are together replaced by an ethylene bridge,
a 3-pyrrolidinyl or a 3- or 4-piperidinyl group which is substituted in each case in the 1-position by an alkyl, aralkyl or arylsulphonyl group,
a 4-piperidinyl group which is substituted in the 1-position by an alkyl, aralkyl or aryl group and is additionally substituted in the 4-position by a carboxy group,
an aralkyl group which is substituted in the aryl moiety by a hydroxy, aminosulphonyl, carboxy, nitro, amino, aminomethyl, 2-amino-1-ethyl, alkoxycarbonyl, 4-hydroxyphenyl or 2*H*-pyridazin-3-on-6-yl group,
a methyl group substituted by a 3-hydroxy-1,3-dihydro-indol-2-on-3-yl or 2-aminocarbonyl-1,3-dihydro-isoindol-5-yl group,
a 2-indanyl group substituted in the aryl moiety by a 3-carboxy-1-propyl group,
an alkyl group substituted by a 1*H*-2-benzimidazolyl or 4-amino-3,5-dichlorophenyl group,
an aralkyl group which is substituted in the alkyl moiety by a hydroxy, amino, alkylamino, dialkylamino, aminocarbonyl, alkylaminocarbonyl, dialkylaminocarbonyl, aralkylaminocarbonyl, carboxy or cyano group and is optionally additionally substituted in the aryl moiety by one or two fluorine, chlorine or bromine atoms or one or two hydroxy or alkoxy groups, while the substituents may be identical or different,
an alkyl group substituted by a carboxy group and additionally by two phenyl groups,
a C₂₋₆-alkyl group substituted by a carboxy group and additionally substituted by a hydroxy, aminocarbonyl, 1H-imidazol-4-yl or benzyloxycarbonylamino group,
an alkyl group substituted by an alkoxycarbonyl group and additionally by a pyridyl group,
a C₃₋₆-alkyl group substituted by a hydroxy group and additionally by an amino, alkylamino, dialkylamino, hydroxy, alkoxy, 1-pyrrolidinyl, 1-piperidinyl or morpholino group,
an aralkyl group which is substituted in the aryl moiety by an alkoxy and additionally by a carboxy or hydroxy group,
an alkyl group substituted by a 2-pyridyl, 3-pyridyl, 4-pyridyl, 2-pyrazinyl, 3-chloro-5-trifluoromethyl-2-pyridyl, 1-methyl-1*H*-pyrrol-2-yl, 1*H*-pyrazol-4-yl, 4-ethoxycarbonyl-1*H*-pyrazol-5-yl, 1*H*-imidazol-1-yl, 1*H*-imidazol-4-yl, 1*H*-indol-3-yl or 6-methoxy-1*H*-benzimidazol-2-yl group,
a 1-pentyl group substituted in the 5-position by an alkoxycarbonyl group, which is additionally substituted in the 5 position by an amino, alkylcarbonylamino, arylcarbonylamino, arylsulphonylamino, alkoxycarbonylamino or aralkoxycarbonylamino group,
while, unless otherwise specified, the abovementioned alkyl, alkylene and alkoxy moieties each contain 1 to 4 carbon atoms,
optionally in the form of the tautomers, racemates, enantiomers, diastereomers and mixtures thereof, and optionally also the pharmacologically acceptable acid addition salts thereof,
while, unless otherwise stated, each carbon atom in the abovementioned alkyl, alkylene or cycloalkylene moieties which is bound to a nitrogen, oxygen or sulphur atom cannot be bound to any other halogen, nitrogen, oxygen or sulphur atom.

4. Compounds of formula I according to one of claims 1 to 3, wherein
R_{b} denotes a 1-naphthyl group or a 2-naphthyl group optionally substituted in the 5 position by a carboxy group,
a 1,3-dihydro-2-oxo-indol-6-yl group, benzotriazol-5-yl, benzimidazol-5-yl, indazol-5-yl, indazol-6-yl or 1-methyl-1*H*-indazol-6-ylamino group,
a phenyl group optionally substituted in the 4 position of the phenyl moiety by a fluorine, chlorine or bromine atom, by a cyano, propyl-2-sulphonyl, aminosulphonyl, methylaminosulphonyl, dimethylaminosulphonyl, morpholinosulphonyl, N-methylpiperazinosulphonyl, homopiperazinosulphonyl, 2,6-dimethylpiperazin-4-yl, 2-aminopyridyl-N-sulphonyl, carboxy, piperidinomethyl, 1,2,4,5-tetrahydro-benzo[*d*]azepin-3-yl-methyl, 2-carboxy-1-ethyl, phenylaminocarbonyl, benzylaminocarbonyl, aminocarbonyl, methoxycarbonyl, (N-methyl-N-methylsulphonyl)amino, diethylaminomethyl, 3-diethylamino-1-propyloxy, morpholino, 4-methyl-1-piperazinyl, 2-H-tetrazol-5-yl, 1-H-imidazol-4-yl or nitro group,
a phenyl group substituted in the 3 position of the phenyl moiety by a chlorine or bromine atom, by a cyano, aminocarbonyl, carboxy, ethoxycarbonyl or nitro group or by a group of formula
-CH₂-NH-(CH₂)ᵣ-CₛF₂ₛ₊₁,
wherein r denotes 1 or 2 and s denotes 1, 2 or 3,
a 3,4-dichlorophenyl, 3,5-dichlorophenyl, 4-amino-3,5-dichlorophenyl, 3-chloro-4-fluorophenyl, 4-chloro-3-methylphenyl, 4-chloro-3-trifluoromethylphenyl, 4-bromo-3-chlorophenyl or 3-hydroxy-4-methylphenýl group, or
a group of formula 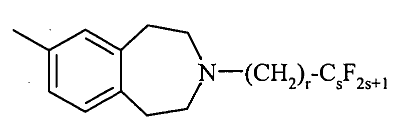 wherein r denotes 1 or 2 and s denotes 1, 2 or 3,
the group R_{c}NR_{d} denotes
a 1-pyrrolidinyl group substituted in the 2 position by a hydroxymethyl,
1-pyrrolidinylmethyl or 2-ethoxycarbonyl-1-ethyl group,
a 1-pyrrolidinyl group substituted in the 3 position by an amino, acetylamino, N-methyl-acetylamino or tert.butyloxycarbonylamino,
a 4-carboxy-3-thiazolidinyl, a 7-methyl-2,7-diaza-spiro[4.4]non-2-yl or a 5-hydroxy-2-methyl-2,8-diaza-spiro[5.5]undec-8-yl group,
a morpholino or S-oxido-thiomorpholino group
a 1-piperidinyl group substituted in the 2 position by an ethoxycarbonyl, hydroxymethyl, 3-hydroxypropyl, 3-diethylamino-1-propyl or 2-(2-diethylaminoethoxy)-1-ethyl group,
a 1-piperidinyl group substituted in the 3 position by a hydroxy, hydroxymethyl, 3-diethylamino-1-propyl, aminocarbonyl, dimethylaminocarbonyl, carboxy, 1-pyrrolidinylmethyl, 4-(1-pyrrolidinyl)-1-butyl, methoxycarbonylmethyl or acetylaminomethyl group,
a 1-piperidinyl group substituted in the 4-position by an ethoxycarbonyl, 3-hydroxypropyl, hydroxy, aminomethyl, 2-(2-diethylaminoethoxy)-1-ethyl, 2-carboxy-1-ethyl, N-(2-methoxycarbonyl-1-ethyl)-N-methyl-amino, 2-(N-(dimethylaminocarbonylmethyl-)-N-methyl-amino)-1-ethyl, N-acetyl-N-methylaminomethyl, 8-methoxy-3,4-dihydro-1H-quinazolin-2-on-3-yl, 1-piperidinyl, 3-hydroxy-1-piperidinyl or 4-ethoxycarbonyl-1-piperidinyl group,
a 3,5-dimethyl-1-piperazinyl, 1,4,6,7-tetrahydro-imidazo[4,5-c]pyridin-5-yl, 2-methyl-1,4,6,7-tetrahydro-imidazo[4,5-c]pyridin-5-yl, 1,4,5,6,7,8-hexahydro-imidazo[4,5-d]azepin-6-yl, 2-methyl-1,4,5,6,7,8-hexahydro-imidazo[4,5-d]azepin-6-yl, 3-phenyl-azepan-4-on-1-yl or 4-carboxy-4-phenyl-1-piperidinyl group,
a 1-piperazinyl group which is optionally substituted in the 4-position by a methyl, acetyl, benzyloxycarbonyl, 2-pyridyl, 2-pyrimidinyl, 2-nitrophenyl, 3-methoxyphenyl, 4-cyanophenyl, 3,4-dimethoxyphenyl, 4-[bis-(4-methoxy-phenyl)]-methyl, 8-methyl-8-aza-bicyclo[3.2.1]oct-3-yl, morpholinocarbonylmethyl, 2-amino-1-ethyl or 3-hydroxy-1-propyl group,
a 1-homopiperazinyl group which is optionally substituted in the 4-position by a methyl group,
a 3-hydroxy-8-aza-bicyclo[3.2.1]oct-8-yl group,
or
R_{c} denotes a hydrogen atom or a methyl, ethyl, 2-methoxyethyl, 2-hydroxyethyl, i-propyl, n-propyl, n-butyl, benzyl or 3-pyridylmethyl group, and
R_{d} denotes a methyl group substituted by a group of formula-C(=NH)NH₂ or a cyano, carboxyl, ethoxycarbonyl, aminocarbonyl, carboxymethylaminocarbonyl, 1-hydroxy-1-cyclohexyl, aminomethylcyclohexyl, 3-hydroxy-8-methyl-8-aza-bicyclo[3.2.1]oct-3-yl, 3-hydroxy-1,3-dihydro-indol-2-on-3-yl, 2-aminocarbonyl-1,3-dihydro-isoindol-5-yl, 2-tetrahydrofuryl, 1-ethyl-2-pyrrolidinyl, 1H-imidazol-4-yl, 1-methyl-4-piperidinyl, 1-(2-dimethylaminoacetyl)-4-piperidinyl, 2-pyridyl, 3-pyridyl, 4-pyridyl, 2-pyrazinyl, 3-chloro-5-trifluoromethyl-2-pyridyl, 4-ethoxycarbonyl-1*H*-pyrazol-5-yl, 2-carboxyphenyl, 3-carboxyphenyl, 2-hydroxyphenyl, 4-hydroxyphenyl, 2-nitrophenyl, 3-nitrophenyl, 4-nitrophenyl, 3-aminophenyl, 4-aminophenyl, 4-(aminosulphonyl)phenyl, 4'-hydroxybiphenyl, 4-(aminomethyl)phenyl or 4-hydroxy-3-methoxyphenyl group,
a C₂₋₅-alkyl group substituted by a carboxy group,
a C₂₋₅-alkyl group substituted by a hydroxy, amino or dimethylamino group, with the proviso that the hetero atoms of the abovementioned substituents are separated from the nitrogen atom of the R_{c}NR_{d} group by at least two carbon atoms,
a benzyl group substituted in the methylene moiety by a carboxy or cyano group,
a methyl group substituted by a carboxy group and a 4-hydroxyphenyl group,
an ethyl group substituted in the 1-position by a methoxycarbonyl or a 1H-benzimidazol-2-yl group,
an ethyl group substituted in the 2 position by a methoxy, diphenylmethoxy, methylthio, methylamino, diethylamino, diisopropylamino, acetylamino, N-methylacetylamino, 2-methoxyacetylamino, 2-dimethylaminoacetylamino, isopropylcarbonylamino, 2-methyl-propylcarbonylamino, phenyl-acetylamino, tert.-butyloxycarbonylamino, methylsulphonylamino, benzoylamino, phenylamino, 1-naphthylamino, 4-nitro-2-pyridyl-amino, cyano, ethoxycarbonyl, aminocarbonyl, methylaminocarbonyl, dimethylaminocarbonyl, 2-hydroxy-1-ethoxy, 2-(2-amino-1-ethoxy)-1-ethoxy, 2-(2-hydroxy-1-ethoxy)-1-ethoxy, 2-amino-1-ethylthio, 1-methyl-2-pyrrolidinyl, 1-pyrrolidinyl, 2-oxo-pyrrolidin-1-yl, 1-piperidinyl, 2-oxo-piperidin-1-yl, morpholino, 4-(2-hydroxyethyl)-1-piperazinyl, 2-(2-dimethylaminoethyl)-1-piperidinyl, 4-methyl-1-piperazinocarbonyl, 3-carboxy-2-methoxy-phenyl, 2-hydroxyphenyl, 3-hydroxyphenyl, 4-hydroxyphenyl, 4-(aminosulphonyl)phenyl, 4-nitrophenyl, 3-methoxycarbonylphenyl, 2-(2-amino-1-ethyl)phenyl, 4-pyridyl, 1*H-*imidazol-1-yl, 1*H*-imidazol-4-yl, 1*H*-pyrazol-4-yl, 1-methyl-1*H*-pyrrol-2-yl, 1*H-*indol-3-yl, 6-methoxy-1*H*-benzoimidazol-2-yl, 4-(2*H*-pyridazin-3-on-6-yl)-phenyl or imidazolidin-2-on-1-yl group,
an ethyl group substituted in the 1-position by a carboxy group and additionally substituted in the 2 position by a hydroxy, aminocarbonyl, 2-chlorophenyl, 4-chlorophenyl, 1*H*-imidazol-4-yl or 4-hydroxyphenyl group,
an ethyl group substituted in the 1-position by an aminocarbonyl group and additionally substituted in the 2 position by a 4-methoxyphenyl group,
an ethyl group substituted in the 1-position by a 4-phenyl-1-butylaminocarbonyl group and additionally substituted in the 2 position by a phenyl group,
an ethyl group substituted in the 2 position by a hydroxy group and additionally substituted in the 2 position by a phenyl, 3-hydroxyphenyl, 4-hydroxyphenyl or 4-hydroxy-3-methoxyphenyl group,
an ethyl group substituted in the 1-position by a phenyl group and additionally substituted in the 2 position by a hydroxy or carboxy group,
an ethyl group substituted in the 1-position by a 3-pyridyl group and additionally substituted in the 2 position by an ethoxycarbonyl group,
an ethyl group substituted in the 1-position by a carboxy group and additionally substituted in the 2 position by two phenyl groups,
an n-propyl group substituted in the 2 position by a hydroxy group and additionally substituted in the 3 position by an amino, hydroxy or morpholino group,
an n-propyl group substituted in the 3 position by a methoxy, isopropylamino, methylamino, diethylamino, dibenzylamino, 1-pyrrolidinyl, 1-piperidinyl, morpholino, 4-methyl-1-piperazinyl, tert.-butyloxycarbonylamino, 2-oxo-1-pyrrolidinyl, 2-oxo-piperidin-1-yl, ethoxycarbonyl, 4-pyridyl, 4-amino-3,5-dichlorophenyl, 3-amino-1-propoxy, 2-(3-amino-1-propoxy)-1-ethoxy, 1*H*-imidazol-1-yl, 2-aza-bicyclo[2.2.1]hept-5-en-2-yl, 4-(3-amino-1-propyl)-1-piperazinyl or 2-diethylaminomethyl-1-piperidinyl group,
an n-butyl group substituted in the 4-position by a 4-hydroxyphenyl group,
an n-butyl group substituted in the 4-position by a dimethylamino group and additionally substituted in the 2 position by a phenyl group,
a 2-methyl-2-butyl group substituted in the 3 position by a phenylaminocarbonylamino or a 1-(4-pyridyl)-3-imidazolin-2-on-3-yl,
an n-pentyl group substituted in the 1-position by a carboxy group and additionally substituted in the 5 position by a benzyloxycarbonylamino group,
a 1-pentyl group substituted in the 5 position by a methoxycarbonyl group and additionally substituted in the 5 position by an acetylamino group,
an n-hexyl group substituted in the 6 position by a hydroxy, amino, tert.-butyloxycarbonylamino or N-methyl-N-phenethylamino group,
a cyclohexyl group substituted in the 2 position by a hydroxy, amino, dimethylamino or hydroxymethyl group,
a cyclohexyl group substituted in the 3 position by an amino or carboxy group,
a cyclohexyl group substituted in the 4-position by a hydroxy, amino, carboxy, 2-carboxyethyl, 3-carboxypropyl, methoxycarbonyl or dimethylamino group,
a cyclohexylmethyl group substituted in the 3 position of the cyclohexyl moiety by an aminomethyl or a tert.-butyloxycarbonylaminomethyl group,
a cyclohexylmethyl group substituted in the 4-position of the cyclohexyl moiety by an aminomethyl, dimethylaminomethyl or 2-carboxyethyl group,
a 4-piperidinyl group substituted in the 1-position by a methyl, benzyl or phenylsulphonyl group,
a 1-methyl-4-carboxy-4-piperidinyl group,
a 1-ethyl-3-piperidinyl, 1-benzyl-3-pyrrolidinyl or 5-(3-carboxy-1-propyl)-indan-2-yl) group,
optionally in the form of the tautomers, racemates, enantiomers, diastereomers and mixtures thereof, and optionally also the pharmacologically acceptable acid addition salts thereof.

5. Compounds of formula I according to one of claims 1 to 4, wherein:
R_{b} denotes a phenyl group optionally substituted in the 4 position of the phenyl moiety by a fluorine, chlorine or bromine atom, by a cyano, propyl-2-sulphonyl, aminosulphonyl, dimethylaminosulphonyl, carboxy, piperidinomethyl, 1,2,4,5-tetrahydro-benzo[*d*]azepin-3-yl-methyl, 2-carboxy-1-ethyl, phenylaminocarbonyl, benzylaminocarbonyl, C₁₋₃-alkylsulphonyl, aminocarbonyl, methoxycarbonyl, (N-methyl-N-methylsulphonyl)amino, diethylaminomethyl, 3-diethylamino-1-propyloxy, morpholino, 4-methyl-1-piperazinyl, 2-H-tetrazol-5-yl, 1-H-imidazol-4-yl or nitro group, or a group of the formula
-CH₂-NH-CH₂-C₂ₛFₛ₊₁
wherein s denotes 1 or 2,
or a phenyl group substituted in the 3 position of the phenyl moiety by a chlorine or bromine atom, a cyano, aminocarbonyl, carboxy, ethoxycarbonyl or nitro group, or a 3,4-dichlorophenyl, 3,5-dichlorophenyl, 4-amino-3,5-dichlorophenyl, 3-chloro-4-fluorophenyl, 4-chloro-3-methylphenyl, 4-chloro-3-trifluoromethylphenyl, 4-bromo-3-chlorophenyl, 3-hydroxy-4-methylphenyl group, benzotriazol-5-yl, benzimidazol-5-yl, indazol-5-yl or indazol-6-yl or a group of the formula 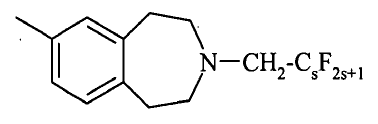 wherein s denotes 1 or 2.

6. Compounds of formula I according to one of claims 1 to 5, wherein:
the group R_{c}NR_{d}
is selected from the following groups:
2-amino-1-ethylamino, 2-acetylamino-ethylamino, 2-aminocarbonyl-1-ethylamino, 2-methoxy-1-ethylamino, 2-morpholino-1-ethylamino, 3-aminopropyl-amino, 1-carboxy-2-propylamino, 4-aminobutylamino, 5-hydroxy-1-pentylamino, 3-(3-aminopropoxy-1-propylamino, 2-(3-hydroxyphenyl)-1-ethyl-amino, 2-(4-hydroxy-3-methoxy-phenyl)-2-hydroxy-1-ethylamino, 2-(2-(2-amino-1-ethyl)-phenyl)-1-ethylamino, 4-hydroxy-cyclohexylamino, 3-amino-cyclohexylamino, 4-aminomethyl-cyclohexylmethylamino, 4-dimethylamino-cyclohexylamino, 1-methyl-piperidin-4-yl-methylamino, N-(4-methyl-piperidin-4-yl)-N-methyl-amino, 3-(2-oxo-pyrrolidin-1-yl)-propyl-1-amino, 1,4,6,7-tetrahydro-imidazo[4,5-c]pyridin-5-yl, 2-hydroxymethyl-pyrrolidin-1-yl, 4-aminomethyl-piperidin-1-yl, 3-hydroxymethyl-piperidin-1-yl, 3-acetylaminomethyl-piperidin-1-yl, 4-(N-acetyl-N-methylaminomethyl)-piperidin-1-yl, 3-(4-(pyrrolidin-1-yl)butyl)-piperidin-1-yl, 3-(2-aza-bicyclo[2.2.1]hept-5-en-2-yl)-propylamino and 7-methyl-2,7-diaza-spiro[4.4]non-2-yl.

7. Physiologically acceptable salts of the compounds of formula I according to one of claims 1 to 6.

8. Compounds of formula I according to one of claims 1 to 6 for use as medicaments.

9. Process for preparing the compounds of formula I according to one of claims 1 to 6, **characterised in that**
a. a compound of general formula 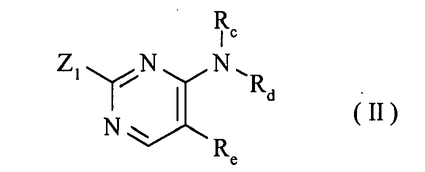 wherein
R_{c} to Rₑ are defined as in claims 1 to 6 and
Z₁ denotes a leaving group, is reacted with an amine of general formula
H-(RₐNR_{b}) (III)
wherein
Rₐ and R_{b} are defined as in claims 1 to 6; or
b. a compound of general formula IV 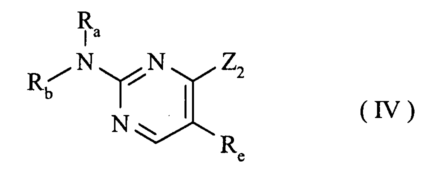
wherein
Rₐ, R_{b} and Rₑ are defined as in claims 1 to 6, and
Z₂ denotes a leaving group, is reacted with an amine of general formula
H-(R_{c}NR_{d}) (V)
wherein
R_{c} and R_{d} are defined as in claims 1 to 6.

10. Use of compounds of formula (I) according to one of claims 1 to 6 for preparing a drug for the treatment and prevention of diseases **characterised by** excessive or abnormal cell proliferation.

11. Pharmaceutical composition, **characterised in that** it contains one or more compounds according to one of claims 1 to 6.

## Revendications

1. Pyrimidines trisubstituées de formule I : 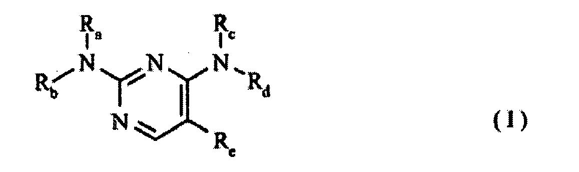 dans laquelle
Rₐ représente un atome d'hydrogène,
R_{b} représente un groupe phényle éventuellement substitué par les restes R₁ à R₃, où
R₁ et R₂ représentent chacun indépendamment,
un atome de fluor, de chlore, de brome ou d'iode, ou
un groupe alkyle en C₁₋₂ ou hydroxy,
un groupe cycloalkyle en C₃₋₇ ou cycloalcoxy en C₄₋₇, qui peuvent être substitués chacun par un ou deux groupes alkyle ou par un groupe aryle,
un groupe alcényle en C₂₋₅ éventuellement substitué par un groupe aryle,
un groupe alcynyle en C₂₋₅ éventuellement substitué par un groupe aryle,
un groupe aryle, aryloxy, aralkyle, aralcoxy, alkylsulfényle, alkylsulfinyle, alkylsulfonyle, alkylsulfonyloxy, trifluorométhylsulfényle, trifluorométhylsulfinyle, trifluorométhylsulfonyle, arylsulfényle, arylsulfinyle, arylsulfonyle, aralkylsulfényle, aralkylsulfinyle ou aralkylsulfonyle,
un groupe méthyle ou méthoxy substitué par 1 à 3 atomes de fluor,
un groupe alkyle en C₂₋₄ ou alcoxy en C₂₋₄ substitué par 1 à 5 atomes de fluor,
un groupe nitro, amino, alkylamino, dialkylamino, (cycloalkyle en C₃₋₇)amino, N-alkyl-N-(cycloalkyle en C₃₋₇)amino, arylamino, N-alkyl-N-arylamino, aralkylamino ou N-alkyl-aralkylamino,
un groupe alkylèneimino à 4 à 7 chaînons, éventuellement substitué par 1 à 4 groupes alkyle, où, dans les groupes alkylèneimino à 5 à 7 chaînons mentionnés, un ou deux des groupes méthylène voisins de l'atome d'azote peuvent être remplacés par un groupe carbonyle ou, dans les groupes alkylèneimino à 6 à 7 chaînons mentionnés, un groupe méthylène en position 4 peut être remplacé par un atome d'oxygène ou de soufre, par un groupe sulfinyle, sulfonyle, imino, N-alkylimino, N-alkylcarbonylimino, N-alkylsulfonylimino, N-aryl-carbonylimino, N-aryl-sulfonylimino, N-arylimino ou N-aralkylimino,
un groupe (alkylèneimino)carbonyle ou (alkylèneimino)sulfonyle à 4 à 7 atomes cycliques dans la partie alkylèneimino, éventuellement substitué par 1 à 4 groupes alkyle, où, dans les parties alkylèneimino à 6 à 7 chaînons mentionnés, chaque fois un groupe méthylène en position 4 peut être remplacé par un atome d'oxygène ou de soufre, par un groupe sulfinyle, sulfonyle, imino, N-alkylimino, N-alkylcarbonylimino, N-alkylsulfonylimino, N-aryl-carbonylimino, N-aryl-sulfonyl-imino, N-arylimino ou N-aralkylimino,
un groupe alkylcarbonylamino, N-alkyl-alkylcarbonylamino, alkylsulfonylamino, N-alkyl-alkylsulfonylamino, arylcarbonylamino, N-alkylarylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, perfluoroalkylsulfonylamino, N-alkyl-perfluoroalkylsulfonylamino, alkylcarbonyle, arylcarbonyle, aralkylcarbonyle, arylhydroxyméthyle, aralkylhydroxyméthyle, carboxy, alcoxycarbonyle, aralcoxycarbonyle, aminocarbonyle, alkylaminocarbonyle, dialkylaminocarbonyle, arylaminocarbonyle, N-alkyl-arylaminocarbonyle, aralkylaminocarbonyle, N-alkyl-aralkylaminocarbonyle, N-hydroxyaminocarbonyle, N-hydroxy-alkylaminocarbonyle, N-alcoxyaminocarbonyle, N-alcoxy-alkylaminocarbonyle, cyano, azido, N-cyanoamino ou N-cyanoalkylamino,
un groupe sulfo, alcoxysulfonyle, aminosulfonyle, alkylaminosulfonyle, dialkylaminosulfonyle, arylaminosulfonyle, pyridylaminosulfonyle, pyrimidinylaminosulfonyle, N-alkyl-arylaminosulfonyle, aralkylaminosulfonyle ou N-alkyl-aralkylaminosulfonyle,
un groupe phosphono, O-alkylphosphono, O,O'-dialkylphosphono, O-aralkylphosphono ou O,O'-diaralkylphosphono,
un groupe alkyle en C₁₋₂substitué par R₄, où
R₄ représente un groupe hydroxy, alcoxy, aryloxy, aralcoxy, amino, alkylamino, halogénoalkylamino, dialkylamino, alkylsulfényle, alkylsulfinyle, alkylsulfonyle, arylsulfényle, arylsulfinyle, arylsulfonyle, aralkylsulfényle, aralkylsulfinyle, aralkylsulfonyle, carboxy, alcoxycarbonyle, aminocarbonyle, alkylaminocarbonyle, dialkylaminocarbonyle ou cyano,
un groupe un groupe alkylèneimino à 4 à 7 chaînons, éventuellement substitué par 1 à 4 groupes alkyle, où, dans les groupes alkylèneimino à 5 à 7 chaînons mentionnés, un ou deux des groupes méthylène voisins de l'atome d'azote peuvent être remplacés par un groupe carbonyle ou, dans les groupes alkylèneimino à 6 à 7 chaînons mentionnés, un groupe méthylène en position 4 peut être remplacé par un atome d'oxygène ou de soufre, par un groupe sulfinyle, sulfonyle, imino, N-alkylimino, N-alkylcarbonylimino, N-alkylsulfonylimino, N-aryl-carbonylimino, N-aryl-sulfonylimino, N-arylimino ou N-aralkylimino, ou
un groupe alkylèneimino à 4 à 7 chaînons, éventuellement substitué par 1 à 4 groupes alkyle, où, dans les groupes alkylèneimino à 5 à 7 chaînons mentionnés, un ou deux des groupes méthylène voisins de l'atome d'azote peuvent être substitués par un groupe carbonyle ou, dans les groupes alkylèneimino à 6 à 7 chaînons mentionnés, par un ou deux groupes hydroxy, alcoxy, aminocarbonyle, alkylaminocarbonyle, dialkylaminocarbonyle, amino, alkylamino et dialkylamino,
un groupe (alkylèneimino)carbonyle à 4 à 7 atomes cycliques dans la partie alkylèneimino, éventuellement substitué par 1 à 4 groupes alkyle, où, dans les parties alkylèneimino à 6 à 7 chaînons mentionnées, un groupe méthylène en position 4 peut être remplacé par un atome d'oxygène ou de soufre, par un groupe sulfinyle, sulfonyle, imino, N-alkylimino, N-alkylcarbonylimino, N-alkylsulfonylimino, N-aryl-carbonylimino, N-aryl-sulfonylimino, N-arylimino ou N-aralkylimino, ou
un groupe de la formule : 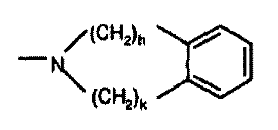 dans laquelle
h et k, qui peuvent être identiques ou différents, représentent les nombres 1 à 3, ou h le nombre 0 et k le nombre 2, 3 ou 4, où, en plus, la partie benzo ci-dessus peut être mono- ou disubstituée par un atome de fluor, de chlore, de brome ou d'iode, par un groupe alkyle, trifluorométhyle, hydroxy, alcoxy, carboxy ou cyano, où les substituants peuvent être identiques ou différents et la partie alkylèneimino cyclique saturée ci-dessus peut être substituée par 1 ou 2 groupes alkyle,
R₃ représente un atome de fluor, de chlore ou de brome, un groupe alkyle en C₁₋₂, alcoxy en C₁₋₂ ou trifluorométhyle, ou
un cycle aromatique hétérocyclique à 5 ou 6 chaînons, avec au moins un atome d'azote et éventuellement un atome de soufre ou d'oxygène, qui peut être substitué par un ou deux restes alkyle, aryle ou aralkyle, ou
un groupe sulfo, alcoxysulfonyle, aminosulfonyle, alkylaminosulfonyle, dialkylaminosulfonyle, arylaminosulfonyle, pyridylaminosulfonyle, pyrimidinylaminosulfonyle, N-alkyl-arylaminosulfonyle, aralkylaminosulfonyle ou N-alkyl-aralkylaminosulfonyle,
R₂ avec R₃, dans la mesure où ceux-ci sont liés à des atomes de carbone voisins l'un de l'autre, représentent un groupe méthylènedioxy éventuellement substitué par un ou deux groupes alkyle, ou
un groupe n-alkylène en C₃₋₆, éventuellement substitué par un ou deux groupes alkyle, dans lequel un groupe méthylène peut être remplacé par un atome d'oxygène ou de soufre, par un groupe sulfinyle, sulfonyle, N-alkylcarbonylimino, N-alkylsulfonylimino, N-arylcarbonylimino ou N-arylsulfonylimino, ou
un groupe 1,3-butadiène-1,4-diyle éventuellement substitué par un ou deux atomes de fluor, de chlore, de brome ou d'iode, par un ou deux groupes hydroxy, alkyle, alcoxy, trifluorométhyle, carboxy, alcoxycarbonyle, aminocarbonyle, alkylaminocarbonyle, dialkylaminocarbonyle ou cyano, où les substituants peuvent être identiques ou différents, ou
un groupe de la formule :
-(CH₂)ₘ-N(R₅)-(CH₂)ₙ-
dans lequel
les groupes méthylène de la partie alkylèneimino cyclique ainsi formée, peuvent être substitués par 1 ou 2 groupes alkyle,
R₅ représente un atome d'hydrogène ou un groupe alkyle, halogénoalkyle, aryle ou aralkyle, et
n et m, qui peuvent être identiques ou différents, représentent les nombres 1, 2 ou 3, où, dans la partie alkylèneimino ainsi formée, un ou deux groupes méthylène voisins de l'atome d'azote peuvent être remplacés par un groupe carbonyle, ou
m représente le nombre 0 et n le nombre 2, 3 ou 4, où, dans la partie alkylèneimino ainsi formée, le groupe méthylène voisin de l'atome d'azote peut être remplacé par un groupe carbonyle, ou
R₂ avec R₃ représentent un groupe de formule -NH-C(=O)-(CH₂)-, -NH-C(=O)-(CH₂)₂-, -NH-N=N-, -NH-N=CH-, -NH-CH=N-, -O-CH=N-, -S-CH=N- ou -NH-CH=CH- et les tautomères des systèmes cycliques définis par -NH-N=N-, -NH-N=CH-, -NH-CH=N-, où chaque atome d'hydrogène peut être remplacé par un groupe alkyle, aryle ou aralkyle,
R_{c}NR_{d} représente un groupe alkylèneimino à 4 à 8 chaînons, éventuellement substitué par 1 à 4 groupes alkyle ou 1 à 2 groupes aryle, qui est en outre substitué par le reste R₆, où R₆ représente un groupe carboxy, alcoxycarbonyle, aminocarbonyle, alkylaminocarbonyle, dialkylaminocarbonyle, cyano, hydroxy, alcoxy, aryloxy, aralcoxy, alkylcarbonyloxy, arylcarbonyloxy, amino, alkylamino, hydroxy(alkyle en C₂₋₄)amino, dialkylamino, cyanamino, formylamino, N-alkyl-N-(hydroxyalkyle en C₂₋₄)amino, bis(hydroxyalkyle en C₂₋₄)amino, alkylsulfényle, alkylsulfinyle, alkylsulfonyle, arylsulfényle, arylsulfinyle, arylsulfonyle, aralkylsulfényle, aralkylsulfinyle, aralkylsulfonyle, alkylcarbonylamino, N-alkyl-alkylcarbonylamino, alkylsulfonylamino, N-alkyl-alkylsulfonylamino, arylcarbonylamino, N-aryl-alkylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, alcoxycarbonylamino, N-alkyl-alcoxycarbonylamino, alcoxycarbonylalkylamino, N-alkyl-N-(alcoxycarbonylalkyl)amino, aralcoxycarbonylamino, N-alkyl-aralcoxycarbonylamino,
un groupe (NR₈R₉)CONR₇- ou (NR₈R₉)SO₂NR₇-, dans lequel R₇, R₈ et R₉, qui peuvent être identiques ou différents, représentent chacun un atome d'hydrogène ou un groupe alkyle, aryle ou pyridyle, ou R₇ et R₈ représentent ensemble un groupe n-alkylène en C₂₋₄ et R₉ représente un atome d'hydrogène ou un groupe alkyle, aryle ou pyridyle,
un groupe un groupe (alkylèneimino)carbonyle à 4 à 7 atomes cycliques dans la partie alkylèneimino, éventuellement substitué par 1 à 4 groupes alkyle, où, dans les parties alkylèneimino à 6 à 7 chaînons mentionnées, un groupe méthylène en position 4 peut être remplacé par un atome d'oxygène ou de soufre, par un groupe sulfinyle, sulfonyle, imino, N-alkylimino, N-alkylcarbonylimino, N-alkylsulfonylimino, N-arylimino ou N-aralkylimino,
un groupe alkylèneimino à 4 à 7 chaînons, éventuellement substitué par 1 à 4 groupes alkyle ou un groupe hydroxyalkyle, où, dans les groupes alkylèneimino à 5 à 7 chaînons mentionnés, un ou deux des groupes méthylène voisins de l'atome d'azote peuvent être remplacés par un groupe carbonyle,
un groupe alkylèneimino à 6 ou 7 chaînons, éventuellement substitué par 1 à 4 groupes alkyle ou un groupe hydroxyalkyle, où le groupe méthylène en position 4 dans la partie alkylèneimino est remplacé par un atome d'oxygène ou de soufre, par un groupe carbonyle, sulfinyle, sulfonyle, imino, N-alkylimino, N-alkylcarbonylimino, N-alkylsulfonylimino, N-arylimino ou N-aralkylimino, et en outre, dans la partie alkylèneimino des groupes cités, un ou deux des groupes méthylène voisins de l'atome d'azote peuvent être remplacés par un groupe carbonyle,
un groupe alkylèneimino à 4 à 7 chaînons, substitué par un groupe hydroxy, alcoxy, amino, alkylamino, dialkylamino, alkylcarbonylamino, N-alkyl-alkylcarbonylamino, alkylsulfonylamino, N-alkyl-alkylsulfonylamino, carboxy, alcoxycarbonyle, aminocarbonyle, alkylaminocarbonyle ou dialkylaminocarbonyle,
un groupe alkyle substitué par un groupe carboxy, alcoxycarbonyle, aminocarbonyle, alkylaminocarbonyle ou dialkylaminocarbonyle, cyano, hydroxy, alcoxy aryloxy, amino, alkylamino, dialkylamino, alkylcarbonylamino, N-alkyl-alkylcarbonylamino, alcoxycarbonylamino, N-alkyl-alcoxycarbonylamino, alkyl-sulfonylamino, N-alkyl-alkylsulfonylamino, arylcarbonylamino, N-alkyl-arylcarbonylamino, arylsulfonylamino, N-alkyl-arylsulfonylamino, aminocarbonylalkylamino, N-(alkyl)-N-(aminocarbonylalkyl)amino, alkylaminocarbonylalkylamino, N-(alkyl)-N-(alkylaminocarbonylalkyl)amino, dialkylaminocarbonylalkylamino, N-(alkyl)-N-(dialkylaminocarbonylalkyl)amino, dialkylaminoalcoxy, alkylsulfényle, alkylsulfinyle, alkylsulfonyle, arylsulfényle, arylsulfinyle ou arylsulfonyle,
un groupe (alkylèneimino)alkyle à 4 à 7 atomes cycliques dans la partie alkylèneimino, éventuellement substitué par 1 à 4 groupes alkyle, où, dans les parties alkylèneimino à 6 à 7 chaînons mentionnées, un groupe méthylène en position 4 peut être remplacé par un atome d'oxygène ou de soufre, par un groupe sulfinyle, sulfonyle, imino, N-alkylimino ou N-alkylcarbonylimino,
un groupe (alkylèneimino)carbonylalkyle à 4 à 7 atomes cycliques dans la partie alkylèneimino, éventuellement substitué par 1 à 4 groupes alkyle, où, dans les parties alkylèneimino à 6 à 7 chaînons mentionnées, un groupe méthylène en position 4 peut être remplacé par un atome d'oxygène ou de soufre, par un groupe sulfinyle, sulfonyle, imino ou N-alkylimino,
un groupe (carboxyalkyl)oxy, (alcoxycarbonyalkyl)oxy, (aminocarbonyalkyl)oxy, (alkylaminocarbonyalkyl)oxy ou (dialkylaminocarbonyalkyl)oxy,
un groupe [(alkylèneimino)carbonylalkyl]oxy à 4 à 7 atomes cycliques dans la partie alkylèneimino, où, dans les parties alkylèneimino à 6 à 7 chaînons mentionnées, un groupe méthylène en position 4 peut être remplacé par un atome d'oxygène ou de soufre, par un groupe sulfinyle, sulfonyle, imino ou N-alkylimino,
un groupe cycloalkyle en C₅₋₇, dans lequel un groupe méthylène est remplacé par un atome d'oxygène ou de soufre, par un groupe sulfinyle, sulfonyle, imino ou N-alkylimino, alkylcarbonylimino, alkylsulfonylimino,
un groupe 3,4-dihydro-1*H*-quinazolin-2-on-3-yle ou 1*H*-benzimidazol-2-on-1-yle, éventuellement substitué dans la partie aryle par un ou deux atomes de fluor, de chlore, de brome ou d'iode ou un ou deux groupes nitro, alkyle, alcoxy ou cyano, où les substituants peuvent être identiques ou différents, ou
R_{c}NR_{d} représente un groupe alkylèneimino à 6 à 8 chaînons, éventuellement substitué par 1 à 4 groupes alkyle ou par un groupe aryle, qui peut être en outre substitué par le reste R₆, où, dans les parties alkylèneimino mentionnées, le groupe méthylène en position 4 peut être remplacé par un atome d'oxygène ou de soufre, par un groupe carbonyle, sulfinyle, sulfonyle, N-oxydo-N-alkylimino ou R₁₀N, où
R₁₀ représente un atome d'hydrogène, un groupe alkyle, hydroxyalkyle en C₂₋₄, (alcoxy)alkyle en C₂₋₄, aminoalkyle en C₂₋₄, (alkylamino)alkyle en C₂₋₄, dialkylamino-alkyle en C₂₋₄, (hydroxyalcoxy en C₂₋₄)alkyle en C₂₋₄, aminocarbonylalkyle, alkylaminocarbonylalkyle, dialkylaminocarbonylalkyle, aryle, formyle, alkylcarbonyle, alkylsulfonyle, arylcarbonyle, arylsulfonyle, aralkylcarbonyle, aralkylsulfonyle, alcoxycarbonyle, aralcoxycarbonyle, cyano, aminocarbonyle, alkylaminocarbonyle ou dialkylaminocarbonyle, un groupe aminoalkylcarbonyle, alkylaminoalkylcarbonyle, (dialkylamino)alkylcarbonyle,
un groupe alkyle substitué par un, deux ou trois groupes aryle,
un groupe 8-alkyl-8-azabicyclo[3.2.1]oct-3-yle,
un groupe aryle ou un groupe 2-, 3- ou 4-pyridyle ou un groupe 2-, 4- ou 5-pyrimidinyle,
un groupe (alkylèneimino)carbonyle ou (alkylèneimino)carbonylalkyle à 4 à 7 atomes cycliques dans la partie alkylèneimino, où, dans les parties alkylèneimino à 6 à 7 chaînons mentionnées, chaque groupe méthylène en position 4 peut être remplacé par un atome d'oxygène ou de soufre, par un groupe sulfinyle, sulfonyle, imino, N-alkylimino, N-alkylcarbonylimino ou N-aralkylimino, ou
R_{c}NR_{d} représente un groupe 3-thiazolidinyle substitué en position 4 par un groupe carbonyle, alcoxycarbonyle, aminocarbonyle, alkylaminocarbonyle, dialkylaminocarbonyle, ou
R_{c}NR_{d} représente un groupe 1-pyrrolidinyle éventuellement substitué par 1 à 4 groupes alkyle, dans lequel deux atomes d'hydrogène sur le squelette carboné sont remplacés par un pont alkylène linéaire, où ce pont contient 2 à 6 atomes de carbone, lorsque les deux atomes d'hydrogène se trouvent sur le même atome de carbone, ou contient 1 à 5 atomes de carbone, lorsque les deux atomes d'hydrogène se trouvent sur des atomes de carbone voisins, ou contient 2 à 4 atomes de carbone, lorsque les deux atomes d'hydrogène se trouvent sur des atomes de carbone, qui sont séparés par un atome, où les groupes 1-pyrrolidinyle mentionnés sont substitués en outre, par le reste R₆, qui est défini comme mentionné ci-dessus,
un groupe 1-pipéridinyle ou 1-azacyclohept-1-yle, éventuellement substitué par 1 à 4 groupes alkyle, dans lequel deux atomes d'hydrogène sur le squelette carboné sont remplacés par un pont alkylène linéaire, où ce pont contient 2 à 6 atomes de carbone lorsque les deux atomes d'hydrogène se trouvent sur le même atome de carbone, ou contient 1 à 5 atomes de carbone lorsque les deux atomes d'hydrogène se trouvent sur des atomes de carbone voisins, ou contient 1 à 4 atomes de carbone lorsque les deux atomes d'hydrogène se trouvent sur des atomes de carbone, qui sont séparés par un atome, ou contient 1 à 3 atomes de carbone lorsque les deux atomes d'hydrogène se trouvent sur des atomes de carbone, qui sont séparés par deux atomes, où les groupes 1-pipéridinyle et 1-azacyclohept-1-yle mentionnés sont substitués en outre, par le reste R₆, qui est défini comme mentionné ci-dessus,
un groupe 1-pyrrolidinyle, éventuellement substitué par 1 à 4 groupes alkyle, dans lequel deux atomes d'hydrogène en position 3 sont remplacés par un groupe -O-CH₂-CH₂-O- ou -O-CH₂CH₂CH₂-O-,
un groupe 1-pipéridinyle ou 1-azacyclohept-1-yle, éventuellement substitué par 1 à 4 groupes alkyle, dans lequel deux atomes d'hydrogène en position 3 ou en position 4 sont substitués par un groupe -O-CH₂CH₂-O- ou -O-CH₂CH₂CH₂-O-,
un groupe 1-azétidinyle, éventuellement substitué par un groupe alkyle, dans lequel les deux atomes d'hydrogène d'un groupe méthylène sont remplacés par un pont alkylène en C₄₋₆ linéaire, où un groupe méthylène dans le pont alkylène en C₄₋₆ est remplacé par un groupe R₁₀N, où R₁₀ est défini comme précédemment, où le bicycle ainsi formé peut être substitué en outre, par un groupe hydroxy, alcoxy, amino, alkylamino, dialkylamino, cyano, alkylcarbonylamino, alkylsulfonylamino, alcoxycarbonylamino, arylcarbonyle, carboxy, alcoxycarbonyle, aminocarbonyle, alkylaminocarbonyle ou dialkylaminocarbonyle,
un groupe 1-pyrrolidinyle, 1-pipéridinyle ou 1-azacyclohept-1-yle, éventuellement substitué par 1 ou 2 groupes alkyle, dans lequel les deux atomes d'hydrogène d'un groupe méthylène sont remplacés par un pont alkylène en C₃₋₆ linéaire, où un groupe méthylène dans le pont alkylène en C₃₋₆ est remplacé par un groupe R₁₀N, où R₁₀ est défini comme précédemment, où le bicycle ainsi formé peut être substitué en outre, par un groupe hydroxy, alcoxy, amino, alkylamino, dialkylamino, cyano, alkylcarbonylamino, alkylsulfonylamino, alcoxycarbonylamino, arylcarbonyle, carboxy, alcoxycarbonyle, aminocarbonyle, alkylaminocarbonyle ou dialkylaminocarbonyle,
un groupe éventuellement substitué dans la partie alkylène, par 1 ou 2 groupes alkyle, de structure : 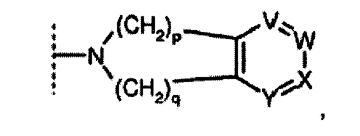 où
p et q, qui peuvent être identiques ou différents, représentent le nombre 1 ou 2, et
l'unité V=W-X=Y représente un des groupes (a), (b), (c), (d) ou (e) :
-N=C-C=C- (a)
-C=N-C=C- (b)
-C=N-N=C- (c)
-N=C-C=N- (d)
-N=C-N=C- (e)
ou -V=W- représentent ensemble un atome d'oxygène ou de soufre et -X=Y-représentent un des groupes -N=C-, -C=N- ou -C=C-,
ou -V=W- représentent ensemble un groupe imino, N-alkylimino, N-aralkylimino ou N-arylimino et -X=Y- représentent un des groupes -N=N-, -N=C-, - C=N- ou -C=C-,
ou dans la mesure où p et q ne sont pas égaux,
-X=Y- représentent ensemble, un atome d'oxygène ou de soufre et -V=W-représentent un des groupes -N=C-, -C=N- ou -C=C-,
ou -X=Y- représentent ensemble, un groupe imino, N-alkylimino, N-aralkylimino ou N-arylimino et -V=W- représentent un des groupes -N=N-, -N=C-, -C=N- ou -C=C-,
où un ou deux des atomes de carbone disponibles de l'unité -V=W-X=Y-peuvent être substitués chacun par un groupe hydroxy, alcoxy, amino, alkylamino, dialkylamino, carboxy, alcoxycarbonyle, aminocarbonyle, alkylaminocarbonyle, dialkylaminocarbonyle ou hydrazinocarbonyle, où les substituants peuvent être identiques ou différents, et les atomes de carbone disponibles restants de l'unité -V=W-X=Y- sont substitués par un atome d'hydrogène, un groupe alkyle, aralkyle ou aryle,
ou
R_{c} représente un atome d'hydrogène ou un groupe alkyle en C₁₋₈,
un groupe cycloalkyle en C₃₋₇, (cycloalkyle en C₃₋₇)alkyle ou aralkyle, qui peuvent être chacun substitués par un ou deux groupes alkyle ou par un groupe aryle, un groupe alkyle, qui est substitué
par un groupe hydroxy, alcoxy, aryloxy, aralcoxy, alkylsulfényle, alkylsulfinyle, alkylsulfonyle, arylsulfényle, arylsulfinyle, arylsulfonyle, amino, alkylamino, dialkylamino, alkylcarbonylamino, N-alkyl-alkylcarbonylamino, alkyl-sulfonylamino, N-alkyl-alkylsulfonylamino, trifluorométhylsulfonylamino, N-alkyl-trifluorométhylsulfonylamino, carboxy, alcoxycarbonyle, aralcoxycarbonyle, aminocarbonyle, alkylaminocarbonyle, dialkylaminocarbonyle, cyano,
par un groupe 2-, 3- ou 4-pyridyle,
par un groupe alkylèneimino ou (alkylèneimino)carbonyle à 4 à 7 atomes cycliques dans la partie alkylèneimino, éventuellement substitué par 1 à 4 groupes alkyle, où, dans les parties alkylèneimino à 6 à 7 chaînons mentionnées, un groupe méthylène en position 4 peut être remplacé par un atome d'oxygène ou de soufre, par un groupe imino, N-alkylimino, N-arylimino, N-aralkylimino, N-arylcarbonylimino ou N-alkylcarbonylimino,
un groupe alcényle en C₃₋₅, éventuellement substitué par un groupe aryle, où la partie vinyle ne peut pas être reliée par l'atome d'azote au groupe R_{c}NR_{d},
un groupe alcynyle en C₃₋₅, éventuellement substitué par un groupe aryle, où la partie éthynyle ne peut pas être reliée par l'atome d'azote au groupe R_{c}NR_{d}, et
R_{d} représente un groupe alkyle en C₁₋₁₆, qui est substitué par un groupe choisi parmi les groupes (a) à (n) :
(a) un groupe carboxy, alcoxycarbonyle, aminocarbonyle, alkylaminocarbonyle, dialkylaminocarbonyle, hydroxy(alkyle en C₂₋₄)-aminocarbonyle, cyano, hydroxy, alcoxy, aryloxy, aralcoxy, (alkylène en C₂₋₄)dioxy, alkylcarbonyloxy, arylcarbonyloxy, formylamino, alkylcarbonylamino, arylcarbonylamino, amino, alkylamino, dialkylamino, naphtylamino, aralkylamino, diaralkylamino ou N-alkyl-aralkylamino,
(b) un groupe phénylamino, N-alkyl-N-phénylamino, pyridylamino ou N-alkyl-N-pyridylamino, éventuellement substitué dans la partie aryle, par un ou deux atomes de fluor, de chlore, de brome ou d'iode, ou un ou deux groupes nitro, trifluorométhyle, alkyle, hydroxy, alcoxy, amino, alkylamino, dialkylamino, carboxy, alcoxycarbonyle, aminocarbonyle, alkylaminocarbonyle, dialkylaminocarbonyle ou cyano, où les substituants peuvent être identiques ou différents,
(c) un groupe alcoxy substitué par un, deux ou trois restes aryle,
(d) un groupe hydroxy(alkyle en C₂₋₄)aminocarbonyle, alcoxy(alkyle en C₂₋₄)aminocarbonyle, amino(alkyle en C₂₋₄)aminocarbonyle, alkylamino(alkyle en C₂₋₄)aminocarbonyle, dialkylamino(alkyle en C₂₋₄)aminocarbonyle, carboxyalkylaminocarbonyle, alcoxycarbonylalkylaminocarbonyle, aminocarbonylalkylaminocarbonyle, alkylaminocarbonylalkylaminocarbonyle, dialkylaminocarbonylalkylaminocarbonyle, arylaminocarbonyle, N-alkyl-arylaminocarbonyle, aralkylaminocarbonyle, N-alkyl-aralkylaminocarbonyle,
(e) un groupe de formule -C(=NH)NH₂ ou -NH-C(=NH)NH₂, qui est substitué éventuellement par un groupe cyano ou alcoxycarbonyle,
(f) un groupe (alkylèneimino)carbonyle à 4 à 7 atomes cycliques dans la partie alkylèneimino, éventuellement substitué par 1 à 4 groupes alkyle, où, dans les parties alkylèneimino à 6 ou 7 chaînons mentionnés, le groupe méthylène en position 4 peut être remplacé par un atome d'oxygène ou de soufre, par un groupe sulfinyle, sulfonyle, imino, N-alkylimino, N-alkylcarbonylimino, N-alkylsulfonylimino, N-arylimino ou N-aralkylimino,
(g) un groupe alkylèneimino à 4 à 7 chaînons, éventuellement substitué par 1 à 4 groupes alkyle, où, dans les parties alkylèneimino de 6 ou 7 chaînons mentionnées, le groupe méthylène en position 4 peut être remplacé par un atome d'oxygène ou de soufre, par un groupe sulfinyle, sulfonyle ou R₁₀N, où R₁₀ est défini comme précédemment, et en outre, dans les parties alkylèneimino de 5 à 7 chaînons mentionnées, un ou deux groupes méthylène voisins des atomes d'azote peuvent être remplacés par un groupe carbonyle,
(h) un groupe alkylèneimino à 5 à 7 chaînons, éventuellement substitué par 1 à 2 groupes alkyle, qui est substitué par un groupe hydroxyalkyle, aminoalkyle, alkylaminoalkyle ou dialkylaminoalkyle,
(i) un groupe alkylsulfonylamino, N-alkyl-alkylsulfonylamino, arylcarbonylamino, N-alkyl-arylcarbonylamino, alkylcarbonylamino, N-alkyl-alkylcarbonylamino, arylsulfonylamino, N-alkyl-arylsulfonylamino, aralkylcarbonylamino, N-alkyl-aralkylcarbonylamino, alcoxyalkylcarbonylamino, alcoxyalkyl-N-alkylcarbonylamino, dialkylamino-alkylcarbonylamino, alkylamino-alkylcarbonylamino, amino-alkylcarbonylamino, aralkylsulfonylamino, N-alkyl-aralkylsulfonylamino, alcoxycarbonylamino, N-alkyl-alcoxycarbonylamino, aralcoxycarbonylamino ou N-alkyl-aralcoxycarbonylamino,
(j) un groupe (R₉NR₈)-CO-NR₇- ou (R₉NR₈)-SO₂-NR₇-, où R₇, R₈ et R₉ sont définis comme précédemment,
(k) un groupe alkylsulfényle, alkylsulfinyle, alkylsulfonyle, arylsulfényle, arylsulfinyle, arylsulfonyle, aralkylsulfényle, aralkylsulfinyle ou aralkylsulfonyle,
(l) un groupe cycloalkyle en C₄₋₇ substitué par R₆ et éventuellement en outre par 1 à 4 groupes alkyle, où R₆ est défini comme précédemment,
(m) un groupe cycloalkyle en C₅₋₇ substitué éventuellement par 1 à 4 groupes alkyle, dans lequel un groupe méthylène est remplacé par un atome d'oxygène ou de soufre, par un groupe sulfinyle, sulfonyle ou NR₁₀, où R₁₀ est défini comme précédemment,
(n) un groupe 4-pipéridinylalkyle substitué éventuellement par 1 à 4 groupes alkyle, qui est substitué en position 1 par R₁₀ et en outre, en position 4 par un groupe hydroxy, où R₁₀ est défini comme précédemment, et pour lequel, en outre, chaque fois un atome d'hydrogène en position 2 et 6 du squelette pipéridinyle est remplacé par un pont alkylène en C₂₋₃,
ou
un groupe méthyle substitué par un groupe 3-hydroxy-1,3-dihydroindol-2-on-3-yle ou 2-aminocarbonyl-1,3-dihydroisoindol-5-yle,
un groupe de structure 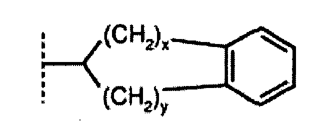 substitué, dans la partie aryle, par un groupe carboxy, alcoxycarbonyle, aminocarbonyle, alkylaminocarbonyle, dialkylaminocarbonyle, carboxyalkyle, alcoxycarbonylalkyle, aminocarbonylalkyle, alkylaminocarbonylalkyle ou dialkylaminocarbonylalkyle, et éventuellement en outre, dans la partie alkyle, par 1 ou 2 groupes alkyle,
où x et y, qui peuvent être identiques ou différents, représentent indépendamment l'un de l'autre, le nombre 0, 1 ou 2, où cependant x et y doivent faire ensemble, au moins le nombre 2,
un groupe alkyle en C₃₋₈, substitué par un groupe hydroxy et en outre, par un groupe amino, alkylamino, dialkylamino, hydroxy, alcoxy, 1-pyrrolidinyle, 1-pipéridinyle ou morpholinyle,
un groupe alkyle en C₂₋₈, substitué par un groupe carboxy et en outre, par un groupe amino, hydroxy, aminocarbonyle ou benzyloxycarbonylamino,
un groupe alkyle en C₂₋₄, qui est substitué par un groupe (alkyle en C₂₋₄)sulfényle ou alcoxy en C₂₋₄, qui est substitué en position ω par un groupe amino, hydroxy ou alcoxy,
un groupe alkyle en C₂₋₄, qui est substitué par un groupe (alcoxy en C₂₋₄)alcoxy en C₂₋₄, qui est substitué en position ω par un groupe amino ou hydroxy,
un groupe cyclopropyle, qui est substitué par un groupe carboxy, alcoxycarbonyle, aminocarbonyle, alkylaminocarbonyle ou dialkylaminocarbonyle, ou par un groupe (alkylèneimino)carbonyle avec 4 à 7 atomes cycliques dans la partie alkylèneimino, éventuellement substitué par 1 à 4 groupes alkyle, où, dans les parties alkylèneimino à 6 à 7 chaînons mentionnés, le groupe méthylène en position 4 peut être remplacé par un atome d'oxygène ou de soufre, par un groupe sulfinyle, sulfonyle, imino, N-alkylimino, N-alkylcarbonylimino, N-alkylsulfonylimino, N-arylimino ou N-aralkylimino,
un groupe cycloalkyle en C₄₋₇, éventuellement substitué par 1 à 4 groupes alkyle, qui est substitué en outre, par R₆, qui est défini comme précédemment,
un groupe cycloalkyle en C₅₋₇, éventuellement substitué par 1 à 2 groupes alkyle, qui est substitué en outre, par un groupe N,N-dialkyl-N-oxydoamino,
un groupe cycloalkyle en C₄₋₇, éventuellement substitué par 1 à 4 groupes alkyle, qui peut être substitué en outre, par R₆, où, dans la partie cycloalkyle, un groupe méthylène est remplacé par un atome d'oxygène ou de soufre, par un groupe sulfinyle, sulfonyle, N-alkyl-N-oxydoimino ou R₁₀N, où R₆ et R₁₀ sont définis comme précédemment,
un groupe cycloalkyle en C₅₋₇ ou (cycloalkyle en C₅₋₇)alkyle, éventuellement substitué par 1 à 4 groupes alkyle, dans lesquels un groupe méthylène dans la partie cycloalkyle, est remplacé par un groupe carbonyle,
un groupe cyclopentyle ou cyclopentylalkyle, éventuellement substitué par 1 à 4 groupes alkyle, dans lesquels deux atomes d'hydrogène dans la partie cyclopentyle sont remplacés par un pont alkylène linéaire, où ce pont contient 2 à 6 atomes de carbone lorsque les deux atomes d'hydrogène se trouvent sur le même carbone, ou contient 1 à 5 atomes de carbone lorsque les deux atomes d'hydrogène se trouvent sur des atomes de carbone voisins, ou contient 2 à 4 atomes de carbone lorsque les deux atomes d'hydrogène se trouvent sur des atomes de carbone, qui sont séparés par un atome de carbone, où les cycles mentionnés sont substitués en outre, par le reste R₆, qui est défini comme précédemment,
un groupe cyclohexyle, cyclohexylalkyle, cycloheptyle ou cycloheptylalkyle, éventuellement substitué par 1 à 4 groupes alkyle, dans lesquels deux atomes d'hydrogène dans la partie cycloalkyle sont remplacés par un pont alkylène linéaire, où ce pont contient 2 à 6 atomes de carbone lorsque les deux atomes d'hydrogène se trouvent sur le même carbone, ou contient 1 à 5 atomes de carbone lorsque les deux atomes d'hydrogène se trouvent sur des atomes de carbone voisins, ou contient 1 à 4 atomes de carbone lorsque les deux atomes d'hydrogène se trouvent sur des atomes de carbone qui sont séparés par un atome de carbone, ou contient 1 à 3 atomes de carbone lorsque les deux atomes d'hydrogène se trouvent sur des atomes de carbone, qui sont séparés par deux atomes de carbone, où les cycles mentionnés sont substitués en outre, par le reste R₆, qui est défini comme précédemment,
un groupe alkyle substitué par un groupe 3-hydroxy-1,3-dihydroindol-2-on-3-yle ou 2-aminocarbonyl-1,3-dihydroisoindol-5-yle,
un groupe alkyle en C₁₋₁₀ substitué par un groupe aryle, où la partie aryle mentionnée est substituée par un groupe alcoxycarbonyle, carboxy, carboxyalkyle, aminosulfonyle, trifluorométhoxy, cyano, aminoalkyle, amino, alkylamino, dialkylamino, nitro, 2H-pyridazin-3-on-6-yle, hydroxyphényle, hydroxyalkyle, hydroxy ou alcoxy,
un groupe aralkyle, qui est substitué dans la partie alkyle par un groupe hydroxy ou alcoxy et en outre, par un groupe carboxy, alcoxycarbonyle, hydroxy ou alcoxy,
un groupe alkyle en C₁₋₁₀ substitué par un groupe pyrrolyle, imidazolyle, pyrazolyle, pyridyle, pyrimidinyle, pyrazinyle, indolyle ou benzimidazolyle, où les parties hétéroaryle mentionnées peuvent être substituées sur les atomes de carbone disponibles, en outre par un ou deux groupes choisis parmi des atomes de fluor, de chlore, de brome ou d'iode, des groupes alkyle, alcoxycarbonyle, carboxy, trifluorométhyle, trifluorométhoxy, cyano, amino, alkylamino, dialkylamino, nitro, hydroxy ou alcoxy, où les substituants peuvent être identiques ou différents,
un groupe alkyle en C₁₋₁₀ substitué par un groupe carboxy, alcoxycarbonyle, aminocarbonyle, alkylaminocarbonyle, dialkylaminocarbonyle, aralkylaminocarbonyle, cyano, hydroxy, alcoxy, aryloxy, amino, alkylamino, dialkylamino, alkylcarbonylamino ou alcoxycarbonylamino, qui est substitué en outre, par un ou deux groupes aryle ou un groupe pyrrolyle, imidazolyle, pyrazolyle, pyridyle, pyrimidinyle, pyrazinyle, indolyle ou benzimidazolyle, où les parties aryle ou hétéroaryle mentionnées peuvent être substitués en outre, sur les atomes de carbone disponibles, par un ou deux groupes choisis parmi des atomes de fluor, de chlore, de brome ou d'iode, des groupes alkyle, alcoxycarbonyle, carboxy, trifluorométhyle, trifluorométhoxy, cyano, amino, alkylamino, dialkylamino, nitro, hydroxy ou alcoxy,
où les substituants peuvent être identiques ou différents,
un groupe alkyle en C₁₋₆ substitué par un groupe aryle, qui est substitué dans la partie aryle, par un groupe hydroxy ou amino et en outre, par deux atomes de fluor, de chlore, de brome ou d'iode, où les substituants peuvent être identiques ou différents,
un groupe alkyle en C₂₋₆ substitué par un groupe carboxy ou alcoxycarbonyle, qui est substitué en outre, par un groupe amino, alkylamino, dialkylamino, alkylcarbonylamino, N-alkyl-alkylcarbonylamino, alkylsulfonylamino, N-alkyl-alkylsulfonylamino, arylcarbonylamino, N-alkyl-arylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, alcoxycarbonylamino, N-alkyl-alcoxycarbonylamino, aralcoxycarbonylamino ou N-alkyl-aralcoxycarbonylamino,
un groupe 3-quinuclidinyle, 4-quinuclidinyle, 2-quinuclidinylalkyle, 3-quinuclidinylalkyle ou 4-quinuclidinylalkyle, ou
R_{c} représente un atome d'hydrogène ou un groupe alkyle et R_{d} représente un groupe hydroxy ou alcoxy, et
R_{c} représente un groupe nitro ou trifluorométhyle,
éventuellement sous la forme de leurs tautomères, leurs racémates, leurs énantiomères, leurs diastéréoisomères et leurs mélanges, ainsi que éventuellement leurs sels d'addition d'acide pharmacologiquement acceptables,
où, sauf indication contraire,
par parties aryle mentionnées dans la définition des restes indiqués ci-dessus, on entend des groupes phényle, dans lesquels un ou deux atomes de carbone peuvent être remplacés chacun, par un atome d'azote, où les parties aryle indiquées peuvent être monosubstituées par R₁₁, mono-, di- ou trisubstituées par R₁₂ ou monosubstituées par R₁₁, et en outre mono- ou disubstituées par R₁₂, où les substituants peuvent être identiques ou différents, et
R₁₁ représente un groupe cyano, carboxy, aminocarbonyle, alkylaminocarbonyle, dialkylaminocarbonyle, alcoxycarbonyle, alkylcarbonyle, alkylsulfényle, alkylsulfinyle, alkylsulfonyle, alkylsulfonyloxy, perfluoroalkyle, perfluoroalcoxy, nitro, amino, alkylcarbonylamino, N-alkyl-alkylcarbonylamino, alkylamino, dialkylamino, hydroxy(alkyle en C₂₋₄)amino, N-alkyl-hydroxy(alkyle en C₂₋₄)amino, bis(hydroxyalkyle en C₂₋₄)amino, phénylalkylcarbonylamino, phénylcarbonylamino, alkylsulfonylamino, phénylalkylsulfonylamino, phényl-sulfonylamino, N-alkyl-phénylalkylcarbonylamino, N-alkyl-phénylcarbonylamino, N-alkyl-alkylsulfonylamino, N-alkyl-phénylalkylsulfonylamino, N-alkyl-phényl-sulfonylamino, aminosulfonyl, alkylaminosulfonyl, dialkylaminosulfonyl, (R₉NR₈)-CO-NR₇- ou (R₉NR₈)-SO₂-NR₇-, où R₇, R₈ et R₉ sont définis comme précédemment,
un groupe alkylèneimino à 5 à 7 chaînons, éventuellement substitué par 1 à 4 groupes alkyle ou un groupe hydroxyalkyle, où, dans les groupes alkylèneimino à 6 à 7 chaînons mentionnés, un groupe méthylène en position 4 peut être remplacé par un atome d'oxygène ou un groupe R₁₀N, où R₁₀ est défini comme précédemment,
un groupe alkylèneimino à 5 à 7 chaînons, éventuellement substitué par 1 à 4 groupes alkyle ou un groupe hydroxyalkyle, où un ou deux groupes méthylène voisins de l'atome d'azote sont remplacés chacun par un groupe carbonyle,
R₁₂ représente un groupe alkyle, hydroxy ou alcoxy, un atome de fluor, de chlore, de brome ou d'iode, où deux restes R₁₂, dans la mesure où ceux-ci sont liés sur des atomes de carbone voisins, peuvent représenter également, un groupe alkylène à 3 à 6 atomes de carbone, un groupe 1,3-butadièe-1,4-diyle ou un groupe méthylènedioxy,
ainsi que sauf indication contraire, les parties alkyle, alkylène et alcoxy mentionnées précédemment contiennent chacune 1 à 4 atomes de carbone,
où, sauf indication contraire,
chaque atome de carbone dans les parties alkyle, alkylène ou cycloalkylène mentionnées précédemment, qui est lié à un atome d'azote, d'oxygène ou de soufre, ne peut pas être lié à un autre atome d'halogène, d'azote, d'oxygène ou de soufre.

2. Composés de formule I selon la revendication 1, dans lesquels
R_{b} représente un groupe phényle éventuellement substitué par les restes R₁ à R₃,
où
R₁ représente un atome de fluor, de chlore, de brome ou d'iode,
un groupe alkyle en C₁₋₂ ou hydroxy,
un groupe cycloalkyle en C₃₋₆ ou cycloalcoxy en C₅₋₆,
un groupe alcényle en C₂₋₅,
un groupe alcynyle en C₂₋₅,
un groupe aryle, aryloxy, aralkyle, aralcoxy, alkylsulfényle, alkylsulfinyle, alkylsulfonyle, alkylsulfonyloxy, trifluorométhylsulfényle, trifluorométhylsulfonyle, arylsulfényle, arylsulfinyle, arylsulfonyle, aralkylsulfényle, aralkylsulfinyle ou aralkylsulfonyle,
un groupe méthyle ou méthoxy substitué par 1 à 3 atomes de fluor,
un groupe alkyle en C₂₋₄ ou alcoxy en C₂₋₄ substitué par 1 à 5 atomes de fluor,
un groupe nitro, amino, alkylamino, dialkylamino, (cycloalkyle en C₃₋₆)amino, N-alkyl-N-(cycloalkyle en C₃₋₆)amino, arylamino, N-alkyl-N-arylamino, aralkylamino ou N-alkyl-aralkylamino,
un groupe alkylèneimino à 5 à 7 chaînons, où un ou deux des groupes méthylène voisins de l'atome d'azote peuvent être remplacés chacun par un groupe carbonyle ou dans les groupes alkylèneimino à 6 à 7 chaînons mentionnés, un groupe méthylène en position 4 peut être remplacé par un atome d'oxygène, par un groupe imino, N-arylimino ou N-alkylimino, et les groupes alkylèneimino peuvent être substitués en outre, par 1 à 2 groupes méthyle,
un groupe (alkylèneimino)carbonyle ou (alkylèneimino)sulfonyle à 5 à 7 atomes cycliques dans la partie alkylèneimino, où, dans les parties alkylèneimino à 6 à 7 chaînons mentionnés, chaque fois un groupe méthylène en position 4 peut être remplacé par un atome d'oxygène, par un groupe imino, N-arylimino ou N-alkylimino,
un groupe alkylcarbonylamino, N-alkyl-alkylcarbonylamino, alkyl-sulfonylamino, N-alkyl-alkylsulfonylamino, arylcarbonylamino, N-alkyl-arylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, trifluorométhylsulfonylamino, N-alkyl-trifluorométhylsulfonylamino, alkylcarbonyle, arylcarbonyle, aralkylcarbonyle, carboxy, alcoxycarbonyle, aminocarbonyle, alkylaminocarbonyle, dialkylaminocarbonyle, arylaminocarbonyle, N-alkyl-arylaminocarbonyle, aralkylaminocarbonyle, N-alkyl-aralkylaminocarbonyle, N-hydroxyaminocarbonyle, N-hydroxy-alkylaminocarbonyle, N-alcoxyaminocarbonyle, N-alcoxy-alkylaminocarbonyle, cyano, azido, N-cyanoamino ou N-cyano-alkylamino,
un groupe sulfo, aminosulfonyle, alkylaminosulfonyle, dialkylaminosulfonyle, arylaminosulfonyle, pyridylaminosulfonyle, N-alkyl-arylaminosulfonyle, aralkylaminosulfonyle ou N-alkyl-aralkylaminosulfonyle,
un groupe alkyle en C₁₋₂ substitué par R₄, où
R₄ représente un groupe hydroxy, alcoxy, aryloxy, amino, alkylamino, fluoroalkylamino, dialkylamino, alkylsulfényle, alkylsulfinyle, alkylsulfonyle, arylsulfényle, arylsulfinyle, arylsulfonyle, carboxy, alcoxycarbonyle, aminocarbonyle, alkylaminocarbonyle, dialkylaminocarbonyle ou cyano,
un groupe un groupe alkylèneimino à 5 à 7 chaînons, éventuellement substitué par 1 ou 2 groupes alkyle, où, dans les groupes alkylèneimino à 6 à 7 chaînons mentionnés, un groupe méthylène en position 4 peut être remplacé par un atome d'oxygène ou de soufre, par un groupe imino, N-alkylimino, N-alkylcarbonylimino, N-alkylsulfonylimino, N-arylcarbonylimino, N-aryl-sulfonylimino, N-arylimino ou N-aralkylimino, ou par un groupe hydroxy, alcoxy, aminocarbonyle, alkylaminocarbonyle, dialkylaminocarbonyle, amino, alkylamino et dialkylamino,
un groupe (alkylèneimino)carbonyle à 5 à 7 atomes cycliques dans la partie alkylèneimino, éventuellement substitué par 1 ou 2 groupes alkyle, où, dans les parties alkylèneimino à 6 à 7 chaînons mentionnées, le groupe méthylène en position 4 peut être remplacé par un atome d'oxygène ou de soufre, par un groupe imino, N-alkylimino ou N-alkylcarbonylimino, ou un groupe de la formule : 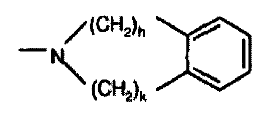 dans laquelle
h et k, qui peuvent être identiques ou différents, représentent les nombres 1 à 2 ou h le nombre 0 et k le nombre 2 ou 3, où en plus la partie benzo ci-dessus peut être substituée par un atome de fluor, de chlore, de brome ou d'iode, par un groupe alkyle, trifluorométhyle, hydroxy, alcoxy, carboxy ou cyano, et la partie imino cyclique saturée ci-dessus peut être substituée par 1 ou 2 groupes alkyle,
R₂ représente un atome de fluor, de chlore ou de brome, un groupe alkyle en C₁₋₂, trifluorométhyle, hydroxy, amino, alkylamino, dialkylamino, alkylcarbonylamino, N-alkyl-alkylcarbonylamino, alkylsulfonylamino, N-alkyl-alkylsulfonylamino, trifluorométhylsulfonylamino, N-alkyl-trifluorométhylsulfonylamino ou cyano, et
R₃ représente un atome de fluor, de chlore ou de brome, un groupe alkyle en C₁₋₂, trifluorométhyle ou alcoxy,
un groupe de structure : 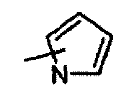
où le point de liaison peut être un atome de carbone ou d'azote et jusqu'à trois atomes de carbone peuvent être remplacés par un atome d'azote, et
le cycle peut être substitué par un ou deux restes alkyle, aryle ou aralkyle, ou
un groupe sulfo, aminosulfonyle, alkylaminosulfonyle, dialkylaminosulfonyle, arylaminosulfonyle, pyridylaminosulfonyle, N-alkyl-arylaminosulfonyle, aralkylaminosulfonyle ou N-alkyl-aralkylaminosulfonyle,
R₂ avec R₃, dans la mesure où ceux-ci sont liés à des atomes de carbone voisins l'un de l'autre, représentent un groupe méthylènedioxy éventuellement substitué par un ou deux groupes alkyle, ou un groupe n-alkylène en C₃₋₅, éventuellement substitué par un ou deux groupes alkyle, dans lequel un groupe méthylène peut être remplacé par un atome d'oxygène, par un groupe imino, N-alkylimino ou N-aralkylimino, ou
un groupe 1,3-butadiène-1,4-diyle éventuellement substitué par un atome de fluor, de chlore ou de brome, par un groupe hydroxy, alkyle, alcoxy, trifluorométhyle, carboxy ou cyano, ou
un groupe de la formule -NH-C(=O)-(CH₂)-, -NH-C(=O)-(CH₂)₂-, qui peuvent être substitués dans la partie alkylène, en outre, par 1 ou 2 groupes alkyle, ou un groupe de la formule -NH-N=N-, -NH-N=CH-, -NH-CH=N-, -O-CH=N-, -S-CH=N- ou -NH-CH=CH- et les tautomères, où chaque atome d'hydrogène peut être substitué par un groupe alkyle, aryle ou aralkyle, ou
un groupe de la formule -(CH₂)ₘ-N(R₅)-(CH₂)ₙ-
dans lequel n et m, qui peuvent être identiques ou différents, représentent 1 ou 2, et
R₅ représente un atome d'hydrogène ou un groupe alkyle en C₁₋₆ ou fluoroalkyle en C₁₋₆, et
R_{c}NR_{d} représente un groupe alkylèneimino à 4 à 7 chaînons, éventuellement substitué par 1 à 2 groupes alkyle ou aryle, qui est en outre substitué par le reste R₆, où
R₆ représente un groupe carboxy, alcoxycarbonyle, aminocarbonyle, alkylaminocarbonyle, dialkylaminocarbonyle, cyano, hydroxy, alcoxy, aryloxy, aralcoxy, alkylcarbonyloxy, arylcarbonyloxy, amino, alkylamino, hydroxy(alkyle en C₂₋₄)amino, dialkylamino, cyanamino, formylamino,
alkylsulfényle, alkylsulfinyle, alkylsulfonyle, arylsulfényle, arylsulfinyle, arylsulfonyle, aralkylsulfényle, aralkylsulfinyle ou aralkylsulfonyle,
un groupe alkylcarbonylamino, N-alkyl-alkylcarbonylamino, alkylsulfonylamino, N-alkyl-alkylsulfonylamino, arylcarbonylamino, N-alkyl-arylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, alcoxycarbonylamino, N-alkyl-alcoxycarbonylamino, alcoxycarbonylalkylamino, N-alkyl-N-(alcoxycarbonylalkyl)amino, aralcoxycarbonylamino, N-alkyl-aralcoxycarbonylamino,
un groupe (NR₈R₉)CONR₇-, dans lequel R₇ et R₈ représentent chacun un atome d'hydrogène ou un groupe alkyle, et R₉ représente un atome d'hydrogène ou un groupe alkyle, aryle ou pyridyle, ou les restes R₇, R₈ et R₉ peuvent être identiques ou différents, ou
R₇ et R₈ représentent ensemble un groupe n-alkylène en C₂₋₄ et R₉ représente un atome d'hydrogène ou un groupe alkyle, aryle ou pyridyle,
un groupe alkylèneimino à 5 à 7 atomes cycliques dans la partie alkylèneimino, éventuellement substitué par 1 à 2 groupes alkyle ou un groupe hydroxyalkyle, où, dans les parties alkylèneimino à 6 à 7 chaînons mentionnées, le groupe méthylène en position 4 dans la partie alkylèneimino peut être remplacé par un atome d'oxygène ou de soufre, par un groupe carbonyle, sulfinyle, sulfonyle, imino, N-alkylimino, N-alkylcarbonylimino, N-alkylsulfonylimino, N-arylimino ou N-aralkylimino,
un groupe un groupe (alkylèneimino)carbonyle à 4 à 7 atomes cycliques dans la partie alkylèneimino, éventuellement substitué par 1 à 2 groupes alkyle, où, dans les parties alkylèneimino à 6 à 7 chaînons mentionnées, le groupe méthylène en position 4 peut être remplacé par un atome d'oxygène ou de soufre, par un groupe sulfinyle, sulfonyle, imino, N-alkylimino, N-alkylcarbonylimino, N-alkylsulfonylimino, N-arylimino, ou N-aralkylimino,
un groupe alkylèneimino à 4 à 7 chaînons, substitué par un groupe hydroxy, alcoxy, amino, alkylamino, dialkylamino, alkylcarbonylamino, N-alkyl-alkylcarbonylamino, alkylsulfonylamino, N-alkyl-alkylsulfonylamino, hydroxyalkyle, carboxy, alcoxycarbonyle, aminocarbonyle, alkylaminocarbonyle ou dialkylaminocarbonyle,
un groupe alkyle substitué par un groupe carboxy, alcoxycarbonyle, aminocarbonyle, alkylaminocarbonyle, dialkylaminocarbonyle, cyano, hydroxy, alcoxy aryloxy, amino, alkylamino, dialkylamino, alkylcarbonylamino, N-alkyl-alkylcarbonylamino, alcoxycarbonylamino, N-alkyl-alcoxycarbonylamino, alkylsulfonylamino, N-alkyl-alkylsulfonylamino, arylcarbonylamino, N-alkyl-arylcarbonylamino, arylsulfonylamino, N-alkyl-arylsulfonylamino, dialkylaminocarbonylalkylamino, N-(alkyl)-N-(dialkylaminocarbonylalkyl)amino, dialkylaminoalcoxy, alkylsulfényle, alkylsulfinyle, alkylsulfonyle, arylsulfényle, arylsulfinyle ou arylsulfonyle,
un groupe (alkylèneimino)alkyle à 4 à 7 atomes cycliques dans la partie alkylèneimino, éventuellement substitué par 1 à 2 groupes alkyle, où, dans les parties alkylèneimino à 6 à 7 chaînons mentionnées, chaque fois un groupe méthylène en position 4 peut être remplacé par un atome d'oxygène ou de soufre, par un groupe sulfinyle, sulfonyle, imino, N-alkylimino ou N-alkylcarbonylimino,
un groupe (alkylèneimino)carbonylalkyle à 4 à 7 atomes cycliques dans la partie alkylèneimino, éventuellement substitué par 1 à 2 groupes alkyle, où, dans les parties alkylèneimino à 6 à 7 chaînons mentionnées, chaque fois un groupe méthylène en position 4 peut être remplacé par un atome d'oxygène ou de soufre, par un groupe sulfinyle, sulfonyle, imino ou N-alkylimino,
un groupe (carboxyalkyl)oxy, (alcoxycarbonyalkyl)oxy, (aminocarbonyalkyl)oxy, (alkylaminocarbonyalkyl)oxy ou (dialkylaminocarbonyalkyl)oxy,
un groupe 3,4-dihydro-1*H*-quinazolin-2-on-3-yle ou 1*H*-benzimidazol-2-on-1-yle, éventuellement substitué dans la partie aryle par un ou deux atomes de fluor, de chlore, de brome ou d'iode ou un ou deux groupes nitro, alkyle, alcoxy ou cyano, où les substituants peuvent être chaque fois, identiques ou différents, ou
R_{c}NR_{d} représente un groupe alkylèneimino à 6 à 7 chaînons, éventuellement substitué par 1 à 2 groupes alkyle ou par un groupe aryle, qui peut être en outre substitué par le reste R₆, où, dans les parties alkylèneimino mentionnées, chaque fois un groupe méthylène en position 4 peut être remplacé par un atome d'oxygène ou de soufre, par un groupe carbonyle, sulfinyle, sulfonyle ou R₁₀N, où
R₁₀ représente un atome d'hydrogène, un groupe alkyle, hydroxyalkyle en C₂₋₄, aminoalkyle en C₂₋₄, (alkylamino)alkyle en C₂₋₄, dialkylamino-alkyle en C₂₋₄, (hydroxyalcoxy en C₂₋₄)alkyle en C₂₋₄, aminocarbonylalkyle, alkylaminocarbonylalkyle, dialkylaminocarbonylalkyle, aryle, formyle, alkylcarbonyle, alkylsulfonyle, arylcarbonyle, arylsulfonyle, aralkylcarbonyle, aralkylsulfonyle, alcoxycarbonyle, aralcoxycarbonyle, cyano, aminocarbonyle, alkylaminocarbonyle ou dialkylaminocarbonyle,
un groupe aminoalkylcarbonyle, alkylaminoalkylcarbonyle, (dialkylamino)alkylcarbonyle,
un groupe méthyle substitué par un ou deux groupes aryle, où la partie aryle peut être substitué indépendamment, chaque fois par un ou deux atomes de fluor, de chlore, de brome ou d'iode, ou un ou deux groupes nitro, aryle, hydroxy ou alcoxy, où les substituants peuvent être identiques ou différents,
un groupe 2-, 3- ou 4-pyridyle,
un groupe 2-, 4- ou 5-pyrimidinyle,
un groupe phényle éventuellement substitué par un ou deux atomes de fluor, de chlore, de brome ou d'iode, ou un ou deux groupes nitro, trifluorométhyle, alkyle, hydroxy, alcoxy, amino, alkylamino, dialkylamino, carboxy, alcoxycarbonyle, aminocarbonyle, alkylaminocarbonyle, dialkylaminocarbonyle ou cyano, où les substituants peuvent être identiques ou différents,
un groupe 8-méthyl-8-azabicyclo[3.2.1]oct-3-yle, ou
un groupe (alkylèneimino)carbonyle ou (alkylèneimino)carbonylalkyle à 5 à 7 atomes cycliques dans la partie alkylèneimino, où, dans les parties alkylèneimino à 6 à 7 chaînons mentionnées, le groupe méthylène en position 4 peut être remplacé par un atome d'oxygène ou de soufre, par un groupe sulfinyle, sulfonyle, imino, N-alkylimino, N-alkylcarbonylimino ou N-aralkylimino, ou
R_{c}NR_{d} représente un groupe 3-thiazolidinyle substitué en position 4 par un groupe carbonyle, alcoxycarbonyle, aminocarbonyle, alkylaminocarbonyle, dialkylaminocarbonyle, ou
R_{c}NR_{d} représente un groupe 1-pipéridinyle éventuellement substitué par 1 à 2 groupes alkyle, dans lequel deux atomes d'hydrogène sur le squelette carboné sont remplacés par un pont alkylène linéaire, où ce pont contient 2 à 6 atomes de carbone lorsque les deux atomes d'hydrogène se trouvent sur le même atome de carbone, ou contient 1 à 5 atomes de carbone lorsque les deux atomes d'hydrogène se trouvent sur des atomes de carbone voisins, ou contient 1 à 4 atomes de carbone lorsque les deux atomes d'hydrogène se trouvent sur des atomes de carbone séparés par un atome, ou contient 1 à 3 atomes de carbone lorsque les deux atomes d'hydrogène se trouvent sur des atomes de carbone séparés par deux atomes, où les groupes 1-pipéridinyle mentionnés sont substitués en outre, par le reste R₆, qui est défini comme mentionné ci-dessus,
un groupe 1-pyrrolidinyle ou 1-pipéridinyle, éventuellement substitué par 1 à 2 groupes alkyle, dans lequel deux atomes d'hydrogène d'un groupe méthylène sont remplacés par un pont alkylène linéaire en C₃₋₆, où un groupe méthylène dans le pont alkylène en C₃₋₆ est remplacé par un groupe R₁₀N, où R₁₀ est défini comme mentionné ci-dessus, où le bicycle ainsi formé peut être substitué en outre, par un groupe hydroxy, alcoxy, amino, alkylamino, dialkylamino, cyano, alkylcarbonylamino, alkylsulfonylamino, alcoxycarbonylamino, arylcarbonyle, carboxy, alcoxycarbonyle, aminocarbonyle, alkylaminocarbonyle ou dialkylaminocarbonyle,
un groupe éventuellement substitué dans la partie alkylène, par 1 ou 2 groupes alkyle, de structure : 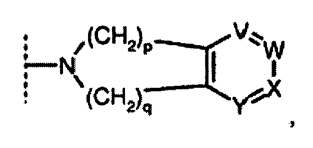 où
p et q, qui peuvent être identiques ou différents, représentent le nombre 1 ou 2, et
l'unité V=W-X=Y représente un des groupes (a) ou (b) :
-N=C-C=C- (a)
-C=N-C=C- (b)
où un ou deux des atomes de carbone disponibles des groupes (a) ou (b) peuvent être substitués par un groupe hydroxy, alcoxy, amino, alkylamino ou dialkylamino, et les atomes de carbone disponibles restants des groupes (a) ou (b) sont substitués par un atome d'hydrogène, un groupe alkyle ou aryle, ou -V=W- représentent ensemble, un atome d'oxygène ou de soufre ou un groupe imino, N-alkylimino, N-aralkylimino ou N-arylimino et -X=Y- représentent un des groupes -N=C-, -C=N- ou -C=C-,
ou dans la mesure où n et m ne sont pas égaux,
-X=Y- représentent ensemble, un atome d'oxygène ou de soufre ou un groupe imino, N-alkylimino ou N-arylimino et -V=W- représentent un des groupes-N=C- ou -C=N-, ou
R_{c} représente un atome d'hydrogène ou un groupe aralkyle ou alkyle en C₁₋₈, un groupe alkyle, qui est substitué par
un groupe hydroxy, alcoxy, aryloxy, aralcoxy, amino, alkylamino, dialkylamino, alkylcarbonylamino, N-alkyl-alkylcarbonylamino, alkylsulfonylamino, N-alkyl-alkylsulfonylamino, trifluorométhylsulfonylamino, N-alkyl-trifluorométhylsulfonylamino, carboxy, alcoxycarbonyle, aminocarbonyle, alkylaminocarbonyle, dialkylaminocarbonyle, cyano, ou par un groupe 2-, 3- ou 4-pyridyle, avec la condition que l'hétéroatome est séparé de l'atome d'azote du groupe R_{c}NR_{d} par deux atomes de carbone ou plus,
un groupe alcényle en C₃₋₅, où la partie vinyle ne peut pas être reliée par l'atome d'azote au groupe R_{c}NR_{d},
un groupe alcynyle en C₃₋₅, où la partie éthynyle ne peut pas être reliée par l'atome d'azote au groupe R_{c}NR_{d}, et
R_{d} représente un groupe alkyle en C₁₋₁₀, qui est substitué par un groupe choisi parmi les groupes (a) à (n) :
(a) un groupe carboxy, alcoxycarbonyle, aminocarbonyle, alkylaminocarbonyle, dialkylaminocarbonyle, cyano, hydroxy, alcoxy, aryloxy, aralcoxy, alkylcarbonylamino, amino, alkylamino, dialkylamino, naphtylamino, aralkylamino, diaralkylamino ou N-alkyl-aralkylamino,
(b) un groupe phénylamino ou pyridylamino, éventuellement substitué, dans la partie aryle, par un atome de fluor, de chlore, de brome ou d'iode, ou par un groupe nitro, trifluorométhyle, alkyle, hydroxy, alcoxy, amino, alkylamino, dialkylamino, carboxy, alcoxycarbonyle, aminocarbonyle, alkylaminocarbonyle, dialkylaminocarbonyle ou cyano,
(c) un groupe méthoxy substitué par un, deux ou trois restes aryle,
(d) un groupe carboxyalkylaminocarbonyle, alcoxycarbonylalkylaminocarbonyle, aminocarbonylalkylaminocarbonyle, alkylaminocarbonylalkylaminocarbonyle, dialkylaminocarbonylalkylaminocarbonyle, arylaminocarbonyle, N-alkyl-arylaminocarbonyle, aralkylaminocarbonyle, N-alkyl-aralkylaminocarbonyle,
(e) un groupe de formule -C(=NH)NH₂,
(f) un groupe (alkylèneimino)carbonyle à 5 à 7 atomes cycliques dans la partie alkylèneimino, éventuellement substitué par 1 à 2 groupes alkyle, où, dans les parties alkylèneimino à 6 ou 7 chaînons mentionnés, un groupe méthylène en position 4 peut être remplacé par un atome d'oxygène ou de soufre, par un groupe sulfinyle, sulfonyle, imino, N-alkylimino, N-alkylcarbonylimino, N-alkylsulfonylimino, N-arylimino ou N-aralkylimino,
(g) un groupe alkylèneimino à 4 à 7 chaînons, éventuellement substitué par 1 à 2 groupes alkyle, où, dans les parties alkylèneimino de 6 ou 7 chaînons mentionnées, chaque groupe méthylène en position 4 peut être remplacé par un atome d'oxygène ou de soufre, par un groupe sulfinyle, sulfonyle ou R₁₀N, où R₁₀ est défini comme précédemment, et en outre, dans les parties alkylèneimino de 5 à 7 chaînons mentionnées, chaque fois un des groupes méthylène voisins des atomes d'azote peut être remplacé par un groupe carbonyle,
(h) un groupe alkylèneimino à 5 à 7 chaînons, éventuellement substitué par 1 à 2 groupes alkyle, qui est substitué par un groupe hydroxyalkyle, aminoalkyle, alkylaminoalkyle ou dialkylaminoalkyle,
(i) un groupe alkylsulfonylamino, N-alkyl-alkylsulfonylamino, arylcarbonylamino, N-alkyl-arylcarbonylamino, alkylcarbonylamino, N-alkyl-alkylcarbonylamino, alcoxyalkylcarbonylamino, dialkylamino-alkylcarbonylamino, arylsulfonylamino, N-alkyl-arylsulfonylamino, aralkylcarbonylamino, N-alkyl-aralkylcarbonylamino, aralkylsulfonylamino, N-alkyl-aralkylsulfonylamino, alcoxycarbonylamino, N-alkyl-alcoxycarbonylamino, aralcoxycarbonylamino ou N-alkyl-aralcoxycarbonylamino,
(j) un groupe (R₉NR₈)-CO-NR₇-, où R₇, R₈ et R₉ sont définis comme précédemment,
(k) un groupe 2-azabicyclo[2.2.1]hept-5-én-2-yle,
(l) un groupe alkylsulfényle, alkylsulfinyle, alkylsulfonyle, arylsulfényle, arylsulfinyle ou arylsulfonyle,
(m) un groupe cycloalkyle en C₄₋₇ substitué par R₆ et éventuellement, en outre par 1 à 2 groupes alkyle, où R₆ est défini comme précédemment,
(n) un groupe cycloalkyle en C₅₋₇ substitué éventuellement par 1 à 4 groupes alkyle, dans lequel un groupe méthylène est remplacé par un atome d'oxygène ou un groupe NR₁₀, où R₁₀ est défini comme précédemment,
un groupe 4-pipéridinylméthyle substitué, en position 1, par R₁₀ et, en outre, en position 4, par un groupe hydroxy, où R₁₀ est défini comme précédemment, et dans lequel, en outre, un atome d'hydrogène en position 2 et 6 du squelette pipéridinyle est remplacé par un pont alkylène en C₂₋₃,
un groupe méthyle substitué par un groupe 3-hydroxy-1,3-dihydroindol-2-on-3-yle ou 2-aminocarbonyl-1,3-dihydroisoindol-5-yle,
un groupe de structure 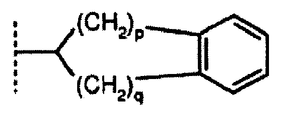 substitué dans la partie aryle, par un groupe carboxy ou carboxyalkyle, et éventuellement, en outre dans la partie alkyle, par 1 ou 2 groupes alkyle,
où p et q, qui peuvent être identiques ou différents, représentent le nombre 0, 1 ou 2, où cependant p et q doivent faire ensemble au moins le nombre 2,
un groupe alkyle en C₃₋₆, substitué par un groupe hydroxy et en outre, par un groupe amino, alkylamino, dialkylamino, hydroxy, alcoxy, 1-pyrrolidinyle, 1-pipéridinyle ou morpholinyle,
un groupe alkyle en C₂₋₆, substitué par un groupe carboxy et en outre, par un groupe amino, hydroxy, aminocarbonyle ou benzyloxycarbonylamino,
un groupe alkyle en C₂₋₄, qui est substitué par un groupe (alkyle en C₂₋₄)sulfényle, qui est substitué en position ω par un groupe amino,
un groupe alkyle en C₂₋₄, qui est substitué par un groupe alcoxy en C₂₋₄, qui est substitué en position ω par un groupe amino, hydroxy ou alcoxy,
un groupe alkyle en C₂₋₄, qui est substitué par un groupe (alcoxy en C₂₋₄)alcoxy en C₂₋₄, qui est substitué en position ω par un groupe amino ou hydroxy,
un groupe cycloalkyle en C₄₋₇, éventuellement substitué par 1 à 2 groupes alkyle, qui est substitué en outre, par R₆, qui est défini comme précédemment,
un groupe cycloalkyle en C₄₋₇, éventuellement substitué par 1 à 2 groupes alkyle, qui peut être substitué en outre, par R₆, où, dans la partie cycloalkyle, un groupe méthylène est remplacé par un atome d'oxygène ou de soufre, par un groupe sulfinyle, sulfonyle ou R₁₀N, où R₆ et R₁₀ sont définis comme précédemment,
un groupe méthyle substitué par un groupe 3-hydroxy-1,3-dihydroindol-2-on-3-yle ou 2-aminocarbonyl-1,3-dihydroisoindol-5-yle,
un groupe alkyle en C₁₋₆ substitué par un groupe aryle, où la partie aryle mentionnée est substituée par un groupe alcoxycarbonyle, carboxy, carboxyalkyle, aminosulfonyle, trifluorométhoxy, cyano, aminoalkyle, amino, alkylamino, dialkylamino, nitro, 2H-pyridazin-3-on-6-yle, hydroxyphényle, hydroxyalkyle, hydroxy ou alcoxy,
un groupe aralkyle, qui est substitué dans la partie alkyle par un groupe hydroxy ou alcoxy et en outre, par un groupe alcoxycarbonyle, carboxy, alcoxy ou hydroxy,
un groupe alkyle en C₁₋₆ substitué par un groupe 2-pyridyle, 3-pyridyle, 4-pyridyle, 2-pyrazinyle, 1*H*-pyrrol-2-yle, 1*H*-pyrazol-4-yle, 1*H*-pyrazol-5-yle, 1*H*-imidazol-1-yle, 1*H*-imidazol-4-yle, 1*H*-indol-3-yle, 1*H*-benzimidazol-2-yle, où les parties hétéroaryle mentionnées peuvent être substitués sur les atomes de carbone disponibles, en outre par un ou deux groupes choisis parmi des atomes de fluor, de chlore, de brome ou d'iode, des groupes alkyle, alcoxycarbonyle, carboxy, trifluorométhyle, trifluorométhoxy, cyano, amino, alkylamino, dialkylamino, nitro, hydroxy ou alcoxy, où les substituants peuvent être identiques ou différents,
un groupe alkyle en C₁₋₆ substitué par un groupe carboxy, alcoxycarbonyle, aminocarbonyle, alkylaminocarbonyle, dialkylaminocarbonyle, aralkylaminocarbonyle, cyano, hydroxy, alcoxy, aryloxy, amino, alkylamino, dialkylamino, alkylcarbonylamino ou alcoxycarbonylamino, qui est substitué en outre, par un ou deux groupes aryle ou un groupe hétéroaryle, où le groupe hétéroaryle représente un groupe 2-pyridyle, 3-pyridyle, 4-pyridyle, 2-pyrazinyle, 1*H*-pyrrol-2-yle, 1*H-*pyrazol-4-yle, 1*H*-pyrazol-5-yle, 1*H*-imidazol-1-yle, 1*H*-imidazol-4-yle, 1*H*-indol-3-yle, 1*H*-benzimidazol-2-yle, où les parties aryle ou hétéroaryle mentionnées peuvent être substitués sur les atomes de carbone disponibles, en outre par un ou deux groupes choisis parmi des atomes de fluor, de chlore, de brome ou d'iode, des groupes alkyle, alcoxycarbonyle, carboxy, trifluorométhyle, trifluorométhoxy, cyano, amino, alkylamino, dialkylamino, nitro, hydroxy ou alcoxy, où les substituants peuvent être identiques ou différents,
un groupe alkyle en C₁₋₆ substitué par un groupe aryle, qui est substitué dans la partie aryle, par un groupe hydroxy ou amino et en outre, par deux atomes de fluor, de chlore, de brome ou d'iode, où les substituants peuvent être identiques ou différents,
un groupe alkyle en C₂₋₆ substitué par un groupe carboxy ou alcoxycarbonyle, qui est substitué en outre, par un groupe amino, alkylamino, dialkylamino, alkylcarbonylamino, arylcarbonylamino, arylsulfonylamino, alcoxycarbonylamino ou aralcoxycarbonylamino,
un groupe 3-quinuclidinyle ou 4-quinuclidinyle, et
où sauf indication contraire, les parties alkyle, alkylène et alcoxy mentionnées précédemment contiennent chacune, 1 à 4 atomes de carbone,
éventuellement sous la forme de leurs tautomères, leurs racémates, leurs énantiomères, leurs diastéréoisomères et leurs mélanges, ainsi que éventuellement leurs sels d'addition d'acide pharmacologiquement acceptables,
où, sauf indication contraire, chaque atome de carbone dans les parties alkyle, alkylène ou cycloalkylène mentionnées précédemment, qui est lié à un atome d'azote, d'oxygène ou de soufre, ne peut pas être lié à un autre atome d'halogène, d'azote, d'oxygène ou de soufre.

3. Composés de formule I selon la revendication 1 ou 2, dans lesquels
R_{b} représente un groupe naphtyle éventuellement substitué par un atome de fluor, de chlore ou de brome ou un groupe carboxy, alkyle en C₁₋₂, alcoxy en C₁₋₂, cyano ou trifluorométhyle,
un groupe 5- ou 6-indazolyle substitué éventuellement par un groupe méthyle sur l'atome d'azote, ou un groupe 1,3-dihydro-2-oxoindol-6-yle, ou
un groupe phényle évenuellement substitué par les restes R₁ à R₃, où
R₁ représente un atome de fluor, de chlore, de brome ou d'iode, un groupe alkyle en C₁₋₂, trifluorométhyle, aminocarbonyle, carboxy, alcoxycarbonyle, cyano, phénylaminocarbonyle, benzylaminocarbonyle, aminosulfonyle, méthylaminosulfonyle, diméthylaminosulfonyle, morpholinosulfonyle, N-méthylpipérazinosulfonyle, homopipérazinosulfonyle, 2,6-diméthylpipérazin-4-yle, 2-aminopyridyl-N-sulfonyle, morpholino, 4-méthyl-1-pipérazinyle, (N-méthyl-N-méthylsulfonyl)amino, 2-carboxy-1-éthyle, diméthylamino-1-éthyle ou nitro,
un groupe méthyle, qui est substitué par un groupe 1,2,4,5-tétra-hydrobenzo[d]azépin-3-yle, dialkylamino, pyrrolidino, pipéridino, 2,6-diméthyl-pipéridino-1-yle, 4-méthoxypipéridino-1-yle, morpholino, S-dioxothiomorpholino, pipérazino ou 4-méthyl-1-pipérazinyle ou un groupe fluoroalkylamino de formule :
-(CH₂)ᵣ-(CH₂)ₛ-Q,
où
r représente 0 ou en nombre entier allant de 1 à 3,
s représente un nombre entier allant de 1 à 3, et
Q représente l'atome d'hydrogène, de fluor ou de chlore,
R₂ représente un atome de fluor ou de chlore, un groupe hydroxy, amino ou méthyle, et
R₃ représente un atome de chlore, ou un groupe tétrazolyle, triazolyle, imidazolyle ou pyrazolyle,
le point de liaison étant un atome de carbone ou un atome d'azote et un atome d'hydrogène sur le cycle pouvant être remplacé par un reste alkyle,
un groupe aminosulfonyle, méthylaminosulfonyle, diméthylaminosulfonyle, morpholinosulfonyle, N-méthylpipérazinosulfonyle, homopipérazinosulfonyle, 2-aminopyridyl-N-sulfonyle, ou
R₂ avec R₃ forment ensemble, un groupe de formule
-(CH₂)ₘ-N(R₅)-(CH₂)ₙ-
où n et m représentent indépendamment l'un de l'autre, 1 ou 2, et
R₅ représente un groupe fluoroalkyle de formule :
-(CH₂)_{r'}-(CH₂)_{s'}-Q',
où
r' représente 0 ou en nombre entier allant de 1 à 3,
s' représente un nombre entier allant de 1 à 3, et
Q' représente l'atome d'hydrogène, de fluor ou de chlore,
le groupe R_{c}NR_{d} représente
un groupe alkylèneimino à 5 à 7 chaînons, substitué par le reste R₆, où
R₆ représente un groupe hydroxy, alcoxy, aryloxy, amino, alkylamino, dialkylamino, alkylcarbonylamino, N-alkyl-alkylcarbonylamino, alcoxycarbonylamino, N-alkyl-alcoxycarbonylamino, alkylsulfényle, alkylsulfinyle, alkylsulfonyle, arylsulfényle, arylsulfinyle, arylsulfonyle, carboxy, alcoxycarbonyle, aminocarbonyle, alkylaminocarbonyle, dialkylaminocarbonyle ou cyano,
un groupe alkyle, qui est substitué par un groupe hydroxy, amino, alkylamino, dialkylamino, alkylcarbonylamino, N-alkyl-alkylcarbonylamino, dialkylaminocarbonylamino, N-(alkyl)-N-(dialkylaminocarbonylalkyl)amino, alcoxycarbonyle, carboxy, dialkylaminoalcoxy, ou par un groupe alkylèneimino à 5 à 7 chaînons, où, dans les groupes alkylèneimino à 6 à 7 chaînons mentionnés, un groupe méthylène en position 4 peut être remplacé par un atome d'oxygène ou de soufre, par un groupe imino, N-alkylimino ou N-alkylcarbonylimino,
un groupe alkylèneimino à 5 à 7 atomes cycliques dans la partie alkylèneimino, où, dans les parties alkylèneimino à 6 à 7 chaînons mentionnées, chaque fois un groupe méthylène en position 4 peut être remplacé par un atome d'oxygène ou de soufre, par un groupe N-alkylimino, N-alkylcarbonylimino ou N-aralkylimino,
un groupe alkylèneimino à 5 à 7 atomes cycliques dans la partie alkylèneimino, substitué par un groupe hydroxy, amino, alkylamino, dialkylamino, carboxy, alcoxycarbonyle, aminocarbonyle, alkylaminocarbonyle ou dialkylaminocarbonyle,
un groupe 3,4-dihydro-1*H*-quinazolin-2-on-3-yle ou 1*H-*benzimidazol-2-on-1-yle, éventuellement substitué dans la partie aryle par un atome de fluor, de chlore ou de brome ou un groupe nitro, alkyle, alcoxy ou cyano,
un groupe alkylèneimino à 6 à 7 chaînons, éventuellement substitué par 1 à 2 groupes alkyle, où un groupe méthylène en position 4 peut être remplacé par un atome d'oxygène ou de soufre, par un groupe sulfinyle, sulfonyle ou R₁₀N, où
R₁₀ représente un atome d'hydrogène, un groupe alkyle, aralkyle, aminoalkyle en C₂₋₄, hydroxyalkyle en C₂₋₄, alkylcarbonyle, aralcoxycarbonyle, alkylsulfonyle, arylcarbonyle, arylsulfonyle,
un groupe (alkylèneimino)carbonyle à 5 à 7 atomes cycliques dans la partie alkylèneimino, où, dans les parties alkylèneimino à 6 à 7 chaînons mentionnées, chaque groupe méthylène en position 4 peut être remplacé par un atome d'oxygène ou de soufre, par un groupe N-alkylimino, N-alkylcarbonylimino ou N-aralkylimino,
un groupe 2-, 3- ou 4-pyridyle,
un groupe 2-, 4- ou 5-pyrimidinyle,
un groupe phényle éventuellement substitué par un ou deux atomes de fluor, de chlore, de brome ou d'iode, ou un ou deux groupes nitro, alkyle, hydroxy, alcoxy, amino, alkylamino, dialkylamino, carboxy, alcoxycarbonyle, aminocarbonyle, alkylaminocarbonyle, dialkylaminocarbonyle ou cyano, où les substituants peuvent être identiques ou différents,
un groupe 8-méthyl-8-azabicyclo[3.2.1]oct-3-yle,
un groupe benzhydryle, dans lequel indépendamment l'une de l'autre, chaque partie phényle peut être substituée par un atome de fluor, de chlore, de brome ou d'iode, ou un groupe nitro, alkyle, hydroxy, alcoxy, , où les substituants peuvent être identiques ou différents,
un groupe alkylèneimino à 6 ou 7 chaînons, substitué par un groupe phényle, qui est substitué en outre par un groupe hydroxy, carboxy, alcoxycarbonyle ou cyano, ou dans lequel un groupe méthylène en position 4 peut être remplacé par un groupe carbonyle,
un groupe 3-thiazolidinyle substitué en position 4 par un groupe carboxy, alcoxycarbonyle, aminocarbonyle, alkylaminocarbonyle, dialkylaminocarbonyle, un groupe de la structure : 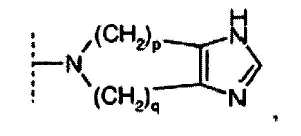 où p et q, qui peuvent être identiques ou différents, représentent indépendamment l'un de l'autre, le nombre 1 ou 2, où le cycle imidazo peut être substitué par un ou deux groupes alkyle ou aryle, où les substituants peuvent être identiques ou différents, un groupe 1-pyrrolidinyle ou 1-pipéridinyle, dans lequel les deux atomes d'hydrogène d'un groupe méthylène sont remplacés par un pont alkylène linéaire en C₃₋₅, où un groupe méthylène dans le pont alkylène en C₃₋₅ est remplacé par un groupe imino, N-alkylimino ou N-(aralkyl)imino, où le bicycle ainsi formé peut être substitué en outre, par un groupe hydroxy,
un groupe 1-pipéridinyle, qui est substitué en position 4 par un groupe hydroxy, alcoxy ou aralcoxy et dans lequel un des atomes d'hydrogène en position 2 et 6 du squelette pipéridinyle sont remplacés par un pont éthylène,
ou
R_{c} représente un atome d'hydrogène ou un groupe alkyle en C₁₋₆,
un groupe alkyle, substitué par un groupe phényle ou par un groupe 2-, 3- ou 4-pyridyle,
un groupe alkyle en C₂₋₄ substitué par un groupe hydroxy ou alcoxy, et
R_{d} représente un groupe alkyle en C₁₋₆, qui est substitué par un groupe choisi parmi les groupes (a) à (j) :
(a) un groupe de formule -C(=NH)NH₂,
(b) un groupe carboxy, alcoxycarbonyle, carboxyméthylaminocarbonyle, aminocarbonyle, alkylaminocarbonyle, alkylcarbonylamino, dialkylaminocarbonyle, arylaminocarbonyle, N-alkyl-arylaminocarbonyle, aralkylaminocarbonyle, N-alkyl-aralkylaminocarbonyle ou cyano,
(c) un groupe hydroxy, amino, alcoxy, alkylamino, dialkylamino, alkylcarbonylamino, N-alkyl-alkylcarbonylamino, alcoxycarbonylamino, alcoxyacétylamino, dialkylaminoacétylamino, N-alkyl-alcoxycarbonylamino, alkylsulfonylamino, N-alkyl-allkylsulfonylamino, arylamino, naphtylamino, aralkylamino, diaralkylamino, N-alkyl-aralkylamino, alkylsulfényle,
(d) un groupe nitro-2-pyridylamino,
(e) un groupe méthoxy substitué par un, deux ou trois restes aryle,
(f) un groupe alkylèneimino à 4 à 7 chaînons, où, dans les parties alkylèneimino de 6 à 7 chaînons mentionnées, chaque groupe méthylène en position 4 peut être remplacé par un atome d'oxygène ou de soufre, par un groupe imino, N-alkylimino, N-(hydroxyalkyle en C₂₋₄)imino ou N-(aminoalkyle en C₂₋₄)-imino, et en outre, dans les parties alkylèneimino de 5 à 7 chaînons mentionnées, chaque fois un des groupes méthylène voisins des atomes d'azote peut être remplacé par un groupe carbonyle,
(g) un groupe 1-pipéridinyle substitué par un groupe dialkylaminoalkyle,
(h) un groupe 2-azabicyclo[2.2.1]hept-5-én-2-yle,
(i) un groupe (alkylèneimino)carbonyle à 5 à 7 chaînons, où, dans les parties alkylèneimino à 6 ou 7 chaînons mentionnés, un groupe méthylène en position 4 peut être remplacé par un atome d'oxygène ou de soufre, par un groupe imino ou N-alkylimino, et
(j) un groupe (R₈R₉N)CONR₇, où
R₇, R₈ et R₉, qui peuvent être identiques ou différents, représentent un atome d'hydrogène ou un groupe méthyle, ou
R₇ et R₈ représentent ensemble un groupe n-alkylène en C₂₋₃ et R₉ représente un atome d'hydrogène ou un groupe méthyle ou 4-pyridyle, ou
R₇ et R₈ représentent un atome d'hydrogène et R₉ représente un groupe aryl(alkyle en C₁₋₂) ou phényle,
un groupe cyclohexyle substitué en position 2, 3 ou 4 par un groupe hydroxy, amino, alkylamino, dialkylamino, aminométhyle, hydroxyméthyle, alcoxycarbonyle, aminocarbonyle, alkylaminocarbonyle, dialkylaminocarbonyle ou carboxy,
un groupe cyclohexyle substitué en position 4 par un groupe carboxyalkyle,
un groupe éthyle substitué en position 2 par un groupe 2-amino-1-éthylthio, 2-hydroxy-1-éthoxy, 2-(2-amino-1-éthoxy)-1-éthoxy ou 2-(2-hydroxy-1-éthoxy)-1-éthoxy,
un groupe propyle substitué en position 3 par un groupe 3-amino-1-propoxy ou 2-(3-amino-1-propoxy)-1-éthoxy,
un groupe alkyle en C₁₋₂ substitué par un groupe cycloalkyle en C₅₋₆, où la partie cycloalkyle est substituée par un groupe hydroxy, aminométhyle, diméthylaminométhyle, 2-carboxyéthyle ou t-butyloxycarbonylaminométhyle et où, dans la partie cycloalkyle, un groupe méthylène est remplacé par un atome d'oxygène, un groupe N-alkylimino ou N-(2-dialkylaminoacétyl)imino,
un groupe 4-pipéridinylméthyle, qui est substitué en position 1 par un groupe alkyle ou aralkyle et en outre, en position 4, par un groupe hydroxy, et pour lequel, en outre, un atome d'hydrogène en position 2 et 6 du squelette pipéridinyle est remplacé par un pont éthylène,
un groupe 3-pyrrolidinyle ou un groupe 3- ou 4-pipéridinyle, qui est substitué en position 1 par un groupe alkyle, aralkyle ou arylsulfonyle,
un groupe 4-pipéridinyle, qui est substitué en position 1 par un groupe alkyle, aralkyle ou arylsulfonyle, et en outre en position 4 par un groupe carboxy,
un groupe aralkyle, qui est substitué dans la partie aryle par un groupe hydroxy, aminosulfonyle, carboxy, nitro, amino, aminométhyle, 2-amino-1-éthyle, alcoxycarbonyle, 4-hydroxyphényle ou 2*H*-pyridazin-3-on-6-yle,
un groupe méthyle substitué par un groupe 3-hydroxy-1,3-dihydroindol-2-on-3-yle ou 2-aminocarbonyl-1,3-dihydroisoindol-5-yle,
un groupe 2-indanyle substitué dans la partie aryle, par un groupe 3-carboxy-1-propyle,
un groupe alkyle substitué par un groupe 1*H*-2-benzimidazole ou 4-amino-3,5-dichlorophényle,
un groupe aralkyle, qui est substitué dans la partie aryle par un groupe hydroxy, amino, alkylamino, dialkylamino, aminocarbonyle, alkylaminocarbonyle, dialkylaminocarbonyle, aralkylaminocarbonyle, carboxy ou cyano, et est substitué en outre, éventuellement, dans la partie aryle, par un ou deux atomes de fluor, de chlore
ou de brome, ou un ou deux groupes hydroxy ou alcoxy, où les substituants peuvent être identiques ou différents,
un groupe alkyle substitué par un groupe carboxy et en outre, par deux groupes phényle,
un groupe alkyle en C₂₋₆, substitué par un groupe carboxy et en outre, par un groupe hydroxy, aminocarbonyle, 1*H*-imidazol-4-yle ou benzyloxycarbonylamino,
un groupe alkyle, qui est substitué par un groupe alcoxycarbonyle, et en outre, par un groupe pyridyle,
un groupe alkyle en C₃₋₆, substitué par un groupe hydroxy et en outre, par un groupe amino, alkylamino, dialkylamino, hydroxy, alcoxy, 1-pyrrolidinyle, 1-pipéridinyle ou morpholino,
un groupe aralkyle, qui est substitué dans la partie aryle par un groupe alcoxy et en outre, par un groupe carboxy ou hydroxy,
un groupe alkyle substitué par un groupe 2-pyridyle, 3-pyridyle, 4-pyridyle, 2-pyrazinyle, 3-chloro-5-trifluorométhyl-2-pyridyle, 1-méthyl-1*H*-pyrrol-2-yle, 1*H-*pyrazol-4-yle, 4-éthoxycarbonyl-1*H*-pyrazol-5-yle, 1*H*-imidazol-4-yle, 1*H*-imidazol-1-yle, 1*H*-indol-3-yle, 6-méthoxy-1*H*-benzimidazol-2-yle,
un groupe 1-pentyle substitué en position 5 par un groupe alcoxycarbonyle, qui est substitué en outre en position 5 par un groupe amino, alkylcarbonylamino, arylcarbonylamino, arylsulfonylamino, alcoxycarbonylamino ou aralcoxycarbonylamino,
où sauf indication contraire, les parties alkyle, alkylène et alcoxy mentionnées précédemment contiennent chacune, 1 à 4 atomes de carbone,
éventuellement sous la forme de leurs tautomères, leurs racémates, leurs énantiomères, leurs diastéréoisomères et leurs mélanges, ainsi que éventuellement leurs sels d'addition d'acide pharmacologiquement acceptables,
où, sauf indication contraire, chaque atome de carbone dans les parties alkyle, alkylène ou cycloalkylène mentionnées précédemment, qui est lié à un atome d'azote, d'oxygène ou de soufre, ne peut pas être lié à un autre atome d'halogène, d'azote, d'oxygène ou de soufre.

4. Composés de formule I selon l'une des revendications 1 à 3, dans lesquels
R_{b} représente un groupe 1-naphtyle ou un groupe 2-naphtyle éventuellement substitué en position 5 par un groupe carboxy,
un groupe 1,3-dihydro-2-oxoindol-6-yle, benzotriazol-5-yle, benzimidazol-5-yle, indazol-5-yle, indazol-6-yle ou 1-méthyl-1*H*-indazol-6-ylamino,
un groupe phényle éventuellement substitué en position 4 de la partie phényle, par un atome de fluor, de chlore ou de brome, un groupe cyano, propyl-2-sulfonyle, aminosulfonyle, méthylaminosulfonyle, diméthylaminosulfonyle, morpholinosulfonyle, N-méthylpipérazinosulfonyle, homopipérazinosulfonyle, 2,6-diméthyl-pipérazin-4-yle, 2-aminopyridyl-N-sulfonyle, carboxy, pipéridinométhyle, 1,2,4,5-tétrahydrobenzo[d]azépin-3-ylméthyle, 2-carboxy-1-éthyle, phénylaminocarbonyle, benzylaminocarbonyle, (alkyle en C₁₋₃)sulfonyle, aminocarbonyle, méthoxycarbonyle, (N-méthyl-N-méthylsulfonyl)amino, diéthylaminométhyle, 3-diéthylamino-1-propyloxy, morpholino, 4-méthyl-1-pipérazinyle, 2H-tétrazol-5-yle, 1H-imidazol-4-yle ou nitro,
un groupe phényle substitué en position 3 de la partie phényle, par un atome de chlore ou de brome, un groupe cyano, aminocarbonyle, carboxy, éthoxycarbonyle ou nitro ou par un groupe de la formule
-CH₂-NH-(CH₂)ᵣ-CₛFₛ₊₁,
où r représente 1 ou 2, et s représente 1, 2 ou 3,
un groupe 3,4-dichlorophényle, 3,5-dichlorophényle, 4-amino-3,5-dichlorophényle, 3-chloro-4-fluorophényle, 4-chloro-3-méthylphényle, 4-chloro-3-trifluorométhyl-phényle, 4-bromo-3-chlorophényle ou 3-hydroxy-4-méthylphényle, ou
un groupe de la formule : 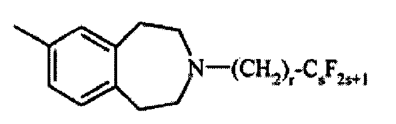 où r représente 1 ou 2, et s représente 1, 2 ou 3,
le groupe R_{c}NR_{d}
un groupe 1-pyrrolidinyle substitué en position 2 par un groupe hydroxyméthyle, 1-pyrrolidinylméthyle ou 2-éthoxycarbonyl-1-éthyle,
un groupe 1-pyrrolidinyle substitué en position 3 par un groupe amino, acétylamino, N-méthyl-acétylamino ou t-butyloxycarbonylamino,
un groupe 4-carboxy-3-thiazolidinyle, un groupe 7-méthyl-2,7-diazaspiro[4.4]non-2-yle ou un groupe 5-hydroxy-2-méthyl-2,8-diazaspiro[5.5]undéc-8-yle,
un groupe morpholino ou S-oxydothiomorpholino,
un groupe 1-pipéridinyle substitué en position 2 par un groupe éthoxycarbonyle, hydroxyméthyle, 3-hydroxypropyle, 3-diéthylamino-1-propyle ou 2-(2-diéthylaminoéthoxy)-1-éthyle,
un groupe 1-pipéridinyle substitué en position 3 par un groupe hydroxy, hydroxyméthyle, 3-diéthylamino-1-propyle, aminocarbonyle, diméthylaminocarbonyle, carboxy, 1-pyrrolidinylméthyle, 4-(1-pyrrolidinyl)-1-butyle, méthoxycarbonylméthyle ou acétylaminométhyle,
un groupe 1-pipéridinyle substitué en position 4 par un groupe éthoxycarbonyle, 3-hydroxypropyle, hydroxy, aminométhyle, 2-(2-diéthylaminoéthoxy)-1-éthyle, 2-carboxy-1-éthyle, N-(2-méthoxycarbonyl-1-éthyl)-N-méthylamino, 2-(N-(diméthylaminocarbonylméthyl)-N-méthylamino)-1-éthyle, N-acétyl-N-méthylaminométhyle, 8-méthoxy-3,4-dihydro-1*H*-quinazolin-2-on-3-yle, 1-pipéridinyle, 3-hydroxy-1-pipéridinyle ou 4-éthoxycarbonyle-1-pipéridinyle,
un groupe 3,5-diméthyl-1-pipérazinyle, 1,4,6,7-tétrahydroimidazo[4,5-c]pyridin-5-yle, 2-méthyl-1,4,6,7-tétrahydroimidazo[4,5-c]pyridin-5-yle, 1,4,5,6,7,8-hexahydro-imidazo[4,5-d]azépin-6-yle, 2-méthyl-1,4,5,6,7,8-hexahydroimidazo[4,5-d]azépin-6-yle, 3-phénylazépan-4-on-1-yle ou 4-carboxy-4-phényl-1-pipéridinyle,
un groupe 1-pipérazinyle, qui est substitué en position 4, éventuellement, par un groupe méthyle, acétyle, benzyloxycarbonyle, 2-pyridyle, 2-pyrimidinyle, 2-nitrophényle, 3-méthoxyphényle, 4-cyanophényle, 3,4-diméthoxyphényle, 4-[bis(4-méthoxyphényl)]méthyle, 8-méthyl-8-azabicyclo[3.2.1]oct-3-yle, morpholino-carbonylméthyle, 2-amino-1-éthyle ou 3-hydroxy-1-propyle,
un groupe homopipérazinyle, qui est substitué en position 4, éventuellement, par un groupe méthyle,
un groupe 3-hydroxy-8-azabicyclo[3.2.1]oct-8-yle, ou
R_{c} représente un atome d'hydrogène ou un groupe méthyle, éthyle, 2-méthoxyéthyle, 2-hydroxyéthyle, i-propyle, n-propyle, n-butyle, benzyle ou 3-pyridylméthyle, et
R_{d} représente un groupe méthyle substitué par un groupe de formule -C(=NH)NH₂, ou un groupe cyano, carboxyle, éthoxycarbonyle, aminocarbonyle, (alkyle en C₁₋₂)-carbonylamino-2,2-diméthyléthyle, (alkyle en C₁₋₂)carbonylamino-1,1-diméthyl-éthyle, (alkyle en C₁₋₂)carbonylamino-2,2-diméthylpropyle, carboxyméthylaminocarbonyle, 1-hydroxy-1-cyclohexyle, aminométhylcyclohexyle, 3-hydroxy-8-méthyl-8-azabicyclo[3.2.1]oct-3-yle, 3-hydroxy-1,3-dihydroindol-2-on-3-yle, 2-aminocarbonyl-1,3-dihydroisoindol-5-yle, 2-tétrahydrofuryle, 1-éthyl-2-pyrrolidinyle, 1*H-*imidazol-4-yle, 1-méthyl-4-pipéridinyle, 1-(2-diméthylaminoacétyl)-4-pipéridinyle, 2-pyridyle, 3-pyridyle, 4-pyridyle, 2-pyrazinyle, 3-chloro-5-trifluorométhyl-2-pyridyle, 4-éthoxycarbonyl-1*H*-pyrazol-5-yle, 2-carboxyphényle, 3-carboxyphényle, 2-hydroxyphényle, 4-hydroxyphényle, 2-nitrophényle, 3-nitrophényle, 4-nitrophényle, 3-aminophényle, 4-aminophényle, 4-(aminosulfonyl)phényle, 4'-hydroxybiphényle, 4-(aminométhyl)-phényle ou 4-hydroxy-3-méthoxyphényle,
un groupe alkyle en C₂₋₅ substitué par un groupe carboxy,
un groupe alkyle en C₂₋₅ substitué par un groupe hydroxy, amino ou diméthylamino, avec la condition que l'hétéroatome des substituants cités soit séparé de l'atome d'azote du groupe R_{c}NR_{d} par au moins deux atomes de carbone,
un groupe benzyle substitué dans la partie méthylène, par un groupe carboxy ou cyano,
un groupe méthyle substitué par un groupe carboxy ou un groupe 4-hydroxyphényle,
un groupe éthyle substitué en position 1 par un groupe méthoxycarbonyle ou un groupe 1H-benzimidazol-2-yle,
un groupe éthyle substitué en position 2 par un groupe méthoxy, diphénylméthoxy, méthylthio, méthylamino, diéthylamino, diisopropylamino, acétylamino, N-méthylacétylamino, 2-méthoxyacétylamino, 2-diméthylaminoacétylamino, isopropylcarbonylamino, 2-méthylpropylcarbonylamino, phénylacétylamino, t-butyloxycarbonylamino, méthylsulfonylamino, benzoylamino, phénylamino, 1-naphtylamino, 4-nitro-2-pyridylamino, cyano, éthoxycarbonyle, aminocarbonyle, méthyl-aminocarbonyle, diméthylaminocarbonyle, 2-hydroxy-1-éthoxy, 2-(2-amino-1-éthoxy)-1-éthoxy, 2-(2-hydroxy-1-éthoxy)-1-éthoxy, 2-amino-1-éthylthio, 1-méthyl-2-pyrrolidinyle, 1-pyrrolidinyle, 2-oxo-pyrrolidin-1-yle, 1-pipéridinyle, 2-oxopipéridin-1-yle, morpholino, 4-(2-hydroxyéthyl)-1-pipérazinyle, 2-(2-diméthyl-aminoéthyl)-1-pipéridinyle, 4-méthyl-1-pipérazinocarbonyle, 3-carboxy-2-méthoxyphényle, 2-hydroxyphényle, 3-hydroxyphényle, 4-hydroxyphényle, 4-(aminosulfonyl)phényle, 4-nitrophényle, 3-méthoxycarbonylphényle, 2-(2-amino-1-éthyl)phényle, 4-pyridyle, 1*H*-imidazol-1-yle, 1*H*-imidazol-4-yle, 1*H*-pyrazol-4-yle, 1-méthyl-1*H*-pyrrol-2-yle, 1*H*-indol-3-yle, 6-méthoxy-1*H*-benzimidazol-2-yle, 4-(2*H*-pyridazin-3-on-6-yl)phényle ou imidazolidin-2-on-1-yle,
un groupe éthyle substitué en position 1 par un groupe carboxy et en position 2, en outre, par un groupe hydroxy, aminocarbonyle, 2-chlorophényle, 4-chlorophényle, 1*H*-imidazol-4-yle ou 4-hydroxyphényle,
un groupe éthyle substitué en position 1 par un groupe aminocarbonyle, et en position 2, en outre, par un groupe 4-méthoxyphényle,
un groupe éthyle substitué en position 1 par un groupe 4-phényl-1-butylaminocarbonyle, et en position 2, en outre, par un groupe phényle,
un groupe éthyle substitué en position 2 par un groupe hydroxy et, en position 2, en outre par un groupe phényle, 3-hydroxyphényle, 4-hydroxyphényle ou 4-hydroxy-3-méthoxyphényle,
un groupe éthyle substitué en position 1 par un groupe phényle et, en position 2, en outre par un groupe hydroxy ou carboxy,
un groupe éthyle substitué en position 1 par un groupe 3-pyridyle et, en position 2, en outre par un groupe éthoxycarbonyle,
un groupe éthyle substitué en position 1 par un groupe carboxy et, en position 2, en outre par deux groupes phényle,
un groupe n-propyle substitué en position 2 par un groupe hydroxy et, en position 3, en outre par un groupe amino, hydroxy ou morpholino,
un groupe n-propyle substitué en position 3 par un groupe méthoxy, isopropylamino, méthylamino, diéthylamino, dibenzylamino, 1-pyrrolidinyle, 1-pipéridinyle, morpholino, 4-méthyl-1-pipérazinyle, t-butyloxycarbonylamino, 2-oxo-1-pyrrolidinyle, 2-oxo-1-pipéridinyle, éthoxycarbonyle, 4-pyridyle, 4-amino-3,5-dichlorophényle, 3-amino-1-propoxy, 2-(3-amino-1-propoxy)-1-éthoxy, 1*H-*imidazol-1-yle, 2-azabicyclo[2.2.1]hept-5-én-2-yle, 4-(3-amino-1-propyl)-1-pipérazinyle ou 2-diéthylaminométhyl-1-pipéridinyle,
un groupe n-butyle substitué en position 4 par un groupe 4-hydroxyphényle,
un groupe n-butyle substitué en position 4 par un groupe diméthylamino et en position 2, en outre, par un groupe phényle,
un groupe 2-méthyl-2-butyle substitué en position 3 par un groupe phénylaminocarbonyle ou 1-(4-pyridyl)-3-imidazolin-2-on-3-yle,
un groupe n-pentyle substitué en position 1 par un groupe carboxy, et en position 5, en outre, par un groupe benzyloxycarbonylamino,
un groupe 1-pentyle substitué en position 5 par un groupe méthoxycarbonyle, et en position 5, en outre, par un groupe acétylamino,
un groupe n-hexyle substitué en position 6 par un groupe hydroxy, amino, t-butyloxycarbonylamino ou N-méthyl-N-phénéthylamino,
un groupe cyclohexyle substitué en position 2 par un groupe hydroxy, amino, diméthylamino ou hydroxyméthyle,
un groupe cyclohexyle substitué en position 3 par un groupe amino ou carboxy
un groupe cyclohexyle substitué en position 4 par un groupe hydroxy, amino, carboxy, 2-carboxyéthyle, 3-carboxypropyle, méthoxycarbonyle ou diméthylamino,
un groupe cyclohexylméthyle substitué en position 3 de la partie cyclohexyle par un groupe aminométhyle ou t-butyloxycarbonylaminométhyle,
un groupe cyclohexylméthyle substitué en position 4 de la partie cyclohexyle par un groupe aminométhyle, diméthylaminométhyle ou 2-carboxyéthyle,
un groupe 4-pipéridinyle substitué en position 1 par un groupe méthyle, benzyle ou phénylsulfonyle,
un groupe 1-méthyl-4-carboxy-4-pipéridinyle,
un groupe 1-éthyl-3-pipéridinyle, 1-benzyl-3-pyrrolidinyle ou 5-(3-carboxy-1-propyl)indan-2-yle,
éventuellement sous la forme de leurs tautomères, leurs racémates, leurs énantiomères, leurs diastéréoisomères et leurs mélanges, ainsi que éventuellement leurs sels d'addition d'acide pharmacologiquement acceptables.

5. Composés de formule I selon l'une des revendications 1 à 4, dans lesquels
R_{b} représente un groupe phényle éventuellement substitué en position 4 de la partie phényle, par un atome de fluor, de chlore ou de brome, un groupe cyano, propyl-2-sulfonyle, aminosulfonyle, diméthylaminosulfonyle, carboxy, pipéridinométhyle, 1,2,4,5-tétrahydrobenzo[d]azépin-3-ylméthyle, 2-carboxy-1-éthyle, phénylaminocarbonyle, benzylaminocarbonyle, (alkyle en C₁₋₃)sulfonyle, aminocarbonyle, méthoxycarbonyle, (N-méthyl-N-méthylsulfonyl)amino, diéthylaminométhyle, 3-diéthylamino-1-propyloxy, morpholino, 4-méthyl-1-pipérazinyle, 2H-tétrazol-5-yle, 1H-imidazol-4-yle ou nitro ou un groupe de la formule
-CH₂-NH-CH₂-CₛFₛ₊₁,
où s représente 1 ou 2,
ou un groupe phényle substitué en position 3 de la partie phényle, par un atome de chlore ou de brome, un groupe cyano, aminocarbonyle, carboxy, éthoxycarbonyle ou nitro, ou un groupe 3,4-dichlorophényle, 3,5-dichlorophényle, 4-amino-3,5-dichlorophényle, 3-chloro-4-fluorophényle, 4-chloro-3-méthylphényle, 4-chloro-3-trifluorométhylphényle, 4-bromo-3-chlorophényle, 3-hydroxy-4-méthylphényle, benzotriazol-5-yle, benzimidazol-5-yle, indazol-5-yle ou indazol-6-yle, ou un groupe de la formule : 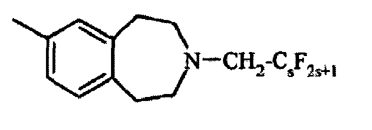 où s représente 1 ou 2.

6. Composés de formule I selon l'une des revendications 1 à 5, dans lesquels
le groupe R_{c}NR_{d} est choisi parmi les groupes suivants :
2-amino-1-éthylamino, 2-acétylamino-éthylamino, 2-aminocarbonyl-1-éthylamino,
2-méthoxy-1-éthylamino, 2-morpholino-1-éthylamino, 3-aminopropylamino, 1-carboxy-2-propylamino, 4-aminobutylamino, 5-hydroxy-1-pentylamino, 3-(3-aminopropoxy)-1-propylamino, 2-(3-hydroxyphényl)-1-éthylamino, 2-(4-hydroxy-3-méthoxyphényl)-2-hydroxy-1-éthylamino, 2-(2-(2-amino-1-éthyl)phényl)-1-éthylamino, 4-hydroxycyclohexylamino, 3-aminocyclohexylamino, 4-aminométhylcyclohexylamino, 4-diméthylaminocyclohexylamino, 1-méthyl-pipéridin-4-ylméthylamino, N-(4-méthylpipéridin-4-yl)-N-méthylamino, 3-(2-oxo-pyrrolidin-1-yl)propyl-1-amino, 1,4,6,7-tétrahydroimidazo[4,5-c]pyridin-5-yle, 2-hydroxyméthylpyrrolidin-4-yle, 4-aminométhylpipéridin-1-yle, 3-hydroxyméthyl-pipéridin-1-yle, 3-acétylaminométhylpipéridin-1-yle, 4-(N-acétyl-N-méthylamino-méthyl)pipéridin-1-yle, 3-(4-(pyrrolidin-1-yl)butyl)pipéridin-1-yle, 3-(2-azabicyclo-[2.2.1]hept-5-én-2-yle)propylamino, 7-méthyl-2,7-diazaspiro[4.4]non-2-yle.

7. Sels physiologiquement acceptables des composés de formule I selon l'une des revendications 1 à 6.

8. Composés de formule I selon l'une des revendications 1 à 6, à utiliser comme agent pharmaceutique.

9. Procédé de préparation des composés de formule I selon l'une des revendications 1 à 6, **caractérisé en ce que**
a. on fait réagir un composé de la formule générale : 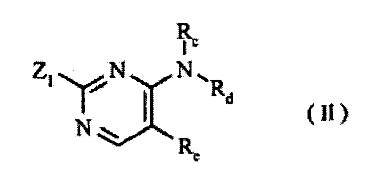 dans laquelle
R_{c} et R_{d} sont définis comme indiqué aux revendications 1 à 6, et
Z₁ représente un groupe partant,
avec une amine de formule générale :
H-(RₐNR_{b}) (III)
dans laquelle
Rₐ et R_{b} sont définis comme indiqué aux revendications 1 à 6 ; ou
b. on fait réagir un composé de formule générale IV : 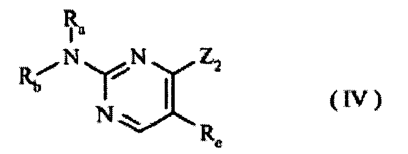 dans laquelle
RₐR_{b} et Rₑ sont définis comme indiqué aux revendications 1 à 6 ; et
Z₂ représente un groupe partant,
avec une amine de formule générale :
H-(R_{c}NR_{d}) (V)
dans laquelle R_{c} et R_{d} sont définis comme indiqué aux revendications 1 à 6.

10. Utilisation des composés de formule I selon l'une des revendications 1 à 6, pour la préparation d'un médicament pour la thérapie et pour la prévention de maladies, qui sont **caractérisées par** une prolifération cellulaire excessive ou anormale.

11. Composition pharmaceutique **caractérisée par le fait qu'**elle contient un ou plusieurs des composés de formule I selon l'une des revendications 1 à 6.
